# EUROPEAN PATENT APPLICATION

(11) **EP 4 321 522 A1**
(43) Date of publication of application: **14.02.2024**
(21) Application number: 22202077.8
(22) Date of filing: 18.10.2022
(51) Int. Cl.: C07K 5/02, A61K 47/68, A61P 35/00

(54) **CYTOTOXIC COMPOUNDS AND CONJUGATES THEREOF**

(30) Priority: 12.08.2022 US 202263397776 P
(71) Applicant: Seagen Inc., Bothell, WA 98021 (US)
(72) Inventor: Moquist, Philip, Bothell, 98021 (US); Doronina, Svetlana, Bothell, 98021 (US); Duncan, Nicole, Bothell, 98021 (US)
(74) Representative: Forresters IP LLP

(57) **Abstract**

Hydrophilic auristatin compounds are described, including Drug-Linker compounds, Ligand-Drug Conjugate compounds, methods of use, and preparations thereof.

## Description

### BACKGROUND OF THE INVENTION

A variety of ligands have been investigated for the targeted delivery of cytotoxic agents to tumor cells, including oligopeptides, antibodies, and other proteins. While various drug classes have been evaluated for targeted delivery by these ligands, only a few drug classes have proved sufficiently active as Ligand-Drug Conjugates, while having a suitable toxicity profile and other pharmacological properties, to warrant clinical development. There is thus a need for additional cytotoxic agents with improved toxicity profile and other pharmacological properties.

### BRIEF SUMMARY OF THE INVENTION

Provided herein is a compound of Formula (I): 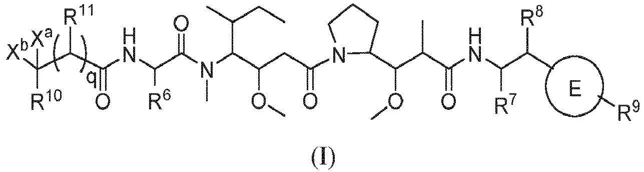 or a salt thereof, wherein
X^{b} is -NR¹R²; and X^{a} is 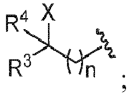 or
X^{a} and X^{b} are taken together with the carbon atom to which they are attached to form 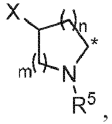 wherein the asterisk denotes the carbon atom of Formula (I) that bears the X^{a} and X^{b} groups;
R¹, R², R³, R⁴, R^{a}, R^{b}, R⁵, and R¹⁰ are each independently H or C₁-C₄ alkyl;
X is H, OH, -C(O)NR^{a}R^{b}, -S(O)₂R^{a}, -S(O)-R^{a} -S(O)₂NR^{a}R^{b}, -NHS(O)₂R^{a}, or -NHC(O)R^{a};
R⁶ is C₁-C₄ alkyl optionally substituted with OH;
R⁷ is H, C₁-C₄ alkyl optionally substituted with one or two OH moieties, or 5-6 membered heteroaryl;
E is phenyl or 5-6 membered heteroaryl;
R⁸, R⁹, and R¹¹ are each independently H or OH;
n is 0, 1, 2, or 3;
m is 1, 2, 3, or 4; and
q is 0 or 1,
wherein when X is H, at least two of R⁷, R⁸, R⁹, and R¹¹ comprise an OH moiety.

Also provided herein is a compound of Formula (II): 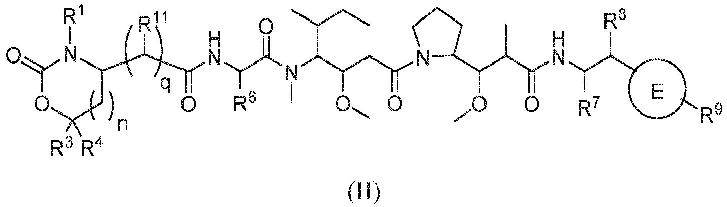 or a salt thereof, wherein
R¹, R³, and R⁴ are independently H or C₁-C₄ alkyl;
R⁶ is C₁-C₄ alkyl optionally substituted with OH;
R⁷ is H, C₁-C₄ alkyl optionally substituted with one or two OH moieties, or 5-6 membered heteroaryl;
E is phenyl or 5-6 membered heteroaryl;
R⁸, R⁹, and R¹¹ are each independently H or OH;
n is 0, 1, or 2; and
q is 0 or 1.

In some embodiments of Formula (I) and (II), q is 0. In some embodiments, q is 1. In some embodiments, R¹¹ is H. In some embodiments, R¹¹ is OH.

In some embodiments of Formula (I), X is OH. In some embodiments, X is -C(O)NR^{a}R^{b}, -S(O)₂R^{a}, -S(O)-R^{a} -S(O)₂NR^{a}R^{b}, -NHS(O)₂R^{a}, or -NHC(O)R^{a}. In some embodiments, X^{b} is -NR¹R² and X^{a} is 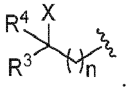 In some embodiments, R³ is H. In some embodiments, R⁴ is H.

In some embodiments, n is 0 or 1. In some embodiments, X^{a} and X^{b} are taken together with the carbon atom to which they are attached to form 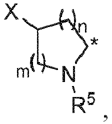 wherein the asterisk denotes the carbon atom of Formula (I) that bears the X^{a} and X^{b} groups. In some embodiments, m is 2 or 3.

In some embodiments of Formula (I) or (II), R¹ is H. In some embodiments, R¹ is C₁-C₄ alkyl. In some embodiments, R¹ is methyl. In some embodiments, R² is methyl. In some embodiments, R¹⁰ is H. In some embodiments, R¹⁰ is methyl. In some embodiments, R⁶ is unsubstituted C₁-C₄ alkyl. In some embodiments, R⁶ is isopropyl. In some embodiments, R⁶ is C₁-C₄ alkyl substituted with OH. In some embodiments, R⁷ C₁-C₄ alkyl substituted with OH. In some embodiments, R⁷ is -CH₂OH. In some embodiments, R⁷ is H. In some embodiments, R⁷ is unsubstituted C₁-C₄ alkyl. In some embodiments, R⁷ is methyl. In some embodiments, R⁷ is 5-6 membered heteroaryl. In some embodiments, R⁸ is H. In some embodiments, R⁸ is OH. In some embodiments, E is phenyl. In some embodiments, E-R⁹ is 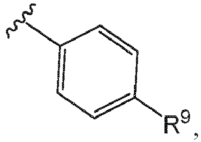 wherein the wavy line indicates the point of attachment of E to the rest of the compound. In some embodiments, E is 5-6 membered heteroaryl. In some embodiments, R⁹ is H. In some embodiments, R⁹ is OH.

In some embodiments of Formula (I),
X^{a} is 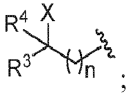
X^{b} is -NR¹R²;
R¹ is H or methyl;
R² is methyl;
X is OH;
R³ and R⁴ are H;
R⁶ is isopropyl;
R⁷ is -CH₂OH;
R⁸ is H;
E is phenyl; and
R⁹ is H.

In some embodiments of Formula (I), the compound is 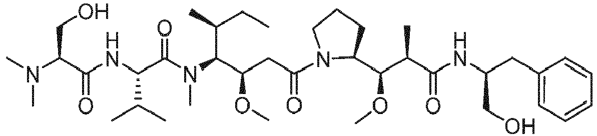 or a salt thereof.

In some embodiments, the compound is a compound of Table 1.

Also provided herein is a Drug-Linker compound of the following formula:

Q-D,

or a salt thereof, wherein
Q is a Linker Unit selected from the group consisting of:
   (i) Z'-A-RL-,
   (ii) Z'-A-RL-Y-,
   (iii) Z'-A-S^{∗}-RL-,
   (iv) Z'-A-S^{∗}-RL-Y-,
   (v) Z'-A-B(S^{∗})-RL-,
   (vi) Z'-A-B(S^{∗})-RL-Y-,
   (vii) Z'-A-,
   (viii) Z'-A-S^{∗}-W-,
   (ix) Z'-A-B(S^{∗})-W-,
   (x) Z'-A-S^{∗}-W-RL-, and
   (xi) Z'-A-B(S^{∗})-W-RL-;
Z' is a Stretcher Unit precursor;
A is a bond or a Connector Unit;
B is a Parallel Connector Unit;
S^{∗} is a Partitioning Agent;
RL is a Releasable Linker;
W is an Amino Acid Unit;
Y is a Spacer Unit; and
D is a Drug Unit of Formula (I'): 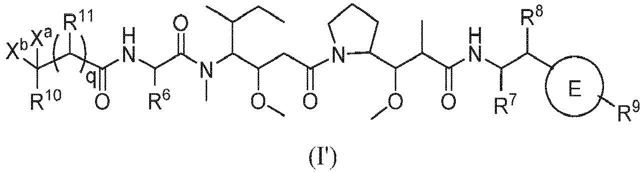 wherein
X^{b} is -NR²-^{#} or -N⁺R¹R⁵-^{#}, wherein the # denotes the point of attachment to Q, and X^{a} 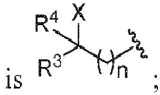 or
X^{a} and X^{b} are taken together with the carbon atom to which they are attached to form 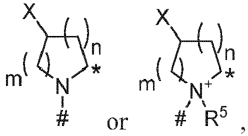 wherein the asterisk denotes the carbon atom of Formula (I) that bears the X^{a} and X^{b} groups, and the # denotes the point of attachment to Q;
R¹ and R⁵ are independently C₁-C₄ alkyl;
X is H, OH, -C(O)NR^{a}R^{b}, -S(O)₂R^{a}, -S(O)-R^{a} -S(O)₂NR^{a}R^{b}, -NHS(O)₂R^{a}, or -NHC(O)R^{a};
R², R³, R⁴, R¹⁰, R^{a}, and R^{b} are each independently H or C₁-C₄ alkyl;
R⁶ is C₁-C₄ alkyl optionally substituted with OH;
R⁷ is H, C₁-C₄ alkyl optionally substituted with one or two OH moieties, or 5-6 membered heteroaryl;
E is phenyl or 5-6 membered heteroaryl;
R⁸, R⁹, and R¹¹ are each independently H or OH;
n is 0, 1, 2, or 3;
m is 1, 2, 3, or 4;
q is 0 or 1; and
wherein when X is H, at least two of R⁷, R⁸, R⁹, and R¹¹ comprise an OH moiety.

In some embodiments, the Linker Unit Q is of formula (i), (ii), (iii), (iv), (x), or (xi). In some embodiments, the Linker Unit Q is of formula (v), (vi), (ix), or (xi). In some embodiments, the Linker Unit Q is of formula (viii), (ix), (x), or (xi).

In some embodimemts, the Stretcher Unit Z' is 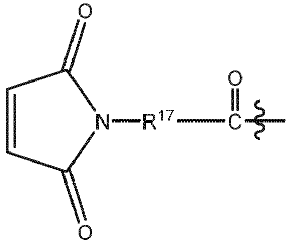 wherein
R¹⁷ is -CH₂CH₂(OCH₂CH₂)ₖ-, -C₁-C₁₀ alkylene-, C₁-C₁₀ heteroalkylene-, -C₃-C₈ carbocyclo-, -O-(C₁-C₈ alkylene)-, -arylene-, -C₁-C₁₀ alkylene-arylene-, -arylene-C₁-C₁₀ alkylene-, -C₁-C₁₀ alkylene-(C₃-C₈ carbocyclo)-, -(C₃-C₈ carbocyclo)-C₁-C₁₀ alkylene-, -C₃-C₈ heterocyclo-, -C₁-C₁₀ alkylene-(C₃-C₈ heterocyclo)-, -(C₃-C₈ heterocyclo)-Ci-Cio alkylene-, -C₁-C₁₀ alkylene-C(=O)-, C₁-C₁₀ heteroalkylene-C(=O)-, -C₃-C₈ carbocyclo-C(=O)-, -O-(C₁-C₈ alkylene)-C(=O)-, -arylene-C(=O)-, -C₁-C₁₀ alkylene-arylene-C(=O)-, -arylene-Ci-Cio alkylene-C(=O)-, -C₁-C₁₀ alkylene-(C₃-C₈ carbocyclo)-C(=O)-,-(C₃-C₈ carbocyclo)-C₁-C₁₀ alkylene-C(=O)-, -C₃-C₈ heterocyclo-C(=O)-, -C₁-C₁₀ alkylene-(C₃-C₈ heterocyclo)-C(=O)-, - (C₃-C₈ heterocyclo)-C₁-C₁₀ alkylene-C(=O)-, -C₁-C₁₀ alkylene-NH-, C₁-C₁₀ heteroalkylene-NH-, -C₃-C₈ carbocyclo-NH-, -O-(C₁-C₈ alkylene)-NH-, -arylene-NH-, -C₁-C₁₀ alkylene-arylene-NH-, -arylene-C₁-C₁₀ alkylene-NH-, -C₁-C₁₀ alkylene-(C₃-C₈ carbocyclo)-NH-, -(C₃-C₈ carbocyclo)-C₁-C₁₀ alkylene-NH-, -C₃-C₈ heterocyclo-NH-, -C₁-C₁₀ alkylene-(C₃-C₈ heterocyclo)-NH-, -(C₃-C₈ heterocyclo)-C₁-C₁₀ alkylene-NH-, -C₁-C₁₀ alkylene-S-, C₁-C₁₀ heteroalkylene-S -, -C₃-C₈ carbocyclo-S -, -O-(C₁-C₈ alkylene)-S -, -arylene-S-, -C₁-C₁₀ alkylene-arylene-S-, -arylene-C₁-C₁₀ alkylene-S-, -C₁-C₁₀ alkylene-(C₃-C₈ carbocyclo)-S-, - (C₃-C₈ carbocyclo)-C₁-C₁₀ alkylene-S-, -C₃-C₈ heterocyclo-S-, -C₁-C₁₀ alkylene-(C₃-C₈ heterocyclo)-S-, or -(C₃-C₈ heterocyclo)-Ci-Cio alkylene-S-;
subscript k is an integer ranging from 1 to 36;
R¹⁷ is optionally substituted by a Basic Unit (BU) such as an aminoalkyl moiety, e.g. -(CH₂)ₓNH₂, -(CH₂)ₓNHR^{a}, and -(CH₂)ₓNR^{a}₂, wherein x is an integer of from 1-4 and each R^{a} is independently selected from the group consisting of C₁₋₆ alkyl and C₁₋₆ haloalkyl, or two R^{a} groups are combined with the nitrogen to which they are attached to form an azetidinyl, pyrrolidinyl or piperidinyl group; and
the wavy line indicates the point of covalent attachment to the rest of the Drug-Linker compound.

In some embodiments, the Stretcher Unit Z' is 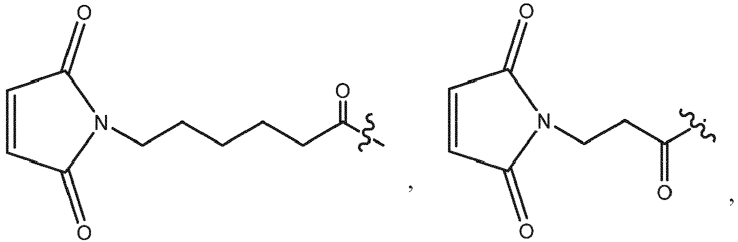 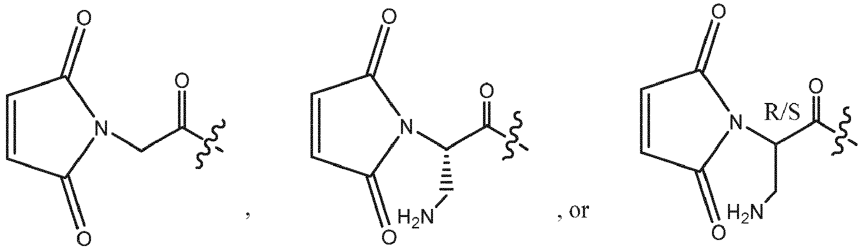 wherein the wavy lines indicate the point of covalent attachment to the rest of the Drug-Linker compound.

In some embodiments, the Connector Unit A is 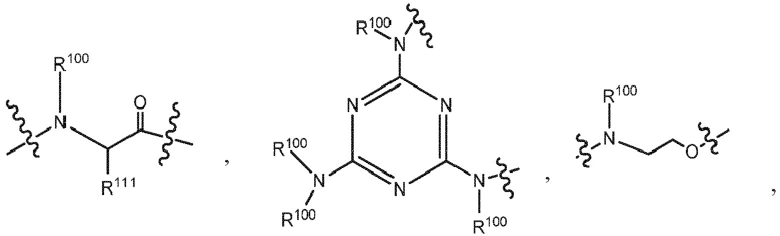 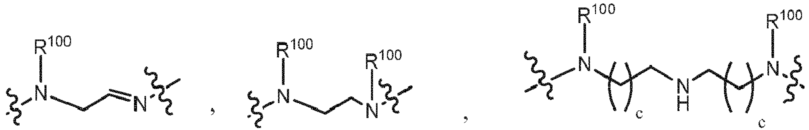 or 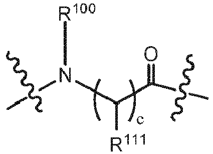 wherein
each R¹⁰⁰ is independently selected from hydrogen or -C₁-C₃ alkyl;
R¹¹¹ is independently selected from the group consisting of hydrogen, p-hydroxybenzyl, methyl, isopropyl, isobutyl, sec-butyl, -CH₂OH, -CH(OH)CH₃, - CH₂CH₂SCH₃, -CH₂CONH₂, -CH₂COOH, -CH₂CH₂CONH₂, -CH₂CH₂COOH, - (CH₂)₃NHC(=NH)NH₂, -(CH₂)₃NH₂, -(CH₂)₃NHCOCH₃, -(CH₂)₃NHCHO, - (CH₂)₄NHC(=NH)NH₂, -(CH₂)₄NH₂, -(CH₂)₄NHCOCH₃, -(CH₂)₄NHCHO, - (CH₂)₃NHCONH₂, -(CH₂)₄NHCONH₂, -CH₂CH₂CH(OH)CH₂NH₂, 2-pyridylmethyl-, 3-pyridylmethyl-, 4-pyridylmethyl-, 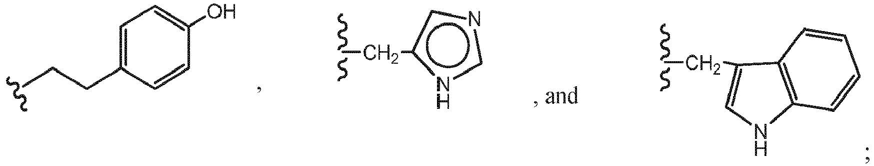
each subscript c is an independently selected integer from 1 to 10; and
the wavy lines indicate attachment of the Connector Unit to the rest of the Drug-Linker compound.

In some embodiments, the Connector Unit A is 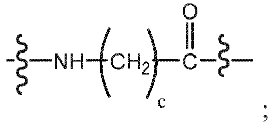
c is an integer ranging from 1 to 6; and
the wavy lines indicate the site of attachment to the rest of the Drug-Linker compound or salt thereof.

In some embodiments, A is a bond.

In some embodiments, B is 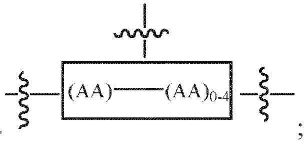
each AA is independently a proteinogenic or non-proteinogenic amino acid; and
the wavy lines indicate points of attachment to the rest of the Drug-Linker compound or salt thereof.

In some embodiments, B is an amino acid. In some embodiments, B is 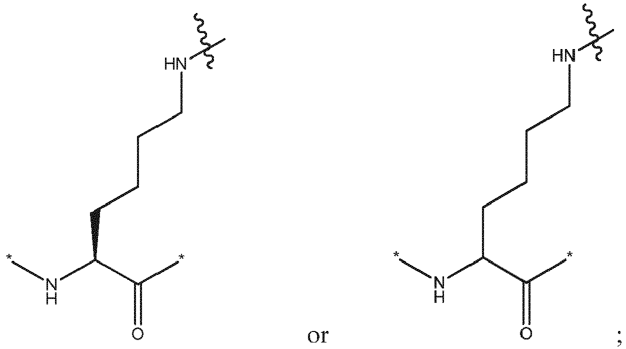
the wavy line indicates the point of attachement to the Partitioning Agent S^{∗}; and
the asterisks indicate points of attachment to the rest of the Drug-Linker structure.

In some embodiments, the Partitioning Agent S^{∗} is a polyethylene glycol (PEG) unit, cyclodextrin unit, polyamide, hydrophilic peptide, polysaccharide, or dendrimer. In some embodiments, Partitioning Agent S^{∗} is a PEG Unit comprising from 4 to 72 (CH₂CH₂O) subunits. In some embodiments, the PEG Unit is 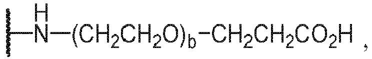 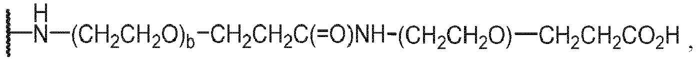 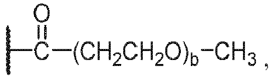 or 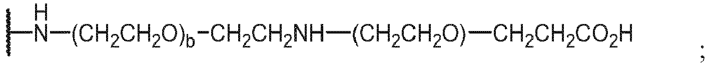
b is selected from the group consisting of 4 to 36; and
the wavy lines indicate the site of attachment to the rest of the Drug-Linker compound or salt thereof.

In some embodiments, the Releasable Linker RL is
-(AA)₁₋₁₂-; and
each AA is independently a proteinogenic or non-proteinogenic amino acid.

In some embodiments, the Releasable Linker RL is -AA₁-AA₂- or -AA₁-AA₂-AA₃-, wherein AA₁ is attached to the Stretcher Unit Z' or the Connector Unit A. In some embodiments, the Releasable Linker RL is 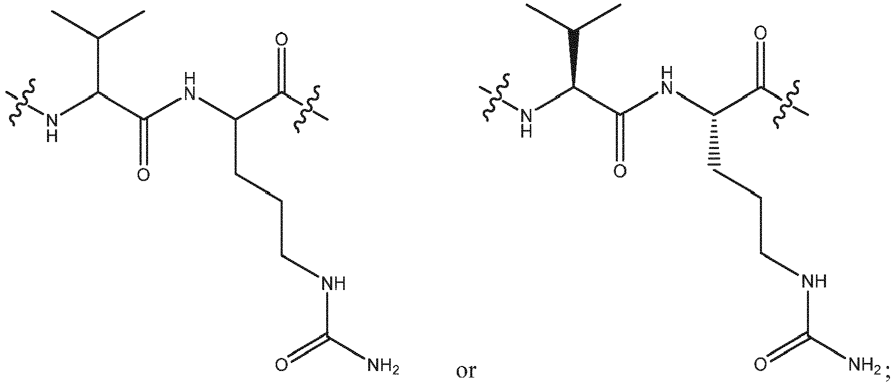 and
the wavy line adjacent to the -NH- group indicates attachment to the Stretcher Unit Z' or the Connector Unit A and the wavy line adjacent to the -C(=O)- group indicates attachment to the Spacer Unit Y or the Drug Unit D.

In some embodiments, the Releasable Linker RL is a glycoside. In some embodiments, the Releasable Linker RL is 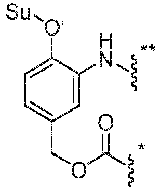 wherein
Su is a hexose form a monosaccharide;
O' represents the oxygen atom of a glycosidic bond that is capable of cleavage by a glycosidase;
the wavy line marked with a single asterisk (^{∗}) indicates the site of covalent attachment to D; and
the wavy line marked with a double asterisk (^{∗∗}) indicates the site of covalent attachment to the remainder of Q.

In some embodiments, the Releasable Linker RL is 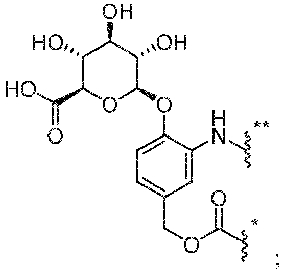
the wavy line marked with a single asterisk (^{∗}) indicates the site of covalent attachment to D; and
the wavy line marked with a double asterisk (^{∗∗}) indicates the site of covalent attachment to the remainder of Q.

In some embodiments, the Spacer Unit Y is 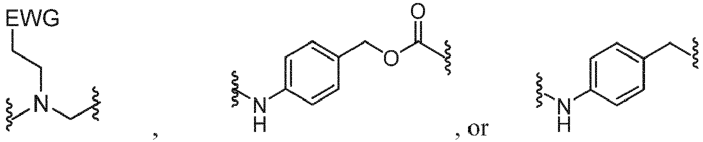
wherein EWG is an electron-withdrawing group; and
the wavy lines indicate the site of attachment to the rest of the Drug-Linker compound or salt thereof.

In some embodiments, the Spacer Unit Y is 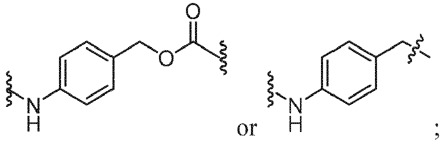 and
the wavy lines indicate the site of attachment to the rest of the Drug-Linker compound or salt thereof.

In some embodiments,
Z' is 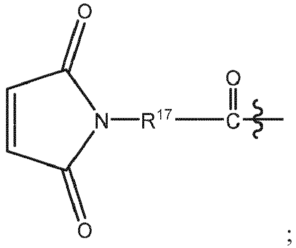
R¹⁷ is C₁-C₁₀ alkylene;
A is a bond;
RL is -AA₁-AA₂-;
AA₁ and AA₂ are each independently a proteinogenic amino acid;
Y is 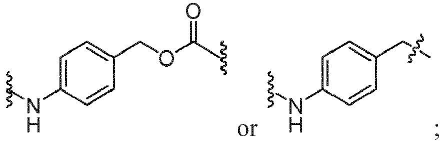 and
the wavy lines indicate the site of attachment to the rest of the Drug-Linker compound or salt thereof.

In some embodiments,
Z' is 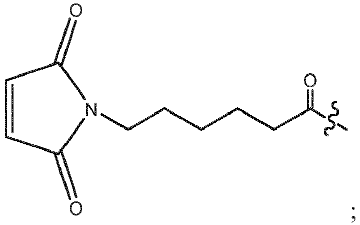
A is a bond;
RL is 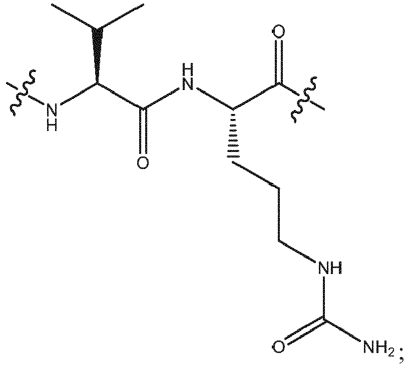 and
Y is

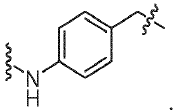

In some embodiments, Y-D is 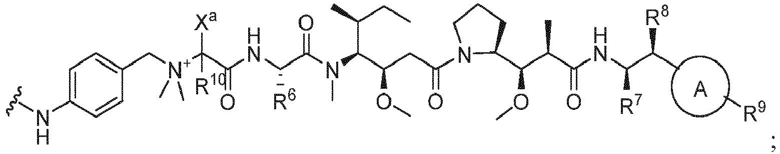 and
the wavy line indicates the site of attachment to the rest of the Drug-Linker compound or salt thereof.

In some embodiments, Y-D is 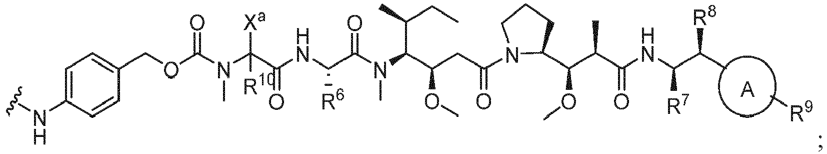 and
the wavy line indicates the site of attachment to the rest of the Drug-Linker compound or salt thereof.

In some embodiments, the compound is 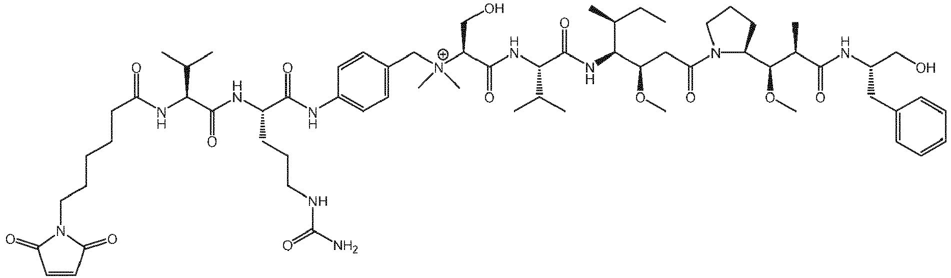 or a salt thereof.

In some embodiments, the compound is 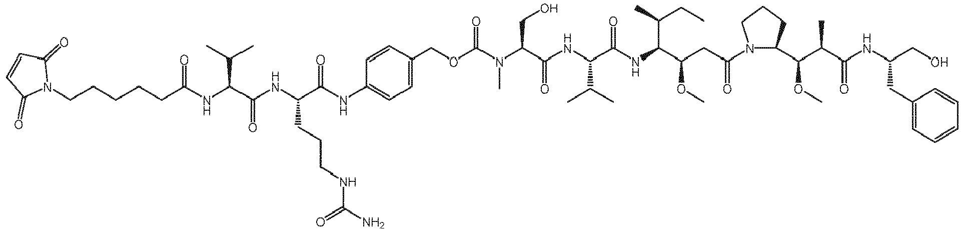 or 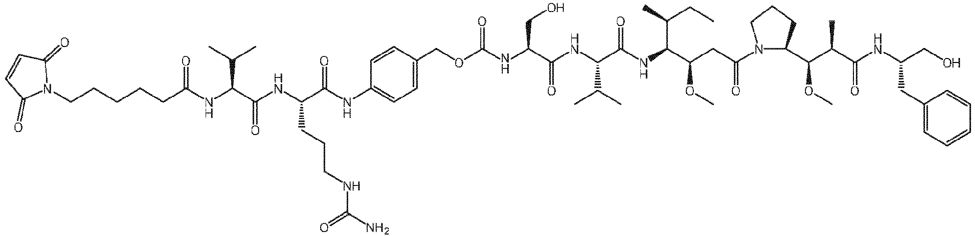 or a salt thereof.

In some embodiments, the Drug-Linker compound is a compound of Table 2.

Also provided herein is a Ligand-Drug Conjugate compound of the formula:

L-(Q-D)ₚ

or a pharmaceutically acceptable salt thereof, wherein
L is a Ligand Unit;
Q is a Linker Unit selected from the group consisting of:
   (i) Z'-A-RL-,
   (ii) Z'-A-RL-Y-,
   (iii) Z'-A-S^{∗}-RL-,
   (iv) Z'-A-S^{∗}-RL-Y-,
   (v) Z'-A-B(S^{∗})-RL-,
   (vi) Z'-A-B(S^{∗})-RL-Y-,
   (vii) Z'-A-,
   (viii) Z'-A-S^{∗}-W-,
   (ix) Z'-A-B(S^{∗})-W-,
   (x) Z'-A-S^{∗}-W-RL-, and
   (xi) Z'-A-B(S^{∗})-W-RL-;
Z' is a Stretcher Unit precursor;
A is a bond or a Connector Unit;
B is a Parallel Connector Unit;
S^{∗} is a Partitioning Agent;
RL is a Releasable Linker;
W is a Amino Acid Unit;
Y is a Spacer Unit; and
D is a Drug Unit of Formula (I'): 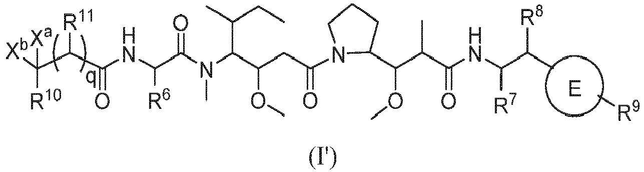 wherein

X^{b} is -NR²-^{#} or -N⁺R¹R⁵-^{#}, wherein the # denotes the point of attachment to Q, and X^{a} 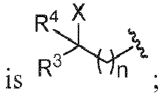 or
X^{a} and X^{b} are taken together with the carbon atom to which they are attached to form 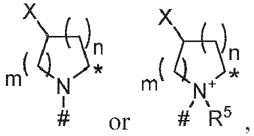 wherein the asterisk denotes the carbon atom of Formula (I) that bears the X^{a} and X^{b} groups, and the # denotes the point of attachment to Q;
R¹ and R⁵ are independently C₁-C₄ alkyl;
X is H, OH, -C(O)NR^{a}R^{b}, -S(O)₂R^{a}, -S(O)-R^{a} -S(O)₂NR^{a}R^{b}, -NHS(O)₂R^{a}, or -NHC(O)R^{a};
R², R³, R⁴, R¹⁰, R^{a}, and R^{b} are each independently H or C₁-C₄ alkyl;
R⁶ is C₁-C₄ alkyl optionally substituted with OH;
R⁷ is H, C₁-C₄ alkyl optionally substituted with one or two OH moieties, or 5-6 membered heteroaryl;
E is phenyl or 5-6 membered heteroaryl;
R⁸, R⁹, and R¹¹ are each independently H or OH;
n is 0, 1, 2, or 3;
m is 1, 2, 3, or 4;
q is 0 or 1; and
wherein when X is H, at least two of R⁷, R⁸, R⁹, and R¹¹ comprise an OH moiety.

In some embodiments, the Linker Unit Q is of formula (i), (ii), (iii), (iv), (x), or (xi). In some embodiments, the Linker Unit Q is of formula (v), (vi), (ix), or (xi). In some embodiments, the Linker Unit Q is of formula (viii), (ix), (x), or (xi).

In some embodiments, the Ligand Unit L and the Stretcher Unit Z together are 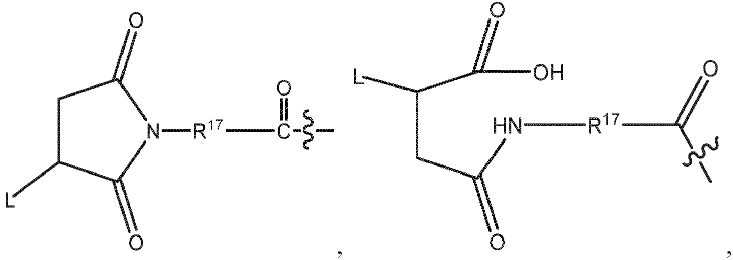 or 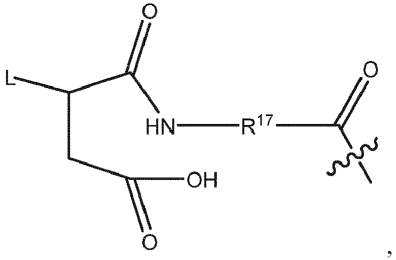 wherein
R¹⁷ is -CH₂CH₂(OCH₂CH₂)ₖ-, -C₁-C₁₀ alkylene-, C₁-C₁₀ heteroalkylene-, -C₃-C₈ carbocyclo-, -O-(C₁-C₈ alkylene)-, -arylene-, -C₁-C₁₀ alkylene-arylene-, -arylene-C₁-C₁₀ alkylene-, -C₁-C₁₀ alkylene-(C₃-C₈ carbocyclo)-, -(C₃-C₈ carbocyclo)-C₁-C₁₀ alkylene-, -C₃-C₈ heterocyclo-, -C₁-C₁₀ alkylene-(C₃-C₈ heterocyclo)-, -(C₃-C₈ heterocyclo)-C₁-C₁₀ alkylene-, -C₁-C₁₀ alkylene-C(=O)-, C₁-C₁₀ heteroalkylene-C(=O)-, -C₃-C₈ carbocyclo-C(=O)-, -O-(C₁-C₈ alkylene)-C(=O)-, -arylene-C(=O)-, -C₁-C₁₀ alkylene-arylene-C(=O)-, -arylene-Ci-Cio alkylene-C(=O)-, -C₁-C₁₀ alkylene-(C₃-C₈ carbocyclo)-C(=O)-,-(C₃-C₈ carbocyclo)-C₁-C₁₀ alkylene-C(=O)-, -C₃-C₈ heterocyclo-C(=O)-, -C₁-C₁₀ alkylene-(C₃-C₈ heterocyclo)-C(=O)-, - (C₃-C₈ heterocyclo)-Ci-Cio alkylene-C(=O)-, -C₁-C₁₀ alkylene-NH-, C₁-C₁₀ heteroalkylene-NH-, -C₃-C₈ carbocyclo-NH-, -O-(C₁-C₈ alkylene)-NH-, -arylene-NH-, -C₁-C₁₀ alkylene-arylene-NH-, -arylene-Ci-Cio alkylene-NH-, -C₁-C₁₀ alkylene-(C₃-C₈ carbocyclo)-NH-, -(C₃-C₈ carbocyclo)-C₁-C₁₀ alkylene-NH-, -C₃-C₈ heterocyclo-NH-, -C₁-C₁₀ alkylene-(C₃-C₈ heterocyclo)-NH-, -(C₃-C₈ heterocyclo)-Ci-Cio alkylene-NH-, -C₁-C₁₀ alkylene-S-, C₁-C₁₀ heteroalkylene-S-, -C₃-C₈ carbocyclo-S -, -O-(C₁-C₈ alkylene)-S -, -arylene-S-, -C₁-C₁₀ alkylene-arylene-S-, -arylene-C₁-C₁₀ alkylene-S-, -C₁-C₁₀ alkylene-(C₃-C₈ carbocyclo)-S-, - (C₃-C₈ carbocyclo)-C₁-C₁₀ alkylene-S-, -C₃-C₈ heterocyclo-S-, -C₁-C₁₀ alkylene-(C₃-C₈ heterocyclo)-S-, or -(C₃-C₈ heterocyclo)-Ci-Cio alkylene-S-;
subscript k is an integer ranging from 1 to 36;
R¹⁷ is optionally substituted by a Basic Unit (BU) such as an aminoalkyl moiety, e.g. -(CH₂)ₓNH₂, -(CH₂)ₓNHR^{a}, and -(CH₂)ₓNR^{a}₂, wherein x is an integer of from 1-4 and each R^{a} is independently selected from the group consisting of C₁₋₆ alkyl and C₁₋₆ haloalkyl, or two R^{a} groups are combined with the nitrogen to which they are attached to form an azetidinyl, pyrrolidinyl or piperidinyl group; and
the wavy line indicates the point of covalent attachment to the rest of the Ligand-Drug Conjugate compound.

In some embodiments, the Ligand Unit L and the Stretcher Unit Z together are 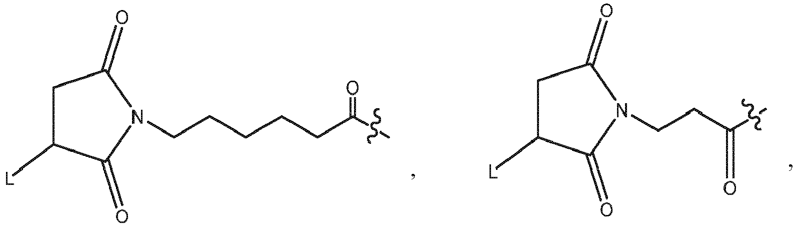 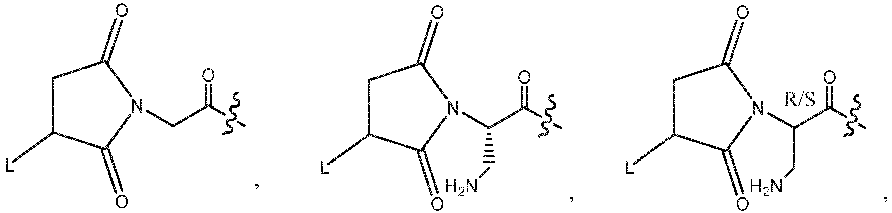 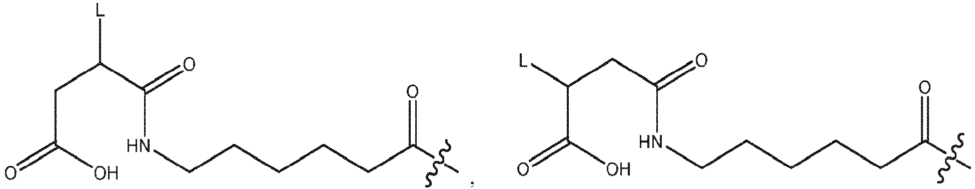 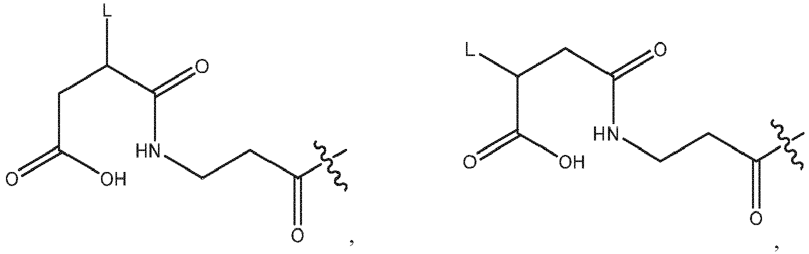 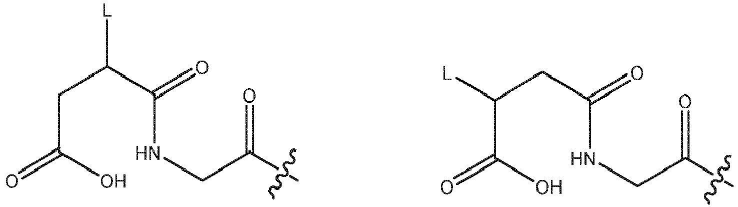 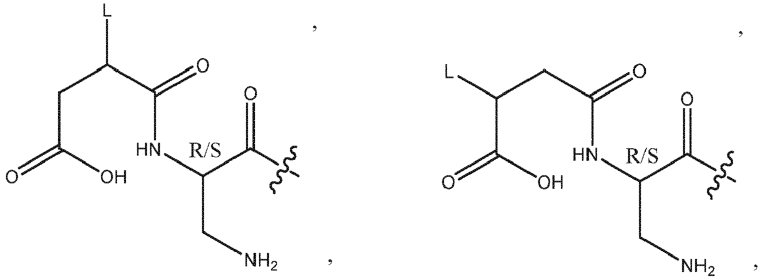 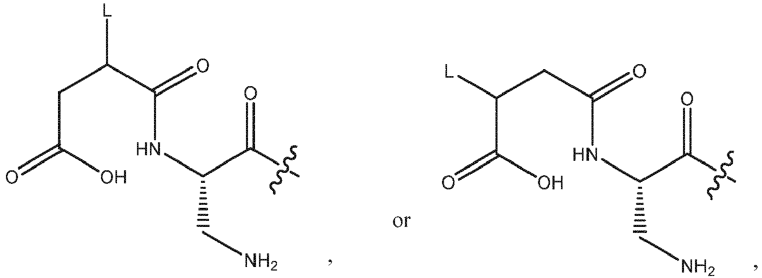 wherein the wavy lines indicate the point of covalent attachment to the rest of the Ligand-Drug Conjugate compound.

In some embodiments, the Connector Unit A is 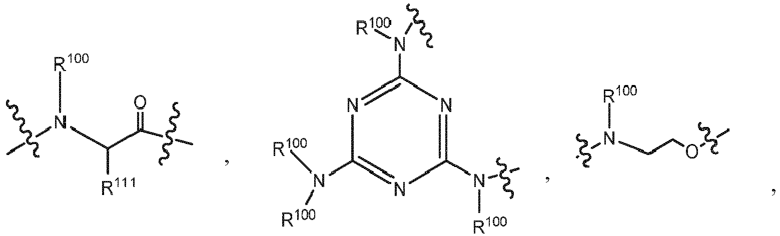 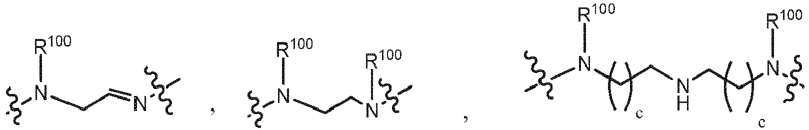 or 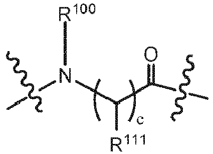 wherein
each R¹⁰⁰ is independently selected from hydrogen or -C₁-C₃ alkyl;
R¹¹¹ is independently selected from the group consisting of hydrogen, p-hydroxybenzyl, methyl, isopropyl, isobutyl, sec-butyl, -CH₂OH, -CH(OH)CH₃, -CH₂CH₂SCH₃, -CH₂CONH₂, -CH₂COOH, -CH₂CH₂CONH₂, -CH₂CH₂COOH, -(CH₂)₃NHC(=NH)NH₂, -(CH₂)₃NH₂, -(CH₂)₃NHCOCH₃, -(CH₂)₃NHCHO, -(CH₂)₄NHC(=NH)NH₂, -(CH₂)₄NH₂, -(CH₂)₄NHCOCH₃, -(CH₂)₄NHCHO, -(CH₂)₃NHCONH₂, -(CH₂)₄NHCONH₂, -CH₂CH₂CH(OH)CH₂NH₂, 2-pyridylmethyl-, 3-pyridylmethyl-, 4-pyridylmethyl-, 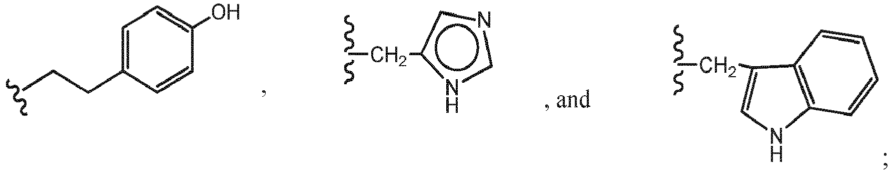
each subscript c is an independently selected integer from 1 to 10; and
the wavy lines indicate attachment of the Connector Unit to the rest of the Ligand-Drug Conjugate compound or pharmaceutically acceptable salt thereof.

In some embodiments, the Connector Unit A is 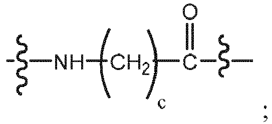
c is an integer ranging from 1 to 6; and
the wavy lines indicate the site of attachment to the rest of the Ligand-Drug Conjugate compound or pharmaceutically acceptable salt thereof.

In some embodiments, A is a bond.

In some embodiments, B is 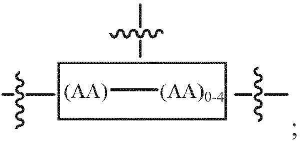
each AA is independently a proteinogenic or non-proteinogenic amino acid; and
the wavy lines indicate points of attachment to the rest of the Ligand-Drug Conjugate compound or pharmaceutically acceptable salt thereof.

In some embodiments, B is an amino acid. In some embodiments, B is 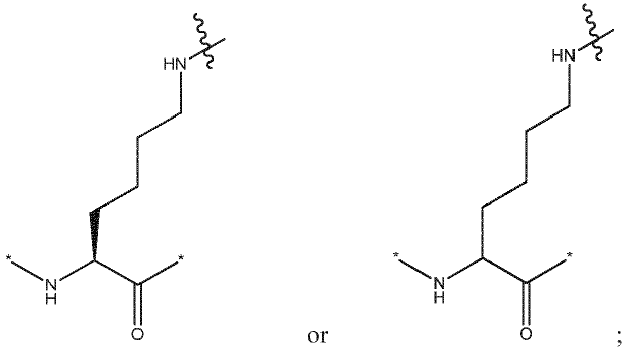
the wavy line indicates the point of attachement to the Partitioning Agent S^{∗}; and
the asterisks indicate points of attachment to the rest of the Ligand-Drug Conjugate compound or pharmaceutically acceptable salt thereof.

In some embodiments, the Partitioning Agent S^{∗} is a polyethylene glycol (PEG) unit, cyclodextrin unit, polyamide, hydrophilic peptide, polysaccharide, or dendrimer. In some embodiments, the Partitioning Agent S^{∗} is a PEG Unit comprising from 4 to 72 (CH₂CH₂O) subunits. In some embodiments, the PEG Unit is 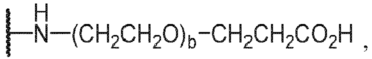 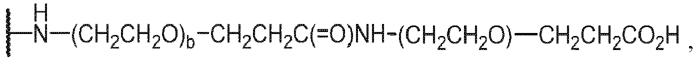 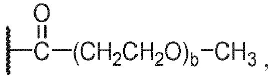 or 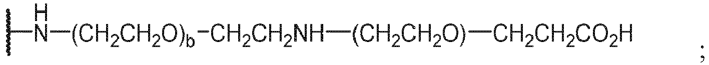
b is selected from the group consisting of 4 to 36; and
the wavy lines indicate the site of attachment to the rest of the Ligand-Drug Conjugate compound or pharmaceutically acceptable salt thereof.

In some embodiments, the Releasable Linker RL is
-(AA)₁₋₁₂-; and
each AA is independently a proteinogenic or non-proteinogenic amino acid.

In some embodiments, the Releasable Linker RL is -AA₁-AA₂- or -AA₁-AA₂-AA₃-, wherein AA₁ is attached to the Stretcher Unit Z or the Connector Unit A.

In some embodiments, the Releasable Linker RL is 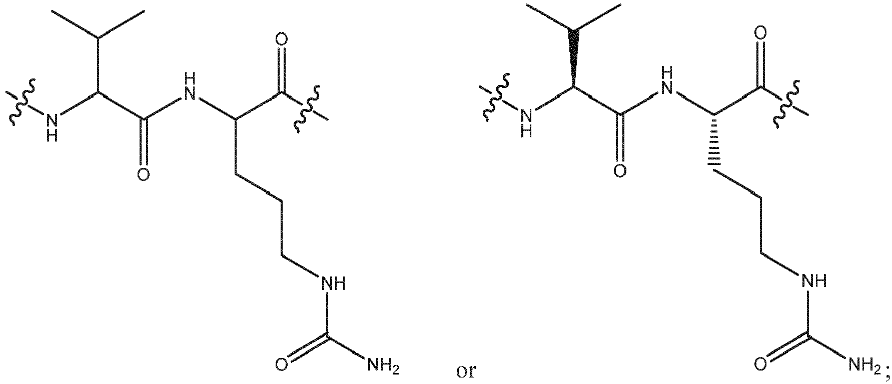 and
the wavy line adjacent to the -NH- group indicates attachment to the Stretcher Unit Z or the Connector Unit A and the wavy line adjacent to the -C(=O)- group indicates attachment to the Spacer Unit Y or the Drug Unit D.

In some embodiments, the Releasable Linker RL is a glycoside.

In some embodiments, the Releasable Linker RL is 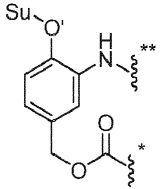 wherein
Su is a hexose form a monosaccharide;
O' represents the oxygen atom of a glycosidic bond that is capable of cleavage by a glycosidase;
the wavy line marked with a single asterisk (^{∗}) indicates the site of covalent attachment to D; and
the wavy line marked with a double asterisk (^{∗∗}) indicates the site of covalent attachment to the remainder of Q.

In some embodiments, the Releasable Linker RL is 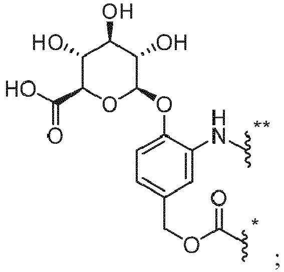
the wavy line marked with a single asterisk (^{∗}) indicates the site of covalent attachment to D; and
the wavy line marked with a double asterisk (^{∗∗}) indicates the site of covalent attachment to the remainder of Q.

In some embodiments, the Spacer Unit Y is 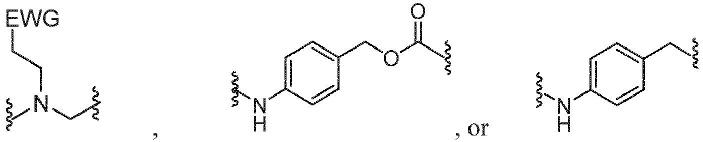
wherein EWG is an electron-withdrawing group; and
the wavy lines indicate the site of attachment to the rest of the Ligand-Drug Conjugate compound or pharmaceutically acceptable salt thereof.

In some embodiments, the Spacer Unit Y is 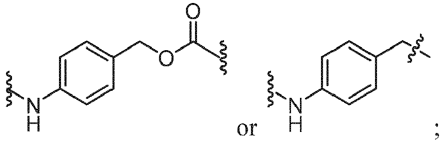 and
the wavy lines indicate the site of attachment to the rest of the Ligand-Drug Conjugate compound or pharmaceutically acceptable salt thereof.

In some embodiments,
R¹⁷ is C₁-C₁₀ alkylene;
A is a bond;
RL is -AA₁-AA₂-;
AA₁ and AA₂ are each independently a proteinogenic amino acid;
Y is 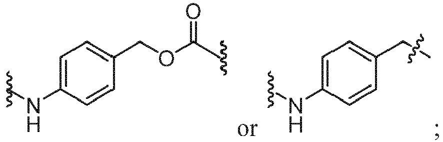 and
the wavy lines indicate the site of attachment to the rest of the Ligand-Drug Conjugate compound or pharmaceutically acceptable salt thereof.

In some embodiments,
A is a bond;
RL is 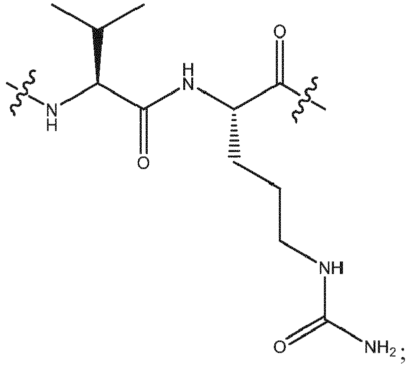 and
Y is

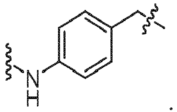

In some embodiments, Y-D is 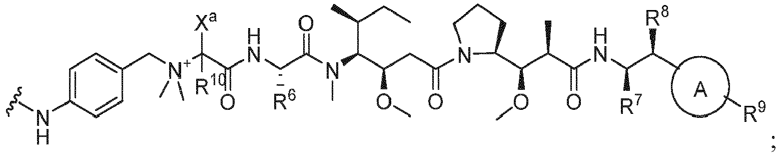 and
the wavy line indicates the site of attachment to the rest of the Ligand-Drug Conjugate compound or pharmaceutically acceptable salt thereof.

In some embodiments, Y-D is 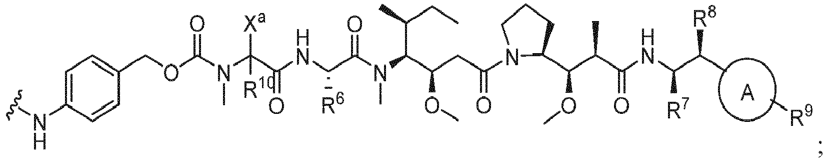 and
the wavy line indicates the site of attachment to the rest of the Ligand-Drug Conjugate compound or pharmaceutically acceptable salt thereof.

In some embodiments, the compound is 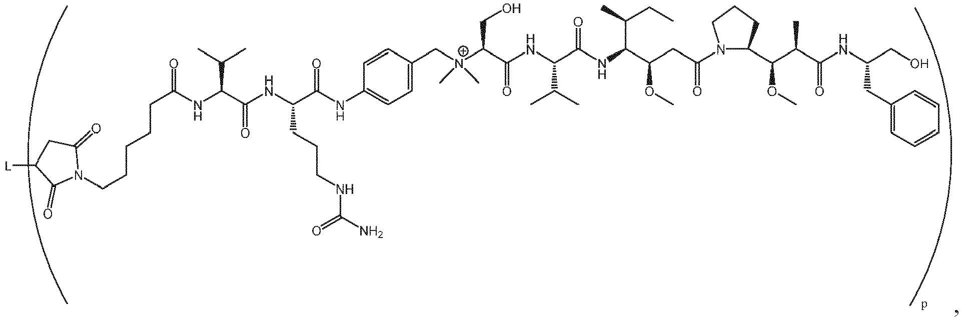 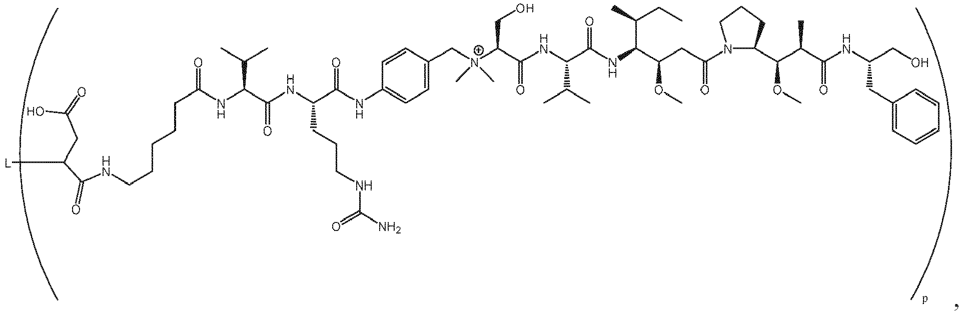 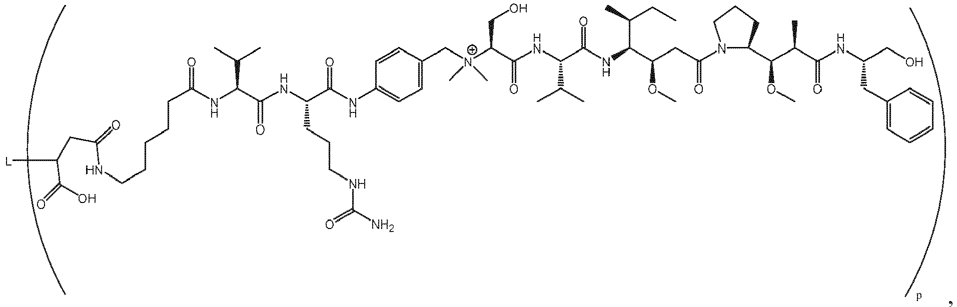 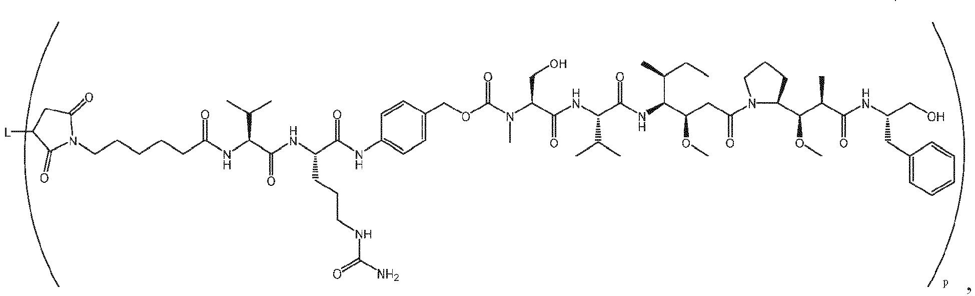 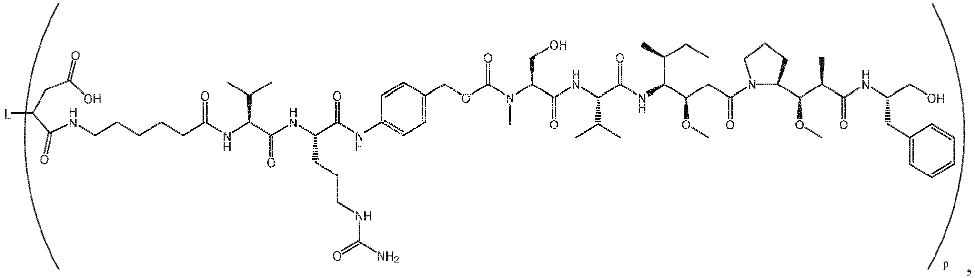 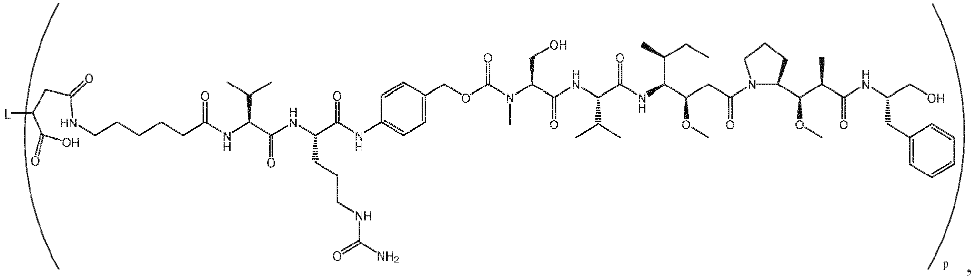 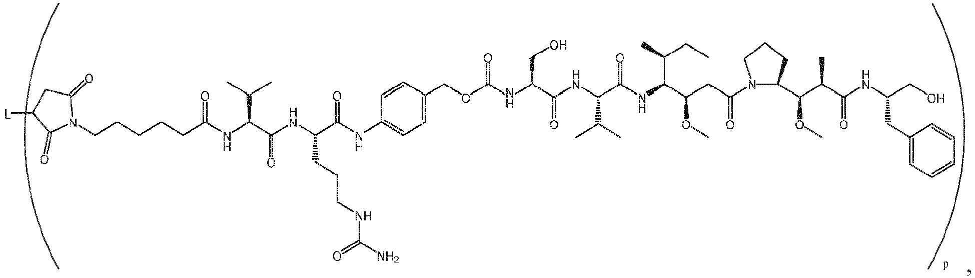 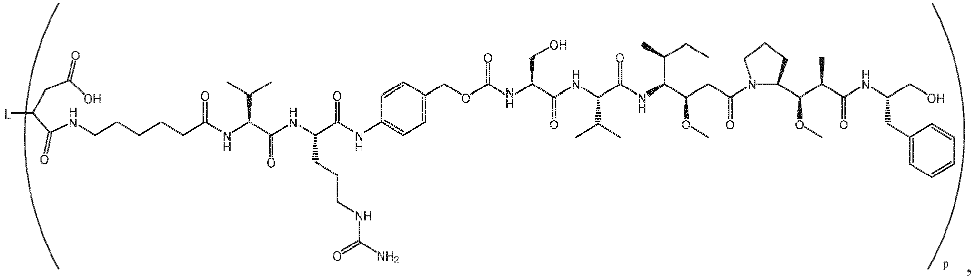 or 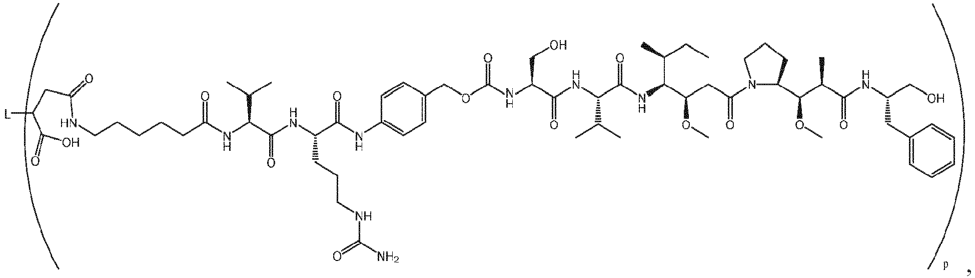 or a pharmaceutically acceptable salt thereof.

In some embodiments, the Ligand-Drug Conjugate compound is a compound of Table 3.

In some embodiments, p is an integer ranging from 2 to 6. In some embodiments, p is 4.

Also provided herein is a pharmaceutical composition comprising a Ligand-Drug Conjguate compound as described herein and a pharmaceutically acceptable excipient. In some embodiments, the composition comprises a plurlaity of Ligand-Drug Conjugate compounds with an average drug loading from 2 to 8. In some embodiments, the average drug loading is about 4. In some embodiments, the average drug loading is from 3.5 to 4.5.

Also provided herein is a method of treating cancer comprising administering a therapeutically effective amount of the Ligand-Drug Conjugate compound of any one of clauses 66-96, or a pharmaceutically acceptable salt thereof, to a subject in need thereof. In some embodiments, the subject tolerates treatment with the Ligand-Drug Conjugate compound better than treatment with another Ligand-Drug Conjugate compound in therapeutically effective doses. In some embodiments, the another Ligand-Drug Conjugate compound comprises a monomethyl auristatin E or monomethyl auristatin F Drug Unit.

### BRIEF DISCRIPTION OF THE FIGURES

**FIG 1****.** Illustrates the *in vivo* mean tumor volume data for various Ag1 ADCs in the A2058 melanoma xenograft model.
**FIG 2****.** Illustrates the *in vivo* mean tumor volume data for various h2A2 ADCs in the Detroit562 pharyngeal cancer xenograft model.
**FIG 3****.** Illustrates the *in vivo* median tumor volume data for various cAC10 ADCs in the Karpas/KarpasBVR admixed Hodgkin lymphoma xenograft model.
**FIG 4****.** Illustrates the *in vivo* mean tumor volume data for various cAC10 ADCs in the Karpas Hodgkin lymphoma xenograft model.
**FIG 5****.** Illustrates the *in vivo* mean tumor volume data for various cAC10 ADCs in the Karpas Hodgkin lymphoma xenograft model.
**FIG 6****.** Summarizes plasma neutrophil levels at day 5 and day 8 following administration by non-binding ADCs to rats.
**FIG 7****.** Summarizes reticulocyte levels at day 5 and day 8 following administration by non-binding ADCs to rats.
**FIG 8****.** Summarizes platelet levels at day 5 and day 8 following administration by non-binding ADCs to rats.
**FIG 9****.** Summarizes aspartate transaminase (AST) levels at day 8 following administration by non-binding ADCs to rats.

### DETAILED DESCRIPTION OF THE INVENTION

One drug class of interest for use in Ligand-Drug Conjguates is auristatins. Auristatins have been shown to be effective payloads in some Ligand-Drug Conjugates (LDCs), but it is believed that their hydrophobicity can contribute to off-target toxicity due to increased permeability and high bystander activity of the free auristatin. Additionally, hydrophobic payloads, such as auristatins, may increase the hydrophobicity of the LDC, resulting in rapid clearance of the LDC from the body of a subject. Therefore, there is a need for auristain conjugates engineered to have optimized cell permeability, pharmacokinetics, and toxicity profile.

Without being bound by theory, it is believed that the hydrophilic groups of the auristatin compounds provided herein influence the properties of the compounds and of resulting conjugates (*e.g*., Ligand-Drug Conjugates). The hydrophilic groups are believed to increase the effectiveness of the resulting conjugates in two ways. Conjugates comprising a hydrophilic auristatin group may improve tumor exposure to the Drug Unit due to decreased plasma clearance, and they may simultaneously demonstrate reduced off-target toxicity due to decreased cell permeability of the free drug after it is released from the conjugate.

### I. Definitions

Unless stated otherwise, the following terms and phrases as used herein are intended to have the following meanings. When trade names are used herein, the trade name includes the product formulation, the generic drug, and the active pharmaceutical ingredient(s) of the trade name product, unless otherwise indicated by context.

The term "antibody" as used herein is used in the broadest sense and specifically covers intact monoclonal antibodies, polyclonal antibodies, monospecific antibodies, multispecific antibodies (*e.g*., bispecific antibodies), and antibody fragments that retain, at least in part, one or more of the the desired biological activities of the full-length antibody, for example, affinity for its cognate antigen. The native form of an antibody is a tetramer and consists of two identical pairs of immunoglobulin chains, each pair having one light chain and one heavy chain. In each pair, the light and heavy chain variable regions (V_{L} and V_{H}) are together primarily responsible for binding to an antigen. The light chain and heavy chain variable domains consist of a framework region interrupted by three hypervariable regions, also called "complementarity determining regions" or "CDRs." In some aspects, the constant regions are recognized by and interact with the immune system (*see, e.g.,* Janeway et al., 2001, Immunol. Biology, 5th Ed., Garland Publishing, New York). The antibodies herein are of any type (*e.g.,* IgG, IgE, IgM, IgD, and IgA), class (*e.g.,* IgG₁, IgG₂, IgG₃, IgG₄, IgA₁ and IgA₂), or subclass thereof. In some aspects, the antibody is derived from a suitable species. In some embodiments, the antibody is of human or murine origin. In some aspects, the antibody is human, humanized, or chimeric.

The term "monoclonal antibody" as used herein refers to an antibody obtained from a population of substantially homogeneous antibodies, *i.e.,* the individual antibodies comprising the population are identical except for possible naturally occurring mutations that are present, in some aspects, in minor amounts. Monoclonal antibodies are highly specific, being directed against a single antigenic site. The modifier "monoclonal" indicates the character of the antibody as being obtained from a substantially homogeneous population of antibodies and is not to be construed as requiring production of the antibody by any particular method.

An "intact antibody" is one which comprises an antigen-binding variable region as well as a light chain constant domain (C_{L}) and heavy chain constant domains, C_{H}1, C_{H}2, C_{H}3 and C_{H}4, as appropriate for the antibody class. In some aspects the constant domains are native sequence constant domains (*e.g*., human native sequence constant domains) and in other aspects are amino acid sequence variants thereof.

An "antibody fragment" comprises a portion of an intact antibody, comprising the antigen-binding or variable region thereof. Examples of antibody fragments include Fab, Fab', F(ab')₂, and Fv fragments, diabodies, triabodies, tetrabodies, linear antibodies, single-chain antibody molecules, scFv, scFv-Fc, multispecific antibody fragments formed from antibody fragment(s), a fragment(s) produced by a Fab expression library, or an epitope-binding fragments of any of the above which immunospecifically bind to a target antigen *(e.g.,* a cancer cell antigen, a viral antigen or a microbial antigen).

An "antigen" is an entity to which an antibody specifically binds.

The terms "specific binding" and "specifically binds" mean that the antibody or antibody derivative will bind, in a highly selective manner, with its corresponding epitope of a target antigen and not with the multitude of other antigens. Typically, the antibody or antibody derivative binds with an affinity of at least about 1×10⁻⁷ M, and preferably 10⁻⁸ M to 10⁻⁹ M, 10⁻¹⁰ M, 10⁻¹¹ M, or 10⁻¹² M and binds to the predetermined antigen with an affinity that is at least two-fold greater than its affinity for binding to a non-specific antigen (*e.g.*, BSA, casein) other than the predetermined antigen or a closely-related antigen.

The term "inhibits" or "inhibition of" means to reduce by a measurable amount, or to prevent entirely.

The term "therapeutically effective amount" refers to an amount of a conjugate effective to treat a disease or disorder in a mammal. In the case of cancer, the therapeutically effective amount of the conjugate may reduce the number of cancer cells; reduce the tumor size; inhibit (*i.e.,* slow to some extent and preferably stop) cancer cell infiltration into peripheral organs; inhibit (*i.e.,* slow to some extent and preferably stop) tumor metastasis; inhibit, to some extent, tumor growth; and/or relieve to some extent one or more of the symptoms associated with the cancer. In some aspects, the drug inhibits growth and/or kills existing cancer cell.In some aspects, the drug is cytostatic and/or cytotoxic. For cancer therapy, efficacy is measured by commonly available tools. In some aspects, efficacy is measured by assessing the time to disease progression (TTP) and/or determining the response rate (RR).

The term "substantial" or "substantially" refers to a majority, *i.e.* >50% of a population, of a mixture or a sample, preferably more than 50%, 55%, 60%, 65%, 70%, 75%, 80%, 85%, 90%, 91%, 92%, 93%, 94%, 95%, 96%, 97 %, 98%, or 99% of a population.

The term "cytotoxic activity" refers to a cell-killing effect of a drug or Ligand-Drug Conjugate compound or an intracellular metabolite of a Ligand-Drug Conjugate compound. In some aspects, cytotoxic activity is expressed as the IC₅₀ value, which is the concentration (molar or mass) per unit volume at which half the cells survive.

The term "cytostatic activity" refers to an anti-proliferative effect of a drug or Ligand-Drug Conjugate compound or an intracellular metabolite of a Ligand-Drug Conjugate compound.

The term "cytotoxic agent" as used herein refers to a substance that has cytotoxic activity and causes destruction of cells. The term is intended to include chemotherapeutic agents, and toxins such as small molecule toxins or enzymatically active toxins of bacterial, fungal, plant or animal origin, including synthetic analogs and derivatives thereof.

The term "cytostatic agent" as used herein refers to a substance that inhibits a function of cells, including cell growth or multiplication. Cytostatic agents include inhibitors such as protein inhibitors, e.g., enzyme inhibitors. Cytostatic agents have cytostatic activity.

The terms "cancer" and "cancerous" refer to or describe the physiological condition or disorder in mammals that is typically characterized by unregulated cell growth. A "tumor" comprises one or more cancerous cells.

An "autoimmune disease" as used herein refers to a disease or disorder arising from and directed against an individual's own tissues or proteins.

"Patient" as used herein refers to a subject to whom is administered a Ligand-Drug Conjugate compound of the present invention. Patient includes, but are not limited to, a human, rat, mouse, guinea pig, non-human primate, pig, goat, cow, horse, dog, cat, bird and fowl. Typically, the patient is a rat, mouse, dog, human or non-human primate, more typically a human.

The terms "treat" or "treatment," unless otherwise indicated by context, refer to therapeutic treatment and prophylactic wherein the object is to inhibit or slow down (lessen) an undesired physiological change or disorder, such as the development or spread of cancer. For purposes of this invention, beneficial or desired clinical results include, but are not limited to, alleviation of symptoms, diminishment of extent of disease, stabilized (*i.e.,* not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder.

In the context of cancer, the term "treating" includes any or all of: killing tumor cells; inhibiting growth of tumor cells, cancer cells, or of a tumor; inhibiting replication of tumor cells or cancer cells, lessening of overall tumor burden or decreasing the number of cancerous cells, and ameliorating one or more symptoms associated with the disease.

In the context of an autoimmune disease, the term "treating" includes any or all of: inhibiting replication of cells associated with an autoimmune disease state including, but not limited to, cells that produce an autoimmune antibody, lessening the autoimmune-antibody burden and ameliorating one or more symptoms of an autoimmune disease.

"Compound" as the term is used herein, refers to and encompasses the chemical compound itself, either named or represented by structure, and salt form(s) thereof, whether explicitly stated or not, unless context makes clear that such salt forms are to be excluded. The term "compound" further encompasses solvate forms of the compound, in which solvent is noncovalently associated with the compound or is reversibly associated covalently with the compound, as when a carbonyl group of the compound is hydrated to form a gem-diol. Solvate forms include those of the compound itself and its salt form(s) and are inclusive of hemisolvates, monosolvates, disolvates, including hydrates; when a compound is associated with two or more solvent molecules, the two or more solvent molecules are the same or different.

In some instances, a compound of the invention will include an explicit reference to one or more of the above forms, e.g., salts and solvates, which does not imply any solid state form of the compound; however, this reference is for emphasis only, and is not to be construed as excluding any other of the forms as identified above. Furthermore, when explicit reference to a salt and/or solvate form of a compound or a Ligand Drug Conjugate composition is not made, that omission is not to be construed as excluding the salt and/or solvate form(s) of the compound or Conjugate unless context make clear that such salt and/or solvate forms are to be excluded.

The phrase "salt thereof" as the phrase is used herein, refers to a salt form of a compound (e.g., a Drug, a Drug-Linker compound or a Ligand-Drug Conjugate compound). A salt form of a compound is of one or more internal salt forms and/or involves the inclusion of another molecule such as an acetate ion, a succinate ion or other counterion. The counterion in a salt form of a compound is typically an organic or inorganic moiety that stabilizes the charge on the parent compound. A salt form of a compound has one or more than one charged atom in its structure. In instances where multiple charged atoms are part of the salt form, multiple counter ions and/or multiple charged counter ions are present. Hence, a salt form of a compound typically has one or more charged atoms corresponding to those of the non-salt form of the compound and one or more counterions. In some aspects, the non-salt form of a compound contains at least one amino group or other basic moeity, and accordingly in the presence of an acid, an acid addition salt with the basic moiety is obtained. In other aspects, the non-salt form of a compound contains at least one carboxylic acid group or other acidic moiety, and accordingly in the presence of a base, a carboxylate or other anionic moiety is obtained. Exemplary salts include, but are not limited to, sulfate, trifluoroacetate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, *p*-toluenesulfonate, and pamoate (*i.e.,* 1,1'-methylene-bis-(2-hydroxy-3-naphthoate)) salts.

A pharmaceutically acceptable salt is a salt form of a compound that is suitable for administration to a subject as described herein and in some aspects includes countercations or counteranions as described by P. H. Stahl and C. G. Wermuth, editors, Handbook of Pharmaceutical Salts: Properties, Selection and Use, Weinheim/Zürich:Wiley-VCH/VHCA, 2002.

A "Linker Unit" as used herein is a bifunctional moiety that connects or is capable of connecting a Drug Unit to a Ligand Unit in a Ligand-Drug Conjugate compound. The Linker Units of the present invention comprise two or more components selected from the group consisting of a Stretcher Unit which in some embodiments will have a Basic Unit; a Connector Unit; a Parallel Connector Unit; a Releasable Linker; and a Spacer Unit.

"PEG", "PEG Unit" or "polyethylene glycol" as used herein is an organic moiety comprising repeating ethylene-oxy subunits and is polydisperse, monodisperse, or discrete (i.e., having discrete number of ethylene-oxy subunits). Polydisperse PEGs are a heterogeneous mixture of sizes and molecular weights whereas monodisperse PEGs are typically purified from heterogeneous mixtures and are therefore provide a single chain length and molecular weight. Preferred PEG Units are discrete PEGs, compounds that are synthesized in stepwise fashion and not via a polymerization process. Discrete PEGs provide a single molecule with defined and specified chain length.

The PEG Unit provided herein comprises one or multiple polyethylene glycol chains, each comprising one or more ethyleneoxy subunits, covalently attached to each other. The polyethylene glycol chains are linked together in any pattern (*e.g.,* in a linear, branched, or star shaped configuration). Typically, at least one of the polyethylene glycol chains prior to incorporation into a Ligand-Drug Conjugate compound is derivitized at one end with an alkyl moiety substituted with an electrophilic group for covalent attachment to the carbamate nitrogen of a methylene carbamate unit (i.e., represents an instance of R). Typically, the terminal ethyleneoxy subunit in each polyethylene glycol chains not involved in covalent attachment to the remainder of the Linker Unit is modified with a PEG Capping Unit, typically H or an optionally substituted alkyl such as -CH₃, -CH₂CH₃ or -CH₂CH₂CO₂H. A preferred PEG Unit has a single polyethylene glycol chain with 4 to 24 -CH₂CH₂O- subunits covalently attached in series and terminated at one end with a PEG Capping Unit.

"Halogen" as the term is used herein by itself or in combination with another term, unless otherwise stated or implied by context, refers to fluorine, chlorine, bromine or iodine and is typically -F or -Cl.

Unless otherwise indicated, the term "alkyl" by itself or as part of another term refers to a straight chain or branched, saturated hydrocarbon having the indicated number of carbon atoms (*e.g.,* "-C₁-C₄ alkyl," "-C₁-C₈ alkyl," or "-C₁-C₁₀ alkyl refer to an alkyl group having from 1 to 4, 1 to 8, or 1 to 10 carbon atoms, respectively). When the number of carbon atoms is not indicated, the alkyl group has from 1 to 8 carbon atoms. Representative straight chain "-C₁-C₈ alkyl" groups include, but are not limited to, -methyl, -ethyl, -n-propyl, -n-butyl, -n-pentyl, -n-hexyl, -n-heptyl and -n-octyl; while branched -C₃-C₈ alkyls include, but are not limited to, -isopropyl, -*sec*-butyl, -isobutyl, -*tert*-butyl, -isopentyl, and -2-methylbutyl.

Unless otherwise indicated, "alkylene," by itself of as part of another term, refers to a saturated, branched or straight chain or cyclic hydrocarbon radical of the stated number of carbon atoms, typically 1-4, 1-8, or 1-10 carbon atoms, and having two monovalent radical centers derived by the removal of two hydrogen atoms from the same or two different carbon atoms of a parent alkane. Typical alkylene radicals include, but are not limited to: methylene (-CH₂-), 1,2-ethylene (-CH₂CH₂-), 1,3-propylene (-CH₂CH₂CH₂-), 1,4-butylene (-CH₂CH₂CH₂CH₂-), and the like. In preferred aspects, an alkylene is a branched or straight chain hydrocarbon (i.e., it is not a cyclic hydrocarbon).

"Alkenyl" as the term is used herein, by itself or as part of another term, unless otherwise stated or implied by context, refers to an organic moiety, substituent or group that comprises one or more double bond functional groups (e.g., a -CH=CH- moiety) or 1, 2, 3, 4, 5 or 6 or more, typically 1, 2 or 3 of such functional groups, more typically one such functional group, and in some aspects may contain non-aromatic linked normal, secondary, tertiary or cyclic carbon atoms, i.e., linear, branched, cyclic or any combination thereof as part of the base moiety unless the alkenyl substituent, moiety or group is a vinyl moiety (e.g., a -CH=CH₂ moiety). An alkenyl moiety, group or substituent having multiple double bonds may have the double bonds arranged contiguously (i.e., a 1,3-butadienyl moiety) or non-contiguously with one or more intervening saturated carbon atoms or a combination thereof, provided that a cyclic, contiguous arrangement of double bonds do not form a cyclic conjugated system of 4n + 2 electrons (i.e., is not aromatic).

An alkenyl moiety, group or substituent contains at least one sp² carbon atom in which that carbon atom is divalent and is doubly bonded to another organic moiety or Markush structure to which it is associated, or contains at least two sp² carbon atoms in conjugation to each other in which one of the sp² carbon atoms is monovalent and is singly bonded to another organic moiety or Markush structure to which it is associated. Typically, when alkenyl is used as a Markush group (i.e., is a substituent) the alkenyl is singly bonded to a Markush formula or another organic moiety with which it is associated through a sp² carbon of an alkene functional group of the alkenyl moiety. In some aspects, when an alkenyl moiety is specified, species encompasses those corresponding to any of the optionally substituted alkyl or carbocyclyl, groups moieties or substituents described herein that has one or more *endo* double bonds in which a sp² carbon atom thereof is monovalent and monovalent moieties derived from removal of a hydrogen atom from a sp² carbon of a parent alkene compound. Such monovalent moieties are exemplified without limitation by vinyl (-CH=CH₂), allyl, 1-methylvinyl, butenyl, iso-butenyl, 3-methyl-2-butenyl, 1-pentenyl, cyclopentenyl, 1-methyl-cyclopentenyl, 1-hexenyl, 3-hexenyl, and cyclohexenyl. In some aspects, the term alkenyl encompasses those and/or other linear, cyclic and branched chained, all carbon-containing moieties containing at least one double bond functional group in which one of the sp² carbon atoms is monovalent.

The number of carbon atoms in an alkenyl moiety is defined by the number of sp² carbon atoms of the alkene functional group(s) that defines it as an alkenyl substituent and the total number of contiguous non-aromatic carbon atoms appended to each of these sp² carbons not including any carbon atom of the other moiety or Markush structure for which the alkenyl moiety is a variable group and carbon atoms from any optional substituent to the alkenyl moiety. That number ranges from 1 to 50 or 1 to 30, typically 1 to 20 or 1 to 12, more typically, 1 to 8, 1 to 6 or 1 to 4 carbon atoms when the double bond functional group is doubly bonded to a Markush structure (e.g. =CH₂), or ranges from 2 to 50, typically 2 to 30, 2 to 20 or 2 to 12, more typically 2 to 8, 2 to 6 or 2 to 4 carbon atoms, when the double bond functional group is singly bonded to the Markush structure (e.g., -CH=CH₂). For example, C₂-C₈ alkenyl or C2-C8 alkenyl means an alkenyl moiety containing 2, 3, 4, 5, 6, 7 or 8 carbon atoms in which at least two are sp² carbon atoms in conjugation with each other with one of these carbon atoms being monovalent, and C₂-C₆ alkenyl or C2-C6 alkenyl means an alkenyl moiety containing 2, 3, 4, 5 or 6 carbon atoms in which at least two are sp² carbons that are in conjugation with each other with one of these carbon atoms being monovalent. In some aspects, an alkenyl substituent or group is a C₂-C₆ or C₂-C₄ alkenyl moiety having only two sp² carbons that are in conjugation with each other with one of these carbon atoms being monovalent. Typically, an alkenyl substituent is a C₂-C₆ or C₂-C₄ alkenyl moiety having only two sp² carbons that are in conjugation with each other. When the number of carbon atoms is not indicated, an alkenyl moiety has from 2 to 8 carbon atoms.

"Alkenylene" as the term is used herein, by itself of as part of another term, unless otherwise stated or implied by context, refers to an organic moiety, substituent or group that comprises one or more double bond moieties, as previously described for alkenyl, of the stated number of carbon atoms and has two radical centers derived by the removal of two hydrogen atoms from the same or two different sp² carbon atoms of an alkene functional group or removal of two hydrogen atoms from two separate alkene functional groups in a parent alkene. In some aspects, an alkenylene moiety is that of an alkenyl radical as described herein in which a hydrogen atom has been removed from the same or different sp² carbon atom of a double bond functional group of the alkenyl radical, or from a sp² carbon from a different double bonded moiety to provide a diradical. Typically, alkenylene moieties encompass diradicals containing the structure of -C=C- or -C=C-X¹-C=C- wherein X¹ is absent or is an alkylene as defined herein, which is typically a C₁-C₆ alkylene. The number of carbon atoms in an alkenylene moiety is defined by the number of sp² carbon atoms of its alkene functional group(s) that defines it as an alkenylene moiety and the total number of contiguous non-aromatic carbon atoms appended to each of its sp² carbons not including any carbon atoms of the other moiety or Markush structure in which the alkenyl moiety is a present as a variable group. That number, unless otherwise specified, ranges from 2 to 50 or 2 to 30, typically from 2 to 20 or 2 to 12, more typically from 2 to 8, 2 to 6 or 2 to 4 carbon atoms. For example, C₂-C₈ alkenylene or C2-C8 alkenylene means an alkenylene moiety containing 2, 3, 4, 5, 6, 7 or 8 carbon atoms, in which at least two are sp² carbons in which one is divalent or both are monovalent, that are in conjugation with each other and C₂-C₆ alkenylene or C2-C6 alkenylene means an alkenyl moiety containing 2, 3, 4, 5 or 6 carbon atoms in which at least two are sp² carbons, in which at least two are sp² carbons in which one is divalent or both are monovalent, that are in conjugation with each other. In some aspects, an alkenylene moiety is a C₂-C₆ or C₂-C₄ alkenylene having two sp² carbons that are in conjugation with each other in which both sp² carbon atoms are monovalent. When the number of carbon atoms is not indicated, an alkenylene moiety has from 2 to 8 carbon atoms.

"Alkynyl" as the term is used herein, by itself or as part of another term, unless otherwise stated or implied by context, refers to an organic moiety, substituent or group that comprises one or more triple bond functional groups (e.g., a -C=C- moiety) or 1, 2, 3, 4, 5, or 6 or more, typically 1, 2, or 3 of such functional groups, more typically one such functional group. An alkynyl moiety, group or substituent having multiple triple bonds may have the triple bonds arranged contiguously or non-contiguously with one or more intervening saturated or unsaturated carbon atoms or a combination thereof, provided that a cyclic, contiguous arrangement of triple bonds do not form a cyclic conjugated system of 4n + 2 electrons (i.e., is not aromatic).

An alkynyl moiety, group or substituent contains at least two sp carbon atom in which the carbon atoms are conjugation to each other and in which one of the sp carbon atoms is singly bonded, to another organic moiety or Markush structure to which it is associated. When alkynyl is used as a Markush group (i.e., is a substituent) the alkynyl is singly bonded to a Markush formula or another organic moiety with which it is associated through a triple-bonded carbon (i.e., a sp carbon) of a terminal alkyne functional group. In some aspects when an alkynyl moiety, group or substituent is specified, species encompasses are any of the alkyl or carbocyclyl, groups moieties or substituents described herein that has one or more *endo* triple bonds and monovalent moieties derived from removal of a hydrogen atom from a sp carbon of a parent alkyne compound. Such monovalent moieties are exemplified without limitation by -C≡CH, and -C≡C-CH₃, and -C≡C-Ph.

The number of carbon atoms in an alkynyl substituent is defined by the number of sp carbon atoms of the alkene functional group that defines it as an alkynyl substituent and the total number of contiguous non-aromatic carbon atoms appended to each of these sp carbons not including any carbon atom of the other moiety or Markush structure for which the alkenyl moiety is a variable group. That number can vary ranging from 2 to 50, typically 2 to 30, 2 to 20, or 2 to 12, more typically 2 to 8, 2 to 6, or 2 to 4 carbon atoms, when the triple bond functional group is singly bonded to the Markush structure (e.g., -CH=CH). For example, C₂-C₈ alkynyl or C2-C8 alkynyl means an alkynyl moiety containing 2, 3, 4, 5, 6, 7, or 8 carbon atoms in which at least two are sp carbon atoms in conjugation with each other with one of these carbon atoms being monovalent, and C₂-C₆ alkynyl or C2-C6 alkynyl means an alkynyl moiety containing 2, 3, 4, 5, or 6 carbon atoms in which at least two are sp carbons that are in conjugation with each other with one of these carbon atoms being monovalent. In some aspects, an alkynyl substituent or group is a C₂-C₆ or C₂-C₄ alkynyl moiety having two sp carbons that are in conjugation with each other with one of these carbon atoms being monovalent. When the number of carbon atoms is not indicated, an alkynyl moiety, group or substituent has from 2 to 8 carbon atoms.

The term "Prodrug" as used herein refers to a less biologically active or inactive compound which is transformed within the body into a more biologically active compound via a chemical or biological process (i.e., a chemical reaction or an enzymatic biotransformation). Typically, a biologically active compound is rendered less biologically active (i.e., is converted to a prodrug) by chemically modifying the compound with a prodrug moiety. In some aspects, the prodrug is a Type II prodrug, which are bioactivated outside cells, e.g., in digestive fluids, or in the body's circulation system, e.g., in blood. Exemplary prodrugs are esters and β-D-glucopyranosides.

Unless otherwise indicated, "aryl," by itself or as part of another term, means amonovalent carbocyclic aromatic hydrocarbon radical of the stated number of carbon atoms, typically 6-20 carbon atoms, derived by the removal of one hydrogen atom from a single carbon atom of a parent aromatic ring system. Some aryl groups are represented in the exemplary structures as "Ar". Typical aryl groups include, but are not limited to, radicals derived from benzene, naphthalene, anthracene, biphenyl, and the like. An exemplary aryl group is a phenyl group.

Unless otherwise indicated, an "arylene," by itself or as part of another term, is an aryl group as defined above which has two covalent bonds (i.e., it is divalent) and is in the ortho, meta, or para orientations as shown in the following structures, with phenyl as the exemplary group:

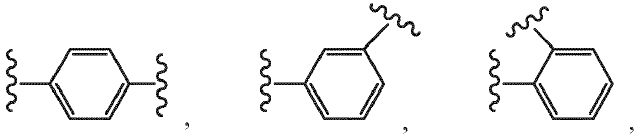

Unless otherwise indicated, a "C₃-C₈ heterocycle," by itself or as part of another term, refers to a monovalentaromatic or non-aromatic monocyclic or bicyclic ring system having from 3 to 8 carbon atoms (also referred to as ring members) and one to four heteroatom ring members independently selected from N, O, P or S, and derived by removal of one hydrogen atom from a ring atom of a parent ring system. In some aspects, one or more N, C or S atoms in the heterocycle is/are oxidized. In some aspects, the ring that includes the heteroatom is aromatic or nonaromatic. Heterocycles in which all the ring atoms are involved in aromaticity are referred to as heteroaryls and otherwise are referred to heterocarbocycles.

Unless otherwise noted, the heterocycle is attached to its pendant group at any heteroatom or carbon atom that results in a stable structure. In some aspects, a heteroaryl is bonded through an aromatic carbon of its aromatic ring system, referred to as a C-linked heteroaryl. In other aspects, a heteroaryl is bonded through a non-double-bonded N atom (i.e., not =N-) in its aromatic ring system, which is referred to as an N-linked heteroaryl. Thus, nitrogen-containing heterocycles are C-linked or N-linked and include pyrrole moieties, such as pyrrol-1-yl (N-linked) and pyrrol-3-yl (C-linked), and imidazole moieties such as imidazol-1-yl and imidazol-3-yl (both N-linked), and imidazol-2-yl, imidazol-4-yl and imidazol-5-yl moieties (all of which are C-linked).

Unless otherwise indicated, a"C₃-C₈ heteroaryl," is an aromatic C₃-C₈ heterocycle in which the subscript denotes the total number of carbons of the cyclic ring system of the heterocycle or the total number of aromatic carbons of the aromatic ring system of the heteroaryl and does not implicate the size of the ring system or the presence or absence of ring fusion. Representative examples of a C₃-C₈ heterocycle include, but are not limited to, pyrrolidinyl, azetidinyl, piperidinyl, morpholinyl, tetrahydrofuranyl, tetrahydropyranyl, benzofuranyl, benzothiophene, indolyl, benzopyrazolyl, pyrrolyl, thiophenyl (thiophene), furanyl, thiazolyl, imidazolyl, pyrazolyl, pyrimidinyl, pyridinyl, pyrazinyl, pyridazinyl, isothiazolyl, and isoxazolyl.

When explicitly given, the size of the ring system of a heterocycle or heteroaryl is indicated by the total number of atoms in the ring. For example, designation as a 5- or 6-membered heteroaryl indicates the total number of aromatic atoms (i.e., 5 or 6) in the heteroaromatic ring system of the heteroaryl but does not imply the number of aromatic heteroatoms or the number of aromatic carbon atoms in that ring system. Fused heteroaryls are explicitly stated or implied by context as such and are typically indicated by the number of aromatic atoms in each aromatic ring that are fused together to make up the fused heteroaromatic ring system. For example, a 5,6-membered heteroaryl is an aromatic 5-membered ring fused to an aromatic 6-membered ring in which one or both rings have aromatic heteroatom(s) or where a heteroatom is shared between the two rings.

A heterocycle fused to an aryl or heteroaryl such that the heterocycle remains non-aromatic and is part of a larger structure through attachment with the non-aromatic portion of the fused ring system is an example of a heterocycle in which the heterocycle is substituted by ring fusion with the aryl or heteroaryl. Likewise, an aryl or heteroaryl fused to heterocycle or carbocycle that is part of a larger structure through attachment with the aromatic portion of the fused ring system is an example of an aryl or heterocycle in which the aryl or heterocycle is substituted by ring fusion with the heterocycle or carbocycle.

Unless otherwise indicated, "C₃-C₈ heterocyclo," by itself or as part of another term, refers to a C₃-C₈ heterocyclic defined above wherein one of the hydrogen atoms of the heterocycle is replaced with a bond (i.e., it is divalent). Unless otherwise indicated, a "C₃-C₈ heteroarylene," by itself or as part of another term, refers to a C₃-C₈ heteroaryl group defined above wherein one of the heteroaryl group's hydrogen atoms is replaced with a bond (i.e., it is divalent). When explicitly given, the size of the ring system of a heteroarylene is indicated by the total number of atoms in the ring. For example, designation as a 5- or 6-membered heteroarylene indicates the total number of atoms (i.e., 5 or 6) in the heterocyclic ring system of the heterocycle, but does not imply the number of heteroatoms or the number of carbon atoms in that ring system.

Unless otherwise indicated, a "C₃-C₈ carbocycle," by itself or as part of another term, is a 3-, 4-, 5-, 6-, 7- or 8-membered monovalent,saturated or unsaturated non-aromatic monocyclic or bicyclic carbocyclic ring derived by the removal of one hydrogen atom from a ring atom of a parent ring system. Representative -C₃-C₈ carbocycles include, but are not limited to, cyclopropyl, cyclobutyl, cyclopentyl, cyclopentadienyl, cyclohexyl, cyclohexenyl, 1,3-cyclohexadienyl, 1,4-cyclohexadienyl, cycloheptyl, 1,3-cycloheptadienyl, 1,3,5-cycloheptatrienyl, cyclooctyl, and cyclooctadienyl.

Unless otherwise indicated, a "C₃-C₈ carbocyclo," by itself or as part of another term, refers to a C₃-C₈ carbocycle group defined above wherein another one of the carbocycle group's hydrogen atoms is replaced with a bond (i.e., it is divalent).

Unless otherwise indicated, the term "heteroalkyl," by itself or in combination with another term, means, unless otherwise stated, a stable straight or branched chain hydrocarbon, or combinations thereof, fully saturated or containing from 1 to 3 degrees of unsaturation, consisting of the stated number of carbon atoms and from one to ten, preferably one to three, heteroatoms selected from the group consisting of O, N, Si and S, and wherein the nitrogen and sulfur atoms are optionally oxidized and the nitrogen heteroatom is optionally quaternized. The heteroatom(s) O, N and S are placed at any interior position of the heteroalkyl group or at the position at which the alkyl group is attached to the remainder of the molecule. The heteroatom Si is placed at any position of the heteroalkyl group, including the position at which the alkyl group is attached to the remainder of the molecule. When explicitly given, the number of atoms in a heteroalkyl or heteroarylene is indicated by the total number of atoms in the group. For example, designation as a C₁-C₂ heteroalkyl indicates the total number of atoms (i.e., 1 or 2) in the heteroalkyl group, but does not imply the number of heteroatoms or the number of carbon atoms in that group.

Examples include -CH₂-CH₂-O-CH₃, -CH₂-CH₂-NH-CH₃, -CH₂-CH₂-N(CH₃)-CH₃, -CH₂-S-CH₂-CH₃, -CH₂-CH₂-S(O)-CH₃, -NH-CH₂-CH₂-NH-C(O)-CH₂-CH₃, -CH₂-CH₂-S(O)₂-CH₃, -CH=CH-O-CH₃, -Si(CH₃)₃, -CH₂-CH=N-O-CH₃, and -CH=CH-N(CH₃)-CH₃. In some aspects, two heteroatoms are consecutive, such as, for example, -CH₂-NH-OCH₃ and -CH₂-O-Si(CH₃)₃. Typically, a C₁ to C₄ heteroalkyl or heteroalkylene has 1 to 4 carbon atoms and 1 or 2 heteroatoms and a C₁ to C₃ heteroalkyl or heteroalkylene has 1 to 3 carbon atoms and 1 or 2 heteroatoms. In some aspects, a heteroalkyl or heteroalkylene is saturated.

Unless otherwise indicated, the term "heteroalkylene" by itself or in combination with another term means a divalent group derived from heteroalkyl (as discussed above), as exemplified by -CH₂-CH₂-S-CH₂-CH₂- and -CH₂-S-CH₂-CH₂-NH-CH₂-. For heteroalkylene groups, heteroatoms can also occupy either or both of the chain termini. Still further, for alkylene and heteroalkylene linking groups, no orientation of the linking group is implied.

Unless otherwise indicated, "aminoalkyl" by itself or in combination with another term means a heteroalkyl wherein an alkyl moiety as defined herein is substituted with an amino, alkylamino, dialkylamino or cycloalkylamino group. Exemplary non-limiting aminoalkyls are -CH₂NH₂, -CH₂CH₂NH₂, -CH₂CH₂NHCH₃ and -CH₂CH₂N(CH₃)₂ and further includes branched species such as -CH(CH₃)NH₂ and -C(CH₃)CH₂NH₂ in the (R)- or (S)- configuration. Alternatively, an aminoalkyl is an alkyl moiety, group, or substituent as defined herein wherein a sp³ carbon other than the radical carbon atom of the alkyl moiety has been replaced with an amino or alkylamino moiety wherein its sp³ nitrogen atom replaces the sp³ carbon of the alkyl moiety provided that at least one sp³ carbon atom of the alkyl moiety remains. When referring to an aminoalkyl moiety as a substituent to a larger structure or another moiety the aminoalkyl is covalently attached to the structure or moiety through the carbon radical of the alkyl moiety of the aminoalkyl.

"Hydroxyalkyl" as the term is used herein by itself or in combination with another term, unless otherwise stated or implied by context, refers to an alkyl moiety, group, or substituent having a hydroxyl radical in place of one or more hydrogen atoms of the alkyl moiety, group, or substituent. In some aspects, one or two hydrogen atoms are each replaced with a hydroxyl substituent in a hydroxyalkyl group. A hydroxyalkyl is typically denoted by the number of contiguous carbon atoms of its alkyl or alkylene moiety. Thus, a C₁ hydroxyalkyl is exemplified without limitation by -CH₂OH, and a C₂ hydroxyalkyl is exemplified without limitation by -CH₂CH₂OH or -CH₂(OH)CH₃.

"Haloalkyl" as the term is used herein by itself or in combination with another term, unless otherwise stated or implied by context, referes to an alkyl moiety, group, or substituent having a halogen in place of one or more hydrogen atoms of the alkyl moiety, group, or substituent. In some aspects, one or two hydrogen atoms are each replaced with a halogen in a haloalkyl group. A haloalkyl is typically denoted by the number of contiguous carbon atoms of its alkyl or alkylene moiety. Thus, a C₁ haloalkyl is exemplified without limitation by -CH₂F, -CH₂Cl, -CH₂Br, or -CH₂I, and a C₂ haloalkyl is exemplified without limitation by -CH₂CH₂F, -CH₂CH₂Cl, -CH₂CH₂Br, -CH₂CH₂I, -CH₂(F)CH₃, -CH₂(Cl)CH₃, -CH₂(Br)CH₃, or -CH₂(I)CH₃. In some embodiments, the term "haloalkyl" refers to an alkyl moiety, group, or substituent having halogens in place of two or more hydrogen atoms. For example, a C₁ haloalkyl is also exemplified without limitation by -CHF₂, -CHCl₂, -CHBr₂, or -CHI₂, and a C₂ haloalkyl is exemplified without limitation by -CH₂CHF₂, -CH₂CHCl₂, - CH₂CHBr₂, -CH₂CHI₂, -CH(F)₂CH₃, -CH(Cl)₂CH₃, -CH(Br)₂CH₃, or -CH(I)₂CH₃. In some aspects, the term "haloalkyl" refers to an alkyl moiety, group, or substituent having halogens in place of all hydrogen atoms. Thus, in some aspects, the term "haloalkyl" encompasses fully halogenated alkyl moieties, groups, or substituents. For example, a C₁ haloalkyl is also exemplified without limitation by -CF₃, -CCl₃, -CBr₃, or -CI₃.

Unless otherwise indicated "alkylamino" and "cycloalkylamino" by itself or in combination with another term means an alkyl or cycloalkyl radical, as described herein, wherein the radical carbon atom of the alkyl or cycloalkyl radical has been replaced with a nitrogen radical, provided that at least one sp³ carbon atom of the alkyl or cycloalkyl radical remains. In those instances where the alkylamino is substituted at its nitrogen with another alkyl moiety the resulting substituted radical is sometimes referred to as a dialkylamino moiety, group or substituent wherein the alkyl moieties substituting nitrogen are independently selected.

Exemplary and non-limiting amino, alkylamino and dialkylamino substituents, include those having the structure of -N(R')₂, wherein R' in these examples are independently hydrogen or C₁₋₆ alkyl, typically hydrogen or methyl, whereas in cycloalkyl amines, which are included in heterocycloalkyls, both R' together with the nitrogen to which they are attached define a heterocyclic ring. When both R' are hydrogen or alkyl, the moiety is sometimes described as a primary amino group and a tertiary amine group, respectively. When one R' is hydrogen and the other is alkyl, then the moiety is sometimes described as a secondary amino group. Primary and secondary alkylamino moieties are typically more reactive as nucleophiles towards carbonyl-containing electrophilic centers whereas tertiary amines are typically more basic.

The term "substituted" means that the specified group or moiety bears one or more substituents. Typical substituents include, but are not limited to a -X, -R", -OH, -OR", -SR", , -N(R")₂, -N(R")₃, =NR", -CX₃, -CN, -NO₂, -NR"C(=O)R", - C(=O)R", -C(=O)N(R")₂, -S(=O)₂R", -S(=O)₂NR", -S(=O)R", -OP(=O)(OR")₂, -P(=O)(OR")₂, -PO₃⁼, PO₃H₂, -C(=O)R", -C(=S)R", -CO₂R", -CO₂⁻, -C(=S)OR", -C(=O)SR", -C(=S)SR", - C(=O)N(R")₂, -C(=S)N(R")₂, and -C(=NR)N(R")₂, where each X is independently selected from the group consisting of a halogen: -F, -Cl, -Br, and -I; and wherein each R" is independently selected from the group consisting of -H, -C₁-C₂₀ alkyl, -C₆-C₂₀ aryl, -C₃-C₁₄ heterocycle, a protecting group, and a prodrug moiety.

More typically substituents are selected from the group consisting of -X, -R", -OH, -OR", -SR", -N(R")₂, -N(R")₃, =NR", -NR"C(=O)R", -C(=O)R", - C(=O)N(R")₂ -S(=O)₂R", -S(=O)₂NR", -S(=O)R", -C(=O)R", -C(=S)R", -C(=O)N(R")₂, - C(=S)N(R")₂, and -C(=NR)N(R")₂, wherein each X is independently selected from the group consisting of-F and -Cl, or are selected from the group consisting of -X, -R", -OH, -OR", - N(R")₂, -N(R")₃, -NR"C(=O)R", -C(=O)N(R")₂, -S(=O)₂R", -S(=O)₂NR", -S(=O)R", - C(=O)R", -C(=O)N(R")₂, -C(=NR)N(R")₂, a protecting group, and a prodrug moiety, wherein each X is -F; and wherein each R' is independently selected from the group consisting of hydrogen, -C₁-C₂₀ alkyl, -C₆-C₂₀ aryl, -C₃-C₁₄ heterocycle, a protecting group, and a prodrug moiety.

In some aspects, a substituent on an alkyl, alkenyl, or alkynyl is selected from the group consisting -N(R")₂, -N(R")₃ and -C(=NR)N(R")₂, wherein R" is selected from the group consisting of hydrogen and -C₁-C₂₀ alkyl. In other aspects, alkyl, alkenyl, or alkynyl is substituted with a series of ethyleneoxy moieties to define a PEG Unit as described herein. In some embodiments, alkylene, carbocycle, carbocyclo, arylene, heteroalkyl, heteroalkylene, heterocycle, heterocyclo, heteroaryl, and heteroarylene groups as described above are similarly substituted.

The term "unsubstituted" means that the specified group bears no substituents. Where the term "substituted is used to described a structural system, the substitution is meant to occur at any valency-allowed position on the system. When a group or moiety bears more than one substituent, it is understood that the substituents may be the same or different from one another. In some embodiments a substituted group or moiety bears from one to five substituents. In some embodiments a substituted group or moiety bears one substituent. In some embodiments a substituted group or moiety bears two substituents. In some embodiments a substituted group or moiety bears three substituents. In some embodiments a substituted group or moiety bears four substituents. In some embodiments a substituted group or moiety bears five substituents.

By "optional" or "optionally" is meant that the subsequently described event or circumstance may or may not occur, and that the description includes instances where the event or circumstance occurs and instances in which it does not. For example, "optionally substituted alkyl" encompasses both "alkyl" and "substituted alkyl" as defined herein. It will be understood by those skilled in the art, with respect to any group containing one or more substituents, that such groups are not intended to introduce any substitution or substitution patterns that are sterically impractical, synthetically non-feasible, and/or inherently unstable. It will also be understood that where a group or moiety is optionally substituted, the disclosure includes both embodiments in which the group or moiety is substituted and embodiments in which the group or moiety is unsubstituted.

"Protecting group" as used here means a moiety that prevents or reduces the ability of the atom or functional group to which it is linked from participating in unwanted reactions. Typical protecting groups for atoms or functional groups are given in Greene (1999), "PROTECTIVE GROUPS IN ORGANIC SYNTHESIS, 3RD ED.", Wiley Interscience, which is incorporated by reference herein. Protecting groups for heteroatoms such as oxygen, sulfur and nitrogen are used in some instances to minimize or avoid unwanted their reactions with electrophilic compounds. In other instances, the protecting group is used to reduce or eliminate the nucleophilicity and/or basicity of the unprotected heteroatom. Non-limiting examples of protected oxygen are given by -OR^{PR}, wherein R^{PR} is a protecting group for hydroxyl, wherein hydroxyl is typically protected as an ester (e.g. acetate, propionate or benzoate). Other protecting groups for hydroxyl avoid interfering with the nucleophilicity of organometallic reagents or other highly basic reagents, where hydroxyl is typically protected as an ether, including alkyl or heterocycloalkyl ethers, (e.g., methyl or tetrahydropyranyl ethers), alkoxymethyl ethers (e.g., methoxymethyl or ethoxymethyl ethers), optionally substituted aryl ethers, and silyl ethers (e.g., trimethylsilyl (TMS), triethylsilyl (TES), tert-butyldiphenylsilyl (TBDPS), tert-butyldimethylsilyl (TBS/TBDMS), triisopropylsilyl (TIPS) and [2-(trimethylsilyl)ethoxy]-methylsilyl (SEM)). Nitrogen protecting groups include those for primary or secondary amines as in -NHR^{PR} or -N(R^{PR})₂-, wherein least one of R^{PR} is a nitrogen atom protecting group or both R^{PR} together comprise a protecting group.

A protecting group is suitable when it is capable of preventing or avoiding unwanted side-reactions or premature loss of the protecting group under reaction conditions required to effect desired chemical transformation elsewhere in the molecule and during purification of the newly formed molecule when desired, and are removable under conditions that do not adversely affect the structure or stereochemical integrity of that newly formed molecule. By way of example and not limitation, a suitable protecting group includes those previously described for protecting otherwise reactive functional groups. A suitable protecting group is sometimes a protecting group used in peptide coupling reactions.

"Electron withdrawing group" as used herein means a functional group or electronegative atom that draws electron density away from an atom to which it is bonded either inductively and/or through resonance, whichever is more dominant (i.e., in some aspects, a functional group or atom is electron withdrawing inductively but is overall electron donating through resonance) and tends to stabilize anions or electron-rich moieties. The electron withdrawing effect is typically transmitted inductively, albeit in attenuated form, to other atoms attached to the bonded atom that has been made electron deficient by the electron withdrawing group (EWG), thus affecting the electrophilicity of a more remote reactive center. Exemplary electron withdrawing groups include, but are not limited to -C(=O), -CN, -NO₂, -CX₃, -X, -C(=O)OR', -C(=O)N(R')₂, -C(=O)R', -C(=O)X, -S(=O)₂R', -S(=O)₂OR', - S(=O)₂NHR', -S(=O)₂N(R')₂, -P(=O)(OR')₂, -P(=O)(CH₃)NHR', -NO, -N(R')₃⁺, wherein X is -F, -Br, -Cl, or -I, and R' in some aspects is, at each occurrence, independently selected from the group consisting of hydrogen and C₁₋₆ alkyl, and certain O-linked moieties as described herein such as acyloxy.

Exemplary EWGs can also include aryl groups (e.g., phenyl) depending on substitution of its aromatic ring and certain heteroaryl groups (e.g., pyridine). Thus, the term "electron withdrawing groups" also includes aryls or heteroaryls that are further substituted with electron withdrawing groups. Typically, electron withdrawing groups on aryls or heteroaryls are -C(=O), -CN, -NO₂, -CX₃, and -X, wherein X independently selected is halogen, typically -F or -Cl. Depending on their substituents, an alkyl moiety may also be an electron withdrawing group.

"Succinimide moiety" as used herein refers to an organic moiety comprising a succinimide ring system, which is present in one type of Stretcher Unit (Z) that typically further comprises an alkylene-containing moiety bonded to the imide nitrogen of that ring system. A succinimide moiety typically results from Michael addition of a sulfhydryl group of a Ligand Unit to the maleimide ring system of a Stretcher Unit precursor (Z') in a Drug Linker compound or maleimide-containing intermediate thereof. A succinimide moiety therefore comprises a thio-substituted succinimide ring system and when present in a Ligand-Drug Conjugate compound has its imide nitrogen substituted with the remainder of the Linker Unit of the Ligand-Drug Conjugate compound and is optionally substituted with substituent(s) that were present on the maleimide ring system of Z'.

"Succinic acid-amide moiety" as used herein refers to a succinic acid moiety wherein one of the two carboxylic acid groups is replaced with an amide substituent that results from the thio-substituted succinimide ring system of a succinimide moiety as defined herein having undergone breakage of one of its carbonyl-nitrogen bonds by hydrolysis. In some aspects, the succinic acid-amide moiety has the structure: 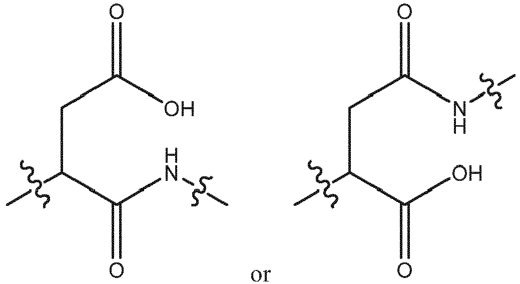 wherein the wavy line on the left indicates attachment to a Ligand Unit or hydrogen atom and the wavy line on the right indicates attachment to the remainder of a Ligand-Drug Conjugate compound, Drug-Linker compound, intermediate, or fragment thereof. Hydrolysis resulting in a succinic acid-amide moiety provides a Linker Unit less likely to suffer premature loss of the Ligand Unit to which it is bonded through elimination of the antibody-thio substituent. Hydrolysis of the succinimide ring system of the thio-substituted succinimide moiety is expected to provide regiochemical isomers of acid-amide moieties that are due to differences in reactivity of the two carbonyl carbons of the succinimide ring system attributable at least in part to any substituent present in the maleimide ring system of the Stretcher Unit precursor and to the thio substituent introduced by the targeting ligand, which is a precursor to the Ligand Unit.

In many instances, the assembly of the conjugates, linkers and components described herein will refer to reactive groups. A "reactive group" or RG is a group that contains a reactive site (RS) capable of forming a bond with either the components of the Linker Unit Q or the Drug Unit D. RS is the reactive site within a Reactive Group (RG). Reactive groups include sulfhydryl groups to form disulfide bonds or thioether bonds, aldehyde, ketone, or hydrazine groups to form hydrazone bonds, carboxylic or amino groups to form peptide bonds, carboxylic or hydroxy groups to form ester bonds, sulfonic acids to form sulfonamide bonds, alcohols to form carbamate bonds, and amines to form sulfonamide bonds or carbamate bonds.

The following table is illustrative of Reactive Groups, Reactive Sites, and exemplary functional groups capable of forming after reaction of the reactive site. The table is not limiting. One of skill in the art will appreciate that the noted R^{∗} and R^{∗∗} portions in the table are effectively any organic moiety (e.g., an alkyl group, aryl group, heteroaryl group, or substituted alkyl, aryl, or heteroaryl, group) that is compatible with the bond formation provided in converting RG to one of the Exemplary Functional Groups. It will also be appreciated that, as applied to the various aspects of the present invention, R^{∗} represents one or more components of the self-stabilizing linker or optional secondary linker, and R^{∗∗} represents one or more components of the optional secondary linker, Drug Unit, stabilizing unit, or detection unit.

| RG | RS | Exemplary Functional Groups |
|---|---|---|
| | | |
| 1) R^{∗}-SH | -S- | R^{∗}-S-R^{∗∗}, R^{∗}-S-S-R^{∗∗} |
| 2) R^{∗}-C(=O)OH | -C(=O)- | R^{∗}-C(=O)NH-R^{∗∗} |
| 3) R^{∗}-C(=O)ONHS | -C(=O)- | R^{∗}-C(=O)NH-R^{∗∗} |
| 4) R^{∗}S(=O)₂-OH | -S(=O)₂- | R^{∗}S(=O)₂NH-R^{∗∗} |
| 5) R^{∗}-CH₂-X (X is Br, I, Cl) | -CH₂- | R^{∗}-CH₂-S-R^{∗∗} |
| 6) R^{∗}-NH₂ | -N- | R^{∗}-NHC(=O)R^{∗∗} |

### II. Embodiments

### A. Auristatin Compounds

The present application is based, in part, on the surprising discovery that it is possible to improve the properties of auristatin-containing Ligand-Drug Conjugate compounds by tuning the hydrophilicity of auristatin drugs with polar moieities, e.g., hydroxyl groups. The hydrophobicity of auristatin drugs is a known barrier to their implementation in pharmaceuticals, including Ligand-Drug Conjugates, due to the potential for off-target toxicity due to high bystander activity and rapid clearance of the drug from the body of subjects. The compounds of the present application attenuate the hydphobicity of the Drug Unit, which in turn attentuates the off-target toxicity and rapid clearance of the drugs. The use of Drug Units with polar moieities in certain locations on the auristatin backbone leads to decreased permeability and on-cell potency of the free drug, and thus fewer off-target effects during treatment, without sacrificing efficacy of intact Ligand-Drug Conjugate compounds that incorporate the Drug Units.

In some embodiments herein, provided are auristatin compounds comprising at least one polar moiety on the auristatin backbone. In some embodiments, provided herein are Drug-Linker compounds comprising an auristatin moiety, as described herein. In some embodiments, provided herein are Ligand-Drug Conjugate compounds comprising an auristatin moiety, as described herein. In some embodiments, provided herein are methods of treating cancer using the Ligand-Drug Conjugate compounds described herein. In some embodiments, provided are methods of making auristatin compounds comprising at least one hydrophilic moiety, Drug-Linkers thereof and their intermediates, and Ligand-Drug Conjugate compounds thereof. In some embodiments, the polar moiety is a polar moiety other than carboxylate. In some embodiments, the polar moiety is a hydroxyl group.

In some embodiments, provided is a compound of formula (I): 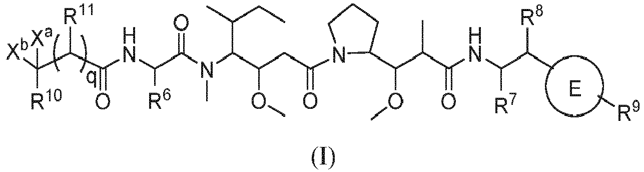 or a salt thereof, wherein
X^{b} is -NR¹R²; and X^{a} is 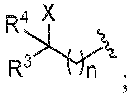 or
X^{a} and X^{b} are taken together with the carbon atom to which they are attached to form 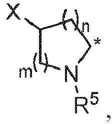 wherein the asterisk denotes the carbon atom of Formula (I) that bears the X^{a} and X^{b} groups;
R¹, R², R³, R⁴, R^{a}, R^{b}, R⁵, and R¹⁰ are each independently H or C₁-C₄ alkyl;
X is H, OH, -C(O)NR^{a}R^{b}, -S(O)₂R^{a}, -S(O)-R^{a} -S(O)₂NR^{a}R^{b}, -NHS(O)₂R^{a}, or -NHC(O)R^{a};
R⁶ is C₁-C₄ alkyl optionally substituted with OH;
R⁷ is H, C₁-C₄ alkyl optionally substituted with one or two OH moieties, or 5-6 membered heteroaryl;
E is phenyl or 5-6 membered heteroaryl;
R⁸, R⁹, and R¹¹ are each independently H or OH;
n is 0, 1, 2, or 3;
m is 1, 2, 3, or 4; and
q is 0 or 1,
wherein when X is H, at least two of R⁷, R⁸, R⁹, and R¹¹ comprise an OH moiety.

In some embodiments, provided is a compound of formula (Iz): 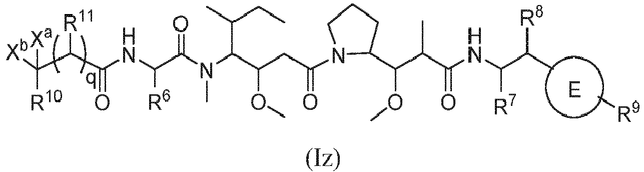 or a salt thereof, wherein
X^{b} is -NR¹R²; and X^{a} is 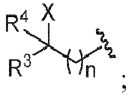 or
X^{a} and X^{b} are taken together with the carbon atom to which they are attached to form 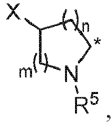 wherein the asterisk denotes the carbon atom of Formula (I) that bears the X^{a} and X^{b} groups;
R¹, R², R³, R⁴, R^{a}, R^{b}, R⁵, and R¹⁰ are each independently H or C₁-C₄ alkyl;
X is OH, -C(O)NR^{a}R^{b}, -S(O)₂R^{a}, -S(O)-R^{a} -S(O)₂NR^{a}R^{b}, -NHS(O)₂R^{a}, or -NHC(O)R^{a};
R⁶ is C₁-C₄ alkyl optionally substituted with OH;
R⁷ is H, C₁-C₄ alkyl optionally substituted with one or two OH moieties, or 5-6 membered heteroaryl;
E is phenyl or 5-6 membered heteroaryl;
R⁸, R⁹, and R¹¹ are each independently H or OH;
n is 0, 1, 2, or 3;
m is 1, 2, 3, or 4; and
q is 0 or 1.

In some embodiments, provided is compound of Formula (II): 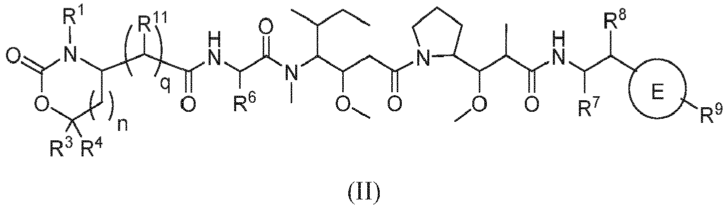 or a salt thereof, wherein
R¹, R³, and R⁴ are independently H or C₁-C₄ alkyl;
R⁶ is C₁-C₄ alkyl optionally substituted with OH;
R⁷ is H, C₁-C₄ alkyl optionally substituted with one or two OH moieties, or 5-6 membered heteroaryl;
E is phenyl or 5-6 membered heteroaryl;
R⁸, R⁹, and R¹¹ are each independently H or OH;
n is 0, 1, or 2; and
q is 0 or 1.

In some embodiments of Formula (I), q is 0. In some embodiments of Formula (I), q is 1. In some embodiments of Formula (II), q is 0. In some embodiments of Formula (II), q is 1.

In some embodiments of Formula (I) or (Iz), X^{b} is -NR¹R² is and X^{a} is 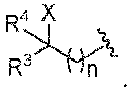 In some embodiments, R¹ and R² are each independently C₁-C₄ alkyl. In some embodiments, R¹ and R² are both H. In some embodiments, R¹ and R² are each independently n-propyl, isopropyl, ethyl, or methyl. In some embodiments, R¹ and R² are each independently ethyl or methyl. In some embodiments, R¹ and R² are both methyl. In some embodiments, R¹ is H and R² is C₁-C₄ alkyl. In some embodiments, R¹ is H and R² is n-propyl, isopropyl, ethyl, or methyl. In some embodiments, R¹ is H and R² is ethyl or methyl. In some embodiments, R¹ is H and R² is methyl. In some emodiments, R³ and R⁴ are each independently C₁-C₄ alkyl. In some embodiments, R³ is H and R⁴ is C₁-C₄ alkyl. In some embodiments, R³ and R⁴ are H. In some embodiments, R³ is H and R⁴ is methyl. In some embodiments, n is 0, 1, 2, or 3. In some embodiments, n is 0, 1, or 2. In some embodiments, n is 0 or 1. In some embodiments, n is 1. In some embodiments, n is 0. In some embodiments, R¹ is H, R² is methyl, R³ is H, R⁴ is H, and n is 0. In some embodiments, R¹ is methyl, R² is methyl, R³ is H, R⁴ is H, and n is 0.

In some embodiments of Formula (I) or (Iz), X^{a} and X^{b} are taken together with the carbon atom to which they are attached to form 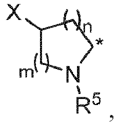 wherein the asterisk denotes the carbon atom of Formula (I) that bears the X^{a} and X^{b} groups. In some embodiments, R⁵ is H, n-propyl, isopropyl, ethyl, or methyl. In some embodiments, R⁵ is H, ethyl, or methyl. In some embodiments, R⁵ is H or methyl. In some embodiments, R⁵ is ethyl or methyl. In some embodiments, R⁵ is H. In some embodiments, R⁵ is ethyl. In some embodiments, R⁵ is methyl. In some embodiments, m is 1, 2, or 3. In some embodiments, m is 1 or 2. In some embodiments, m is 2 or 3. In some embodiments, m is 2. In some embodiments, n is 0, 1, or 2. In some embodiments, n is 0 or 1. In some embodiments, n is 0 or 1 and m is 0, 1, or 2. In some embodiments, n is 0 and m is 2. In some embodiments, n is 1 and m is 1. In some embodiments, m is 2 and n is 1. In some embodiments, X is H or OH. In some embodiments, X is H. In some embodiments, X is OH.

In some embodiments of Formula (I), X is H, OH, -C(O)NR^{a}R^{b}, -S(O)₂R^{a}, -S(O)-R^{a}, -S(O)₂NR^{a}R^{b}, -NHS(O)₂R^{a}, or -NHC(O)R^{a}. In some embodiments, X is OH, -S(O)₂R^{a}, -S(O)-R^{a}, -S(O)₂NR^{a}R^{b}, -NHS(O)₂R^{a}, or -NHC(O)R^{a}. In some embodiments, X is H or OH. In some embodiments, X is OH. In some embodiments, X is OH, -NHS(O)₂R^{a}, or -NHC(O)R^{a}. In some embodiments, X is OH, -S(O)₂R^{a}, -S(O)-R^{a}, or -S(O)₂NR^{a}R^{b}. In some embodiments, X is -NHS(O)₂R^{a}, or -NHC(O)R^{a}. In some embodiments, X is -S(O)₂R^{a}, -S(O)-R^{a}, or -S(O)₂NR^{a}R^{b}. In some embodiments, X is H, OH, -NHS(O)₂R^{a}, or -NHC(O)R^{a}. In some embodiments, X is H, OH, -S(O)₂R^{a}, -S(O)-R^{a}, or -S(O)₂NR^{a}R^{b}. In some embodiments, X is H, -NHS(O)₂R^{a}, or -NHC(O)R^{a}. In some embodiments, X is H, -S(O)₂R^{a}, -S(O)-R^{a}, or -S(O)₂NR^{a}R^{b}. In some embodiments, X is H, OH, or -C(O)NR^{a}R^{b}. In some embodiments, X is OH or -C(O)NR^{a}R^{b}. In some embodiments, X is H or -C(O)NR^{a}R^{b}. In some embodiments, R^{a} and R^{b} are independently H, n-propyl, isopropyl, ethyl, or methyl. In some embodiments, R^{a} and R^{b} are independently H, ethyl, or methyl. In some embodiments, R^{a} and R^{b} are independently H or methyl. In some embodiments, R^{a} is H and R^{b} is n-propyl, isopropyl, ethyl, or methyl. In some embodiments, R^{a} is H and R^{b} is methyl.

In some embodiments of Formula (Iz), X is OH, -C(O)NR^{a}R^{b}, -S(O)₂R^{a}, -S(O)-R^{a}, -S(O)₂NR^{a}R^{b}, -NHS(O)₂R^{a}, or -NHC(O)R^{a}. In some embodiments, X is OH. In some embodiments, X is OH, -NHS(O)₂R^{a}, or -NHC(O)R^{a}. In some embodiments, X is OH, -S(O)₂R^{a}, -S(O)-R^{a}, or -S(O)₂NR^{a}R^{b}. In some embodiments, X is -NHS(O)₂R^{a}, or -NHC(O)R^{a}. In some embodiments, X is -S(O)₂R^{a}, -S(O)-R^{a}, or -S(O)₂NR^{a}R^{b}. In some embodiments, X is OH or -C(O)NR^{a}R^{b}. In some embodiments, X is -C(O)NR^{a}R^{b}. In some embodiments, R^{a} and R^{b} are independently H, n-propyl, isopropyl, ethyl, or methyl. In some embodiments, R^{a} and R^{b} are independently H, ethyl, or methyl. In some embodiments, R^{a} and R^{b} are independently H or methyl. In some embodiments, R^{a} is H and R^{b} is n-propyl, isopropyl, ethyl, or methyl. In some embodiments, R^{a} is H and R^{b} is methyl.

In some embodiments of Formula (I) or (Iz), X^{b} is -NR¹R² is and X^{a} is 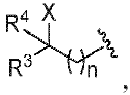 X is OH, R¹ is H or methyl, R² is methyl, R³ is H, R⁴ is H, and n is 0. In some embodiments, X is OH, R¹ is methyl, R² is methyl, R³ is H, R⁴ is H, and n is 0. In some embodiments, X is OH, R¹ is H, R² is methyl, R³ is H, R⁴ is H, and n is 0. In some embodiments, X is OH, R³ and R⁴ are H, and n is 0. In some embodiments, X is OH, R¹ is H, R² is H, R³ is H, R⁴ is H, and n is 0.

In some embodiments of Formula (II), R¹ is H, n-propyl, isopropyl, ethyl, or methyl. In some embodiments, R¹ is H, ethyl, or methyl. In some embodiments, R¹ is H or methyl. In some embodiments, R¹ is ethyl. In some embodiments, R¹ is methyl. In some embodiments, R¹ is H. In some embodiments, n is 0 or 1. In some embodiments, n is 1. In some embodiments, n is 0.

In some embodiments of Formula (II), R³ and R⁴ are H. In some embodiments, R³ and R⁴ are methyl. In some embodiments, R³ is H and R⁴ is methyl.

In some embodiments or Formula (I), (Iz), or (II), R⁶ is n-propyl, isopropyl, ethyl, or methyl. In some embodiments, R⁶ is ethyl or methyl. In some embodiments, R⁶ is isopropyl. In some embodiments, R⁶ is n-propyl, isopropyl, ethyl, or methyl, each of which is substituted with OH. In some embodiments, R⁶ is isopropyl substituted with OH. In some embodiments, R⁶ is ethyl substituted with OH. In some embodiments, R⁶ is methyl substituted with OH.

In some embodiments of Formula (I), (Iz), or (II), R⁷ is C₁-C₄ alkyl substituted with OH. In some embodiments, R⁷ is C₁-C₄ alkyl subsituted by two OH moieties. In some embodiments, R⁷ is n-propyl, isopropyl, ethyl, or methyl, each of which is substituted with OH. In some embodiments, R⁷is C₁-C₄ alkyl. In some embodiments, R⁷ is n-propyl, isopropyl, ethyl, or methyl. In some embodiments, R⁷ is methyl. In some embodiments, R⁷ is -CH₂CH₂OH or CH₂OH. In some embodiments, R⁷ is -CH₂OH. In some embodiments, R⁷ is 5-6 membered heteroaryl. In some embodiments, R⁷ is 5-membered heteroaryl. In some embodiments, R⁷ is thiazolyl.

In some embodiments of Formula (I), (Iz), or (II), R⁸, R⁹, and R¹¹ are H. In some embodiments, R⁸ is OH, R⁹ is H, and R¹¹ is H. In some embodiments, R⁸ and R⁹ are each OH and R¹¹ is H. In some embodiments, R⁸ is OH, R⁹ is H, and R¹¹ is OH. In some embodiments, R⁸ is H, R⁹ is H, and R¹¹ is OH.

In some embodiments of Formula (I), (Iz), or (II), E is a 6-membered ring. In some embodiments, E is pyridine or phenyl. In some embodiments, E is phenyl.

In some embodiments of Formula (I), (Iz), or (II), R⁷ is -CH₂OH, R⁸ is H, R⁹ is H, and E is phenyl.

In some embodiments, provided is a compound of Formula (Ia): 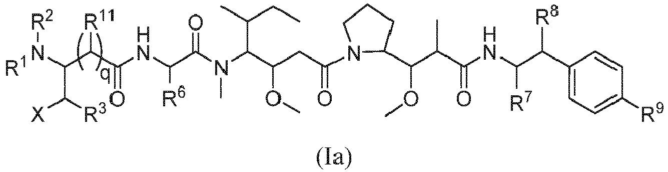 or a salt thereof, wherein
R¹, R², R³, R⁴, R^{a}, and R^{b} are each independently H or C₁-C₄ alkyl;
X is H, OH, -C(O)NR^{a}R^{b}, -S(O)₂R^{a}, -S(O)-R^{a} -S(O)₂NR^{a}R^{b}, -NHS(O)₂R^{a}, or -NHC(O)R^{a}; R⁶ is C₁-C₄ alkyl;
R⁷ is H, C₁-C₄ alkyl optionally substituted with one or two OH moieties, or 5-6 membered heteroaryl;
R⁸, R⁹, and R¹¹ are each independently H or OH;
q is 0 or 1; and
wherein when X is H, at least two of R⁷, R⁸, R⁹, and R¹¹ comprise an OH moiety.

In some embodiments of Formula (la), q is 0. In some embodiments, q is 1.

In some embodiments of Formula (la), R¹ is H and R² is C₁-C₄-alkyl. In some embodiments, R¹ is H and R² is n-propyl, isopropyl, ethyl, or methyl. In some embodiments, R¹ is H and R² is ethyl or methyl. In some embodiments, R¹ is H and R² is methyl. In some embodiments, R¹ and R² are each independently C₁-C₄ alkyl. In some embodiments, R¹ and R² are each independently n-propyl, isopropyl, ethyl, or methyl. In some embodiments, R¹ and R² are each independently ethyl or methyl. In some embodiments, R¹ and R² are methyl. In some embodiments, R¹ and R² are both H.

In some embodiments of Formula (la), X is H, OH, -C(O)NR^{a}R^{b}, -S(O)₂R^{a}, -S(O)-R^{a}, -S(O)₂NR^{a}R^{b}, -NHS(O)₂R^{a}, or -NHC(O)R^{a}. In some embodiments, X is OH, -S(O)₂R^{a}, -S(O)-R^{a}, -S(O)₂NR^{a}R^{b}, -NHS(O)₂R^{a}, or -NHC(O)R^{a}. In some embodiments, X is H or OH. In some embodiments, X is OH. In some embodiments, X is OH, -NHS(O)₂R^{a}, or -NHC(O)R^{a}. In some embodiments, X is OH, -S(O)₂R^{a}, -S(O)-R^{a}, or -S(O)₂NR^{a}R^{b}. In some embodiments, X is -NHS(O)₂R^{a}, or -NHC(O)R^{a}. In some embodiments, X is -S(O)₂R^{a}, -S(O)-R^{a}, or -S(O)₂NR^{a}R^{b}. In some embodiments, X is H, OH, -NHS(O)₂R^{a}, or -NHC(O)R^{a}. In some embodiments, X is H, OH, -S(O)₂R^{a}, -S(O)-R^{a}, or -S(O)₂NR^{a}R^{b}. In some embodiments, X is H, -NHS(O)₂R^{a}, or -NHC(O)R^{a}. In some embodiments, X is H, -S(O)₂R^{a}, -S(O)-R^{a}, or -S(O)₂NR^{a}R^{b}. In some embodiments, X is H, OH, or -C(O)NR^{a}R^{b}. In some embodiments, X is OH or -C(O)NR^{a}R^{b}. In some embodiments, X is H or -C(O)NR^{a}R^{b}. In some embodiments, R^{a} and R^{b} are independently H, n-propyl, isopropyl, ethyl, or methyl. In some embodiments, R^{a} and R^{b} are independently H, ethyl, or methyl. In some embodiments, R^{a} and R^{b} are independently H or methyl. In some embodiments, R^{a} is H and R^{b} is n-propyl, isopropyl, ethyl, or methyl. In some embodiments, R^{a} is H and R^{b} is methyl.

In some embodiments of Formula (la), X is OH, -C(O)NR^{a}R^{b}, -S(O)₂R^{a}, -S(O)-R^{a}, -S(O)₂NR^{a}R^{b}, -NHS(O)₂R^{a}, or -NHC(O)R^{a}. In some embodiments, X is OH. In some embodiments, X is OH, -NHS(O)₂R^{a}, or -NHC(O)R^{a}. In some embodiments, X is OH, -S(O)₂R^{a}, -S(O)-R^{a}, or -S(O)₂NR^{a}R^{b}. In some embodiments, X is -NHS(O)₂R^{a}, or -NHC(O)R^{a}. In some embodiments, X is -S(O)₂R^{a}, -S(O)-R^{a}, or -S(O)₂NR^{a}R^{b}. In some embodiments, X is OH or -C(O)NR^{a}R^{b}. In some embodiments, X is -C(O)NR^{a}R^{b}. In some embodiments, R^{a} and R^{b} are independently H, n-propyl, isopropyl, ethyl, or methyl. In some embodiments, R^{a} and R^{b} are independently H, ethyl, or methyl. In some embodiments, R^{a} and R^{b} are independently H or methyl. In some embodiments, R^{a} is H and R^{b} is n-propyl, isopropyl, ethyl, or methyl. In some embodiments, R^{a} is H and R^{b} is methyl.

In some embodiments of Formula (la), R⁶ is n-propyl, isopropyl, ethyl, or methyl. In some embodiments, R⁶ is ethyl or methyl. In some embodiments, R⁶ is isopropyl. In some embodiments, R⁶ is n-propyl, isopropyl, ethyl, or methyl, each of which is substituted with OH. In some embodiments, R⁶ is isopropyl substituted with OH. In some embodiments, R⁶ is ethyl substituted with OH. In some embodiments, R⁶ is methyl substituted with OH.

In some embodiments of Formula (la), R⁷ is C₁-C₄ alkyl substituted with OH. In some embodiments, R⁷ is n-propyl, isopropyl, ethyl, or methyl, each of which is substituted with OH. In some embodiments, R⁷ is -CH₂CH₂OH or CH₂OH. In some embodiments, R⁷ is -CH₂OH. In some embodiments, R⁷ is C₁-C₄ alkyl. In some embodiments, R⁷ is n-propyl, isopropyl, ethyl, or methyl. In some embodiments, R⁷ is methyl. In some embodiments, R⁷ is 5-6 membered heteroaryl. In some embodiments, R⁷ is 5-membered heteroaryl. In some embodiments, R⁷ is thiazolyl.

In some embodiments of Formula (la), R⁸, R⁹, and R¹¹ are H. In some embodiments, R⁸ is OH, R⁹ is H, and R¹¹ is H. In some embodiments, R⁸ and R⁹ are each OH and R¹¹ is H. In some embodiments, R⁸ is OH, R⁹ is H, and R¹¹ is OH.

In some embodiments of Formula (la), X is OH, R³ is H or methyl, R¹ is H or C₁-C₄ alkyl, R² is C₁-C₄ alkyl, R⁶ is C₁-C₄ alkyl, R⁷ is C₁-C₄ alkyl optionally substituted with OH, R⁸ is H or C₁-C₄ alkyl, R⁹ is H, and q is 0. In some embodiments, X is OH, R³ is H, R¹ is H or methyl, R² is methyl, R⁶ is isopropyl, R⁷ is CH₂OH, R⁸ is H, R⁹ is H, and q is 0.

In some embodiments, provided is a compound of Formula (Ib): 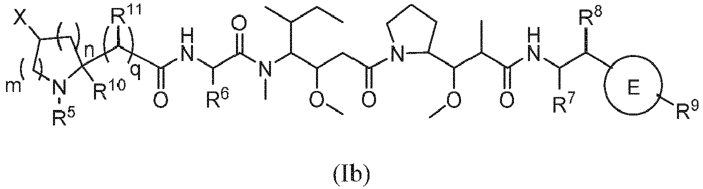 or a salt thereof, wherein the variables are as defined for Formula (I).

In some embodiments of Formula (Ib), X is H. In some embodiments, X is H or OH. In some embodiments, R⁵ is H or methyl. In some embodiments, R⁵ is H. In some embodiments, m is 1 or 2. In some embodiments, m is 2. In some embodiments, n is 2. In some embodiments, n is 0 or 1. In some embodiments, n is 1. In some embodiments, n is 0. In some embodiments, m is 2 and n is 0. In some embodiments, m is 1 and n is 1.

In some embodiments or Formula (Ib), R⁶ is n-propyl, isopropyl, ethyl, or methyl. In some embodiments, R⁶ is ethyl or methyl. In some embodiments, R⁶ is isopropyl. In some embodiments, R⁶ is n-propyl, isopropyl, ethyl, or methyl, each of which is substituted with OH. In some embodiments, R⁶ is isopropyl substituted with OH. In some embodiments, R⁶ is ethyl substituted with OH. In some embodiments, R⁶ is methyl substituted with OH.

In some embodiments of Formula (Ib), R⁷ is C₁-C₄ alkyl substituted with OH. In some embodiments, R⁷ is n-propyl, isopropyl, ethyl, or methyl, each of which substituted with OH. In some embodiments, R⁷is C₁-C₄ alkyl. In some embodiments, R⁷ is n-propyl, isopropyl, ethyl, or methyl. In some embodiments, R⁷ is methyl. In some embodiments, R⁷ is -CH₂CH₂OH or CH₂OH. In some embodiments, R⁷ is -CH₂OH. In some embodiments, R⁷ is 5-6 membered heteroaryl. In some embodiments, R⁷ is 5-membered heteroaryl. In some embodiments, R⁷ is thiazolyl.

In some embodiments of Formula (Ib), R⁸, R⁹, and R¹¹ are each H. In some embodiments, R⁸ is OH, R⁹ is H, and R¹¹ is H. In some embodiments, R⁸ and R⁹ are each OH and R¹¹ is H. In some embodiments, R⁸ is OH, R⁹ is H, and R¹¹ is OH. In some embodiments, R⁸ is H, R⁹ is H, and R¹¹ is OH.

In some embodiments of Formula (Ib), E is a 6-membered ring. In some embodiments, E is pyridine or phenyl. In some embodiments, E is phenyl.

In some embodiments of Formula (Ib), R⁷ is -CH₂OH, R⁸ is H, R⁹ is H, and E is phenyl.

In some embodiments of Formula (Ib), R⁷ is -CH₂OH, R⁸ is H, and R⁹ is H. In some embodiments, m is 2, n is 0, X is H, R¹⁰ is methyl, R⁶ is isopropyl, R⁷ is H, R⁸ is OH, and R⁹ is OH. In some embodiments, m is 1, n is 1, X is H, R¹⁰ is methyl, R⁶ is isopropyl, R⁷ is H, R⁸ is OH, and R⁹ is OH. In some embodiments, m is 2, n is 1, X is H, R¹⁰ is methyl, R⁶ is isopropyl, R⁷ is H, R⁸ is OH, and R⁹ is OH.

In some embodiments, provided is a compound of Formula (Ic): 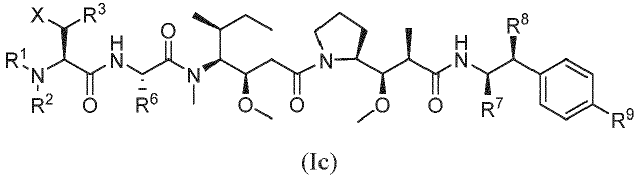 or a salt thereof, wherein the variables are as defined for Formula (I), (Iz), or (la).

In some embodiments, provided is a compound of Formula (Id): 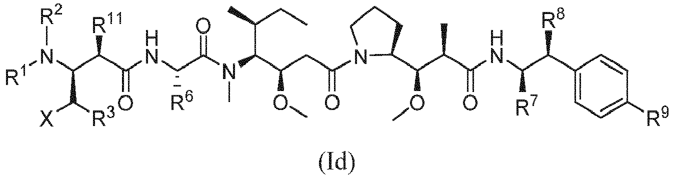 or a salt thereof, wherein the variables are as defined for Formula (I), (Iz), or (la).

In some embodiments, provided is a compound of Formula (Ie): 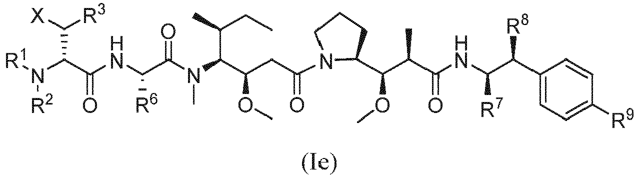 or a salt thereof, wherein the variables are as defined for Formula (I), (Iz), or (la).

In some embodiments, provided is a compound of Formula (Id): 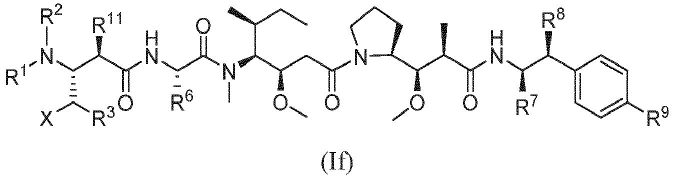 or a salt thereof, wherein the variables are as defined for Formula (I), (Iz), or (la).

In some embodiments of Formula (Ic), (Id), (Ie), or (If), R¹ and R¹ are both H. R¹ is H and R² is C₁-C₄-alkyl. In some embodiments, R¹ is H and R² is n-propyl, isopropyl, ethyl, or methyl. In some embodiments, R¹ is H and R² is ethyl or methyl. In some embodiments, R¹ is H and R² is methyl. In some embodiments, R¹ and R² are each independently C₁-C₄ alkyl. In some embodiments, R¹ and R² are each independently n-propyl, isopropyl, ethyl, or methyl. In some embodiments, R¹ and R² are each independently ethyl or methyl. In some embodiments, R¹ and R² are methyl.

In some embodiments of Formula (Ic), (Id), (Ie), or (If), X is H, OH, -C(O)NR^{a}R^{b}, or -NHC(O)R^{a}. In some embodiments, X is H, OH, -C(O)NR^{a}R^{b}, or -NHC(O)R^{a}. In some embodiments, X is H or OH. In some embodiments, X is OH. In some embodiments, R³ is H or C₁-C₄ alkyl. In some embodiments, R³ is H, n-propyl, isopropyl, ethyl, or methyl. In some embodiments, R³ is H, ethyl, or methyl. In some embodiments, X is H or OH and R³ is H or methyl. In some embodiments, X is OH and R³ is H. In some embodiments, X is OH and R³ is methyl. In some embodiments, R^{a} is ethyl or methyl. In some embodiments, R^{a} is methyl.

In some embodiments of Formula (Ic), (Id), (Ie), or (If), R⁶ is n-propyl, isopropyl, ethyl, or methyl. In some embodiments, R⁶ is ethyl or methyl. In some embodiments, R⁶ is isopropyl. In some embodiments, R⁶ is n-propyl, isopropyl, ethyl, or methyl, each of which is substituted with OH. In some embodiments, R⁶ is isopropyl substituted with OH. In some embodiments, R⁶ is ethyl substituted with OH. In some embodiments, R⁶ is methyl substituted with OH.

In some embodiments of Formula (Ic), (Id), (Ie), or (If), R⁷ is C₁-C₄ alkyl substituted with OH. In some embodiments, R⁷ is C₁-C₄ alkyl subsituted by two OH moieties. In some embodiments, R⁷ is n-propyl, isopropyl, ethyl, or methyl, each of which is substituted with OH. In some embodiments, R⁷ is -CH₂CH₂OH or CH₂OH. In some embodiments, R⁷ is -CH₂OH. In some embodiments, R⁷is C₁-C₄ alkyl. In some embodiments, R⁷ is n-propyl, isopropyl, ethyl, or methyl. In some embodiments, R⁷ is methyl. In some embodiments, R⁷ is 5-6 membered heteroaryl. In some embodiments, R⁷ is 5-membered heteroaryl. In some embodiments, R⁷ is thiazolyl.

In some embodiments of Formula (Ic) or (Ie), R⁸ and R⁹ are H. In some embodiments, R⁸ is OH and R⁹ is H. In some embodiments, R⁸ and R⁹ are each OH.

In some embodiments of Formaul (Id) or (If), R⁸, R⁹, and R¹¹ are H. In some embodiments, R⁸ is OH, R⁹ is H, and R¹¹ is H. In some embodiments, R⁸ and R⁹ are each OH and R¹¹ is H. In some embodiments, R⁸ is OH, R⁹ is H, and R¹¹ is OH. In some embodiments, R⁸ is H, R⁹ is H, and R¹¹ is OH.

In some embodiments of Formula (Ic), (Id), (Ie), or (If), X is OH, R³ is H or methyl, R¹ is H or C₁-C₄ alkyl, R² is C₁-C₄ alkyl, R⁶ is C₁-C₄ alkyl, R⁷ is C₁-C₄ alkyl optionally substituted with OH, R⁸ is H or C₁-C₄ alkyl, and R⁹ is H. In some embodiments, X is OH, R³ is H, R¹ is H or methyl, R² is methyl, R⁶ is isopropyl, R⁷ is CH₂OH, R⁸ is H, and R⁹ is H.

In some embodiments of Formula (Ic), R⁷ is -CH₂OH, R⁸ is H, and R⁹ is H.

In some embodiments, provided is a compound of Formula (IIa): 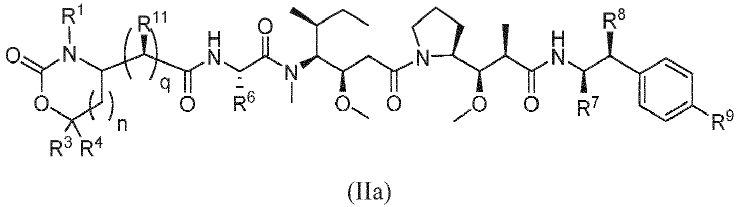 or a salt thereof, wherein the variables are as defined for Formula (II).

In some embodiments of Formula (IIa), q is 0. In some embodiments, q is 1.

In some embodiments of Formula (IIa), R¹ is H, n-propyl, isopropyl, ethyl, or methyl. In some embodiments, R¹ is H, ethyl, or methyl. In some embodiments, R¹ is H or methyl. In some embodiments, R¹ is ethyl. In some embodiments, R¹ is methyl. In some embodiments, R¹ is H. In some embodiments, n is 0 or 1. In some embodiments, n is 1. In some embodiments, n is 0.

In some embodiments or Formula (IIa), R⁶ is n-propyl, isopropyl, ethyl, or methyl. In some embodiments, R⁶ is ethyl or methyl. In some embodiments, R⁶ is isopropyl. In some embodiments, R⁶ is n-propyl, isopropyl, ethyl, or methyl, each of which is substituted with OH. In some embodiments, R⁶ is isopropyl substituted with OH. In some embodiments, R⁶ is ethyl substituted with OH. In some embodiments, R⁶ is methyl substituted with OH.

In some embodiments of Formula (IIa), R⁷ is C₁-C₄ alkyl substituted with OH. In some embodiments, R⁷ is C₁-C₄ alkyl subsituted by two OH moieties. In some embodiments, R⁷ is n-propyl, isopropyl, ethyl, or methyl, each of which is substituted with OH. In some embodiments, R⁷is C₁-C₄ alkyl. In some embodiments, R⁷ is n-propyl, isopropyl, ethyl, or methyl. In some embodiments, R⁷ is methyl. In some embodiments, R⁷ is -CH₂CH₂OH or CH₂OH. In some embodiments, R⁷ is -CH₂OH. In some embodiments, R⁷ is 5-6 membered heteroaryl. In some embodiments, R⁷ is 5-membered heteroaryl. In some embodiments, R⁷ is thiazolyl.

In some embodiments of Formula (IIa), R⁸, R⁹, and R¹¹ are H. In some embodiments, R⁸ is OH, R⁹ is H, and R¹¹ is H. In some embodiments, R⁸ and R⁹ are each OH and R¹¹ is H. In some embodiments, R⁸ is OH, R⁹ is H, and R¹¹ is OH. In some embodiments, R⁸ is H, R⁹ is H, and R¹¹ is OH.

In some embodiments of Formula (IIa), E is a 6-membered ring. In some embodiments, E is pyridine or phenyl. In some embodiments, E is phenyl.

In some embodiments of Formula (IIa), R⁷ is -CH₂OH, R⁸ is H, R⁹ is H, and R¹¹ is H.

In some embodiments, provided is a compound of Table 1, or a salt thereof (e.g., a pharmaceutically acceptable salt).

**Table 1. Hydrophilic auristatin compounds**

| Compound # | Compound Structure |
|---|---|
| 1.1 | 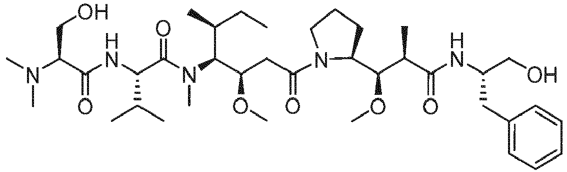 |
| 1.2 | 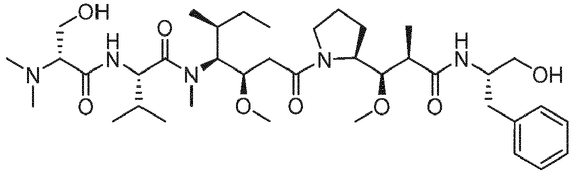 |
| 1.3 | 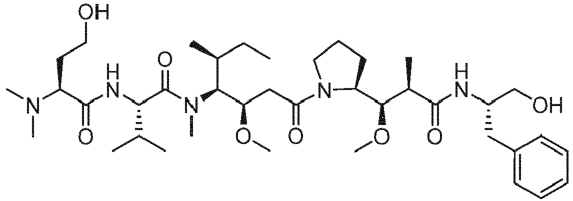 |
| 1.4 | 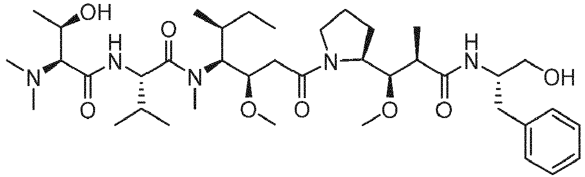 |
| 1.5 | 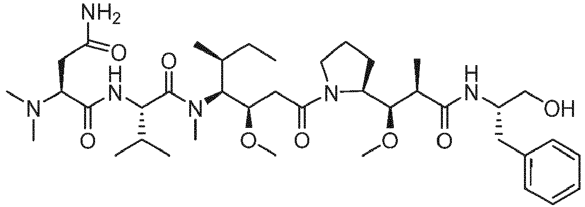 |
| 1.6 | 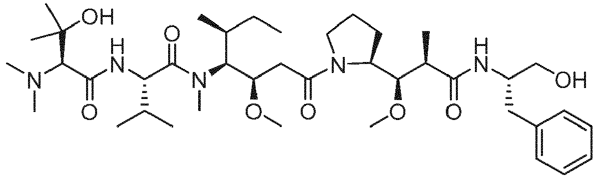 |
| 1.7 | 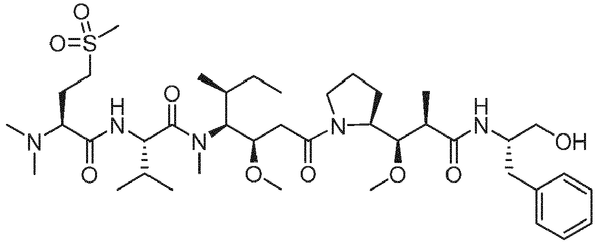 |
| 1.8 | 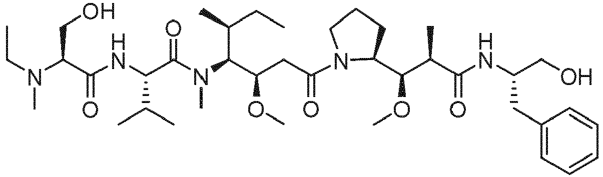 |
| 1.9 | 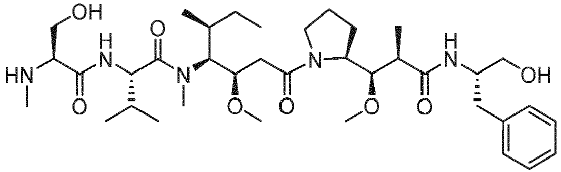 |
| 1.10 | 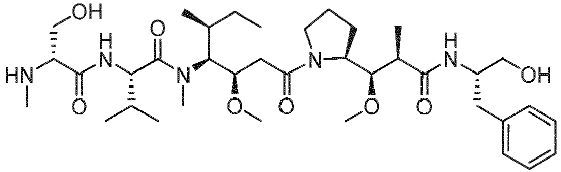 |
| 1.11 | 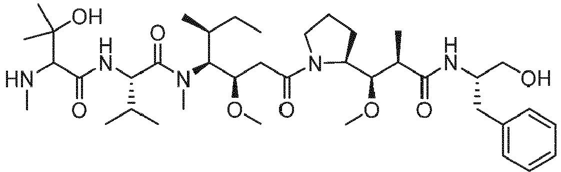 |
| 1.12 | 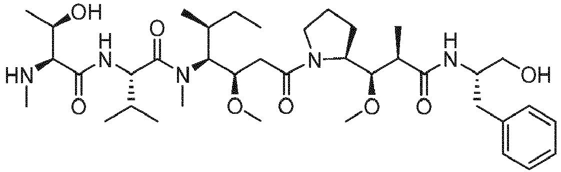 |
| 1.13 | 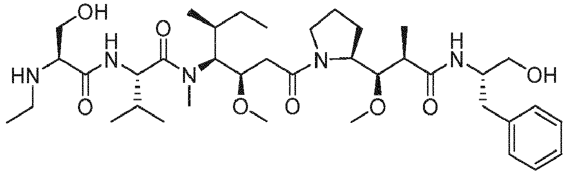 |
| 1.14 | 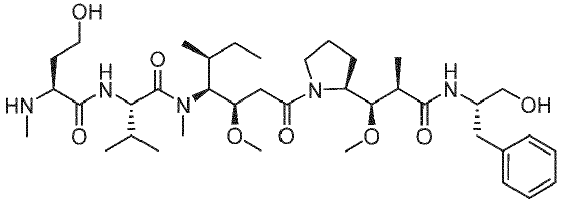 |
| 1.15 | 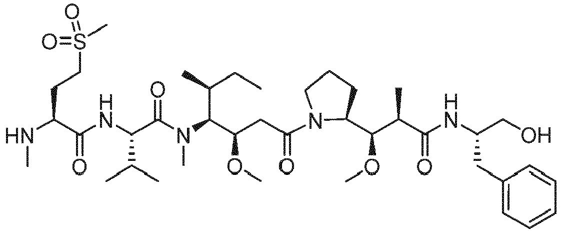 |
| 1.16 | 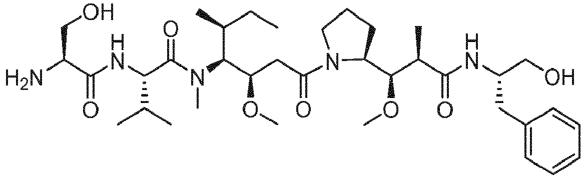 |
| 1.17 | 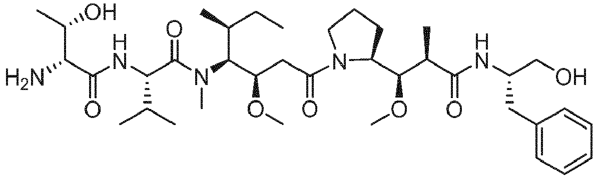 |
| 1.18 | 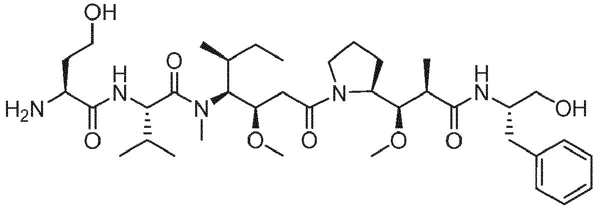 |
| 1.19 | 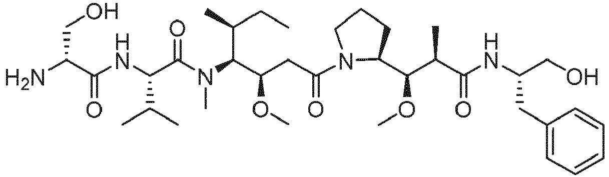 |
| 1.20 | 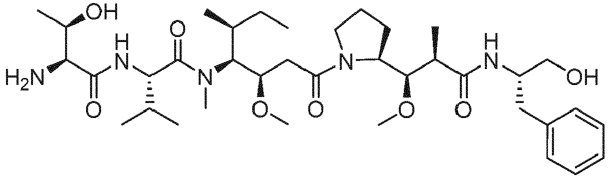 |
| 1.21 | 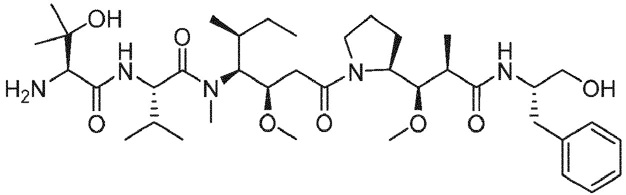 |
| 1.22 | 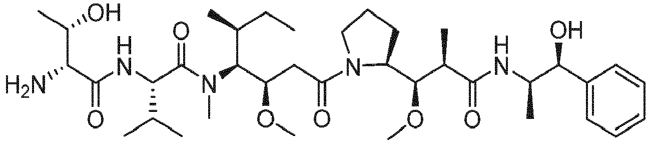 |
| 1.23 | 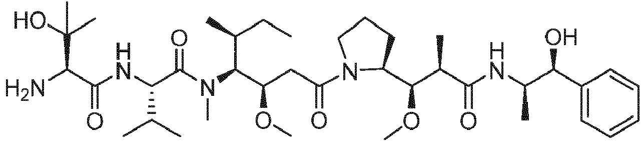 |
| 1.24 | 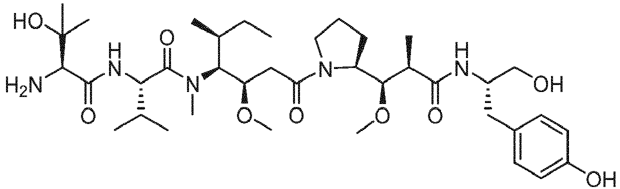 |
| 1.25 | 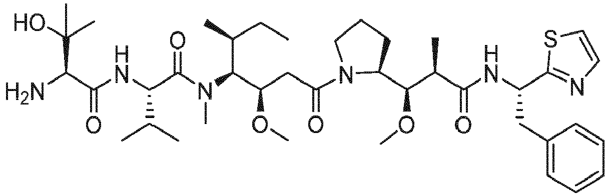 |
| 1.26 | 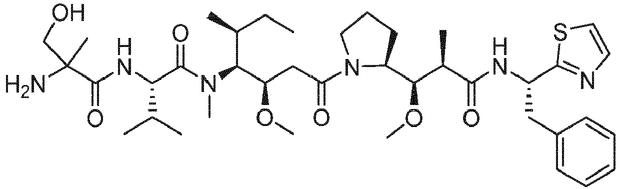 |
| 1.27 | 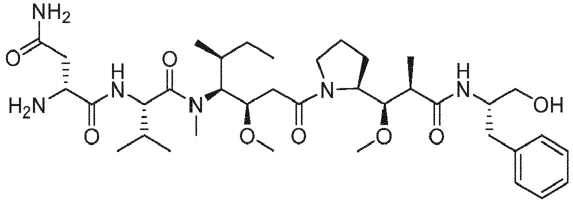 |
| 1.28 | 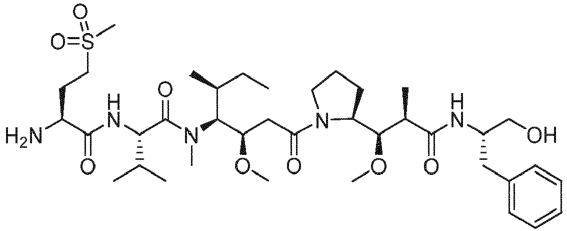 |
| 1.29 | 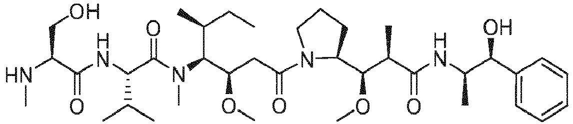 |
| 1.30 | 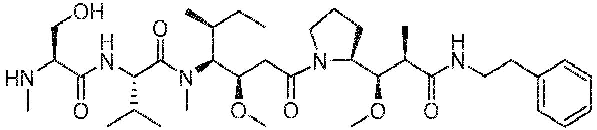 |
| 1.31 | 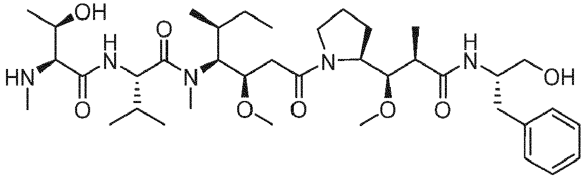 |
| 1.32 | 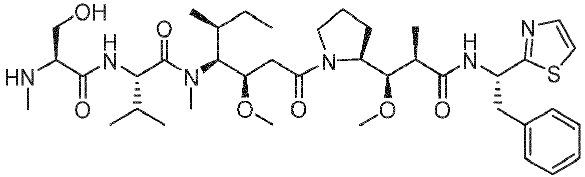 |
| 1.33 | 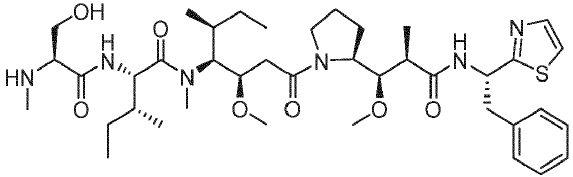 |
| 1.34 | 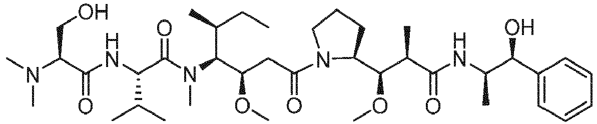 |
| 1.35 | 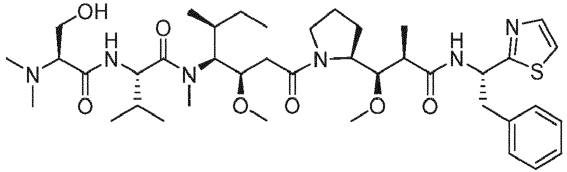 |
| 1.36 | 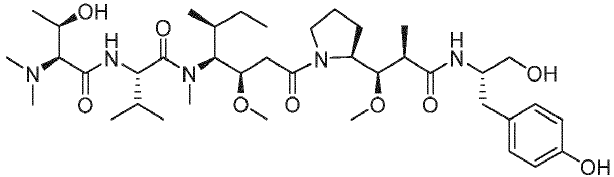 |
| 1.37 | 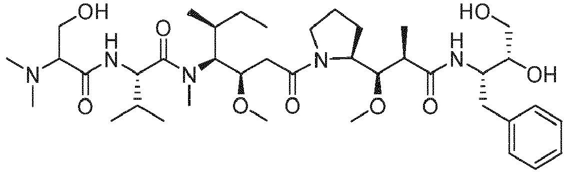 |
| 1.38 | 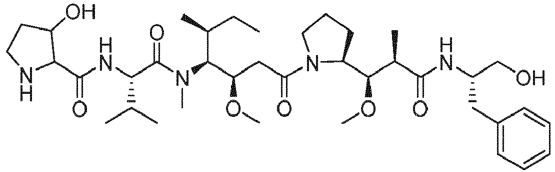 |
| 1.39 | 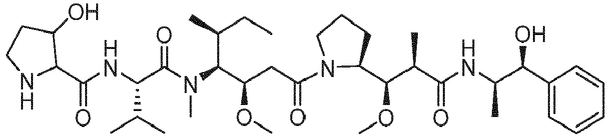 |
| 1.40 | 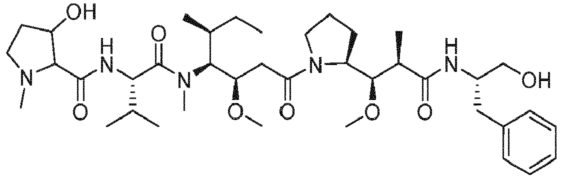 |
| 1.41 | 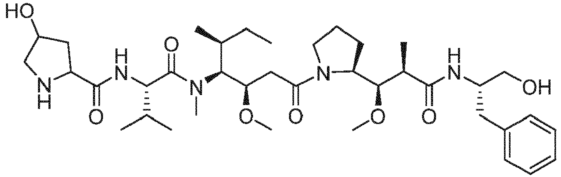 |
| 1.42 | 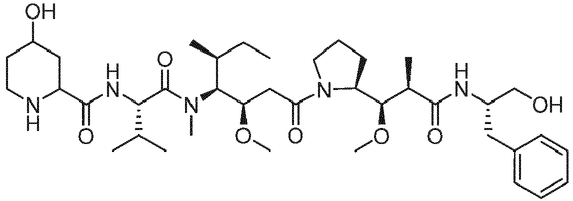 |
| 1.43 | 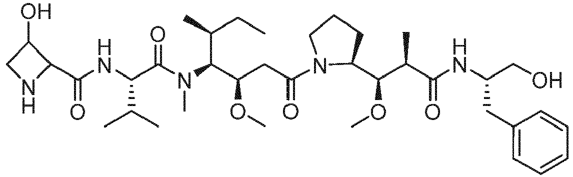 |
| 1.44 | 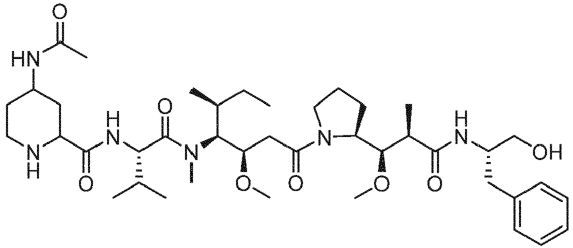 |
| 1.45 | 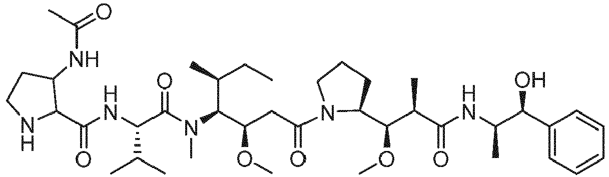 |
| 1.46 | 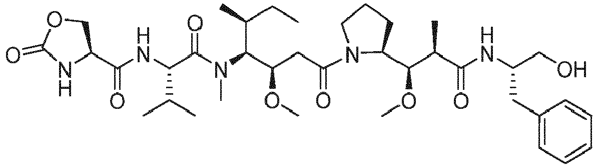 |
| 1.47 | 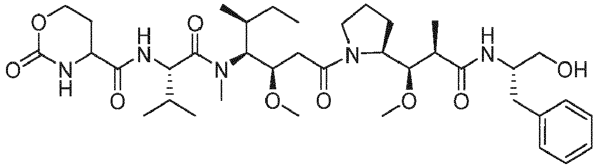 |
| 1.48 | 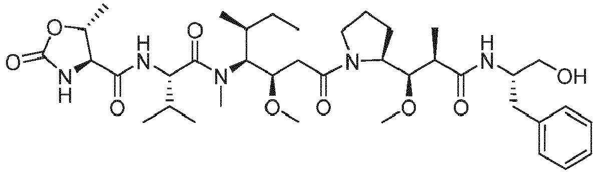 |
| 1.49 | 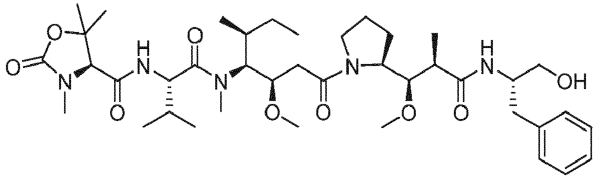 |
| 1.50 | 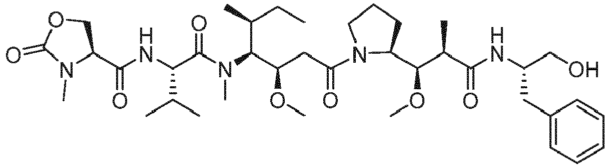 |
| 1.51 | 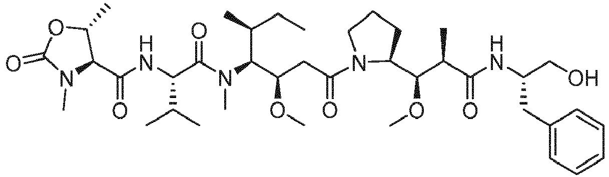 |
| 1.52 | 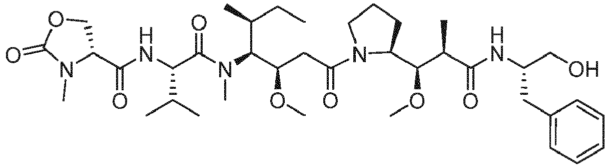 |
| 1.53 | 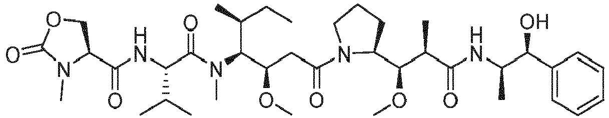 |
| 1.54 | 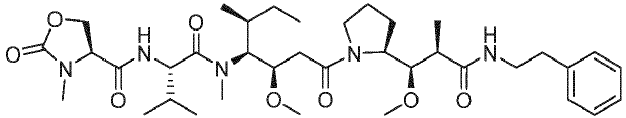 |
| 1.55 | 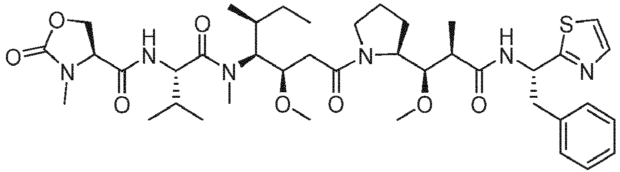 |
| 1.56 | 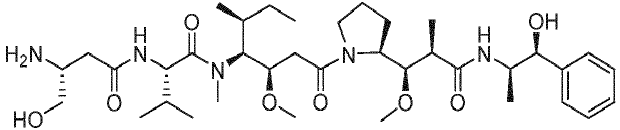 |
| 1.57 | 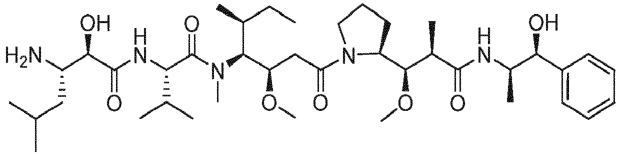 |
| 1.58 | 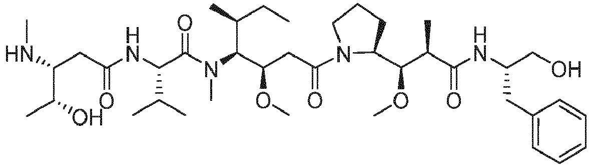 |
| 1.59 | 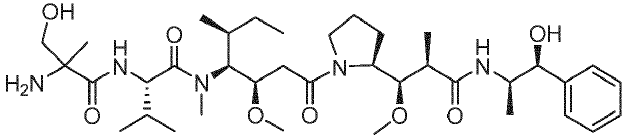 |
| 1.60 | 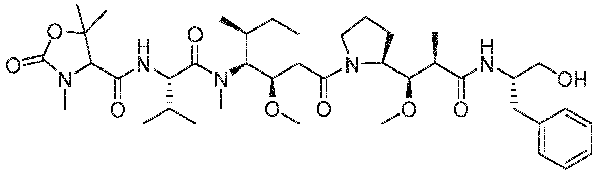 |

### Drug-Linker Compounds

In some embodiments, when preparing Ligand-Drug Conjugate compounds described herein, it will be desirable to synthesize the full Drug-Linker compound prior to conjugation to a targeting agent, which becomes the Ligand Unit of the Ligand-Drug conjugate compound. In such embodiments, Drug-Linker compounds as described herein, are intermediate compounds. In those embodiments, the Stretcher Unit in a Drug-Linker compound is not yet covalently attached to the Ligand Unit (*i.e.,* is a Stretcher Unit precursor, Z'), and therefore has a functional group for conjugation to a targeting agent. In one embodiment, a Drug-Linker compound comprises an auristatin moiety (shown herein as Formulae (I) and (II), or any subformula thereof) or, and a Linker Unit (Q) through which the Ligand Unit is connected to the Drug Unit.

In another embodiment, a Drug-Linker compound comprises an auristatin compound of Formula (I), or any subformula thereof, as a Drug Unit and a Linker Unit (Q) comprising a Releasable Linker (RL) that is other than a Glycoside (e.g., Glucuronide) Unit through which the Ligand Unit is connected to the conjugated auristatin compound. The Linker Unit comprises, in addition to RL, a Stretcher Unit precursor (Z') comprising a functional group for conjugation to a targeting agent that is the precursor to the Ligand Unit and thus is capable of (directly or indirectly) connecting the RL to the Ligand Unit. In some of those embodiments a Parallel Connector Unit (B) is present when it is desired to add a Partitioning Agent (S^{∗}) as a side chain appendage. In any one of those embodiments, a Connector Unit (A) is present when it is desirable to add more distance between the Stretcher Unit and RL.

In one group of embodiments, a Drug-Linker compound comprises an auristatin compound of Formula (I), or any subformula thereof, and a Linker Unit (Q), wherein Q comprises a Releasable Linker (RL) that is a Glycoside (e.g., Glucuronide) Unit, directly attached to a Stretcher Unit precursor (Z') or indirectly to Z' through attachment to intervening component(s) of the Drug-Linker compound's Linker Unit (i.e., A, S^{∗} and/or B(S^{∗})), wherein Z' comprises a functional group capable of forming a covalent bond to a targeting agent.

In another group of embodiments, a Drug-Linker compound comprises an auristatin of Formula (I), or any subformula thereof, and a Linker Unit (Q), wherein Q comprises a Releasable Linker (RL) that is other than a Glycoside (e.g., Glucuronide) Unit (RL), directly attached to a Stretcher Unit precursor (Z') or indirectly to Z' through attachment to intervening component(s) of the Drug-Linker compound's Linker Unit (i.e., A, S^{∗} and/or B(S^{∗})), wherein Z' comprises a functional group capable of forming a covalent bond to a targeting agent.

In some embodiments, the Drug-Linker compound has the formula:

Q-D,

or a salt thereof, wherein
Q is a Linker Unit selected from the group consisting of:
   (i) Z'-A-RL-,
   (ii) Z'-A-RL-Y-,
   (iii) Z'-A-S^{∗}-RL-,
   (iv) Z'-A-S^{∗}-RL-Y-,
   (v) Z'-A-B(S^{∗})-RL-,
   (vi) Z'-A-B(S^{∗})-RL-Y-,
   (vii) Z'-A-,
   (viii) Z'-A-S^{∗}-W-,
   (ix) Z'-A-B(S^{∗})-W-,
   (x) Z'-A-S^{∗}-W-RL-, and
   (xi) Z'-A-B(S^{∗})-W-RL-;
Z' is a Stretcher Unit precursor;
A is a bond or a Connector Unit;
B is a Parallel Connector Unit;
S^{∗} is a Partitioning Agent;
RL is a Releasable Linker;
W is a Amino Acid Unit;
Y is a Spacer Unit; and
D is a Drug Unit of Formula (I'): 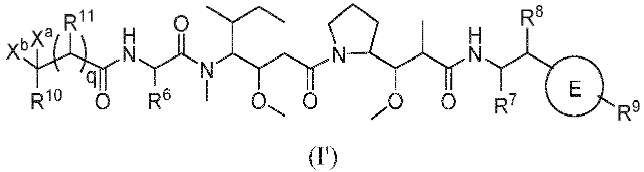 wherein
   X^{b} is -NR²-^{#} or -N⁺R¹R⁵-^{#}, wherein the # denotes the point of attachment to Q, and X^{a} is 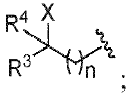 or
   X^{a} and X^{b} are taken together with the carbon atom to which they are attached to form 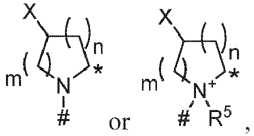 wherein the asterisk denotes the carbon atom of Formula (I) that bears the X^{a} and X^{b} groups, and the # denotes the point of attachment to Q;
   R¹ and R⁵ are independently C₁-C₄ alkyl;
   X is H, OH, -C(O)NR^{a}R^{b}, -S(O)₂R^{a}, -S(O)-R^{a} -S(O)₂NR^{a}R^{b}, -NHS(O)₂R^{a}, or -NHC(O)R^{a};
   R², R³, R⁴, R¹⁰, R^{a}, and R^{b} are each independently H or C₁-C₄ alkyl;
   R⁶ is C₁-C₄ alkyl optionally substituted with OH;
   R⁷ is H, C₁-C₄ alkyl optionally substituted with one or two OH moieties, or 5-6 membered heteroaryl;
   E is phenyl or 5-6 membered heteroaryl;
   R⁸, R⁹, and R¹¹ are each independently H or OH;
   n is 0, 1, 2, or 3;
   m is 1, 2, 3, or 4;
   q is 0 or 1; and
   wherein when X is H, at least two of R⁷, R⁸, R⁹, and R¹¹ comprise an OH moiety.

In the context of the Drug-Linker Compounds - the assembly is best described in terms of its component groups. While some procedures for the preparation of Drug-Linker compounds are described herein, the order of assembly and the general conditions to prepare the compounds will be well understood by one of skill in the art in view of the teachings of the application.

### C. Component groups

### 1. Drug Unit D

The Drug Units of the Drug-Linker compounds, or Ligand-Drug Conjugate thereof, provided herein are auristatin moieities of the compounds disclosed herein and are referred to herein as Drug Units.

In some embodiments, the Drug Unit D has Formula (I'), as described above.

In some embodiments of Formula (I'), q is 0. In some embodiments, q is 1.

In some embodiments, the Drug Unit D has Formula (Iz'): 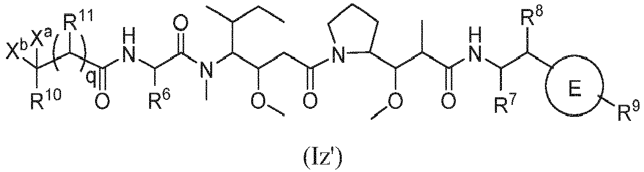 wherein
X^{b} is -NR²-^{#} or -N⁺R¹R⁵-^{#}, wherein the # denotes the point of attachment to Q, and X^{a} is 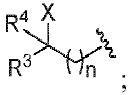 or
X^{a} and X^{b} are taken together with the carbon atom to which they are attached to form 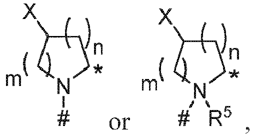 wherein the asterisk denotes the carbon atom of Formula (I) that bears the X^{a} and X^{b} groups, and the # denotes the point of attachment to Q;
R¹ and R⁵ are independently C₁-C₄ alkyl;
X is OH, -C(O)NR^{a}R^{b}, -S(O)₂R^{a}, -S(O)-R^{a} -S(O)₂NR^{a}R^{b}, -NHS(O)₂R^{a}, or - NHC(O)R^{a};
R², R³, R⁴, R¹⁰, R^{a}, and R^{b} are each independently H or C₁-C₄ alkyl;
R⁶ is C₁-C₄ alkyl optionally substituted with OH;
R⁷ is H, C₁-C₄ alkyl optionally substituted with one or two OH moieties, or 5-6 membered heteroaryl;
E is phenyl or 5-6 membered heteroaryl;
R⁸, R⁹, and R¹¹ are each independently H or OH;
n is 0, 1, 2, or 3;
m is 1, 2, 3, or 4; and
q is 0 or 1.

In some embodiments of Formula (I') or (Iz'), q is 0. In some embodiments, q is 1.

In some embodiments, of Formula (I') or (Iz'), X^{b} is -NR²-^{#} or -N⁺R¹R⁵-^{#}, wherein the # denotes the point of attachment to Q, and X^{a} is 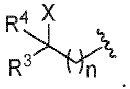 In some embodiments, R¹ and R² are each independently C₁-C₄ alkyl. In some embodiments, R¹ and R⁵ are each independently n-propyl, isopropyl, ethyl, or methyl. In some embodiments, R¹ and R⁵ are each independently ethyl or methyl. In some embodiments, R¹ and R⁵ are methyl. In some emodiments, R³ and R⁴ are each independently C₁-C₄ alkyl. In some embodiments, R³ is H and R⁴ is C₁-C₄ alkyl. In some embodiments, R³ and R⁴ are H. In some embodiments, R³ is H and R⁴ is methyl. In some embodiments, n is 0, 1, 2, or 3. In some embodiments, n is 0, 1, or 2. In some embodiments, n is 0 or 1. In some embodiments, n is 1. In some embodiments, n is 0. In some embodiments, R¹ is methyl, R⁵ is methyl, R³ is H, R⁴ is H, and n is 0. In some embodiments, R² is n-propyl, isopropyl, ethyl or methyl. In some embodiments, R² is ethyl or methyl. In some embodiments, R² is methyl. In some embodiments, R² is H.

In some embodiments of Formula (I') or (Iz'), X^{a} and X^{b} are taken together with the carbon atom to which they are attached to form 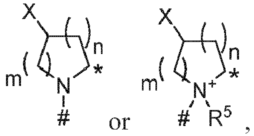 wherein the asterisk denotes the carbon atom of Formula (I) that bears the X^{a} and X^{b} groups, and the # denotes the point of attachment to Q. In some embodiments, R⁵ is n-propyl, isopropyl, ethyl, or methyl. In some embodiments, R⁵ is ethyl or methyl. In some embodiments, R⁵ is ethyl. In some embodiments, R⁵ is methyl. In some embodiments, m is 1, 2, or 3. In some embodiments, m is 1 or 2. In some embodiments, m is 2 or 3. In some embodiments, m is 2. In some embodiments, n is 0, 1, or 2. In some embodiments, n is 0 or 1. In some embodiments, m is 1 or 2 and n is 0 or 1.

In some embodiments of Formula (I'), X is H, OH, -C(O)NR^{a}R^{b}, -S(O)₂R^{a}, -S(O)-R^{a}, -S(O)₂NR^{a}R^{b}, -NHS(O)₂R^{a}, or -NHC(O)R^{a}. In some embodiments, X is OH, -S(O)₂R^{a}, -S(O)-R^{a}, -S(O)₂NR^{a}R^{b}, -NHS(O)₂R^{a}, or -NHC(O)R^{a}. In some embodiments, X is H or OH. In some embodiments, X is OH. In some embodiments, X is OH, -NHS(O)₂R^{a}, or -NHC(O)R^{a}. In some embodiments, X is OH, -S(O)₂R^{a}, -S(O)-R^{a}, or -S(O)₂NR^{a}R^{b}. In some embodiments, X is -NHS(O)₂R^{a}, or -NHC(O)R^{a}. In some embodiments, X is -S(O)₂R^{a}, -S(O)-R^{a}, or -S(O)₂NR^{a}R^{b}. In some embodiments, X is H, OH, -NHS(O)₂R^{a}, or -NHC(O)R^{a}. In some embodiments, X is H, OH, -S(O)₂R^{a}, -S(O)-R^{a}, or -S(O)₂NR^{a}R^{b}. In some embodiments, X is H, -NHS(O)₂R^{a}, or -NHC(O)R^{a}. In some embodiments, X is H, -S(O)₂R^{a}, -S(O)-R^{a}, or -S(O)₂NR^{a}R^{b}. In some embodiments, X is H, OH, or -C(O)NR^{a}R^{b}. In some embodiments, X is OH or -C(O)NR^{a}R^{b}. In some embodiments, X is H or -C(O)NR^{a}R^{b}. In some embodiments, R^{a} and R^{b} are independently H, n-propyl, isopropyl, ethyl, or methyl. In some embodiments, R^{a} and R^{b} are independently H, ethyl, or methyl. In some embodiments, R^{a} and R^{b} are independently H or methyl. In some embodiments, R^{a} is H and R^{b} is n-propyl, isopropyl, ethyl, or methyl. In some embodiments, R^{a} is H and R^{b} is methyl.

In some embodiments of Formula (Iz'), X is OH, -C(O)NR^{a}R^{b}, -S(O)₂R^{a}, -S(O)-R^{a}, -S(O)₂NR^{a}R^{b}, -NHS(O)₂R^{a}, or -NHC(O)R^{a}. In some embodiments, X is OH. In some embodiments, X is OH, -NHS(O)₂R^{a}, or -NHC(O)R^{a}. In some embodiments, X is OH, -S(O)₂R^{a}, -S(O)-R^{a}, or -S(O)₂NR^{a}R^{b}. In some embodiments, X is -NHS(O)₂R^{a}, or -NHC(O)R^{a}. In some embodiments, X is -S(O)₂R^{a}, -S(O)-R^{a}, or -S(O)₂NR^{a}R^{b}. In some embodiments, X is OH or -C(O)NR^{a}R^{b}. In some embodiments, X is -C(O)NR^{a}R^{b}. In some embodiments, R^{a} and R^{b} are independently H, n-propyl, isopropyl, ethyl, or methyl. In some embodiments, R^{a} and R^{b} are independently H, ethyl, or methyl. In some embodiments, R^{a} and R^{b} are independently H or methyl. In some embodiments, R^{a} is H and R^{b} is n-propyl, isopropyl, ethyl, or methyl. In some embodiments, R^{a} is H and R^{b} is methyl.

In some embodiments of Formula (I') or (Iz'), X^{b} is-N⁺R¹R⁵-^{#} and X^{a} is 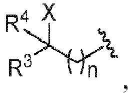 X is OH, R¹ is methyl or methyl, R⁵ is methyl, R³ is H, R⁴ is H, and n is 0. In some embodiments, X is OH, R¹ is methyl, R⁵ is methyl, R³ is H, R⁴ is H, and n is 0. In some embodiments, X is OH, R³ and R⁴ are H, and n is 0.

In some embodiments of Formula (I') or (Iz'), X^{b} is -NR²-^{#}. X^{a} is 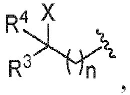 X is OH, and R² is H or C₁-C₄ alkyl. In some embodiments, X^{b} is -NR²-^{#} and X^{a} is 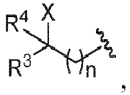 X is OH, and R² is H. In some embodiments, X^{b} is -NR²-^{#} and X^{a} is 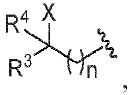 X is OH, and R² is C₁-C₄ alkyl.

In some embodiments or Formula (I') or (Iz'), R⁶ is n-propyl, isopropyl, ethyl, or methyl. In some embodiments, R⁶ is ethyl or methyl. In some embodiments, R⁶ is isopropyl. In some embodiments, R⁶ is n-propyl, isopropyl, ethyl, or methyl, each of which is substituted with OH. In some embodiments, R⁶ is isopropyl substituted with OH. In some embodiments, R⁶ is ethyl substituted with OH. In some embodiments, R⁶ is methyl substituted with OH.

In some embodiments of Formula (I') or (Iz'), R⁷ is C₁-C₄ alkyl substituted with OH. In some embodiments, R⁷ is C₁-C₄ alkyl substituted by two OH moieties. In some embodiments, R⁷ is n-propyl, isopropyl, ethyl, or methyl, each of which is substituted with OH. In some embodiments, R⁷is C₁-C₄ alkyl. In some embodiments, R⁷ is n-propyl, isopropyl, ethyl, or methyl. In some embodiments, R⁷ is methyl. In some embodiments, R⁷ is -CH₂CH₂OH or CH₂OH. In some embodiments, R⁷ is -CH₂OH. In some embodiments, R⁷ is 5-6 membered heteroaryl. In some embodiments, R⁷ is 5-membered heteroaryl. In some embodiments, R⁷ is thiazolyl.

In some embodiments of Formula (I') or (Iz'), R⁸, R⁹, and R¹¹ are H. In some embodiments, R⁸ is OH, R⁹ is H, and R¹¹ is H. In some embodiments, R⁸ and R⁹ are each OH and R¹¹ is H. In some embodiments, R⁸ is OH, R⁹ is H, and R¹¹ is OH. In some embodiments, R⁸ is H, R⁹ is H, and R¹¹ is OH.

In some embodiments of Formula (I') or (Iz'), E is an optionally substituted 6-membered ring. In some embodiments, E is pyridine or phenyl, each of which is optionally substituted. In some embodiments, E is optionally substituted phenyl. In some embodiments, E is optionally substituted 5-6 membered heteroaryl. In some embodiments, E is optionally substituted 5-membered heteroaryl. In some embodiments, E is pyridine or phenyl, each of which is unsubstituted. In some embodiments, E is unsubstituted phenyl. In some embodiments, E is unsubstituted 5-6 membered heteroaryl. In some embodiments, E is unsubstituted 5-membered heteroaryl.

In some embodiments of Formula (I') or (Iz'), R⁷ is -CH₂OH, R⁸ is H, R⁹ is H, and E is phenyl.

In some embodiments, the Drug Unit D has Formula (Ia') or (Ia"): 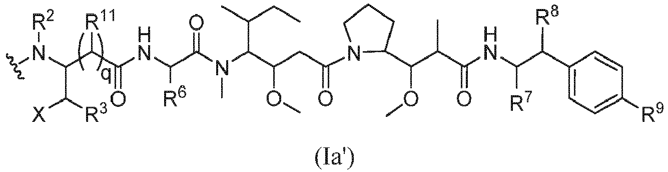 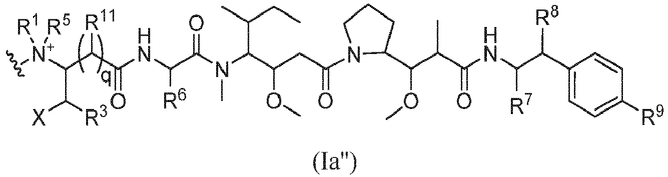 or a salt thereof, wherein
R¹ and R⁵ are independently C₁-C₄ alkyl;
X is H, OH, -C(O)NR^{a}R^{b}, -S(O)₂R^{a}, -S(O)-R^{a} -S(O)₂NR^{a}R^{b}, -NHS(O)₂R^{a}, or -NHC(O)R^{a}; R², R³, R^{a}, and R^{b} are each independently H or C₁-C₄ alkyl;
R⁶ is C₁-C₄ alkyl;
R⁷ is H, C₁-C₄ alkyl optionally substituted with OH, or 5-6 membered heteroaryl;
R⁸, R⁹, and R¹¹ are each independently H or OH;
q is 0 or 1; and
the wavy line is the site of attachment to the rest of the Drug-Linker compound.

In some embodiments of Formula (Ia') and (Ia"), q is 0. In some embodiments, q is 1.

In some embodiments of Formula (Ia'), R² is C₁-C₄-alkyl. In some embodiments, R² is n-propyl, isopropyl, ethyl, or methyl. R² is ethyl or methyl. R² is methyl. In some embodiments, R² is H. In some embodiments, R² is ethyl, methyl, or H. In some embodiments, R² is methyl or H.

In some embodiments of Formula (Ia"), R¹ and R⁵ are each independently n-propyl, isopropyl, ethyl, or methyl. In some embodiments, R¹ and R⁵ are each independently ethyl or methyl. In some embodiments, R¹ and R⁵ are each methyl. In some embodiments, R¹ is methyl and R⁵ is n-propyl, isopropyl, ethyl, or methyl. In some embodiments, R¹ is methyl and R⁵ is ethyl or methyl. In some embodiments, R¹ and R⁵ are methyl.

In some embodiments of Formula (Ia') and (Ia"), X is H, OH, -C(O)NR^{a}R^{b}, -S(O)₂R^{a}, -S(O)-R^{a}, -S(O)₂NR^{a}R^{b}, -NHS(O)₂R^{a}, or -NHC(O)R^{a}. In some embodiments, X is OH, -S(O)₂R^{a}, -S(O)-R^{a}, -S(O)₂NR^{a}R^{b}, -NHS(O)₂R^{a}, or -NHC(O)R^{a}. In some embodiments, X is H or OH. In some embodiments, X is OH. In some embodiments, X is OH, -NHS(O)₂R^{a}, or -NHC(O)R^{a}. In some embodiments, X is OH, -S(O)₂R^{a}, -S(O)-R^{a}, or -S(O)₂NR^{a}R^{b}. In some embodiments, X is -NHS(O)₂R^{a}, or -NHC(O)R^{a}. In some embodiments, X is -S(O)₂R^{a}, -S(O)-R^{a}, or -S(O)₂NR^{a}R^{b}. In some embodiments, X is H, OH, -NHS(O)₂R^{a}, or -NHC(O)R^{a}. In some embodiments, X is H, OH, -S(O)₂R^{a}, -S(O)-R^{a}, or -S(O)₂NR^{a}R^{b}. In some embodiments, X is H, -NHS(O)₂R^{a}, or -NHC(O)R^{a}. In some embodiments, X is H, -S(O)₂R^{a}, -S(O)-R^{a}, or -S(O)₂NR^{a}R^{b}. In some embodiments, X is H, OH, or -C(O)NR^{a}R^{b}. In some embodiments, X is OH or -C(O)NR^{a}R^{b}. In some embodiments, X is H or -C(O)NR^{a}R^{b}. In some embodiments, R^{a} and R^{b} are independently H, n-propyl, isopropyl, ethyl, or methyl. In some embodiments, R^{a} and R^{b} are independently H, ethyl, or methyl. In some embodiments, R^{a} and R^{b} are independently H or methyl. In some embodiments, R^{a} is H and R^{b} is n-propyl, isopropyl, ethyl, or methyl. In some embodiments, R^{a} is H and R^{b} is methyl.

In some embodiments of Formula (Ia') and (Ia"), R³ is H or methyl. In some embodiments, R³ is H. In some embodiments, R³ is methyl.

In some embodiments of Formula (Ia') and (Ia"), R⁶ is n-propyl, isopropyl, ethyl, or methyl. In some embodiments, R⁶ is ethyl or methyl. In some embodiments, R⁶ is isopropyl. In some embodiments, R⁶ is n-propyl, isopropyl, ethyl, or methyl, each of which is substituted with OH. In some embodiments, R⁶ is isopropyl substituted with OH. In some embodiments, R⁶ is ethyl substituted with OH. In some embodiments, R⁶ is methyl substituted with OH.

In some embodiments of Formula (Ia') and (Ia"), R⁷ is C₁-C₄ alkyl substituted with OH. In some embodiments, R⁷ is n-propyl, isopropyl, ethyl, or methyl, each of which is substituted with OH. In some embodiments, R⁷ is -CH₂CH₂OH or CH₂OH. In some embodiments, R⁷ is -CH₂OH. In some embodiments, R⁷ is C₁-C₄ alkyl. In some embodiments, R⁷ is n-propyl, isopropyl, ethyl, or methyl. In some embodiments, R⁷ is methyl. In some embodiments, R⁷ is 5-6 membered heteroaryl. In some embodiments, R⁷ is 5-membered heteroaryl. In some embodiments, R⁷ is thiazolyl.

In some embodiments of Formula (Ia') and (Ia"), R⁸, R⁹, and R¹¹ are H. In some embodiments, R⁸ is OH, R⁹ is H, and R¹¹ is H. In some embodiments, R⁸ and R⁹ are each OH and R¹¹ is H. In some embodiments, R⁸ is OH, R⁹ is H, and R¹¹ is OH. In some embodiments, R⁸ is H, R⁹ is H, and R¹¹ is OH.

In some embodiments of Formula (Ia'), X is OH, R³ is H or methyl, R² is C₁-C₄ alkyl, R⁶ is C₁-C₄ alkyl, R⁷ is C₁-C₄ alkyl optionally substituted with OH, R⁸ is H or C₁-C₄ alkyl, and R⁹ is H. In some embodiments, q is 0, X is OH, R³ is H or methyl, R² is C₁-C₄ alkyl, R⁶ is C₁-C₄ alkyl, R⁷ is C₁-C₄ alkyl optionally substituted with OH, R⁸ is H or C₁-C₄ alkyl, and R⁹ is H.

In some embodiments of Formula (Ia"), X is OH, R³ is H or methyl, R¹ C₁-C₄ alkyl, R⁵ is C₁-C₄ alkyl, R⁶ is C₁-C₄ alkyl, R⁷ is C₁-C₄ alkyl optionally substituted with OH, R⁸ is H or C₁-C₄ alkyl, and R⁹ is H. In some embodiments, q is 0, X is OH, R³ is H or methyl, R¹ C₁-C₄ alkyl, R⁵ is C₁-C₄ alkyl, R⁶ is C₁-C₄ alkyl, R⁷ is C₁-C₄ alkyl optionally substituted with OH, R⁸ is H or C₁-C₄ alkyl, and R⁹ is H.

In some embodiments, of the Drug Unit D has Formula (Ib') or (Ib"): 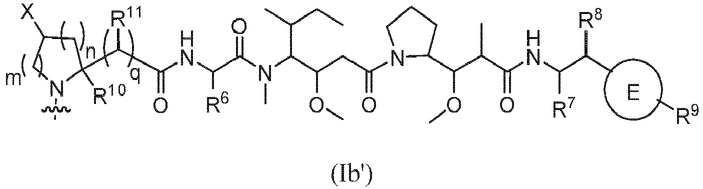 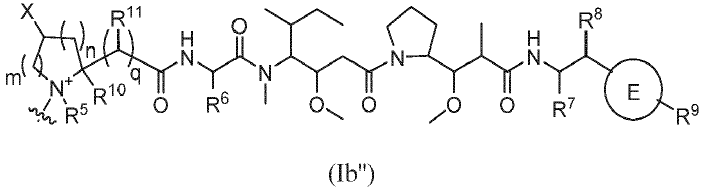 wherein the variables are as defined for Formula (Ib) and the wavy line is the site of attachment to the rest of the Drug-Linker compound.

In some embodiments of Formula (Ib') and (Ib"), R⁷ is -CH₂OH, R⁸ is H, and R⁹ is H.

In some embodiments, the Drug Unit D has Formula (Ic') or (Ic"): 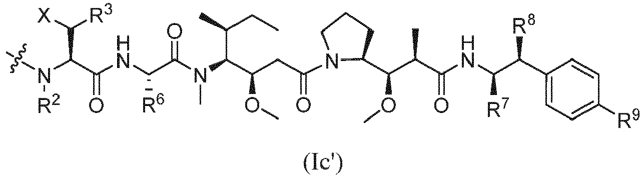 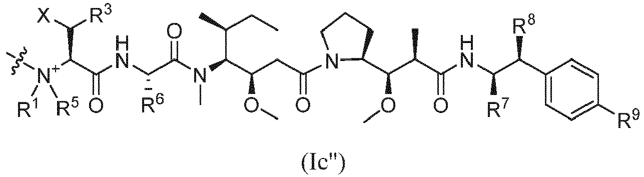 wherein the variables are as defined for Formula (Ic) and the wavy line is the site of attachment to the rest of the Drug-Linker compound.

In some embodiments of Formula (Ic') or (Ic"), X is OH and R³ is H. In some embodiments, R⁷ is -CH₂OH, R⁸ is H, and R⁹ is H. In some embodiments, X is OH, R⁷ is -CH₂OH, R⁸ is H, and R⁹ is H. In some embodiments, X is OH, R³ is H, R⁷ is -CH₂OH, R⁸ is H, and R⁹ is H.

In some embodiments, the Drug Unit D has the Formula (Id') or (Id"): 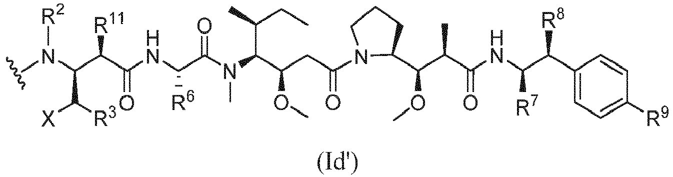 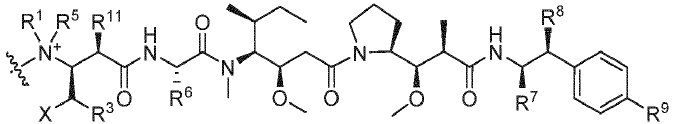 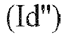 wherein the variables are as defined for Formula (Id) and the wavy line is the site of attachement to the rest of the Drug-Linker compound.

In some embodiments, the Drug Unit D has Formula (Ie') or (Ie"): 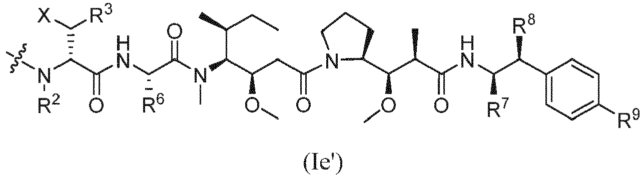 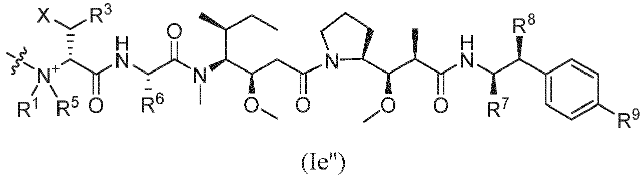 wherein the variables are as defined for Formula (Ie) and the wavy line is the site of attachment to the rest of the Drug-Linker compound.

In some embodiments, the Drug Unit D has the Formula (Id') or (Id"): 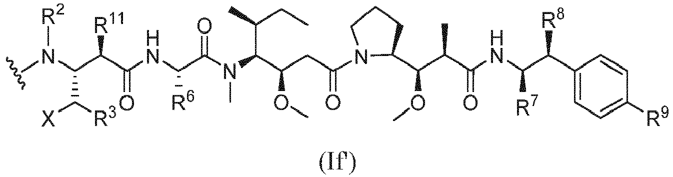 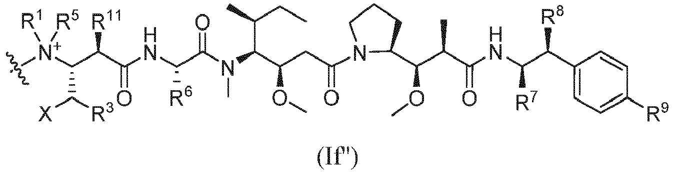 wherein the variables are as defined for Formula (If) and the wavy line is the site of attachement to the rest of the Drug-Linker compound.

In some embodiments of Formula (Id'), (Id"), (If), or (If), X is OH and R³ is H. In some embodiments, R⁷ is -CH₂OH, R⁸ is H, and R⁹ is H. In some embodiments, X is OH, R⁷ is -CH₂OH, R⁸ is H, and R⁹ is H. In some embodiments, X is OH, R³ is H, R⁷ is -CH₂OH, R⁸ is H, and R⁹ is H.

In some embodiments of Formula (Ia"), (Ic"), (Id"), (Ie"), or (If") the quaternized nitrogen atom prevents cyclization of the Drug Unit. In some embodiments, the quaternized nitrogen atom prevents premature release of the Drug Unit from the Drug-Linker compound or moiety (*e.g.,* in a Ligand-Drug Conjugate compound). In some embodiments, the quaternized nitrogen atom prevents cyclization and premature release of the Drug Unit from the Drug-Linker compound or moiety (e.g., in a Ligand-Drug Conjugate compound).

### 2. Linker Unit Q

As noted above, is some embodiments, the Linker Unit Q has a formula selected from the group consisting of:
(i) Z'-A-RL-,
(ii) Z'-A-RL-Y-,
(iii) Z'-A-S^{∗}-RL-,
(iv) Z'-A-S^{∗}-RL-Y-,
(v) Z'-A-B(S^{∗})-RL-,
(vi) Z'-A-B(S^{∗})-RL-Y-,
(vii) Z'-A-,
(viii) Z'-A-S^{∗}-W-,
(ix) Z'-A-B(S^{∗})-W-,
(x) Z'-A-S^{∗}-W-RL-, and
(xi) Z'-A-B(S^{∗})-W-RL-;
wherein Z' is a Stretcher Unit; A is a bond or a Connector Unit; B is a Parallel Connector Unit; S^{∗} is a Partitioning Agent; RL is Releasable Linker; W is an Amino Acid Unit; and Y is a Spacer Unit.

In other embodiments, the Linker Unit Q has a formula selected from the group consisting of:
(i) Z'-A-RL-,
(ii) Z'-A-RL-Y-,
(iii) Z'-A-S^{∗}-RL-,
(iv) Z'-A-S^{∗}-RL-Y-,
(x) Z'-A-S^{∗}-W-RL-, and
(xi) Z'-A-B(S^{∗})-W-RL-.

In some embodiments, the Linker Unit Q has a formula selected from the group consisting of:
(v) Z'-A-B(S^{∗})-RL-,
(vi) Z'-A-B(S^{∗})-RL-Y-,
(ix) Z'-A-B(S^{∗})-W-, and
(xi) Z'-A-B(S^{∗})-W-RL-.

In some embodiments, the Linker Unit Q has a formula selected from the group consisting of:
(iii) Z'-A-S^{∗}-RL-,
(iv) Z'-A-S^{∗}-RL-Y-,
(v) Z'-A-B(S^{∗})-RL-,
(vi) Z'-A-B(S^{∗})-RL-Y-,
(viii) Z'-A-S^{∗}-W-,
(ix) Z'-A-B(S^{∗})-W-,
(x) Z'-A-S^{∗}-W-RL-, and
(xi) Z'-A-B(S^{∗})-W-RL-.

In some embodiments, the Linker Unit Q has a formula selected from the group consisting of:
(viii) Z'-A-S^{∗}-W-,
(ix) Z'-A-B(S^{∗})-W-,
(x) Z'-A-S^{∗}-W-RL-, and
(xi) Z'-A-B(S^{∗})-W-RL-.

### 3. Stretcher Unit Z'

A Stretcher Unit (Z) is a component of a Ligand-Drug Conjugate that acts to connect the Ligand Unit to the remainder of the conjugate. A Stretcher Unit precursor (Z') is a component of a Drug-Linker compound or intermediate thereof has a functional group that can form a bond with a functional group of a targeting ligand to form a Stretcher Unit (Z).

In some embodiments, a Stretcher Unit precursor (Z') has an electrophilic group that is capable of interacting with a reactive nucleophilic group present on a Ligand Unit (e.g., an antibody) to provide a covalent bond between a Ligand Unit and the Stretcher Unit of a Linker Unit. Nucleophilic groups on an antibody having that capability include but are not limited to, sulfhydryl, hydroxyl and amino functional groups. In some aspects, the heteroatom of the nucleophilic group of an antibody is reactive to an electrophilic group on a Stretcher Unit precursor and can provide a covalent bond between the Ligand Unit and Stretcher Unit of a Linker Unit or Drug-Linker moiety. Useful electrophilic groups for that purpose include, but are not limited to, maleimide, haloacetamide groups, and NHS esters. The electrophilic group provides a convenient site for antibody attachment to form a Ligand-Drug Conjugate compound or Ligand Unit-Linker intermediate compound.

In other embodiments, a Stretcher Unit precursor has a reactive site which has a nucleophilic group that is reactive to an electrophilic group present on a Ligand Unit (e.g., an antibody). Useful electrophilic groups on an antibody for that purpose include, but are not limited to, aldehyde and ketone carbonyl groups. The heteroatom of a nucleophilic group of a Stretcher Unit precursor can react with an electrophilic group on an antibody and form a covalent bond to the antibody. Useful nucleophilic groups on a Stretcher Unit precursor for that purpose include, but are not limited to, hydrazide, hydroxylamine, amino, hydrazine, thiosemicarbazone, hydrazine carboxylate, and arylhydrazide. The electrophilic group on an antibody provides a convenient site for antibody attachment to form a Ligand-Drug Conjugate compound or Ligand Unit-Linker intermediate compound.

In some embodiments, a sulfur atom of a Ligand Unit is bound to a succinimide ring system of a Stretcher Unit formed by reaction of a thiol functional group of a targeting ligand with a maleimide moiety of the corresponding Stretcher Unit precursor. In other embodiments, a thiol functional group of a Ligand Unit reacts with an alpha haloacetamide moiety to provide a sulfur-bonded Stretcher Unit by nucleophilic displacement of its halogen substituent.

Illustrative Stretcher Units prior to conjugation to the Ligand Unit (i.e., Stretcher Unit precursors) comprise a maleimide moiety and are represented by structures including that of formula Z'a 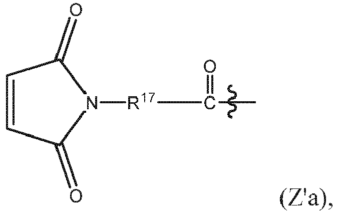 wherein the wavy line adjacent the carbonyl carbon atom indicates attachment to B, A, or S^{∗}, in the formulae above, depending on the presence or absence of A and/or B, R¹⁷ is -(CH₂)₁₋₅- or -CH₂CH₂(OCH₂CH₂)₁₋₃₆-. In some embodiments, R¹⁷ is -CH₂-, -(CH₂)₂-, -(CH₂)₃-, -(CH₂)₄-, -(CH₂)₅-, -CH₂CH₂OCH₂CH₂-, -CH₂CH₂(OCH₂CH₂)₂-, -CH₂CH₂(OCH₂CH₂)₃-, -CH₂CH₂(OCH₂CH₂)₄-, -CH₂CH₂(OCH₂CH₂)₅-, -CH₂CH₂(OCH₂CH₂)₆-, -CH₂CH₂(OCH₂CH₂)₇-, -CH₂CH₂(OCH₂CH₂)₈-, -CH₂CH₂(OCH₂CH₂)₁₀-, -CH₂CH₂(OCH₂CH₂)₁₂-, -CH₂CH₂(OCH₂CH₂)₁₄-, -CH₂CH₂(OCH₂CH₂)₁₆-, -CH₂CH₂(OCH₂CH₂)₁₈-, -CH₂CH₂(OCH₂CH₂)₂₀-, -CH₂CH₂(OCH₂CH₂)₂₄- -CH₂CH₂(OCH₂CH₂)₂₈-, -CH₂CH₂(OCH₂CH₂)₃₂-, or -CH₂CH₂(OCH₂CH₂)₃₆-.

Other illustrative Stretcher Units prior to conjugation to the Ligand Unit (i.e., Stretcher Unit precursors) comprises a maleimide moiety and are represented by structures including that of formula Z'a-BU 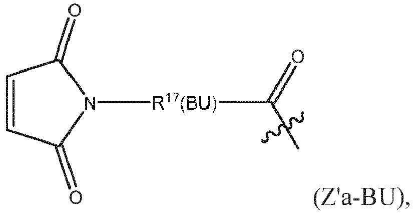 wherein the wavy line adjacent the carbonyl carbon atom indicates attachment to B, A, or S^{∗}, in the formulae above, depending on the presence or absence of A and/or B, R¹⁷ is R¹⁷ is -(CH₂)₁₋₅- or -CH₂CH₂(OCH₂CH₂)₁₋₅-, substituted with a Basic Unit (BU), such as an optionally substituted aminoalkyl, e.g., -(CH₂ )ₓNH₂, -(CH2 )ₓNHR^{a}, and -(CH₂ )ₓN(R^{a}) ₂, wherein subscript x is an integer of from 1-4, preferably R¹⁷ is -CH₂- or -CH₂CH₂- and subscript x is 1 or 2, and each R^{a} is independently selected from the group consisting of C₁₋₆ alkyl and C₁₋₆ haloalkyl, or two R^{a} groups are combined with the nitrogen to which they are attached to form an azetidinyl, pyrrolidinyl or piperidinyl group.

In some embodiments of formula Z'a, a Stretcher Unit precursor (Z') is represented by one of the following structures: 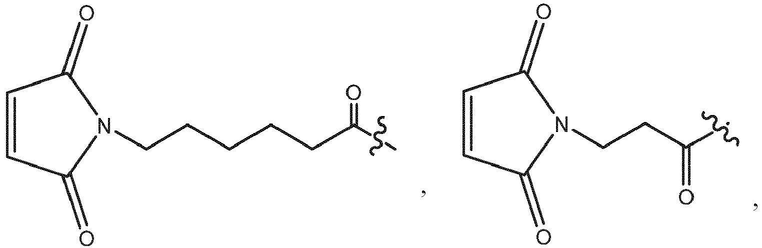 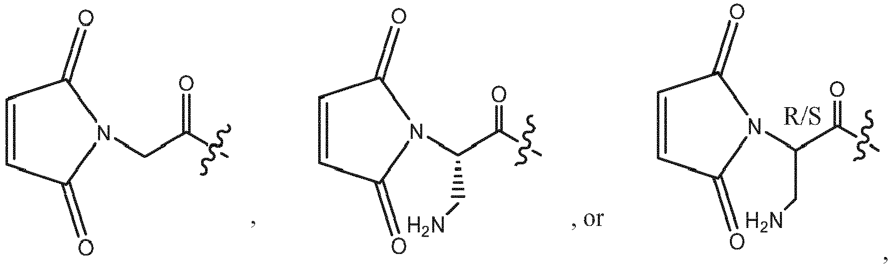 wherein the wavy line adjacent to the carbonyl is as defined for Z'a or Z'a-BU.

In other embodiments the Stretcher unit precursor (Z') comprises a maleimide moiety and is represented by the structure of: 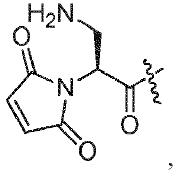 wherein the wavy line adjacent to the carbonyl is as defined for Z'a and the amino group is optional protonated or protected by an amino protecting group.

In Stretcher Units having a BU moiety, it will be understood that the amino functional group of that moiety is typically protected by an amino protecting group during synthesis, e.g., an acid labile protecting group (e.g., BOC).

Illustrative Stretcher Unit precursors covalently attached to a Connector Unit that comprise the structure of Z'a or Z'a-BU in which -R¹⁷- or -R¹⁷(BU)- is -CH₂-, -CH₂CH₂- or -CH(CH₂NH₂)- have the following structures: 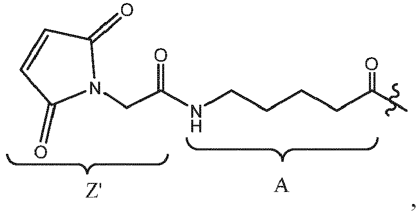 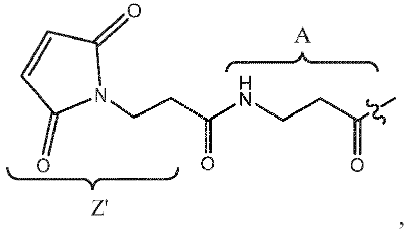 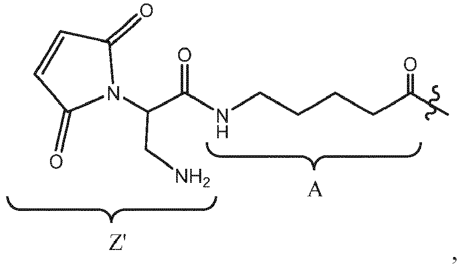 wherein the wavy line adjacent to the carbonyl is as defined for Z'a or Z'a-BU.

Other Stretcher Unit precursors bonded a Connector Unit (A) have the structures above wherein A in any one of the above Z'-A- and Z'(BU)-A- structures is replaced by a Parallel Connector Unit and Partitioning Agent (-B(S^{∗})-) having the structure of 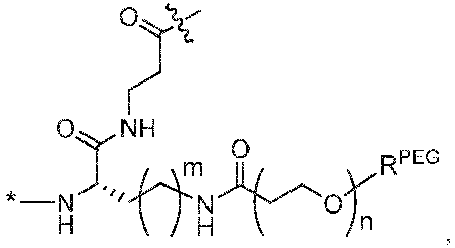 wherein subscript m ranges from 1 to 6; n ranges from 8 to 24; R^{PEG} is a PEG Capping Unit, preferably H, -CH₃, or -CH₂CH₂CO₂H, the asterisk (^{∗}) indicates covalent attachment to the Stretcher Unit precursor corresponding in structure to formula Z'a and the wavy line indicates covalent attachment to RL. In instances such as those shown here, the shown PEG group is meant to be exemplary of a variety of Partitioning Agents including PEG groups of different lengths and other Partitioning Agents that are directly attached or modified for attachment to the Parallel Connector Unit.

In some aspects of the prevent invention the Stretcher Unit has a mass of no more than about 1000 daltons, no more than about 500 daltons, no more than about 200 daltons, from about 30, 50 or 100 daltons to about 1000 daltons, from about 30, 50 or 100 daltons to about 500 daltons, or from about 30, 50 or 100 daltons to about 200 daltons.

### Connector Unit (A)

In some embodiments, a Connector Unit (A), is included in a Drug-Linker Compound in instances where it is desirable to add additional distance between the Stretcher Unit precursor (Z') and the Releasable Linker. In some embodiments, the extra distance will aid with activation within RL. Accordingly, the Connector Unit (A), when present, extends the framework of the Linker Unit. In that regard, a Connector Unit (A) is covalently bonded with the Stretcher Unit (or its precursor) at one terminus and is covalently bonded to the optional Parallel Connector Unit or the Partitioning Agent (S^{∗}) at its other terminus.

The skilled artisan will appreciate that the Connector Unit is any group that serves to provide for attachment of the Releasable Linker to the remainder of the Linker Unit (Q). The Connector Unit can, for example, comprise one or more (e.g., 1-10, preferably, 1, 2, 3, or 4) proteinogenic or non-proteinogenic amino acid, amino alcohol, amino aldehyde, diamino residues. In some embodiments, the Connector Unit is a single proteinogenic or non-proteinogenic amino acid, amino alcohol, amino aldehyde, or diamino residue. An exemplary amino acid capable of acting as Connector units is β-alanine.

In some of those embodiments, the Connector Unit has the formula denoted below: 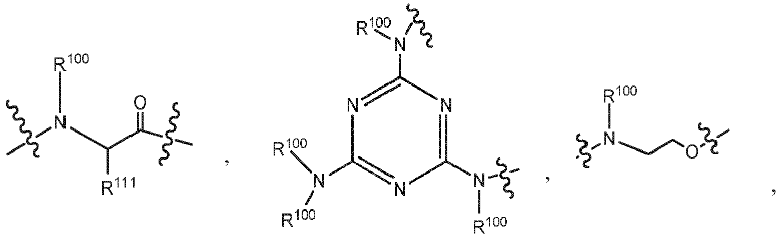 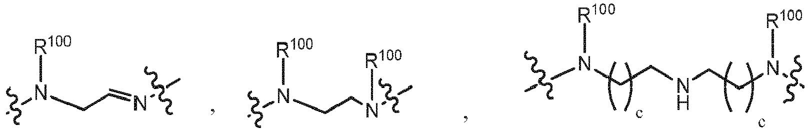 or 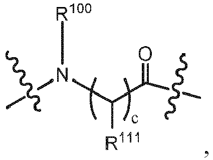 wherein the wavy lines indicate attachment of the Connector Unit within the Drug-Linker Compound; and wherein R¹¹¹ is independently selected from the group consisting of hydrogen, *p*-hydroxybenzyl, methyl, isopropyl, isobutyl, *sec*-butyl, -CH₂OH, -CH(OH)CH₃, - CH₂CH₂SCH₃, -CH₂CONH₂, -CH₂COOH, -CH₂CH₂CONH₂, -CH₂CH₂COOH, - (CH₂)₃NHC(=NH)NH₂, -(CH₂)₃NH₂, -(CH₂)₃NHCOCH₃, -(CH₂)₃NHCHO, - (CH₂)₄NHC(=NH)NH₂, -(CH₂)₄NH₂, -(CH₂)₄NHCOCH₃, -(CH₂)₄NHCHO, - (CH₂)₃NHCONH₂, -(CH₂)₄NHCONH₂, -CH₂CH₂CH(OH)CH₂NH₂, 2-pyridylmethyl-, 3-pyridylmethyl-, 4-pyridylmethyl-, 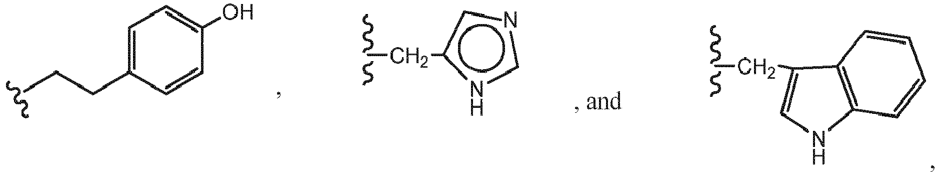 and each R¹⁰⁰ is independently selected from hydrogen or -C₁-C₃ alkyl, preferably hydrogen or CH₃; and subscript c is an independently selected integer from 1 to 10, preferably 1 to 3.

A representative Connector Unit having a carbonyl group for attachment to the Partitioning Agent (S^{∗}) or to -B(S^{∗})- is as follows: 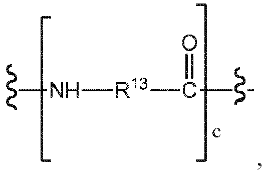 wherein in each instance R¹³ is independently selected from the group consisting of -CH₂CH₂(OCH₂CH₂)ₖ-, -C₁-C₆ alkylene-, -C₃-C₈carbocyclo-, -arylene-, -C₁-C₁₀ heteroalkylene-, -C₃-C₈heterocyclo-, -C₁-C₁₀alkylene-arylene-, -arylene-Ci-Cioalkylene-, -C₁-C₁₀alkylene-(C₃-C₈carbocyclo)-, -(C₃-C₈carbocyclo)-C₁-C₁₀alkylene-, -C₁-C₁₀alkylene-(C₃-C₈ heterocyclo)-, and -(C₃-C₈ heterocyclo)-Ci-Cio alkylene-, wherein subscript k is an integer ranging from 1 to 36 and the subscript c is an integer ranging from 1 to 4. In some embodiments R¹³ is -C₁-C₆ alkylene and c is 1.

Another representative Connector Unit having a carbonyl group for attachment to Partitioning Agent (S^{∗}) or to -B(S^{∗})- is as follows: 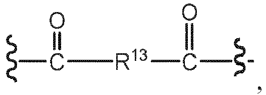 wherein R¹³ is -CH₂CH₂(OCH₂CH₂)ₖ-, -C₁-C₆ alkylene-, -C₃-C₈carbocyclo-, -arylene-, -C₁-C₁₀ heteroalkylene-, -C₃-C₈heterocyclo-, -C₁-C₁₀alkylene-arylene-, -arylene-Ci-Cioalkylene-, -C₁-C₁₀alkylene-(C₃-C₈carbocyclo)-, -(C₃-C₈carbocyclo)-C₁-C₁₀alkylene-, -C₁-C₁₀alkylene-(C₃-C₈ heterocyclo)-, or -(C₃-C₈ heterocyclo)-Ci-Cio alkylene-, wherein subscript k is an integer ranging from 1 to 36. In some embodiments R¹³ is -C₁-C₆ alkylene.

A representative Connector Unit having a NH moiety that attaches to Partitioning Agent (S^{∗}) or to -B(S^{∗})- is as follows: 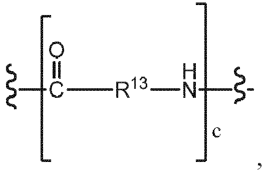 wherein in each instance, R¹³ is independently selected from the group consisting of -CH₂CH₂(OCH₂CH₂)ₖ-, -C₁-C₆ alkylene-, -C₃-C₈carbocyclo-, -arylene-, -C₁-C₁₀ heteroalkylene-, -C₃-C₈heterocyclo-, -C₁-C₁₀alkylene-arylene-, -arylene-Ci-Cioalkylene-, -C₁-C₁₀alkylene-(C₃-C₈carbocyclo)-, -(C₃-C₈carbocyclo)-C₁-C₁₀alkylene-, -C₁-C₁₀alkylene-(C₃-C₈ heterocyclo)-, and -(C₃-C₈ heterocyclo)-C₁-C₁₀ alkylene-, wherein subscript k is an integer ranging from 1 to 36 and subscript c is an integer ranging from 1 to 36. In some embodiments R¹³ is -C₁-C₆ alkylene and subscript c is 1.

Another representative Connector Unit having a NH moiety that attaches to Partitioning Agent (S^{∗}) or to -B(S^{∗})- is as follows: 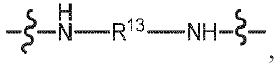 wherein R¹³ is -CH₂CH₂(OCH₂CH2)ₖ-, -C₁-C₆ alkylene-, -C₃-C₈carbocyclo-, -arylene-, -C₁-C₁₀ heteroalkylene-, -C₃-C₈heterocyclo-, -C₁-C₁₀alkylene-arylene-, -arylene-C₁-C₁₀alkylene-, -C₁-C₁₀alkylene-(C₃-C₈carbocyclo)-, -(C₃-C₈carbocyclo)-C₁-C₁₀alkylene-, -C₁-C₁₀alkylene-(C₃-C₈ heterocyclo)-, -(C₃-C₈ heterocyclo)-Ci-Cio alkylene-, -C(=O)C₁-C₆ alkylene- or -C₁-C₆ alkylene-C(=O)-C₁-C₆ alkylene, wherein subscript k is an integer ranging from 1 to 36.

Selected embodiments of Connector Units include those having the following structure of: 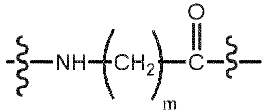 or 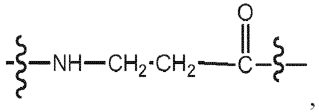 wherein the wavy line adjacent to the nitrogen indicates covalent attachment a Stretcher Unit (Z) (or its precursor Z'), and the wavy line adjacent to the carbonyl indicates covalent attachment to Partitioning Agent (S^{∗}) or to -B(S^{∗})-; and m is an integer ranging from 1 to 6, preferably 2 to 6, more preferably 2 to 4.

### Releasable Linker (RL)

The Releasable Linker (RL) is capable of linking to the Spacer Unit (Y) or the Drug Unit (D). RL comprises a cleavable bond (i.e., a reactive site) that upon action by an enzyme present within a hyper-proliferating cell or hyper-activated immune cells or characteristic of the immediate environment of these abnormal or unwanted cells, or upon non-enzymatic action due to conditions more likely experienced by hyper-proliferating cells in comparison to normal cells, releases free drug. Alternatively, RL comprises a cleavable bond that is more likely acted upon intracellularly in a hyper-proliferating cell or hyper-activated immune cell due to preferential entry into such cells in comparison to normal cells.

### Peptide Releasable Linkers

In some embodiments, the Releasable Linker is a Peptide Releasable Linker. In some embodiments, the Peptide Releasable Linker (RL) will comprise one or more contiguous or non-contiguous sequences of amino acids (e.g., so that RL has 1 to no more than 12 amino acids). The Peptide Releasable Linker can comprise or consist of, for example, an amino acid, a dipeptide, tripeptide, tetrapeptide, pentapeptide, hexapeptide, heptapeptide, octapeptide, nonapeptide, decapeptide, undecapeptide or dodecapeptide unit. In some aspects, in the presence of an enzyme (e.g., a tumor-associated protease), an amide linkage between the amino acids is cleaved, which ultimately leads to release of free drug.

Each amino acid is proteinogenic or non-proteinogenic and/or a D- or L-isomer provided that RL comprises a cleavable bond that, when cleaved, initiates release of the Drug Unit. In some embodiments, the Peptide Releasable Linker will comprise only proteinogenic amino acids. In some aspects, the Peptide Releasable Linker will have from 1 to no more than 12 amino acids in contiguous sequence.

In some embodiments, each amino acid is independently selected from the group consisting of alanine, arginine, aspartic acid, asparagine, histidine, glycine, glutamic acid, glutamine, phenylalanine, lysine, leucine, serine, tyrosine, threonine, isoleucine, proline, tryptophan, valine, cysteine, methionine, selenocysteine, ornithine, penicillamine, β-alanine, aminoalkanoic acid, aminoalkynoic acid, aminoalkanedioic acid, aminobenzoic acid, amino-heterocyclo-alkanoic acid, heterocyclo-carboxylic acid, citrulline, statine, diaminoalkanoic acid, and derivatives thereof. In some embodiments, each amino acid is independently selected from the group consisting of alanine, arginine, aspartic acid, asparagine, histidine, glycine, glutamic acid, glutamine, phenylalanine, lysine, leucine, serine, tyrosine, threonine, isoleucine, proline, tryptophan, valine, cysteine, methionine, and selenocysteine. In some embodiments, each amino acid is independently selected from the group consisting of alanine, arginine, aspartic acid, asparagine, histidine, glycine, glutamic acid, glutamine, phenylalanine, lysine, leucine, serine, tyrosine, threonine, isoleucine, proline, tryptophan, and valine. In some embodiments, each amino acid is selected from the proteinogenic or the non-proteinogenic amino acids.

In another embodiment, each amino acid is independently selected from the group consisting of the following L-(proteinogenic) amino acids: alanine, arginine, aspartic acid, asparagine, histidine, glycine, glutamic acid, glutamine, phenylalanine, lysine, leucine, serine, tyrosine, threonine, isoleucine, tryptophan and valine.

In another embodiment, each amino acid is independently selected from the group consisting of the following D-isomers of these proteinogenic amino acids: alanine, arginine, aspartic acid, asparagine, histidine, glycine, glutamic acid, glutamine, phenylalanine, lysine, leucine, serine, tyrosine, threonine, isoleucine, tryptophan and valine.

In certain embodiments, the Peptide Releasable Linker comprises only proteinogenic amino acids. In other embodiments, the Peptide Releasable Linker comprises only non-proteinogenic amino acids. In some embodiments, the Peptide Releasable Linker comprises a proteinogenic amino acid attached to a non-proteinogenic amino acid. In some embodiments, Peptide Releasable Linker comprises a proteinogenic amino acid attached to a D-isomer of a proteinogenic amino acid.

In another embodiment, each amino acid is independently selected from the group consisting of β-alanine, N-methylglycine, glycine, lysine, valine and phenylalanine.

Exemplary Peptide Releasable Linkers include dipeptides or tripeptides with-Val-Lys-Gly-, -Val-Cit-, -Phe-Lys- or -Val-Ala-.

Useful Peptide Releasable Linkers are designed and optimized in their selectivity for enzymatic cleavage by a particular enzyme, for example, a tumor-associated protease. In some embodiments, cleavage of a linkage is catalyzed by cathepsin B, C or D, or a plasmin protease.

In some embodiments, the Peptide Releasable Linker (RL) will be represented by -(-AA-)₁₋₁₂-, or (-AA-AA-)₁₋₆ wherein AA is at each occurrence independently selected from proteinogenic or non-proteinogenic amino acids. In one aspect, AA is at each occurrence independently selected from proteinogenic amino acids. In another aspect, RL is a tripeptide having the formula: AA₁-AA₂- AA₃, wherein AA₁, AA₂ and AA₃ are each independently an amino acid and wherein AA₁ attaches to -NH- and AA₃ attaches to S^{∗}. In yet another aspect, AA₃ is gly or β-ala.

In some embodiments, the Peptide Releasable Linker has the formula denoted below in the square brackets, the subscript w is an integer ranging from 1 to 12; or w is 1, 2, 3, 4, 5, 6, 7, 8, 9, 10, 11, or 12; or w is 2, 3, or 4; or w is 3; or w is 4: 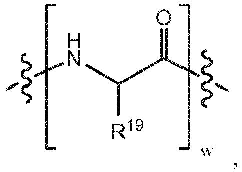 wherein R¹⁹ is, in each instance, independently selected from the group consisting of hydrogen, methyl, isopropyl, isobutyl, sec-butyl, benzyl, p-hydroxybenzyl, -CH₂OH, - CH(OH)CH₃, -CH₂CH₂SCH₃, -CH₂CONH₂, -CH₂COOH, -CH₂CH₂CONH₂, - CH₂CH₂COOH, -(CH₂)₃NHC(=NH)NH₂, -(CH₂)₃NH₂, -(CH₂)₃NHCOCH₃, -(CH₂)₃NHCHO, -(CH₂)₄NHC(=NH)NH₂, -(CH₂)₄NH₂, -(CH₂)₄NHCOCH₃, -(CH₂)₄NHCHO, - (CH₂)₃NHCONH₂, -(CH₂)₄NHCONH₂, -CH₂CH₂CH(OH)CH₂NH₂, 2-pyridylmethyl-, 3-pyridylmethyl-, 4-pyridylmethyl-, phenyl, cyclohexyl, 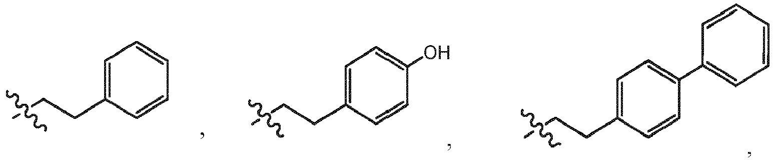 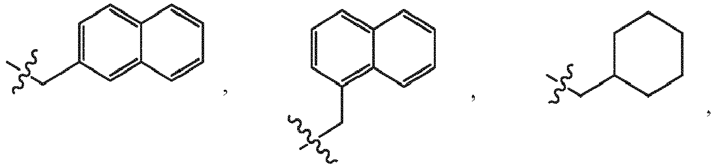 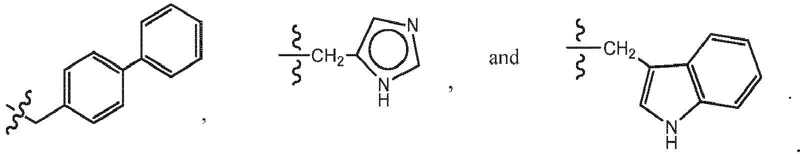 In some aspects, the subscript w is not 3.

In some aspects, each R¹⁹ is independently hydrogen, methyl, isopropyl, isobutyl, *sec-*butyl, -(CH₂)₃NH₂, or -(CH₂)₄NH₂. In some aspects, each R¹⁹ is independently hydrogen, isopropyl, or -(CH₂)₄NH₂.

Illustrative Peptide Releasable Linkers are represented by formulae (Pa), (Pb) and (Pc): 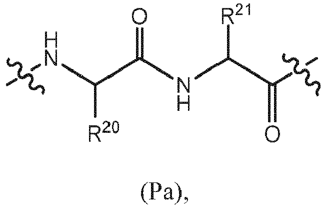 wherein R²⁰ and R²¹ are as follows:

| R²⁰ | R²¹ |
|---|---|
| benzyl | (CH₂)₄NH₂; |
| methyl | (CH₂)₄NH₂; |
| isopropyl | (CH₂)₄NH₂; |
| isopropyl | (CH₂)₃NHCONH₂; |
| benzyl | (CH₂)₃NHCONH₂; |
| isobutyl | (CH₂)₃NHCONH₂; |
| *sec*-butyl | (CH₂)₃NHCONH₂; |
| 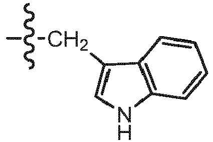 | (CH₂)₃NHCONH₂; |
| benzyl | methyl; and |
| benzyl | (CH₂)₃NHC(=NH)NH₂; |
| 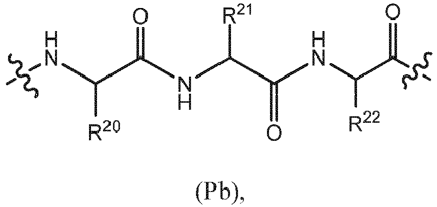 | |

wherein R²⁰, R²¹ and R²² are as follows:

| R²⁰ | R²¹ | R²² |
|---|---|---|
| benzyl | benzyl | -(CH₂)₄NH₂ |
| isopropyl | benzyl | -(CH₂)₄NH₂ |
| H | Benzyl | -(CH₂)₄NH₂ |
| isopropyl | -(CH₂)₄NH₂ | -H |
| | 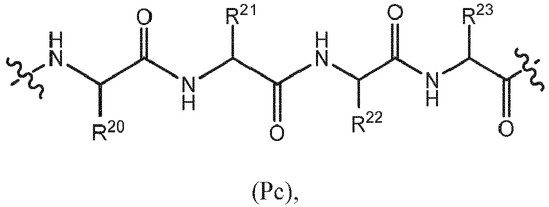 | |

wherein R²⁰, R²¹, R²² and R²³ are as follows:

| R²⁰ | R²¹ | R²² | R²³ |
|---|---|---|---|
| H | benzyl | isobutyl | H; and |
| methyl | isobutyl | methyl | isobutyl. |

In some embodiments, RL comprises a peptide selected from the group consisting of gly-gly, gly-gly-gly, gly-gly-gly-gly, val-gly-gly, val-cit-gly, val-gln-gly, val-glu-gly, phe-lys-gly, leu-lys-gly, gly-val-lys-gly, val-lys-gly-gly, val-lys-gly, val-lys-ala, val-lys-leu, leu-leu-gly, gly-gly-phe-gly, gly-gly-phe-gly-gly, val-gly, and val-lys-β-ala.

In other embodiments, RL comprises a peptide selected from the group consisting of gly-gly-gly, gly-gly-gly-gly, val-gly-gly, val-cit-gly, val-gln-gly, val-glu-gly, phe-lys-gly, leu-lys-gly, gly-val-lys-gly, val-lys-gly-gly, val-lys-gly, val-lys-ala, val-lys-leu, leu-leu-gly, gly-gly-phe-gly, and val-lys-β-ala.

In still other embodiments, RL comprises a peptide selected from the group consisting of gly-gly-gly, val-gly-gly, val-cit-gly, val-gln-gly, val-glu-gly, phe-lys-gly, leu-lys-gly, val-lys-gly, val-lys-ala, val-lys-leu, leu-leu-gly and val-lys-β-ala.

In yet other embodiments, RL comprises a peptide selected from the group consisting of gly-gly-gly-gly, gly-val-lys-gly, val-lys-gly-gly, and gly-gly-phe-gly.

In other embodiments, RL is a peptide selected from the group consisting of val-gln-gly, val-glu-gly, phe-lys-gly, leu-lys-gly, val-lys-gly, val-lys-ala, val-lys-leu, leu-leu-gly and val-lys-β-ala.

In still other embodiments, RL is val-lys-gly.

In still other embodiments, RL is val-lys-β-ala.

In some embodiments, the Releasable Linker RL is 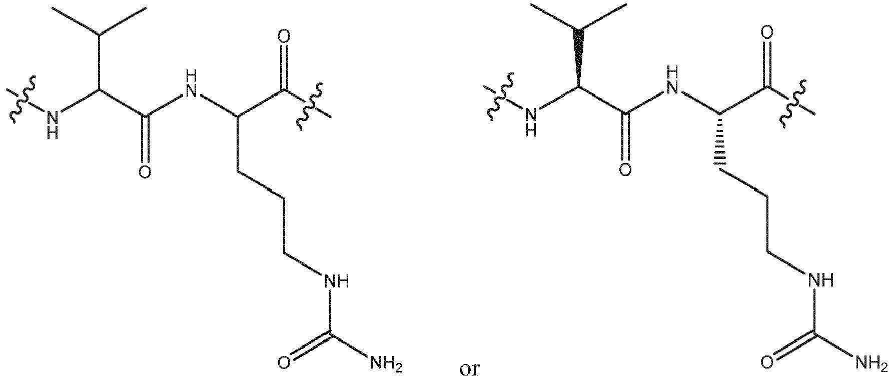 wherein the wavy line adjacent to the -NH- group indicates attachment to the Stretcher Unit Z' or the Connector Unit A and the wavy line adjacent to the -C(=O)- group indicates attachment to the Spacer Unit Y or the Drug Unit D.

### Glycoside Unit Releasable Linkers

In some embodiments, the Releasable Linker is a Glycoside (e.g., Glucuronide) Unit. In such embodiments, a self-immolation cascade is activated by operation of a glycosidase on a carbohydrate moiety of the Glycoside (e.g., Glucuronide) Unit. A number of sugars are useful in the embodiments described herein. Particular carbohydrate moieties include those of Galactose, Glucose, Mannose, Xylose, Arabinose, Mannose-6-phosphate, Fucose, Rhamnose, Gulose, Allose, 6-deoxy-glucose, Lactose, Maltose, Cellobiose, Gentiobiose, Maltotriose, GlcNAc, GalNAc and maltohexaose.

A Glycoside (e.g., Glucuronide) Unit typically comprises a sugar moiety (Su) linked via an oxygen glycosidic bond to a self-immolative spacer. Cleavage of the oxygen glycosidic bond initiates the self-immolation reaction sequence that result in release of free drug. In some embodiments, the self-immolation sequence is activated from cleavage by β-glucuronidase of a Glycoside (e.g., Glucuronide) Unit, which is an exemplary glycoside unit. The Glycoside (e.g., Glucuronide) Unit comprises an activation unit and a self-immolative Spacer Unit. The Glycoside (e.g., Glucuronide) Unit comprises a sugar moiety (Su) linked via an oxygen glycosidic bond to a self-immolative Spacer Unit.

In some embodiments, a Glycoside (e.g., Glucuronide) Unit comprises a sugar moiety (Su) linked via an oxygen glycoside bond (-O'-) to a Self-immolative Unit (SP) of the formula: 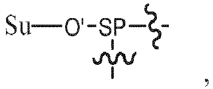 wherein the wavy lines indicate covalent attachment to the Drug Unit or to a Spacer Unit that is attached to the Drug Unit, and to the Stretcher Unit precursor (Z'), either directly or indirectly through the Connector Unit (A) or Parallel Connector Unit (B), Partitioning Agent (S^{∗}) or combinations of the Connector Unit and Parallel Connector Unit, as the case may be.

The oxygen glycosidic bond (-O'-) is typically a β-glucuronidase-cleavage site (i.e., Su is from glucuronide), such as a glycoside bond cleavable by human, lysosomal β-glucuronidase.

In some embodiments, the Glycoside (e.g., Glucuronide) Unit is represented by formula Ga, Gb, or Gc: 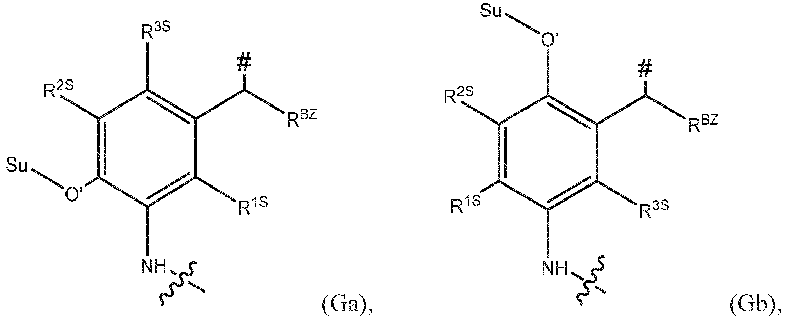 or 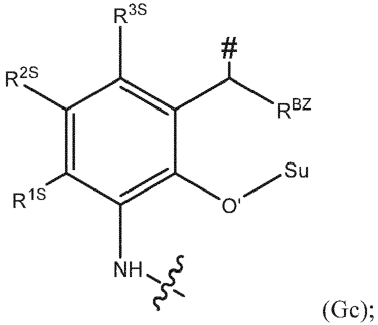 wherein Su is a Sugar moiety, -O'- represents an oxygen glycosidic bond; R^{1S}, R^{2S} and R^{3S} independently are hydrogen, a halogen, -CN,-NO₂, or other electron withdrawing group, or an electron donating group; R^{BZ} is selected from the group consisting of C₁-C₆ alkyl, C₁-C₆ haloalkyl, a PEG unit, a cyclodextrin unit, a polyamide, a hydrophilic peptide, a polysaccharide, and a dendrimer; and wherein the wavy line indicates attachment to a Stretcher Unit precursor (Z'), either directly or indirectly through a Connector Unit or Parallel Connector Unit or Connector unit and Parallel Connector Unit); and # indicates attachment to the Drug Unit or to a Spacer (either directly or indirectly via an intervening functional group or other moiety).

In some embodiments, R^{1S}, R^{2S}, and R^{3S} are independently selected from hydrogen, halogen, -CN, or -NO₂. In some embodiments, R^{1S}, R^{2S} and R^{3S} are each hydrogen. In some embodiments, R^{2S} is an electron withdrawing group, preferably NO₂, and R^{1S} and R^{3S} are each hydrogen.

In some such aspects the activatable self-immolative group capable of glycosidase cleavage to initiate the self-immolative reaction sequence is represented by the formula Gd: 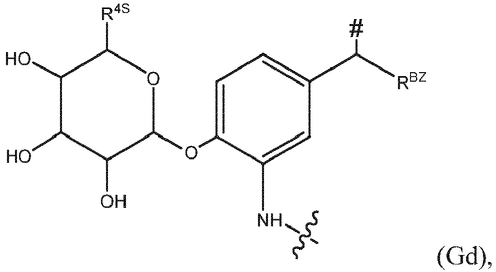 wherein R^{4S} is CH₂OH or -CO₂H, the wavy line indicates covalent attachment to a Stretcher Unit (Z) (or its precursor Z'), either directly or indirectly through a Connector Unit or Parallel Connector Unit or Connector unit and Parallel Connector Unit, and the hash mark (#) indicates covalent attachment to the methylene carbamate unit.

In some embodiments wherein the activatable self-immolative moiety comprises a Glycoside (e.g., Glucuronide) Unit, it is represented by the following formula Ge: 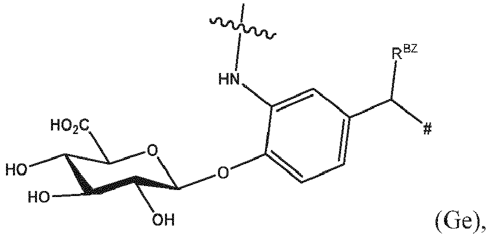 wherein the wavy line indicates covalent attachment to a Stretcher Unit (Z) (or its precursor Z'), either directly or indirectly through a Connector Unit or Parallel Connector Unit or Connector unit and Parallel Connector Unit and the hash mark (#) indicates covalent attachment of the benzylic carbon of a Spacer or functional group attached to the Drug Unit. In some embodiments, the structure of formula Ge is attached to the Drug Unit via a quaternized tertiary amine (N⁺), wherein the nitrogen atom is from a tertiary amine functional group on the unconjugated Drug Unit.

Another type of Releasable Linker that provides a mechanism for separation of the Drug Unit from the Ligand Unit and other components of the Linker Unit through activation of a self-immolation cascade within the Linker Unit comprises a p-aminobenzyloxycarbonyl (PAB) moiety whose phenylene component is substituted with Jₘ wherein the subscript m indicating the number of substituents is an integer ranging from 0-4, and each J is independently -C₁-C₈ alkyl, -O-(C₁-C₈ alkyl), -halogen, -nitro or -cyano.

In some embodiments, RL is a self-immolative group capable of releasing -D without the need for a separate hydrolysis step or subsequent self-immolative event. In some embodiments, -RL- is a PAB moiety that is linked to the carbonyl of -W- via the amino nitrogen atom of the PAB group, and connected directly to -D via a carbonate group. In related embodiments, -RL- comprises a PAB moiety that is linked to the carbonyl of -A-, -S^{∗}- or -B- via the amino nitrogen atom of the PAB group, and connected directly to -D via a carbonate group. Without being bound by any particular theory or mechanism, a possible mechanism of Drug release from RL comprises a PAB moiety in which RL is attached directly to -D via a carbonate group is shown in Toki et al. (2002) J Org. Chem. 67:1866-1872.

In some embodiments, RL units containing a PAB moiety are represented by the formula: 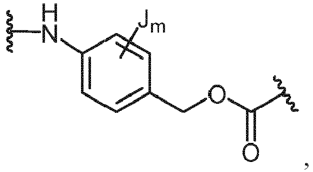 wherein subscript m is an integer ranging from 0-4, and each J is independently -C₁-C₈ alkyl, -O-(C₁-C₈ alkyl), -halogen, -nitro or -cyano.

Other examples of self-immolative groups include, but are not limited to, aromatic compounds that are electronically similar to the PAB moiety such as 2-aminoimidazol-5-methanol derivatives (Hay et al. (1999) Bioorg. Med. Chem. Lett. 9:2237) and ortho or para-aminobenzylacetals. Other RLs undergo cyclization upon amide bond hydrolysis, such as substituted and unsubstituted 4-aminobutyric acid amides (Rodrigues *et al.,* Chemistry Biology, 1995, 2, 223), appropriately substituted bicyclo[2.2.1] and bicyclo[2.2.2] ring systems (Storm, *et al.,* J. Amer. Chem. Soc., 1972, 94, 5815) and 2-aminophenylpropionic acid amides (Amsberry, *et al.,* J. Org. Chem., 1990, 55, 5867).

In one embodiment, RL is a branched bis(hydroxymethyl)styrene (BHMS) unit.

In some embodiments, RL has the formula: 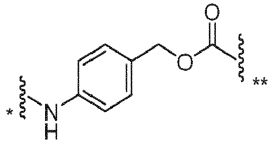 wherein the wavy line marked with ^{∗∗} indicates the site of attachment to D; and the wavy line marked with ^{∗} indicates the point of attachment to additional linker components of Q. In some embodiments, a PAB-containing RL is directly attached to the Drug Unit.

### Partitioning Agent S^{∗}

The Ligand-Drug Conjugates described herein can also include a Partitioning Agent (S^{∗}). The Partitioning Agent portions are useful, for example, to mask the hydrophobicity of particular Drug Units or Linking Unit components.

Representative Partitioning Agents include polyethylene glycol (PEG) units, cyclodextrin units, polyamides, hydrophilic peptides, polysaccharides and dendrimers.

When the polyethylene glycol (PEG) units, cyclodextrin units, polyamides, hydrophilic peptides, polysaccharides or dendrimers are included in Q, the groups may be present as an 'in line' component or as a side chain or branched component. For those embodiments in which a branched version is present, the Linker Units can include a lysine residue (or Parallel Connector Unit, B) that provides simple functional conjugation of, for example, the PEG unit, to the remainder of the Linking Unit.

### Polyethylene Glycol (PEG) Unit

When present, polydisperse PEGs, monodisperse PEGs, and discrete PEGs are used as part of the Partitioning Agents in Compounds of the present invention. Polydisperse PEGs are a heterogeneous mixture of sizes and molecular weights whereas monodisperse PEGs are typically purified from heterogeneous mixtures and are therefore provide a single chain length and molecular weight. Preferred PEG Units are discrete PEGs, compounds that are synthesized in stepwise fashion and not via a polymerization process. Discrete PEGs provide a single molecule with defined and specified chain length.

The PEG Unit provided herein can comprise one or multiple polyethylene glycol chains. A polyethylene glycol chain is composed of at least two ethylene oxide (CH₂CH₂O) subunits. In some embodiments the polyethylene glycol chains are linked together, for example, in a linear, branched or star shaped configuration. Typically, at least one of the PEG chains is derivitized at one end for covalent attachment to an appropriate site on a component of the Linker Unit (e.g. B) or is used as an in-line (e.g., bifunctional) linking group within to covalently join two of the Linker Unit components (e.g., Z-A-S^{∗}-RL-, Z-A-S^{∗}-RL-Y-). Exemplary attachments within the Linker Unit are by means of non-conditionally cleavable linkages or via conditionally cleavable linkages. Exemplary attachments are via amide linkage, ether linkages, ester linkages, hydrazone linkages, oxime linkages, disulfide linkages, peptide linkages or triazole linkages. In some embodiments, attachment within the Linker Unit is by means of a non-conditionally cleavable linkage. In some embodiments, attachment within the Linker Unit is not via an ester linkage, hydrazone linkage, oxime linkage, or disulfide linkage. In some embodiments, attachment within the Linker Unit is not via a hydrazone linkage.

A conditionally cleavable linkage refers to a linkage that is not substantially sensitive to cleavage while circulating in the plasma but is sensitive to cleavage in an intracellular or intratumoral environment. A non-conditionally cleavable linkage is one that is not substantially sensitive to cleavage in any biological environment. Chemical hydrolysis of a hydrazone, reduction of a disulfide, and enzymatic cleavage of a peptide bond or glycosidic linkage are examples of conditionally cleavable linkages.

In some embodiments, the PEG Unit is directly attached to a Parallel Connector Unit B, wherein the other terminus (or termini) of the PEG Unit is free and untethered and may take the form of a methoxy, carboxylic acid, alcohol or other suitable functional group. The methoxy, carboxylic acid, alcohol or other suitable functional group acts as a cap for the terminal PEG subunit of the PEG Unit. By untethered, it is meant that the PEG Unit will not be attached at that untethered site to a Drug Unit, to an antibody, or to another linking component. The skilled artisan will understand that the PEG Unit in addition to comprising repeating ethylene glycol subunits may also contain non-PEG material (e.g., to facilitate coupling of multiple PEG chains to each other). Non-PEG material refers to the atoms in the PEG Unit that are not part of the repeating -CH₂CH₂O- subunits. In some embodiments provided herein, the PEG Unit comprises two monomeric PEG chains attached to each other via non-PEG elements. In other embodiments provided herein, the PEG Unit comprises two linear PEG chains attached to a central core or Parallel Connector Unit (i.e., the PEG Unit itself is branched).

There are a number of PEG attachment methods available to those skilled in the art, [see, e.g., Goodson, et al. (1990) Bio/Technology 8:343 (PEGylation of interleukin-2 at its glycosylation site after site-directed mutagenesis); EP 0 401 384 (coupling PEG to G-CSF); Malik, et al., (1992) Exp. Hematol. 20:1028-1035 (PEGylation of GM-CSF using tresyl chloride); PCT Pub. No. WO 90/12874 (PEGylation of erythropoietin containing a recombinantly introduced cysteine residue using a cysteine-specific mPEG derivative); U.S. Pat. No. 5,757,078 (PEGylation of EPO peptides); U.S. Pat. No. 5,672,662 (Poly(ethylene glycol) and related polymers monosubstituted with propionic or butanoic acids and functional derivatives thereof for biotechnical applications); U.S. Pat. No. 6,077,939 (PEGylation of an N-terminal .alpha.-carbon of a peptide); Veronese et al., (1985) Appl. Biochem. Biotechnol 11:141-142 (PEGylation of an N-terminal α-carbon of a peptide with PEG-nitrophenylcarbonate ("PEG-NPC") or PEG-trichlorophenylcarbonate); and Veronese (2001) Biomaterials 22:405-417 (Review article on peptide and protein PEGylation)].

For example, PEG may be covalently bound to amino acid residues via a reactive group. Reactive groups are those to which an activated PEG molecule may be bound (e.g., a free amino or carboxyl group). For example, N-terminal amino acid residues and lysine (K) residues have a free amino group; and C-terminal amino acid residues have a free carboxyl group. Thiol groups (e.g., as found on cysteine residues) can also be useful as a reactive group for attaching PEG. In addition, enzyme-assisted methods for introducing activated groups (e.g., hydrazide, aldehyde, and aromatic-amino groups) specifically at the C-terminus of a polypeptide have been described (see Schwarz, et al. (1990) Methods Enzymol. 184:160; Rose, et al. (1991) Bioconjugate Chem. 2:154; and Gaertner, et al. (1994) J. Biol. Chem. 269:7224].

In some embodiments, PEG molecules may be attached to amino groups using methoxylated PEG ("mPEG") having different reactive moieties. Non-limiting examples of such reactive moieties include succinimidyl succinate (SS), succinimidyl carbonate (SC), mPEG-imidate, para-nitrophenylcarbonate (NPC), succinimidyl propionate (SPA), and cyanuric chloride. Non-limiting examples of such mPEGs include mPEG-succinimidyl succinate (mPEG-SS), mPEG₂-succinimidyl succinate (mPEG₂-SS); mPEG-succinimidyl carbonate (mPEG-SC), mPEG₂-succinimidyl carbonate (mPEG₂-SC); mPEG-imidate, mPEG-para-nitrophenylcarbonate (mPEG-NPC), mPEG-imidate; mPEG₂-para-nitrophenylcarbonate (mPEG₂-NPC); mPEG-succinimidyl propionate (mPEG-SPA); mPEG₂-succinimidyl propionate (mPEG₂-SPA); mPEG-N-hydroxy-succinimide (mPEG-NHS); mPEG₂-N-hydroxy-succinimide (mPEG₂-NHS); mPEG-cyanuric chloride; mPEG₂-cyanuric chloride; mPEG₂-Lysinol-NPC, and mPEG₂-Lys-NHS.

Generally, at least one of the PEG chains that make up the PEG Unit is functionalized so that it is capable of covalent attachment to other Linker Unit components.

Functionalization includes, for example, via an amine, thiol, NHS ester, maleimide, alkyne, azide, carbonyl, or other functional group. In some embodiments, the PEG Unit further comprises non-PEG material (i.e., material does not comprise -CH₂CH₂O-) that provides coupling to other Linker Unit components or to facilitate coupling of two or more PEG chains.

The presence of the PEG Unit (or other Partitioning Agent) in the Linker Unit can have two potential impacts upon the pharmacokinetics of the resulting Ligand-Drug Conjugate. The desired impact is a decrease in clearance (and consequent increase in exposure) that arises from the reduction in non-specific interactions induced by the exposed hydrophobic elements of the Ligand-Drug Conjugate or to the Drug Unit itself. The second impact is undesired and is a decrease in volume and rate of distribution that sometimes arises from the increase in the molecular weight of the Ligand-Drug Conjugate.

Increasing the number of PEG subunits increases the hydrodynamic radius of a conjugate, typically resulting in decreased diffusivity. In turn, decreased diffusivity typically diminishes the ability of the Ligand-Drug Conjugate to penetrate into a tumor (Schmidt and Wittrup, Mol Cancer Ther 2009;8:2861-2871). Because of these two competing pharmacokinetic effects, it is desirable to use a PEG that is sufficiently large to decrease the Ligand-Drug Conjugate clearance thus increasing plasma exposure, but not so large as to greatly diminish its diffusivity, to an extent that it interferes with the ability of the Ligand-Drug Conjugate to reach the intended target cell population. See the examples (e.g., examples 1, 18, and 21) of US2016/0310612, which are incorporated by reference herein, for methodology for selecting an optimal PEG size for a particular drug-linker.

In one group of embodiments, the PEG Unit comprises one or more linear PEG chains each having at least 2 subunits, at least 3 subunits, at least 4 subunits, at least 5 subunits, at least 6 subunits, at least 7 subunits, at least 8 subunits, at least 9 subunits, at least 10 subunits, at least 11 subunits, at least 12 subunits, at least 13 subunits, at least 14 subunits, at least 15 subunits, at least 16 subunits, at least 17 subunits, at least 18 subunits, at least 19 subunits, at least 20 subunits, at least 21 subunits, at least 22 subunits, at least 23 subunits, or at least 24 subunits. In some embodiments, the PEG Unit comprises a combined total of at least 4 subunits, at least 6 subunits, at least 8 subunits, at least 10 subunits, or at least 12 subunits. In some such embodiments, the PEG Unit comprises no more than a combined total of about 72 subunits, preferably no more than a combined total of about 36 subunits.

In one group of embodiments, the PEG Unit comprises one or more linear PEG chains each having 2 subunits, 3 subunits, 4 subunits, 5 subunits, 6 subunits, 7 subunits, 8 subunits, 9 subunits, 10 subunits, 11 subunits, 12 subunits, 13 subunits, 14 subunits, 15 subunits, 16 subunits, 17 subunits, 18 subunits, 19 subunits, 20 subunits, 21 subunits, 22 subunits, 23 subunits, or 24 subunits. In some embodiments, the PEG Unit comprises a combined total of 4 subunits, 6 subunits, 8 subunits, 10 subunits, or 12 subunits. In some such embodiments, the PEG Unit comprises no more than a combined total of about 72 subunits, preferably no more than a combined total of about 36 subunits.

In another group of embodiments, the PEG Unit comprises a combined total of from 4 to 72, 4 to 60, 4 to 48, 4 to 36 or 4 to 24 subunits, from 5 to 72, 5 to 60, 5 to 48, 5 to 36 or 5 to 24 subunits, from 6 to 72, 6 to 60, 6 to 48, 6 to 36 or from 6 to 24 subunits, from 7 to 72, 7 to 60, 7 to 48, 7 to 36 or 7 to 24 subunits, from 8 to 72, 8 to 60, 8 to 48, 8 to 36 or 8 to 24 subunits, from 9 to 72, 9 to 60, 9 to 48, 9 to 36 or 9 to 24 subunits, from 10 to 72, 10 to 60, 10 to 48, 10 to 36 or 10 to 24 subunits, from 11 to 72, 11 to 60, 11 to 48, 11 to 36 or 11 to 24 subunits, from 12 to 72, 12 to 60, 12 to 48, 12 to 36 or 12 to 24 subunits, from 13 to 72, 13 to 60, 13 to 48, 13 to 36 or 13 to 24 subunits, from 14 to 72, 14 to 60, 14 to 48, 14 to 36 or 14 to 24 subunits, from 15 to 72, 15 to 60, 15 to 48, 15 to 36 or 15 to 24 subunits, from 16 to 72, 16 to 60, 16 to 48, 16 to 36 or 16 to 24 subunits, from 17 to 72, 17 to 60, 17 to 48, 17 to 36 or 17 to 24 subunits, from 18 to 72, 18 to 60, 18 to 48, 18 to 36 or 18 to 24 subunits, from 19 to 72, 19 to 60, 19 to 48, 19 to 36 or 19 to 24 subunits, from 20 to 72, 20 to 60, 20 to 48, 20 to 36 or 20 to 24 subunits, from 21 to 72, 21 to 60, 21 to 48, 21 to 36 or 21 to 24 subunits, from 22 to 72, 22 to 60, 22 to 48, 22 to 36 or 22 to 24 subunits, from 23 to 72, 23 to 60, 23 to 48, 23 to 36 or 23 to 24 subunits, or from 24 to 72, 24 to 60, 24 to 48, 24 to 36 or 24 subunits.

In some embodiments, the Partitioning Agent S^{∗} is a linear PEG Unit comprising from 2 to 20, or from 2 to 12, or from 4 to 12, or 4, 8, or 12 -CH₂CH₂O- subunits. In some embodiments, the linear PEG Unit is connected at one end of the PEG Unit to the RL Unit and at the other end of the PEG Unit to the Stretcher/Connector Units (Z-A-). In some embodiments, the PEG Unit is connected to the RL Unit via a -CH₂CH₂C(O)- group that forms an amide bond with the RL Unit (e.g., -(CH₂CH₂O)ₙ-CH₂CH₂C(O)-RL) and to the Stretcher Unit/Connector Unit (Z-A-) via an -NH- group (e.g., Z-A-NH-(CH₂CH₂O)ₙ-) that forms an amide bond with the Z-A- portion.

Illustrative embodiments for PEG Units that are connected to the RL and Stretcher/Connector Units (Z-A-) are shown below: 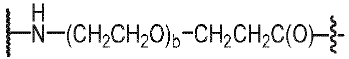 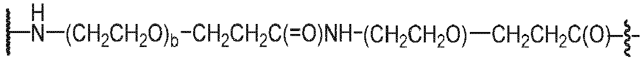 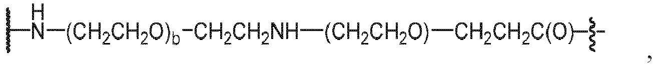 and in a particular embodiment, the PEG Unit is: 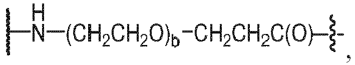 wherein the wavy line on the left indicates the site of attachment to Z-A-, the wavy line on the right indicates the site of attachment to RL, and each b is independently selected from 2 to 72, 4 to 72, 6 to 72, 8 to 72, 10 to 72, 12 to 72, 2 to 24, 4 to 24, 6 to 24, or 8 to 24, 2 to 12, 4 to 12, 6 to 12, and 8 to 12. In some embodiments, subscript b is 2, 4, 8, 12, or 24. In some embodiments, subscript b is 2. In some embodiments, subscript b is 4. In some embodiments, subscript b is 8. In some embodiments, subscript b is 12.

In some embodiments, the linear PEG Unit that is connected to the Parallel Connector Unit at one end and comprises a terminal cap at the other end. In some embodiments, the PEG Unit is connected to the Parallel Connector Unit via a carbonyl group that forms an amide bond with the Parallel Connector Unit lysine residue amino group (e.g., - CH₂CH₂(OCH₂CH₂)ₖ-C(O)-B-, wherein k is an integer from 1 to 36) and includes a PEG Unit terminal cap group selected from the group consisting of C₁₋₄alkyl and C₁₋₄alkyl-CO₂H. In some embodiments, the Partitioning Agent S^{∗} is a linear PEG Unit comprising 4, 8, or 12 - CH₂CH₂O- subunits and a terminal methyl cap.

Illustrative linear PEG Units that are used in any of the embodiments provided herein are as follows: 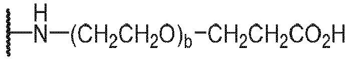 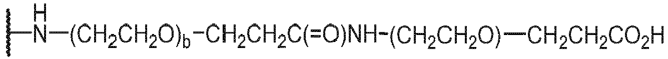 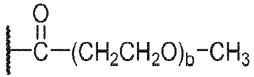 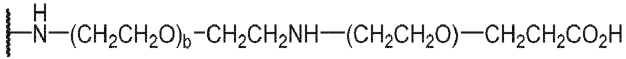 and in a particular embodiment, the PEG Unit is: 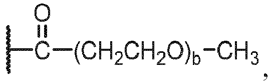 wherein the wavy line indicates site of attachment to the Parallel Connector Unit (B), and each n is independently selected from 4 to 72, 6 to 72, 8 to 72, 10 to 72, 12 to 72, 6 to 24, or 8 to 24. In some embodiments, subscript b is about 4, about 8, about 12, or about 24.

As used to herein, terms "PEG2", "PEG4", "PEG8", and "PEG12" refers to specific embodiments of PEG Unit which comprises the number of PEG subunits (i.e., the number of subscription "b"). For example, "PEG2" refers to embodiments of PEG Unit that comprises 2 PEG subunits, "PEG4" refers to embodiments of PEG Unit that comprises 4 PEG subunits, "PEG8" refers to embodiments of PEG Unit that comprises 8 PEG subunits, and "PEG12" refers to embodiments of PEG Unit that comprises 12 PEG subunits.

As described herein, the PEG unit is selected such that it improves clearance of the resultant Ligand-Drug Conjugate but does not significantly impact the ability of the Conjugate to penetrate into the tumor. In embodiments, the PEG unit to be selected for use will preferably have from 2 subunits to about 24 subunits, from 4 subunits to about 24 subunits, more preferably about 4 subunits to about 12 subunits.

In some embodiments of the present disclosure the PEG Unit is from about 300 daltons to about 5 kilodaltons; from about 300 daltons, to about 4 kilodaltons; from about 300 daltons, to about 3 kilodaltons; from about 300 daltons, to about 2 kilodaltons; or from about 300 daltons, to about 1 kilodalton. In some such aspects, the PEG Unit has at least 6 subunits or at least 8, 10 or 12 subunits. In some such aspects, the PEG Unit has at least 6 subunits or at least 8, 10 or 12 subunits but no more than 72 subunits, preferably no more than 36 subunits.

It will be appreciated that when referring to PEG subunits, and depending on context, the number of subunits can represent an average number, e.g., when referring to a population of Ligand-Drug Conjugates or Drug-Linker Compounds, and/or using polydisperse PEGs.

### Parallel Connector Unit (B)

In some embodiments, the Ligand-Drug Conjugates and Drug-Linker Compounds will comprise a Parallel Connector Unit to provide a point of attachment to a Partitioning Agent (shown in the Linker Units as -B(S^{∗})-). In some embodiments, the PEG Unit is attached to a Parallel Connector Unit such as lysine as shown below wherein the wavy line and asterisks indicate covalent linkage within the Linker Unit of a Ligand-Drug Conjugate or Drug-Linker Compound:

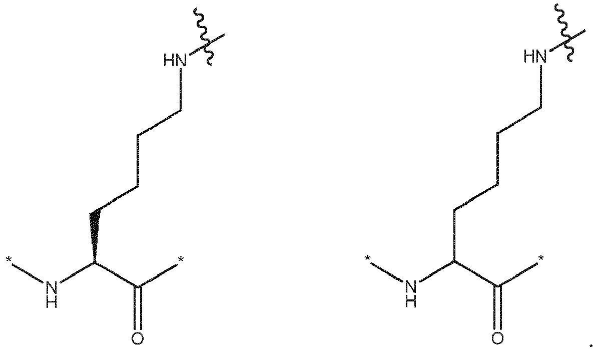

In some embodiments, the Parallel Connector Unit (B) and Partitioning Agent (S^{∗}) (together, -B(S^{∗})-) have the structure of 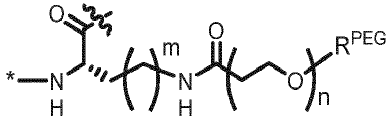 wherein m ranges from 0 to 6; n ranges from 2 to 24; R^{PEG} is a PEG Capping Unit, preferably H, -CH₃, or -CH₂CH₂CO₂H, the asterisk (^{∗}) indicates covalent attachment to a Connector Unit A corresponding in formula Za, Za', Zb' or Zc' and the wavy line indicates covalent attachment to the Releasable Linker (RL). In some embodiments, the structure is attached to a Connector Unit A in formula Za or Za'. In some embodiments, n is 2, 4, 8, or 12. In instances such as those shown here, the shown PEG group is meant to be exemplary of a variety of Partitioning Agents including PEG groups of different lengths and other Partitioning Agents that are directly attached or modified for attachment to the Parallel Connector Unit.

### Spacer Unit (Y)

In some embodiments, the Ligand-Drug Conjugates provided herein will have a Spacer (Y) between the Releasable Linker (RL) and the Drug Unit. The Spacer Unit is a functional group to facilitate attachment of RL to the Drug Unit, or provides additional structural components to further facilitate release of the Drug Unit from the remainder of the Conjugate (e.g., a methylene carbamate unit or a self-immolative para-aminobenzyl (PAB) component).

In those embodiments to further facilitate release of the Drug Unit as free drug, the Spacer Unit Y is represented by one of the following formulae: 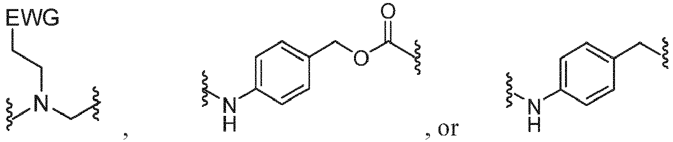 wherein EWG represents an electron-withdrawing group and the wavy lines indicate the site of attachment to the rest of the Drug-Linker compound or salt thereof. In some embodiments, EWG is selected from the group consisting of -CN, -NO₂, -CX₃, -X, -C(=O)OR', - C(=O)N(R')₂, -C(=O)R', -C(=O)X, -S(=O)₂R', -S(=O)₂OR', -S(=O)₂NHR', -S(=O)₂N(R')₂,-P(=O)(OR')₂, -P(=O)(CH₃)NHR', -NO, -N(R')₃⁺, wherein X is -F, -Br, -Cl, or -I, and R' is independently selected from the group consisting of hydrogen and C₁-C₆alkyl.

In some embodiments, the Spacer Unit-Drug Unit group (-Y-T^{∗}-D) is represented by one of the following formulae: 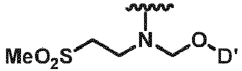 wherein the wavy line adjacent is the point of covalent attachment to RL, T^{∗} is as defined above, and D' represents the remainder of the Drug Unit, wherein T^{∗} and D' together form a Drug Unit of Formula (Ia), or any subformula thereof.

In some embodiments, the Spacer Unit is represented by the formula: 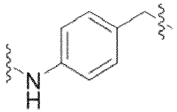 wherein the wavy line adjacent to the nitrogen atom is the point of covalent attachment to RL, as defined above, and the wavy line next to the benzylic carbon atom connects to a Drug Unit. In some embodiments, the Drug Unit is attached to the benzylic carbon atom via a quaternized tertiary amine (N+) of D.

In still other embodiments, the Spacer Unit is represented by the formula: 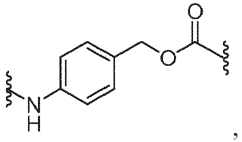 wherein the wavy line adjacent to the nitrogen atom is the point of covalent attachment to RL, as defined above, and the wavy line next to the -OC(O)- group connects to a Drug Unit. In some embodiments, the Drug Unit is attached via a primary or secondary amine.

In some embodiments, provided herein is a Drug-Linker compound of Table 2, or a salt thereof.

**Table 2. Drug-Linker compounds**

| # | **Drug-Linker structure** |
|---|---|
| 2.1 | 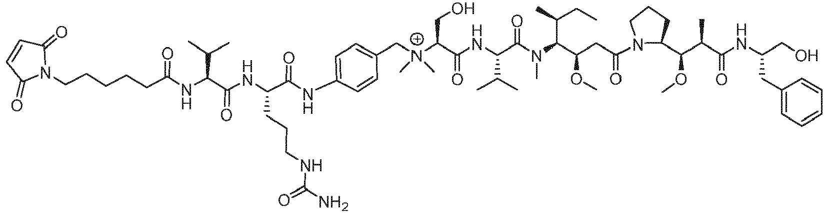 |
| 2.2 | 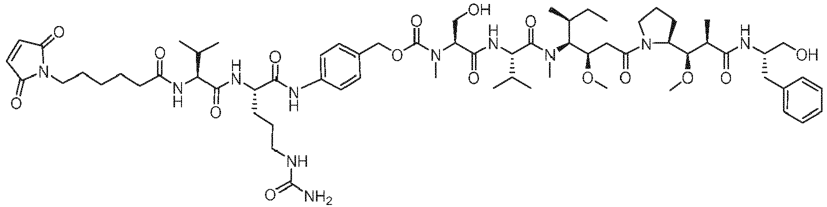 |
| 2.3 | 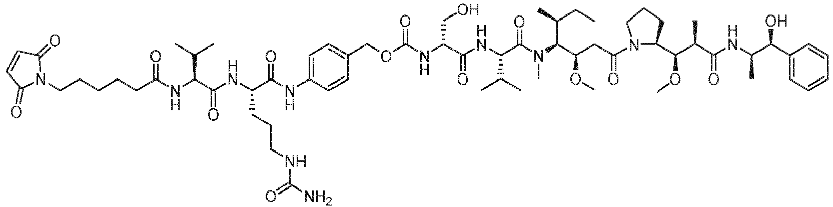 |
| 2.4 | 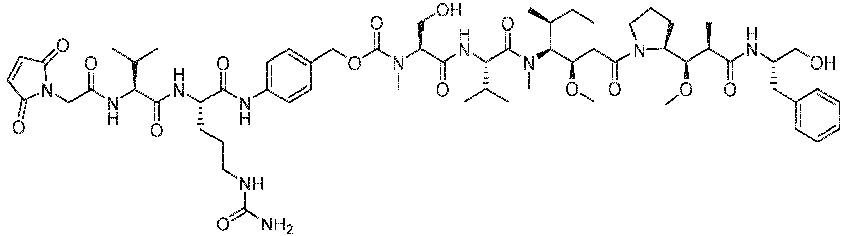 |
| 2.5 | 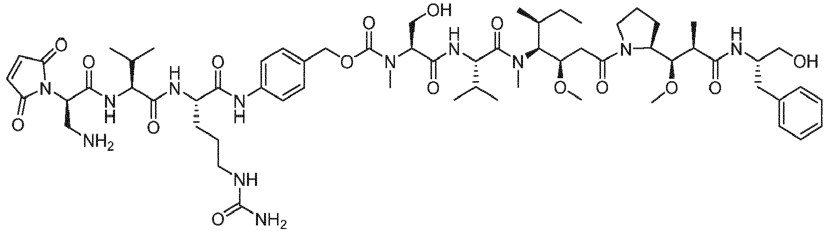 |
| 2.6 | 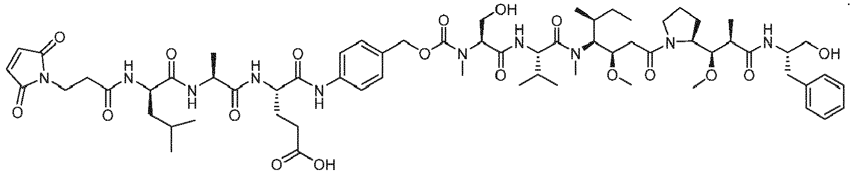 |
| 2.7 | 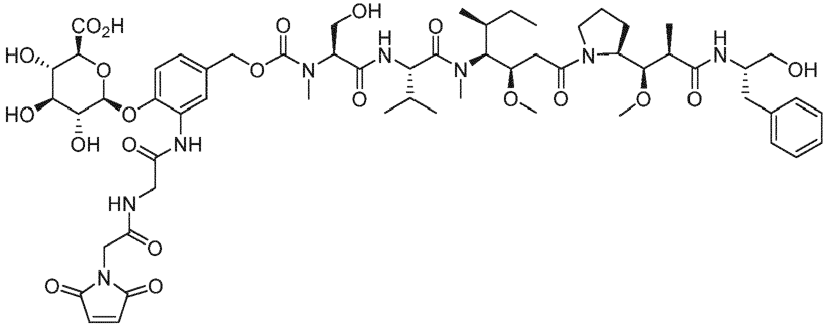 |
| 2.8 | 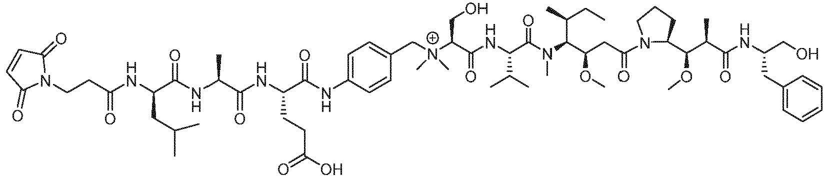 |
| 2.9 | 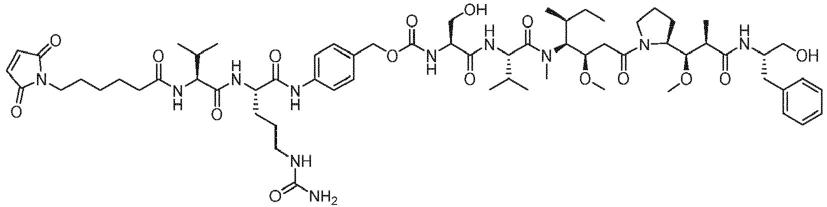 |
| 2.10 | 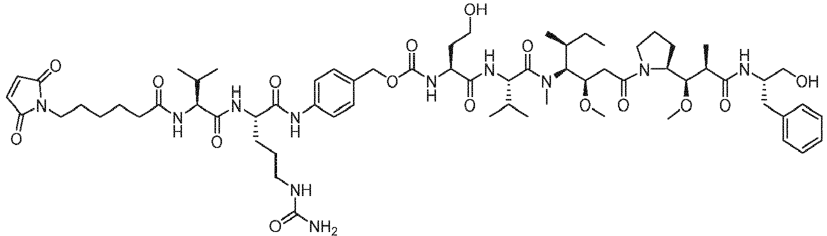 |
| 2.11 | 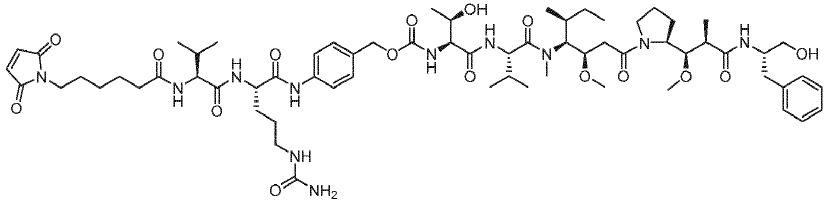 |
| 2.12 | 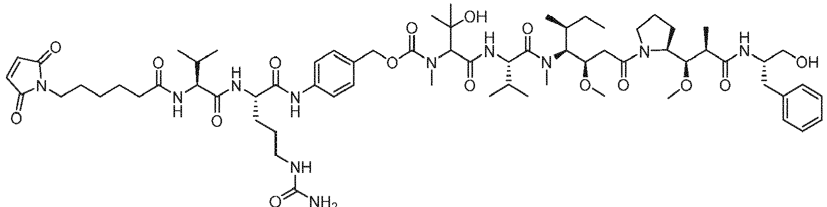 |
| 2.13 | 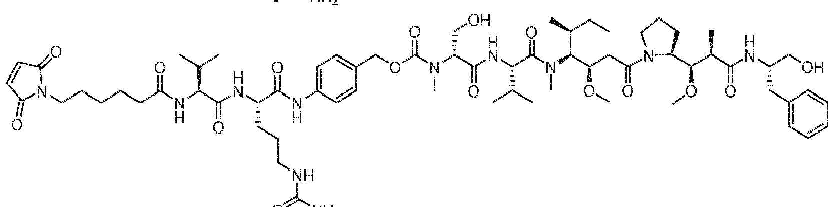 |
| 2.14 | 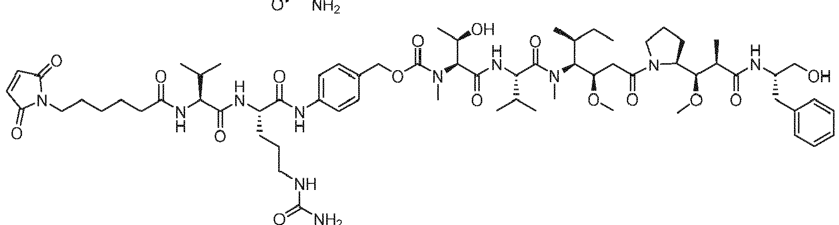 |
| 2.15 | 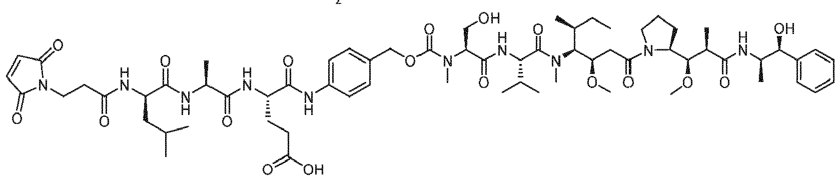 |
| 2.16 | 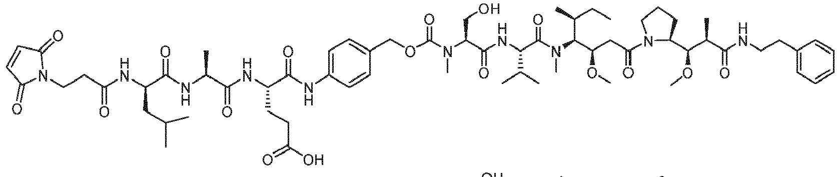 |
| 2.17 | 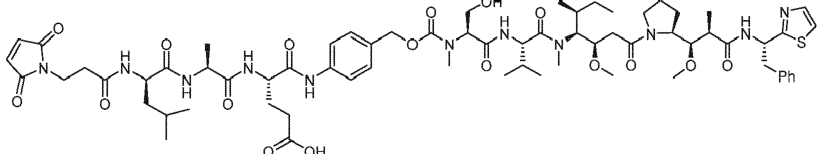 |
| 2.18 | 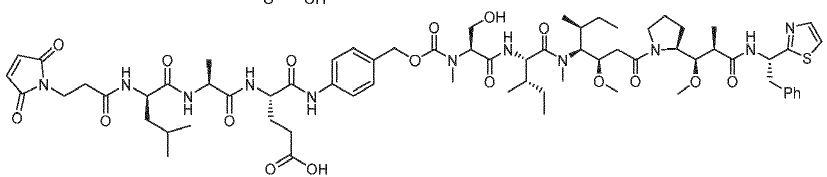 |
| 2.19 | 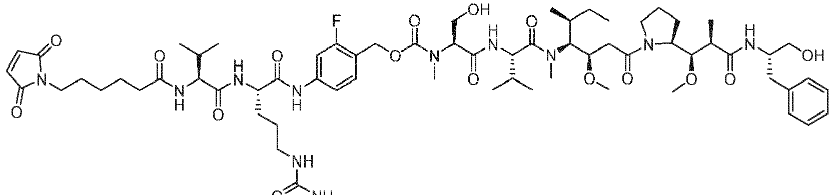 |
| 2.20 | 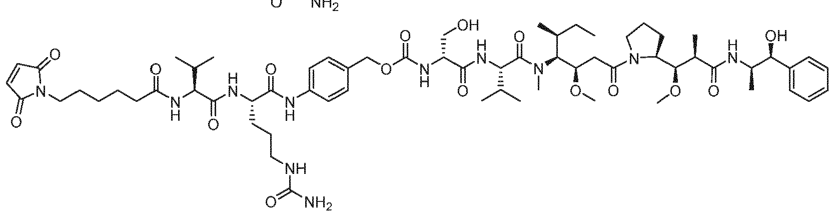 |
| 2.21 | 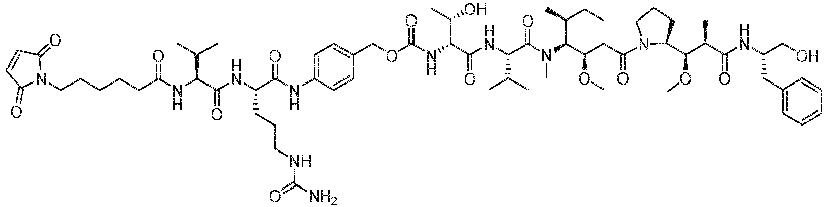 |
| 2.22 | 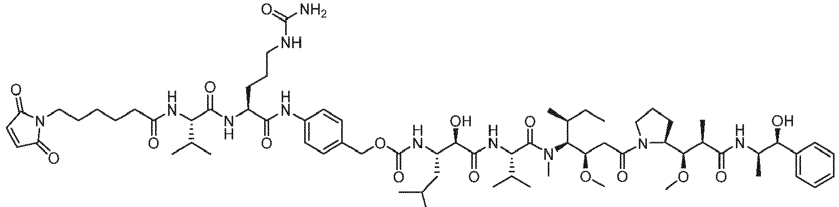 |
| 2.23 | 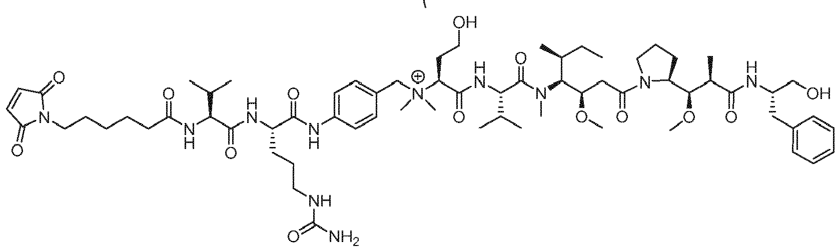 |
| 2.24 | 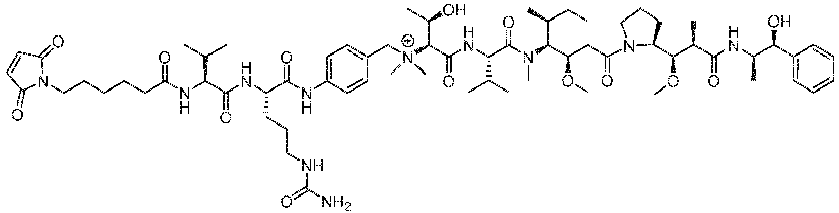 |
| 2.25 | 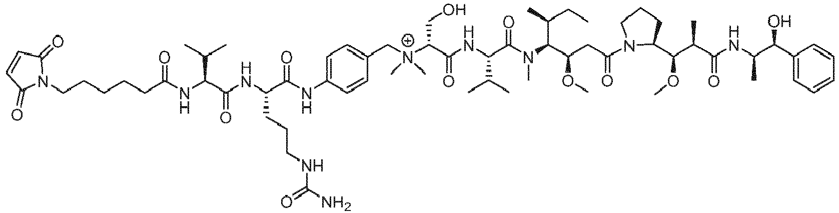 |
| 2.26 | 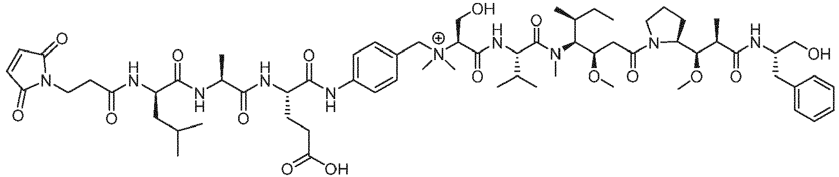 |
| 2.27 | 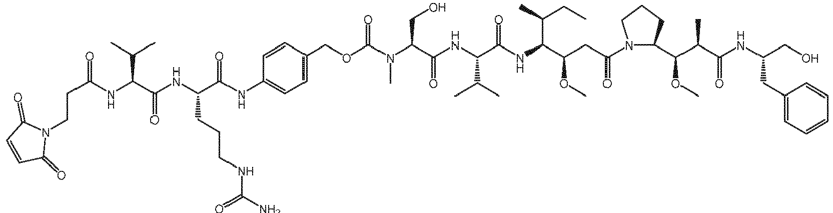 |
| 2.28 | 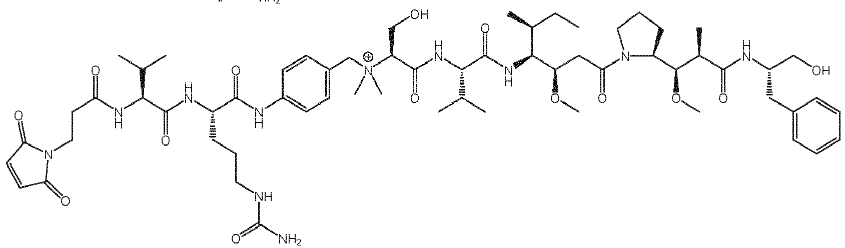 |

### Ligand-Drug Conjugate compounds

In context of Ligand-Drug Conjugate compounds - the assembly is described by the component groups as described for Drug-Linker compounds, with the exception of the Stretcher Unit Z and the Ligand Unit L. The Stretcher Unit Z is coordinated to the Ligand Unit L in Ligand-Drug Conjugate compounds, as described below. While some procedures for the preparation of Ligand-Drug Conjugate compounds are described herein, the order of assembly and the general conditions to prepare the compounds will be well understood by one of skill in the art.

In some embodiments, a Ligand-Drug Conjugate compound comprises an auristatin compound of Formula (I), or any subformula thereof, a Linker Unit (Q) comprising a Releasable Linker (RL) that is other than a Glycoside (e.g., Glucuronide) Unit through which the Ligand Unit is connected to the conjugated auristatin compound, and a Ligand Unit (L). The Linker Unit comprises, in addition to RL, a Stretcher Unit (Z) connected to the Ligand Unit and is capable of (directly or indirectly) connecting the RL to the Ligand Unit. In some embodiments, a Parallel Connector Unit (B) is present when it is desired to add a Partitioning Agent (S^{∗}) as a side chain appendage. In any one of those embodiments, a Connector Unit (A) is present when it is desirable to add more distance between the Stretcher Unit and RL.

In some embodiments, a Ligand-Drug Conjugate compound comprises an auristatin compound of Formula (I), or any subformula thereof, and a Linker Unit (Q), wherein Q comprises a Releasable Linker (RL) that is a Glycoside (e.g., Glucuronide) Unit, directly attached to a Stretcher Unit (Z) or indirectly to Z through attachment to intervening component(s) of the Ligand-Drug Conjugate compound's Linker Unit (i.e., A, S^{∗} and/or B(S^{∗})), wherein Z forms a covalent bond to a targeting agent (*e.g.,* a Ligand Unit).

In another group of embodiments, a Ligand-Drug Conjugate compound comprises an auristatin of Formula (I), or any subformula thereof, a Linker Unit (Q), wherein Q comprises a Releasable Linker (RL) that is other than a Glycoside (e.g., Glucuronide) Unit (RL), directly attached to a Stretcher Unit (Z) or indirectly to Z through attachment to intervening component(s) of the Ligand-Drug Conjugate compound's Linker Unit (i.e., A, S^{∗} and/or B(S^{∗})), wherein Z forms a covalent bond to a targeting agent (*e.g.,* a Ligand Unit).

In some embodiments, the Ligand-Drug Conjugate compound has the formula:

L-(Q-D)ₚ

or a pharmaceutically acceptable salt thereof, wherein
L is a Ligand Unit;
Q is a Linker Unit selected from the group consisting of:
   (i) Z'-A-RL-,
   (ii) Z'-A-RL-Y-,
   (iii) Z'-A-S^{∗}-RL-,
   (iv) Z'-A-S^{∗}-RL-Y-,
   (v) Z'-A-B(S^{∗})-RL-,
   (vi) Z'-A-B(S^{∗})-RL-Y-,
   (vii) Z'-A-,
   (viii) Z'-A-S^{∗}-W-,
   (ix) Z'-A-B(S^{∗})-W-,
   (x) Z'-A-S^{∗}-W-RL-, and
   (xi) Z'-A-B(S^{∗})-W-RL-;
Z' is a Stretcher Unit precursor;
A is a bond or a Connector Unit;
B is a Parallel Connector Unit;
S^{∗} is a Partitioning Agent;
RL is a Releasable Linker;
W is a Amino Acid Unit;
Y is a Spacer Unit; and
D is a Drug Unit of Formula (I'): 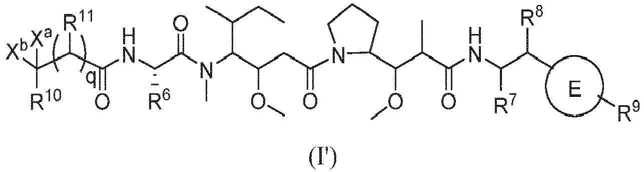 wherein
   X^{b} is -NR²-^{#} or -N⁺R¹R⁵-^{#}, wherein the # denotes the point of attachment to Q, and X^{a} is 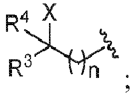 or
   X^{a} and X^{b} are taken together with the carbon atom to which they are attached to form 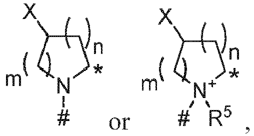 wherein the asterisk denotes the carbon atom of Formula (I) that bears the X^{a} and X^{b} groups, and the # denotes the point of attachment to Q;
   R¹ and R⁵ are independently C₁-C₄ alkyl;
   X is H, OH, -C(O)NR^{a}R^{b}, -S(O)₂R^{a}, -S(O)-R^{a} -S(O)₂NR^{a}R^{b}, -NHS(O)₂R^{a}, or -NHC(O)R^{a};
   R², R³, R⁴, R¹⁰, R^{a}, and R^{b} are each independently H or C₁-C₄ alkyl;
   R⁶ is C₁-C₄ alkyl optionally substituted with OH;
   R⁷ is H, C₁-C₄ alkyl optionally substituted with OH, or 5-6 membered heteroaryl;
   E is phenyl or 5-6 membered heteroaryl;
   R⁸, R⁹, and R¹¹ are each independently H or OH;
   n is 0, 1, 2, or 3;
   m is 1, 2, 3, or 4;
   q is 0 or 1; and
   p is an integer ranging from 1 to 12,
   wherein when X is H, at least two of R⁷, R⁸, R⁹, and R¹¹ comprise an OH moiety.

In some embodiments, provided herein are Ligand-Drug Conjugate compounds of formula L-(Q-D)ₚ, wherein the Drug Unit D has Formula (Iz^{∗}): 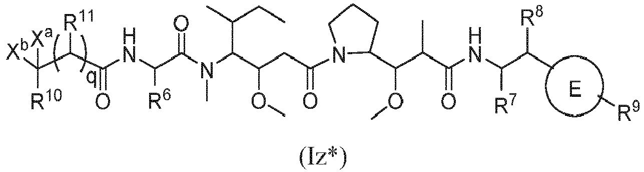 wherein
X^{b} is -NR²-^{#} or -N⁺R¹R⁵-^{#}, wherein the # denotes the point of attachment to Q, and X^{a} is 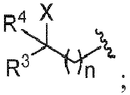 or
X^{a} and X^{b} are taken together with the carbon atom to which they are attached to form 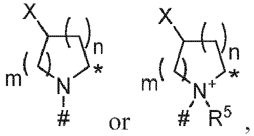 wherein the asterisk denotes the carbon atom of Formula (I) that bears the X^{a} and X^{b} groups, and the # denotes the point of attachment to Q;
R¹ and R⁵ are independently C₁-C₄ alkyl;
X is OH, -S(O)₂R^{a}, -S(O)-R^{a} -S(O)₂NR^{a}R^{b}, -NHS(O)₂R^{a}, or -NHC(O)R^{a};
R², R³, R⁴, R¹⁰, R^{a}, and R^{b} are each independently H or C₁-C₄ alkyl;
R⁶ is C₁-C₄ alkyl optionally substituted with OH;
R⁷ is H, C₁-C₄ alkyl optionally substituted with OH, or 5-6 membered heteroaryl;
E is phenyl or 5-6 membered heteroaryl;
R⁸, R⁹, and R¹¹ are each independently H or OH;
n is 0, 1, 2, or 3;
m is 1, 2, 3, or 4;
q is 0 or 1; and
p is an integer ranging from 1 to 12.

In some embodiments, the Ligand-Drug Conjugate compound has Formula (Ia^{∗}) or (Ia^{∗∗}): 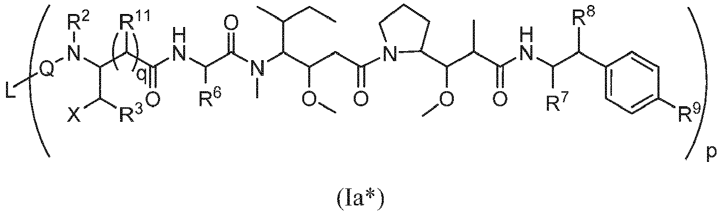 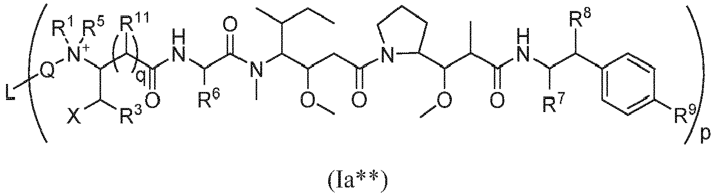 or a salt thereof, wherein
L is a Ligand Unit;
Q is a Linker Unit as described above;
p is an integer ranging from 1 to 12; and
the remaining variables are as described for Drug-Linker moieties of Formula (Ia') or Formula (Ia") above.

In some embodiments, the Ligand-Drug Conjugate compound has Formula (Ib^{∗}) or (Ib^{∗∗}): 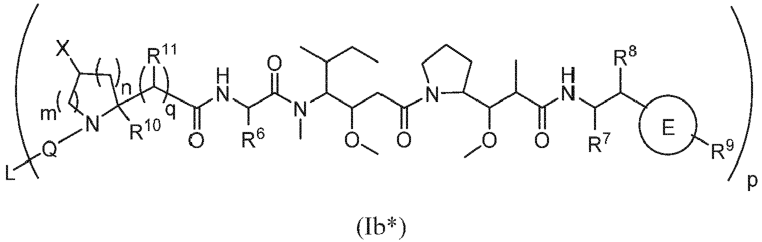 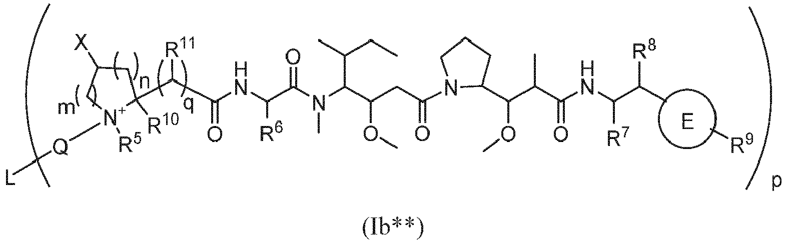 or a salt thereof, wherein
L is a Ligand Unit;
Q is a Linker Unit as described above;
p is an integer ranging from 1 to 12; and
the remaining variables are as described for Drug-Linker moieties of Formula (Ib') or Formula (Ib") above.

In some embodiments, the Ligand-Drug Conjugate compound has Formula (Ic^{∗}) or (Ic^{∗∗}): 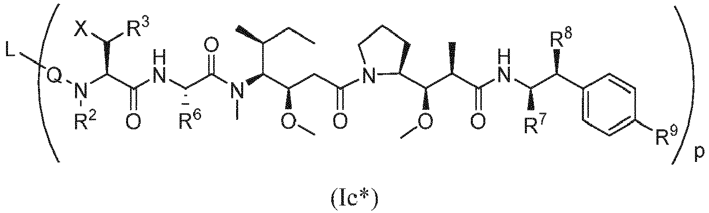 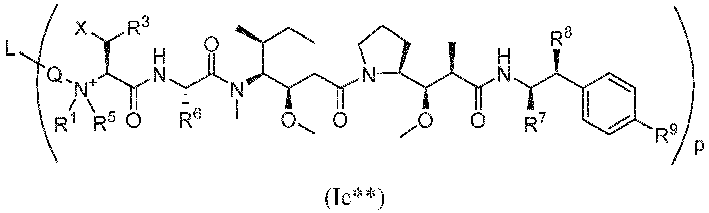 or a salt thereof, wherein
L is a Ligand Unit;
Q is a Linker Unit as described above;
p is an integer ranging from 1 to 12; and
the remaining variables are as described for Drug-Linker moieties of Formula (Ic') or Formula (Ic") above.

In some embodiments, the Ligand-Drug Conjugate compound has Formula (Id^{∗}) or (Id^{∗∗}): 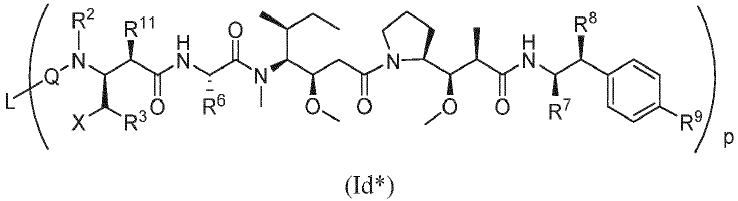 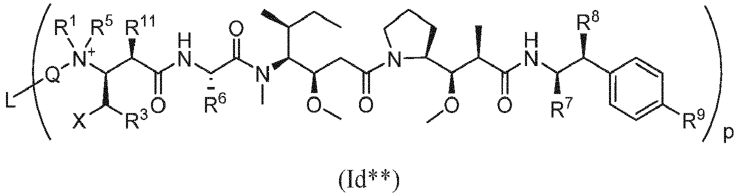 or a salt thereof, wherein
L is a Ligand Unit;
Q is a Linker Unit as described above;
p is an integer ranging from 1 to 12; and
the remaining variables are as described for Drug-Linker moieties of Formula (Id') or Formula (Id") above.

In some embodiments, the Ligand-Drug Conjugate compound has Formula (Ie^{∗}) or (Ie^{∗∗}): 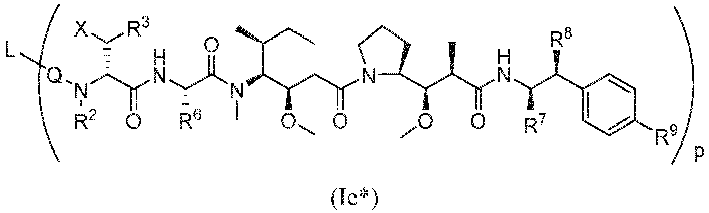 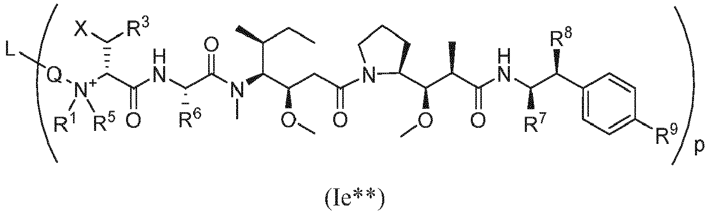 or a salt thereof, wherein
L is a Ligand Unit;
Q is a Linker Unit as described above;
p is an integer ranging from 1 to 12; and
the remaining variables are as described for Drug-Linker moieties of Formula (Ie') or Formula (Ie") above.

In some embodiments, the Ligand-Drug Conjugate compound has Formula (If^{∗}) or (If^{∗∗}): 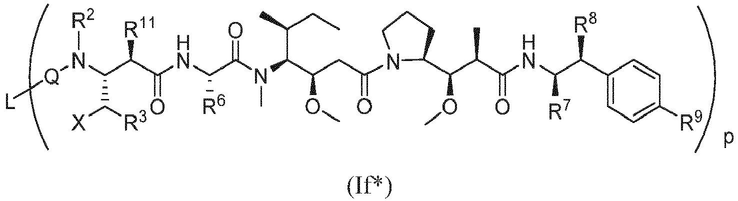 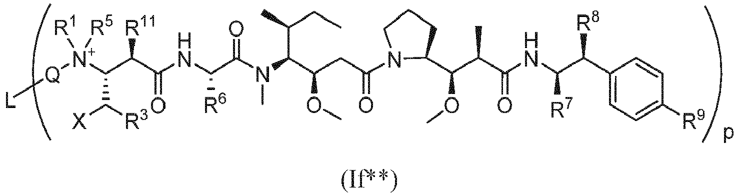 or a salt thereof, wherein
L is a Ligand Unit;
Q is a Linker Unit as described above;
p is an integer ranging from 1 to 12; and
the remaining variables are as described for Drug-Linker moieties of Formula (If') or Formula (If") above.

In the context of the Ligand-Drug Conjugate compounds - the assembly is best described in terms of its component groups. While some procedures for the preparation of Ligand-Drug Conjugate compounds are described herein, the order of assembly and the general conditions to prepare the compounds will be well understood by one of skill in the art. The component groups of the Ligand-Drug Conjugate compounds described are in many cases identical to the component groups for Drug-Linker compounds as described above, including A, B, S^{∗}, RL, W, Y, and D. It is to be understood that embodiments are contemplated wherein the Drug Unit D of the Ligand-Drug Conjugates described herein is conforms to the description of Formula (I'), or any subformula thereof. Other component groups are described below.

### Stretcher Unit Z

Representative Stretcher Units of such embodiments include those having the structures of: 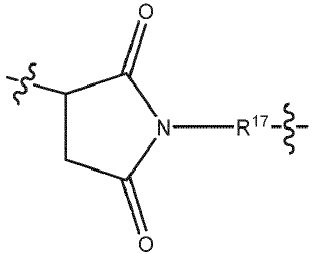 and 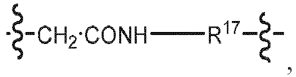 wherein the wavy line adjacent to R¹⁷ indicates attachment to the Parallel Connector Unit (B) or Connector Unit (A) if B is absent, or a Partitioning Agent (S^{∗}), if B is absent, the other wavy line indicates covalent attachment to a sulfur atom of a Ligand Unit, and R¹⁷ is -CH₂CH₂(OCH₂CH₂)ₖ-, -C₁-C₁₀ alkylene-, C₁-C₁₀ heteroalkylene-, -C₃-C₈ carbocyclo-, -O-(C₁-C₈ alkylene)-, -arylene-, -C₁-C₁₀ alkylene-arylene-, -arylene-C₁-C₁₀ alkylene-, -C₁-C₁₀ alkylene-(C₃-C₈ carbocyclo)-, -(C₃-C₈ carbocyclo)-C₁-C₁₀ alkylene-, -C₃-C₈ heterocyclo-, - C₁-C₁₀ alkylene-(C₃-C₈ heterocyclo)-, -(C₃-C₈ heterocyclo)-Ci-Cio alkylene-, -C₁-C₁₀ alkylene-C(=O)-, C₁-C₁₀ heteroalkylene-C(=O)-, -C₃-C₈ carbocyclo-C(=O)-, -O-(C₁-C₈ alkylene)-C(=O)-, -arylene-C(=O)-, -C₁-C₁₀ alkylene-arylene-C(=O)-, -arylene-Ci-Cio alkylene-C(=O)-, -C₁-C₁₀ alkylene-(C₃-C₈ carbocyclo)-C(=O)-, -(C₃-C₈ carbocyclo)-C₁-C₁₀ alkylene-C(=O)-, -C₃-C₈ heterocyclo-C(=O)-, -C₁-C₁₀ alkylene-(C₃-C₈ heterocyclo)-C(=O)-, - (C₃-C₈ heterocyclo)-C₁-C₁₀ alkylene-C(=O)-, -C₁-C₁₀ alkylene-NH-, -C₁-C₁₀ heteroalkylene-NH-, -C₃-C₈ carbocyclo-NH-, -O-(C₁-C₈ alkylene)-NH-, -arylene-NH-, -C₁-C₁₀ alkylene-arylene-NH-, -arylene-Ci-Cio alkylene-NH-, -C₁-C₁₀ alkylene-(C₃-C₈ carbocyclo)-NH-, -(C₃-C₈ carbocyclo)-C₁-C₁₀ alkylene-NH-, -C₃-C₈ heterocyclo-NH-, -C₁-C₁₀ alkylene-(C₃-C₈ heterocyclo)-NH-, -(C₃-C₈ heterocyclo)-Ci-Cio alkylene-NH-, -C₁-C₁₀ alkylene-S-, C₁-C₁₀ heteroalkylene-S -, -C₃-C₈ carbocyclo-S -, -O-(C₁-C₈ alkylene)-S -, -arylene-S-, -C₁-C₁₀ alkylene-arylene-S-, -arylene-C₁-C₁₀ alkylene-S-, -C₁-C₁₀ alkylene-(C₃-C₈ carbocyclo)-S-, - (C₃-C₈ carbocyclo)-C₁-C₁₀ alkylene-S-, -C₃-C₈ heterocyclo-S-, -C₁-C₁₀ alkylene-(C₃-C₈ heterocyclo)-S-, or -(C₃-C₈ heterocyclo)-Ci-Cio alkylene-S-, wherein subscript k is an integer ranging from 1 to 36.

In some embodiments, the R¹⁷ group is optionally substituted by a Basic Unit (BU) such as an aminoalkyl moiety, e.g. -(CH₂)ₓNH₂, -(CH₂ )ₓNHR^{a}, and -(CH₂ )ₓNR^{a}₂, wherein subscript x is an integer of from 1-4 and each R^{a} is independently selected from the group consisting of C₁₋₆ alkyl and C₁₋₆ haloalkyl, or two R^{a} groups are combined with the nitrogen to which they are attached to form an azetidinyl, pyrrolidinyl or piperidinyl group.

An illustrative Stretcher Unit is that of Formula Za or Za-BU in which R¹⁷ is -CH₂CH₂(OCH₂CH₂)ₖ-, -C₁-C₁₀ alkylene-C(=O)-, -C₁-C₁₀ heteroalkylene-C(=O)-, -C₃-C₈ carbocyclo-C(=O)-, -O-(C₁-C₈ alkylene)-C(=O)-, -arylene-C(=O)-, -C₁-C₁₀ alkylene-arylene-C(=O)-, -arylene-C₁-C₁₀ alkylene-C(=O)-, -C₁-C₁₀ alkylene-(C₃-C₈ carbocyclo)-C(=O)-,-(C₃-C₈ carbocyclo)-C₁-C₁₀ alkylene-C(=O)-, -C₃-C₈ heterocyclo-C(=O)-, -C₁-C₁₀ alkylene-(C₃-C₈ heterocyclo)-C(=O)-, or -(C₃-C₈ heterocyclo)-Ci-Cio alkylene-C(=O), wherein subscript k is an integer ranging from 1 to 36: 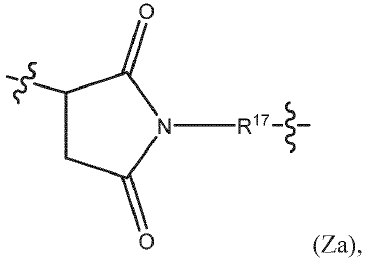 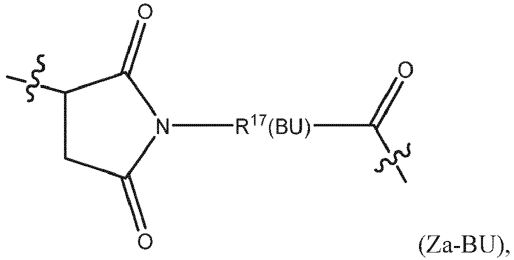 wherein the wavy line adjacent the carbonyl carbon atom indicates attachment to L^{P}, B, A, or S^{∗}, in the formulae above, depending on the presence or absence of A and/or B, and the other wavy line indicates covalent bonding of the succinimide ring carbon atom to a sulfur atom of a Ligand Unit. In some aspects, the basic amino functional group of the Basic Unit (BU) is protected by a protecting group during synthesis.

In some embodiments, Stretcher Units of formula Za and Za-BU are as follows: 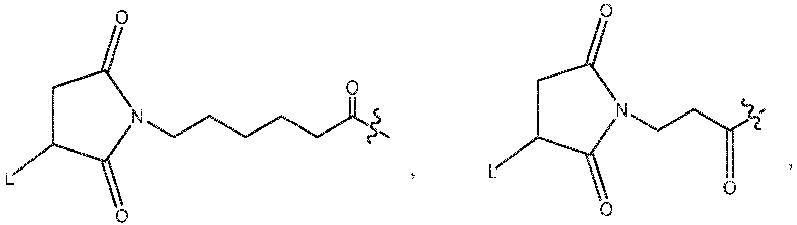 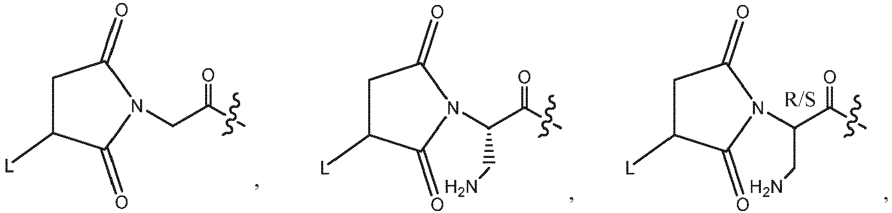 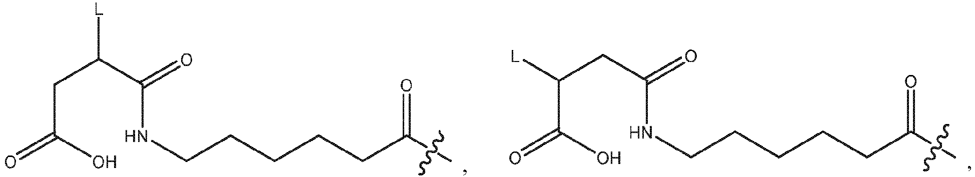 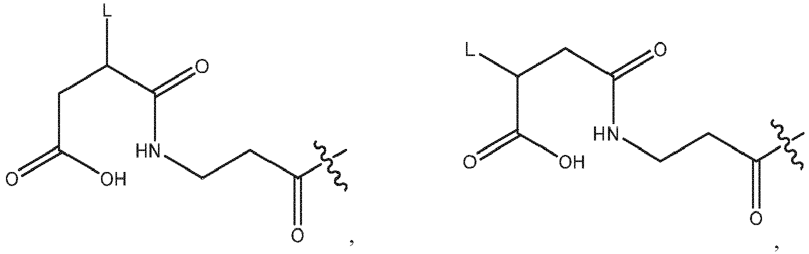 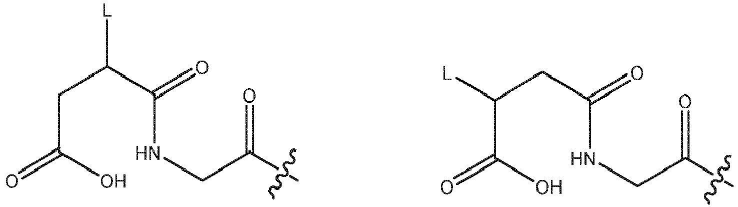 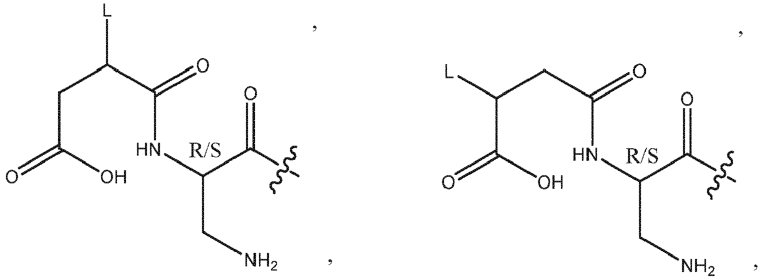 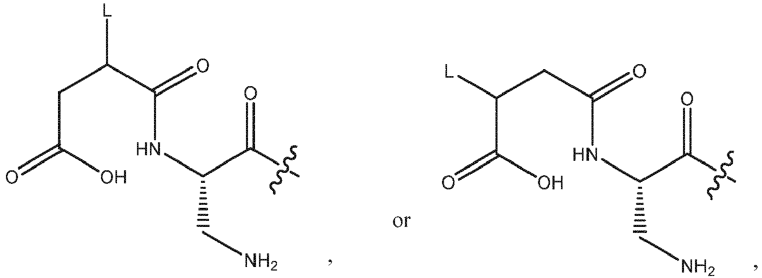 wherein the wavy line adjacent the carbonyl carbon atom indicates attachment to B, A, or S^{∗}, in the formulae above, depending on the presence or absence of A and/or B, and the other wavy line indicates covalent bonding of the succinimide ring carbon atom to a sulfur atom of a Ligand Unit.

It will be understood that a Ligand Unit-substituted succinimide may exist in hydrolyzed form(s). Those forms are exemplified below for hydrolysis of Za or Za-BU, wherein the structures representing the regioisomers from that hydrolysis have formula Zb and Zc or Zb-BU and Zc-BU.

Accordingly, in some embodiments, a Stretcher unit (Z) comprises a succinic acid-amide moiety represented by the following: 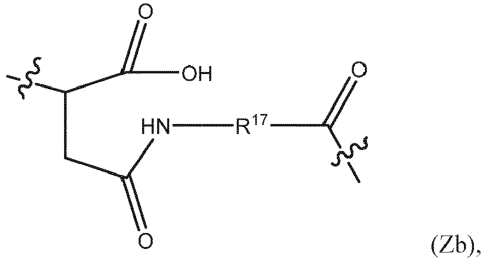 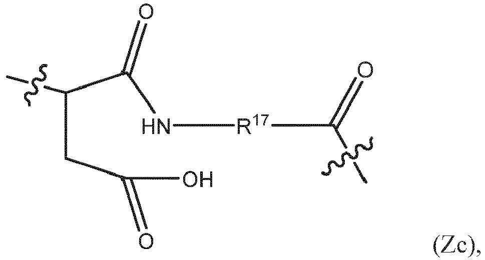 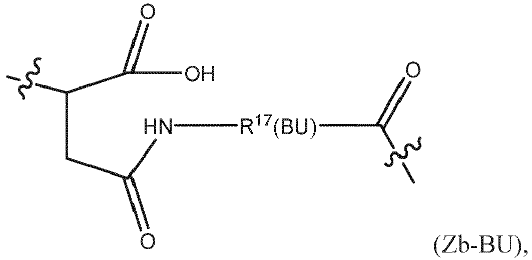 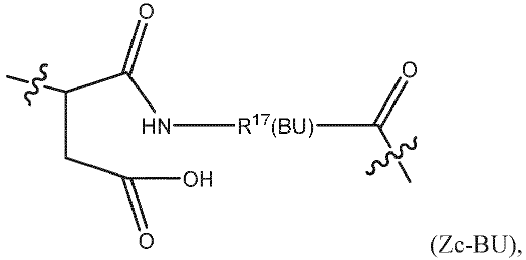 wherein the wavy line adjacent to the carbonyl carbon atom bonded to R¹⁷ and the wavy line adjacent to the carbon atom of the succinic acid-amide moiety is as defined for Za or Za-BU, depending on the presence or absence of A and/or B; and R¹⁷ is -C₁-C₅ alkylene-, wherein in Zb-BU and Zc-BU the alkylene is substituted by a Basic Unit (BU), wherein BU is -(CH₂)ₓNH₂, -(CH₂)ₓNHR^{a}, or -(CH₂)ₓN(R^{a})₂, wherein subscript x is an integer of from 1-4 and each R^{a} is independently selected from the group consisting of C₁₋₆ alkyl and C₁₋₆ haloalkyl, or both R^{a} together with the nitrogen to which they are attached define an azetidinyl, pyrrolidinyl or piperidinyl group.

In some embodiments, -Z-A- comprises a moiety derived from a maleimido-alkanoic acid moiety or an mDPR moiety. See, for example, see WO 2013/173337. In one group of embodiments, Z-A- is derived from a maleimido-propionyl moiety.

In some embodiments, a Stretcher unit (Z) comprises an succinic acid-amide moiety represented by the structure of formula Zb', Zc', (R/S)-Zb'-BU, (S)-Zb'-BU, (R/S)-Zc'-BU or (S)-Zc'-BU as follows: 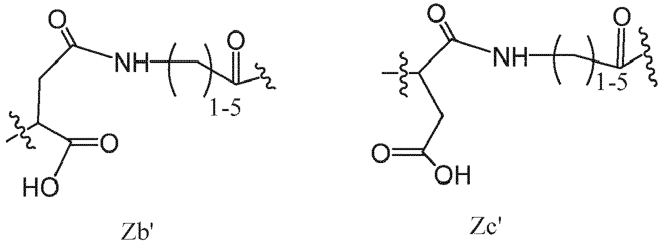 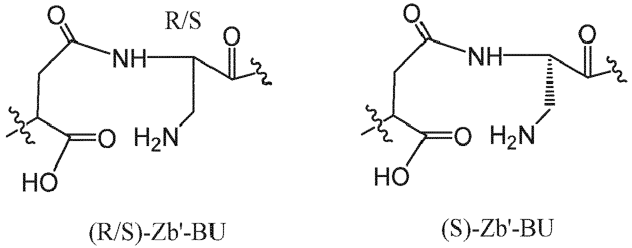 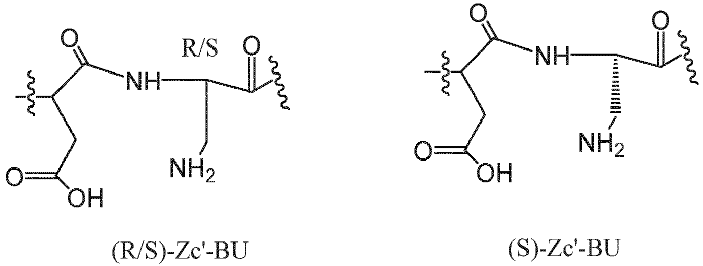 wherein the wavy lines are as defined for Za or Za-BU.

In some embodiments, a Stretcher unit (Z) comprises a succinimide moiety represented by the structure of 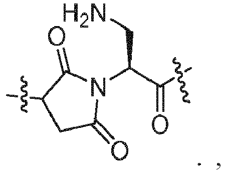 which may be generated from a maleimido-amino-propionyl (mDPR) analog (a 3-amino-2-(2,5-dioxo-2,5-dihydro-1H-pyrrol-1-yl)propanoic acid derivative), or comprises a succinic acid-amide moiety represented by the structure of:

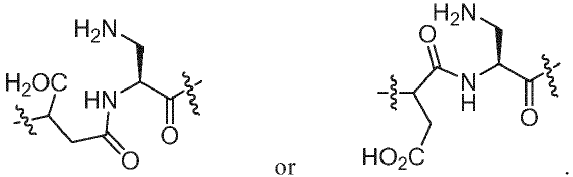

Illustrative Stretcher Units bonded to a Connector Unit (A) which comprise Za', Zb' or Zc', in which -R¹⁷- of Za, Zb or Zc is -CH₂- or -CH₂CH₂-, or comprise Za'-BU, Zb'-BU or Zc'-BU in which -R¹⁷(BU)- of Za'-BU, Zb'-BU or Zc'-BU is -CH(CH₂NH₂)-, have the following structures: 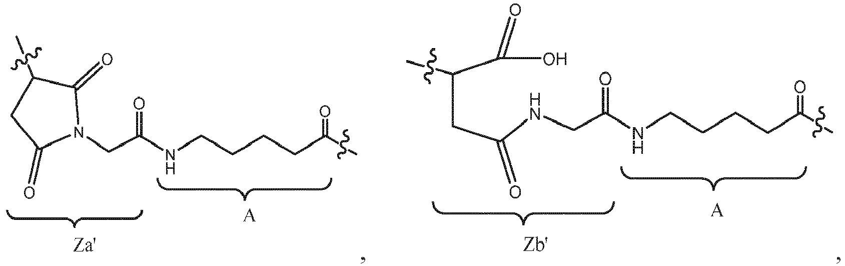 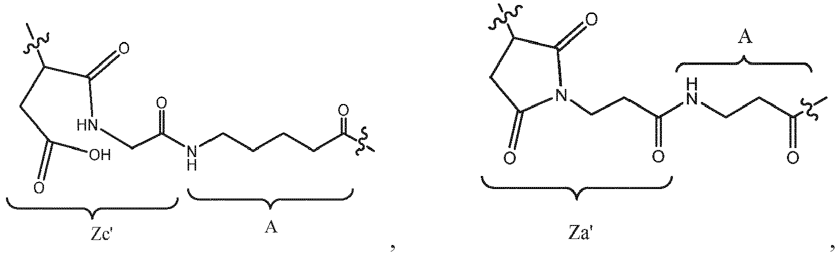 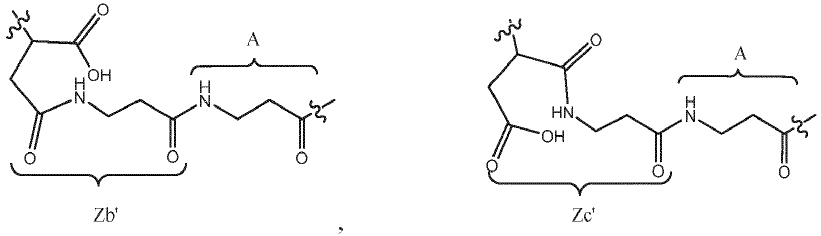 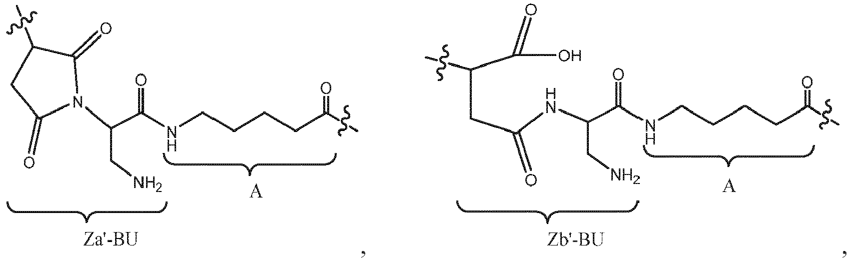 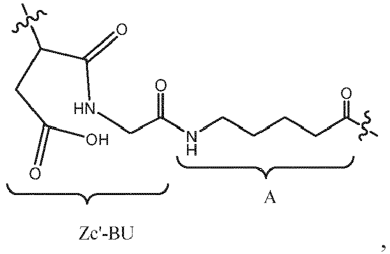 wherein the wavy lines are as defined for Za or Za-BU.

Other Stretcher Units bonded to a Ligand Unit (L) and a Connector Unit (A) have the structures above wherein A in any one of the above -Za'-A-, -Za'(BU)-A-, -Za'-A-, - Za'(BU)-A-, -Zb'-A-, -Zb'(BU)-A-, -Zb'-A-, -Zb'(BU)-, -Zc'-A- and Zc'(BU)-A- structures is replaced by a Parallel Connector Unit having the structure of: 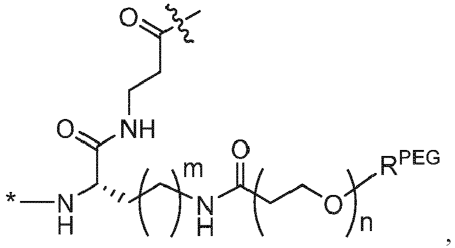 wherein subscript m ranges from 1 to 6; n ranges from 8 to 24; R^{PEG} is a PEG Capping Unit, preferably H, -CH₃, or -CH₂CH₂CO₂H, the asterisk (^{∗}) indicates covalent attachment to a Stretcher Unit corresponding in structure to formula Za, Za', Zb' or Zc' and the wavy line indicates covalent attachment to the Releasable Linker (RL).

In another embodiment, the Stretcher Unit is attached to the Ligand Unit via a disulfide bond between a sulfur atom of the Ligand Unit and a sulfur atom of the Stretcher unit. A representative Stretcher Unit of this embodiment is depicted within the square brackets of Formula Zb: 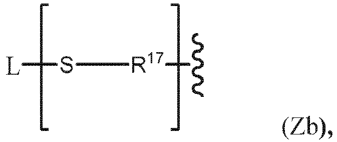 wherein the wavy line indicates attachment to the Parallel Connector Unit (B) or Connector Unit (A) if B is absent or a Partitioning Agent (S^{∗}), if A and B are absent and R¹⁷ is -CH₂CH₂(OCH₂CH₂)ₖ-, -C₁-C₁₀ alkylene-, C₁-C₁₀ heteroalkylene-, -C₃-C₈ carbocyclo-, -O-(C₁-C₈ alkylene)-, -arylene-, -C₁-C₁₀ alkylene-arylene-, -arylene-C₁-C₁₀ alkylene-, -C₁-C₁₀ alkylene-(C₃-C₈ carbocyclo)-, -(C₃-C₈ carbocyclo)-C₁-C₁₀ alkylene-, -C₃-C₈ heterocyclo-, - C₁-C₁₀ alkylene-(C₃-C₈ heterocyclo)-, -(C₃-C₈ heterocyclo)-Ci-Cio alkylene-, -C₁-C₁₀ alkylene-C(=O)-, C₁-C₁₀ heteroalkylene-C(=O)-, -C₃-C₈ carbocyclo-C(=O)-, -O-(C₁-C₈ alkylene)-C(=O)-, -arylene-C(=O)-, -C₁-C₁₀ alkylene-arylene-C(=O)-, -arylene-Ci-Cio alkylene-C(=O)-, -C₁-C₁₀ alkylene-(C₃-C₈ carbocyclo)-C(=O)-,-(C₃-C₈ carbocyclo)-C₁-C₁₀ alkylene-C(=O)-, -C₃-C₈ heterocyclo-C(=O)-, -C₁-C₁₀ alkylene-(C₃-C₈ heterocyclo)-C(=O)-, - (C₃-C₈ heterocyclo)-Ci-Cio alkylene-C(=O)-, -C₁-C₁₀ alkylene-NH-, C₁-C₁₀ heteroalkylene-NH-, -C₃-C₈ carbocyclo-NH-, -O-(C₁-C₈ alkylene)-NH-, -arylene-NH-, -C₁-C₁₀ alkylene-arylene-NH-, -arylene-Ci-Cio alkylene-NH-, -C₁-C₁₀ alkylene-(C₃-C₈ carbocyclo)-NH-, -(C₃-C₈ carbocyclo)-C₁-C₁₀ alkylene-NH-, -C₃-C₈ heterocyclo-NH-, -C₁-C₁₀ alkylene-(C₃-C₈ heterocyclo)-NH-, -(C₃-C₈ heterocyclo)-Ci-Cio alkylene-NH-, -C₁-C₁₀ alkylene-S-, C₁-C₁₀ heteroalkylene-S -, -C₃-C₈ carbocyclo-S -, -O-(C₁-C₈ alkylene)-S -, -arylene-S-, -C₁-C₁₀ alkylene-arylene-S-, -arylene-C₁-C₁₀ alkylene-S-, -C₁-C₁₀ alkylene-(C₃-C₈ carbocyclo)-S-, - (C₃-C₈ carbocyclo)-C₁-C₁₀ alkylene-S-, -C₃-C₈ heterocyclo-S-, -C₁-C₁₀ alkylene-(C₃-C₈ heterocyclo)-S-, or -(C₃-C₈ heterocyclo)-Ci-Cio alkylene-S-, wherein subscript k is an integer ranging from 1 to 36.

In yet another embodiment, the reactive group of a Stretcher Unit precursor contains a reactive site that can form a bond with a primary or secondary amino group of a Ligand Unit. Examples of these reactive sites include, but are not limited to, activated esters such as succinimide esters, 4-nitrophenyl esters, pentafluorophenyl esters, tetrafluorophenyl esters, anhydrides, acid chlorides, sulfonyl chlorides, isocyanates and isothiocyanates. Representative Stretcher Units of this embodiment are depicted within the square brackets of Formulas Zci, Zcii and Zciii: 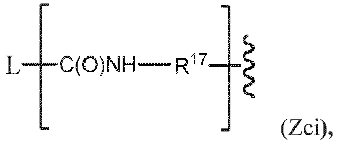 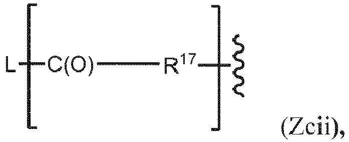 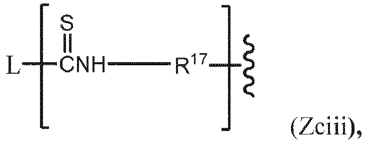 wherein the wavy line indicates attachment to the Parallel Connector Unit (B) or Connector Unit (A) if B is absent or a Partitioning Agent (S^{∗}), if A and B are absent and R¹⁷ is -CH₂CH₂(OCH₂CH₂)ₖ-, -C₁-C₁₀ alkylene-, C₁-C₁₀ heteroalkylene-, -C₃-C₈ carbocyclo-, -O-(C₁-C₈ alkylene)-, -arylene-, -C₁-C₁₀ alkylene-arylene-, -arylene-C₁-C₁₀ alkylene-, -C₁-C₁₀ alkylene-(C₃-C₈ carbocyclo)-, -(C₃-C₈ carbocyclo)-C₁-C₁₀ alkylene-, -C₃-C₈ heterocyclo-, - C₁-C₁₀ alkylene-(C₃-C₈ heterocyclo)-, -(C₃-C₈ heterocyclo)-Ci-Cio alkylene-, -C₁-C₁₀ alkylene-C(=O)-, C₁-C₁₀ heteroalkylene-C(=O)-, -C₃-C₈ carbocyclo-C(=O)-, -O-(C₁-C₈ alkylene)-C(=O)-, -arylene-C(=O)-, -C₁-C₁₀ alkylene-arylene-C(=O)-, -arylene-Ci-Cio alkylene-C(=O)-, -C₁-C₁₀ alkylene-(C₃-C₈ carbocyclo)-C(=O)-,-(C₃-C₈ carbocyclo)-C₁-C₁₀ alkylene-C(=O)-, -C₃-C₈ heterocyclo-C(=O)-, -C₁-C₁₀ alkylene-(C₃-C₈ heterocyclo)-C(=O)-, - (C₃-C₈ heterocyclo)-C₁-C₁₀ alkylene-C(=O)-, -C₁-C₁₀ alkylene-NH-, C₁-C₁₀ heteroalkylene-NH-, -C₃-C₈ carbocyclo-NH-, -O-(C₁-C₈ alkylene)-NH-, -arylene-NH-, -C₁-C₁₀ alkylene-arylene-NH-, -arylene-Ci-Cio alkylene-NH-, -C₁-C₁₀ alkylene-(C₃-C₈ carbocyclo)-NH-, -(C₃-C₈ carbocyclo)-C₁-C₁₀ alkylene-NH-, -C₃-C₈ heterocyclo-NH-, -C₁-C₁₀ alkylene-(C₃-C₈ heterocyclo)-NH-, -(C₃-C₈ heterocyclo)-Ci-Cio alkylene-NH-, -C₁-C₁₀ alkylene-S-, C₁-C₁₀ heteroalkylene-S -, -C₃-C₈ carbocyclo-S -, -O-(C₁-C₈ alkylene)-S -, -arylene-S-, -C₁-C₁₀ alkylene-arylene-S-, -arylene-C₁-C₁₀ alkylene-S-, -C₁-C₁₀ alkylene-(C₃-C₈ carbocyclo)-S-, - (C₃-C₈ carbocyclo)-C₁-C₁₀ alkylene-S-, -C₃-C₈ heterocyclo-S-, -C₁-C₁₀ alkylene-(C₃-C₈ heterocyclo)-S-, or -(C₃-C₈ heterocyclo)-Ci-Cio alkylene-S-, wherein subscript k is an integer ranging from 1 to 36.

In still other embodiments, the reactive group of the Stretcher Unit precursor contains a reactive nucleophile that is capable of reacting with an electrophile present on, or introduced to, a Ligand Unit. For example, in some aspects, a carbohydrate moiety on a targeting ligand is mildly oxidized using a reagent such as sodium periodate and the resulting electrophilic functional group (-CHO) of the oxidized carbohydrate is condensed with a Stretcher Unit precursor that contains a reactive nucleophile such as a hydrazide, an oxime, a primary or secondary amine, a hydrazine, a thiosemicarbazone, a hydrazine carboxylate, or an arylhydrazide such as those described by Kaneko, T. et al. (1991) Bioconjugate Chem. 2:133-41. Representative Stretcher Units of this embodiment are depicted within the square brackets of Formulas Zdi, Zdii, and Zdiii:

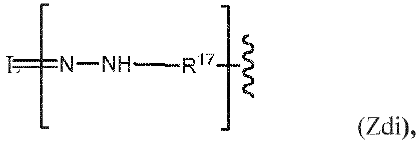

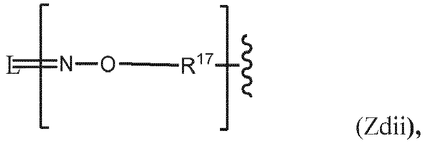

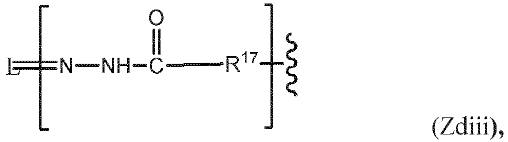

wherein the wavy line indicates attachment to the Parallel Connector Unit (B) or Connector Unit (A), or a Partitioning Agent (S^{∗}), if A and B are absent and R¹⁷ is -CH₂CH₂(OCH₂CH₂)ₖ-, -C₁-C₁₀ alkylene-, C₁-C₁₀ heteroalkylene-, -C₃-C₈ carbocyclo-, -O-(C₁-C₈ alkylene)-, -arylene-, -C₁-C₁₀ alkylene-arylene-, -arylene-C₁-C₁₀ alkylene-, -C₁-C₁₀ alkylene-(C₃-C₈ carbocyclo)-, -(C₃-C₈ carbocyclo)-C₁-C₁₀ alkylene-, -C₃-C₈ heterocyclo-, - C₁-C₁₀ alkylene-(C₃-C₈ heterocyclo)-, -(C₃-C₈ heterocyclo)-Ci-Cio alkylene-, -C₁-C₁₀ alkylene-C(=O)-, C₁-C₁₀ heteroalkylene-C(=O)-, -C₃-C₈ carbocyclo-C(=O)-, -O-(C₁-C₈ alkylene)-C(=O)-, -arylene-C(=O)-, -C₁-C₁₀ alkylene-arylene-C(=O)-, -arylene-C₁-C₁₀ alkylene-C(=O)-, -C₁-C₁₀ alkylene-(C₃-C₈ carbocyclo)-C(=O)-,-(C₃-C₈ carbocyclo)-C₁-C₁₀ alkylene-C(=O)-, -C₃-C₈ heterocyclo-C(=O)-, -C₁-C₁₀ alkylene-(C₃-C₈ heterocyclo)-C(=O)-, - (C₃-C₈ heterocyclo)-C₁-C₁₀ alkylene-C(=O)-, -C₁-C₁₀ alkylene-NH-, C₁-C₁₀ heteroalkylene-NH-, -C₃-C₈ carbocyclo-NH-, -O-(C₁-C₈ alkylene)-NH-, -arylene-NH-, -C₁-C₁₀ alkylene-arylene-NH-, -arylene-Ci-Cio alkylene-NH-, -C₁-C₁₀ alkylene-(C₃-C₈ carbocyclo)-NH-, -(C₃-C₈ carbocyclo)-C₁-C₁₀ alkylene-NH-, -C₃-C₈ heterocyclo-NH-, -C₁-C₁₀ alkylene-(C₃-C₈ heterocyclo)-NH-, -(C₃-C₈ heterocyclo)-Ci-Cio alkylene-NH-, -C₁-C₁₀ alkylene-S-, C₁-C₁₀ heteroalkylene-S -, -C₃-C₈ carbocyclo-S -, -O-(C₁-C₈ alkylene)-S -, -arylene-S-, -C₁-C₁₀ alkylene-arylene-S-, -arylene-C₁-C₁₀ alkylene-S-, -C₁-C₁₀ alkylene-(C₃-C₈ carbocyclo)-S-, - (C₃-C₈ carbocyclo)-C₁-C₁₀ alkylene-S-, -C₃-C₈ heterocyclo-S-, -C₁-C₁₀ alkylene-(C₃-C₈ heterocyclo)-S-, or -(C₃-C₈ heterocyclo)-Ci-Cio alkylene-S-, wherein subscript k is an integer ranging from 1 to 36.

In some embodiments, provided herein is a Ligand-Drug Conjguate compound of Table 3, or a pharmaceutically acceptable salt thereof. Ligand-Drug Conjugate compounds corresponding to the compounds of Table 3, but having a succinic acid-amide moiety in place of the succinimide moiety are contemplated.

**Table 3. Ligand-Drug Conjugate compounds**

| # | **Ligand-Drug Conjugate compound structure** |
|---|---|
| 3.1 | 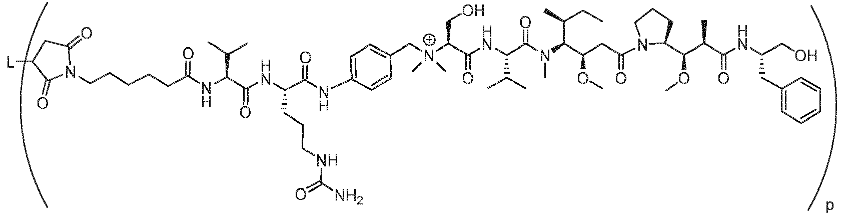 |
| 3.2 | 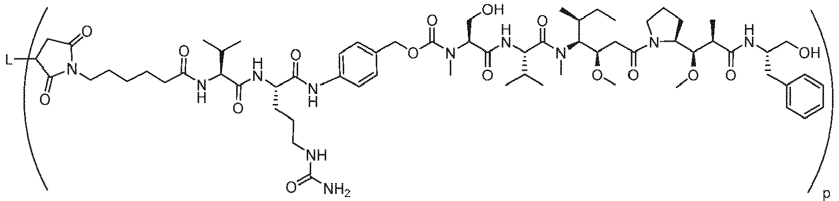 |
| 3.3 | 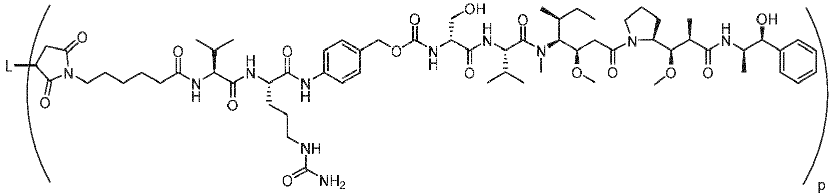 |
| 3.4 | 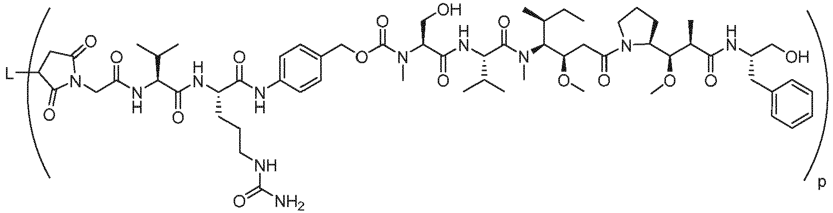 |
| 3.5 | 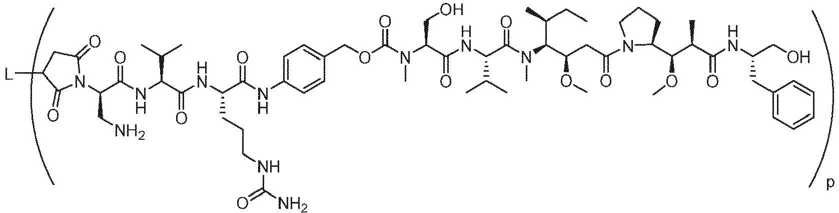 |
| 3.6 | 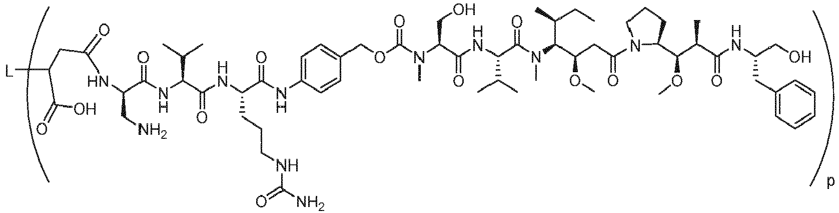 |
| 3.7 | 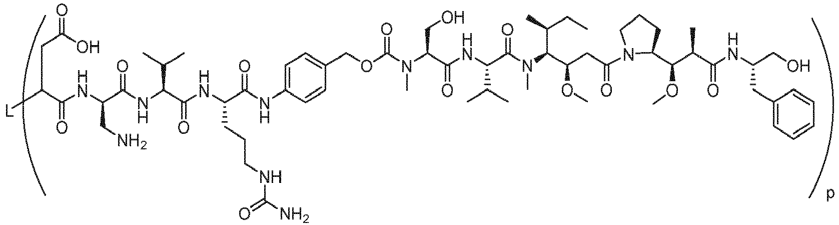 |
| 3.8 | 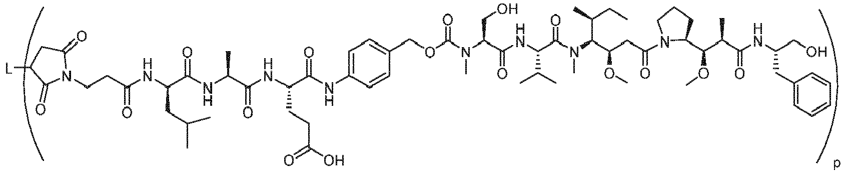 |
| 3.9 | 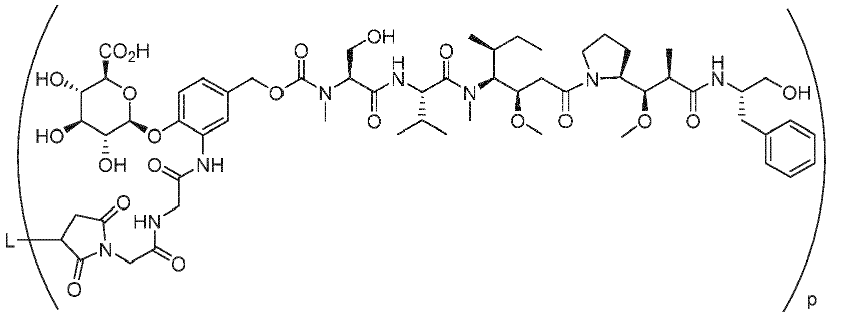 |
| 3.10 | 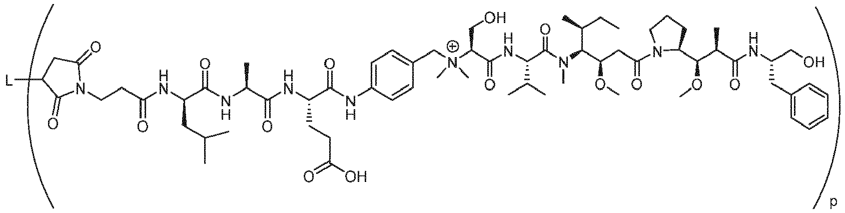 |
| 3.11 | 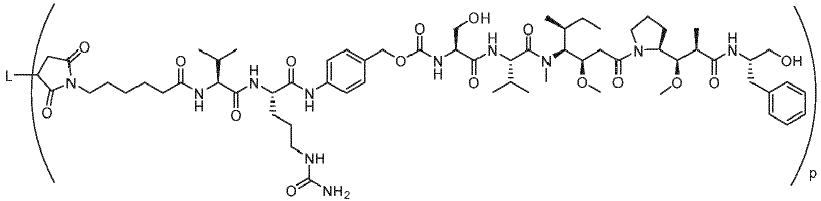 |
| 3.12 | 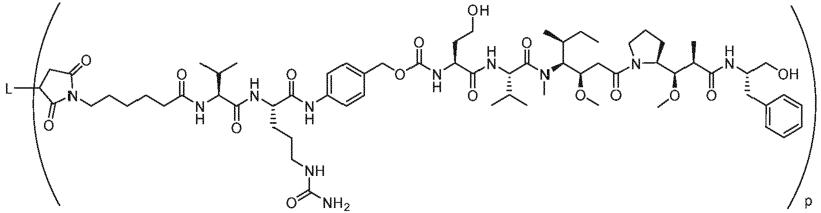 |
| 3.13 | 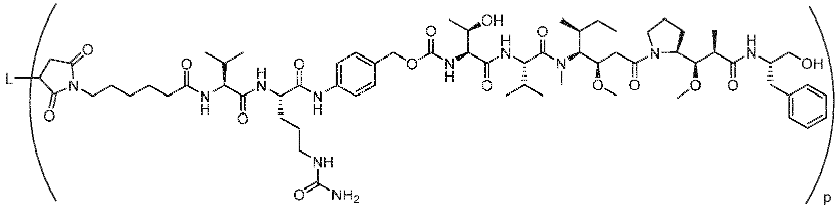 |
| 3.14 | 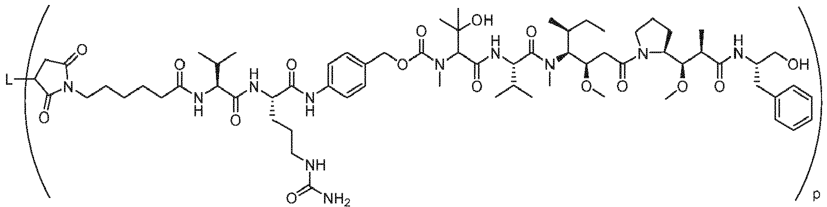 |
| 3.15 | 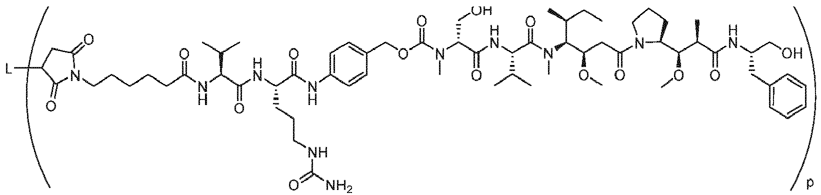 |
| 3.16 | 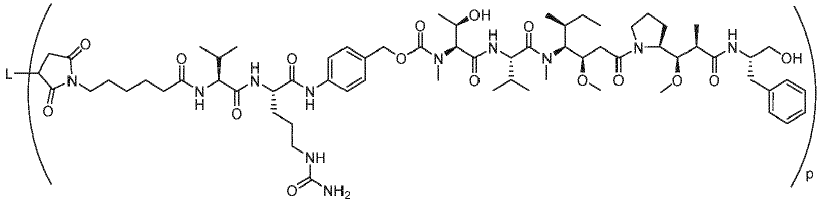 |
| 3.17 | 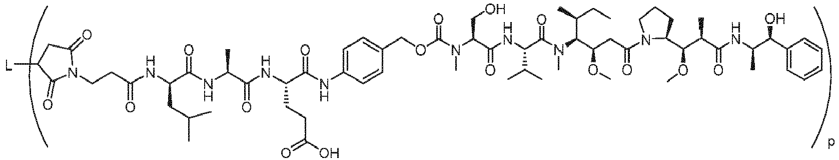 |
| 3.18 | 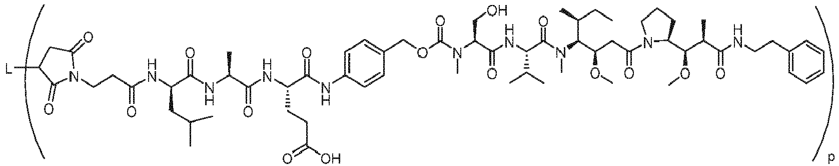 |
| 3.19 | 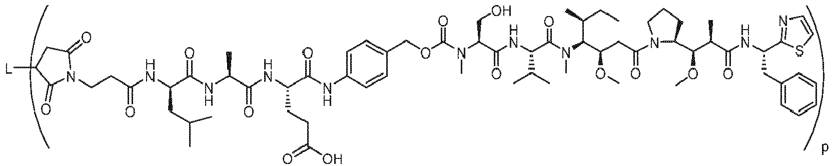 |
| 3.20 | 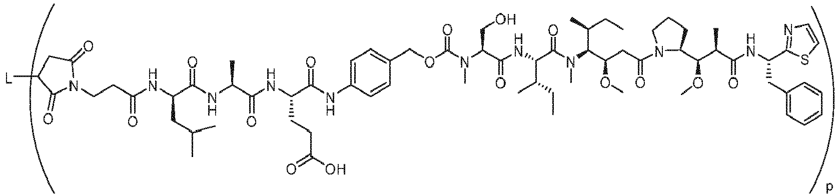 |
| 3.21 | 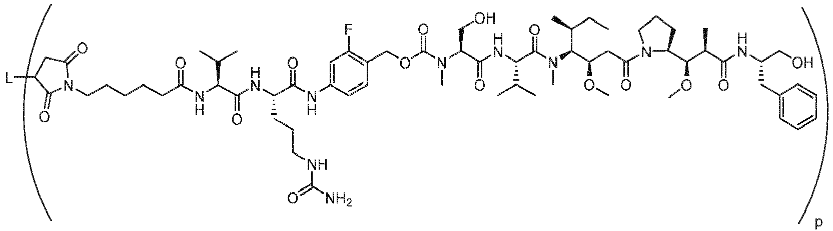 |
| 3.22 | 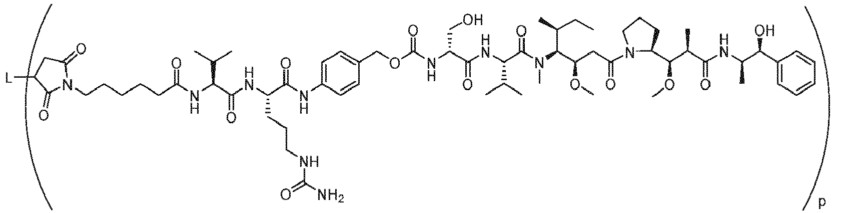 |
| 3.23 | 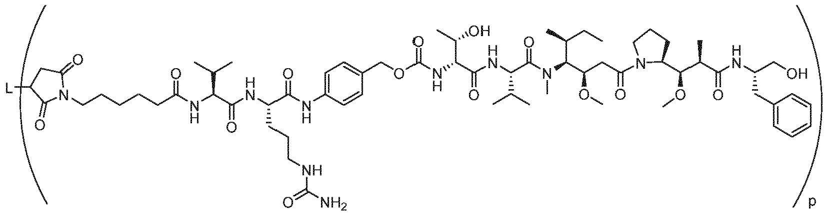 |
| 3.24 | 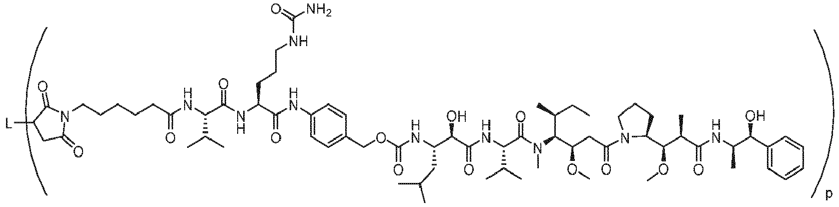 |
| 3.25 | 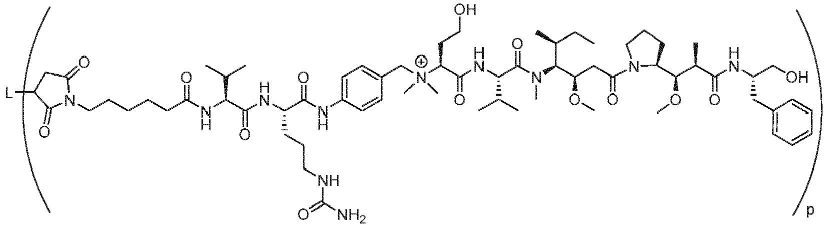 |
| 3.26 | 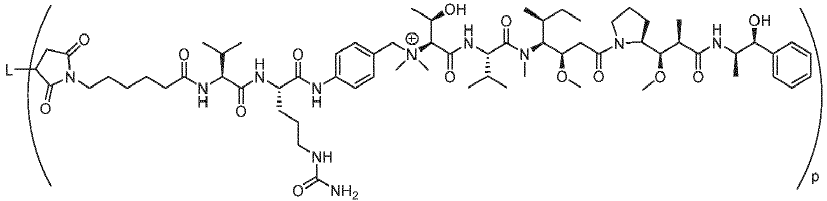 |
| 3.27 | 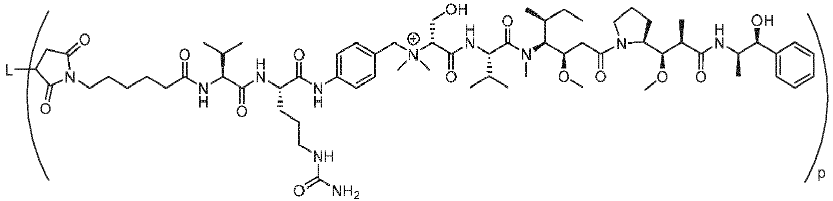 |
| 3.28 | 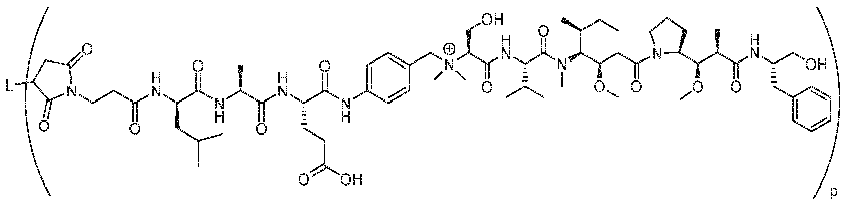 |
| 3.29 | 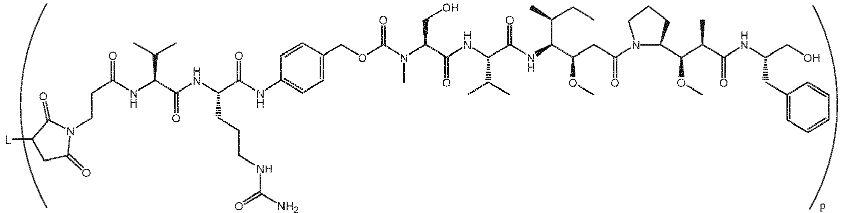 |
| 3.30 | 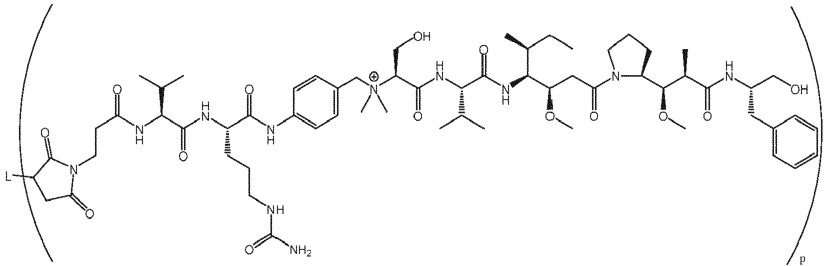 |

### Subscript p

Subscript p represents the number of Drug Linker moieties on a Ligand Unit of an individual Ligand-Drug Conjugate compound and is an integer ranging from 1 to 16, 1 to 12, 1 to 10, or 1 to 8. In any of the embodiments herein, there are 1, 2, 3, 4, 5, 6, 7, 8, 9, 10, 11, 12, 13, 14, 15, or 16 Drug Linker moieties conjugated to a Ligand Unit of an individual Ligand-Drug Conjugate compound.

In some embodiments, any of the structures and descriptions as described herein represent a population of individual Ligand-Drug Conjugate compounds substantially identical except for the number of Drug-Linker moieties bound to each Ligand Unit (i.e., a Ligand-Drug Conjugate composition) so that subscript p represents the average number of Drug-Linker moieties bound to the Ligand Units of the Ligand-Drug Conjugate composition. In that group of embodiments, subscript p is a number ranging from 1 to about 16, 1 to about 12, 1 to about 10, or 1 to about 8, from 2 to about 16, 2 to about 12, 2 to about 10, or 2 to about 8. In some aspects, p is about 2. In some aspects, p is about 4. In some aspects, p is about 8. In some aspects, p is about 16. In some aspects, p is 2. In some aspects, p is 4. In some aspects, p is 8. In some aspects, p is 16. In some embodiments, the value of subscript p refers to the average drug loading as well as the drug loading of the predominate Ligand-Drug Conjugate compound in the composition.

In some embodiments, conjugation will be via the reduced interchain disulfides and there will from 1 to about 8 Drug-Linker Compound molecules conjugated to a targeting agent that becomes a Ligand Unit. In some embodiments, conjugation will be via an introduced cysteine residue as well as reduced interchain disulfides and there will be from 1 to 10 or 1 to 12 or 1 to 14 or 1 to 16 Drug-Linker Compound moieties conjugated to a Ligand Unit. In some embodiments, conjugation will be via an introduced cysteine residue and there will be 2 or 4 Drug-Linker Compound molecules conjugated to a Ligand Unit.

### Ligand Unit L

In some embodiments of the invention, a Ligand Unit is present. The Ligand Unit (L-) is a targeting agent that specifically binds to a target moiety. In one group of embodiments, the Ligand Unit specifically and selectively binds to a cell component (a Cell Binding Agent) or to another target molecule of interest. The Ligand Unit acts to target and present the Drug Unit (such as one of Formula (I) or any subformula thereof) to the particular target cell population with which the Ligand Unit interacts due to the presence of its targeted component or molecule and allows for subsequent release of free drug within (i.e., intracellularly) or within the vicinity of the target cells (i.e., extracellularly). Ligand Units, L, include, but are not limited to, proteins, polypeptides and peptides. Suitable Ligand Units include, for example, antibodies, e.g., full-length antibodies and antigen binding fragments thereof, interferons, lymphokines, hormones, growth factors and colony-stimulating factors, vitamins, nutrient-transport molecules (such as, but not limited to, transferrin), or any other cell binding molecule or substance. In some embodiments, the Ligand Unit (L) is from an antibody or a non-antibody protein targeting agent.

In one group of embodiments a Ligand Unit is bonded to Q (a Linker Unit) which comprises a Glucuronide Releasable Linker. As noted above, in some aspects other linking components are present in the conjugates described herein to serve the purpose of providing additional space between the Drug Unit compound and the Ligand Unit (e.g., a Stretcher Unit and optionally a Connector Unit, A), or providing attributes to the composition to increases solubility (e.g., a Partitioning Agent, S^{∗}). In some of those embodiments, the Ligand Unit is bonded to Z of the Linker Unit via a heteroatom of the Ligand Unit. Heteroatoms that may be present on a Ligand Unit for that bonding include sulfur (in one embodiment, from a sulfhydryl group of a targeting ligand), oxygen (in one embodiment, from a carboxyl or hydroxyl group of a targeting ligand) and nitrogen, optionally substituted (in one embodiment, from a primary or secondary amine functional group of a targeting ligand or in another embodiment from an optionally substituted amide nitrogen). Those heteroatoms are present on the targeting ligand in the ligand's natural state, for example in a naturally occurring antibody, or are introduced into the targeting ligand via chemical modification or biological engineering.

In some embodiments, the Ligand Unit is an antibody.

Useful polyclonal antibodies are heterogeneous populations of antibody molecules derived from the sera of immunized animals. Useful monoclonal antibodies are homogeneous populations of antibodies to a particular antigenic determinant (e.g., a cancer or immune cell antigen, a protein, a peptide, a carbohydrate, a chemical, nucleic acid, or fragments thereof). A monoclonal antibody (mAb) to an antigen-of-interest can be prepared by using any technique known in the art which provides for the production of antibody molecules by continuous cell lines in culture.

Useful monoclonal antibodies include, but are not limited to, human monoclonal antibodies, humanized monoclonal antibodies, or chimeric human-mouse (or other species) monoclonal antibodies. The antibodies include full-length antibodies and antigen binding fragments thereof. Human monoclonal antibodies may be made by any of numerous techniques known in the art (*e.g.,* Teng et al., 1983, Proc. Natl. Acad. Sci. USA. 80:7308-7312; Kozbor et al., 1983, Immunology Today 4:72-79; and Olsson et al., 1982, Meth. Enzymol. 92:3-16).

In some embodiments, an antibody includes a functionally active fragment, derivative or analog of an antibody that binds specifically to target cells (e.g., cancer cell antigens) or other antibodies bound to cancer cells or matrix. In this regard, "functionally active" means that the fragment, derivative or analog is able to bind specifically to target cells. To determine which CDR sequences bind the antigen, synthetic peptides containing the CDR sequences are typically used in binding assays with the antigen by any binding assay method known in the art (e.g., the Biacore assay) (*See, e.g.,* Kabat et al., 1991, Sequences of Proteins of Immunological Interest, Fifth Edition, National Institute of Health, Bethesda, Md; Kabat E et al., 1980, J. Immunology 125(3):961-969).

Additionally, recombinant antibodies, such as chimeric and humanized monoclonal antibodies, comprising both human and non-human portions, which are typically obtained using standard recombinant DNA techniques, are useful antibodies. A chimeric antibody is a molecule in which different portions are derived from different animal species, such as for example, those having a variable region derived from a murine monoclonal and a constant region derived from a human immunoglobulin. *See,* e.g., U.S. Patent No. 4,816,567; and U.S. Patent No. 4,816,397, which are incorporated herein by reference in their entireties. Humanized antibodies are antibody molecules from non-human species having one or more CDRs from the non-human species and a framework region from a human immunoglobulin molecule. *See,* e.g., U.S. Patent No. 5,585,089, which is incorporated herein by reference in its entirety. Such chimeric and humanized monoclonal antibodies can be produced by recombinant DNA techniques known in the art, for example using methods described in International Publication No. WO 87/02671; European Patent Publication No. 0 184 187; European Patent Publication No. 0 171 496; European Patent Publication No. 0 173 494; International Publication No. WO 86/01533; U.S. Patent No. 4,816,567; European Patent Publication No. 012 023; Berter et al., 1988, Science 240:1041-1043; Liu et al., 1987, Proc. Natl. Acad. Sci. USA 84:3439-3443; Liu et al., 1987, J. Immunol. 139:3521-3526; Sun et al., 1987, Proc. Natl. Acad. Sci. USA 84:214-218; Nishimura et al., 1987, Cancer. Res. 47:999-1005; Wood et al., 1985, Nature 314:446-449; and Shaw et al., 1988, J. Natl. Cancer Inst. 80:1553-1559; Morrison, 1985, Science 229:1202-1207; Oi et al., 1986, BioTechniques 4:214; U.S. Patent No. 5,225,539; Jones et al., 1986, Nature 321: 522-525; Verhoeyan et al., 1988, Science 239:1534; and Beidler et al., 1988, J. Immunol. 141:4053-4060; each of which is incorporated herein by reference in its entirety.

In some embodiments, an antibody is a completely human antibody. In some embodiments, an antibody is produced using transgenic mice that are incapable of expressing endogenous immunoglobulin heavy and light chain genes, but which are capable of expressing human heavy and light chain genes.

In some embodiments, an antibody is an intact or fully-reduced antibody. The term 'fully-reduced' is meant to refer to an antibody in which all four inter-chain disulfide linkages have been reduced to provide eight thiols that can be attached to a linker (L).

Attachment to an antibody can be via thioether linkages from native and/or engineered cysteine residues, or from an amino acid residue engineered to participate in a cycloaddition reaction (such as a click reaction) with the corresponding linker intermediate. *See,* e.g., Maerle, et al., PLOS One 2019: 14(1); e0209860. In some embodiments, an antibody is an intact or fully-reduced antibody, or is an antibody bearing engineered an cysteine group that is modified with a functional group that can participate in, for example, click chemistry or other cycloaddition reactions for attachment of other components of the ADC as described herein (e.g., Diels-Alder reactions or other [3+2] or [4+2] cycloadditions). *See,* e.g., Agard, et al., J. Am. Chem. Soc. Vol. 126, pp. 15046-15047 (2004); Laughlin, et al., Science, Vol. 320, pp. 664-667 (2008); Beatty, et al., ChemBioChem, Vol. 11, pp. 2092-2095 (2010); and Van Geel, et al., Bioconjug. Chem. Vol. 26, pp.2233-2242 (2015).

Antibodies that bind specifically to a cancer or immune cell antigen are available commercially or produced by any method known to one of skill in the art such as, e.g., chemical synthesis or recombinant expression techniques. The nucleotide sequences encoding antibodies that bind specifically to a cancer or immune cell antigen are obtainable, e.g., from the GenBank database or similar database, literature publications, or by routine cloning and sequencing.

In some embodiments, the antibody can be used for the treatment of a cancer (e.g., an antibody approved by the FDA and/or EMA). Antibodies that bind specifically to a cancer or immune cell antigen are available commercially or produced by any method known to one of skill in the art such as, e.g., recombinant expression techniques. The nucleotide sequences encoding antibodies that bind specifically to a cancer or immune cell antigen are obtainable, e.g., from the GenBank database or similar database, literature publications, or by routine cloning and sequencing.

In some cases, the antibody has a mutation or post-translational modification which affects non-Fab mediated uptake. Peripheral immune cells, which are highly responsive to TLR7/8 agonists can facilitate strong, systemic responses when activated through non-antigen specific, Fc-mediated uptake of the TLR7/8 antibody drug conjugate which may be harmful or otherwise undesirable. In such cases, the antibody can have an effector function-diminishing mutation, such as L234A/L235A, D265A/N297A, D270A, K322A, P329A, P329G or a combination thereof, which diminishes non-immune uptake (e.g., FcgR-mediated uptake).

In some embodiments, an antibody can be configured to bind to a surface antigen of a cell. The antibody, or a complex comprising the antibody, can be configured to internalize within a cell upon binding to the surface antigen. For example, the antibody or an ADC comprising the antibody can be configured to endocytose upon binding to a surface antigen of a cell. In some embodiments, the antibody (or an ADC comprising the antibody) is configured to internalize within a cancer cell. In some embodiments, the antibody (or an ADC comprising the antibody) is configured to internalize within an immune cell. In some embodiments, the immune cell is a tumor associated macrophage. In some embodiments, the surface antigen is a receptor or a receptor complex (e.g., expressed on lymphocytes). In some embodiments, the receptor or receptor complex comprises an immunoglobulin gene superfamily member, a TNF receptor superfamily member, an integrin, a cytokine receptor, a chemokine receptor, a major histocompatibility protein, a lectin, or a complement control protein or other immune cell expressed surface receptor.

In some embodiments, an antibody is configured to bind specifically to a cancer cell antigen. In some embodiments, an antibody is configured to bind specifically to an immune cell antigen. In some embodiments, the immune cell antigen is a tumor associated macrophage antigen. In some embodiments, an antibody is configured to bind specifically to EphA2. It will be understood that the antibody component in an ADC is an antibody in residue form such that "Ab" in the ADC structures described herein incorporates the structure of the antibody.

Non-limiting examples of antibodies that can be used for treatment of cancer and antibodies that bind specifically to tumor associated antigens are disclosed in Franke, A. E., Sievers, E. L., and Scheinberg, D. A., "Cell surface receptor-targeted therapy of acute myeloid leukemia: a review" Cancer Biother Radiopharm. 2000,15, 459-76; Murray, J. L., "Monoclonal antibody treatment of solid tumors: a coming of age" Semin Oncol. 2000, 27, 64-70; Breitling, F., and Dubel, S., Recombinant Antibodies, John Wiley, and Sons, New York, 1998, each of which is hereby incorporated by reference in its entirety.

Non-limiting examples of antigens which antibodies of the present disclosure may target include ADAM12 (e.g., Catalog #14139-1-AP); ADAM9 (e.g., IMGC936); AFP (e.g., ThermoFisher Catalog #PA5-25959); AGR2 (e.g., ThermoFisher Catalog #PA5-34517); AKAP-4 (e.g., Catalog #PA5-52230); ALK (e.g., DLX521); ALPP (e.g., Catalog #MA5-15652); ALPPL2 (e.g., Catalog #PA5-22336); AMHR2 (e.g., ThermoFisher Catalog #PA5-13902); androgen receptor (e.g., ThermoFisher Catalog #MA5-13426); ANTXR1 (e.g., Catalog #MA1-91702); ANXA1 (e.g., Catalog #71-3400); ARTN (e.g., ThermoFisher Catalog #PA5-47063); ASCT2 (e.g., idactamab); Axl (e.g., BA3011; tilvestamab); B7-DC (e.g., Catalog #PA5-20344); B7-H3 (e.g., enoblituzumab, omburtamab, MGD009, MGC018, DS-7300); B7-H4 (e.g., Catalog #14-5949-82); B7-H6 (e.g., Catalog #12-6526-42); B7-H7; BAFF-R (e.g., Catalog #14-9117-82); BCMA; BCR-ABL; BMPR2; BORIS; C4.4a; CanAg; C5 complement (e.g., BCD-148; CAN106); CA-125; CA19-9 (e.g., AbGn-7; MVT-5873); CA9 (e.g., girentuximab); CALCR *(see,* e.g., International Publication No. WO 2015077826); CAMPATH-1 (e.g., alemtuzumab; ALLO-647; ANT1034); carcinoembryonic antigen (e.g., arcitumomab; cergutuzumab; amunaleukin; labetuzumab); CCNB1; CD112 *(see,* e.g., U.S. Publication No. 20100008928); CD115 (e.g., axatilimab; cabiralizumab; emactuzumab); CD123 (e.g., BAY-943; CSL360); CD137 (e.g., ADG106; CTX-471); CD138; CD142; CD166; CD147 (e.g., gavilimomab; metuzumab); CD155 (e.g., U.S. Publication No. 2018/0251548); CD19 (e.g., ALLO-501); CD20 (e.g., divozilimab; ibritumomab tiuxetan); CD24 *(see,* e.g., U.S. Patent No. 8,614,301); CD244 (e.g., R&D AF1039); CD247 (e.g., AFM15); CD27 (e.g., varlilumab); CD274 (e.g., adebrelimab; atezolizumab; garivulimab); CD3 (e.g., otelixizumab; visilizumab); CD30 (e.g., iratumumab); CD33 (e.g., lintuzumab; BI 836858; AMG 673); CD288; CD352 (e.g., SGN-CD352A); CD37 (e.g., lilotomab; GEN3009); CD38 (e.g., felzartamab; AMG 424); CD3D; CD3E (e.g., foralumab; teplizumab); CD3G; CD45 (e.g., apamistamab); CD47 (e.g., letaplimab; magrolimab); CD48 (e.g., SGN-CD48A); CD5 (e.g., MAT 304; zolimomab aritox); CD56; CD59; CD70 (e.g., cusatuzumab); CD74 (e.g., milatuzumab); CD79A *(see,* e.g., International Publication No. WO 2020252110); CD79b; CD96; CD97; CD-262 (e.g., tigatuzumab); CDCP1 (e.g., RG7287); CDH17 *(see,* e.g., International Publication No. WO 2018115231); CDH3 (e.g., PCA062); CDH6 (e.g., HKT288); CEACAM1; CEACAM5; CEACAM6; CLDN1 (e.g., INSERM anti-Claudin-1); CLDN16; CLDN18.1 (e.g., zolbetuximab); CLDN18.2 (e.g., zolbetuximab); CLDN19; CLDN2 *(see,* e.g., International Publication No. WO 2018123949); CLEC12A (e.g., tepoditamab); CS1; CLPTM1L; CSPG4 (e.g., U.S. Patent No. 10,822,427); CXCR4 (e.g., ulocuplumab); CYP1B1; c-Met; DCLK1 *(see,* e.g., International Publication No. WO 2018222675); DDR1; de2-7 EGFR (e.g., MAb 806); DLL-3; DPEP1; DPEP3; DPP4; DR4 (e.g., mapatumumab); DSG2 *(see,* e.g., U.S. Patent No. 10,836,823); EGF; EGFR; endosialin (e.g., ontuxizumab); ENPP1; EPCAM (e.g., adecatumumab); EPHA receptors; EPHA2; ERBB2 (e.g., trastuzumab); ERBB3; ERVMER34_1; ETV6-AML (e.g., Catalog #PA5-81865); FAS; FasL; Fas-related antigen 1; FBP; FGFR1 (e.g., RG7992); FGFR2 (e.g., aprutumab); FGFR3 (e.g., vofatamab); FGFR4 (e.g., MM-161); FLT3 (e.g., 4G8SDIEM); FN; FN1; FOLR1 (e.g., farletuzumab); FRa; FSHR; FucGM1 (e.g., BMS-986012); FZD5; FZD8; G250; GAGE; GCC; GD2 (e.g., dinutuximab); GD3 (e.g., mitumomab); GITR (e.g., ragifilimab); GloboH; GM2 (e.g., BIW-8962); GM3 (e.g., racotumomab); gp100; GPA33 (e.g., KRN330); GPC3 (e.g., codrituzumab); gpNMB (e.g., glembatumumab); GPR87; GUCY2C (e.g., indusatumab); HAS3; HAVCR2; HLA-E; HLA-F; HLA-G (e.g., TTX-080); HPV E6 E7; hTERT; ICAM1; IDOl; IFNAR1 (e.g., faralimomab); IFNAR2; IL13Ra2; IL1RAP (e.g., nidanilimab); IL-21R (e.g., PF-05230900); IL-5R (e.g., benralizumab); ITGAV (e.g., abituzumab); ITGB6; ITGB8; KISS1R; L1CAM (e.g., JCAR023); LAG-3 (e.g., encelimab); LAMP1; LCK; legumain; LMP2; LY6G6D (e.g., PA5-23303); LY9 (e.g., PA5-95601); LYPD1 (e.g., ThermoFisher Catalog #PA5-26749); MAD-CT-1; MAD-CT-2; MAGEA1 (e.g., Catalog #MA5-11338); MAGEA3 (e.g., ThermoFisher Catalog #60054-1-IG); MAGEA4 (e.g., Catalog #MA5-26117); MAGEC2 (e.g., ThermoFisher Catalog #PA5-64010); MELTF (e.g., ThermoFisher Catalog #H00004241-M04A); MerTk (e.g., DS5MMER, Catalog #12-5751-82); a metalloproteinase; MFSD13A; MICA (e.g., 1E2C8, Catalog #66384-1-IG); MICB (e.g., Catalog #MA5-29422); Mincle (e.g., OTI2A8, Catalog #TA505101); MLANA (e.g., Catalog #MA5-15237); ML-IAP (e.g., 88C570, ThermoFisher Catalog #40958); MSLN (e.g., 5B2, Catalog #MA5-11918); MUC1 (e.g., MH1 (CT2), ThermoFisher Catalog #MA5-11202); MUC5AC (e.g., 45M1, Catalog #MA5-12178); MYCN (e.g., NCM-II 100, ThermoFisher Catalog #MA1-170); NA17; NCAM1 (e.g., ThermoFisher Catalog #MA5-11563); Nectin-4 (e.g., enfortumab); NOX1 (e.g., Catalog #PA5-103220); NT5E (e.g., 7G2, ThermoFisher Catalog #41-0200); NY-BR-1 (e.g., NY-BR-1 No. 2, Catalog #MA5-12645); NY-ESO-1 (e.g., E978m, Catalog #35-6200); OX40 (e.g., ABM193); OY-TES1; p53; p53mutant; PAP; PAX3 (e.g., GT1210, ThermoFisher Catalog #MA5-31583); PAX5; PDGFR-B (e.g., rinucumab); PDPN (e.g., ThermoFisher Catalog #14-5381-82); PLAV1; PMSA; polysialic acid *(see,* e.g., Watzlawik et al. J Nat Sci. 2015; 1(8):e141); PR1; PROM1 (e.g., Catalog #14-1331-82); PSA (e.g., ThermoFisher Catalog #PA1-38514; Daniels-Wells et al. *BMC Cancer* 2013; 13:195); PSCA (e.g., AGS-1C4D4); PSMA (e.g., BAY 2315497); PTK7 (e.g., cofetuzumab); PVRIG; Ras mutant (e.g., Shin et al. *Sci Adv.* 2020; 6(3):eaay2174); RET (e.g., WO2020210551); RGS5 (e.g., TF-TA503075); RhoC (e.g., ThermoFisher Catalog PA5-77866); ROR1 (e.g., cirmtuzumab); ROR2 (e.g., BA3021); ROS1 (e.g., WO 2019107671); Sarcoma translocation breakpoints; SART3 (e.g., TF 18025-1-AP); Sialyl-Thomsen-nouveau-antigen (e.g., Eavarone et al. PLoS One. 2018; 13(7): e0201314); Siglecs 1-16 *(see,* e.g., Angata et al. Trends Pharmacol Sci. 2015; 36(10): 645-660); SIRPa (e.g., Catalog #17-1729-42); SIRPg (e.g., PA5-104381); SIT1 (e.g., PA5-53825); SLAMF7 (e.g., elotuzumab); SLC10A2 (e.g., ThermoFisher Catalog #PA5-18990); SLC12A2 (e.g., ThermoFisher Catalog #13884-1-AP); SLC17A2 (e.g., ThermoFisher Catalog #PA5-106752); SLC38A1 (e.g., ThermoFisher Catalog #12039-1-AP); SLC39A5 (e.g., ThermoFisher Catalog #MA5-27260); SLC39A6 (e.g., ladiratuzumab); SLC44A4 (e.g., ASG-5ME); SLC6A15 (e.g., ThermoFisher Catalog #PA5-52586); SLC6A6 (e.g., ThermoFisher Catalog #PA5-53431); SLC7A11 (e.g., ThermoFisher Catalog #PA1-16893); SLC7A5; sLe; SLITRK6 (e.g., sirtratumab); Sperm protein 17 (e.g., BS-5754R); SSX2 (e.g., ThermoFisher Catalog #MA5-24971); survivin (e.g., PA1-16836); TACSTD2 (e.g., PA5-47074); TAG-72 (e.g., MA1-25956); tenascin; TF (e.g., tisotumab); Tie3; TLR2/4/1 (e.g., tomaralimab); TM4SF5 (e.g., 18239-1-AP); TMEM132A (e.g., Catalog #PA5-62524); TMEM40 (e.g., PA5-60636); TMPRSS11D (e.g., PA5-30927); Tn; TNFRSF12 (e.g., BAY-356); TRAIL (e.g., Catalog #12-9927-42); TRAIL1; TRP-2 (e.g., PA5-52736); ULBP1/2/3/4/5/6 (e.g., PA5-82302); uPAR (e.g., ATN-658); UPK1B (e.g., ThermoFisher Catalog #PA5-56863); UPK2 (e.g., ThermoFisher Catalog #PA5-60318); UPK3B (e.g., ThermoFisher Catalog #PA5-52696); VEGF (e.g., GNR-011); VEGFR2 (e.g., gentuximab); VSIR (e.g., ThermoFisher Catalog #PA5-52493); WT1 (e.g., ThermoFisher Catalog #MA5-32215); and XAGE1 (e.g., ThermoFisher Catalog #PA5-46413).

Non-limiting examples of target antigens include Axl (e.g., BA3011; tilvestamab); B7-1 (e.g., galiximab); B7-2 (e.g., Catalog #12-0862-82); B7-DC (e.g., Catalog #PA5-20344); B7-H3 (e.g., enoblituzumab, omburtamab, MGD009, MGC018, DS-7300); B7-H4 (e.g., Catalog #14-5949-82); B7-H6 (e.g., Catalog #12-6526-42); B7-H7; BAFF-R (e.g., Catalog #14-9117-82); BCMA; C5 complement (e.g., BCD-148; CAN106); CCR4 (e.g., AT008; mogamulizumab-kpkc); CCR8 (e.g., JTX-1811); CD112 *(see,* e.g., U.S. Publication No. 20100008928); CD115 (e.g., axatilimab; cabiralizumab; emactuzumab); CD123 (e.g., BAY-943; CSL360); CD137 (e.g., ADG106; CTX-471); CD155 (e.g., U.S. Publication No. 2018/0251548); CD163 (e.g., TBI 304H); CD19 (e.g., ALLO-501); CD2 (e.g., BTI-322; siplizumab); CD20 (e.g., divozilimab; ibritumomab); CD24 *(see,* e.g., U.S. Patent No. 8,614,301); CD244 (e.g., R&D AF1039); CD247 (e.g., AFM15); CD25 (e.g., basiliximab); CD27 (e.g., varlilumab); CD274 (e.g., adebrelimab; atezolizumab; garivulimab); CD278 (e.g., feladilimab; vopratelimab); CD28 (e.g., REGN5668); CD3 (e.g., otelixizumab; visilizumab); CD30 (e.g., iratumumab); CD30L *(see,* e.g., U.S. Patent No. 9,926,373); CD32 (e.g., mAb 2B6); CD33 (e.g., lintuzumab; BI 836858; AMG 673); CD352 (e.g., SGN-CD352A); CD37 (e.g., lilotomab; GEN3009); CD38 (e.g., felzartamab; AMG 424); CD3D; CD3E (e.g., foralumab; teplizumab); CD3G; CD40 (e.g., dacetuzumab; lucatumumab); CD44 (e.g., RG7356); CD45 (e.g., apamistamab); CD47 (e.g., letaplimab; magrolimab); CD48 (e.g., SGN-CD48A); CD5 (e.g., MAT 304; zolimomab aritox); CD51; CD70 (e.g., cusatuzumab); CD74 (e.g., milatuzumab); CD79A *(see,* e.g., International Publication No. WO 2020252110); CD83 (e.g., CBT004); CD97; CD262 (e.g., tigatuzumab); CLEC12A (e.g., tepoditamab); CTLA4 (e.g., ipilimumab); CXCR4 (e.g., ulocuplumab); DCIR; DCSIGN *(see,* e.g., International Publication No. WO 2018134389); Dectin1 *(see,* e.g., U.S. Patent No. 9,045,542); Dectin2 (e.g., ThermoFisher Catalog #MA5-16250); DR4 (e.g., mapatumumab); endosialin (e.g., ontuxizumab); FasL; FLT3 (e.g., 4G8SDIEM); GITR (e.g., ragifilimab); HAVCR2; HER2; HER3; HLA-DR; HLA-E; HLA-F; HLA-G (e.g., TTX-080); ICAM1; IDO1; IFNAR1 (e.g., faralimomab); IFNAR2; IGF-1R; IL1RAP (e.g., nidanilimab); IL-21R (e.g., PF-05230900); IL-5R (e.g., benralizumab); Integrin αvβ6; LAG-3 (e.g., encelimab); LAMP1; LAYN; LCK; LILRB2; LILRB4; MerTk (e.g., DS5MMER, Catalog #12-5751-82); Mesothelin; MICA (e.g., 1E2C8, Catalog #66384-1-IG); MICB (e.g., Catalog #MA5-29422); MICA; Mincle (e.g., OTI2A8, Catalog #TA505101); MRC1 (e.g., ThermoFisher Catalog #12-2061-82); MUC1; Muc16; NcaPi2B; Nectin-4; OX40 (e.g., ABM193); PD-1 (e.g., balstilimab; budigalimab; geptanolimab); PD-L1; Prolactin receptor; PTK7; PVRIG; ROR-1; Sialyl-Thomsen-nouveau-antigen (e.g., Eavarone et al. PLoS One, 2018; 13(7): e0201314); Siglecs 1-16 *(see,* e.g., Angata et al. Trends Pharmacol Sci. 2015; 36(10): 645-660); ); SIRPa (e.g., Catalog #17-1729-42); SIRPg (e.g., PA5-104381); SIT1 (e.g., PA5-53825); SLAMF7 (e.g., elotuzumab); SLTRK6; STEAP1; TIGIT (e.g., etigilimab); TLR2/4/1 (e.g., tomaralimab); Trem2 (e.g., PY314); TROP2; Tyrol; ULBP1/2/3/4/5/6 (e.g., PA5-82302); uPAR (e.g., ATN-658); VSIR (e.g., ThermoFisher Catalog #PA5-52493); ZIP6 (Anti-Integrin αvβ6).

### (i) Heavy Chain and Light Chain Variable Regions

In some cases, an antibody target is selected from the group consisting of ADAM9, ASCT2, Axl, B7-H3, B7H4, BCMA, BCMA, C4.4a, CanAg, CD123, CD138, CD142, CD166, CD19, CD20, CD228, CD25, CD30, CD33, CD352, CD38, CD48, CD56, CD59, CD70, CD74, CD79b, CDCP1, CEACAM5, Claudin-18.2, c-Met, gpNMB, CS1, DLL-3, DPEP-3, EGFR, EpCAM, EphA2, FGFR2, FRa, GCC, gpA33, GPC3, Integrin αvβ6 , h2A2, H2G12/STn, HER2, HER3, ZIP6, IGF-1R, IL1Rap, ITGav/CD51, Mesothelin, MICA, MUC-1, Muc16, NaPi2B, Nectin-4, PD-L1, Prolactin receptor, PTK7, ROR-1, SLAMF7, SLTRK6, STEAP1, TIGIT, and TROP2.

In some cases, an antibody of the present disclosure comprises a heavy chain variable region having at least 80% sequence identity to a first sequence and a light chain variable region having at least 80% sequence identity to a second sequence, and wherein: the first sequence is SEQ ID NO: 19 and the second sequence is SEQ ID NO: 20; the first sequence is SEQ ID NO: 44 and the second sequence is SEQ ID NO: 45; the first sequence is SEQ ID NO: 55 and the second sequence is SEQ ID NO: 56; the first sequence is SEQ ID NO: 69 and the second sequence is SEQ ID NO: 70; the first sequence is SEQ ID NO: 83 and the second sequence is SEQ ID NO: 84; the first sequence is SEQ ID NO: 97 and the second sequence is SEQ ID NO: 98; the first sequence is SEQ ID NO: 105 and the second sequence is SEQ ID NO: 106; the first sequence is SEQ ID NO:113 and the second sequence is SEQ ID NO: 114; the first sequence is SEQ ID NO: 121 and the second sequence is SEQ ID NO: 122; the first sequence is SEQ ID NO: 129 and the second sequence is SEQ ID NO: 130; the first sequence is SEQ ID NO: 137 and the second sequence is SEQ ID NO: 138; the first sequence is SEQ ID NO: 153 and the second sequence is SEQ ID NO: 154; the first sequence is SEQ ID NO: 161 and the second sequence is SEQ ID NO: 162; the first sequence is SEQ ID NO: 169 and the second sequence is SEQ ID NO: 170; the first sequence is SEQ ID NO: 177 and the second sequence is SEQ ID NO: 178; the first sequence is SEQ ID NO: 185 and the second sequence is SEQ ID NO: 186; the first sequence is SEQ ID NO: 193 and the second sequence is SEQ ID NO: 194; the first sequence is SEQ ID NO: 201 and the second sequence is SEQ ID NO: 202; the first sequence is SEQ ID NO: 209 and the second sequence is SEQ ID NO: 210; the first sequence is SEQ ID NO: 217 and the second sequence is SEQ ID NO: 218; the first sequence is SEQ ID NO: 225 and the second sequence is SEQ ID NO: 226; the first sequence is SEQ ID NO: 233 and the second sequence is SEQ ID NO: 234; the first sequence is SEQ ID NO: 241 and the second sequence is SEQ ID NO: 242; the first sequence is SEQ ID NO: 249 and the second sequence is SEQ ID NO: 250; the first sequence is SEQ ID NO: 297 and the second sequence is SEQ ID NO: 298; the first sequence is SEQ ID NO: 307 and the second sequence is SEQ ID NO: 308; the first sequence is SEQ ID NO: 315 and the second sequence is SEQ ID NO: 316; the first sequence is SEQ ID NO: 323 and the second sequence is SEQ ID NO: 324; the first sequence is SEQ ID NO: 331 and the second sequence is SEQ ID NO: 332; the first sequence is SEQ ID NO: 339 and the second sequence is SEQ ID NO: 340; the first sequence is SEQ ID NO: 347 and the second sequence is SEQ ID NO: 348; the first sequence is SEQ ID NO: 355 and the second sequence is SEQ ID NO: 356; the first sequence is SEQ ID NO: 363 and the second sequence is SEQ ID NO: 364; the first sequence is SEQ ID NO: 371 and the second sequence is SEQ ID NO: 372; the first sequence is SEQ ID NO: 379 and the second sequence is SEQ ID NO: 380; the first sequence is SEQ ID NO: 387 and the second sequence is SEQ ID NO: 388; the first sequence is SEQ ID NO: 395 and the second sequence is SEQ ID NO: 396; the first sequence is SEQ ID NO: 403 and the second sequence is SEQ ID NO: 404; the first sequence is SEQ ID NO: 411 and the second sequence is SEQ ID NO: 412; the first sequence is SEQ ID NO: 419 and the second sequence is SEQ ID NO: 420; the first sequence is SEQ ID NO: 427 and the second sequence is SEQ ID NO: 428; the first sequence is SEQ ID NO: 435 and the second sequence is SEQ ID NO: 436; the first sequence is SEQ ID NO: 443 and the second sequence is SEQ ID NO: 444; the first sequence is SEQ ID NO: 451 and the second sequence is SEQ ID NO: 452; the first sequence is SEQ ID NO: 459 and the second sequence is SEQ ID NO: 460; the first sequence is SEQ ID NO: 467 and the second sequence is SEQ ID NO: 468; the first sequence is SEQ ID NO: 475 and the second sequence is SEQ ID NO: 476; the first sequence is SEQ ID NO: 483 and the second sequence is SEQ ID NO: 484; the first sequence is SEQ ID NO: 491 and the second sequence is SEQ ID NO: 492; the first sequence is SEQ ID NO: 501 and the second sequence is SEQ ID NO: 502; the first sequence is SEQ ID NO: 509 and the second sequence is SEQ ID NO: 510; the first sequence is SEQ ID NO: 517 and the second sequence is SEQ ID NO: 518; the first sequence is SEQ ID NO: 525 and the second sequence is SEQ ID NO: 526; the first sequence is SEQ ID NO: 533 and the second sequence is SEQ ID NO: 534; the first sequence is SEQ ID NO: 541 and the second sequence is SEQ ID NO: 542; the first sequence is SEQ ID NO: 549 and the second sequence is SEQ ID NO: 550; the first sequence is SEQ ID NO: 557 and the second sequence is SEQ ID NO: 558; the first sequence is SEQ ID NO: 565 and the second sequence is SEQ ID NO: 566; the first sequence is SEQ ID NO: 574 and the second sequence is SEQ ID NO: 574; the first sequence is SEQ ID NO: 581 and the second sequence is SEQ ID NO: 582; the first sequence is SEQ ID NO: 589 and the second sequence is SEQ ID NO: 590; the first sequence is SEQ ID NO: 597 and the second sequence is SEQ ID NO: 598; the first sequence is SEQ ID NO: 605 and the second sequence is SEQ ID NO: 606; the first sequence is SEQ ID NO: 613 and the second sequence is SEQ ID NO: 614; the first sequence is SEQ ID NO: 621 and the second sequence is SEQ ID NO: 622; the first sequence is SEQ ID NO: 629 and the second sequence is SEQ ID NO: 630; the first sequence is SEQ ID NO: 637 and the second sequence is SEQ ID NO: 638; the first sequence is SEQ ID NO: 645 and the second sequence is SEQ ID NO: 646; the first sequence is SEQ ID NO: 653 and the second sequence is SEQ ID NO: 654; the first sequence is SEQ ID NO: 661 and the second sequence is SEQ ID NO: 662; the first sequence is SEQ ID NO: 669 and the second sequence is SEQ ID NO: 670; the first sequence is SEQ ID NO: 677 and the second sequence is SEQ ID NO: 678; the first sequence is SEQ ID NO: 685 and the second sequence is SEQ ID NO: 686; the first sequence is SEQ ID NO: 693 and the second sequence is SEQ ID NO: 694; the first sequence is SEQ ID NO: 701 and the second sequence is SEQ ID NO: 702; the first sequence is SEQ ID NO: 703 and the second sequence is SEQ ID NO: 704; the first sequence is SEQ ID NO: 711 and the second sequence is SEQ ID NO: 712; the first sequence is SEQ ID NO: 713 and the second sequence is SEQ ID NO: 714; the first sequence is SEQ ID NO: 715 and the second sequence is SEQ ID NO: 716; the first sequence is SEQ ID NO: 731 and the second sequence is SEQ ID NO: 732; the first sequence is SEQ ID NO: 739 and the second sequence is SEQ ID NO: 740; the first sequence is SEQ ID NO: 747 and the second sequence is SEQ ID NO: 748; the first sequence is SEQ ID NO: 755 and the second sequence is SEQ ID NO: 756; the first sequence is SEQ ID NO: 765 and the second sequence is SEQ ID NO: 766; the first sequence is SEQ ID NO: 773 and the second sequence is SEQ ID NO: 774; the first sequence is SEQ ID NO: 781 and the second sequence is SEQ ID NO: 782; the first sequence is SEQ ID NO: 789 and the second sequence is SEQ ID NO: 790; the first sequence is SEQ ID NO: 797 and the second sequence is SEQ ID NO: 798; the first sequence is SEQ ID NO: 805 and the second sequence is SEQ ID NO: 806; the first sequence is SEQ ID NO: 813 and the second sequence is SEQ ID NO: 814; the first sequence is SEQ ID NO: 821 and the second sequence is SEQ ID NO: 822; the first sequence is SEQ ID NO: 829 and the second sequence is SEQ ID NO: 830; the first sequence is SEQ ID NO: 837 and the second sequence is SEQ ID NO: 838; the first sequence is SEQ ID NO: 845 and the second sequence is SEQ ID NO: 846; the first sequence is SEQ ID NO: 853 and the second sequence is SEQ ID NO: 854; the first sequence is SEQ ID NO: 861 and the second sequence is SEQ ID NO: 862; the first sequence is SEQ ID NO: 869 and the second sequence is SEQ ID NO: 870; the first sequence is SEQ ID NO: 877 and the second sequence is SEQ ID NO: 878; the first sequence is SEQ ID NO: 885 and the second sequence is SEQ ID NO: 886; the first sequence is SEQ ID NO: 893 and the second sequence is SEQ ID NO: 894; the first sequence is SEQ ID NO: 900 and the second sequence is SEQ ID NO: 901; the first sequence is SEQ ID NO: 909 and the second sequence is SEQ ID NO: 910; the first sequence is SEQ ID NO: 917 and the second sequence is SEQ ID NO: 918; the first sequence is SEQ ID NO: 925 and the second sequence is SEQ ID NO: 926; the first sequence is SEQ ID NO: 933 and the second sequence is SEQ ID NO: 934; the first sequence is SEQ ID NO: 941 and the second sequence is SEQ ID NO: 942; the first sequence is SEQ ID NO: 943 and the second sequence is SEQ ID NO: 944; the first sequence is SEQ ID NO: 951 and the second sequence is SEQ ID NO: 952; the first sequence is SEQ ID NO: 959 and the second sequence is SEQ ID NO: 960; the first sequence is SEQ ID NO: 967 and the second sequence is SEQ ID NO: 968; the first sequence is SEQ ID NO: 975 and the second sequence is SEQ ID NO: 976; the first sequence is SEQ ID NO: 983 and the second sequence is SEQ ID NO: 984; the first sequence is SEQ ID NO: 991 and the second sequence is SEQ ID NO: 992; the first sequence is SEQ ID NO: 993 and the second sequence is SEQ ID NO: 994; the first sequence is SEQ ID NO: 995 and the second sequence is SEQ ID NO: 996; the first sequence is SEQ ID NO: 1003 and the second sequence is SEQ ID NO: 1004; the first sequence is SEQ ID NO: 1011 and the second sequence is SEQ ID NO: 1012; the first sequence is SEQ ID NO: 1019 and the second sequence is SEQ ID NO: 1020; the first sequence is SEQ ID NO: 1027 and the second sequence is SEQ ID NO: 1028; the first sequence is SEQ ID NO: 1035 and 1036; or the first sequence is SEQ ID NO: 1043 and the second sequence is SEQ ID NO: 1044.

In some cases, an antibody of the present disclosure targets an immune checkpoint selected from the group consisting of PDL1, B7H4, B7H3, and TIGIT. For example, in some cases, the antibody comprises a heavy chain variable region having at least 80% sequence identity to a first sequence and a light chain variable region having at least 80% sequence identity to a second sequence, wherein: the first sequence is SEQ ID NO: 19 and the second sequence is SEQ ID NO: 20; the first sequence is SEQ ID NO: 83 and the second sequence is SEQ ID NO: 84; the first sequence is SEQ ID NO: 97 and the second sequence is SEQ ID NO: 98; the first sequence is SEQ ID NO: 105 and the second sequence is SEQ ID NO: 106; the first sequence is SEQ ID NO:113 and the second sequence is SEQ ID NO: 114; the first sequence is SEQ ID NO: 121 and the second sequence is SEQ ID NO: 122; the first sequence is SEQ ID NO: 129 and the second sequence is SEQ ID NO: 130; the first sequence is SEQ ID NO: 137 and the second sequence is SEQ ID NO: 138; the first sequence is SEQ ID NO: 153 and the second sequence is SEQ ID NO: 154; the first sequence is SEQ ID NO: 161 and the second sequence is SEQ ID NO: 162; the first sequence is SEQ ID NO: 169 and the second sequence is SEQ ID NO: 170; the first sequence is SEQ ID NO: 177 and the second sequence is SEQ ID NO: 178; the first sequence is SEQ ID NO: 185 and the second sequence is SEQ ID NO: 186; the first sequence is SEQ ID NO: 193 and the second sequence is SEQ ID NO: 194; the first sequence is SEQ ID NO: 201 and the second sequence is SEQ ID NO: 202; the first sequence is SEQ ID NO: 209 and the second sequence is SEQ ID NO: 210; the first sequence is SEQ ID NO: 217 and the second sequence is SEQ ID NO: 218; the first sequence is SEQ ID NO: 225 and the second sequence is SEQ ID NO: 226; the first sequence is SEQ ID NO: 233 and the second sequence is SEQ ID NO: 234; the first sequence is SEQ ID NO: 241 and the second sequence is SEQ ID NO: 242; the first sequence is SEQ ID NO: 249 and the second sequence is SEQ ID NO: 250; the first sequence is SEQ ID NO: 629 and the second sequence is SEQ ID NO: 630; the first sequence is SEQ ID NO: 637 and the second sequence is SEQ ID NO: 638; the first sequence is SEQ ID NO: 645 and the second sequence is SEQ ID NO: 646; the first sequence is SEQ ID NO: 653 and the second sequence is SEQ ID NO: 654; the first sequence is SEQ ID NO: 661 and the second sequence is SEQ ID NO: 662; the first sequence is SEQ ID NO: 669 and the second sequence is SEQ ID NO: 670; the first sequence is SEQ ID NO: 677 and the second sequence is SEQ ID NO: 678; the first sequence is SEQ ID NO: 685 and the second sequence is SEQ ID NO: 686; the first sequence is SEQ ID NO: 693 and the second sequence is SEQ ID NO: 694; the first sequence is SEQ ID NO: 701 and the second sequence is SEQ ID NO: 702; the first sequence is SEQ ID NO: 703 and the second sequence is SEQ ID NO: 704; the first sequence is SEQ ID NO: 711 and the second sequence is SEQ ID NO: 712; the first sequence is SEQ ID NO: 713 and the second sequence is SEQ ID NO: 714; the first sequence is SEQ ID NO: 715 and the second sequence is SEQ ID NO: 716; or the first sequence is SEQ ID NO: 1027 and the second sequence is SEQ ID NO: 1028.

In some cases, an antibody of the present disclosure comprises a heavy chain variable region having at least 80% sequence identity to a first sequence and a light chain variable region having at least 80% sequence identity to a second sequence, wherein: the first sequence is SEQ ID NO: 19 and the second sequence is SEQ ID NO: 20; the first sequence is SEQ ID NO: 83 and the second sequence is SEQ ID NO: 84; the first sequence is SEQ ID NO: 97 and the second sequence is SEQ ID NO: 98; the first sequence is SEQ ID NO: 105 and the second sequence is SEQ ID NO: 106; the first sequence is SEQ ID NO: 113 and the second sequence is SEQ ID NO: 114; the first sequence is SEQ ID NO: 121 and the second sequence is SEQ ID NO: 122; the first sequence is SEQ ID NO: 129 and the second sequence is SEQ ID NO: 130; the first sequence is SEQ ID NO: 137 and the second sequence is SEQ ID NO: 138; the first sequence is SEQ ID NO: 153 and the second sequence is SEQ ID NO: 154; the first sequence is SEQ ID NO: 161 and the second sequence is SEQ ID NO: 162; the first sequence is SEQ ID NO: 169 and the second sequence is SEQ ID NO: 170; the first sequence is SEQ ID NO: 177 and the second sequence is SEQ ID NO: 178; the first sequence is SEQ ID NO: 185 and the second sequence is SEQ ID NO: 186; the first sequence is SEQ ID NO: 193 and the second sequence is SEQ ID NO: 194; the first sequence is SEQ ID NO: 201 and the second sequence is SEQ ID NO: 202; the first sequence is SEQ ID NO: 209 and the second sequence is SEQ ID NO: 210; the first sequence is SEQ ID NO: 217 and the second sequence is SEQ ID NO: 218; the first sequence is SEQ ID NO: 225 and the second sequence is SEQ ID NO: 226; the first sequence is SEQ ID NO: 233 and the second sequence is SEQ ID NO: 234; the first sequence is SEQ ID NO: 241 and the second sequence is SEQ ID NO: 242; the first sequence is SEQ ID NO: 249 and the second sequence is SEQ ID NO: 250; or the first sequence is SEQ ID NO: 1027 and the second sequence is SEQ ID NO: 1028. In some cases, the first sequence is SEQ ID NO: 19 and the second sequence is SEQ ID NO: 20.

In some cases, the heavy chain variable region has at least 85% sequence identity to the first sequence and the light chain variable region has at least 85% sequence identity to the second sequence. In some cases, the heavy chain variable region has at least 90% sequence identity to the first sequence and the light chain variable region has at least 90% sequence identity to the second sequence. In some cases, the heavy chain variable region has at least 95% sequence identity to the first sequence and the light chain variable region has at least 95% sequence identity to the second sequence. In some cases, the heavy chain variable region has at least 98% sequence identity to the first sequence and the light chain variable region has at least 98% sequence identity to the second sequence. In some cases, the heavy chain variable region has at least 99% sequence identity to the first sequence and the light chain variable region has at least 99% sequence identity to the second sequence. In some cases, the heavy chain variable region comprises the first sequence and the light chain variable region comprises the second sequence.

### (ii) Heavy and Light Chains

In some cases, an antibody of the present disclosure comprises a heavy chain having at least 80% sequence identity to a first sequence and a light chain having at least 80% sequence identity to a second sequence, wherein: the first sequence is SEQ ID NO: 1 and the second sequence is SEQ ID NO: 5; the first sequence is SEQ ID NO: 2 and the second sequence is SEQ ID NO: 5; the first sequence is SEQ ID NO: 3 and the second sequence is SEQ ID NO: 5; the first sequence is SEQ ID NO: 4 and the second sequence is SEQ ID NO: 5; the first sequence is SEQ ID NO: 6 and the second sequence is SEQ ID NO: 10; the first sequence is SEQ ID NO: 7 and the second sequence is SEQ ID NO: 10; the first sequence is SEQ ID NO: 8 and the second sequence is SEQ ID NO: 10; the first sequence is SEQ ID NO: 9 and the second sequence is SEQ ID NO: 10; the first sequence is SEQ ID NO: 46 and the second sequence is SEQ ID NO: 48; the first sequence is SEQ ID NO: 47 and the second sequence is SEQ ID NO: 48; the first sequence is SEQ ID NO: 57 and the second sequence is SEQ ID NO: 59; the first sequence is SEQ ID NO: 58 and the second sequence is SEQ ID NO: 59; the first sequence is SEQ ID NO: 60 and the second sequence is SEQ ID NO: 62; the first sequence is SEQ ID NO: 61 and the second sequence is SEQ ID NO: 62; the first sequence is SEQ ID NO: 71 and the second sequence is SEQ ID NO: 73; the first sequence is SEQ ID NO: 72 and the second sequence is SEQ ID NO: 73; the first sequence is SEQ ID NO: 74 and the second sequence is SEQ ID NO: 76; the first sequence is SEQ ID NO: 75 and the second sequence is SEQ ID NO: 76; the first sequence is SEQ ID NO: 85 and the second sequence is SEQ ID NO: 87; the first sequence is SEQ ID NO: 86 and the second sequence is SEQ ID NO: 87; the first sequence is SEQ ID NO: 88 and the second sequence is SEQ ID NO: 90; the first sequence is SEQ ID NO: 89 and the second sequence is SEQ ID NO: 90; the first sequence is SEQ ID NO: 251 and the second sequence is SEQ ID NO: 252; the first sequence is SEQ ID NO: 253 and the second sequence is SEQ ID NO: 254; the first sequence is SEQ ID NO: 255 and the second sequence is SEQ ID NO: 256; the first sequence is SEQ ID NO: 257 and the second sequence is SEQ ID NO: 258; the first sequence is SEQ ID NO: 259 and the second sequence is SEQ ID NO: 260; the first sequence is SEQ ID NO: 261 and the second sequence is SEQ ID NO: 262; the first sequence is SEQ ID NO: 263 and the second sequence is SEQ ID NO: 264; the first sequence is SEQ ID NO: 265 and the second sequence is SEQ ID NO: 266; the first sequence is SEQ ID NO: 267 and the second sequence is SEQ ID NO: 268; the first sequence is SEQ ID NO: 269 and the second sequence is SEQ ID NO: 270; the first sequence is SEQ ID NO: 271 and the second sequence is SEQ ID NO: 272; the first sequence is SEQ ID NO: 273 and the second sequence is SEQ ID NO: 274; the first sequence is SEQ ID NO: 275 and the second sequence is SEQ ID NO: 276; the first sequence is SEQ ID NO: 277 and the second sequence is SEQ ID NO: 278; the first sequence is SEQ ID NO: 279 and the second sequence is SEQ ID NO: 280; the first sequence is SEQ ID NO: 281 and the second sequence is SEQ ID NO: 282; the first sequence is SEQ ID NO: 283 and the second sequence is SEQ ID NO: 284; the first sequence is SEQ ID NO: 285 and the second sequence is SEQ ID NO: 286; the first sequence is SEQ ID NO: 287 and the second sequence is SEQ ID NO: 288; the first sequence is SEQ ID NO: 289 and the second sequence is SEQ ID NO: 290; the first sequence is SEQ ID NO: 299 and the second sequence is SEQ ID NO: 300; the first sequence is SEQ ID NO: 493 and the second sequence is SEQ ID NO: 494; the first sequence is SEQ ID NO: 717 and the second sequence is SEQ ID NO: 718; the first sequence is SEQ ID NO: 719 and the second sequence is SEQ ID NO: 720; the first sequence is SEQ ID NO: 721 and the second sequence is SEQ ID NO: 722; the first sequence is SEQ ID NO: 723 and the second sequence is SEQ ID NO: 724; or the first sequence is SEQ ID NO: 757 and the second sequence is SEQ ID NO: 758.

In some cases, an antibody of the present disclosure comprises a heavy chain having at least 80% sequence identity to a first sequence and a light chain having at least 80% sequence identity to a second sequence, wherein: the first sequence is SEQ ID NO: 1 and the second sequence is SEQ ID NO: 5; the first sequence is SEQ ID NO: 2 and the second sequence is SEQ ID NO: 5; the first sequence is SEQ ID NO: 3 and the second sequence is SEQ ID NO: 5; the first sequence is SEQ ID NO: 4 and the second sequence is SEQ ID NO: 5; the first sequence is SEQ ID NO: 6 and the second sequence is SEQ ID NO: 10; the first sequence is SEQ ID NO: 7 and the second sequence is SEQ ID NO: 10; the first sequence is SEQ ID NO: 8 and the second sequence is SEQ ID NO: 10; the first sequence is SEQ ID NO: 9 and the second sequence is SEQ ID NO: 10; the first sequence is SEQ ID NO: 85 and the second sequence is SEQ ID NO: 87; the first sequence is SEQ ID NO: 86 and the second sequence is SEQ ID NO: 87; the first sequence is SEQ ID NO: 88 and the second sequence is SEQ ID NO: 90; the first sequence is SEQ ID NO: 89 and the second sequence is SEQ ID NO: 90; the first sequence is SEQ ID NO: 251 and the second sequence is SEQ ID NO: 252; the first sequence is SEQ ID NO: 253 and the second sequence is SEQ ID NO: 254; the first sequence is SEQ ID NO: 255 and the second sequence is SEQ ID NO: 256; the first sequence is SEQ ID NO: 257 and the second sequence is SEQ ID NO: 258; the first sequence is SEQ ID NO: 259 and the second sequence is SEQ ID NO: 260; the first sequence is SEQ ID NO: 261 and the second sequence is SEQ ID NO: 262; the first sequence is SEQ ID NO: 263 and the second sequence is SEQ ID NO: 264; the first sequence is SEQ ID NO: 265 and the second sequence is SEQ ID NO: 266; the first sequence is SEQ ID NO: 267 and the second sequence is SEQ ID NO: 268; the first sequence is SEQ ID NO: 269 and the second sequence is SEQ ID NO: 270; the first sequence is SEQ ID NO: 271 and the second sequence is SEQ ID NO: 272; the first sequence is SEQ ID NO: 273 and the second sequence is SEQ ID NO: 274; the first sequence is SEQ ID NO: 275 and the second sequence is SEQ ID NO: 276; the first sequence is SEQ ID NO: 277 and the second sequence is SEQ ID NO: 278; the first sequence is SEQ ID NO: 279 and the second sequence is SEQ ID NO: 280; the first sequence is SEQ ID NO: 281 and the second sequence is SEQ ID NO: 282; the first sequence is SEQ ID NO: 283 and the second sequence is SEQ ID NO: 284; the first sequence is SEQ ID NO: 285 and the second sequence is SEQ ID NO: 286; the first sequence is SEQ ID NO: 287 and the second sequence is SEQ ID NO: 288; the first sequence is SEQ ID NO: 289 and the second sequence is SEQ ID NO: 290; the first sequence is SEQ ID NO: 717 and the second sequence is SEQ ID NO: 718; the first sequence is SEQ ID NO: 719 and the second sequence is SEQ ID NO: 720; the first sequence is SEQ ID NO: 721 and the second sequence is SEQ ID NO: 722; or the first sequence is SEQ ID NO: 723 and the second sequence is SEQ ID NO: 724.

In some cases, an antibody of the present disclosure comprises a heavy chain having at least 80% sequence identity to a first sequence and a light chain having at least 80% sequence identity to a second sequence, wherein: the first sequence is SEQ ID NO: 1 and the second sequence is SEQ ID NO: 5; the first sequence is SEQ ID NO: 2 and the second sequence is SEQ ID NO: 5; the first sequence is SEQ ID NO: 3 and the second sequence is SEQ ID NO: 5; the first sequence is SEQ ID NO: 4 and the second sequence is SEQ ID NO: 5; the first sequence is SEQ ID NO: 6 and the second sequence is SEQ ID NO: 10; the first sequence is SEQ ID NO: 7 and the second sequence is SEQ ID NO: 10; the first sequence is SEQ ID NO: 8 and the second sequence is SEQ ID NO: 10; the first sequence is SEQ ID NO: 9 and the second sequence is SEQ ID NO: 10; the first sequence is SEQ ID NO: 85 and the second sequence is SEQ ID NO: 87; the first sequence is SEQ ID NO: 86 and the second sequence is SEQ ID NO: 87; the first sequence is SEQ ID NO: 88 and the second sequence is SEQ ID NO: 90; the first sequence is SEQ ID NO: 89 and the second sequence is SEQ ID NO: 90; the first sequence is SEQ ID NO: 251 and the second sequence is SEQ ID NO: 252; the first sequence is SEQ ID NO: 253 and the second sequence is SEQ ID NO: 254; the first sequence is SEQ ID NO: 255 and the second sequence is SEQ ID NO: 256; the first sequence is SEQ ID NO: 257 and the second sequence is SEQ ID NO: 258; the first sequence is SEQ ID NO: 259 and the second sequence is SEQ ID NO: 260; the first sequence is SEQ ID NO: 261 and the second sequence is SEQ ID NO: 262; the first sequence is SEQ ID NO: 263 and the second sequence is SEQ ID NO: 264; the first sequence is SEQ ID NO: 265 and the second sequence is SEQ ID NO: 266; the first sequence is SEQ ID NO: 267 and the second sequence is SEQ ID NO: 268; the first sequence is SEQ ID NO: 269 and the second sequence is SEQ ID NO: 270; the first sequence is SEQ ID NO: 271 and the second sequence is SEQ ID NO: 272; the first sequence is SEQ ID NO: 273 and the second sequence is SEQ ID NO: 274; the first sequence is SEQ ID NO: 275 and the second sequence is SEQ ID NO: 276; the first sequence is SEQ ID NO: 277 and the second sequence is SEQ ID NO: 278; the first sequence is SEQ ID NO: 279 and the second sequence is SEQ ID NO: 280; the first sequence is SEQ ID NO: 281 and the second sequence is SEQ ID NO: 282; the first sequence is SEQ ID NO: 283 and the second sequence is SEQ ID NO: 284; the first sequence is SEQ ID NO: 285 and the second sequence is SEQ ID NO: 286; the first sequence is SEQ ID NO: 287 and the second sequence is SEQ ID NO: 288; or the first sequence is SEQ ID NO: 289 and the second sequence is SEQ ID NO: 290.

In some cases, an antibody of the present disclosure comprises a heavy chain having at least 80% sequence identity to a first sequence and a light chain having at least 80% sequence identity to a second sequence, wherein: the first sequence is SEQ ID NO: 1 and the second sequence is SEQ ID NO: 5; the first sequence is SEQ ID NO: 2 and the second sequence is SEQ ID NO: 5; the first sequence is SEQ ID NO: 3 and the second sequence is SEQ ID NO: 5; the first sequence is SEQ ID NO: 4 and the second sequence is SEQ ID NO: 5; the first sequence is SEQ ID NO: 6 and the second sequence is SEQ ID NO: 10; the first sequence is SEQ ID NO: 7 and the second sequence is SEQ ID NO: 10; the first sequence is SEQ ID NO: 8 and the second sequence is SEQ ID NO: 10; or the first sequence is SEQ ID NO: 9 and the second sequence is SEQ ID NO: 10.

In some cases, an antibody of the present disclosure comprises a heavy chain having at least 80% sequence identity to a first sequence and a light chain having at least 80% sequence identity to a second sequence, wherein: the first sequence is SEQ ID NO: 3 or SEQ ID NO: 4 and the second sequence is SEQ ID NO: 5.

In some cases, the heavy chain has at least 85% sequence identity to the first sequence and the light chain has at least 85% sequence identity to the second sequence. In some cases, the heavy chain has at least 90% sequence identity to the first sequence and the light chain has at least 90% sequence identity to the second sequence. In some cases, the heavy chain has at least 95% sequence identity to the first sequence and the light chain has at least 95% sequence identity to the second sequence. In some cases, the heavy chain has at least 98% sequence identity to the first sequence and the light chain has at least 98% sequence identity to the second sequence. In some cases, the heavy chain has at least 99% sequence identity to the first sequence and the light chain has at least 99% sequence identity to the second sequence. In some cases, the heavy chain comprises the first sequence and the light chain comprises the second sequence.

### (iii) Complementarity-Determining Regions

In some cases, an antibody of the present disclosure comprises CDR-H1, CDR-H2, CDR-H3, CDR-L1, CDR-L2, and CDR-L3 comprising at least 80% sequence identity to: SEQ ID NO: 13, 14, 15, 16, 17, and 18, respectively (i.e., CDR-H1 has at least 80% sequence identity to SEQ ID NO: 13, CDR-H2 has at least 80% sequence identity to SEQ ID NO: 14, CDR-H3 has at least 80% sequence identity to SEQ ID NO: 15, CDR-L1 has at least 80% sequence identity to SEQ ID NO: 16, CDR-L2 has at least 80% sequence identity to SEQ ID NO: 17, and CDR-L3 has at least 80% sequence identity to SEQ ID NO: 18); SEQ ID NO: 21, 22, 23, 24, 25, and 26, respectively; SEQ ID NO: 38, 39, 40, 41, 42, and 43, respectively; SEQ ID NO: 49, 50, 51, 52, 53, and 54, respectively; SEQ ID NO: 63, 64, 65, 66, 67, and 68, respectively; SEQ ID NO: 77, 78, 79, 80, 81, and 82, , respectively; SEQ ID NO: 91, 92, 93, 94, 95, and 96, respectively; SEQ ID NO: 99, 100, 101, 102, 103, and 104, respectively; SEQ ID NO: 107, 108, 109, 110, 111, and 112, respectively; SEQ ID NO: 115, 116, 117, 118, 119, and 120, respectively; SEQ ID NO: 123, 124, 125, 126, 127, and 128, respectively; SEQ ID NO: 131, 132, 133, 134, 135, and 136, respectively; SEQ ID NO: 139, 140, 141, 142, 143, and 144, respectively; SEQ ID NO: 147, 148, 149, 150, 151, and 152, respectively; SEQ ID NO: 155, 156, 157, 158, 159, and 160, respectively; SEQ ID NO: 163, 164, 165, 166, 167, and 168, respectively; SEQ ID NO: 171, 172, 173, 174, 175, and 176, respectively; SEQ ID NO: 179, 180, 181, 182, 183, and 184, respectively; SEQ ID NO: 187, 188, 189, 190, 191, and 192, respectively; SEQ ID NO: 195, 196, 197, 198, 199, and 200, respectively; SEQ ID NO: 203, 204, 205, 206, 207 and 208, respectively; SEQ ID NO: 211, 212, 213, 214, 215, and 216, respectively; SEQ ID NO: 219, 220, 221, 222, 223, and 224, respectively; SEQ ID NO: 227, 228, 229, 230, 231, and 232, respectively; SEQ ID NO: 235, 236, 237, 238, 239, and 240, respectively; SEQ ID NO: 243, 244, 245, 246, 247, and 248, respectively; SEQ ID NO: 291, 292, 293, 294, 295, and 296, respectively; SEQ ID NO: 301, 302, 303, 304, 305, and 306, respectively; SEQ ID NO: 309, 310, 311, 312, 313, and 314, respectively; SEQ ID NO: 317, 318, 319, 320, 321, and 322, respectively; SEQ ID NO: 325, 326, 327, 328, 329, and 330, respectively; SEQ ID NO: 333, 334, 335, 336, 337, and 338, respectively; SEQ ID NO: 341, 342, 343, 344, 345, and 346, respectively; SEQ ID NO: 349, 350, 351, 352, 353, and 354, respectively; SEQ ID NO: 357, 358, 359, 360, 361, and 362, respectively; SEQ ID NO: 365, 366, 367, 368, 369, and 370, respectively; SEQ ID NO: 373, 374, 375, 376, 377, and 378, respectively; SEQ ID NO: 381, 382, 383, 384, 385, and 386, respectively; SEQ ID NO: 389, 390, 391, 392, 393, and 394, respectively; SEQ ID NO: 397, 398, 399, 400, 401, and 402, respectively; SEQ ID NO: 405, 406, 407, 408, 409, and 410, respectively; SEQ ID NO: 413, 414, 415, 416, 417, and 418, respectively; SEQ ID NO: 421, 422, 423, 424, 425, and 426, respectively; SEQ ID NO: 429, 430, 431, 432, 433, and 434, respectively; SEQ ID NO: 437, 438, 439, 440, 441, and 442, respectively; SEQ ID NO: 445, 446, 447, 448, 449, and 450, respectively; SEQ ID NO: 453, 454, 455, 456, 457, and 458, respectively; SEQ ID NO: 461, 462, 463, 464, 465, and 466, respectively; SEQ ID NO: 469, 470, 471, 472, 473, and 474, respectively; SEQ ID NO: 477, 478, 479, 480, 481, and 482, respectively; SEQ ID NO: 485, 486, 487, 488, 489, and 490, respectively; SEQ ID NO: 495, 496, 497, 498, 499, and 500, respectively; SEQ ID NO: 503, 504, 505, 506, 507, and 508, respectively; SEQ ID NO: 511, 512, 513, 514, 515, and 516, respectively; SEQ ID NO: 519, 520, 521, 522, 523, and 524, respectively; SEQ ID NO: 527, 528, 529, 530, 531, and 532, respectively; SEQ ID NO: 535, 536, 537, 538, 539, and 540, respectively; SEQ ID NO: 543, 544, 545, 546, 547, and 548, respectively; SEQ ID NO: 551, 552, 553, 554, 555, and 556, respectively; SEQ ID NO: 559, 560, 561, 562, 563, and 564, respectively; SEQ ID NO: 567, 568, 569, 570, 571, and 572, respectively; SEQ ID NO: 575, 576, 577, 578, 579, and 580, respectively; SEQ ID NO: 583, 584, 585, 586, 587, and 588, respectively; SEQ ID NO: 591, 592, 593, 594, 595, and 596, respectively; SEQ ID NO: 599, 600, 601, 602, 603, and 604, respectively; SEQ ID NO: 607, 608, 609, 610, 611, and 612, respectively; SEQ ID NO: 615, 616, 617, 618, 619, and 620, respectively; SEQ ID NO: 623, 624, 625, 626, 627, and 628, respectively; SEQ ID NO: 631, 632, 633, 634, 635, and 636, respectively; SEQ ID NO: 639, 640, 641, 642, 643, and 644, respectively; SEQ ID NO: 647, 648, 649, 650, 651, and 652, respectively; SEQ ID NO: 655, 656, 657, 658, 659, and 660, respectively; SEQ ID NO: 663, 664, 665, 666, 667, and 668, respectively; SEQ ID NO: 671, 672, 673, 674, 675, and 676, respectively; SEQ ID NO: 679, 680, 681, 682, 683, and 684, respectively; SEQ ID NO: 687, 688, 689, 690, 691, and 692, respectively; SEQ ID NO: 695, 696, 697, 698, 699, and 700, respectively; SEQ ID NO: 705, 706, 707, 708, 709, and 710, respectively; SEQ ID NO: 725, 726, 727, 728, 729, and 730, respectively; SEQ ID NO: 733, 734, 735, 736, 737, and 738, respectively; SEQ ID NO: 741, 742, 743, 744, 745, and 746, respectively; SEQ ID NO: 749, 750, 751, 752, 753, and 754, respectively; SEQ ID NO: 759, 760, 761, 762, 763, and 764, respectively; SEQ ID NO: 767, 768, 769, 770, 771, and 772, respectively; SEQ ID NO: 775, 776, 777, 778, 779, and 780, respectively; SEQ ID NO: 783, 784, 785, 786, 787, and 788, respectively; SEQ ID NO: 791, 792, 793, 794, 795, and 796, respectively; SEQ ID NO: 799, 800, 801, 802, 803, and 804, respectively; SEQ ID NO: 807, 808, 809, 810, 811, and 812, respectively; SEQ ID NO: 815, 816, 817, 818, 819, and 820, respectively; SEQ ID NO: 823, 824, 825, 826, 827, and 828, respectively; SEQ ID NO: 831, 832, 833, 834, 835, and 836, respectively; SEQ ID NO: 839, 840, 841, 842, 843, and 844, respectively; SEQ ID NO: 847, 848, 849, 850, 851, and 852, respectively; SEQ ID NO: 855, 856, 857, 858, 859, and 860, respectively; SEQ ID NO: 863, 864, 865, 866, 867, and 868, respectively; SEQ ID NO: 871, 872, 873, 874, 875, and 876, respectively; SEQ ID NO: 879, 880, 881, 882, 883, and 884, respectively; SEQ ID NO: 887, 888, 889, 890, 891, and 892, respectively; SEQ ID NO: 895, 896, 897, 898, 899, and 900, respectively; SEQ ID NO: 903, 904, 905, 906, 907, and 908, respectively; SEQ ID NO: 911, 912, 913, 914, 915, and 916, respectively; SEQ ID NO: 919, 920, 921, 922, 923, and 924, respectively; SEQ ID NO: 927, 928, 929, 930, 931, and 932, respectively; SEQ ID NO: 935, 936, 937, 938, 939, and 940, respectively; SEQ ID NO: 945, 946, 947, 948, 949, and 950, respectively; SEQ ID NO: 953, 954, 955, 956, 957, and 958, respectively; SEQ ID NO: 961, 962, 963, 964, 965, and 966, respectively; SEQ ID NO: 969, 970, 971, 972, 973, and 974, respectively; SEQ ID NO: 977, 978, 979, 980, 981, and 982, respectively; SEQ ID NO: 985, 986, 987, 988, 989, and 990, respectively; SEQ ID NO: 997, 998, 999, 1000, 1001, and 1002, respectively; SEQ ID NO: 1005, 1006, 1007, 1008, 1009, and 1010, respectively; SEQ ID NO: 1013, 1014, 1015, 1016, 1017, and 1018, respectively; SEQ ID NO: 1021, 1022, 1023, 1024, 1025, and 1026, respectively; SEQ ID NO: 1029, 1030, 1031, 1032, 1033, and 1034, respectively; or SEQ ID NO: 1037, 1038, 1039, 1040, 1041, and 1042, respectively.

In some cases, an antibody of the present disclosure targets an immune checkpoint selected from the group consisting of PDL1, B7H4, B7H3, and TIGIT. For example, in some cases, an antibody of the present disclosure comprises CDR-H1, CDR-H2, CDR-H3, CDR-L1, CDR-L2, and CDR-L3 comprising at least 80% sequence identity to: SEQ ID NO: 13, 14, 15, 16, 17, and 18, respectively; SEQ ID NO: 77, 78, 79, 80, 81, and 82, , respectively; SEQ ID NO: 91, 92, 93, 94, 95, and 96, respectively; SEQ ID NO: 99, 100, 101, 102, 103, and 104, respectively; SEQ ID NO: 107, 108, 109, 110, 111, and 112, respectively; SEQ ID NO: 115, 116, 117, 118, 119, and 120, respectively; SEQ ID NO: 123, 124, 125, 126, 127, and 128, respectively; SEQ ID NO: 131, 132, 133, 134, 135, and 136, respectively; SEQ ID NO: 139, 140, 141, 142, 143, and 144, respectively; SEQ ID NO: 147, 148, 149, 150, 151, and 152, respectively; SEQ ID NO: 155, 156, 157, 158, 159, and 160, respectively; SEQ ID NO: 163, 164, 165, 166, 167, and 168, respectively; SEQ ID NO: 171, 172, 173, 174, 175, and 176, respectively; SEQ ID NO: 179, 180, 181, 182, 183, and 184, respectively; SEQ ID NO: 187, 188, 189, 190, 191, and 192, respectively; SEQ ID NO: 195, 196, 197, 198, 199, and 200, respectively; SEQ ID NO: 203, 204, 205, 206, 207 and 208, respectively; SEQ ID NO: 211, 212, 213, 214, 215, and 216, respectively; SEQ ID NO: 219, 220, 221, 222, 223, and 224, respectively; SEQ ID NO: 227, 228, 229, 230, 231, and 232, respectively; SEQ ID NO: 235, 236, 237, 238, 239, and 240, respectively; SEQ ID NO: 243, 244, 245, 246, 247, and 248, respectively; SEQ ID NO: 623, 624, 625, 626, 627, and 628, respectively; SEQ ID NO: 631, 632, 633, 634, 635, and 636, respectively; SEQ ID NO: 639, 640, 641, 642, 643, and 644, respectively; SEQ ID NO: 647, 648, 649, 650, 651, and 652, respectively; SEQ ID NO: 655, 656, 657, 658, 659, and 660, respectively; SEQ ID NO: 663, 664, 665, 666, 667, and 668, respectively; SEQ ID NO: 671, 672, 673, 674, 675, and 676, respectively; SEQ ID NO: 679, 680, 681, 682, 683, and 684, respectively; SEQ ID NO: 687, 688, 689, 690, 691, and 692, respectively; SEQ ID NO: 695, 696, 697, 698, 699, and 700, respectively; SEQ ID NO: 705, 706, 707, 708, 709, and 710, respectively; or SEQ ID NO: 1021, 1022, 1023, 1024, 1025, and 1026, respectively.

In some cases, the CDR-H1, CDR-H2, CDR-H3, CDR-L1, CDR-L2, and CDR-L3 comprise at least 85% sequence identity to the respective 6 indicated sequences. In some cases, the CDR-H1, CDR-H2, CDR-H3, CDR-L1, CDR-L2, and CDR-L3 each comprise at least 90% sequence identity to the respective 6 indicated sequences. In some cases, the CDR-H1, CDR-H2, CDR-H3, CDR-L1, CDR-L2, and CDR-L3 each comprise at least 95% sequence identity to the respective 6 indicated sequences. In some cases, the CDR-H1, CDR-H2, CDR-H3, CDR-L1, CDR-L2, and CDR-L3 each comprise at most one mutation relative to the respective 6 indicated sequences.

### (iv) Exemplary Antibodies

In some cases, an antibody provided herein binds to PDL1. PDL1 is expressed in a broad range of cancers, as well as certain immune cells, and often serves as a primary mechanism for immune suppression in tumor microenvironments. The PDL1 agonism of PD-1 can act as an immune checkpoint, diminishing lymphocyte tumor infiltration, and T-cell receptor mediated proliferation and signaling. While PDL1 antagonism can reverse immune suppression, PDL1 targeting can also localize treatments to the sites of cancers, allowing drugs to selectively target cancer cells or stimulate immune responses in the presence of tumors.

In some cases, an anti-PDL1 antibody comprises CDR-H1, CDR-H2, CDR-H3, CDR-L1, CDR-L2, and CDR-L3 comprising at least 80% sequence identity, at least 85% sequence identity, at least 90% sequence identity, at least 95%, at least 98%, or at least 99% sequence identity to the amino acid sequences of SEQ ID NOs: 13, 14, 15, 16, 17, and 18 respectively. In some cases, the antibody comprises CDR-H1, CDR-H2, CDR-H3, CDR-L1, CDR-L2, and CDR-L3 each comprising at most one mutation relative to the amino acid sequences of SEQ ID NOs: 13, 14, 15, 16, 17, and 18, respectively.

In some embodiments, the antibody comprises a heavy chain comprising an amino acid sequence that is at least 80%, at least 85%, at least 90%, at least 95%, at least 98%, or 99% identical to the amino acid sequence of any one of SEQ ID NO: 1-4 and a light chain comprising an amino acid sequence that is at least 95% at least 96%, at least 97%, at least 98%, at least 99%, or 100% identical to the amino acid sequence of SEQ ID NO: 5. In some embodiments, the antibody comprises a heavy chain comprising an amino acid sequence that is at least 80%, at least 85%, at least 90%, at least 95%, at least 98%, or 99% identical to the amino acid sequence of SEQ ID NO: 1 and a light chain comprising an amino acid sequence that is at least 95% at least 96%, at least 97%, at least 98%, at least 99%, or 100% identical to the amino acid sequence of SEQ ID NO: 5. In some embodiments, the antibody comprises a heavy chain comprising an amino acid sequence that is at least 80%, at least 85%, at least 90%, at least 95%, at least 98%, or 99% identical to the amino acid sequence of SEQ ID NO: 2 and a light chain comprising an amino acid sequence that is at least 95% at least 96%, at least 97%, at least 98%, at least 99%, or 100% identical to the amino acid sequence of SEQ ID NO: 5. In some embodiments, the antibody comprises a heavy chain comprising an amino acid sequence that is at least 80%, at least 85%, at least 90%, at least 95%, at least 98%, or 99% identical to the amino acid sequence of SEQ ID NO: 3 and a light chain comprising an amino acid sequence that is at least 95% at least 96%, at least 97%, at least 98%, at least 99%, or 100% identical to the amino acid sequence of SEQ ID NO: 5. In some embodiments, the antibody comprises a heavy chain comprising an amino acid sequence that is at least 80%, at least 85%, at least 90%, at least 95%, at least 98%, or 99% identical to the amino acid sequence of SEQ ID NO: 4 and a light chain comprising an amino acid sequence that is at least 95% at least 96%, at least 97%, at least 98%, at least 99%, or 100% identical to the amino acid sequence of SEQ ID NO: 5.

In some embodiments, the antibody comprises a heavy chain variable region comprising an amino acid sequence that is at least 80%, at least 85%, at least 90%, at least 95%, at least 98%, or 99% identical to the amino acid sequence of any one of SEQ ID NO: 6-9 and a light chain variable region comprising an amino acid sequence that is at least 95% at least 96%, at least 97%, at least 98%, at least 99%, or 100% identical to the amino acid sequence of SEQ ID NO: 10. In some embodiments, the antibody comprises a heavy chain variable region comprising an amino acid sequence that is at least 80%, at least 85%, at least 90%, at least 95%, at least 98%, or 99% identical to the amino acid sequence of any one of SEQ ID NO: 6 and a light chain variable region comprising an amino acid sequence that is at least 95% at least 96%, at least 97%, at least 98%, at least 99%, or 100% identical to the amino acid sequence of SEQ ID NO: 10. In some embodiments, the antibody comprises a heavy chain variable region comprising an amino acid sequence that is at least 80%, at least 85%, at least 90%, at least 95%, at least 98%, or 99% identical to the amino acid sequence of any one of SEQ ID NO: 7 and a light chain variable region comprising an amino acid sequence that is at least 95% at least 96%, at least 97%, at least 98%, at least 99%, or 100% identical to the amino acid sequence of SEQ ID NO: 10. In some embodiments, the antibody comprises a heavy chain variable region comprising an amino acid sequence that is at least 80%, at least 85%, at least 90%, at least 95%, at least 98%, or 99% identical to the amino acid sequence of any one of SEQ ID NO: 8 and a light chain variable region comprising an amino acid sequence that is at least 95% at least 96%, at least 97%, at least 98%, at least 99%, or 100% identical to the amino acid sequence of SEQ ID NO: 10. In some embodiments, the antibody comprises a heavy chain variable region comprising an amino acid sequence that is at least 80%, at least 85%, at least 90%, at least 95%, at least 98%, or 99% identical to the amino acid sequence of any one of SEQ ID NO: 9 and a light chain variable region comprising an amino acid sequence that is at least 95% at least 96%, at least 97%, at least 98%, at least 99%, or 100% identical to the amino acid sequence of SEQ ID NO: 10.

In some embodiments, the antibody comprises a heavy chain variable region comprising an amino acid sequence that is at least 80%, at least 85%, at least 90%, at least 95%, at least 98%, or 99% identical to the amino acid sequence of SEQ ID NO: 19 and a light chain variable region comprising an amino acid sequence that is at least 95% at least 96%, at least 97%, at least 98%, at least 99%, or 100% identical to the amino acid sequence of SEQ ID NO: 20.

In some embodiments, an antibody provided herein binds to EphA2. In some embodiments, the antibody comprises CDR-H1, CDR-H2, CDR-H3, CDR-L1, CDR-L2, and CDR-L3 comprising the amino acid sequences that are at least 80%, at least 85%, at least 90%, at least 95%, at least 98%, or at least 99% identical to the amino acid sequences of SEQ ID NOs: 21, 22, 23, 24, 25, and 26, respectively.

In some embodiments, the anti-EphA2 antibody comprises a heavy chain variable region comprising an amino acid sequence that is at least 80%, at least 85%, at least 90%, at least 95%, at least 98%, or at least 99% identical to the amino acid sequence of SEQ ID NO: 27 and a light chain variable region comprising an amino acid sequence that is at least 80%, at least 85%, at least 90%, at least 95%, at least 98%, or at least 99% identical to the amino acid sequence of SEQ ID NO: 28. In some embodiments, the anti-EphA2 antibody comprises a heavy chain comprising an amino acid sequence that is at least 80%, at least 85%, at least 90%, at least 95%, at least 98%, or at least 99% identical to the amino acid sequence of SEQ ID NO: 29 or SEQ ID NO: 30 and a light chain comprising an amino acid sequence that is at least 80%, at least 85%, at least 90%, at least 95%, at least 98%, or at least 99% identical to the amino acid sequence of the amino acid sequence of SEQ ID NO: 31. In some embodiments, the anti-EphA2 antibody comprises a heavy chain comprising the amino acid sequence that is at least 80%, at least 85%, at least 90%, at least 95%, at least 98%, or at least 99% identical to the amino acid sequence of SEQ ID NO: 32 or SEQ ID NO: 33 and a light chain comprising the amino acid sequence of SEQ ID NO: 34. In some embodiments, the anti-EphA2 antibody comprises a heavy chain that is at least 80%, at least 85%, at least 90%, at least 95%, at least 98%, or at least 99% identical to the amino acid sequence of SEQ ID NO: 35 or SEQ ID NO: 36 and a light chain comprising the amino acid sequence of SEQ ID NO: 37. In some embodiments, the antibody is h1C1 or 1C1.

In some embodiments, the anti-EphA2 antibody comprises a heavy chain variable region comprising an amino acid sequence that is at least 80%, at least 85%, at least 90%, at least 95%, at least 98%, or 99% identical to the amino acid sequence of SEQ ID NO: 27 and a light chain variable region comprising an amino acid sequence that is at least 80% at least 85%, at least 90%, at least 95%, at least 98%, or 99% identical to the amino acid sequence of SEQ ID NO: 28.

In some cases, an antibody provided herein binds to CD30. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 38, 39, 40, 41, 42, and 43, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 38, 39, 40, 41, 42, and 43, respectively. In some embodiments, the anti-CD30 antibody comprises a heavy chain comprising an amino acid sequence that is at least 80%, at least 85%, at least 90%, at least 95%, at least 98%, or 99% identical to the amino acid sequence of either SEQ ID NO: 46 or 47 and a light chain comprising an amino acid sequence that is at least 80% at least 85%, at least 90%, at least 95%, at least 98%, or 99% identical to the amino acid sequence of SEQ ID NO: 48. In some embodiments, the anti-CD30 antibody comprises a heavy chain variable region comprising an amino acid sequence that is at least 80%, at least 85%, at least 90%, at least 95%, at least 98%, or 99% identical to the amino acid sequence of SEQ ID NO: 44 and a light chain variable region comprising an amino acid sequence that is at least 80% at least 85%, at least 90%, at least 95%, at least 98%, or 99% identical to the amino acid sequence of SEQ ID NO: 45.

In some cases, an antibody provided herein binds to CD228. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 49, 50, 51, 52, 53, and 54, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 49, 50, 51, 52, 53, and 54, respectively. In some embodiments, the anti-CD228 antibody comprises a heavy chain variable region comprising an amino acid sequence that is at least 80%, at least 85%, at least 90%, at least 95%, at least 98%, or 99% identical to the amino acid sequence of SEQ ID NO: 55 and a light chain variable region comprising an amino acid sequence that is at least 80% at least 85%, at least 90%, at least 95%, at least 98%, or 99% identical to the amino acid sequence of SEQ ID NO: 56. In some embodiments, the anti-CD228 antibody comprises a heavy chain comprising an amino acid sequence that is at least 80%, at least 85%, at least 90%, at least 95%, at least 98%, or 99% identical to the amino acid sequence of either of SEQ ID NO: 57 or 58 and a light chain comprising an amino acid sequence that is at least 80% at least 85%, at least 90%, at least 95%, at least 98%, or 99% identical to the amino acid sequence of SEQ ID NO: 59. In some embodiments, the anti-CD228 antibody comprises a heavy chain comprising an amino acid sequence that is at least 80%, at least 85%, at least 90%, at least 95%, at least 98%, or 99% identical to the amino acid sequence of either of SEQ ID NO: 60 or 61 and a light chain comprising an amino acid sequence that is at least 80% at least 85%, at least 90%, at least 95%, at least 98%, or 99% identical to the amino acid sequence of SEQ ID NO: 62.

In some cases, an antibody provided herein binds to avB6. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 63, 64, 65, 66, 67, and 68, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 63, 64, 65, 66, 67, and 68, respectively. In some embodiments, the anti-H2A2 antibody comprises a heavy chain variable region comprising an amino acid sequence that is at least 80%, at least 85%, at least 90%, at least 95%, at least 98%, or 99% identical to the amino acid sequence of SEQ ID NO: 69 and a light chain variable region comprising an amino acid sequence that is at least 80% at least 85%, at least 90%, at least 95%, at least 98%, or 99% identical to the amino acid sequence of SEQ ID NO: 70. In some embodiments, the anti-H2A2 antibody comprises a heavy chain comprising an amino acid sequence that is at least 80%, at least 85%, at least 90%, at least 95%, at least 98%, or 99% identical to the amino acid sequence of either SEQ ID NO: 71 or 72 and a light chain comprising an amino acid sequence that is at least 80% at least 85%, at least 90%, at least 95%, at least 98%, or 99% identical to the amino acid sequence of SEQ ID NO: 73. In some embodiments, the anti-H2A2 antibody comprises a heavy chain comprising an amino acid sequence that is at least 80%, at least 85%, at least 90%, at least 95%, at least 98%, or 99% identical to the amino acid sequence of either SEQ ID NO: 74 or 75 and a light chain comprising an amino acid sequence that is at least 80% at least 85%, at least 90%, at least 95%, at least 98%, or 99% identical to the amino acid sequence of SEQ ID NO: 76.

In some cases, an antibody provided herein binds to B7H4. In some cases, the antibody comprises a set of CDR sequences (CDR-H1, CDR-H2, CDR-H3, CDR-L1, CDR-L2, and CDR-L3, respectively) of which each sequence comprises at least 80% sequence identity, at least 85% sequence identity, at least 90% sequence identity, at least 95%, or 100% sequence identity to amino acid sequences from a set of amino acid sequences selected from the group consisting of SEQ ID NOs: 77-82, SEQ ID NOs: 91-96, SEQ ID NOs: 99-104, SEQ ID NOs: 107-112, SEQ ID NOs: 115-120, SEQ ID NOs: 123-128, SEQ ID NOs: 131-136, SEQ ID NOs: 139-144, SEQ ID NOs: 147-152, SEQ ID NOs: 155-160, SEQ ID NOs: 163-168, SEQ ID NOs: 171-176, SEQ ID NOs: 179-184, SEQ ID NOs: 187-192, SEQ ID NOs: 195-200, SEQ ID NOs: 203-208, SEQ ID NOs: 211-216, SEQ ID NOs: 219-224, SEQ ID NOs: 227-232, SEQ ID NOs: 235-240, and SEQ ID NOs: 243-248. In some cases, the antibody comprises a set of CDR sequences (CDR-H1, CDR-H2, CDR-H3, CDR-L1, CDR-L2, and CDR-L3, respectively) each comprising at most one mutation relative to an amino acid sequence from a set of amino acid sequences selected from the group consisting of SEQ ID NOs: 77-82, SEQ ID NOs: 91-96, SEQ ID NOs: 99-104, SEQ ID NOs: 107-112, SEQ ID NOs: 115-120, SEQ ID NOs: 123-128, SEQ ID NOs: 131-136, SEQ ID NOs: 139-144, SEQ ID NOs: 147-152, SEQ ID NOs: 155-160, SEQ ID NOs: 163-168, SEQ ID NOs: 171-176, SEQ ID NOs: 179-184, SEQ ID NOs: 187-192, SEQ ID NOs: 195-200, SEQ ID NOs: 203-208, SEQ ID NOs: 211-216, SEQ ID NOs: 219-224, SEQ ID NOs: 227-232, SEQ ID NOs: 235-240, and SEQ ID NOs: 243-248. In some cases, the anti-B7H4 antibody comprises a heavy chain and a light chain comprising amino acid sequences that are at least 80%, at least 85%, at least 90%, at least 95%, at least 98%, at least 99%, or at least 100% identical to the amino acid sequences of SEQ ID NO: 85 and 87, SEQ ID NO: 86 and 87, SEQ ID NO: 88 and 90, SEQ ID NO: 89 and 90, SEQ ID NO: 251 and 252, , SEQ ID NO: 253 and 254, SEQ ID NO: 255 and 256, SEQ ID NO: 257 and 258, SEQ ID NO: 259 and 260, SEQ ID NO: 261 and 262, SEQ ID NO: 263 and 264, SEQ ID NO: 265 and 266, SEQ ID NO: 267 and 268, SEQ ID NO: 269 and 270, SEQ ID NO: 271 and 272, SEQ ID NO: 273 and 274, SEQ ID NO: 275 and 276, SEQ ID NO: 277 and 278, SEQ ID NO: 279 and 280, SEQ ID NO: 281 and 282, SEQ ID NO: 283 and 284, SEQ ID NO: 285 and 286, SEQ ID NO: 287 and 288, or SEQ ID NO: 289 and 290, respectively. In some embodiments, the anti-B7H4 antibody comprises a heavy chain variable region and a light chain variable region comprising amino acid sequences that are at least 80%, at least 85%, at least 90%, at least 95%, at least 98%, at least 99%, or at least 100% identical to the amino acid sequences of SEQ ID NO: 83 and 84, SEQ ID NO: 97 and 98, SEQ ID NO: 105 and 106, SEQ ID NO: 113 and 114, SEQ ID NO: 121 and 122, SEQ ID NO: 129 and 130, SEQ ID NO: 137 and 138, SEQ ID NO: 153 and 154, SEQ ID NO: 161 and 162, SEQ ID NO: 169 and 170, SEQ ID NO: 177 and 178, SEQ ID NO: 185 and 186, SEQ ID NO: 193 and 194, SEQ ID NO: 201 and 202, SEQ ID NO: 209 and 210, SEQ ID NO: 217 and 218, SEQ ID NO: 225 and 226, SEQ ID NO: 233 and 234, SEQ ID NO: 241 and 242, or SEQ ID NO: 249 and 250, respectively.

In some cases, an antibody provided herein binds to CD70. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 291, 292, 293, 294, 295, and 296, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 291, 292, 293, 294, 295, and 296, respectively. In some embodiments, the anti-CD70 antibody comprises a heavy chain variable region comprising an amino acid sequence that is at least 80%, at least 85%, at least 90%, at least 95%, at least 98%, or 99% identical to the amino acid sequence of SEQ ID NO: 297 and a light chain variable region comprising an amino acid sequence that is at least 80% at least 85%, at least 90%, at least 95%, at least 98%, or 99% identical to the amino acid sequence of SEQ ID NO: 298. In some embodiments, the anti-CD70 antibody comprises a heavy chain comprising an amino acid sequence that is at least 80%, at least 85%, at least 90%, at least 95%, at least 98%, or 99% identical to the amino acid sequence of SEQ ID NO: 299 and a light chain comprising an amino acid sequence that is at least 80% at least 85%, at least 90%, at least 95%, at least 98%, or 99% identical to the amino acid sequence of SEQ ID NO: 300.

In some cases, an antibody provided herein binds to TROP2. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 301, 302, 303, 304, 305, and 306, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 301, 302, 303, 304, 305, and 306, respectively. In some cases, an antibody provided herein binds to TROP2. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 309, 310, 311, 312, 313, and 314 respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 309, 310, 311, 312, 313, and 314, respectively. In some embodiments, the anti-TROP2 antibody comprises a heavy chain variable region comprising an amino acid sequence that is at least 80%, at least 85%, at least 90%, at least 95%, at least 98%, or 99% identical to the amino acid sequence of SEQ ID NO: 307 and a light chain variable region comprising an amino acid sequence that is at least 80% at least 85%, at least 90%, at least 95%, at least 98%, or 99% identical to the amino acid sequence of SEQ ID NO: 308. In some embodiments, the anti-TROP2 antibody comprises a heavy chain variable region comprising an amino acid sequence that is at least 80%, at least 85%, at least 90%, at least 95%, at least 98%, or 99% identical to the amino acid sequence of SEQ ID NO: 315 and a light chain variable region comprising an amino acid sequence that is at least 80% at least 85%, at least 90%, at least 95%, at least 98%, or 99% identical to the amino acid sequence of SEQ ID NO: 316.

In some cases, an antibody provided herein binds to MICA. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 317, 318, 319, 320, 321, and 322, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 317, 318, 319, 320, 321, and 322, respectively. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 325, 326, 327, 328, 329, and 330, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 325, 326, 327, 328, 329, and 330, respectively. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 333, 334, 335, 336, 337, and 338, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 333, 334, 335, 336, 337, and 338, respectively. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 341, 342, 343, 344, 345, and 346, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 341, 342, 343, 344, 345, and 346, respectively. In some embodiments, the anti-MICA antibody comprises a heavy chain variable region comprising an amino acid sequence that is at least 80%, at least 85%, at least 90%, at least 95%, at least 98%, or 99% identical to the amino acid sequence of SEQ ID NO: 323 and a light chain variable region comprising an amino acid sequence that is at least 80% at least 85%, at least 90%, at least 95%, at least 98%, or 99% identical to the amino acid sequence of SEQ ID NO: 324. In some embodiments, the anti-MICA antibody comprises a heavy chain variable region comprising an amino acid sequence that is at least 80%, at least 85%, at least 90%, at least 95%, at least 98%, or 99% identical to the amino acid sequence of SEQ ID NO: 331 and a light chain variable region comprising an amino acid sequence that is at least 80% at least 85%, at least 90%, at least 95%, at least 98%, or 99% identical to the amino acid sequence of SEQ ID NO: 332. In some embodiments, the anti-MICA antibody comprises a heavy chain variable region comprising an amino acid sequence that is at least 80%, at least 85%, at least 90%, at least 95%, at least 98%, or 99% identical to the amino acid sequence of SEQ ID NO: 340 and a light chain variable region comprising an amino acid sequence that is at least 80% at least 85%, at least 90%, at least 95%, at least 98%, or 99% identical to the amino acid sequence of SEQ ID NO: 340. In some embodiments, the anti-MICA antibody comprises a heavy chain variable region comprising an amino acid sequence that is at least 80%, at least 85%, at least 90%, at least 95%, at least 98%, or 99% identical to the amino acid sequence of SEQ ID NO: 347 and a light chain variable region comprising an amino acid sequence that is at least 80% at least 85%, at least 90%, at least 95%, at least 98%, or 99% identical to the amino acid sequence of SEQ ID NO: 348.

In some cases, an antibody provided herein binds to CD51. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 349, 350, 351, 352, 353, and 354, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 349, 350, 351, 352, 353, and 354, respectively. In some cases, an antibody provided herein binds to CD51. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 357, 358, 359, 360, 361, and 362, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 357, 358, 359, 360, 361, and 362, respectively. In some embodiments, the anti-CD51 antibody comprises a heavy chain variable region comprising an amino acid sequence that is at least 80%, at least 85%, at least 90%, at least 95%, at least 98%, or 99% identical to the amino acid sequence of SEQ ID NO: 355 and a light chain variable region comprising an amino acid sequence that is at least 80% at least 85%, at least 90%, at least 95%, at least 98%, or 99% identical to the amino acid sequence of SEQ ID NO: 356. In some embodiments, the anti-CD51 antibody comprises a heavy chain variable region comprising an amino acid sequence that is at least 80%, at least 85%, at least 90%, at least 95%, at least 98%, or 99% identical to the amino acid sequence of SEQ ID NO: 363 and a light chain variable region comprising an amino acid sequence that is at least 80% at least 85%, at least 90%, at least 95%, at least 98%, or 99% identical to the amino acid sequence of SEQ ID NO: 364.

In some cases, an antibody provided herein binds to gpA33. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 365, 366, 367, 368, 369, and 370, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 365, 366, 367, 368, 369, and 370, respectively. In some embodiments, the anti-gpA33 antibody comprises a heavy chain variable region comprising an amino acid sequence that is at least 80%, at least 85%, at least 90%, at least 95%, at least 98%, or 99% identical to the amino acid sequence of SEQ ID NO: 371 and a light chain variable region comprising an amino acid sequence that is at least 80% at least 85%, at least 90%, at least 95%, at least 98%, or 99% identical to the amino acid sequence of SEQ ID NO: 372.

In some cases, an antibody provided herein binds to IL1Rap. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 373, 374, 375, 376, 377, and 378, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 373, 374, 375, 376, 377, and 378, respectively. In some embodiments, the anti-ILlRap antibody comprises a heavy chain variable region comprising an amino acid sequence that is at least 80%, at least 85%, at least 90%, at least 95%, at least 98%, or 99% identical to the amino acid sequence of SEQ ID NO: 379 and a light chain variable region comprising an amino acid sequence that is at least 80% at least 85%, at least 90%, at least 95%, at least 98%, or 99% identical to the amino acid sequence of SEQ ID NO: 380.

In some cases, an antibody provided herein binds to EpCAM. In some cases, the antibody comprises a set of CDR sequences (CDR-H1, CDR-H2, CDR-H3, CDR-L1, CDR-L2, and CDR-L3, respectively) of which each sequence comprises at least 80% sequence identity, at least 85% sequence identity, at least 90% sequence identity, at least 95%, or 100% sequence identity to amino acid sequences from a set of amino acid sequences selected from the group consisting of SEQ ID NOs: 381-386, SEQ ID NOs: 389-394, SEQ ID NOs: 397-402, SEQ ID NOs: 405-410, SEQ ID NOs: 413-418, or SEQ ID NOs: 421-426. In some embodiments, the anti-EpCAM antibody comprises a heavy chain variable region and a light chain variable region comprising amino acid sequences that are at least 80%, at least 85%, at least 90%, at least 95%, at least 98%, at least 99%, or at least 100% identical to the amino acid sequences of SEQ ID NO: 387 and 388, SEQ ID NO: 395 and 396, SEQ ID NO: 403 and 404, SEQ ID NO: 411 and 412, SEQ ID NO: 419 and 420, or SEQ ID NO: 427 and 428, respectively.

In some cases, an antibody provided herein binds to CD352. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 429, 430, 431, 432, 433, and 434, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 429, 430, 431, 432, 433, and 434, respectively. In some embodiments, the anti-CD352 antibody comprises a heavy chain variable region comprising an amino acid sequence that is at least 80%, at least 85%, at least 90%, at least 95%, at least 98%, or 99% identical to the amino acid sequence of SEQ ID NO: 435 and a light chain variable region comprising an amino acid sequence that is at least 80% at least 85%, at least 90%, at least 95%, at least 98%, or 99% identical to the amino acid sequence of SEQ ID NO: 436.

In some cases, an antibody provided herein binds to CS1. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 437, 438, 439, 440, 441, and 442, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 437, 438, 439, 440, 441, and 442, respectively. In some embodiments, the anti-CS1 antibody comprises a heavy chain variable region comprising an amino acid sequence that is at least 80%, at least 85%, at least 90%, at least 95%, at least 98%, or 99% identical to the amino acid sequence of SEQ ID NO: 443 and a light chain variable region comprising an amino acid sequence that is at least 80% at least 85%, at least 90%, at least 95%, at least 98%, or 99% identical to the amino acid sequence of SEQ ID NO: 444.

In some cases, an antibody provided herein binds to CD38. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 445, 446, 447, 448, 449, and 450, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 445, 446, 447, 448, 449, and 450, respectively. In some embodiments, the anti-CD38 antibody comprises a heavy chain variable region comprising an amino acid sequence that is at least 80%, at least 85%, at least 90%, at least 95%, at least 98%, or 99% identical to the amino acid sequence of SEQ ID NO: 451 and a light chain variable region comprising an amino acid sequence that is at least 80% at least 85%, at least 90%, at least 95%, at least 98%, or 99% identical to the amino acid sequence of SEQ ID NO: 452.

In some cases, an antibody provided herein binds to CD25. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 453, 454, 455, 456, 457, and 458, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 453, 454, 455, 456, 457, and 458, respectively. In some embodiments, the anti-CD25 antibody comprises a heavy chain variable region comprising an amino acid sequence that is at least 80%, at least 85%, at least 90%, at least 95%, at least 98%, or 99% identical to the amino acid sequence of SEQ ID NO: 459 and a light chain variable region comprising an amino acid sequence that is at least 80% at least 85%, at least 90%, at least 95%, at least 98%, or 99% identical to the amino acid sequence of SEQ ID NO: 460.

In some cases, an antibody provided herein binds to ADAM9. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 461, 462, 463, 464, 465, and 466, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 461, 462, 463, 464, 465, and 466, respectively. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 469, 470, 471, 472, 473, and 474, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 469, 470, 471, 472, 473, and 474, respectively. In some embodiments, the anti-ADAM9 antibody comprises a heavy chain variable region comprising an amino acid sequence that is at least 80%, at least 85%, at least 90%, at least 95%, at least 98%, or 99% identical to the amino acid sequence of SEQ ID NO: 467 and a light chain variable region comprising an amino acid sequence that is at least 80% at least 85%, at least 90%, at least 95%, at least 98%, or 99% identical to the amino acid sequence of SEQ ID NO: 468. In some embodiments, the anti-ADAM9 antibody comprises a heavy chain variable region comprising an amino acid sequence that is at least 80%, at least 85%, at least 90%, at least 95%, at least 98%, or 99% identical to the amino acid sequence of SEQ ID NO: 475 and a light chain variable region comprising an amino acid sequence that is at least 80% at least 85%, at least 90%, at least 95%, at least 98%, or 99% identical to the amino acid sequence of SEQ ID NO: 476.

In some cases, an antibody provided herein binds to CD59. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 477, 478, 479, 480, 481, and 482, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 477, 478, 479, 480, 481, and 482, respectively. In some embodiments, the anti-CD59 antibody comprises a heavy chain variable region comprising an amino acid sequence that is at least 80%, at least 85%, at least 90%, at least 95%, at least 98%, or 99% identical to the amino acid sequence of SEQ ID NO: 483 and a light chain variable region comprising an amino acid sequence that is at least 80% at least 85%, at least 90%, at least 95%, at least 98%, or 99% identical to the amino acid sequence of SEQ ID NO: 484.

In some cases, an antibody provided herein binds to CD19. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 485, 486, 487, 488, 489, and 490, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 485, 486, 487, 488, 489, and 490, respectively. In some embodiments, the anti-CD19 antibody comprises a heavy chain variable region comprising an amino acid sequence that is at least 80%, at least 85%, at least 90%, at least 95%, at least 98%, or 99% identical to the amino acid sequence of SEQ ID NO: 491 and a light chain variable region comprising an amino acid sequence that is at least 80% at least 85%, at least 90%, at least 95%, at least 98%, or 99% identical to the amino acid sequence of SEQ ID NO: 492. In some embodiments, the anti-CD19 antibody comprises a heavy chain comprising an amino acid sequence that is at least 80%, at least 85%, at least 90%, at least 95%, at least 98%, or 99% identical to the amino acid sequence of SEQ ID NO: 493 and a light chain comprising an amino acid sequence that is at least 80% at least 85%, at least 90%, at least 95%, at least 98%, or 99% identical to the amino acid sequence of SEQ ID NO: 494.

In some cases, an antibody provided herein binds to CD138. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 495, 496, 497, 498, 499, and 500, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 495, 496, 497, 498, 499, and 500, respectively. In some embodiments, the anti-CD138 antibody comprises a heavy chain variable region comprising an amino acid sequence that is at least 80%, at least 85%, at least 90%, at least 95%, at least 98%, or 99% identical to the amino acid sequence of SEQ ID NO: 501 and a light chain variable region comprising an amino acid sequence that is at least 80% at least 85%, at least 90%, at least 95%, at least 98%, or 99% identical to the amino acid sequence of SEQ ID NO: 502.

In some cases, an antibody provided herein binds to CD166. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 503, 504, 505, 506, 507, and 508, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 503, 504, 505, 506, 507, and 508, respectively. In some embodiments, the anti-CD166 antibody comprises a heavy chain variable region comprising an amino acid sequence that is at least 80%, at least 85%, at least 90%, at least 95%, at least 98%, or 99% identical to the amino acid sequence of SEQ ID NO: 509 and a light chain variable region comprising an amino acid sequence that is at least 80% at least 85%, at least 90%, at least 95%, at least 98%, or 99% identical to the amino acid sequence of SEQ ID NO: 510.

In some cases, an antibody provided herein binds to CD56. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 511, 512, 513, 514, 515, and 516, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 511, 512, 513, 514, 515, and 516, respectively. In some embodiments, the anti-CD56 antibody comprises a heavy chain variable region comprising an amino acid sequence that is at least 80%, at least 85%, at least 90%, at least 95%, at least 98%, or 99% identical to the amino acid sequence of SEQ ID NO: 517 and a light chain variable region comprising an amino acid sequence that is at least 80% at least 85%, at least 90%, at least 95%, at least 98%, or 99% identical to the amino acid sequence of SEQ ID NO: 518.

In some cases, an antibody provided herein binds to CD74. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 519, 520, 521, 522, 523, and 524, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 519, 520, 521, 522, 523, and 524, respectively. In some embodiments, the anti-CD74 antibody comprises a heavy chain variable region comprising an amino acid sequence that is at least 80%, at least 85%, at least 90%, at least 95%, at least 98%, or 99% identical to the amino acid sequence of SEQ ID NO: 525 and a light chain variable region comprising an amino acid sequence that is at least 80% at least 85%, at least 90%, at least 95%, at least 98%, or 99% identical to the amino acid sequence of SEQ ID NO: 526.

In some cases, an antibody provided herein binds to CEACAM5. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 527, 528, 529, 530, 531, and 532, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 527, 528, 529, 530, 531, and 532, respectively. In some embodiments, the anti-CEACAM5 antibody comprises a heavy chain variable region comprising an amino acid sequence that is at least 80%, at least 85%, at least 90%, at least 95%, at least 98%, or 99% identical to the amino acid sequence of SEQ ID NO: 533 and a light chain variable region comprising an amino acid sequence that is at least 80% at least 85%, at least 90%, at least 95%, at least 98%, or 99% identical to the amino acid sequence of SEQ ID NO: 534.

In some cases, an antibody provided herein binds to CanAg. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 535, 536, 537, 538, 539, and 540, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 535, 536, 537, 538, 539, and 540, respectively. In some embodiments, the anti-CanAg antibody comprises a heavy chain variable region comprising an amino acid sequence that is at least 80%, at least 85%, at least 90%, at least 95%, at least 98%, or 99% identical to the amino acid sequence of SEQ ID NO: 541 and a light chain variable region comprising an amino acid sequence that is at least 80% at least 85%, at least 90%, at least 95%, at least 98%, or 99% identical to the amino acid sequence of SEQ ID NO: 542.

In some cases, an antibody provided herein binds to DLL-3. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 543, 544, 545, 546, 547, and 548, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 543, 544, 545, 546, 547, and 548, respectively. In some embodiments, the anti-DLL-3 antibody comprises a heavy chain variable region comprising an amino acid sequence that is at least 80%, at least 85%, at least 90%, at least 95%, at least 98%, or 99% identical to the amino acid sequence of SEQ ID NO: 549 and a light chain variable region comprising an amino acid sequence that is at least 80% at least 85%, at least 90%, at least 95%, at least 98%, or 99% identical to the amino acid sequence of SEQ ID NO: 550.

In some cases, an antibody provided herein binds to DPEP-3. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 551, 552, 553, 554, 555, and 556, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 551, 552, 553, 554, 555, and 556, respectively. In some embodiments, the anti-DPEP-3 antibody comprises a heavy chain variable region comprising an amino acid sequence that is at least 80%, at least 85%, at least 90%, at least 95%, at least 98%, or 99% identical to the amino acid sequence of SEQ ID NO: 557 and a light chain variable region comprising an amino acid sequence that is at least 80% at least 85%, at least 90%, at least 95%, at least 98%, or 99% identical to the amino acid sequence of SEQ ID NO: 558.

In some cases, an antibody provided herein binds to EGFR. In some cases, the antibody comprises a set of CDR sequences (CDR-H1, CDR-H2, CDR-H3, CDR-L1, CDR-L2, and CDR-L3, respectively) of which each sequence comprises at least 80% sequence identity, at least 85% sequence identity, at least 90% sequence identity, at least 95%, or 100% sequence identity to amino acid sequences from a set of amino acid sequences selected from the group consisting of SEQ ID NOs: 559-564, SEQ ID NOs: 567-572, SEQ ID NOs: 575-580, and SEQ ID NOs: 895-900. In some embodiments, the anti-EGFR antibody comprises a heavy chain variable region and a light chain variable region comprising amino acid sequences that are at least 80%, at least 85%, at least 90%, at least 95%, at least 98%, at least 99%, or at least 100% identical to the amino acid sequences of SEQ ID NO: 565 and 566, SEQ ID NO: 573 and 574, SEQ ID NO: 581 and 582, or SEQ ID NO: 901 and 902, respectively.

In some cases, an antibody provided herein binds to FRa. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 583, 584, 585, 586, 587, and 588, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 583, 584, 585, 586, 587, and 588, respectively. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 591, 592, 593, 594, 595, and 596, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 591, 592, 593, 594, 595, and 596, respectively. In some embodiments, the anti-FRa antibody comprises a heavy chain variable region comprising an amino acid sequence that is at least 80%, at least 85%, at least 90%, at least 95%, at least 98%, or 99% identical to the amino acid sequence of SEQ ID NO: 589 and a light chain variable region comprising an amino acid sequence that is at least 80% at least 85%, at least 90%, at least 95%, at least 98%, or 99% identical to the amino acid sequence of SEQ ID NO: 590. In some embodiments, the anti-FRa antibody comprises a heavy chain variable region comprising an amino acid sequence that is at least 80%, at least 85%, at least 90%, at least 95%, at least 98%, or 99% identical to the amino acid sequence of SEQ ID NO: 597 and a light chain variable region comprising an amino acid sequence that is at least 80% at least 85%, at least 90%, at least 95%, at least 98%, or 99% identical to the amino acid sequence of SEQ ID NO: 598.

In some cases, an antibody provided herein binds to MUC-1. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 599, 600, 601, 602, 603, and 604, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 599, 600, 601, 602, 603, and 604, respectively. In some embodiments, the anti-MUC-1 antibody comprises a heavy chain variable region comprising an amino acid sequence that is at least 80%, at least 85%, at least 90%, at least 95%, at least 98%, or 99% identical to the amino acid sequence of SEQ ID NO: 605 and a light chain variable region comprising an amino acid sequence that is at least 80% at least 85%, at least 90%, at least 95%, at least 98%, or 99% identical to the amino acid sequence of SEQ ID NO: 606.

In some cases, an antibody provided herein binds to Mesothelin. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 607, 608, 609, 610, 611, and 612, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 607, 608, 609, 610, 611, and 612, respectively. In some embodiments, the anti-Mesothelin antibody comprises a heavy chain variable region comprising an amino acid sequence that is at least 80%, at least 85%, at least 90%, at least 95%, at least 98%, or 99% identical to the amino acid sequence of SEQ ID NO: 613 and a light chain variable region comprising an amino acid sequence that is at least 80% at least 85%, at least 90%, at least 95%, at least 98%, or 99% identical to the amino acid sequence of SEQ ID NO: 614.

In some cases, an antibody provided herein binds to ROR-1. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 615, 616, 617, 618, 619, and 620, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 615, 616, 617, 618, 619, and 620, respectively. In some embodiments, the anti-ROR-1 antibody comprises a heavy chain variable region comprising an amino acid sequence that is at least 80%, at least 85%, at least 90%, at least 95%, at least 98%, or 99% identical to the amino acid sequence of SEQ ID NO: 621 and a light chain variable region comprising an amino acid sequence that is at least 80% at least 85%, at least 90%, at least 95%, at least 98%, or 99% identical to the amino acid sequence of SEQ ID NO: 622.

In some cases, an antibody provided herein binds to B7H3. In some cases, the antibody comprises a set of CDR sequences (CDR-H1, CDR-H2, CDR-H3, CDR-L1, CDR-L2, and CDR-L3, respectively) of which each sequence comprises at least 80% sequence identity, at least 85% sequence identity, at least 90% sequence identity, at least 95%, or 100% sequence identity to amino acid sequences from a set of amino acid sequences selected from the group consisting of SEQ ID NOs: 623-628, SEQ ID NOs: 631-636, SEQ ID NOs: 639-644, SEQ ID NOs: 647-652, SEQ ID NOs: 655-660, SEQ ID NOs: 663-668, SEQ ID NOs: 671-676, SEQ ID NOs: 679-684, SEQ ID NOs: 687-692, SEQ ID NOs: 695-700, and SEQ ID NOs: 705-710. In some embodiments, the anti-B7H3 antibody comprises a heavy chain variable region and a light chain variable region comprising amino acid sequences that are at least 80%, at least 85%, at least 90%, at least 95%, at least 98%, at least 99%, or at least 100% identical to the amino acid sequences of SEQ ID NO: 629 and 630, SEQ ID NOs: 637 and 638, SEQ ID NOs: 645 and 646, SEQ ID NOs: 653 and 654, SEQ ID NOs: 661 and 662, SEQ ID NOs: 669 and 670, SEQ ID NOs: 677 and 678, SEQ ID NOs: 685 and 686, SEQ ID NOs: 693 and 694, SEQ ID NOs: 701 and 702, SEQ ID NOs: 703 and 704, SEQ ID NOs: 711 and 712, SEQ ID NOs: 713 and 714, and SEQ ID NOs: 715 and 716, respectively.

In some cases, an antibody provided herein binds to HER3. In some embodiments, the anti-HER3 antibody comprises a heavy chain comprising an amino acid sequence that is at least 80%, at least 85%, at least 90%, at least 95%, at least 98%, or 99% identical to the amino acid sequence of SEQ ID NO: 717 and a light chain comprising an amino acid sequence that is at least 80% at least 85%, at least 90%, at least 95%, at least 98%, or 99% identical to the amino acid sequence of SEQ ID NO: 718. In some cases, an antibody provided herein binds to HER3. In some embodiments, the anti-HER3 antibody comprises a heavy chain comprising an amino acid sequence that is at least 80%, at least 85%, at least 90%, at least 95%, at least 98%, or 99% identical to the amino acid sequence of SEQ ID NO: 719 and a light chain comprising an amino acid sequence that is at least 80% at least 85%, at least 90%, at least 95%, at least 98%, or 99% identical to the amino acid sequence of SEQ ID NO: 720. In some cases, an antibody provided herein binds to HER3. In some embodiments, the anti-HER3 antibody comprises a heavy chain comprising an amino acid sequence that is at least 80%, at least 85%, at least 90%, at least 95%, at least 98%, or 99% identical to the amino acid sequence of SEQ ID NO: 721 and a light chain comprising an amino acid sequence that is at least 80% at least 85%, at least 90%, at least 95%, at least 98%, or 99% identical to the amino acid sequence of SEQ ID NO: 722. In some cases, an antibody provided herein binds to HER3. In some embodiments, the anti-HER3 antibody comprises a heavy chain comprising an amino acid sequence that is at least 80%, at least 85%, at least 90%, at least 95%, at least 98%, or 99% identical to the amino acid sequence of SEQ ID NO: 723 and a light chain comprising an amino acid sequence that is at least 80% at least 85%, at least 90%, at least 95%, at least 98%, or 99% identical to the amino acid sequence of SEQ ID NO: 724.

In some cases, an antibody provided herein binds to PTK7. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 725, 726, 727, 728, 729, and 730, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 725, 726, 727, 728, 729, and 730, respectively. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 733, 734, 735, 736, 737, and 738, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 733, 734, 735, 736, 737, and 738, respectively. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 741, 742, 743, 744, 745, and 746, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 741, 742, 743, 744, 745, and 746, respectively. In some cases, the anti-PTK7 antibody comprises a heavy chain comprising an amino acid sequence that is at least 80%, at least 85%, at least 90%, at least 95%, at least 98%, or 99% identical to the amino acid sequence of SEQ ID NO: 731 and a light chain comprising an amino acid sequence that is at least 80% at least 85%, at least 90%, at least 95%, at least 98%, or 99% identical to the amino acid sequence of SEQ ID NO: 732. In some cases, the anti-PTK7 antibody comprises a heavy chain comprising an amino acid sequence that is at least 80%, at least 85%, at least 90%, at least 95%, at least 98%, or 99% identical to the amino acid sequence of SEQ ID NO: 739 and a light chain comprising an amino acid sequence that is at least 80% at least 85%, at least 90%, at least 95%, at least 98%, or 99% identical to the amino acid sequence of SEQ ID NO: 740. In some cases, the anti-PTK7 antibody comprises a heavy chain comprising an amino acid sequence that is at least 80%, at least 85%, at least 90%, at least 95%, at least 98%, or 99% identical to the amino acid sequence of SEQ ID NO: 747 and a light chain comprising an amino acid sequence that is at least 80% at least 85%, at least 90%, at least 95%, at least 98%, or 99% identical to the amino acid sequence of SEQ ID NO: 748.

In some cases, an antibody provided herein binds to ZIP6. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 749, 750, 751, 752, 753, and 754, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 749, 750, 751, 752, 753, and 754, respectively. In some cases, the anti-ZIP6 antibody comprises a heavy chain variable region comprising an amino acid sequence that is at least 80%, at least 85%, at least 90%, at least 95%, at least 98%, or 99% identical to the amino acid sequence of SEQ ID NO: 755 and a light chain variable region comprising an amino acid sequence that is at least 80% at least 85%, at least 90%, at least 95%, at least 98%, or 99% identical to the amino acid sequence of SEQ ID NO: 756. In some embodiments, the anti-ZIP6 antibody comprises a heavy chain comprising an amino acid sequence that is at least 80%, at least 85%, at least 90%, at least 95%, at least 98%, or 99% identical to the amino acid sequence of SEQ ID NO: 757 and a light chain comprising an amino acid sequence that is at least 80% at least 85%, at least 90%, at least 95%, at least 98%, or 99% identical to the amino acid sequence of SEQ ID NO: 758.

In some cases, an antibody provided herein binds to Integrin αvβ6. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 759, 760, 761, 762, 763, and 764, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 759, 760, 761, 762, 763, and 764, respectively. In some embodiments, the anti- Integrin αvβ6 antibody comprises a heavy chain variable region comprising an amino acid sequence that is at least 80%, at least 85%, at least 90%, at least 95%, at least 98%, or 99% identical to the amino acid sequence of SEQ ID NO: 765 and a light chain variable region comprising an amino acid sequence that is at least 80% at least 85%, at least 90%, at least 95%, at least 98%, or 99% identical to the amino acid sequence of SEQ ID NO: 766.

In some cases, an antibody provided herein binds to CD48. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 767, 768, 769, 770, 771, and 772, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 767, 768, 769, 770, 771, and 772, respectively. In some embodiments, the anti-CD48 antibody comprises a heavy chain variable region comprising an amino acid sequence that is at least 80%, at least 85%, at least 90%, at least 95%, at least 98%, or 99% identical to the amino acid sequence of SEQ ID NO: 773 and a light chain variable region comprising an amino acid sequence that is at least 80% at least 85%, at least 90%, at least 95%, at least 98%, or 99% identical to the amino acid sequence of SEQ ID NO: 774.

In some cases, an antibody provided herein binds to IGF-1R. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 775, 776, 777, 778, 779, and 780, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 775, 776, 777, 778, 779, and 780, respectively. In some embodiments, the anti-IGF-1R antibody comprises a heavy chain variable region comprising an amino acid sequence that is at least 80%, at least 85%, at least 90%, at least 95%, at least 98%, or 99% identical to the amino acid sequence of SEQ ID NO: 781 and a light chain variable region comprising an amino acid sequence that is at least 80% at least 85%, at least 90%, at least 95%, at least 98%, or 99% identical to the amino acid sequence of SEQ ID NO: 782.

In some cases, an antibody provided herein binds to Claudin-18.2. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 783, 784, 785, 786, 787, and 788, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 783, 784, 785, 786, 787, and 788, respectively. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 791, 792, 793, 794, 795, and 796, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 791, 792, 793, 794, 795, and 796, respectively. In some embodiments, the anti-Claudin-18.2 antibody comprises a heavy chain variable region comprising an amino acid sequence that is at least 80%, at least 85%, at least 90%, at least 95%, at least 98%, or 99% identical to the amino acid sequence of SEQ ID NO: 789 and a light chain variable region comprising an amino acid sequence that is at least 80% at least 85%, at least 90%, at least 95%, at least 98%, or 99% identical to the amino acid sequence of SEQ ID NO: 790. In some embodiments, the anti-Claudin-18.2 antibody comprises a heavy chain variable region comprising an amino acid sequence that is at least 80%, at least 85%, at least 90%, at least 95%, at least 98%, or 99% identical to the amino acid sequence of SEQ ID NO: 797 and a light chain variable region comprising an amino acid sequence that is at least 80% at least 85%, at least 90%, at least 95%, at least 98%, or 99% identical to the amino acid sequence of SEQ ID NO: 798.

In some cases, an antibody provided herein binds to Nectin-4. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 799, 800, 801, 802, 803, and 804, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 799, 800, 801, 802, 803, and 804, respectively. In some embodiments, the anti-Nectin-4 antibody comprises a heavy chain variable region comprising an amino acid sequence that is at least 80%, at least 85%, at least 90%, at least 95%, at least 98%, or 99% identical to the amino acid sequence of SEQ ID NO: 805 and a light chain variable region comprising an amino acid sequence that is at least 80% at least 85%, at least 90%, at least 95%, at least 98%, or 99% identical to the amino acid sequence of SEQ ID NO: 806.

In some cases, an antibody provided herein binds to SLTRK6. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 807, 808, 809, 810, 811, and 812, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 807, 808, 809, 810, 811, and 812, respectively. In some embodiments, the anti-SLTRK6 antibody comprises a heavy chain variable region comprising an amino acid sequence that is at least 80%, at least 85%, at least 90%, at least 95%, at least 98%, or 99% identical to the amino acid sequence of SEQ ID NO: 813 and a light chain variable region comprising an amino acid sequence that is at least 80% at least 85%, at least 90%, at least 95%, at least 98%, or 99% identical to the amino acid sequence of SEQ ID NO: 814.

In some cases, an antibody provided herein binds to CD142. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 815, 816, 817, 818, 819, and 820, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 815, 816, 817, 818, 819, and 820, respectively. In some embodiments, the anti-CD142 antibody comprises a heavy chain variable region comprising an amino acid sequence that is at least 80%, at least 85%, at least 90%, at least 95%, at least 98%, or 99% identical to the amino acid sequence of SEQ ID NO: 821 and a light chain variable region comprising an amino acid sequence that is at least 80% at least 85%, at least 90%, at least 95%, at least 98%, or 99% identical to the amino acid sequence of SEQ ID NO: 822.

In some cases, an antibody provided herein binds to Sialyl-Thomsen-nouveau antigen (STn). In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 823, 824, 825, 826, 827, and 828, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 823, 824, 825, 826, 827, and 828, respectively.

In some embodiments, the anti-STn antibody comprises a heavy chain variable region comprising an amino acid sequence that is at least 80%, at least 85%, at least 90%, at least 95%, at least 98%, or 99% identical to the amino acid sequence of SEQ ID NO: 829 and a light chain variable region comprising an amino acid sequence that is at least 80% at least 85%, at least 90%, at least 95%, at least 98%, or 99% identical to the amino acid sequence of SEQ ID NO: 830.

In some cases, an antibody provided herein binds to CD20. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 831, 832, 833, 834, 835, and 836, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 831, 832, 833, 834, 835, and 836, respectively. In some embodiments, the anti-CD20 antibody comprises a heavy chain variable region comprising an amino acid sequence that is at least 80%, at least 85%, at least 90%, at least 95%, at least 98%, or 99% identical to the amino acid sequence of SEQ ID NO: 837 and a light chain variable region comprising an amino acid sequence that is at least 80% at least 85%, at least 90%, at least 95%, at least 98%, or 99% identical to the amino acid sequence of SEQ ID NO: 838.

In some cases, an antibody provided herein binds to HER2. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 839, 840, 841, 842, 843, and 844, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 839, 840, 841, 842, 843, and 844, respectively. In some embodiments, the anti-HER2 antibody comprises a heavy chain variable region comprising an amino acid sequence that is at least 80%, at least 85%, at least 90%, at least 95%, at least 98%, or 99% identical to the amino acid sequence of SEQ ID NO: 845 and a light chain variable region comprising an amino acid sequence that is at least 80% at least 85%, at least 90%, at least 95%, at least 98%, or 99% identical to the amino acid sequence of SEQ ID NO: 846.

In some cases, an antibody provided herein binds to CD79b. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 847, 848, 849, 850, 851, and 852, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 847, 848, 849, 850, 851, and 852, respectively. In some embodiments, the anti-CD79b antibody comprises a heavy chain variable region comprising an amino acid sequence that is at least 80%, at least 85%, at least 90%, at least 95%, at least 98%, or 99% identical to the amino acid sequence of SEQ ID NO: 853 and a light chain variable region comprising an amino acid sequence that is at least 80% at least 85%, at least 90%, at least 95%, at least 98%, or 99% identical to the amino acid sequence of SEQ ID NO: 854.

In some cases, an antibody provided herein binds to NaPi2B. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 855, 856, 857, 858, 859, and 860, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 855, 856, 857, 858, 859, and 860, respectively. In some embodiments, the anti-NaPi2B antibody comprises a heavy chain variable region comprising an amino acid sequence that is at least 80%, at least 85%, at least 90%, at least 95%, at least 98%, or 99% identical to the amino acid sequence of SEQ ID NO: 861 and a light chain variable region comprising an amino acid sequence that is at least 80% at least 85%, at least 90%, at least 95%, at least 98%, or 99% identical to the amino acid sequence of SEQ ID NO: 862.

In some cases, an antibody provided herein binds to Muc16. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 863, 864, 865, 866, 867, and 868, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 863, 864, 865, 866, 867, and 868, respectively. In some embodiments, the anti-Muc16 antibody comprises a heavy chain variable region comprising an amino acid sequence that is at least 80%, at least 85%, at least 90%, at least 95%, at least 98%, or 99% identical to the amino acid sequence of SEQ ID NO: 869 and a light chain variable region comprising an amino acid sequence that is at least 80% at least 85%, at least 90%, at least 95%, at least 98%, or 99% identical to the amino acid sequence of SEQ ID NO: 870.

In some cases, an antibody provided herein binds to STEAP1. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 871, 872, 873, 874, 875, and 876, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 871, 872, 873, 874, 875, and 876, respectively. In some embodiments, the anti-STEAP1 antibody comprises a heavy chain variable region comprising an amino acid sequence that is at least 80%, at least 85%, at least 90%, at least 95%, at least 98%, or 99% identical to the amino acid sequence of SEQ ID NO: 877 and a light chain variable region comprising an amino acid sequence that is at least 80% at least 85%, at least 90%, at least 95%, at least 98%, or 99% identical to the amino acid sequence of SEQ ID NO: 878.

In some cases, an antibody provided herein binds to BCMA. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 879, 880, 881, 882, 883, and 884, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 879, 880, 881, 882, 883, and 884, respectively. In some embodiments, the anti-BCMA antibody comprises a heavy chain variable region comprising an amino acid sequence that is at least 80%, at least 85%, at least 90%, at least 95%, at least 98%, or 99% identical to the amino acid sequence of SEQ ID NO: 885 and a light chain variable region comprising an amino acid sequence that is at least 80% at least 85%, at least 90%, at least 95%, at least 98%, or 99% identical to the amino acid sequence of SEQ ID NO: 886.

In some cases, an antibody provided herein binds to c-Met. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 887, 888, 889, 890, 891, and 892, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 887, 888, 889, 890, 891, and 892, respectively. In some embodiments, the anti-c-Met antibody comprises a heavy chain variable region comprising an amino acid sequence that is at least 80%, at least 85%, at least 90%, at least 95%, at least 98%, or 99% identical to the amino acid sequence of SEQ ID NO: 893 and a light chain variable region comprising an amino acid sequence that is at least 80% at least 85%, at least 90%, at least 95%, at least 98%, or 99% identical to the amino acid sequence of SEQ ID NO: 893.

In some cases, an antibody provided herein binds to SLAMF7. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 903, 904, 905, 906, 907, and 908, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 903, 904, 905, 906, 907, and 908, respectively. In some embodiments, the anti-SLAMF7 antibody comprises a heavy chain variable region comprising an amino acid sequence that is at least 80%, at least 85%, at least 90%, at least 95%, at least 98%, or 99% identical to the amino acid sequence of SEQ ID NO: 909 and a light chain variable region comprising an amino acid sequence that is at least 80% at least 85%, at least 90%, at least 95%, at least 98%, or 99% identical to the amino acid sequence of SEQ ID NO: 910.

In some cases, an antibody provided herein binds to C4.4a. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 911, 912, 913, 914, 915, and 916, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 911, 912, 913, 914, 915, and 916, respectively. In some embodiments, the anti-C4.4a antibody comprises a heavy chain variable region comprising an amino acid sequence that is at least 80%, at least 85%, at least 90%, at least 95%, at least 98%, or 99% identical to the amino acid sequence of SEQ ID NO: 917 and a light chain variable region comprising an amino acid sequence that is at least 80% at least 85%, at least 90%, at least 95%, at least 98%, or 99% identical to the amino acid sequence of SEQ ID NO: 918.

In some cases, an antibody provided herein binds to GCC. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 919, 920, 921, 922, 923, and 924, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 919, 920, 921, 922, 923, and 924, respectively. In some embodiments, the anti-GCC antibody comprises a heavy chain variable region comprising an amino acid sequence that is at least 80%, at least 85%, at least 90%, at least 95%, at least 98%, or 99% identical to the amino acid sequence of SEQ ID NO: 925 and a light chain variable region comprising an amino acid sequence that is at least 80% at least 85%, at least 90%, at least 95%, at least 98%, or 99% identical to the amino acid sequence of SEQ ID NO: 926.

In some cases, an antibody provided herein binds to Axl. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 927, 928, 929, 930, 931, and 932, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 927, 928, 929, 930, 931, and 932, respectively. In some embodiments, the anti-Axl antibody comprises a heavy chain variable region comprising an amino acid sequence that is at least 80%, at least 85%, at least 90%, at least 95%, at least 98%, or 99% identical to the amino acid sequence of SEQ ID NO: 933 and a light chain variable region comprising an amino acid sequence that is at least 80% at least 85%, at least 90%, at least 95%, at least 98%, or 99% identical to the amino acid sequence of SEQ ID NO: 933.

In some cases, an antibody provided herein binds to transmembrane glycoprotein NMB (gpNMB). In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 935, 936, 937, 938, 939, and 940, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 935, 936, 937, 938, 939, and 940, respectively. In some embodiments, the anti-gpNMB antibody comprises a heavy chain variable region comprising an amino acid sequence that is at least 80%, at least 85%, at least 90%, at least 95%, at least 98%, or 99% identical to the amino acid sequence of SEQ ID NO: 941 and a light chain variable region comprising an amino acid sequence that is at least 80% at least 85%, at least 90%, at least 95%, at least 98%, or 99% identical to the amino acid sequence of SEQ ID NO: 942. In some embodiments, the anti-gpNMB antibody comprises a heavy chain variable region comprising an amino acid sequence that is at least 80%, at least 85%, at least 90%, at least 95%, at least 98%, or 99% identical to the amino acid sequence of SEQ ID NO: 943 and a light chain variable region comprising an amino acid sequence that is at least 80% at least 85%, at least 90%, at least 95%, at least 98%, or 99% identical to the amino acid sequence of SEQ ID NO: 944.

In some cases, an antibody provided herein binds to Prolactin receptor. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 945, 946, 947, 948, 949, and 950, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 945, 946, 947, 948, 949, and 950, respectively. In some embodiments, the anti-Prolactin receptor antibody comprises a heavy chain variable region comprising an amino acid sequence that is at least 80%, at least 85%, at least 90%, at least 95%, at least 98%, or 99% identical to the amino acid sequence of SEQ ID NO: 951 and a light chain variable region comprising an amino acid sequence that is at least 80% at least 85%, at least 90%, at least 95%, at least 98%, or 99% identical to the amino acid sequence of SEQ ID NO: 952.

In some cases, an antibody provided herein binds to FGFR2. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 953, 954, 955, 956, 957, and 958, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 953, 954, 955, 956, 957, and 958, respectively. In some embodiments, the anti-FGFR2 antibody comprises a heavy chain variable region comprising an amino acid sequence that is at least 80%, at least 85%, at least 90%, at least 95%, at least 98%, or 99% identical to the amino acid sequence of SEQ ID NO: 959 and a light chain variable region comprising an amino acid sequence that is at least 80% at least 85%, at least 90%, at least 95%, at least 98%, or 99% identical to the amino acid sequence of SEQ ID NO: 960.

In some cases, an antibody provided herein binds to CDCP1. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 961, 962, 963, 964, 965, and 966, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 969, 970, 971, 972, 973, and 974, respectively. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 961, 962, 963, 964, 965, and 966, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 969, 970, 971, 972, 973, and 974, respectively. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 977, 978, 979, 980, 981, and 982, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 977, 978, 979, 980, 981, and 982, respectively. In some cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 985, 986, 987, 988, 989, and 990, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 985, 986, 987, 988, 989, and 990, respectively. In some embodiments, the anti-CDCP1 antibody comprises a heavy chain variable region comprising an amino acid sequence that is at least 80%, at least 85%, at least 90%, at least 95%, at least 98%, or 99% identical to the amino acid sequence of SEQ ID NO: 967 and a light chain variable region comprising an amino acid sequence that is at least 80% at least 85%, at least 90%, at least 95%, at least 98%, or 99% identical to the amino acid sequence of SEQ ID NO: 968. In some embodiments, the anti-CDCP1 antibody comprises a heavy chain variable region comprising an amino acid sequence that is at least 80%, at least 85%, at least 90%, at least 95%, at least 98%, or 99% identical to the amino acid sequence of SEQ ID NO: 975 and a light chain variable region comprising an amino acid sequence that is at least 80% at least 85%, at least 90%, at least 95%, at least 98%, or 99% identical to the amino acid sequence of SEQ ID NO: 976. In some embodiments, the anti-CDCP1 antibody comprises a heavy chain variable region comprising an amino acid sequence that is at least 80%, at least 85%, at least 90%, at least 95%, at least 98%, or 99% identical to the amino acid sequence of SEQ ID NO: 983 and a light chain variable region comprising an amino acid sequence that is at least 80% at least 85%, at least 90%, at least 95%, at least 98%, or 99% identical to the amino acid sequence of SEQ ID NO: 984. In some embodiments, the anti-CDCP1 antibody comprises a heavy chain variable region comprising an amino acid sequence that is at least 80%, at least 85%, at least 90%, at least 95%, at least 98%, or 99% identical to the amino acid sequence of SEQ ID NO: 991 and a light chain variable region comprising an amino acid sequence that is at least 80% at least 85%, at least 90%, at least 95%, at least 98%, or 99% identical to the amino acid sequence of SEQ ID NO: 992.

In some cases, an antibody provided herein binds to ASCT2. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 997, 998, 999, 1000, 1001, and 1002, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 997, 998, 999, 1000, 1001, and 1002, respectively. In some embodiments, the anti-ASCT2 antibody comprises a heavy chain variable region comprising an amino acid sequence that is at least 80%, at least 85%, at least 90%, at least 95%, at least 98%, or 99% identical to the amino acid sequence of SEQ ID NO: 993 and a light chain variable region comprising an amino acid sequence that is at least 80% at least 85%, at least 90%, at least 95%, at least 98%, or 99% identical to the amino acid sequence of SEQ ID NO: 994. In some embodiments, the anti-ASCT2 antibody comprises a heavy chain variable region comprising an amino acid sequence that is at least 80%, at least 85%, at least 90%, at least 95%, at least 98%, or 99% identical to the amino acid sequence of SEQ ID NO: 995 and a light chain variable region comprising an amino acid sequence that is at least 80% at least 85%, at least 90%, at least 95%, at least 98%, or 99% identical to the amino acid sequence of SEQ ID NO: 996. In some embodiments, the anti-ASCT2 antibody comprises a heavy chain variable region comprising an amino acid sequence that is at least 80%, at least 85%, at least 90%, at least 95%, at least 98%, or 99% identical to the amino acid sequence of SEQ ID NO: 1003 and a light chain variable region comprising an amino acid sequence that is at least 80% at least 85%, at least 90%, at least 95%, at least 98%, or 99% identical to the amino acid sequence of SEQ ID NO: 1004.

In some cases, an antibody provided herein binds to CD123. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 1005, 1006, 1007, 1008, 1009, and 1010, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 1005, 1006, 1007, 1008, 1009, and 1010, respectively. In some embodiments, the anti-CD123 antibody comprises a heavy chain variable region comprising an amino acid sequence that is at least 80%, at least 85%, at least 90%, at least 95%, at least 98%, or 99% identical to the amino acid sequence of SEQ ID NO: 1011 and a light chain variable region comprising an amino acid sequence that is at least 80% at least 85%, at least 90%, at least 95%, at least 98%, or 99% identical to the amino acid sequence of SEQ ID NO: 1012.

In some cases, an antibody provided herein binds to GPC3. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 1013, 1014, 1015, 1016, 1017, and 1018 respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 1013, 1014, 1015, 1016, 1017, and 1018, respectively. In some embodiments, the anti-TIGIT antibody comprises a heavy chain variable region comprising an amino acid sequence that is at least 80%, at least 85%, at least 90%, at least 95%, at least 98%, or 99% identical to the amino acid sequence of SEQ ID NO: 1027 and a light chain variable region comprising an amino acid sequence that is at least 80% at least 85%, at least 90%, at least 95%, at least 98%, or 99% identical to the amino acid sequence of SEQ ID NO: 1028.

In some cases, an antibody provided herein binds to TIGIT. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 1021, 1022, 1023, 1024, 1025, and 1026, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 1021, 1022, 1023, 1024, 1025, and 1026, respectively. In some embodiments, the anti-BCMA antibody comprises a heavy chain variable region comprising an amino acid sequence that is at least 80%, at least 85%, at least 90%, at least 95%, at least 98%, or 99% identical to the amino acid sequence of SEQ ID NO: 1043 and a light chain variable region comprising an amino acid sequence that is at least 80% at least 85%, at least 90%, at least 95%, at least 98%, or 99% identical to the amino acid sequence of SEQ ID NO: 1044.

In some cases, an antibody provided herein binds to CD33. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 1029, 1030, 1031, 1032, 1033, and 1034, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 1029, 1030, 1031, 1032, 1033, and 1034, respectively. In some embodiments, the anti-CD33 antibody comprises a heavy chain variable region comprising an amino acid sequence that is at least 80%, at least 85%, at least 90%, at least 95%, at least 98%, or 99% identical to the amino acid sequence of SEQ ID NO: 1035 and a light chain variable region comprising an amino acid sequence that is at least 80% at least 85%, at least 90%, at least 95%, at least 98%, or 99% identical to the amino acid sequence of SEQ ID NO: 1036.

In some cases, an antibody provided herein binds to BCMA. In some such cases, the antibody comprises CDR-H1, CDR-H2, CDR-H3, CDR-L1, CDR-L2, and CDR-L3 sequences comprising at least 80% sequence identity, at least 85% sequence identity, at least 90% sequence identity, or at least 95% sequence identity to the amino acid sequences of SEQ ID NOs: 1037, 1038, 1039, 1040, 1041, and 1042, respectively. In some cases, the antibody comprises CDR-H1, CDR-H2, CDR-H3, CDR-L1, CDR-L2, and CDR-L3 sequences each comprising at most one mutation relative to the amino acid sequences of SEQ ID NOs: 1037, 1038, 1039, 1040, 1041, and 1042, respectively. In some embodiments, the anti-BCMA antibody comprises a heavy chain variable region comprising an amino acid sequence that is at least 80%, at least 85%, at least 90%, at least 95%, at least 98%, or 99% identical to the amino acid sequence of SEQ ID NO: 1043 and a light chain variable region comprising an amino acid sequence that is at least 80% at least 85%, at least 90%, at least 95%, at least 98%, or 99% identical to the amino acid sequence of SEQ ID NO: 1044.

Throughout this application, unless the context indicates otherwise, references to a compound of Formula (I), (I'), (I^{∗}), (II), or any subformula thereof, includes all subgroups of Formula (I), (I'), (I^{∗}), (II), or any subformula thereof, defined herein, including all substructures, subgenera, preferences, embodiments, examples and particular compounds defined and/or described herein. References to a compound of Formula (I), (I'), (I^{∗}), (II), or any subformula thereof, include ionic forms, polymorphs, pseudopolymorphs, amorphous forms, solvates, co-crystals, chelates, isomers, tautomers, oxides (e.g., N-oxides, S-oxides), esters, prodrugs, isotopes and/or protected forms thereof. In some embodiments, references to a compound of Formula (I), (I'), (I^{∗}), (II), or any subformula thereof, include polymorphs, solvates, co-crystals, isomers, tautomers and/or oxides thereof. In some embodiments, references to a compound of Formula (I), (I'), (I^{∗}), (II), or any subformula thereof, include polymorphs, solvates, and/or co-crystals thereof. In some embodiments, references to a compound of Formula (I), (I'), (I^{∗}), (II), or any subformula thereof, include isomers, tautomers and/or oxides thereof. In some embodiments, references to a compound of Formula (I), (I'), (I^{∗}), (II), or any subformula thereof, include solvates thereof. Similarly, the term "salts" includes solvates of salts of compounds.

Any formula given herein, such as Formula (I), (I'), (I^{∗}), (II), or any subformula thereof, is intended to represent compounds having structures depicted by the structural formula as well as certain variations or forms. In particular, compounds of any formula given herein may have asymmetric centers and therefore exist in different enantiomeric or diastereomeric forms. All optical isomers and stereoisomers of the compounds of the general formula, and mixtures thereof in any ratio, are considered within the scope of the formula. Thus, any formula given herein is intended to represent a racemate, one or more enantiomeric forms, one or more diastereomeric forms, one or more atropisomeric forms, and mixtures thereof in any ratio. Where a compound of one of Tables 1, 2, or 3 is depicted with a particular stereochemical configuration, also provided herein is any alternative stereochemical configuration of the compound, as well as a mixture of stereoisomers of the compound in any ratio. For example, where a compound of Tables 1, 2, or 3 has a stereocenter that is in an "S" stereochemical configuration, also provided herein is enantiomer of the compound wherein that stereocenter is in an "R" stereochemical configuration. Likewise, when a compound of Tables 1, 2, or 3 has a stereocenter that is in an "R" configuration, also provided herein is enantiomer of the compound in an "S" stereochemical configuration. Also provided are mixtures of the compound with both the "S" and the "R" stereochemical configuration. Additionally, if a compound of Tables 1, 2, or 3 has two or more stereocenters, also provided are any enantiomer or diastereomer of the compound. For example, if a compound of Tables 1, 2, or 3 contains a first stereocenter and a second stereocenter with "R" and "R" stereochemical configurations, respectively, also provided are stereoisomers of the compound having first and second stereocenters with "S" and "S" stereochemical configurations, respectively, "S" and "R" stereochemical configurations, respectively, and "R" and "S" stereochemical configurations, respectively. If a compound of Tables 1, 2, or 3 contains a first stereocenter and a second stereocenter with "S" and "S" stereochemical configurations, respectively, also provided are stereoisomers of the compound having first and second stereocenters with "R" and "R" stereochemical configurations, respectively, "S" and "R" stereochemical configurations, respectively, and "R" and "S" stereochemical configurations, respectively. If a compound of Tables 1, 2, or 3 contains a first stereocenter and a second stereocenter with "S" and "R" stereochemical configurations, respectively, also provided are stereoisomers of the compound having first and second stereocenters with "R" and "S" stereochemical configurations, respectively, "R" and "R" stereochemical configurations, respectively, and "S" and "S" stereochemical configurations, respectively. Similarly, if a compound of Tables 1, 2, or 3 contains a first stereocenter and a second stereocenter with "R" and "S" stereochemical configurations, respectively, also provided are stereoisomers of the compound having first and second stereocenters with "S" and "R" stereochemical configurations, respectively, "R" and "R" stereochemical configurations, respectively, and "S" and "S" stereochemical configurations, respectively. Furthermore, certain structures may exist as geometric isomers (i.e., *cis* and *trans* isomers), as tautomers, or as atropisomers. Additionally, any formula given herein is intended to refer also to any one of hydrates, solvates, and amorphous and polymorphic forms of such compounds, and mixtures thereof, even if such forms are not listed explicitly. In some embodiments, the solvent is water and the solvates are hydrates.

The compounds depicted herein may be present as salts even if salts are not depicted, and it is understood that the compositions and methods provided herein embrace all salts and solvates of the compounds depicted here, as well as the non-salt and non-solvate form of the compound, as is well understood by the skilled artisan. In some embodiments, the salts of the compounds provided herein are pharmaceutically acceptable salts.

Embodiments are contemplated wherein any variation or embodiment of Q, D, Z or Z', A, B, S^{∗}, RL, Y, W, L, Z^{a}, X^{b}, R¹, R², R³, R⁴, R⁵, R⁶, R⁷, R⁸, R⁹, R¹⁰, R^{a}, R^{b}, E, X, subscript n, subscript n, subscript q, and/or subscript p provided herein is combined with every other variation or embodiment of Q, D, Z or Z', A, B, S^{∗}, RL, Y, W, L, Z^{a}, X^{b}, R¹, R², R³, R⁴, R⁵, R⁶, R⁷, R⁸, R⁹, R¹⁰, R^{a}, R^{b}, E, X, subscript n, subscript n, subscript q, and/or subscript p, as if each combination had been individually and specifically described.

In some embodiments, any of the compounds described herein, such as a compound of Formula (I), (I'), (I^{∗}), (II), or any sub formula thereof, or a compound of any one of Tables 1, 2 or 3, or a salt of any of the foregoing, may be deuterated (e.g., a hydrogen atom is replaced by a deuterium atom). In some of these variations, the compound is deuterated at a single site. In other variations, the compound is deuterated at multiple sites. Deuterated compounds are sometimes prepared from deuterated starting materials in a manner similar to the preparation of the corresponding non-deuterated compounds. In some aspects, hydrogen atoms are be replaced with deuterium atoms using other methods known in the art.

Other embodiments will be apparent to those skilled in the art from the following detailed description.

As used herein, when any variable occurs more than one time in a chemical formula, its definition on each occurrence is independent of its definition at every other occurrence.

### Ligand-Drug Conjugate Mixtures and Compositions

The present invention provides Ligand-Drug Conjugate mixtures and pharmaceutical compositions comprising any of the Ligand-Drug Conjugates described herein. The mixtures and pharmaceutical compositions comprise a plurality of conjugates. In some embodiments, each of the conjugates in the mixture or composition is identical or substantially identical, however, the distribution of drug-linkers on the ligands in the mixture or compositions may vary as well as the drug loading. For example, the conjugation technology used to conjugate drug-linkers to antibodies as the targeting agent in some embodiments results in a composition or mixture that is heterogeneous with respect to the distribution of Drug-Linker compounds on the antibody (Ligand Unit) within the mixture and/or composition. In some of those embodiments, the loading of Drug-Linker compounds on each of the antibody molecules in a mixture or composition of such molecules is an integer that ranges from 1 to 16.

In those embodiments, when referring to the composition as a whole, the loading of drug-linkers is a number ranging from 1 to about 16. Within the composition or mixture, there sometimes is a small percentage of unconjugated antibodies. The average number of drug-linkers per Ligand Unit in the mixture or composition (i.e., average drug-load) is an important attribute as it determines the maximum amount of drug that is delivered to the target cell. Typically, the average drug load is 1, 2 or about 2, 3 or about 3, 4 or about 4, 5 or about 5, 6 or about 6, 7 or about 7, 8 or about 8, 9 or about 9, 10 or about 10, 11 or about 11, 12 or about 12, 13 or about 13, 14 or about 14, 15 or about 15, 16 or about 16. In some embodiments, the average drug loading is from about 1 to about 16, from about 2 to about 12, from about 3 to about 8, or about 4. In some embodiments, the average drug loading is from about 3 to about 5, from about 3.5 to about 4.5, from about 3.7 to about 4.3, from about 3.9 to about 4.3, or about 4.

In some embodiments, the mixtures and pharmaceutical compositions comprise a plurality (i.e., population) of conjugates, however, the conjugates are identical or substantially identical and are substantially homogenous with respect to the distribution of drug-linkers on the ligand molecules within the mixture and/or composition and with respect to loading of drug-linkers on the ligand molecules within the mixture and/or composition. In some such embodiments, the loading of drug-linkers on an antibody Ligand Unit is 2 or 4. Within the composition or mixture, there may also be a small percentage of unconjugated antibodies. The average drug load in such embodiments is about 2 or about 4. Typically, such compositions and mixtures result from the use of site-specific conjugation techniques and conjugation is due to an introduced cysteine residue.

The average number of Drug Units or Drug-Linker compounds per Ligand Unit in a preparation from a conjugation reaction may be characterized by conventional means such as mass spectrometry, ELISA assay, HPLC (e.g., HIC). In those instances, the quantitative distribution of Ligand-Drug Conjugates in terms of subscript p may also be determined. In other instances, separation, purification, and characterization of homogeneous Ligand-Drug Conjugates may be achieved by conventional means such as reverse phase HPLC or electrophoresis.

In some embodiments, the compositions are pharmaceutical compositions comprising the Ligand-Drug Conjugates described herein and a pharmaceutically acceptable carrier. In some of those embodiments, the pharmaceutical composition is in liquid form. In some embodiments, the pharmaceutical composition is a solid. In other of those embodiments, the pharmaceutical composition is a lyophilized powder.

The compositions, including pharmaceutical compositions, are provided in purified form. As used herein, "purified" means that when isolated, the isolate contains at least 95%, and in other embodiments at least 98%, of Conjugate by weight of the isolate.

### Methods of Use

### Treatment of Cancer

The Ligand-Drug Conjugates are useful for inhibiting the multiplication of a tumor cell or cancer cell, causing apoptosis in a tumor or cancer cell, or for treating a cancer in a patient. The Ligand-Drug Conjugates are used accordingly in a variety of settings for the treatment of cancers. The Ligand-Drug Conjugates are intended to deliver a drug to a tumor cell or cancer cell. Without being bound by theory, in one embodiment, the Ligand Unit of a Ligand-Drug Conjugate binds to or associates with a cancer-cell or a tumor-cell-associated antigen, and the Ligand-Drug Conjugate is taken up (internalized) inside the tumor cell or cancer cell through receptor-mediated endocytosis or other internalization mechanism. In some embodiments, the antigen is attached to a tumor cell or cancer cell or is an extracellular matrix protein associated with the tumor cell or cancer cell. Once inside the cell, via activation of the Activation Unit, the drug is released within the cell. In an alternative embodiment, the free drug is released from the Ligand-Drug Conjugate outside the tumor cell or cancer cell, and the free drug subsequently penetrates the cell.

In one embodiment, the Ligand Unit binds to the tumor cell or cancer cell.

In another embodiment, the Ligand Unit binds to a tumor cell or cancer cell antigen which is on the surface of the tumor cell or cancer cell.

In another embodiment, the Ligand Unit binds to a tumor cell or cancer cell antigen that is an extracellular matrix protein associated with the tumor cell or cancer cell.

The specificity of the Ligand Unit for a particular tumor cell or cancer cell is an important consideration for determining the tumors or cancers that are most effectively treated. For example, Ligand-Drug Conjugates that target a cancer cell antigen present on hematopoietic cancers are useful treating hematologic malignancies (e.g., anti-CD30, anti-CD70, anti-CD19, anti-CD33 binding Ligand Unit (e.g., antibody) are useful for treating hematologic malignancies). Ligand-Drug Conjugates that target a cancer cell antigen present on solid tumors in some embodiments are useful treating such solid tumors.

Cancers that are intended to be treated with a Drug-Linker Conjugate include, but are not limited to, hematopoietic cancers such as, for example, lymphomas (Hodgkin Lymphoma and Non-Hodgkin Lymphomas) and leukemias and solid tumors. Examples of hematopoietic cancers include, follicular lymphoma, anaplastic large cell lymphoma, mantle cell lymphoma, acute myeloblastic leukemia, chronic myelocytic leukemia, chronic lymphocytic leukemia, diffuse large B cell lymphoma, and multiple myeloma. Examples of solid tumors include fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophageal cancer, stomach cancer, oral cancer, nasal cancer, throat cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular cancer, small cell lung carcinoma, bladder carcinoma, lung cancer, epithelial carcinoma, glioma, glioblastoma multiforme, astrocytoma, medulloblastoma, craniopharyngioma, ependymoma, pinealoma, hemangioblastoma, acoustic neuroma, oligodendroglioma, meningioma, neuroblastoma, and retinoblastoma.

In some embodiments, the treated cancer is any one of the above-listed lymphomas and leukemias.

### Multi-Modality Therapy for Cancer

Cancers, including, but not limited to, a tumor, metastasis, or other disease or disorder characterized by uncontrolled cell growth are intended to be treated or inhibited by administration of an effective amount of a Ligand-Drug Conjugate.

In one group of embodiments, methods for treating cancer are provided, including administering to a patient in need thereof an effective amount of a Ligand-Drug Conjugate and a chemotherapeutic agent. In one embodiment the chemotherapeutic agent is one in which treatment of the cancer has not been found to be refractory to that agent. In another embodiment, the chemotherapeutic agent one in which the treatment of cancer has been found to be refractory to that agent.

In another group of embodiments, the Ligand-Drug Conjugates is administered to a patient that has also undergone surgery as treatment for the cancer. In such embodiments a chemotherapeutic agent is typically administered over a series of sessions, or one or a combination of the chemotherapeutic agents, such a standard of care chemotherapeutic agent(s), is administered.

In either group of embodiments, the patient also receives an additional treatment, such as radiation therapy. In a specific embodiment, the Ligand-Drug Conjugate is administered concurrently with the chemotherapeutic agent or with radiation therapy. In another specific embodiment, the chemotherapeutic agent or radiation therapy is administered prior or subsequent to administration of a Ligand-Drug Conjugate.

Additionally, methods of treatment of cancer with a Ligand-Drug Conjugate are provided as an alternative to chemotherapy or radiation therapy where the chemotherapy or the radiation therapy has proven or can prove too toxic, e.g., results in unacceptable or unbearable side effects, for the subject being treated. The patient being treated is optionally treated with another cancer treatment such as surgery, radiation therapy or chemotherapy, depending on which treatment is found to be acceptable or bearable.

### Treatment of Autoimmune Diseases

The Ligand-Drug Conjugates are intended to be useful for killing or inhibiting the unwanted replication of cells that produce an autoimmune disease or for treating an autoimmune disease.

The Ligand-Drug Conjugates are used accordingly in a variety of settings for the treatment of an autoimmune disease in a patient. The Ligand-Drug Conjugates are typically used to deliver a Drug Unit to a target cell. Without being bound by theory, in one embodiment, the Ligand-Drug Conjugate associates with an antigen on the surface of a pro-inflammatory or inappropriately stimulated immune cell, and the Ligand-Drug Conjugate is then taken up inside the targeted cell through receptor-mediated endocytosis. Once inside the cell, the Linker Unit is cleaved, resulting in release of the Drug Unit as free drug. The free drug is then able to migrate within the cytosol and induce a cytotoxic or cytostatic activity. In an alternative embodiment, the Drug Unit is cleaved from the Ligand-Drug Conjugate outside the target cell, and the free drug resulting from that release subsequently penetrates the cell.

In one embodiment, the Ligand Unit binds to an autoimmune antigen. In one such embodiment, the antigen is on the surface of a cell involved in an autoimmune condition.

In one embodiment, the Ligand Unit binds to activated lymphocytes that are associated with the autoimmune disease state.

In a further embodiment, the Ligand-Drug Conjugate kills or inhibits the multiplication of cells that produce an autoimmune antibody associated with a particular autoimmune disease.

Particular types of autoimmune diseases intended to be treated with the Ligand-Drug Conjugates include, but are not limited to, Th2 lymphocyte related disorders (*e.g.,* atopic dermatitis, atopic asthma, rhinoconjunctivitis, allergic rhinitis, Omenn's syndrome, systemic sclerosis, and graft versus host disease); Th1 lymphocyte-related disorders (*e.g.,* rheumatoid arthritis, multiple sclerosis, psoriasis, Sjorgren's syndrome, Hashimoto's thyroiditis, Grave's disease, primary biliary cirrhosis, Wegener's granulomatosis, and tuberculosis); and activated B lymphocyte-related disorders (e.g., systemic lupus erythematosus, Goodpasture's syndrome, rheumatoid arthritis, and type I diabetes).

### Multi-Drug Therapy of Autoimmune Diseases

Methods for treating an autoimmune disease are also disclosed including administering to a patient in need thereof an effective amount of a Ligand-Drug Conjugate and another therapeutic agent known for the treatment of an autoimmune disease.

For any of the methods of treatment described herein, the Ligand-Drug Conjugate compound may be administered to a subject who is unable to tolerate another Ligand-Drug Conjugate compound in therapeutically effective doses. In some embodiments, the other Ligand-Drug Conjugate compound comprises monomethyl auristain E (MMAE) or monomethyl auristatin F (MMAF). In some embodiments, the patient has greater tolerance for a Ligand-Drug Conjugate compound provided herein as compared to another Ligand-Drug Conjugate compound (e.g., a Ligand-Drug Conjugate compound comprising MMAE or MMAF). Tolerability may be determined using known methods or any of the methods decribed herein.

### Compositions and Methods of Administration

The present invention provides pharmaceutical compositions comprising the Ligand-Drug Conjugates described herein and at least one pharmaceutically acceptable carrier. The pharmaceutical composition is in any form that allows the compound to be administered to a patient for treatment of a disorder associated with expression of the antigen to which the Ligand unit binds. For example, the conjugates are in the form of a liquid or solid. The preferred route of administration is parenteral. Parenteral administration includes subcutaneous injections, intravenous, intramuscular, intrasternal injection or infusion techniques. In one embodiment, the pharmaceutical compositions is administered parenterally. In one embodiment, the conjugates are administered intravenously. Administration is by any convenient route, for example by infusion or bolus injection.

Pharmaceutical compositions are formulated to allow a Ligand-Drug Conjugate to be bioavailable upon administration of the composition to a patient. Compositions sometimes take the form of one or more dosage units.

Materials used in preparing the pharmaceutical compositions are preferably nontoxic in the amounts used. It will be evident to those of ordinary skill in the art that the optimal dosage of the active ingredient(s) in the pharmaceutical composition will depend on a variety of factors. Relevant factors include, without limitation, the type of animal (e.g., human), the particular form of the compound, the manner of administration, and the composition employed.

The composition in some embodiments is in the form of a liquid. The liquid in some of those embodiments is useful for delivery by injection. In some embodiments a composition for administration by injection, in addition to the Ligand-Drug Conjugate, contains one or more excipients selected from the group consisting of a surfactant, preservative, wetting agent, dispersing agent, suspending agent, buffer, stabilizer and isotonic agent.

The liquid compositions, whether they are solutions, suspensions or other like form, in some embodiments include one or more of the following: sterile diluents such as water for injection, saline solution, preferably physiological saline, Ringer's solution, isotonic sodium chloride, fixed oils such as synthetic mono or digylcerides which can serve as the solvent or suspending medium, polyethylene glycols, glycerin, cyclodextrin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as amino acids, acetates, citrates or phosphates; detergents, such as nonionic surfactants, polyols; and agents for the adjustment of tonicity such as sodium chloride or dextrose. A parenteral composition is sometimes enclosed in ampoule, a disposable syringe or a multiple-dose vial made of glass, plastic or other material. Physiological saline is an exemplary adjuvant. An injectable composition is preferably sterile.

The amount of the conjugate that is effective in the treatment of a particular disorder or condition will depend on the nature of the disorder or condition, which in some embodiments is determined by standard clinical techniques. In addition, *in vitro* or *in vivo* assays are optionally employed to help identify optimal dosage ranges. The precise dose to be employed in the compositions will also depend on the route of administration, and the seriousness of the disease or disorder, and should be decided according to the judgment of the practitioner and each patient's circumstances.

The compositions comprise an effective amount of a Ligand-Drug Conjugate such that a suitable dosage amount will be obtained. Typically, that amount is at least about 0.01% of a compound by weight of the composition.

For intravenous administration, the pharmaceutical composition typically comprises from about 0.01 to about 100 mg of a Ligand-Drug Conjugate per kg of the animal's body weight. In one embodiment, the composition can include from about 1 to about 100 mg of a Ligand-Drug Conjugate per kg of the animal's body weight. In another aspect, the amount administered will be in the range from about 0.1 to about 25 mg/kg of body weight of a compound. In some aspects, depending on the drug used, the dosage is even lower, for example, 1.0 µg/kg to 5.0 mg/kg, 4.0 mg/kg, 3.0 mg/kg, 2.0 mg/kg or 1.0 mg/kg, or 1.0 µg/kg to 500.0 µg/kg of the subject's body weight.

Generally, the dosage of a conjugate administered to a patient is typically about 0.01 mg/kg to about 100 mg/kg of the subject's body weight or from 1.0 µg/kg to 5.0 mg/kg of the subject's body weight. In some embodiments, the dosage administered to a patient is between about 0.01 mg/kg to about 15 mg/kg of the subject's body weight. In some embodiments, the dosage administered to a patient is between about 0.1 mg/kg and about 15 mg/kg of the subject's body weight. In some embodiments, the dosage administered to a patient is between about 0.1 mg/kg and about 20 mg/kg of the subject's body weight. In some embodiments, the dosage administered is between about 0.1 mg/kg to about 5 mg/kg or about 0.1 mg/kg to about 10 mg/kg of the subject's body weight. In some embodiments, the dosage administered is between about 1 mg/kg to about 15 mg/kg of the subject's body weight. In some embodiments, the dosage administered is between about 1 mg/kg to about 10 mg/kg of the subject's body weight. In some embodiments, the dosage administered is between about 0.1 to 4 mg/kg, even more preferably 0.1 to 3.2 mg/kg, or even more preferably 0.1 to 2.7 mg/kg of the subject's body weight over a treatment cycle.

The term "carrier" refers to a diluent, adjuvant or excipient, with which a compound is administered. Such pharmaceutical carriers in some embodiments is a liquid, such as water and oils, including those of petroleum, animal, vegetable or synthetic origin, such as peanut oil, soybean oil, mineral oil, sesame oil. Other carriers include saline, gum acacia, gelatin, starch paste, talc, keratin, colloidal silica, urea. In addition, auxiliary, stabilizing, thickening, lubricating and coloring agents are sometimes used. In one embodiment, when administered to a patient, the Ligand-Drug Conjugate or compositions thereof and pharmaceutically acceptable carriers are sterile.

Water is an exemplary carrier when the compounds are administered intravenously. Saline solutions and aqueous dextrose and glycerol solutions are often employed as liquid carriers, particularly for injectable solutions. Suitable pharmaceutical carriers also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol. The present compositions, if desired, also contain minor amounts of wetting or emulsifying agents, or pH buffering agents.

In an embodiment, the conjugates are formulated in accordance with routine procedures as a pharmaceutical composition adapted for intravenous administration to animals, particularly human beings. Typically, the carriers or vehicles for intravenous administration are sterile isotonic aqueous buffer solutions. Where necessary, the compositions include a solubilizing agent. Compositions for intravenous administration optionally comprise a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachets indicating the quantity of active agent. Where a conjugate is to be administered by infusion, it is typically dispensed, for example, with an infusion bottle containing sterile pharmaceutical grade water or saline. In some aspects, where the conjugate is administered by injection, an ampoule of sterile water for injection or saline is sometimes provided and the ingredients are mixed prior to administration.

The pharmaceutical compositions are generally formulated as sterile, substantially isotonic and in full compliance with all Good Manufacturing Practice (GMP) regulations of the U.S. Food and Drug Administration.

### Methods of Preparing Drug-Linker compounds and Ligand-Drug Conjugate compounds

Provided herein are methods for preparing Drug-Linker compounds from the auristatin compounds provided herein. In addition to the methods recited below, method of preparing Drug-Linker compounds from auristatin compounds will be well known to a person of skill in the art.

Provided herein are methods for preparing Ligand-Drug Conjugate compounds from the auristatin compounds and Drug-Linker compounds provided herein. Methods for preparing the Ligand-Drug Conjugate compounds will be well known to a person of skill in the art.

### NUMBERED EMBODIMENTS

Aspects of the invention will now be described with reference to the following numbered clauses:
1. A compound of Formula (I): 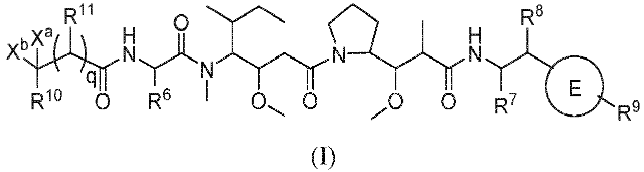 or a salt thereof, wherein
   X^{b} is -NR¹R²; and X^{a} is 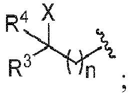 or
   X^{a} and X^{b} are taken together with the carbon atom to which they are attached to form 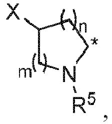 wherein the asterisk denotes the carbon atom of Formula (I) that bears the X^{a} and X^{b} groups;
   R¹, R², R³, R⁴, R^{a}, R^{b}, R⁵, and R¹⁰ are each independently H or C₁-C₄ alkyl;
   X is H, OH, -C(O)NR^{a}R^{b}, -S(O)₂R^{a}, -S(O)-R^{a} -S(O)₂NR^{a}R^{b}, -NHS(O)₂R^{a}, or -NHC(O)R^{a};
   R⁶ is C₁-C₄ alkyl optionally substituted with OH;
   R⁷ is H, C₁-C₄ alkyl optionally substituted with one or two OH moieties, or 5-6 membered heteroaryl;
   E is phenyl or 5-6 membered heteroaryl;
   R⁸, R⁹, and R¹¹ are each independently H or OH;
   n is 0, 1, 2, or 3;
   m is 1, 2, 3, or 4; and
   q is 0 or 1,
   wherein when X is H, at least two of R⁷, R⁸, R⁹, and R¹¹ comprise an OH moiety.
2. A compound of Formula (II): 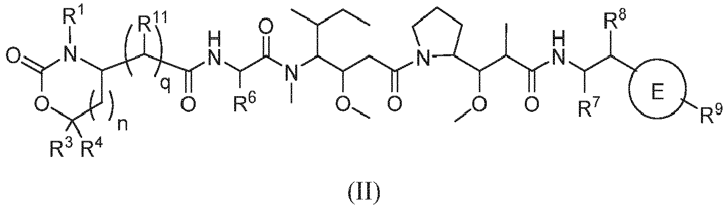 or a salt thereof, wherein
   R¹, R³, and R⁴ are independently H or C₁-C₄ alkyl;
   R⁶ is C₁-C₄ alkyl optionally substituted with OH;
   R⁷ is H, C₁-C₄ alkyl optionally substituted with one or two OH moieties, or 5-6 membered heteroaryl;
   E is phenyl or 5-6 membered heteroaryl;
   R⁸, R⁹, and R¹¹ are each independently H or OH;
   n is 0, 1, or 2; and
   q is 0 or 1.
3. The compound of clause 1 or 2, wherein q is 0.
4. The compound of clause 1 or 2, wherein q is 1.
5. The compound of clause 1, or a salt thereof, wherein
   X is OH.
6. The compound of clause 1, or a salt thereof, wherein
   X is -C(O)NR^{a}R^{b}, -S(O)₂R^{a}, -S(O)-R^{a} -S(O)₂NR^{a}R^{b}, -NHS(O)₂R^{a}, or -NHC(O)R^{a}.
7. The compound of any one of clauses 1 or 3-6, or a salt thereof, wherein
   X^{b} is -NR¹R²; and
   X^{a} is 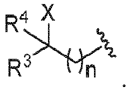
8. The compound of any one of clauses 1-7, or a salt thereof, wherein R³ is H.
9. The compound of any one of clauses 1-8, or a salt thereof, wherein R⁴ is H.
10. The compound of any one of clauses 1-9, or a salt thereof, wherein n is 0 or 1.
11. The compound of any one of clauses 1 or 3-6, or a salt thereof, wherein
   X^{a} and X^{b} are taken together with the carbon atom to which they are attached to form 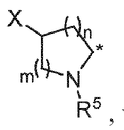 , wherein the asterisk denotes the carbon atom of Formula (I) that bears the X^{a} and X^{b} groups.
12. The compound of any one of clauses 1 or 3-11, wherein m is 1 or 2.
13. The compound of any one of clauses 1-10, or a salt thereof, wherein R¹ is H.
14. The compound of any one of clauses 1-10, or a salt thereof, wherein R¹ is C₁-C₄ alkyl.
15. The compound of any one of clauses 1-10 or 14, or a salt thereof, wherein R¹ is methyl.
16. The compound of any one of clauses 1, 3-10, or 13-15, or a salt thereof, wherein R² is methyl.
17. The compound of any one of clauses 1-16, or a salt thereof, wherein R¹⁰ is H.
18. The compound of any one of clauses 1-16, or a salt thereof, wherein R¹⁰ is methyl.
19. The compound of any one of clauses 1-18, or a salt thereof, wherein R⁶ is unsubstituted C₁-C₄ alkyl.
20. The compound of any one of clauses 1-19, or a salt thereof, wherein R⁶ is isopropyl.
21. The compound of any one of clauses 1-18, or a salt thereof, wherein R⁶ is C₁-C₄ alkyl substituted with OH.
22. The compound of any one of clauses 1-21, or a salt thereof, wherein R⁷ C₁-C₄ alkyl substituted with OH.
23. The compound of any one of clauses 1-22, or a salt thereof, wherein R⁷ is -CH₂OH.
24. The compound of any one of clauses 1-21, or a salt thereof, wherein R⁷ is H.
25. The compound of any one of clauses 1-21, or a salt thereof, wherein R⁷ is unsubstituted C₁-C₄ alkyl.
26. The compound of any one of clauses 1-21 or 25, or a salt thereof, wherein R⁷ is methyl.
27. The compound of any one of clauses 1-21, or a salt thereof, wherein R⁷ is 5-6 membered heteroaryl.
28. The compound of any one of clauses 1-27, or a salt thereof, wherein R⁸ is H.
29. The compound of any one of clauses 1-28, or a salt thereof, wherein R⁸ is OH.
30. The compound of any one of clauses 1-29, or a salt thereof, wherein E is phenyl.
31. The compound of any one of clauses 1-30, or a salt thereof, wherein E-R⁹ is 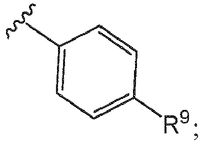 and
   the wavy line indicates the point of attachment of E to the rest of the compound.
32. The compound of any one of clauses 1-29, or a salt thereof, wherein E is 5-6 membered heteroaryl.
33. The compound of any one of clauses 1-32, or a salt thereof, wherein R⁹ is H.
34. The compound of any one of clauses 1-33, or a salt thereof, wherein R⁹ is OH.
35. The compound of clause 1, or a salt thereof, wherein
   X^{a} is 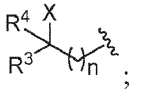
   X^{b} is -NR¹R²;
   R¹ is H or methyl;
   R² is methyl;
   X is OH;
   R³ and R⁴ are H;
   R⁶ is isopropyl;
   R⁷ is -CH₂OH;
   R⁸ is H;
   E is phenyl; and
   R⁹ is H.
36. The compound of clause 1, wherein the compound is 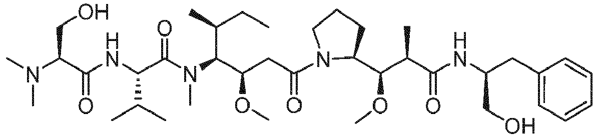 or a salt thereof.
37. A Drug-Linker compound of the following formula:

   Q-D,

   or a salt thereof, wherein
   Q is a Linker Unit selected from the group consisting of:
      (i) Z'-A-RL-,
      (ii) Z'-A-RL-Y-,
      (iii) Z'-A-S^{∗}-RL-,
      (iv) Z'-A-S^{∗}-RL-Y-,
      (v) Z'-A-B(S^{∗})-RL-,
      (vi) Z'-A-B(S^{∗})-RL-Y-,
      (vii) Z'-A-,
      (viii) Z'-A-S^{∗}-W-,
      (ix) Z'-A-B(S^{∗})-W-,
      (x) Z'-A-S^{∗}-W-RL-, and
      (xi) Z'-A-B(S^{∗})-W-RL-;
   Z' is a Stretcher Unit precursor;
   A is a bond or a Connector Unit;
   B is a Parallel Connector Unit;
   S^{∗} is a Partitioning Agent;
   RL is a Releasable Linker;
   W is an Amino Acid Unit;
   Y is a Spacer Unit; and
   D is a Drug Unit of Formula (I'): 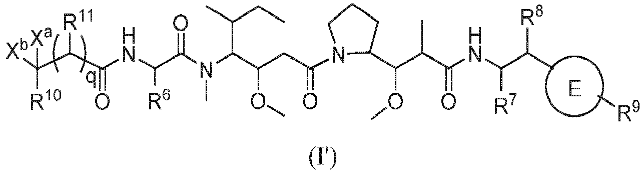 wherein
      X^{b} is -NR²-^{#} or -N⁺R¹R⁵-^{#}, wherein the # denotes the point of attachment to Q, and X^{a} is 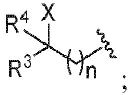 or
      X^{a} and X^{b} are taken together with the carbon atom to which they are attached to form 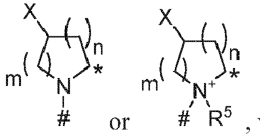 wherein the asterisk denotes the carbon atom of Formula (I) that bears the X^{a} and X^{b} groups, and the # denotes the point of attachment to Q;
      R¹ and R⁵ are independently C₁-C₄ alkyl;
      X is H, OH, -C(O)NR^{a}R^{b}, -S(O)₂R^{a}, -S(O)-R^{a} -S(O)₂NR^{a}R^{b}, -NHS(O)₂R^{a}, or -NHC(O)R^{a};
      R², R³, R⁴, R¹⁰, R^{a}, and R^{b} are each independently H or C₁-C₄ alkyl;
      R⁶ is C₁-C₄ alkyl optionally substituted with OH;
      R⁷ is H, C₁-C₄ alkyl optionally substituted with one or two OH moieties, or 5-6 membered heteroaryl;
      E is phenyl or 5-6 membered heteroaryl;
      R⁸, R⁹, and R¹¹ are each independently H or OH;
      n is 0, 1, 2, or 3;
      m is 1, 2, 3, or 4;
      q is 0 or 1; and
      wherein when X is H, at least two of R⁷, R⁸, R⁹, and R¹¹ comprise an OH moiety.
38. The Drug-Linker compound of clause 37, or a salt thereof, wherein the Linker Unit Q is of formula (i), (ii), (iii), (iv), (x), or (xi).
39. The Drug-Linker compound of clause 37, or a salt thereof, wherein the Linker Unit Q is of formula (v), (vi), (ix), or (xi).
40. The Drug-Linker compound of clause 37, or a salt thereof, wherein the Linker Unit Q is of formula (viii), (ix), (x), or (xi).
41. The Drug-Linker compound of any one of clauses 37-40, or a salt thereof, wherein the Stretcher Unit Z' is 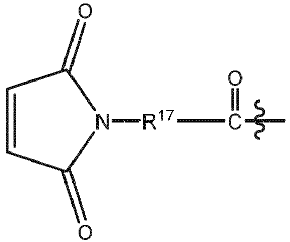 wherein
   R¹⁷ is -CH₂CH₂(OCH₂CH₂)ₖ-, -C₁-C₁₀ alkylene-, C₁-C₁₀ heteroalkylene-, -C₃-C₈ carbocyclo-, -O-(C₁-C₈ alkylene)-, -arylene-, -C₁-C₁₀ alkylene-arylene-, -arylene-C₁-C₁₀ alkylene-, -C₁-C₁₀ alkylene-(C₃-C₈ carbocyclo)-, -(C₃-C₈ carbocyclo)-C₁-C₁₀ alkylene-, -C₃-C₈ heterocyclo-, -C₁-C₁₀ alkylene-(C₃-C₈ heterocyclo)-, -(C₃-C₈ heterocyclo)-Ci-Cio alkylene-, -C₁-C₁₀ alkylene-C(=O)-, C₁-C₁₀ heteroalkylene-C(=O)-, -C₃-C₈ carbocyclo-C(=O)-, -O-(C₁-C₈ alkylene)-C(=O)-, -arylene-C(=O)-, -C₁-C₁₀ alkylene-arylene-C(=O)-, -arylene-C₁-C₁₀ alkylene-C(=O)-, -C₁-C₁₀ alkylene-(C₃-C₈ carbocyclo)-C(=O)-,-(C₃-C₈ carbocyclo)-C₁-C₁₀ alkylene-C(=O)-, -C₃-C₈ heterocyclo-C(=O)-, -C₁-C₁₀ alkylene-(C₃-C₈ heterocyclo)-C(=O)-, - (C₃-C₈ heterocyclo)-Ci-Cio alkylene-C(=O)-, -C₁-C₁₀ alkylene-NH-, C₁-C₁₀ heteroalkylene-NH-, -C₃-C₈ carbocyclo-NH-, -O-(C₁-C₈ alkylene)-NH-, -arylene-NH-, -C₁-C₁₀ alkylene-arylene-NH-, -arylene-C₁-C₁₀ alkylene-NH-, -C₁-C₁₀ alkylene-(C₃-C₈ carbocyclo)-NH-, -(C₃-C₈ carbocyclo)-C₁-C₁₀ alkylene-NH-, -C₃-C₈ heterocyclo-NH-, -C₁-C₁₀ alkylene-(C₃-C₈ heterocyclo)-NH-, -(C₃-C₈ heterocyclo)-Ci-Cio alkylene-NH-, -C₁-C₁₀ alkylene-S-, C₁-C₁₀ heteroalkylene-S -, -C₃-C₈ carbocyclo-S -, -O-(C₁-C₈ alkylene)-S -, -arylene-S-, -C₁-C₁₀ alkylene-arylene-S-, -arylene-C₁-C₁₀ alkylene-S-, -C₁-C₁₀ alkylene-(C₃-C₈ carbocyclo)-S-, - (C₃-C₈ carbocyclo)-C₁-C₁₀ alkylene-S-, -C₃-C₈ heterocyclo-S-, -C₁-C₁₀ alkylene-(C₃-C₈ heterocyclo)-S-, or -(C₃-C₈ heterocyclo)-Ci-Cio alkylene-S-;
   subscript k is an integer ranging from 1 to 36;
   R¹⁷ is optionally substituted by a Basic Unit (BU) such as an aminoalkyl moiety, e.g. -(CH₂ )ₓNH₂, -(CH₂ )ₓNHR^{a}, and -(CH2 )ₓNR^{a} ₂, wherein x is an integer of from 1-4 and each R^{a} is independently selected from the group consisting of C₁₋₆ alkyl and C₁₋₆ haloalkyl, or two R^{a} groups are combined with the nitrogen to which they are attached to form an azetidinyl, pyrrolidinyl or piperidinyl group; and
   the wavy line indicates the point of covalent attachment to the rest of the Drug-Linker compound.
42. The Drug-Linker of any one of clauses 37-41, or a salt thereof, wherein the Stretcher Unit Z' is 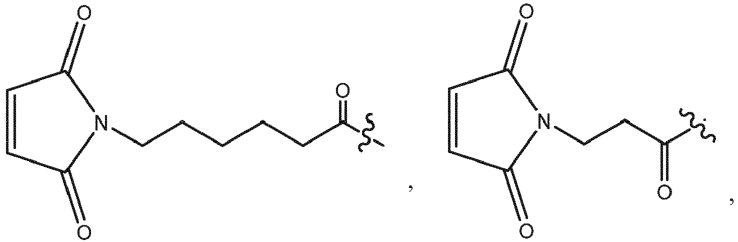 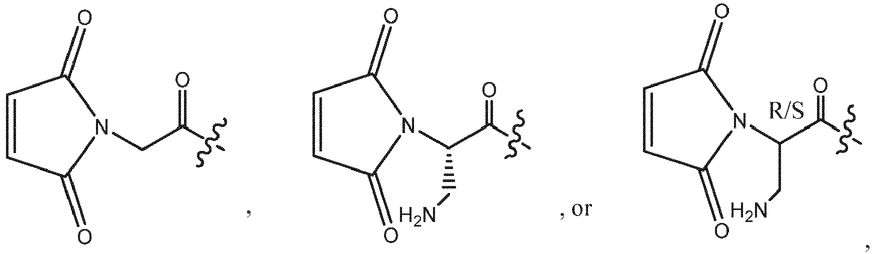 wherein the wavy lines indicate the point of covalent attachment to the rest of the Drug-Linker compound.
43. The Drug-Linker compound of any one of clauses 37-42, or a salt thereof, wherein the Connector Unit A is 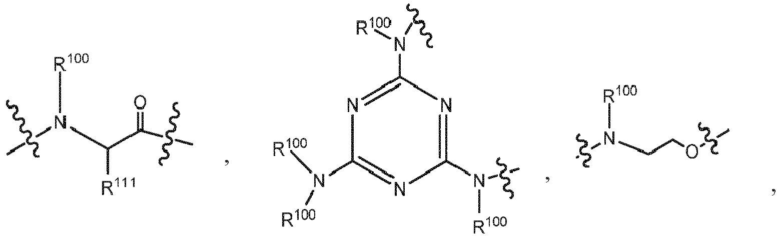 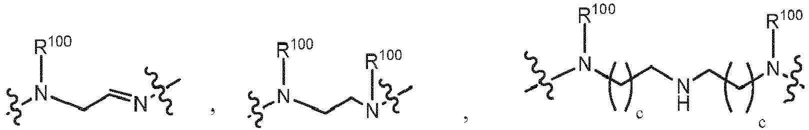 or 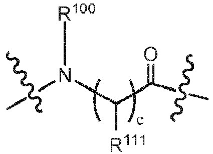 wherein
   each R¹⁰⁰ is independently selected from hydrogen or -C₁-C₃ alkyl;
   R¹¹¹ is independently selected from the group consisting of hydrogen, p-hydroxybenzyl, methyl, isopropyl, isobutyl, *sec*-butyl, -CH₂OH, -CH(OH)CH₃, - CH₂CH₂SCH₃, -CH₂CONH₂, -CH₂COOH, -CH₂CH₂CONH₂, -CH₂CH₂COOH, - (CH₂)₃NHC(=NH)NH₂, -(CH₂)₃NH₂, -(CH₂)₃NHCOCH₃, -(CH₂)₃NHCHO, - (CH₂)₄NHC(=NH)NH₂, -(CH₂)₄NH₂, -(CH₂)₄NHCOCH₃, -(CH₂)₄NHCHO, - (CH₂)₃NHCONH₂, -(CH₂)₄NHCONH₂, -CH₂CH₂CH(OH)CH₂NH₂, 2-pyridylmethyl-, 3-pyridylmethyl-, 4-pyridylmethyl-, 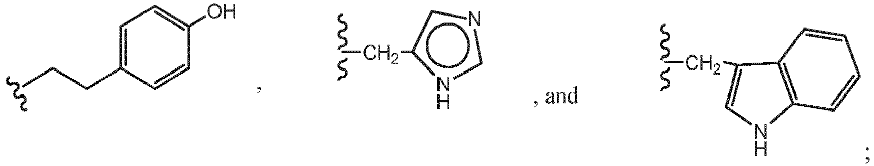
   each subscript c is an independently selected integer from 1 to 10; and
   the wavy lines indicate attachment of the Connector Unit to the rest of the Drug-Linker compound.
44. The Drug-Linker compound of any one of clauses 37-43, or a salt thereof, wherein the Connector Unit A is 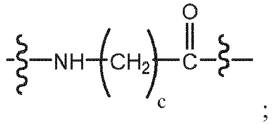
   c is an integer ranging from 1 to 6; and
   the wavy lines indicate the site of attachment to the rest of the Drug-Linker compound or salt thereof.
45. The Drug-Linker compound of any one of clauses 37-42, or a salt thereof, wherein A is a bond.
46. The Drug-Linker compound of clauses 37-45, or a salt thereof, wherein B is 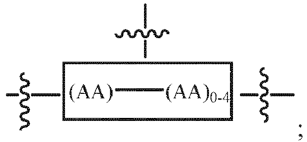 each AA is independently a proteinogenic or non-proteinogenic amino acid; and the wavy lines indicate points of attachment to the rest of the Drug-Linker compound or salt thereof.
47. The Drug-Linker compound of any one of clauses 37-46, or a salt thereof, wherein B is an amino acid.
48. The Drug-Linker compound of any one of clauses 37-47, or a salt thereof, wherein B is 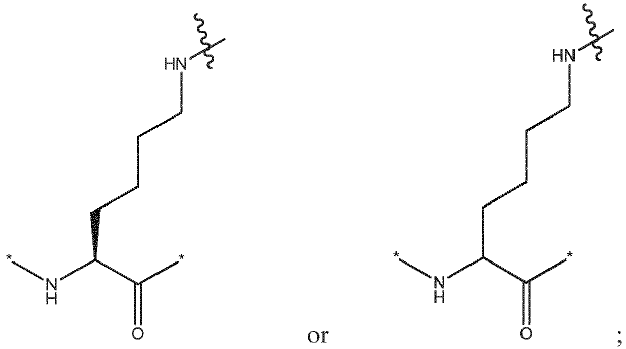
   the wavy line indicates the point of attachement to the Partitioning Agent S^{∗}; and
   the asterisks indicate points of attachment to the rest of the Drug-Linker structure.
49. The Drug-Linker compound of any one of clauses 37-48, or a salt thereof, wherein the Partitioning Agent S^{∗} is a polyethylene glycol (PEG) unit, cyclodextrin unit, polyamide, hydrophilic peptide, polysaccharide, or dendrimer.
50. The Drug-Linker compound of any one of clauses 37-49, or a salt thereof, wherein the Partitioning Agent S^{∗} is a PEG Unit comprising from 4 to 72 (CH₂CH₂O) subunits.
51. The Drug Linker of any one of clauses 37-50, or a salt thereof, wherein the PEG Unit is 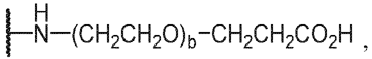 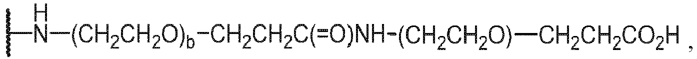 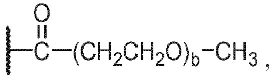 or 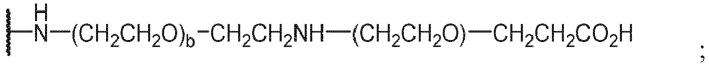
   b is selected from the group consisting of 4 to 36; and
   the wavy lines indicate the site of attachment to the rest of the Drug-Linker compound or salt thereof.
52. The Drug-Linker compound of any one of clauses 37-51, or a salt thereof, wherein the Releasable Linker RL is
   -(AA)₁₋₁₂-; and
   each AA is independently a proteinogenic or non-proteinogenic amino acid.
53. The Drug-Linker compound of any one of clauses 37-52, or a salt thereof, wherein the Releasable Linker RL is
   -AA₁-AA₂- or -AA₁-AA₂-AA₃-, wherein AA₁ is attached to the Stretcher Unit Z' or the Connector Unit A.
54. The Drug-Linker compound of any one of clauses 37-53, or a salt thereof, wherein the Releasable Linker RL is 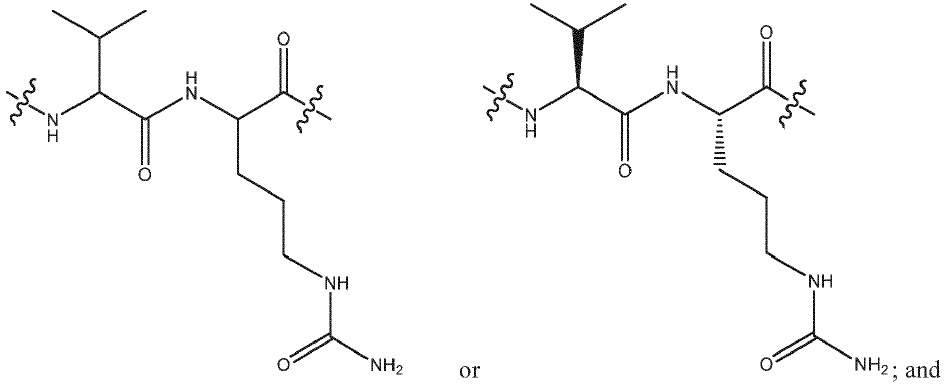 the wavy line adjacent to the -NH- group indicates attachment to the Stretcher Unit Z' or the Connector Unit A and the wavy line adjacent to the -C(=O)- group indicates attachment to the Spacer Unit Y or the Drug Unit D.
55. The Drug-Linker compound of any one of clauses 37-54, or a salt thereof, wherein the Releasable Linker RL is a glycoside.
56. The Drug-Linker compound of any one of clauses 37-55, or a salt thereof, wherein the Releasable Linker RL is 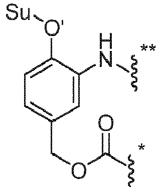 wherein
   Su is a hexose form a monosaccharide;
   O' represents the oxygen atom of a glycosidic bond that is capable of cleavage by a glycosidase;
   the wavy line marked with a single asterisk (^{∗}) indicates the site of covalent attachment to D; and
   the wavy line marked with a double asterisk (^{∗∗}) indicates the site of covalent attachment to the remainder of Q.
57. The Drug-Linker compound of any one of clauses 37-56, or a salt thereof, wherein the Releasable Linker RL is 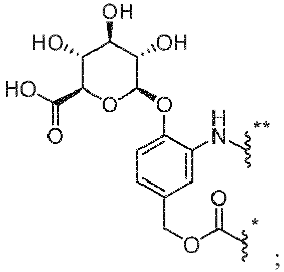
   the wavy line marked with a single asterisk (^{∗}) indicates the site of covalent attachment to D; and
   the wavy line marked with a double asterisk (^{∗∗}) indicates the site of covalent attachment to the remainder of Q.
58. The Drug-Linker compound of any one of clauses 37-39 or 41-57, or a salt thereof, wherein the Spacer Unit Y is 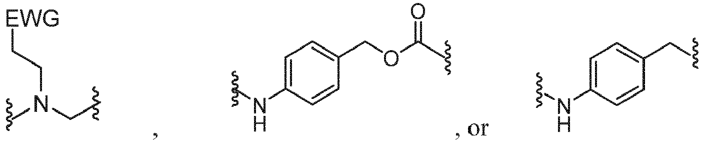
   wherein EWG is an electron-withdrawing group; and
   the wavy lines indicate the site of attachment to the rest of the Drug-Linker compound or salt thereof.
59. The Drug-Linker compound of any one of clauses 37-39 or 41-58, or a salt thereof, wherein the Spacer Unit Y is 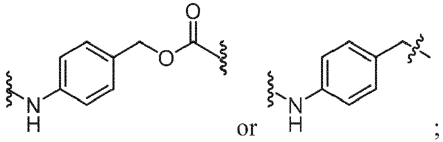 and
   the wavy lines indicate the site of attachment to the rest of the Drug-Linker compound or salt thereof.
60. The Drug-Linker compound of any one of clauses 37, 41-42, 45-48, 52-54, or 58-59, or a salt thereof, wherein
   Z' is 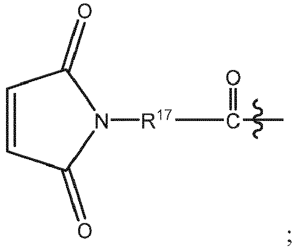
   R¹⁷ is C₁-C₁₀ alkylene;
   A is a bond;
   RL is -AA₁-AA₂-;
   AA₁ and AA₂ are each independently a proteinogenic amino acid;
   Y is 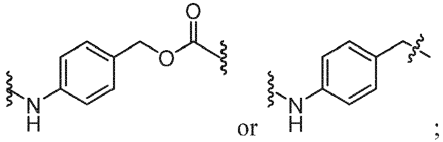 and
   the wavy lines indicate the site of attachment to the rest of the Drug-Linker compound or salt thereof.
61. The Drug-Linker compound of any one of clauses 37, 41-42, 45-48, 52-54, or 58-60, or a salt thereof, wherein
   Z' is 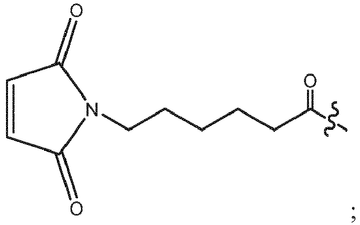
   A is a bond;
   RL is 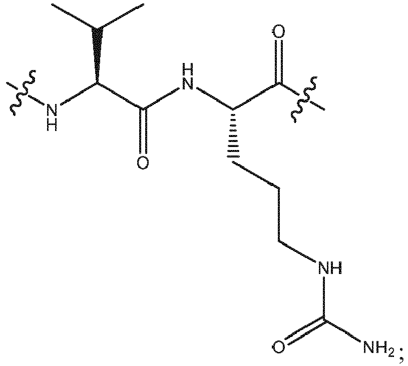 and
   Y is 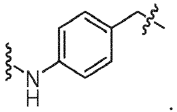
62. The Drug-Linker compound of any one of clauses 37-38 or 41-61, wherein Y-D is 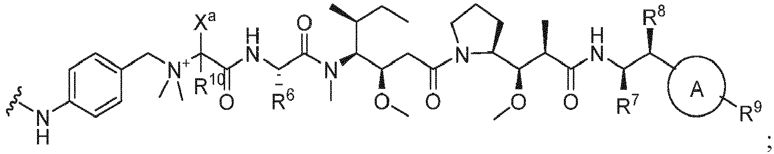 and
   the wavy line indicates the site of attachment to the rest of the Drug-Linker compound or salt thereof.
63. The Drug-Linker compound of any one of clauses 37-38 or 41-61, wherein Y-D is 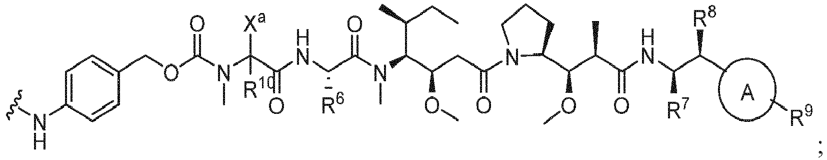 and
   the wavy line indicates the site of attachment to the rest of the Drug-Linker compound or salt thereof.
64. The Drug-Linker compound of any one of clauses 37, 41-42, 45-48, 52-54, 58-61, or 62, wherein the compound is 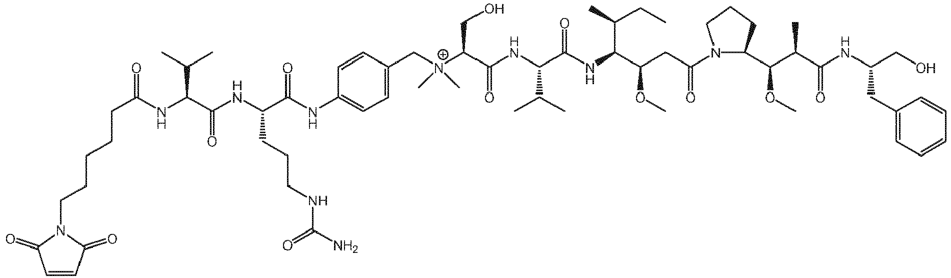 or a salt thereof.
65. The Drug-Linker compound of any one of clauses 37, 41-42, 45-48, 52-54, 58-61, or 63, wherein the compound is 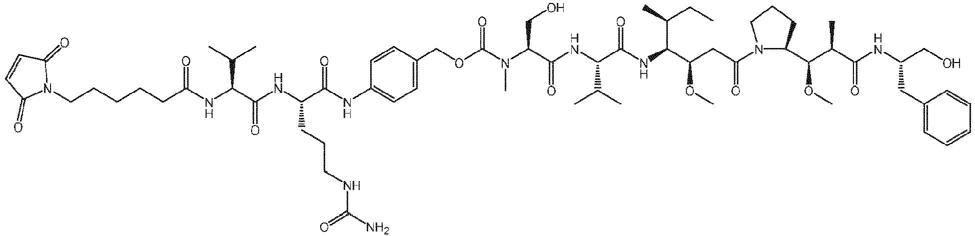 or 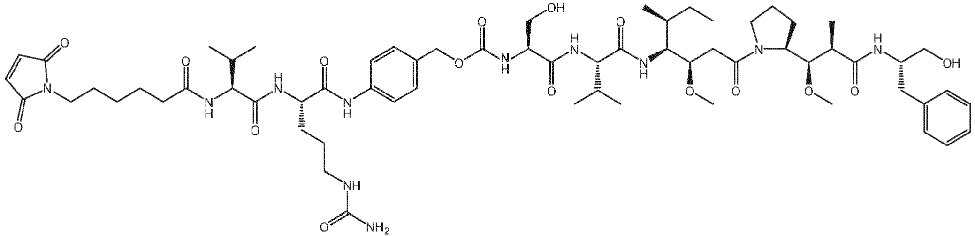 or a salt thereof.
66. A Ligand-Drug Conjugate compound of the formula:

   L-(Q-D)ₚ

   or a pharmaceutically acceptable salt thereof, wherein
   L is a Ligand Unit;
   Q is a Linker Unit selected from the group consisting of:
      (i) Z'-A-RL-,
      (ii) Z'-A-RL-Y-,
      (iii) Z'-A-S^{∗}-RL-,
      (iv) Z'-A-S^{∗}-RL-Y-,
      (v) Z'-A-B(S^{∗})-RL-,
      (vi) Z'-A-B(S^{∗})-RL-Y-,
      (vii) Z'-A-,
      (viii) Z'-A-S^{∗}-W-,
      (ix) Z'-A-B(S^{∗})-W-,
      (x) Z'-A-S^{∗}-W-RL-, and
      (xi) Z'-A-B(S^{∗})-W-RL-;
   Z' is a Stretcher Unit precursor;
   A is a bond or a Connector Unit;
   B is a Parallel Connector Unit;
   S^{∗} is a Partitioning Agent;
   RL is a Releasable Linker;
   W is a Amino Acid Unit;
   Y is a Spacer Unit; and
   D is a Drug Unit of Formula (I'): 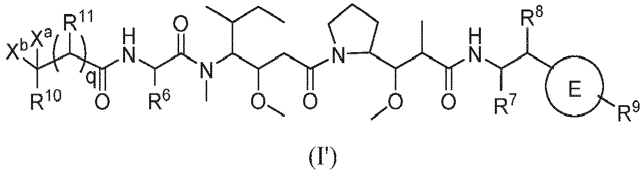 wherein
      X^{b} is -NR²-^{#} or -N⁺R¹R⁵-^{#}, wherein the # denotes the point of attachment to Q, and X^{a} is 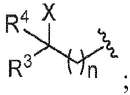 or
      X^{a} and X^{b} are taken together with the carbon atom to which they are attached to form 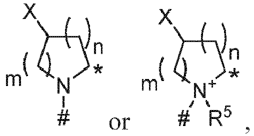 wherein the asterisk denotes the carbon atom of Formula (I) that bears the X^{a} and X^{b} groups, and the # denotes the point of attachment to Q;
      R¹ and R⁵ are independently C₁-C₄ alkyl;
      X is H, OH, -C(O)NR^{a}R^{b}, -S(O)₂R^{a}, -S(O)-R^{a} -S(O)₂NR^{a}R^{b}, -NHS(O)₂R^{a}, or -NHC(O)R^{a};
      R², R³, R⁴, R¹⁰, R^{a}, and R^{b} are each independently H or C₁-C₄ alkyl;
      R⁶ is C₁-C₄ alkyl optionally substituted with OH;
      R⁷ is H, C₁-C₄ alkyl optionally substituted with one or two OH moieties, or 5-6 membered heteroaryl;
      E is phenyl or 5-6 membered heteroaryl;
      R⁸, R⁹, and R¹¹ are each independently H or OH;
      n is 0, 1, 2, or 3;
      m is 1, 2, 3, or 4;
      q is 0 or 1;
      p is an integer ranging from 1 to 12; and
      wherein when X is H, at least two of R⁷, R⁸, R⁹, and R¹¹ comprise an OH moiety.
67. The Ligand-Drug Conjugate compound of clause 66, or a pharmaceutically acceptable salt thereof, wherein the Linker Unit Q is of formula (i), (ii), (iii), (iv), (x), or (xi).
68. The Ligand-Drug Conjugate compound of clause 66, or a pharmaceutically acceptable salt thereof, wherein the Linker Unit Q is of formula (v), (vi), (ix), or (xi).
69. The Ligand-Drug Conjugate compound of clause 66, or a pharmaceutically acceptable salt thereof, wherein the Linker Unit Q is of formula (viii), (ix), (x), or (xi).
70. The Ligand-Drug Conjugate compound of any one of clauses 66-69, or a pharmaceutically acceptable salt thereof, wherein the Ligand Unit L and the Stretcher Unit Z together are 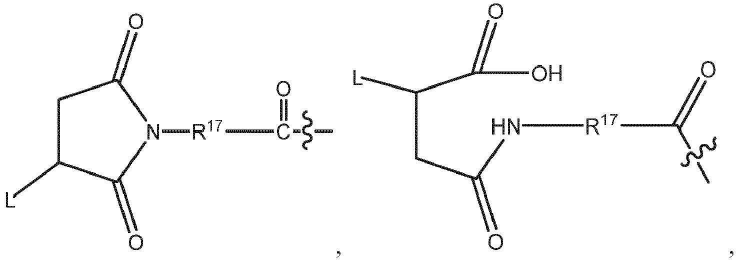 or 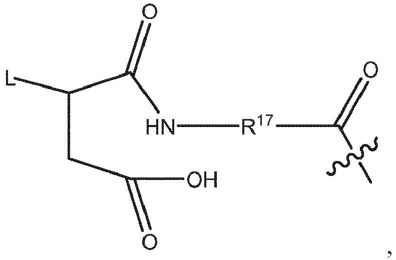 wherein
   R¹⁷ is -CH₂CH₂(OCH₂CH₂)ₖ-, -C₁-C₁₀ alkylene-, C₁-C₁₀ heteroalkylene-, -C₃-C₈ carbocyclo-, -O-(C₁-C₈ alkylene)-, -arylene-, -C₁-C₁₀ alkylene-arylene-, -arylene-C₁-C₁₀ alkylene-, -C₁-C₁₀ alkylene-(C₃-C₈ carbocyclo)-, -(C₃-C₈ carbocyclo)-C₁-C₁₀ alkylene-, -C₃-C₈ heterocyclo-, -C₁-C₁₀ alkylene-(C₃-C₈ heterocyclo)-, -(C₃-C₈ heterocyclo)-C₁-C₁₀ alkylene-, -C₁-C₁₀ alkylene-C(=O)-, C₁-C₁₀ heteroalkylene-C(=O)-, -C₃-C₈ carbocyclo-C(=O)-, -O-(C₁-C₈ alkylene)-C(=O)-, -arylene-C(=O)-, -C₁-C₁₀ alkylene-arylene-C(=O)-, -arylene-C₁-C₁₀ alkylene-C(=O)-, -C₁-C₁₀ alkylene-(C₃-C₈ carbocyclo)-C(=O)-,-(C₃-C₈ carbocyclo)-C₁-C₁₀ alkylene-C(=O)-, -C₃-C₈ heterocyclo-C(=O)-, -C₁-C₁₀ alkylene-(C₃-C₈ heterocyclo)-C(=O)-, - (C₃-C₈ heterocyclo)-C₁-C₁₀ alkylene-C(=O)-, -C₁-C₁₀ alkylene-NH-, C₁-C₁₀ heteroalkylene-NH-, -C₃-C₈ carbocyclo-NH-, -O-(C₁-C₈ alkylene)-NH-, -arylene-NH-, -C₁-C₁₀ alkylene-arylene-NH-, -arylene-Ci-Cio alkylene-NH-, -C₁-C₁₀ alkylene-(C₃-C₈ carbocyclo)-NH-, -(C₃-C₈ carbocyclo)-C₁-C₁₀ alkylene-NH-, -C₃-C₈ heterocyclo-NH-, -C₁-C₁₀ alkylene-(C₃-C₈ heterocyclo)-NH-, -(C₃-C₈ heterocyclo)-C₁-C₁₀ alkylene-NH-, -C₁-C₁₀ alkylene-S-, C₁-C₁₀ heteroalkylene-S -, -C₃-C₈ carbocyclo-S -, -O-(C₁-C₈ alkylene)-S -, -arylene-S-, -C₁-C₁₀ alkylene-arylene-S-, -arylene-C₁-C₁₀ alkylene-S-, -C₁-C₁₀ alkylene-(C₃-C₈ carbocyclo)-S-, - (C₃-C₈ carbocyclo)-C₁-C₁₀ alkylene-S-, -C₃-C₈ heterocyclo-S-, -C₁-C₁₀ alkylene-(C₃-C₈ heterocyclo)-S-, or -(C₃-C₈ heterocyclo)-Ci-Cio alkylene-S-;
   subscript k is an integer ranging from 1 to 36;
   R¹⁷ is optionally substituted by a Basic Unit (BU) such as an aminoalkyl moiety, e.g. -(CH₂ )ₓNH₂, -(CH2 )ₓNHR^{a}, and -(CH2 )ₓNR^{a} ₂, wherein x is an integer of from 1-4 and each R^{a} is independently selected from the group consisting of C₁₋₆ alkyl and C₁₋₆ haloalkyl, or two R^{a} groups are combined with the nitrogen to which they are attached to form an azetidinyl, pyrrolidinyl or piperidinyl group; and
   the wavy line indicates the point of covalent attachment to the rest of the Ligand-Drug Conjugate compound.
71. The Ligand-Drug Conjugate compound of any one of clauses 66-70, or a pharmaceutically acceptable salt thereof, wherein the Ligand Unit L and the Stretcher Unit Z together are 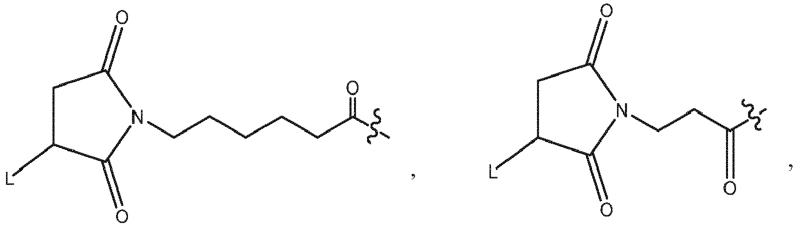 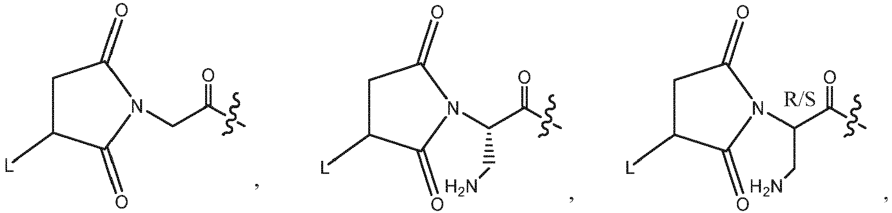 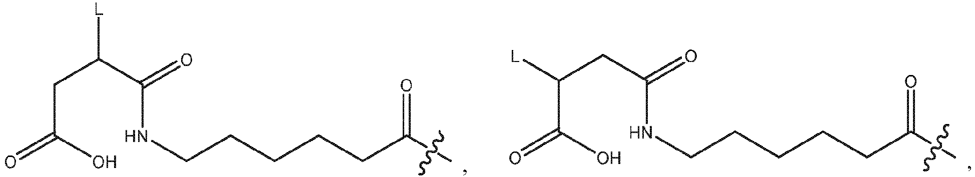 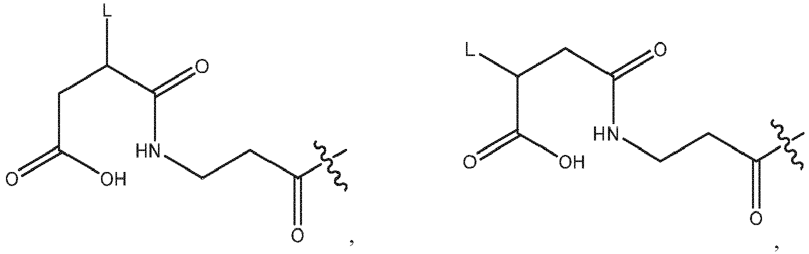 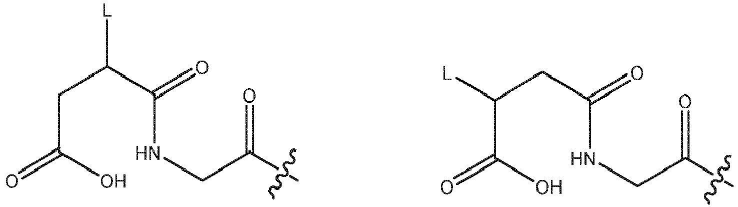 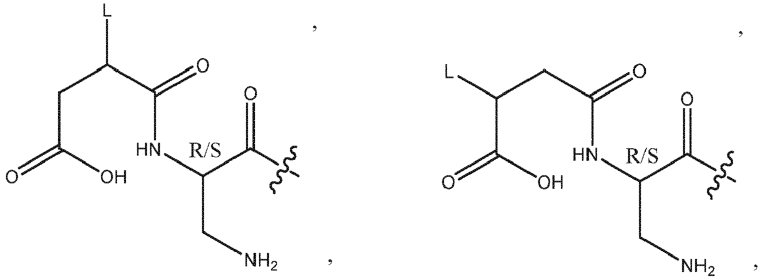 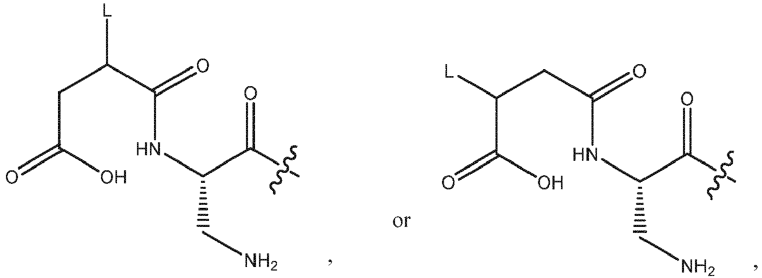 wherein the wavy lines indicate the point of covalent attachment to the rest of the Ligand-Drug Conjugate compound.
72. The Ligand-Drug Conjugate compound of any one of clauses 66-71, or a pharmaceutically acceptable salt thereof, wherein the Connector Unit A is 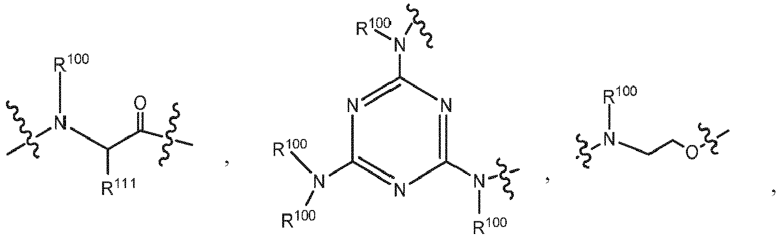 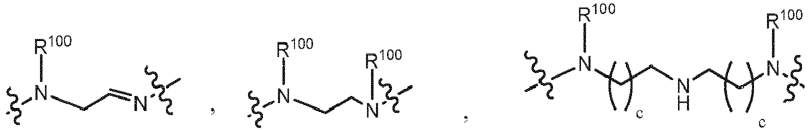 or 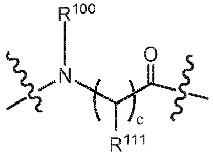 wherein
   each R¹⁰⁰ is independently selected from hydrogen or -C₁-C₃ alkyl;
   R¹¹¹ is independently selected from the group consisting of hydrogen, *p*-hydroxybenzyl, methyl, isopropyl, isobutyl, *sec*-butyl, -CH₂OH, -CH(OH)CH₃, -CH₂CH₂SCH₃, -CH₂CONH₂, -CH₂COOH, -CH₂CH₂CONH₂, -CH₂CH₂COOH, -(CH₂)₃NHC(=NH)NH₂, -(CH₂)₃NH₂, -(CH₂)₃NHCOCH₃, -(CH₂)₃NHCHO, -(CH₂)₄NHC(=NH)NH₂, -(CH₂)₄NH₂, - (CH₂)₄NHCOCH₃, -(CH₂)₄NHCHO, -(CH₂)₃NHCONH₂, -(CH₂)₄NHCONH₂, - CH₂CH₂CH(OH)CH₂NH₂, 2-pyridylmethyl-, 3-pyridylmethyl-, 4-pyridylmethyl-, 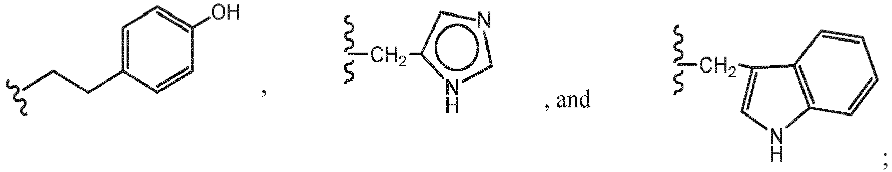
   each subscript c is an independently selected integer from 1 to 10; and
   the wavy lines indicate attachment of the Connector Unit to the rest of the Ligand-Drug Conjugate compound or pharmaceutically acceptable salt thereof.
73. The Ligand-Drug Conjugate compound of any one of clauses 66-72, or a pharmaceutically acceptable salt thereof, wherein the Connector Unit A is 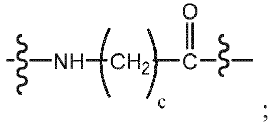
   c is an integer ranging from 1 to 6; and
   the wavy lines indicate the site of attachment to the rest of the Ligand-Drug Conjugate compound or pharmaceutically acceptable salt thereof.
74. The Ligand-Drug Conjugate compound of any one of clauses 66-71, or a pharmaceutically acceptable salt thereof, wherein A is a bond.
75. The Ligand-Drug Conjugate compound of clauses 66-74, or a salt thereof, wherein B is 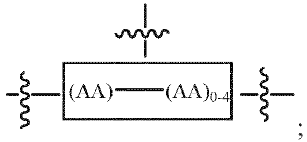
   each AA is independently a proteinogenic or non-proteinogenic amino acid; and
   the wavy lines indicate points of attachment to the rest of the Ligand-Drug Conjugate compound or pharmaceutically acceptable salt thereof.
76. The Ligand-Drug Conjugate compound of any one of clauses 66-75, or a pharmaceutically acceptable salt thereof, wherein B is an amino acid.
77. The Ligand-Drug Conjugate compound of any one of clauses 66-76, or a pharmaceutically acceptable salt thereof, wherein B is 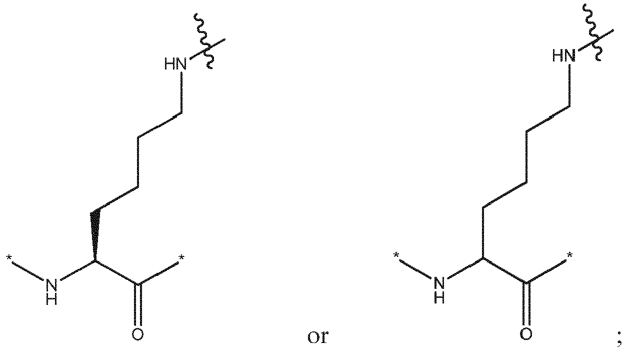
   the wavy line indicates the point of attachement to the Partitioning Agent S^{∗}; and
   the asterisks indicate points of attachment to the rest of the Ligand-Drug Conjugate compound or pharmaceutically acceptable salt thereof.
78. The Ligand-Drug Conjugate compound of any one of clauses 66-77, or a pharmaceutically acceptable salt thereof, wherein the Partitioning Agent S^{∗} is a polyethylene glycol (PEG) unit, cyclodextrin unit, polyamide, hydrophilic peptide, polysaccharide, or dendrimer.
79. The Ligand-Drug Conjugate compound of any one of clauses 66-78, or a pharmaceutically acceptable salt thereof, wherein the Partitioning Agent S^{∗} is a PEG Unit comprising from 4 to 72 (CH₂CH₂O) subunits.
80. The Ligand-Drug Conjugate compound of any one of clauses 66-79, or a pharmaceutically acceptable salt thereof, wherein the PEG Unit is 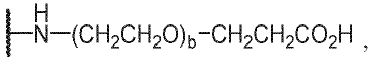 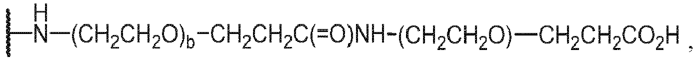 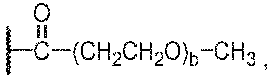 or 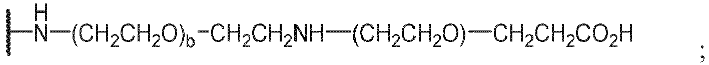
   b is selected from the group consisting of 4 to 36; and
   the wavy lines indicate the site of attachment to the rest of the Ligand-Drug Conjugate compound or pharmaceutically acceptable salt thereof.
81. The Ligand-Drug Conjugate compound of any one of clauses 66-80, or a pharmaceutically acceptable salt thereof, wherein the Releasable Linker RL is
   -(AA)₁₋₁₂-; and
   each AA is independently a proteinogenic or non-proteinogenic amino acid.
82. The Ligand-Drug Conjugate compound of any one of clauses 66-81, or a pharmaceutically acceptable salt thereof, wherein the Releasable Linker RL is -AA₁-AA₂- or -AA₁-AA₂-AA₃-, wherein AA₁ is attached to the Stretcher Unit Z or the Connector Unit A.
83. The Ligand-Drug Conjugate compound of any one of clauses 66-82, or a pharmaceutically acceptable salt thereof, wherein the Releasable Linker RL is 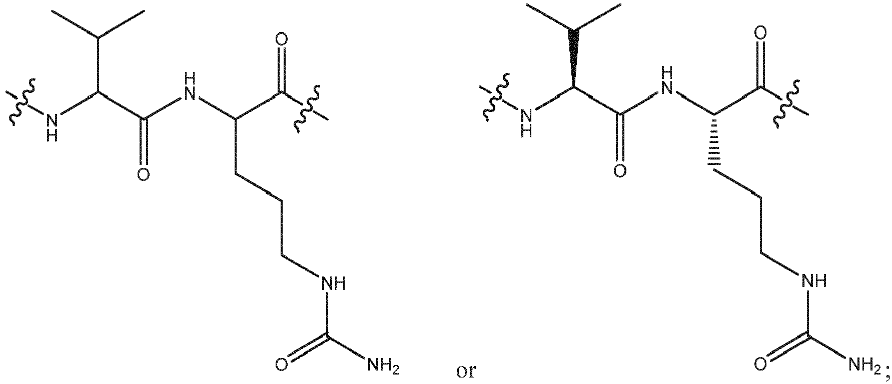 and
   the wavy line adjacent to the -NH- group indicates attachment to the Stretcher Unit Z or the Connector Unit A and the wavy line adjacent to the -C(=O)- group indicates attachment to the Spacer Unit Y or the Drug Unit D.
84. The Ligand-Drug Conjugate compound of any one of clauses 66-83, or a pharmaceutically acceptable salt thereof, wherein the Releasable Linker RL is a glycoside.
85. The Ligand-Drug Conjugate compound of any one of clauses 66-84, or a pharmaceutically acceptable salt thereof, wherein the Releasable Linker RL is 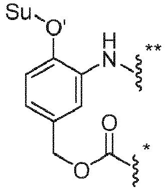 wherein
   Su is a hexose form a monosaccharide;
   O' represents the oxygen atom of a glycosidic bond that is capable of cleavage by a glycosidase;
   the wavy line marked with a single asterisk (^{∗}) indicates the site of covalent attachment to D; and
   the wavy line marked with a double asterisk (^{∗∗}) indicates the site of covalent attachment to the remainder of Q.
86. The Ligand-Drug Conjugate compound of any one of clauses 66-85, or a pharmaceutically acceptable salt thereof, wherein the Releasable Linker RL is 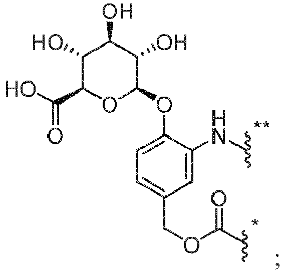
   the wavy line marked with a single asterisk (^{∗}) indicates the site of covalent attachment to D; and
   the wavy line marked with a double asterisk (^{∗∗}) indicates the site of covalent attachment to the remainder of Q.
87. The Ligand-Drug Conjugate compound of any one of clauses 66-68 or 70-86, or a pharmaceutically acceptable salt thereof, wherein the Spacer Unit Y is 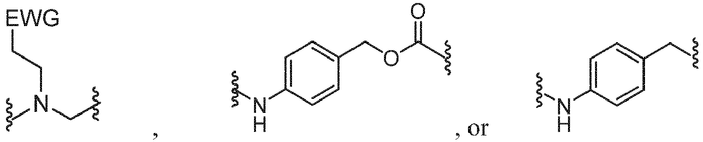
   wherein EWG is an electron-withdrawing group; and
   the wavy lines indicate the site of attachment to the rest of the Ligand-Drug Conjugate compound or pharmaceutically acceptable salt thereof.
88. The Ligand-Drug Conjugate compound of any one of clauses 66-68 or 70-87, or a pharmaceutically acceptable salt thereof, wherein the Spacer Unit Y is 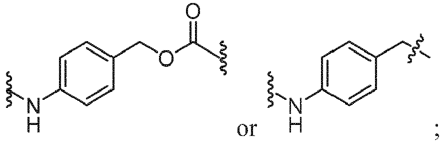 and the wavy lines indicate the site of attachment to the rest of the Ligand-Drug Conjugate compound or pharmaceutically acceptable salt thereof.
89. The Ligand-Drug Conjugate compound of any one of clauses 66-67, 70-71, 74, 81-83, or 87-88, or a pharmaceutically acceptable salt thereof, wherein
   R¹⁷ is C₁-C₁₀ alkylene;
   A is a bond;
   RL is -AA₁-AA₂-;
   AA₁ and AA₂ are each independently a proteinogenic amino acid;
   Y is 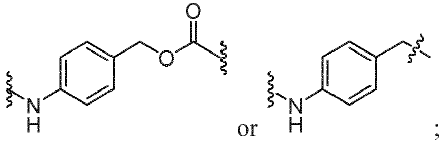 and
   the wavy lines indicate the site of attachment to the rest of the Ligand-Drug Conjugate compound or pharmaceutically acceptable salt thereof.
90. The Ligand-Drug Conjugate compound of any one of clauses 66-67, 70-71, 74, 81-83, or 87-89, or a pharmaceutically acceptable salt thereof, wherein
   A is a bond;
   RL is 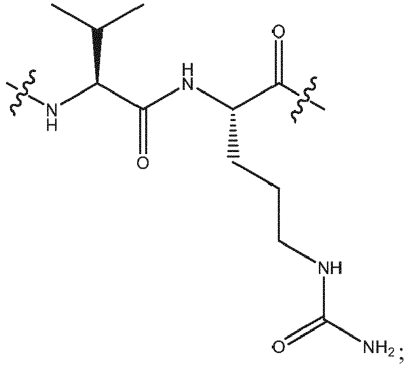 and
   Y is 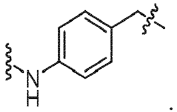
91. The Ligand-Drug Conjugate compound of any one of clauses 66-67, 70-71, 74, 81-83, or 87-90, or a pharmaceutically acceptable salt thereof, wherein Y-D is 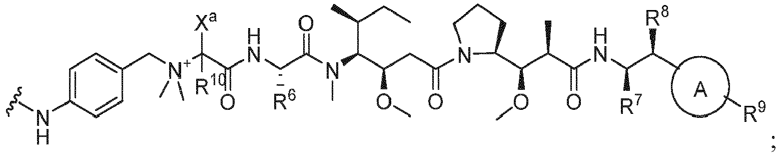 and
   the wavy line indicates the site of attachment to the rest of the Ligand-Drug Conjugate compound or pharmaceutically acceptable salt thereof.
92. The Ligand-Drug Conjugate compound of any one of clauses 66-67, 70-71, 74, 81-83, or 87-90, or a pharmaceutically acceptable salt thereof, wherein Y-D is 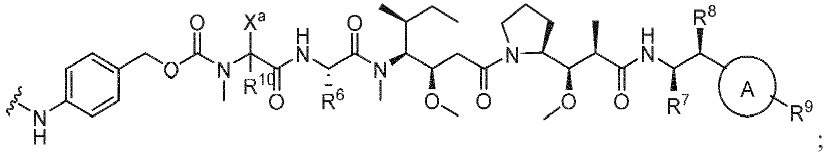 and
   the wavy line indicates the site of attachment to the rest of the Ligand-Drug Conjugate compound or pharmaceutically acceptable salt thereof.
93. The Ligand-Drug Conjugate compound of any one of clauses 66-67, 70-71, 74, 81-83, or 87-91, wherein the compound is 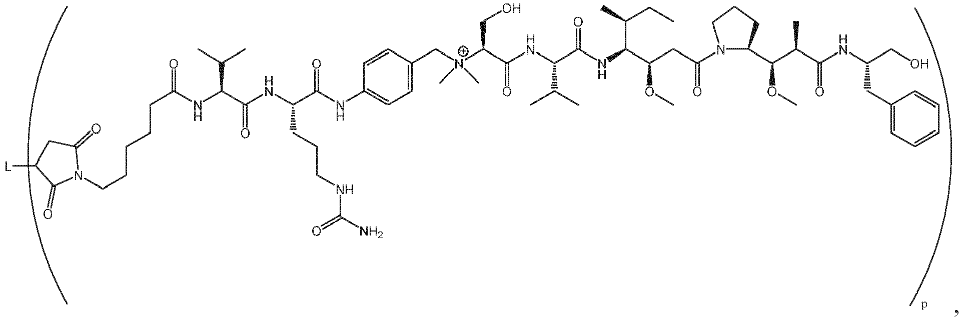 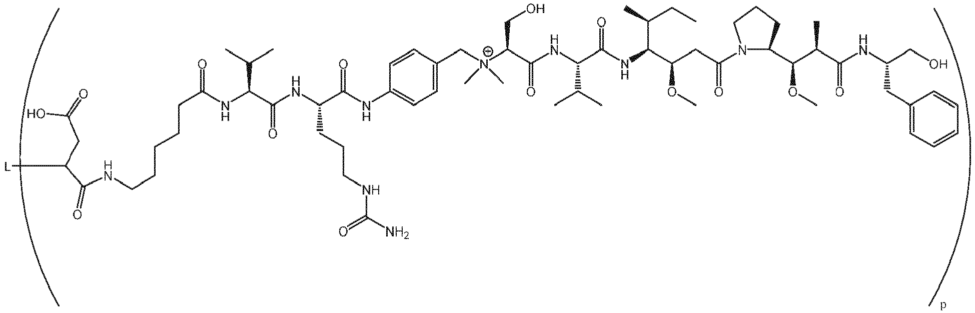 , or 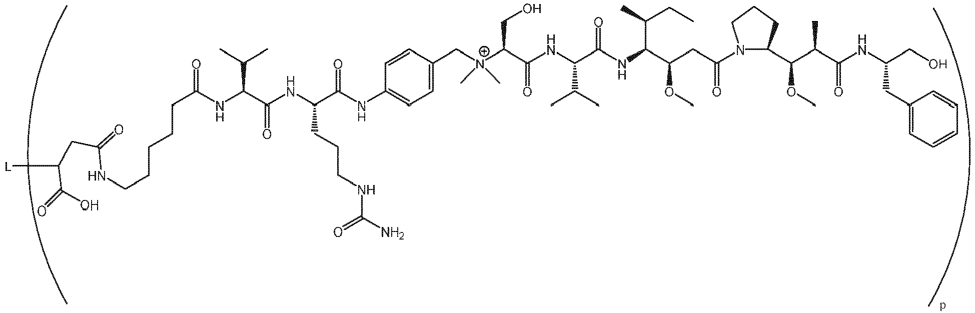 or a pharmaceutically acceptable salt thereof.
94. The Ligand-Drug Conjugate compound of any one of clauses 66-67, 70-71, 74, 81-83, 87-90, or 92, wherein the compound is 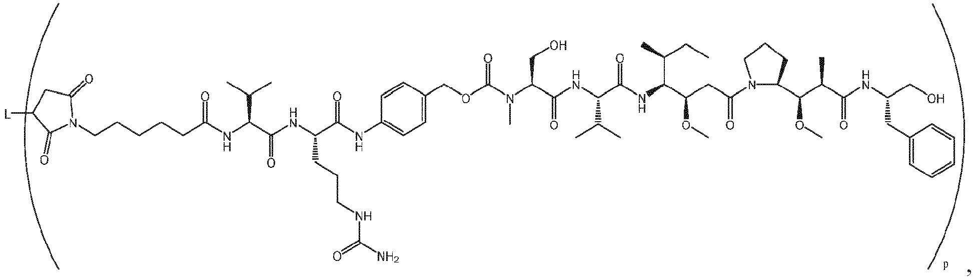 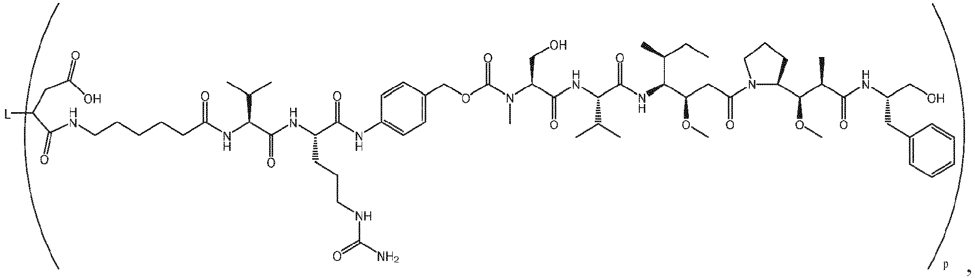 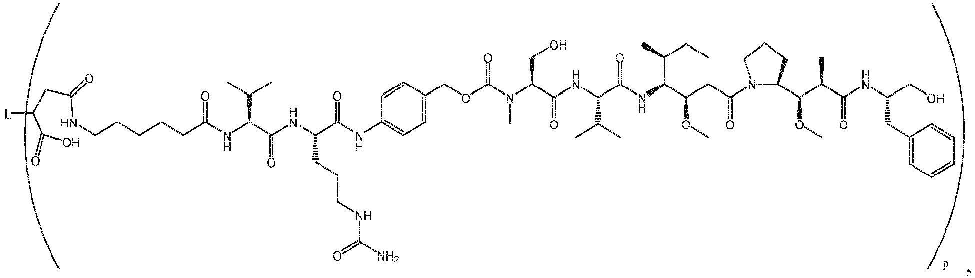 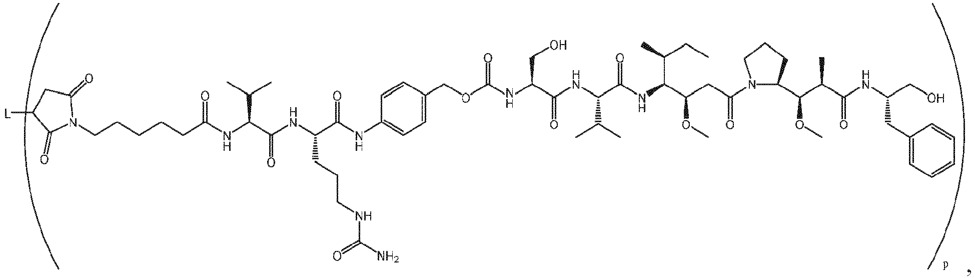 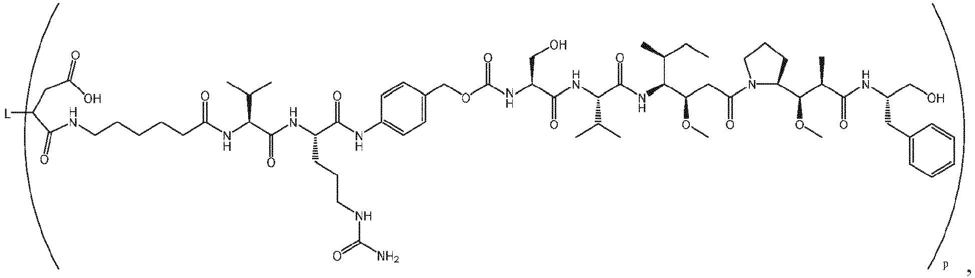 or 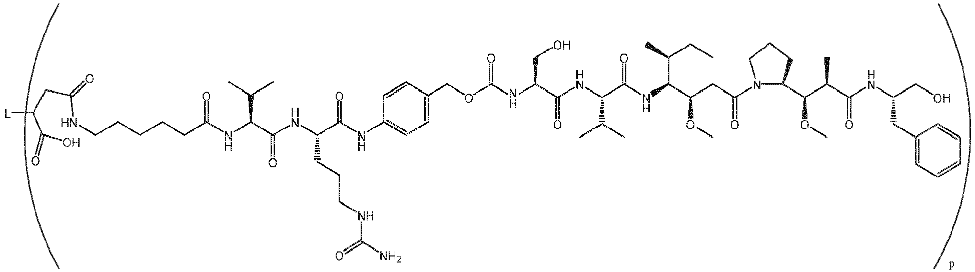 or a pharmaceutically acceptable salt thereof.
95. The Ligand-Drug Conjugate compound of any one of clauses 66-94, wherein p is an integer ranging from 2 to 8.
96. The Ligand-Drug Conjugate compound of any one of clauses 66-95, wherein p is 4.
97. A pharmaceutical composition comprising the Ligand-Drug Conjugate compound of any one of clauses 66-94 and a pharmaceutically acceptable excipient.
98. The pharmaceutical composition of clause 97, wherein the composition comprises a plurality of Ligand-Drug Conjugate compounds with an average drug-loading from 2 to 8.
99. The pharmaceutical composition of clause 98, wherein the average drug loading is about 4.
100. The pharmaceutical composition clause 98, wherein the average drug loading is from 3.5 to 4.5.
101. A method of treating cancer comprising administering a therapeutically effective amount of the Ligand-Drug Conjugate compound of any one of clauses 66-96, or a pharmaceutically acceptable salt thereof, to a subject in need thereof.
102. The method of clause 101, wherein the subject tolerates treatment from the Ligand-Drug Conjugate compound better than another Ligand-Drug Conjugate compound in therapeutically effective doses.
103. The method of clause 102, wherein the another Ligand-Drug Conjugate compound comprises a monomethyl auristatin E or monomethyl auristatin F Drug Unit.
104. A compound, wherein the compound is selected from the group consisting of the compounds listed in Table 1 or a salt thereof.
105. A Drug-Linker compound, wherein the Drug-Linker compound is selected from the group consisting of the compounds listed in Table 2 or a salt thereof.
106. A Ligand-Drug Conjugate compound, wherein the Ligand-Drug Conjugate compound is selected from the group consisting of the compounds listed in Table 3, or a salt thereof, and p is an integer ranging from 1 to 12.

### EXAMPLES

**General Information Chemical Synthesis.** All commercially available anhydrous solvents and reagents were used without further purification. UPLC-MS system 1 consisted of a Waters SQ mass detector 2 interfaced to an Acquity Ultra Performance LC equipped with a CORTECS UPLC C18 2.1 × 50 mm, 1.6 µm reverse phase column. The acidic mobile phase (0.1% formic acid) consisted of a gradient of 3% acetonitrile/97% water to 100% acetonitrile (flow rate = 0.5 mL/min). Preparative HPLC was carried out on a Waters 2545 Binary Gradient Module with a Waters 2998 Photodiode Array Detector. Products were purified over a C12 Phenomenex Synergi 250 × 10.0 mm, 4 µm, 80 Å reverse phase column (<10 mg scale) or a C12 Phenomenex Synergi 250 × 50 mm, 10 µm, 80 Å reverse phase column (10-100 mg scale) eluting with 0.1% trifluoroacetic acid in water (solvent A) and 0.1% trifluoroacetic acid in acetonitrile (solvent B). The purification methods generally consisted of linear gradients of solvent A to solvent B, ramping from 90% aqueous solvent A to 5% solvent A. The flow rate was 4.6 mL/min with monitoring at 220 nm. NMR spectral data were collected on a Varian Mercury 400 MHz spectrometer. Coupling constants (*J*) are reported in hertz.

Throughout the Examples, reference is made to the following structures:

### MMAE:

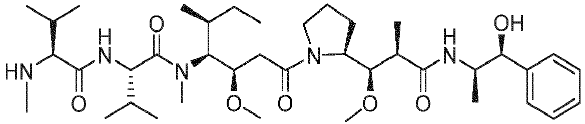

### MMAF:

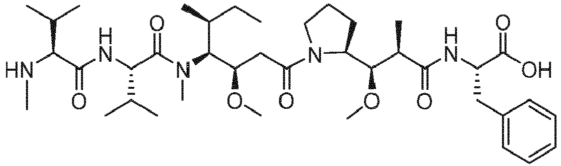

### MC-VC-MMAE:

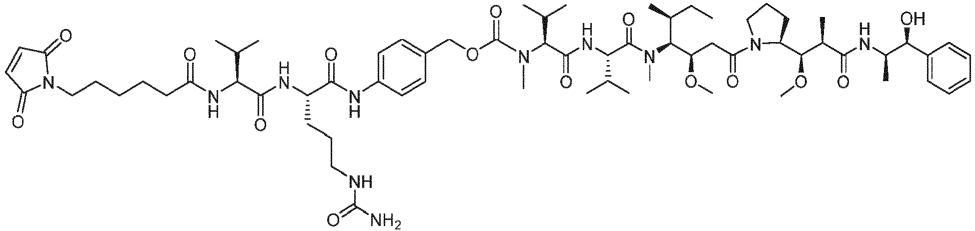

### MC-VC-MMAF:

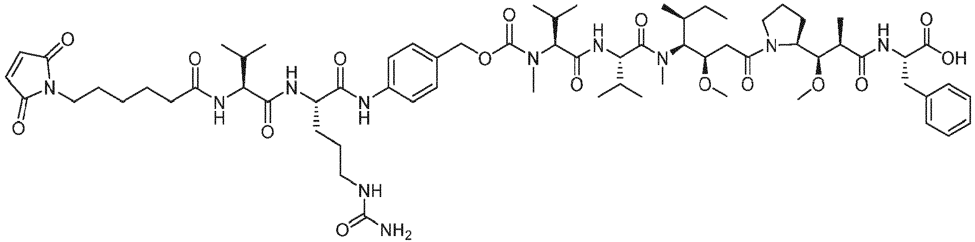

### Example 1. General procedure for solid-phase/solution-phase synthesis of auristatins

A similar procedure for Fmoc deprotection, amide coupling, alkylation and resin cleavage has been published (Moquist, P. N. et al. Mol. Cancer Ther. 2021, 20, 320-328).

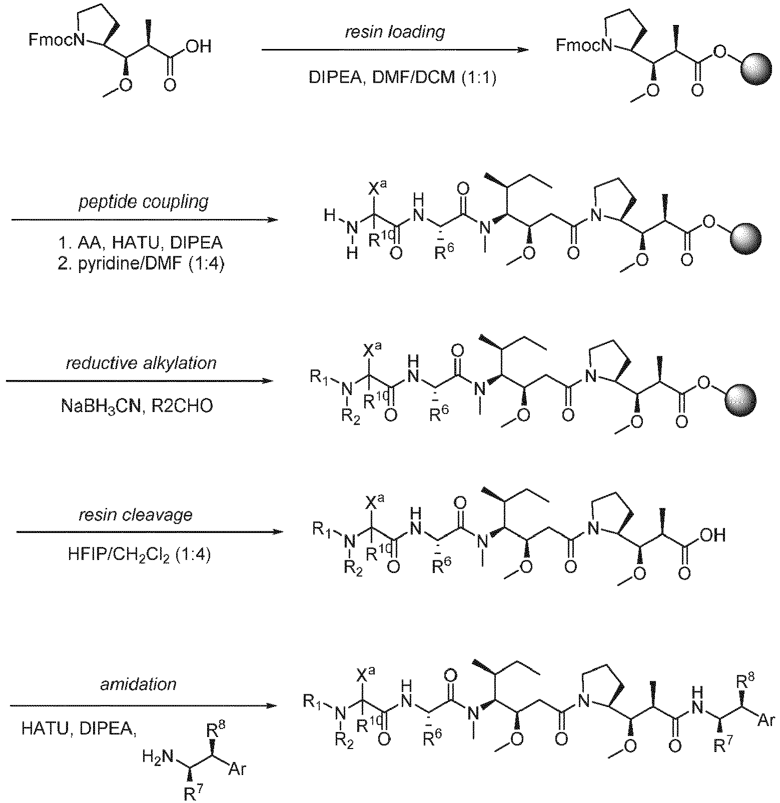

### Example 2. General Procedure for resin loading.

2-chlorotrityl resin (100-200 mesh), 1% DVB (Millipore Sigma, 855017) was loaded into a vessel and DCM (5V) was added to swell the resin for 30 min. The solvent was removed, and the resin was treated with solution of Fmoc-Dap (1 equivalent to resin loading) in DMF and DCM (1:1, 10V) and shaken for 2 h. The solution was filtered, and the resin resin washed with DMF (3 × 3 min), DCM (3 × 3 min), and Et2O (3 × 3 min) and vacuum-dried in a desiccator.

### Example 3. General procedure for Fmoc deprotection

The resin was treated with a solution of piperidine in DMF (1:4 V/V) and shaken for 30 min. The solution was removed, and the resin was resubjected three additional times to the same conditions. The solution was filtered, and the resin are washed with DMF (3 × 3 min).

### Example 4. General procedure for amide coupling

Fmoc-amino acid (2 equiv) was dissolved in dry DMF (0.2 M final concentration). DIPEA (4 equiv) and HATU (1.9 equiv) were added successively, and the reaction was stirred for 5 min. The resin was treated with the solution of activated Fmoc-amino acid and shaken for 2 h. The solution was filtered, and the resin was washed with DMF (3 × 3 min).

### Example 5. General procedure for resin cleavage

Prior to cleavage the resin was washed with DCM (3 × 3 min). Resin was cleaved with a solution of 20:80 (V/V) solution of HFIP/ DCM (10 min). Solution is removed in vacuo, and crude was dissolved for UPLC analysis and preparative HPLC. HPLC fraction were frozen and dried by lyophilization.

### Example 6. General procedure for reductive alkylation

Aldehyde (5 equiv.) was dissolved in a 0.6 mL solution of 1% (V/V) AcOH in DMF, followed by the addition of NaBH3CN (4 equiv.). The resin was treated with the solution and shaken for 2 h. The solution was filtered, and the resin was washed with DMF (3 × 3 mL) and DCM (3 × 3 mL) and vacuum-dried in a desiccator.

### Example 7. General procedure for C-terminal amine coupling

Tetrapeptide (1 equiv) was dissolved in dry DMF (0.3 M final concentration). DIPEA (4 equiv) and HATU (0.95 equiv) were added successively, and the reaction was stirred for 15 min. The P5 amine was added to the activated ester and the reaction was stirred for 2 h. The solvent was removed in vacuo and purified by preparative HPLC. HPLC fractions were frozen and dried by lyophilization.

### Auristatin compounds

Examples 8 to 33 were synthesized according to the procedures of Examples 1 to 7.

### Example 8.

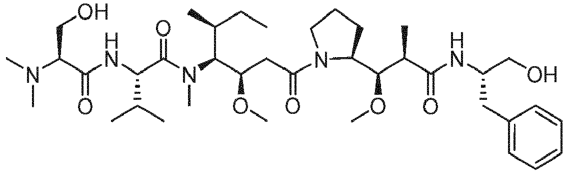

Prepared by dimethylation of tetrapeptide and amide coupling of tetrapeptide with L-phenylalaninol. Analytical UPLC-MS: t_{R} = 1.33 min, *m*/*z* (ES+) 720.49 (M+H)⁺, found 720.85.

### Example 9.

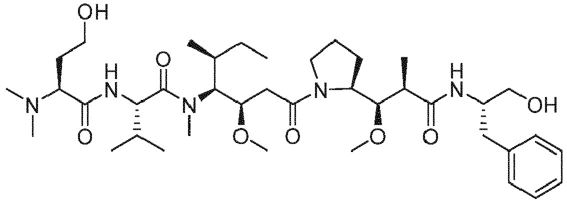

Prepared by dimethylation of tetrapeptide and amide coupling of tetrapeptide with L-phenylalaninol. Analytical UPLC-MS: t_{R} = 1.16 min, *m*/*z* (ES+) 734.50 (M+H)⁺, found 734.49.

### Example 10.

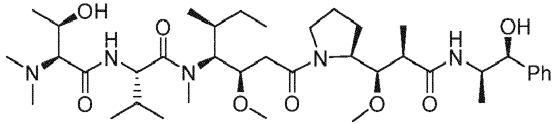

Prepared by dimethylation of tetrapeptide and amide coupling of tetrapeptide with (1*S*,2*R*)-(+)-norephedrine. Analytical UPLC-MS: t_{R} = 1.29 min, *m*/*z* (ES+) 734.50 (M+H)⁺, found 734.48.

### Example 11.

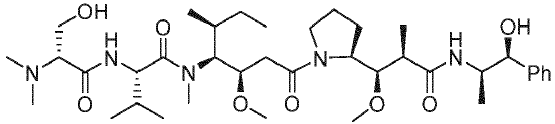

Prepared by dimethylation of tetrapeptide and amide coupling of tetrapeptide with (1*S*,2*R*)-(+)-norephedrine. Analytical UPLC-MS: t_{R} = 1.26 min, *m*/*z* (ES+) 720.48 (M+H)⁺, found 720.51.

### Example 12.

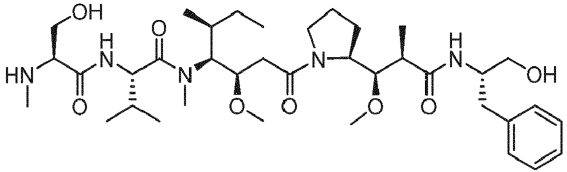

Prepared by amide coupling of tetrapeptide with L-phenylalaninol. Analytical UPLC-MS: t_{R} = 1.30 min *m*/*z* (ES+) 706.47 (M+H)⁺, found 707.62.

### Example 13.

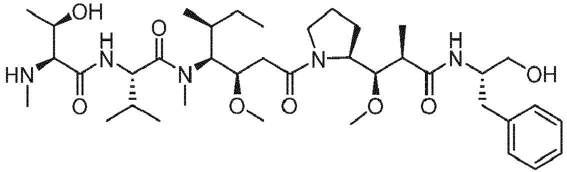

Prepared by amide coupling of tetrapeptide with L-phenylalaninol. Analytical UPLC-MS: t_{R} = 1.40 min, *m*/*z* (ES+) 719.48 (M+H)⁺, found 720.99.

### Example 14.

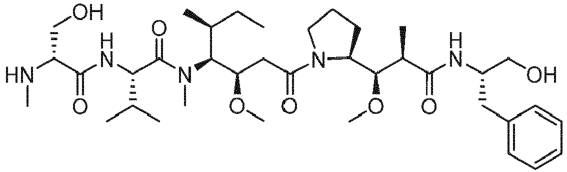

Prepared by amide coupling of tetrapeptide with L-phenylalaninol. Analytical UPLC-MS: t_{R} = 1.27 min, *m*/*z* (ES+) 706.47 (M+H)⁺, found 706.57.

### Example 15.

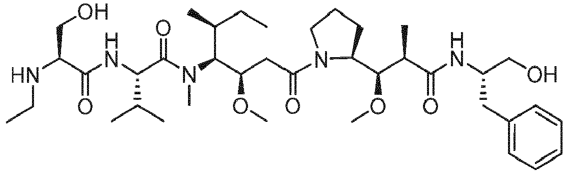

Prepared by reductive alkylation of tetrapeptide with acetaldehyde followed by amide coupling of tetrapeptide with L-phenylalaninol. Analytical UPLC-MS: t_{R} = 1.21 min, *m*/*z* (ES+) 720.49 (M+H)⁺, found 720.57.

### Example 16.

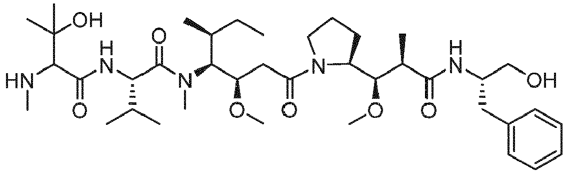

Prepared by amide coupling of tetrapeptide with L-phenylalaninol. Analytical UPLC-MS: t_{R} = 1.47 min, *m*/*z* (ES+) 734.51 (M+H)⁺, found 734.85.

### Example 17.

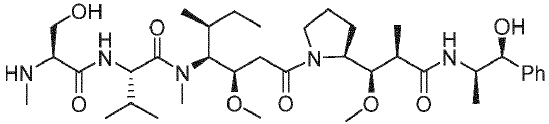

Prepared by amide coupling of tetrapeptide with (1*S*,2*R*)-(+)-norephedrine. Analytical UPLC-MS: t_{R} = 1.58 min, *m*/*z* (ES+) 728.46 (M+Na)⁺, found 728.73.

### Example 18.

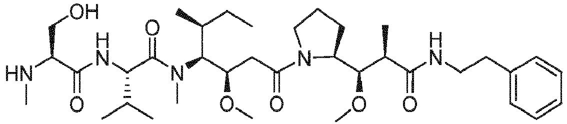

Prepared by amide coupling of tetrapeptide with phenethylamine. Analytical UPLC-MS: t_{R} = 1.70 min, *m*/*z* (ES+) 698.45 (M+Na)⁺, found 698.50.

### Example 19.

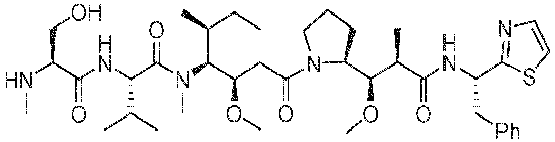

Prepared by amide coupling of tetrapeptide with *S*-dolaphenine. Analytical UPLC-MS: t_{R} = 1.85 min, *m*/*z* (ES+) 759.45 (M+H)⁺, found 760.05.

### Example 20.

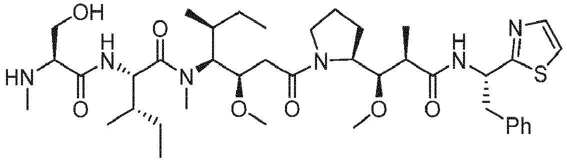

Prepared by amide coupling of tetrapeptide with *S*-dolaphenine. Analytical UPLC-MS: t_{R} = 1.89 min, *m*/*z* (ES+) 773.46 (M+H)⁺, found 773.58.

### Example 21.

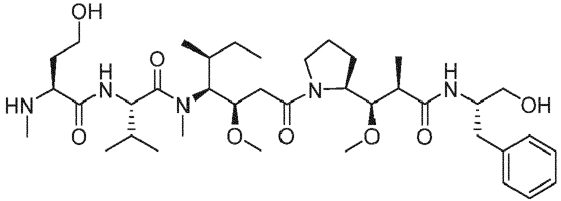

*N*-terminal amino acid Fmoc-(*N*-methyl)-L-h-serine was synthesized from L-h-Serine(OtBu)-OH by reaction with formaldehyde (37% aqueous, 1.5 equiv), NaH₂PO₄ (2 equiv) in water, Zn (dust, 2 equiv), and AcOH (1 equiv.) at RT overnight similar to known procedure (Da Silva, R. A. et al. Tetrahedron Lett. 2007, 48, 7680-7682). Fmoc protecting group was then installed as described in a reported procedure (Wuts, P; Green, T. "Protective Groups in Organic Synthesis" New York, 2006**).**

Prepared by amide coupling of tetrapeptide with L-phenylalaninol. Analytical UPLC-MS: t_{R} = 1.13 min, *m*/*z* (ES+) 720.45 (M+H)⁺, found 720.49.

### Example 22.

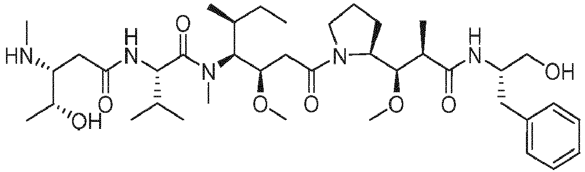

*N*-terminal amino acid Fmoc-(N-methyl)-ph-threonine was synthesized from βh-threonine-(OtBu)-OH by reaction with formaldehyde (37% aqueous, 1.5 equiv), NaH₂PO₄ (2 equiv) in water, Zn (dust, 2 equiv) and AcOH (1 equiv.) at RT overnight similar to known procedure (Da Silva, R. A. et al. Tetrahedron Lett. 2007, 48, 7680-7682). Fmoc protecting group was then installed as described in a reported procedure (Wuts, P; Green, T. "Protective Groups in Organic Synthesis" New York, 2006**).**

Prepared by amide coupling of tetrapeptide with L-phenylalaninol. Analytical UPLC-MS: t_{R} = 1.20 min, *m*/*z* (ES+) 734.50 (M+H)⁺, found 734.52.

### Example 23.

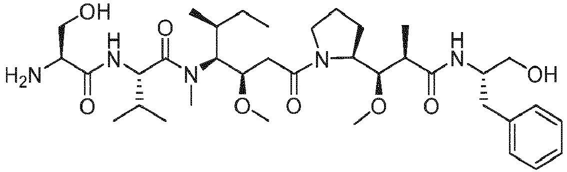

Prepared by amide coupling of tetrapeptide with L-phenylalaninol. Analytical UPLC-MS: t_{R} = 1.38 min, *m*/*z* (ES+) 692.46 (M+H)⁺, found 692.93.

### Example 24.

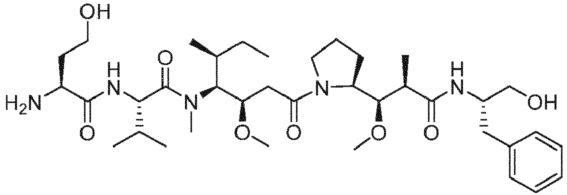

Prepared by amide coupling of tetrapeptide with L-phenylalaninol. Analytical UPLC-MS: t_{R} = 1.37 min, *m*/*z* (ES+) 706.47 (M+H)⁺, found 706.55.

### Example 25.

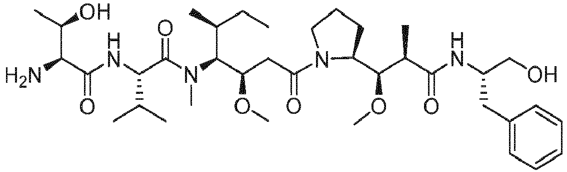

Prepared by amide coupling of tetrapeptide with L-phenylalaninol. Analytical UPLC-MS: t_{R} = 1.30 min, *m*/*z* (ES+) 706.47 (M+H)⁺, found 706.39.

### Example 26.

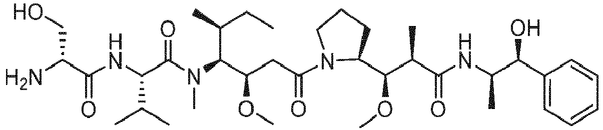

Prepared by amide coupling of tetrapeptide with (1*S*,2*R*)-(+)-norephedrine. Analytical UPLC-MS: t_{R} = 1.19 min, *m*/*z* (ES+) 692.46 (M+H)⁺, found 692.49.

### Example 27.

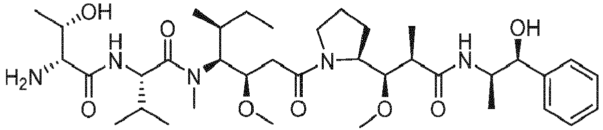

Prepared by amide coupling of tetrapeptide with (1*S*,2*R*)-(+)-norephedrine. Analytical UPLC-MS: t_{R} = 1.25 min, *m*/*z* (ES+) 706.47 (M+H)⁺, found 706.56.

### Example 28.

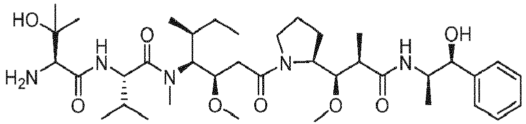

Prepared by amide coupling of tetrapeptide with (1*S*,2*R*)-(+)-norephedrine. Analytical UPLC-MS: t_{R} = 1.28 min, *m*/*z* (ES+) 720.48 (M+H)⁺, found 720.76.

### Example 29.

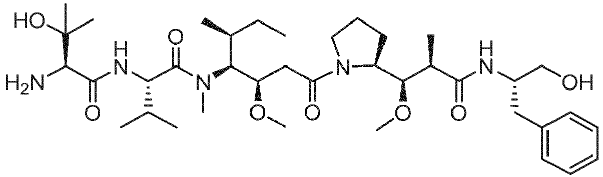

Prepared by amide coupling of tetrapeptide with L-phenylalaninol. Analytical UPLC-MS: t_{R} = 1.30 min, *m*/*z* (ES+) 720.49 (M+H)⁺, found 720.85.

### Example 30.

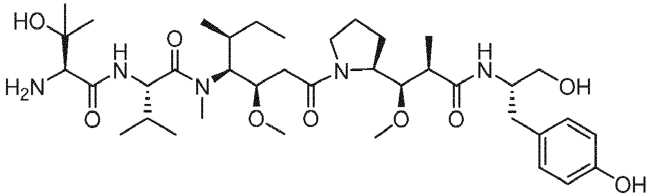

Prepared by amide coupling of tetrapeptide with L-tyrosinol. Analytical UPLC-MS: t_{R} = 1.15 min, *m*/*z* (ES+) 736.49 (M+H)⁺, found 736.79.

### Example 31.

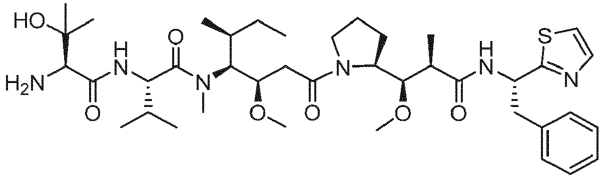

Prepared by amide coupling of tetrapeptide with S-dolaphenine. Analytical UPLC-MS: t_{R} = 1.49 min, *m*/*z* (ES+) 773.04 (M+H)⁺, found 773.61.

### Example 32.

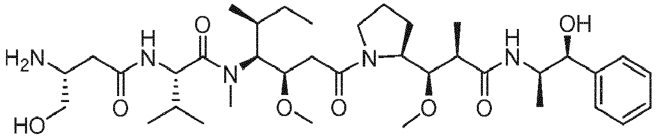

Prepared by amide coupling of tetrapeptide with (1*S*,2*R*)-(+)-norephedrine. Analytical UPLC-MS: t_{R} = 1.25 min, *m*/*z* (ES+) 706.48 (M+H)⁺, found 706.80.

### Example 33.

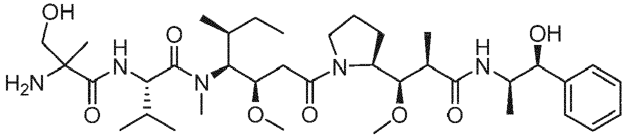

Prepared by amide coupling of tetrapeptide with (1*S*,2*R*)-(+)-norephedrine. Analytical UPLC-MS: t_{R} = 1.30 min, *m*/*z* (ES+) 706.48 (M+H)⁺, found 706.71.

### Example 34.

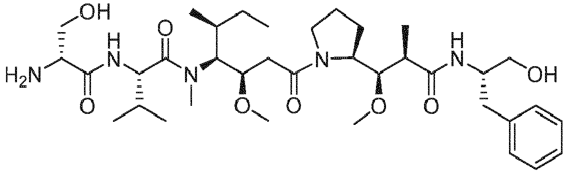

Can be prepared by amide coupling of tetrapeptide with L-phenylalaninol.

### Example 35.

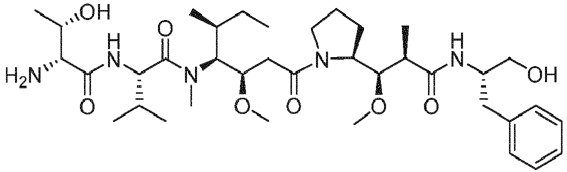

Can be prepared by amide coupling of tetrapeptide with L-phenylalaninol.

### Example 36. General procedure to synthesize cyclic carbamate auristatins

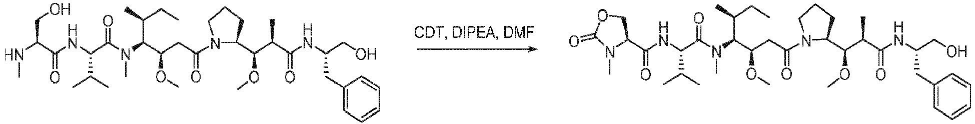

Auristatin (1 equiv) was dissolved in DMF (0.3 M final concentration). DIPEA (5 equiv) and CDI (4 equiv) were added successively, and the reaction was stirred for 2 h. Upon reaction completion, a solution of LiOH was added to hydrolyze any undesired carbonate formation. The organic lay was separated, and the solvent was removed in vacuo. The crude mixture was purified by preparative HPLC. HPLC fractions were frozen and dried by lyophilization.

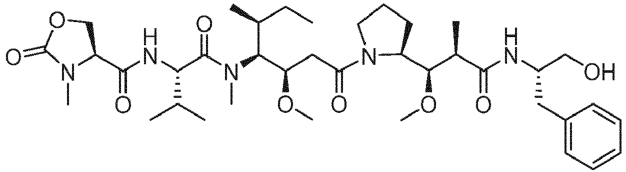

Analytical UPLC-MS: t_{R} = 1.67 min, *m*/*z* (ES+) 732.45 (M+H)⁺, found 732.99.

### Example 37.

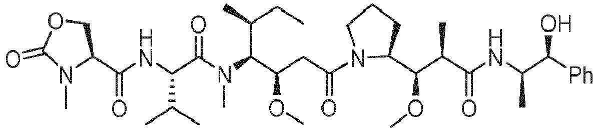

Analytical UPLC-MS: t_{R} = 2.04 min, *m*/*z* (ES+) 732.45 (M+H)⁺, found 732.90.

### Example 38.

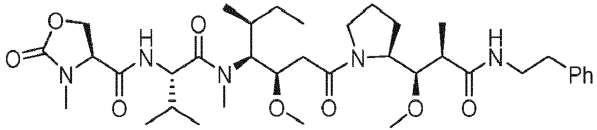

Analytical UPLC-MS: t_{R} = 2.23 min, *m*/*z* (ES+) 702.44 (M+H)⁺, found 702.52.

### Example 39.

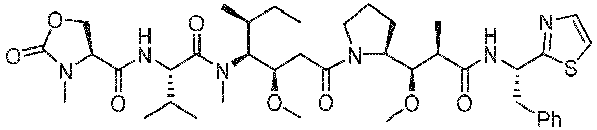

Analytical UPLC-MS: t_{R} = 2.04 min, *m*/*z* (ES+) 785.43 (M+H)⁺, found 785.48.

### Example 40.

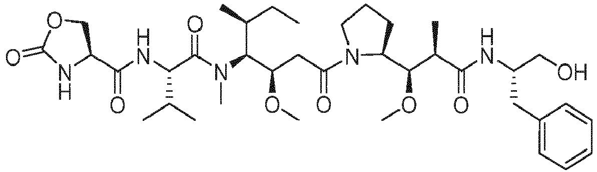

Analytical UPLC-MS: t_{R} = 1.57 min, *m*/*z* (ES+) 718.44 (M+H)⁺, found 718.84.

### Example 41.

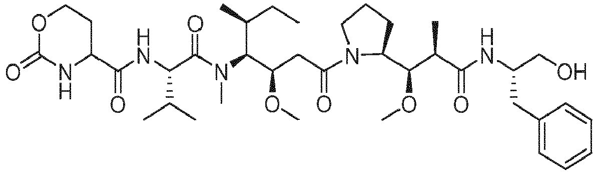

Analytical UPLC-MS: t_{R} = 1.58 min, *m*/*z* (ES+) 732.45 (M+H)⁺, found 732.99.

### Example 42.

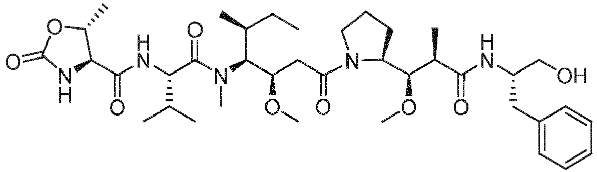

Analytical UPLC-MS: t_{R} = 1.70 min, *m*/*z* (ES+) 732.45 (M+H)⁺, found 732.73.

### Example 43.

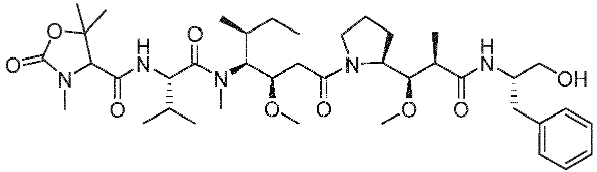

Analytical UPLC-MS: t_{R} = 1.78 min, *m*/*z* (ES+) 760.49 (M+H)⁺, found 760.85.

### Example 44.

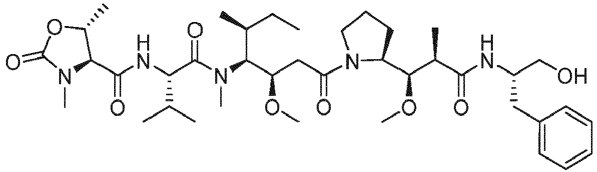

Analytical UPLC-MS: t_{R} = 1.70 min, *m*/*z* (ES+) 732.45 (M+H)⁺, found 732.73.

### Example 45.

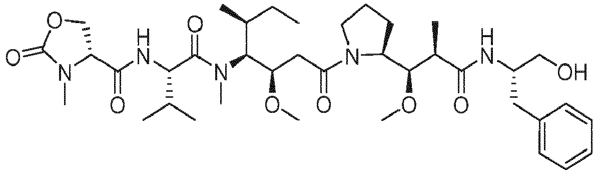

Analytical UPLC-MS: t_{R} = 1.61 min, *m*/*z* (ES+) 732.45 (M+H)⁺, found 732.55.

### General procedures for solution phase synthesis of auristatins

### Example 46. General procedure for synthesis of dipeptide carbamate linkers (Method A)

### Step 1. Synthesis of MC-VC-PAB-OPFP

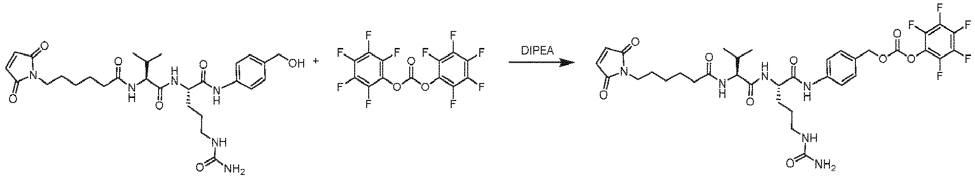

A 20 mL vial equipped with stir bar was charged with MC-VC-PABA (300 mg, 0.52 mmol), PFP carbonate (413mg, 1.04 mmol), DMF (5 mL), and DIPEA (137uL, 0.78 mmol). The mixture was stirred at RT for 3 hours. The solvent was removed in vacuo and redissolved in 20% piperidine in DMF (5 mL). The reaction was stirred for 1 hour. The solvent was removed in vacuo and was purified by Biotage reverse phase flash column chromatography with acetonitrile + 0.05% TFA and water + 0.05% TFA gradient up to 95% acetonitrile. The product fractions were lyophilized to afford 177 mg (43% yield). Analytical UPLC-MS: t_{R} = 2.05 min, *m*/*z* (ES+) 783.27 (M+H)⁺, found 783.42.

### Step 2. Drug-Linker formation

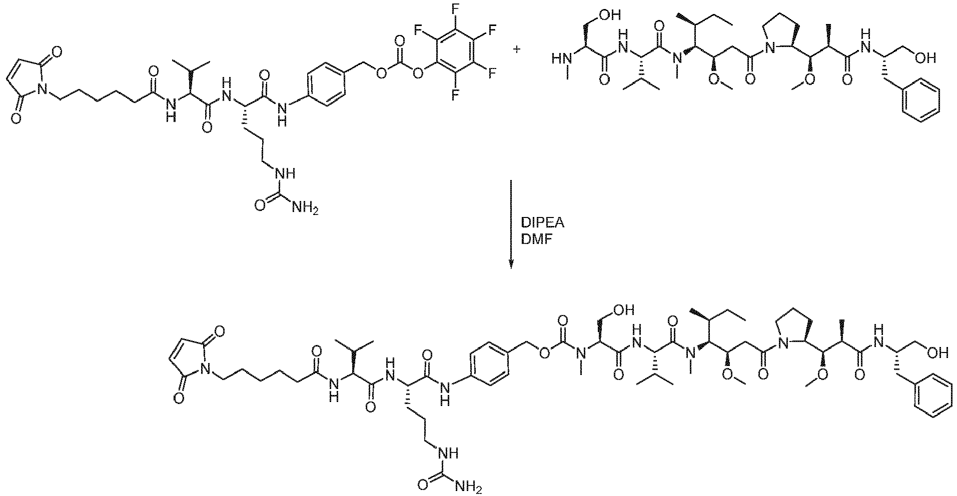

A 20 mL vial equipped with stir bar was charged with the compound of Example 12 (70 mg, 0.1 mmol), MC-VC-PAB-OPFP (78 mg, 0.1 mmol), DMF (5 mL), and DIPEA (26 uL, 0.15 mmol). The mixture was stirred at RT overnight. The solvent was removed in vacuo and was purified by preparative HPLC. The product fractions were lyophilized to afford 80.7 mg (62 % yield). Analytical UPLC-MS: t_{R} = 1.82 min, *m*/*z* (ES+) 1304.75 (M+H)⁺ found 1305.54.

### Example 47. General procedure for synthesis of (D)Leu-Ala-Glu tripeptide carbamate drug-linkers (Method B)

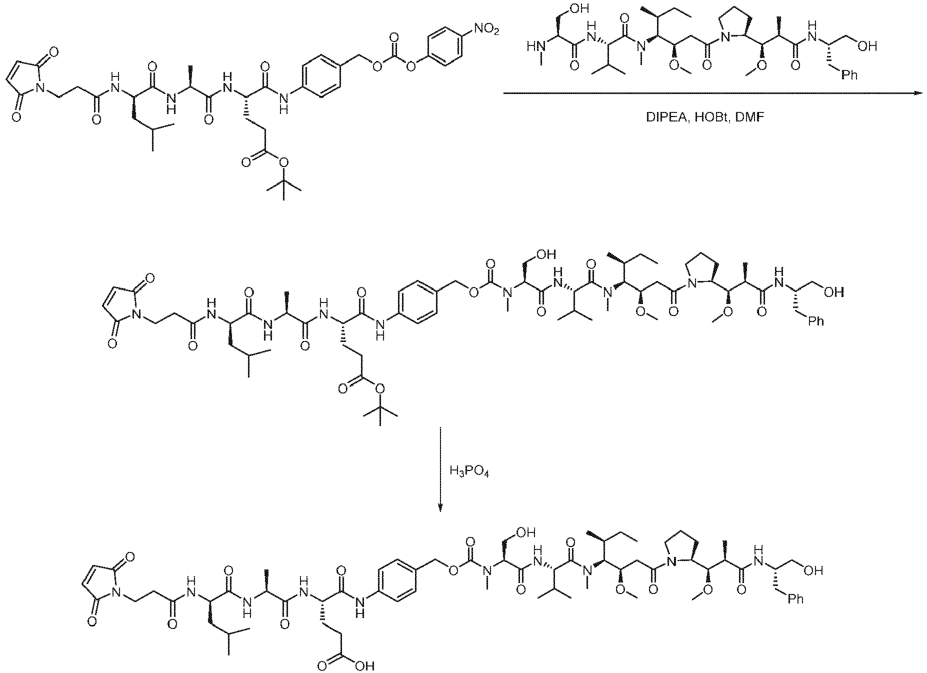

This is a modified version of a previously reported procedure. *See* WO 2021/055865.

A 4 mL vial equipped with stir bar was charged with the compound from Example 12 (100 mg, 0.14 mmol), MC-(D)-Leu-Ala-Glu(OtBu)-PABA-OₚNP (114 mg, 0.14 mmol), DMF (5 mL), DIPEA (51 uL, 0.28 mmol), and HOBt (37 mg, 0.28 mmol). The mixture was stirred at RT overnight. The solvent was removed in vacuo.

The crude mixture was dissolved in propionitrile (3 mL), and H₃PO₄ (3 mL) was slowly added to the reaction mixture at RT. The reaction mixture was stirred for 2 h and priopionitrile (6 mL) was added to the mixture. The organic layer was separated, and the reaction was concentrated and purified by HPLC. The product fractions were lyophilized to afford 54 mg (26% yield).

Analytical UPLC-MS: t_{R} = 1.71 min, *m*/*z* (ES+) 1319.71 (M+H)⁺, found 1320.06.

### Example 48. General procedure for synthesis of quaternary linkers (Method C)

### Step 1. Synthesis of MC-VC-PAB-CI

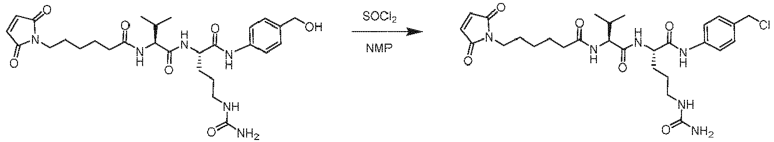

A 20 mL vial equipped with stir bar was charged with solution of MC-Val-Cit-PAB-OH (200 mg, 349.25 umol) in N-methyl-2-pyrrolidone (NMP, 3 mL) and then cooled to 0°C. A solution of SOCl₂ (70.64 mg, 593.73 umol) in NMP (0.5 mL) was added dropwise. The mixture was stirred at 20°C for 30 min. TLC (ethyl acetate: methanol = 3: 1, Rf = 0.5) showed the reaction was completed. The mixture was poured into ice water and filtered, washed with acetonitrile and ethyl acetate, dried in high vacuum to give MC-Val-Cit-PAB-Cl (100 mg, yield 48.4%). Analytical UPLC-MS: t_{R} = 2.05 min, *m*/*z* (ES+) 591.27 (M+H)⁺, found 591.42. 1H NMR: (400MHz, DMSO-d6) δ = 10.16 - 9.89 (m, 1H), 8.08 (br d, J=7.4 Hz, 1H), 7.79 (br d, J=8.6 Hz, 1H), 7.60 (br d, J=8.5 Hz, 2H), 7.36 (d, J=8.5 Hz, 2H), 7.00 (s, 2H), 5.96 (br t, J=5.4 Hz, 1H), 5.52 - 5.20 (m, 2H), 4.71 (s, 2H), 4.45 - 4.31 (m, 1H), 4.19 (br t, J=7.6 Hz, 1H), 3.45 - 3.34 (m, 2H), 3.09 - 2.85 (m, 2H), 2.14 (tq, J=7.2, 14.5 Hz, 2H), 2.03 - 1.85 (m, 1H), 1.75 - 1.55 (m, 2H), 1.54 - 1.27 (m, 6H), 1.26 - 1.10 (m, 2H), 0.83 (br dd, J=6.8, 12.9 Hz, 6H).

### Step 2. Drug-Linker formation

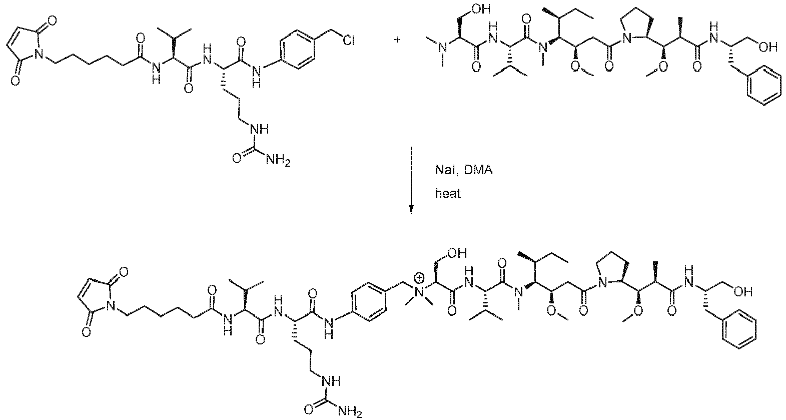

A 4 mL vial equipped with stir bar was charged with the compound of Example 8 (3.3 mg, 0.004 mmol), mc-VC-PAB-Cl (4.8 mg, 0.008 mmol), DMA (0.5 mL), and NaI (1.2 mg, 0.008 mmol). The mixture was stirred at 70° C for 4 hours. The reaction was purified by preparative HPLC. The product fractions were lyophilized to afford 3.9 mg (71% yield) of the title compound. Analytical UPLC-MS : t_{R} = 1.51 min, *m*/*z* (ES+) 1275.62 (M)⁺, found 1275.46.

### Example 49.

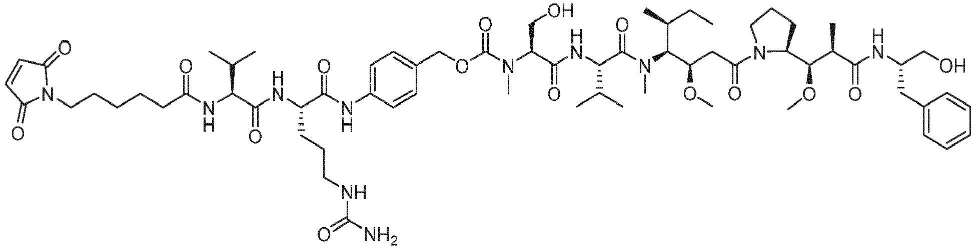

Compound was synthesized using method A. 62% yield. Analytical UPLC-MS: t_{R} = 1.82 min, *m*/*z* (ES+) 1304.75 (M+H)⁺, found 1305.54.

### Example 50.

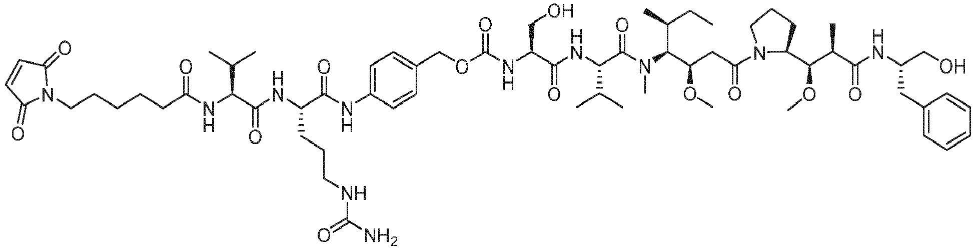

Compound was synthesized using method A. 43% yield. Analytical UPLC-MS: t_{R} = 1.72 min *m*/*z* (ES+) 1290.73 (M+H)⁺, found 1291.59.

### Example 51.

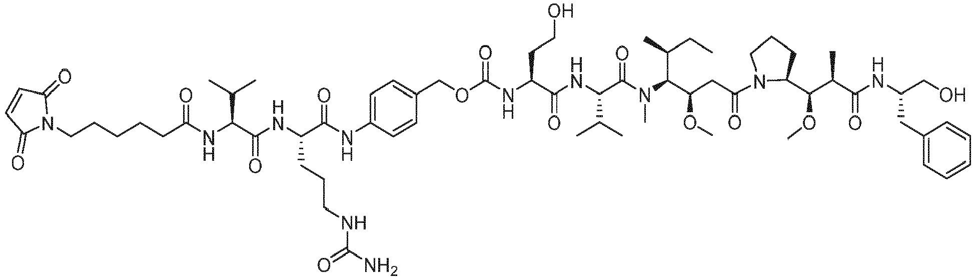

Compound was synthesized using method A. 52% yield. Analytical UPLC-MS: t_{R} = 1.74 min, *m*/*z* (ES+) 1304.75 (M+H)⁺, found 1305.28.

### Example 52.

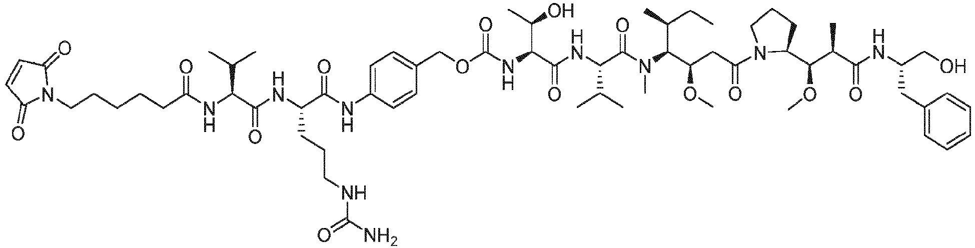

Compound was synthesized using method A. 64% yield. Analytical UPLC-MS: t_{R} = 1.77 min, *m*/*z* (ES+) 1304.75 (M+H)⁺, found 1304.61.

### Example 53.

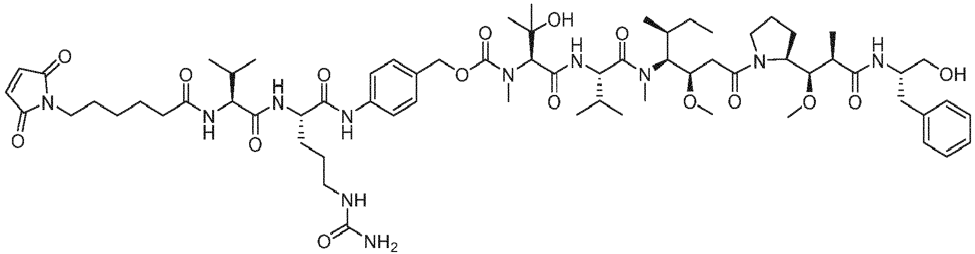

Compound was synthesized using method A. 15% yield. Analytical UPLC-MS: t_{R} = 1.92 min *m*/*z* (ES+) 1332.78 (M+H)⁺, found 1333.51.

### Example 54.

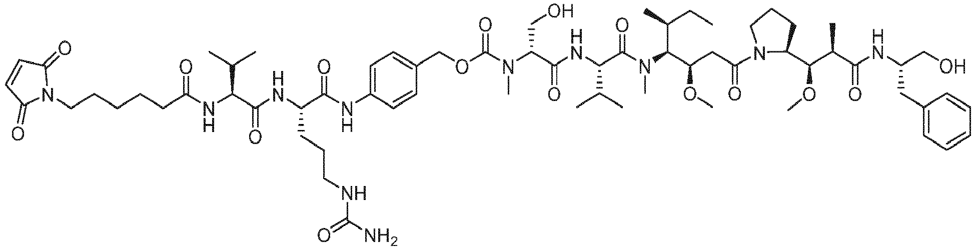

Compound was synthesized using method A. 57% yield. Analytical UPLC-MS: t_{R} = 1.68 min, *m*/*z* (ES+) 1304.75 (M+H)⁺, found 1305.43.

### Example 55.

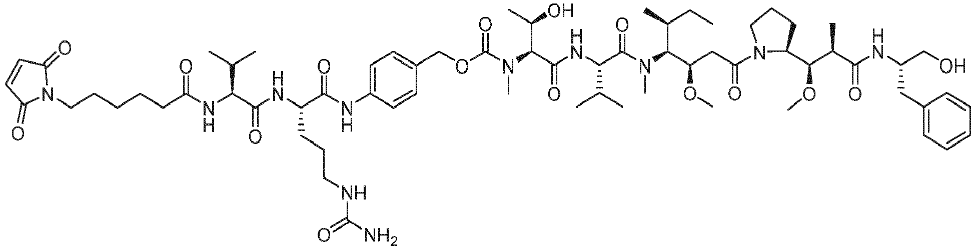

Compound was synthesized using method A. 47% yield. Analytical UPLC-MS: t_{R} = 1.72 min, *m*/*z* (ES+) 1318.77 (M+H)⁺, found 1319.31.

### Example 56.

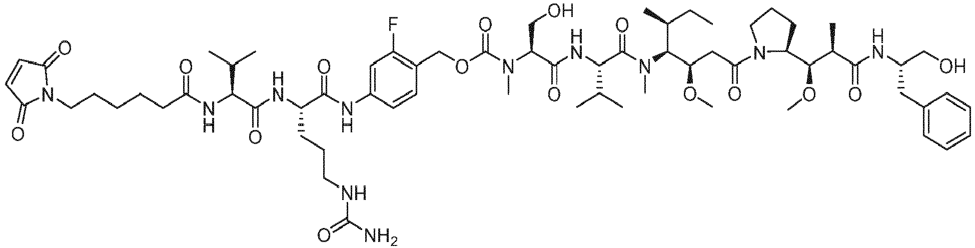

Compound was synthesized using method A. 80% yield. Analytical UPLC-MS: t_{R} = 1.75 min, *m*/*z* (ES+) 1322.74 (M+H)⁺, found 1323.30.

### Example 57.

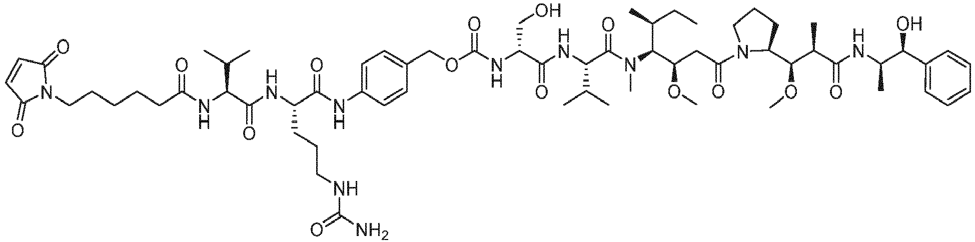

Compound was synthesized using method A. 22% yield. Analytical UPLC-MS: t_{R} = 1.67 min, *m*/*z* (ES+) 1291.58 (M+H)⁺, found 1291.42.

### Example 58.

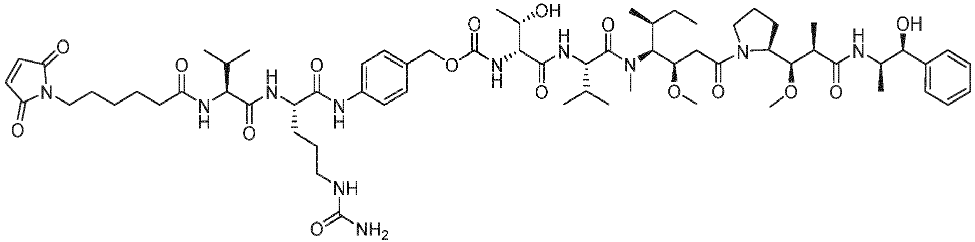

Compound was synthesized using method A. Analytical UPLC-MS: t_{R} = 1.71 min, *m*/*z* (ES+) 1305.60 (M+H)⁺, found 1304.91.

### Example 60.

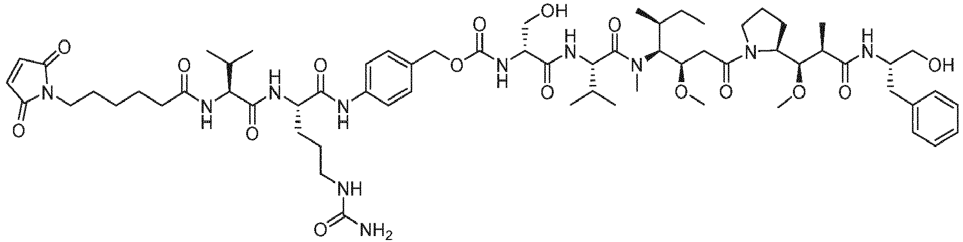

Compound was synthesized using method A.

### Example 61.

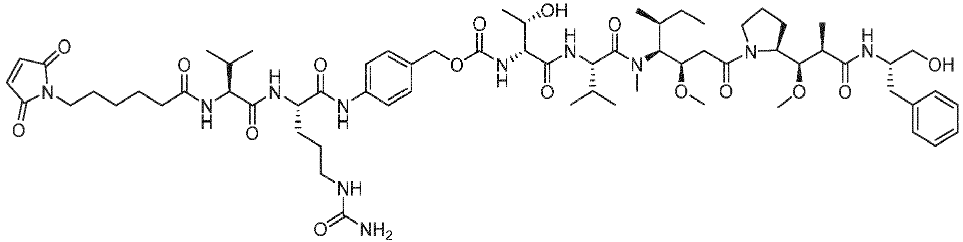

Compound was synthesized using method A.

### Example 62.

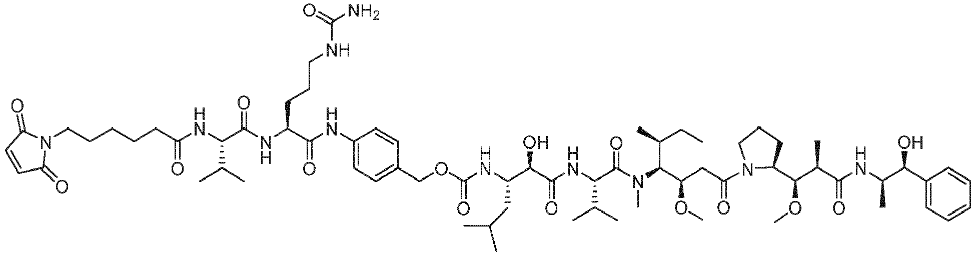

Compound was synthesized using method A. 27% yield. Analytical UPLC-MS: t_{R} = 1.87 min, *m*/*z* (ES+) 1346.80 (M+H)⁺, found 1347.50.

### Example 63.

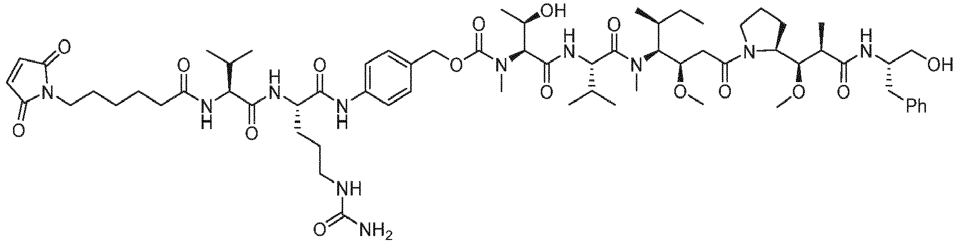

Compound was synthesized using method A. Analytical UPLC-MS: t_{R} = 1.57 min, *m*/*z* (ES+) 1332.77 (M+H)⁺, found 1332.81.

### Example 64.

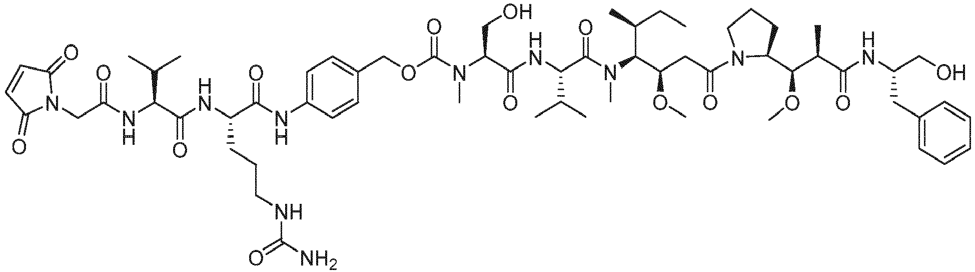

Compound was synthesized using method A. Analytical UPLC-MS: t_{R} = 1.64 min, *m*/*z* (ES+) 1248.69 (M+H)⁺, found 1249.42.

### Example 65.

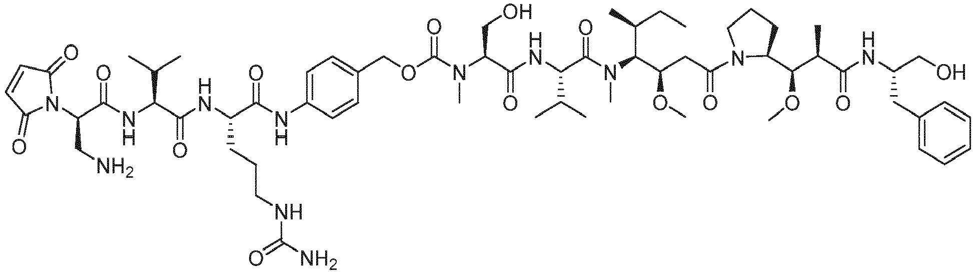

Compound was synthesized using method A. Analytical UPLC-MS: t_{R} = 1.85 min, *m*/*z* (ES+) 1277.71 (M+H)⁺, found 1278.21.

### Example 66.

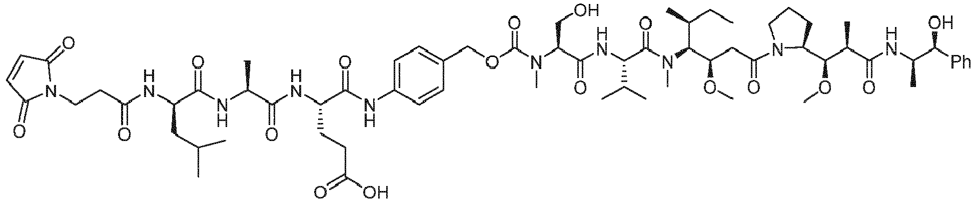

Compound was synthesized using method B. Analytical UPLC-MS: t_{R} = 1.69 min, *m*/*z* (ES+) 1319.71 (M+H)⁺, found 1319.75.

### Example 67.

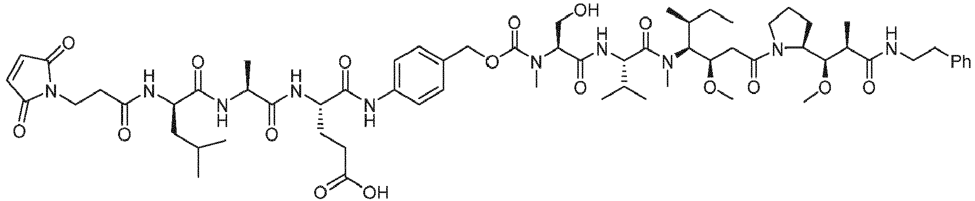

Compound was synthesized using method B. Analytical UPLC-MS: t_{R} = 1.71 min, *m*/*z* (ES+) 1289.70 (M+H)⁺, found 1289.83.

### Example 68.

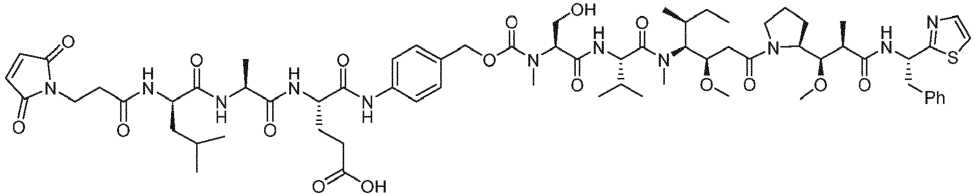

Compound was synthesized using method B. Analytical UPLC-MS: t_{R} = 1.75 min, *m*/*z* (ES+) 1372.69 (M+H)⁺, found 1372.75.

### Example 69.

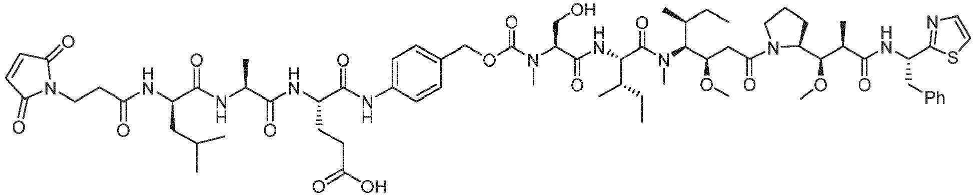

Compound was synthesized using method B. Analytical UPLC-MS: t_{R} = 1.81 min, *m*/*z* (ES+) 1386.70 (M+H)⁺, found 1386.73.

### Example 70.

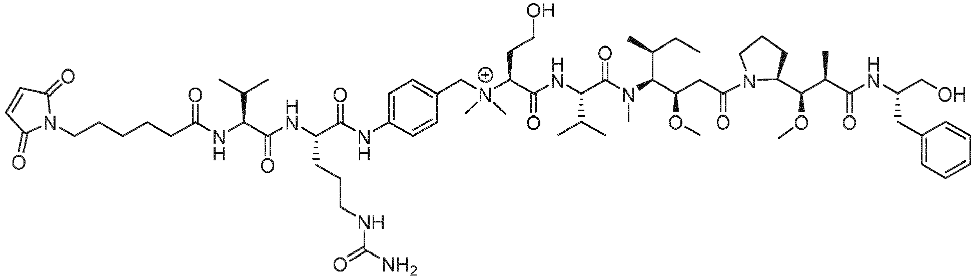

Compound was synthesized using method C. Analytical UPLC-MS: t_{R} = 1.52 min, *m*/*z* (ES+) 1288.79 (M)⁺, found 1288.88.

### Example 71.

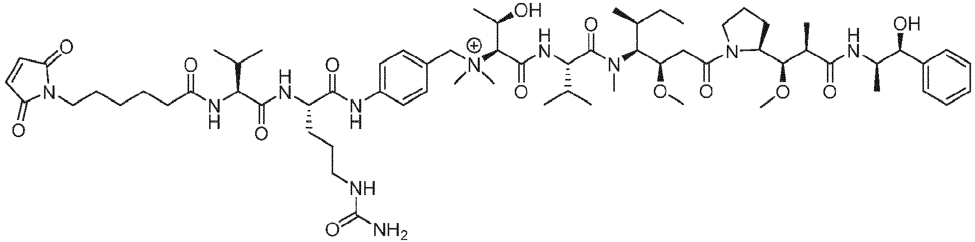

Compound was synthesized using method C. Analytical UPLC-MS: t_{R} = 1.39 min, *m*/*z* (ES+) 1288.79 (M)⁺, found 1288.82.

### Example 72.

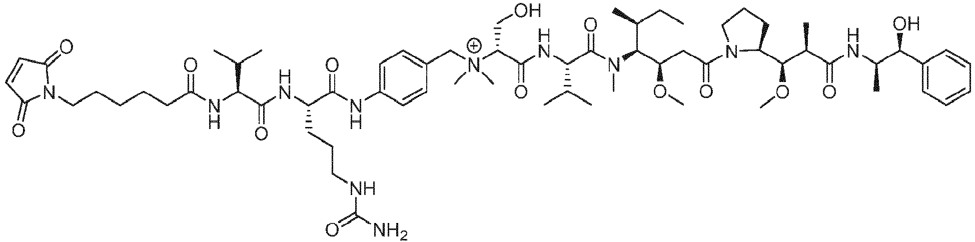

Compound was synthesized using method C. Analytical UPLC-MS: t_{R} = 1.51 min, *m*/*z* (ES+) 1274.78 (M)⁺, found 1274.88.

### Example 73.

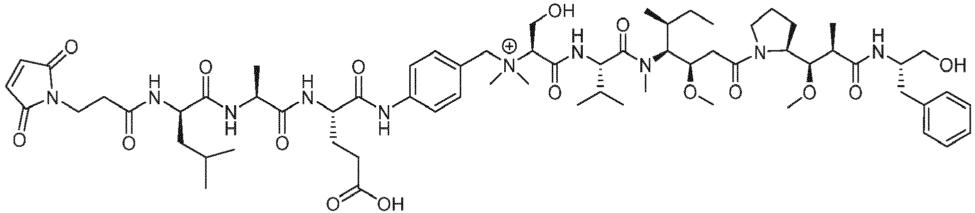

Compound was synthesized using method C. Analytical UPLC-MS: t_{R} = 1.60 min, *m*/*z* (ES+) 1289.74 (M)⁺, found 1289.92.

### Example 74.

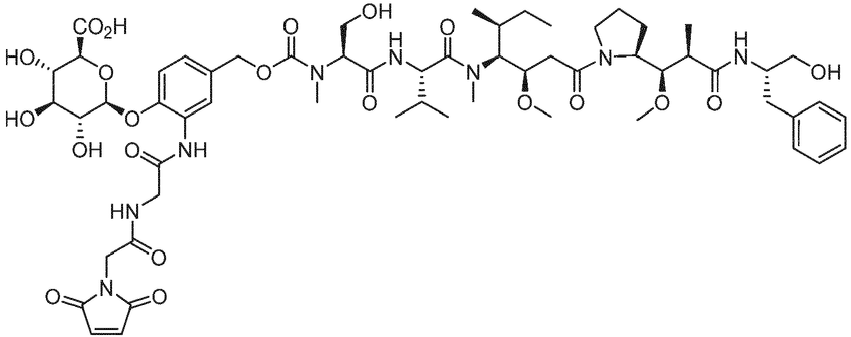

Modified synthesized according to a known procedure (Burke, P. J. et al. Mol. Cancer Ther. 2017, 16, 116-123).

Analytical UPLC-MS: t_{R} = 1.68 min, *m*/*z* (ES+) 1241.58 (M+H)⁺, found 1241.25.

### Example 75. Measurement of LogD

The LogD (distribution coefficient) of the compounds was measured using a miniaturized 1-octanol/pH 7.4 buffer shake flask method followed by LC/MS/MS analysis. Values were determined by measuring the compound partitioned in the organic and buffered layers. Results are shown in Table 4.

### Example 76. In vitro cytotoxicity

The cytotoxicity of free drugs and drug conjugates were measured by a cell proliferation assay employing the protocol described in Promega Corp. Technical Bulletin TB288; and Mendoza et al., 2002, Cancer Res. 62:5485-5488), the methods of which are specifically incorporated by reference herein. Briefly, an aliquot of 40 µl of cell culture containing about 400 cells in medium is deposited in each well of a 384-well, opaque-walled plate. A 10 µL aliquot of free drug or drug conjugate is added to the experimental wells and incubated for 96 h and are then equilibrated to room temperature for approximately 30 minutes whereupon a volume of CellTiter-GloTM reagent equal to the volume of cell culture medium present in each well is added. The contents are mixed for 2 minutes on an orbital shaker to induce cell lysis and the plate is incubated at room temperature for 10 minutes to stabilize the luminescence signal for recordation. Results are shown in Table 4.

**Table 4. Auristatin free drug LogD values and in vitro ICso (nM) values on cancer cell lines.**

| **Free drug Example** # | DEL ALCL | Karpas299 NHL | L540cy HL | LogD |
|---|---|---|---|---|
| **26** | 4.2 | 5 | 20 | |
| **32** | 64 | 56 | 920 | 0.3 |
| **12** | 1.1 | 0.3 | 4.8 | 1.2 |
| **28** | 7.8 | 9.4 | 28 | 2.0 |
| **36** | 6.7 | 27 | 197 | |
| **23** | 17 | 66 | 662 | |
| **40** | 12.3 | 60 | 442 | |
| **24** | 13 | 43 | 621 | |
| **41** | 15 | 62 | 457 | |
| **25** | 18.9 | 62 | 457 | |
| **42** | 16 | 64 | 481 | |
| **13** | 1.2 | 2.2 | 5 | 1.4 |
| **43** | 3.2 | 5.9 | 19 | |
| **17** | 2.5 | 7.6 | 16.2 | 1.1 |
| **18** | 0.6 | 2.3 | 16 | 1.9 |
| **19** | 0.4 | 0.5 | 2.5 | 2.4 |
| **37** | >1000 | >1000 | >1000 | |
| **38** | 148 | >1000 | 512 | |
| **39** | 512 | >1000 | >1000 | |
| **8** | 0.7 | 1.4 | 6.6 | 1.6 |
| **45** | 8.5 | 40.8 | 272 | 1.5 |
| **14** | 3.5 | 7.4 | 10 | 1.1 |
| **20** | 0.3 | 1 | 4.7 | 1.9 |
| **15** | 0.7 | 2.3 | 3.9 | |
| **22** | 5.8 | 13 | 16 | |
| **33** | 1.8 | 1.7 | 6.4 | 3.2 |
| **29** | 12.7 | 13.7 | 58 | 2.0 |
| **30** | 396 | 481 | >1000 | 1.2 |
| **31** | 2.1 | 1.7 | 4.7 | 3.4 |
| **27** | 1.9 | 2.3 | 13 | |
| **9** | 0.8 | 0.9 | 4 | |
| **10** | 0.4 | 0.5 | 3 | |
| **11** | 1 | 1 | 6 | |
| **MMAE** | 0.05 | 0.1 | 0.1 | 1.9 |
| **MMAF** | 44 | 41 | 47 | 0.03 |

### Example 77. Conjugation of Drug-Linker compounds to antibodies to form Ligand-Drug Conjugate compounds

Antibodies were partially reduced using the appropriate equivalents of TCEP according to the procedure, which is specifically incorporated by reference herein, of US 2005/0238649. Briefly, the antibody in phosphate buffered saline with 2 mM EDTA, pH 7.4, was treated with 2.1 eq. TCEP and then incubated at 37° C for about 45 minutes. The thiol/Ab value was checked by reacting the reduced antibody with MC-VC-MMAE and using hydrophobic interaction chromatography to determine the loading.

The auristatin drug linker compounds were conjugated to the partially reduced antibody using the method, which is specifically incorporated by reference herein, of US 2005/0238649. Briefly, Drug-Linker compound (50% excess) in DMSO, was added to the reduced antibody in PBS with EDTA along with additional DMSO for a total reaction cosolvent of 10-20%. After 30 minutes at ambient temperature, an excess of QuadraSil MPTM was added to the mixture to quench all unreacted maleimide groups. The resulting Ligand-Drug Conjugate compound was then purified, and buffer exchanged by desalting using Sephadex G25 resin into pH 6.5 histidine buffer and kept at -80° C until further use. The protein concentration of the resulting ADC composition was determined at 280 nm. The drug-antibody ratio (DAR) was confirmed by comparing predicted and observed changes in the molecular weights of heavy and light chains using PLRP-MS analysis and confirming absence of underloaded species.

### Example 78. In vivo cytotoxicity of Ligand-Drug Conjugate compounds

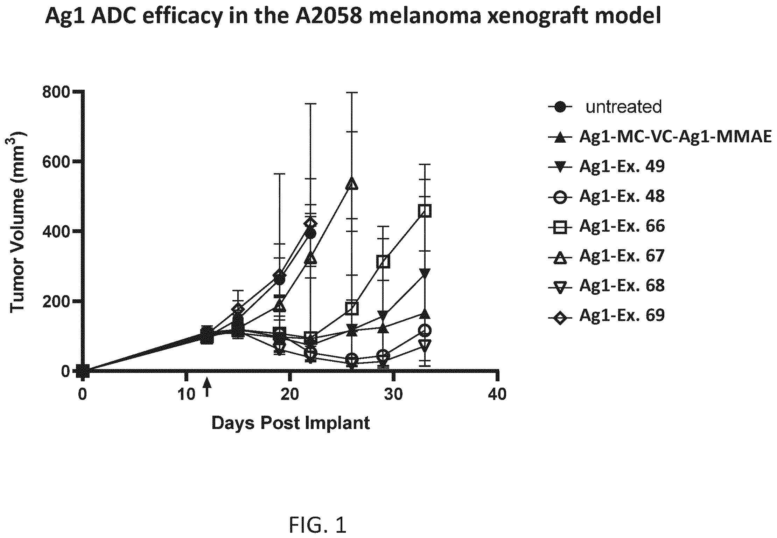

Cancer cells were implanted into mice. Upon tumor engraftment, mice were randomized to study groups (n = 5) once the average tumor volume reached approximately 100 mm³. ADC prepared from reduced antibody and compound was administered via an intraperitoneal or intravenous injection. Tumor volumes are calculated using the formula (0.5×L×W2) where L and W are the longer and shorter of two bidirectional measurements. Complete response (CR) is defined here as absence of measurable mass at tumor site during an experiment. Results are showin in FIGs. 1 to 5 and in Table 5 for Ligand-Drug Conjugate compounds with an average drug loading of 4 Drug-Linker moieties per antibody.

FIG. 1 shows the efficacy of a 6mg/kg dose of a variety of Ag1 ADCs as measured in the A2058 melanoma xenograft model. The data show that Ag1 ADCs comprising hydrophilic auristatin Drug Units have efficacy comparable to ADCs comprising MMAE.

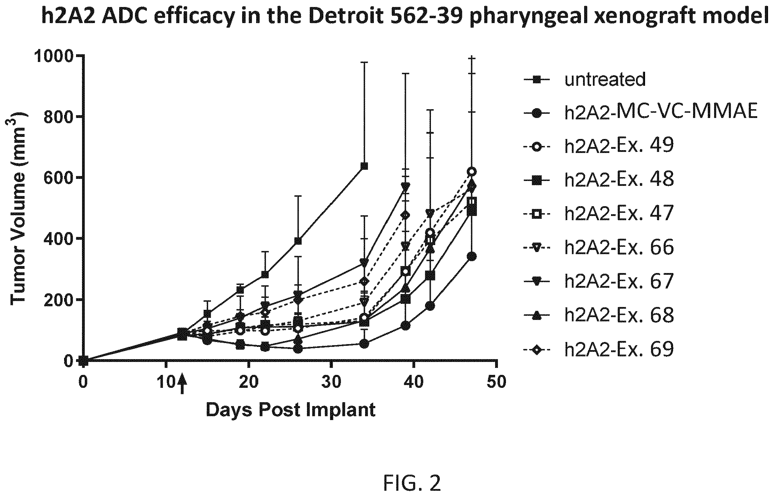

FIG. 2 shows the efficacy of a 3mg/kg dose of a variety of h2A2 ADCs as measured in the Detroit 562-39 pharyngeal xenograft model. The data show that h2A2 ADCs comprising hydrophilic auristatin Drug Units have efficacy comparable to ADCs comprising MMAE.

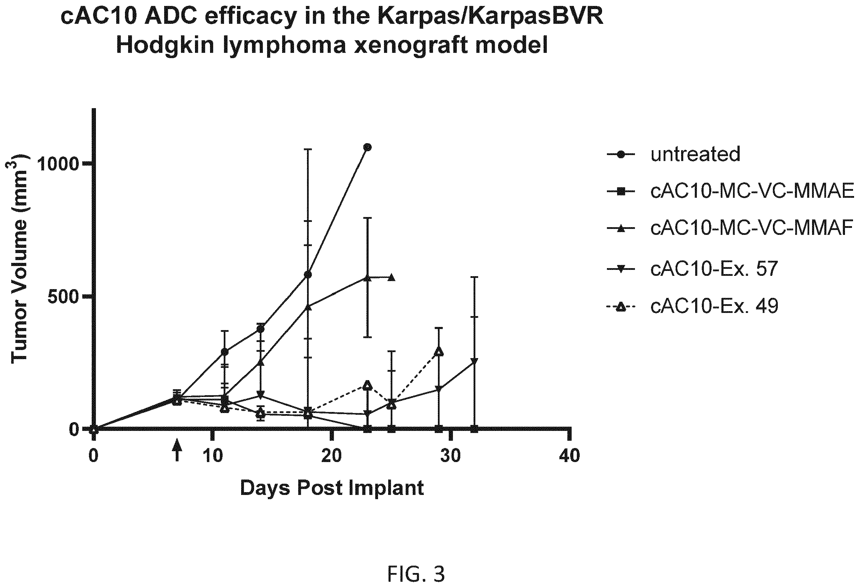

FIG. 3 shows the efficacy of a 3 mg/kg dose of a variety of cAC10 ADCs as measured in the Karpas/KarpasBVR admixed Hodgkin lymphoma xenograft model. The data show that cAC 10 ADCs with hydrophilic auristatin Drug Units have bystander activity between that of MC-VC-MMAE and MC-VC-MMAF.

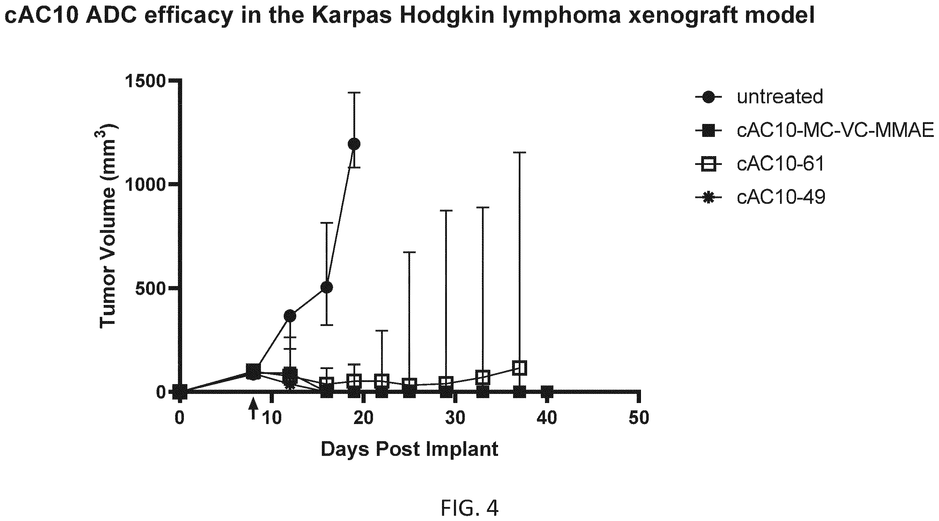

FIG. 4 shows the efficacy of a 1 mg/kg dose of a variety of cAC10 ADCs in the Karpas Hodgkin lymphoma xenograft model. The data show that cAC10 ADCs comprising hydrophilic auristatin Drug Units have efficacy comparable to ADCs comprising MMAE.

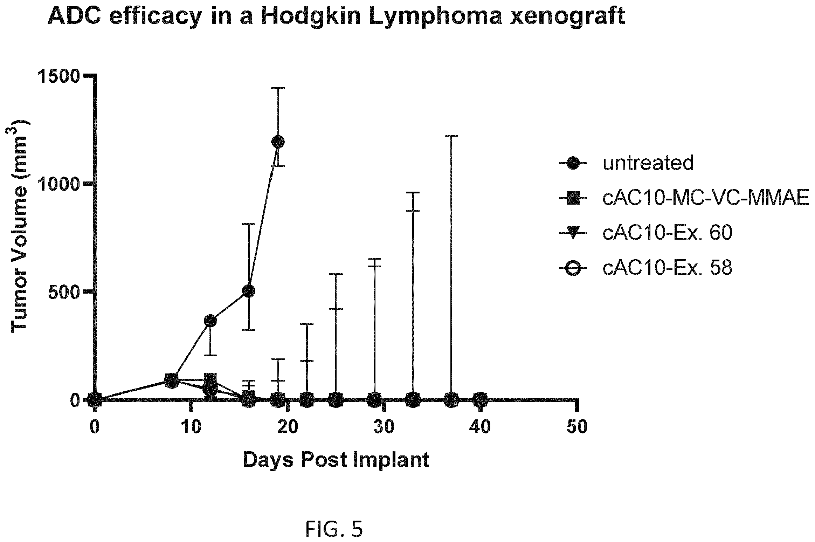

FIG. 5 shows the efficacy of a 1 mg/kg dose of a variety of cAC10 ADCs in the Karpas Hodgkin xenograft model. The data show that cAC10 ADCs comprising hydrophilic auristatin Drug Units have efficacy comparable to ADCs comprising MMAE.

### Example 79. Toxicity Determinations of Ligand-Drug Conjugate compounds.

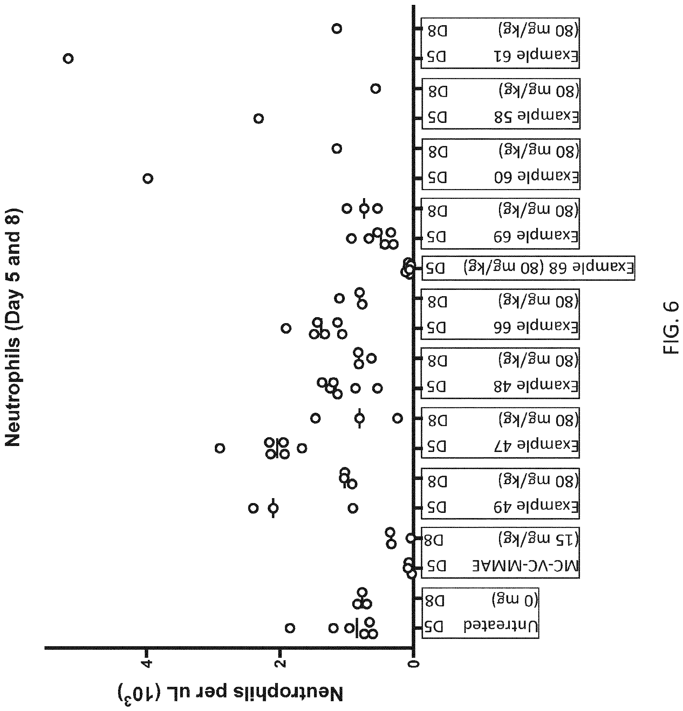

*In vivo* rat safety studies were done in naive female Sprague Dawley rats (Envigo). Studies had 3 animals/group/timepoint and included a vehicle control group (1X PBS pH 7.4). Animals were administered a single dose of 15 or 80 mg/kg of an h00 ADC (DAR = 4) via intravenous injection. Blood was drawn at days 5 and day 8. Blood was sampled under isoflurane anesthesia via the jugular vein for periodic hematology analysis on the Sysmex XT-2000iV. At necropsy, blood was collected from the caudal vena cava for hematology and clinical chemistry panels. Clinical chemistry was analyzed on a Beckman Coulter AU680. Results are shown in FIGs. 6 to 9.

FIG. 6 demonstrates that neutrophil counts are higher at on Day 5 and recovery of neutrophil counts on Day 8 are higher after administering Ligand-Drug Conjugate compounds comprising hydrophilic auristatin Drug Units when compared to MMAE containing conjugates.

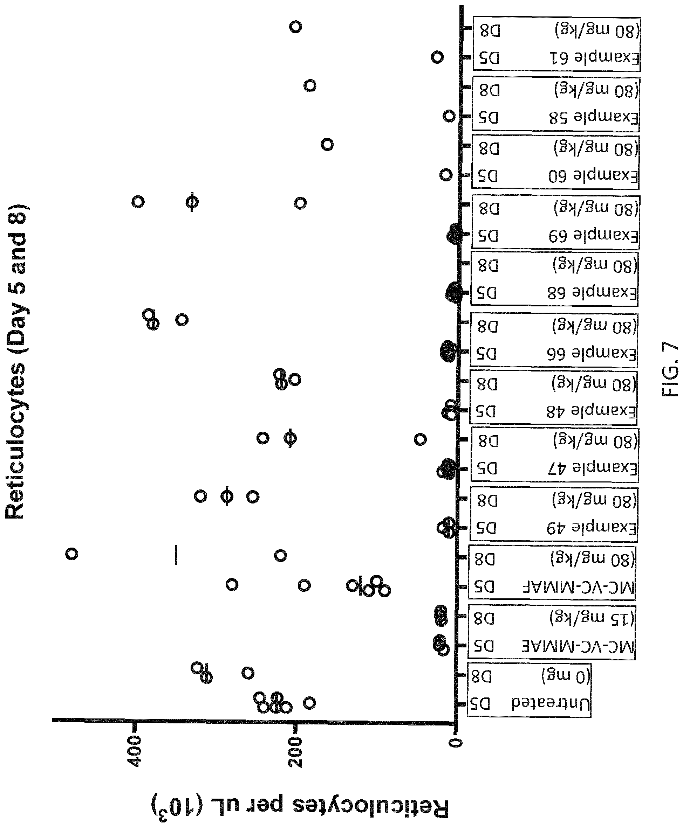

FIG. 7 demonstrates that recovery of reticulocytes is greatly improved after administering Ligand-Drug Conjugate compounds comprising hydrophilic auristatin Drug Units when compared to MMAE containing conjugates.

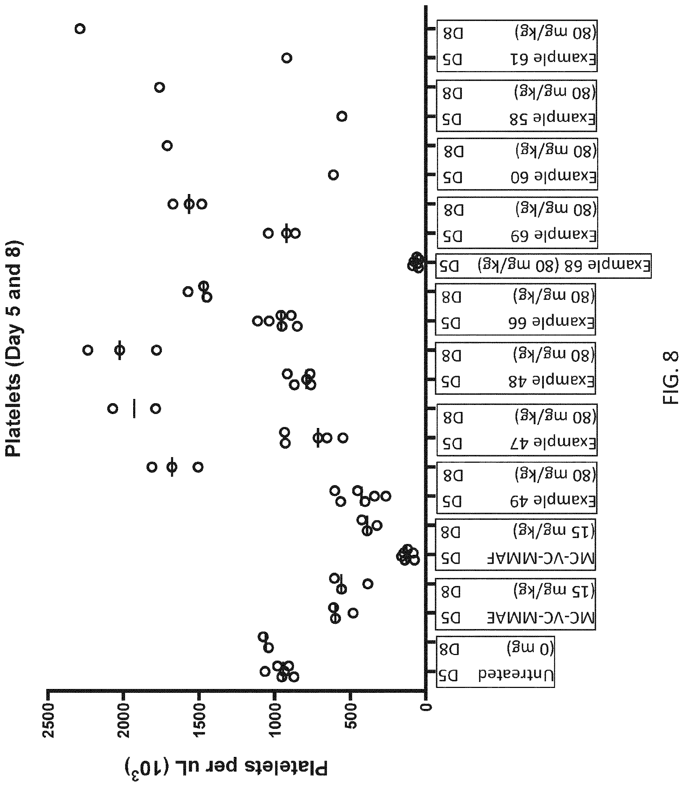

FIG. 8 shows that administering Ligand-Drug Conjugate compounds with hydrophilic auristatin Drug Units does not impact the platelet count as heavily as administering an MMAF containing conjugate. Recovery of platets is also better after administering Ligand-Drug Conjugate compounds comprising hydrophilic auristatin Drug Units when compared to MMAE and MMAF containing conjugates.

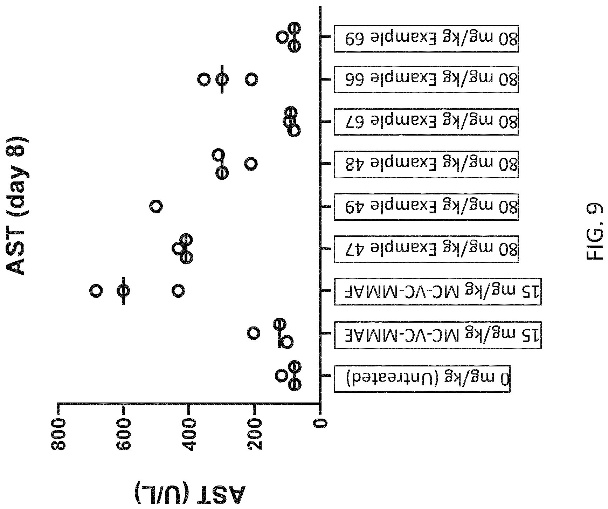

FIG. 9 shows that aspartate transaminease (AST) levels indicating liver damage are lower 8 days after administering 80 mg/kg of igand-Drug Conjugate compounds comprising hydrophilic auristatin Drug Units when compared to after administering 15 mg/kg of an MMAF containing conjugates. The AST levels 8 days following administration are comparable for an 80 mg/kg dose of some Ligand-Drug Conjugate compounds comprising hydrophilic auristatin Drug Units when compared to a 15 mg/kg dose of an MMAE conjugate.

**Table 5. In vitro IC₅₀ (ng/mL) values of auristatin anti-CD30 ADCs on CD30-expressing cell lines using cAC10 ADCs with an average drug loading of about 4 of selected Drug-Linker compounds.**

| **Drug-Linker moiety Example** # | DEL ALCL | Karpas299 NHL | L540cy HL |
|---|---|---|---|
| **57** | 0.01 | 0.2 | NA |
| **60** | NA | 7 | 9 |
| **61** | NA | 7 | 15 |
| **49** | 0.01 | 0.6 | 6.3 |
| **47** | 0.1 | 1.2 | 9.3 |
| **51** | 12 | 290 | >1000 |
| **50** | 3.8 | >1000 | >1000 |
| **52** | 22 | >1000 | >1000 |
| **65** | NA | NA | NA |
| **53** | 0.04 | 1.2 | 3.8 |
| **74** | 0.01 | 0.5 | 3.6 |
| **64** | 0.01 | 0.2 | 3.2 |
| **48** | 0.09 | 0.4 | 4.3 |
| **54** | 0.3 | 5.1 | 4.9 |
| **55** | 1.7 | 29 | 33 |
| **56** | 1 | 15 | 35 |
| **66** | 0.4 | 5.6 | 8.2 |
| **67** | 1.3 | 58 | >1000 |
| **68** | 1.5 | 0.9 | 4.7 |
| **69** | 1.4 | >1000 | >1000 |
| **58** | 0.03 | 1 | 1.9 |
| **73** | 0.01 | 0.1 | 13 |
| **62** | >1000 | >1000 | >1000 |
| **70** | 0.1 | 0.1 | 1 |
| **71** | 0.2 | 0.3 | 4 |
| **72** | 0.1 | 0.1 | 4 |
| **MC-VC-MMAE** | 0.4 | 0.6 | 2 |
| **MC-VC-MMAF** | 0.1 | 1 | 3 |

### Example 80. Comparison of compound tolerability

Tolerability of an ADC comprising the compound of Example 8 of the present application was compared to tolerability of ADCs comprising the known compounds MMAE and MMAF. Without being bound by theory, the improvements in tolerability are believed to be related to reduced toxicities in the bone marrow and in the liver that are a result of the OH moieties on the compound of Example 8. See Table 6 below.

**Table 6. Tolerability comparison of compound 1.1 to MMAE and MMAF**

| **Compound** | **LogD** | **Avg IC50 Free drug ^ (nM)** | **Avg IC50 of corresponding dipeptide linked ADC^* (ng/mL)** | **Maximum tolerated dose** (mg/kg)** | |
|---|---|---|---|---|---|
| | | | | Based on reduced Neutrophil counts | Based on elevated AST level |
| **MMAE** | 1.9 | 0.2 | 1 | 15 | >15 |
| **Example 8** | 1.6 | 2.9 | 1.5 | >80 | >80 |
| **MMAF** | 0.03 | 44 | 1.3 | >15 | 15 |

| | | | | | |
|---|---|---|---|---|---|
| ^ **Average cytotoxicity of DEL, Karpas299, and L540cy cell lines** * **anti-CD30 ADCs** ** **Dosed in Sprague-Dawley Rats** | | | | | |

While the foregoing written description of the compounds, uses, and methods described herein enables one of ordinary skill to make and use the compounds, uses, and methods described herein, those of ordinary skill will understand and appreciate the existence of variations, combinations, and equivalents of the specific embodiment, method, and examples herein. The compounds, uses, and methods provided herein should therefore not be limited by the above-described embodiments, methods, or examples, but rather encompasses all embodiments and methods within the scope and spirit of the compounds, uses, and methods provided herein.

All references disclosed herein are incorporated by reference in their entirety.

Unless otherwise indicated, all sequences and SEQ IDs referenced in the application correspond to the appropriately numbered sequence in the table below.

The Table of Sequences below provides exemplary antibody sequences consistent with the present disclosure. In this table, all targets correspond to human orthologs unless otherwise specified.

| SEQ ID NO | Description | Sequence |
|---|---|---|
| 1 | Anti-PD-L1 HC | QVQLVQSGAEVKKPGSSVKVSCKTSGDTFSTAAISWVRQAPGQGLEWMGGIIPIFGKAHY AQKFQGRVTITADESTSTAYMELSSLRSEDTAVYFCARKFHFVSGSPFGMDVWGQGTTVT VSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKLTVDK SRWQQGNVFSCSVMHEALHNHYTQKSLSLSPGK |
| 2 | Anti-PD-L1 H C v2 | QVQLVQSGAEVKKPGSSVKVSCKTSGDTFSTAAISWVRQAPGQGLEWMGGIIPIFGKAHY AQKFQGRVTITADESTSTAYMELSSLRSEDTAVYFCARKFHFVSGSPFGMDVWGQGTTVT VSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKLTVDK SRWQQGNVFSCSVMHEALHNHYTQKSLSLSPG |
| 3 | Anti-PD-L1 LALA HC | QVQLVQSGAEVKKPGSSVKVSCKTSGDTFSTAAISWVRQAPGQGLEWMGGIIPIFGKAHY AQKFQGRVTITADESTSTAYMELSSLRSEDTAVYFCARKFHFVSGSPFGMDVWGQGTTVT VSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKLTVDK SRWQQGNVFSCSVMHEALHNHYTQKSLSLSPGK |
| 4 | Anti-PD-L1 LALA HC v2 | QVQLVQSGAEVKKPGSSVKVSCKTSGDTFSTAAISWVRQAPGQGLEWMGGIIPIFGKAHY AQKFQGRVTITADESTSTAYMELSSLRSEDTAVYFCARKFHFVSGSPFGMDVWGQGTTVT VSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKLTVDK SRWQQGNVFSCSVMHEALHNHYTQKSLSLSPG |
| 5 | Anti-PD-L1 LC | EIVLTQSPATLSLSPGERATLSCRASQSVSSYLAWYQQKPGQAPRLLIYDASNRATGIPA RFSGSGSGTDFTLTISSLEPEDFAVYYCQQRSNWPTFGQGTKVEIKRTVAAPSVFIFPPS DEQLKSGTASVVCLLNNFYPREAKVQWKVDNALQSGNSQESVTEQDSKDSTYSLSSTLTL SKADYEKHKVYACEVTHQGLSSPVTKSFNRGEC |
| 6 | Anti-PD-L1 (engineered cysteines) LALA HC | QVQLVQSGAEVKKPGSSVKVSCKTSGDTFSTAAISWVRQAPGQGLEWMGGIIPIFGKAHY AQKFQGRVTITADESTSTAYMELSSLRSEDTAVYFCARKFHFVSGSPFGMDVWGQGTTVT VSSASTKGPSVFPLAPSSKSTSGGTAALGCLVKDYFPEPVTVSWNSGALTSGVHTFPAVL |
| | | QSSGLYSLSSVVTVPSSSLGTQTYICNVNHKPSNTKVDKKVEPKSCDKTHTCPPCPAPEA AGGPCVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKLTVDK SRWQQGNVFSCSVMHEALHNHYTQKSLSLSPGK |
| 7 | Anti-PD-L1 (engineered cysteines) LALA HC v2 | QVQLVQSGAEVKKPGSSVKVSCKTSGDTFSTAAISWVRQAPGQGLEWMGGIIPIFGKAHY AQKFQGRVTITADESTSTAYMELSSLRSEDTAVYFCARKFHFVSGSPFGMDVWGQGTTVT VSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEA AGGPCVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKLTVDK SRWQQGNVFSCSVMHEALHNHYTQKSLSLSPG |
| 8 | Anti-PD-L1 mIgG2a LALA HC | QVQLVQSGAEVKKPGSSVKVSCKTSGDTFSTAAISWVRQAPGQGLEWMGGIIPIFGKAHY AQKFQGRVTITADESTSTAYMELSSLRSEDTAVYFCARKFHFVSGSPFGMDVWGQGTTVT VSSAKTTAPSVYPLAPVCGDTTGSSVTLGCLVKGYFPEPVTLTWNSGSLSSGVHTFPAVL QSDLYTLSSSVTVTSSTWPSQSITCNVAHPASSTKVDKKIEPRGPTIKPCPPCKCPAPNA AGGPSVFIFPPKIKDVLMISLSPIVTCVVVDVSEDDPDVQISWFVNNVEVHTAQTQTHRE DYNSTLRVVSALPIQHQDWMSGKEFKCKVNNKDLPAPIERTISKPKGSVRAPQVYVLPPP EEEMTKKQVTLTCMVTDFMPEDIYVEWTNNGKTELNYKNTEPVLDSDGSYFMYSKLRVEK KNWVERNSYSCSVVHEGLHNHHTTKSFSRTPGK |
| 9 | Anti-PD-L1 mIgG2a LALA HC v2 | QVQLVQSGAEVKKPGSSVKVSCKTSGDTFSTAAISWVRQAPGQGLEWMGGIIPIFGKAHY AQKFQGRVTITADESTSTAYMELSSLRSEDTAVYFCARKFHFVSGSPFGMDVWGQGTTVT VSSAKTTAPSVYPLAPVCGDTTGSSVTLGCLVKGYFPEPVTLTWNSGSLSSGVHTFPAVL QSDLYTLSSSVTVTSSTWPSQSITCNVAHPASSTKVDKKIEPRGPTIKPCPPCKCPAPNA AGGPSVFIFPPKIKDVLMISLSPIVTCVVVDVSEDDPDVQISWFVNNVEVHTAQTQTHRE DYNSTLRVVSALPIQHQDWMSGKEFKCKVNNKDLPAPIERTISKPKGSVRAPQVYVLPPP EEEMTKKQVTLTCMVTDFMPEDIYVEWTNNGKTELNYKNTEPVLDSDGSYFMYSKLRVEK KNWVERNSYSCSVVHEGLHNHHTTKSFSRTPG |
| 10 | Anti-PD-L1 m K LC | EIVLTQSPATLSLSPGERATLSCRASQSVSSYLAWYQQKPGQAPRLLIYDASNRATGIPA RFSGSGSGTDFTLTISSLEPEDFAVYYCQQRSNWPTFGQGTKVEIKRADAAPTVSIFPPS SEQLTSGGASVVCFLNNFYPKDINVKWKIDGSERQNGVLNSWTDQDSKDSTYSMSSTLTL TKDEYERHNSYTCEATHKTSTSPIVKSFNRNEC |
| 11 | Anti-PD-L1 mIgG2a (engineered cysteines) LALA HC | QVQLVQSGAEVKKPGSSVKVSCKTSGDTFSTAAISWVRQAPGQGLEWMGGIIPIFGKAHY AQKFQGRVTITADESTSTAYMELSSLRSEDTAVYFCARKFHFVSGSPFGMDVWGQGTTVT VSSAKTTAPSVYPLAPVCGDTTGSSVTLGCLVKGYFPEPVTLTWNSGSLSSGVHTFPAVL QSDLYTLSSSVTVTSSTWPSQSITCNVAHPASSTKVDKKIEPRGPTIKPCPPCKCPAPNA AGGPCVFIFPPKIKDVLMISLSPIVTCVVVDVSEDDPDVQISWFVNNVEVHTAQTQTHRE DYNSTLRVVSALPIQHQDWMSGKEFKCKVNNKDLPAPIERTISKPKGSVRAPQVYVLPPP EEEMTKKQVTLTCMVTDFMPEDIYVEWTNNGKTELNYKNTEPVLDSDGSYFMYSKLRVEK KNWVERNSYSCSVVHEGLHNHHTTKSFSRTPGK |
| 12 | Anti-PD-L1 mIgG2a (engineered cysteines) LALA HC v2 | QVQLVQSGAEVKKPGSSVKVSCKTSGDTFSTAAISWVRQAPGQGLEWMGGIIPIFGKAHY AQKFQGRVTITADESTSTAYMELSSLRSEDTAVYFCARKFHFVSGSPFGMDVWGQGTTVT VSSAKTTAPSVYPLAPVCGDTTGSSVTLGCLVKGYFPEPVTLTWNSGSLSSGVHTFPAVL QSDLYTLSSSVTVTSSTWPSQSITCNVAHPASSTKVDKKIEPRGPTIKPCPPCKCPAPNA AGGPCVFIFPPKIKDVLMISLSPIVTCVVVDVSEDDPDVQISWFVNNVEVHTAQTQTHRE DYNSTLRVVSALPIQHQDWMSGKEFKCKVNNKDLPAPIERTISKPKGSVRAPQVYVLPPP EEEMTKKQVTLTCMVTDFMPEDIYVEWTNNGKTELNYKNTEPVLDSDGSYFMYSKLRVEK KNWVERNSYSCSVVHEGLHNHHTTKSFSRTPG |
| 13 | Anti-PD-L1 CDR-H1 | TAAIS |
| 14 | Anti-PD-L1 CDR-H2 | GIIPIFGKAHYAQKFQG |
| 15 | Anti-PD-L1 CDR-H3 | KFHFVSGSPFGMDV |
| 16 | Anti-PD-L1 CDR-L1 | RASQSVSSYLA |
| 17 | Anti-PD-L1 CDR-L2 | DASNRAT |
| 18 | Anti-PD-L1 CDR-L3 | QQRSNWPT |
| 19 | Anti-PD-L1 VH | QVQLVQSGAEVKKPGSSVKVSCKTSGDTFSTAAISWVRQAPGQGLEWMGGIIPIFGKAHY AQKFQGRVTITADESTSTAYMELSSLRSEDTAVYFCARKFHFVSGSPFGMDVWGQGTTVT VSS |
| 20 | Anti-PD-L1 VL | EIVLTQSPATLSLSPGERATLSCRASQSVSSYLAWYQQKPGQAPRLLIYDASNRATGIPA RFSGSGSGTDFTLTISSLEPEDFAVYYCQQRSNWPTFGQGTKVEIK |
| 21 | Anti-EphA2 CDR-H1 | HYMMA |
| 22 | Anti-EphA2 CDR-H2 | RIGPSGGPTHYADSVKG |
| 23 | Anti-EphA2 CDR-H3 | YDSGYDYVAVAGPAEYFQH |
| 24 | Anti-EphA2 CDR-L1 | RASQSISTWLA |
| 25 | Anti-EphA2 CDR-L2 | KASNLHT |
| 26 | Anti-EphA2 CDR-L3 | QQYNSYSRT |
| 27 | Anti-EphA2 VH | EVQLLESGGGLVQPGGSLRLSCAASGFTFSHYMMAWVRQAPGKGLEWVSRIGPSGGPTHY ADSVKGRFTISRDNSKNTLYLQMNSLRAEDTAVYYCAGYDSGYDYVAVAGPAEYFQHWGQ GTLVTVSS |
| 28 | Anti-EphA2 VL | DIQMTQSPSSLSASVGDRVTITCRASQSISTWLAWYQQKPGKAPKLLIYKASNLHTGVPS RFSGSGSGTEFSLTISGLQPDDFATYYCQQYNSYSRTFGQGTKVEIK |
| 29 | Anti-EphA2 HC | EVQLLESGGGLVQPGGSLRLSCAASGFTFSHYMMAWVRQAPGKGLEWVSRIGPSGGPTHY ADSVKGRFTISRDNSKNTLYLQMNSLRAEDTAVYYCAGYDSGYDYVAVAGPAEYFQHWGQ GTLVTVSSASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPKSCDKTHTCPPC PAPELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSK LTVDKSRWQQGNVFSCSVMHEALHNHYTQKSLSLSPGK |
| 30 | Anti-EphA2 HC v2 | EVQLLESGGGLVQPGGSLRLSCAASGFTFSHYMMAWVRQAPGKGLEWVSRIGPSGGPTHY ADSVKGRFTISRDNSKNTLYLQMNSLRAEDTAVYYCAGYDSGYDYVAVAGPAEYFQHWGQ GTLVTVSSASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPKSCDKTHTCPPC PAPELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSK LTVDKSRWQQGNVFSCSVMHEALHNHYTQKSLSLSPG |
| 31 | Anti-EphA2 LC | DIQMTQSPSSLSASVGDRVTITCRASQSISTWLAWYQQKPGKAPKLLIYKASNLHTGVPS RFSGSGSGTEFSLTISGLQPDDFATYYCQQYNSYSRTFGQGTKVEIKRTVAAPSVFIFPP SDEQLKSGTASVVCLLNNFYPREAKVQWKVDNALQSGNSQESVTEQDS KDSTYSLSSTLTLSKADYEKHKVYACEVTHQGLSSPVTKSFNRGEC |
| 32 | Anti-EphA2 mIgG2a HC | EVQLLESGGGLVQPGGSLRLSCAASGFTFSHYMMAWVRQAPGKGLEWVSRIGPSGGPTHY ADSVKGRFTISRDNSKNTLYLQMNSLRAEDTAVYYCAGYDSGYDYVAVAGPAEYFQHWGQ GTLVTVSSAKTTAPSVYPLAPVCGDTTGSSVTLGCLVKGYFPEPVTLTWNSGSLSSGVHT FPAVLQSDLYTLSSSVTVTSSTWPSQSITCNVAHPASSTKVDKKIEPRGPTIKPCPPCKC PAPNLLGGPSVFIFPPKIKDVLMISLSPIVTCVVVDVSEDDPDVQISWFVNNVEVHTAQT QTHREDYNSTLRVVSALPIQHQDWMSGKEFKCKVNNKDLPAPIERTISKPKGSVRAPQVY VLPPPEEEMTKKQVTLTCMVTDFMPEDIYVEWTNNGKTELNYKNTEPVLDSDGSYFMYSK LRVEKKNWVERNSYSCSVVHEGLHNHHTTKSFSRTPGK |
| 33 | Anti-EphA2 mIgG2a HC v2 | EVQLLESGGGLVQPGGSLRLSCAASGFTFSHYMMAWVRQAPGKGLEWVSRIGPSGGPTHY ADSVKGRFTISRDNSKNTLYLQMNSLRAEDTAVYYCAGYDSGYDYVAVAGPAEYFQHWGQ GTLVTVSSAKTTAPSVYPLAPVCGDTTGSSVTLGCLVKGYFPEPVTLTWNSGSLSSGVHT FPAVLQSDLYTLSSSVTVTSSTWPSQSITCNVAHPASSTKVDKKIEPRGPTIKPCPPCKC PAPNLLGGPSVFIFPPKIKDVLMISLSPIVTCVVVDVSEDDPDVQISWFVNNVEVHTAQT QTHREDYNSTLRVVSALPIQHQDWMSGKEFKCKVNNKDLPAPIERTISKPKGSVRAPQVY VLPPPEEEMTKKQVTLTCMVTDFMPEDIYVEWTNNGKTELNYKNTEPVLDSDGSYFMYSK LRVEKKNWVERNSYSCSVVHEGLHNHHTTKSFSRTPG |
| 34 | Anti-EphA2 mIgG2a LC | DIQMTQSPSSLSASVGDRVTITCRASQSISTWLAWYQQKPGKAPKLLIYKASNLHTGVPS |
| | | RFSGSGSGTEFSLTISGLQPDDFATYYCQQYNSYSRTFGQGTKVEIKRADAAPTVSIFPP SSEQLTSGGASVVCFLNNFYPKDINVKWKIDGSERQNGVLNSWTDQDSKDSTYSMSSTLT LTKDEYERHNSYTCEATHKTSTSPIVKSFNRNEC |
| 35 | Anti-EphA2 mIgG2a LALAPG HC | EVQLLESGGGLVQPGGSLRLSCAASGFTFSHYMMAWVRQAPGKGLEWVSRIGPSGGPTHY ADSVKGRFTISRDNSKNTLYLQMNSLRAEDTAVYYCAGYDSGYDYVAVAGPAEYFQHWGQ GTLVTVSSAKTTAPSVYPLAPVCGDTTGSSVTLGCLVKGYFPEPVTLTWNSGSLSSGVHT FPAVLQSDLYTLSSSVTVTSSTWPSQSITCNVAHPASSTKVDKKIEPRGPTIKPCPPCKC PAPNAAGGPSVFIFPPKIKDVLMISLSPIVTCVVVDVSEDDPDVQISWFVNNVEVHTAQT QTHREDYNSTLRVVSALPIQHQDWMSGKEFKCKVNNKDLGAPIERTISKPKGSVRAPQVY VLPPPEEEMTKKQVTLTCMVTDFMPEDIYVEWTNNGKTELNYKNTEPVLDSDGSYFMYSK LRVEKKNWVERNSYSCSVVHEGLHNHHTTKSFSRTPGK |
| 36 | Anti-EphA2 mIgG2a LALAPG HC v2 | EVQLLESGGGLVQPGGSLRLSCAASGFTFSHYMMAWVRQAPGKGLEWVSRIGPSGGPTHY ADSVKGRFTISRDNSKNTLYLQMNSLRAEDTAVYYCAGYDSGYDYVAVAGPAEYFQHWGQ GTLVTVSSAKTTAPSVYPLAPVCGDTTGSSVTLGCLVKGYFPEPVTLTWNSGSLSSGVHT FPAVLQSDLYTLSSSVTVTSSTWPSQSITCNVAHPASSTKVDKKIEPRGPTIKPCPPCKC PAPNAAGGPSVFIFPPKIKDVLMISLSPIVTCVVVDVSEDDPDVQISWFVNNVEVHTAQT QTHREDYNSTLRVVSALPIQHQDWMSGKEFKCKVNNKDLGAPIERTISKPKGSVRAPQVY VLPPPEEEMTKKQVTLTCMVTDFMPEDIYVEWTNNGKTELNYKNTEPVLDSDGSYFMYSK LRVEKKNWVERNSYSCSVVHEGLHNHHTTKSFSRTPG |
| 37 | Anti-EphA2 mIgG2a LALAPG LC | DIQMTQSPSSLSASVGDRVTITCRASQSISTWLAWYQQKPGKAPKLLIYKASNLHTGVPS RFSGSGSGTEFSLTISGLQPDDFATYYCQQYNSYSRTFGQGTKVEIKRADAAPTVSIFPP SSEQLTSGGASVVCFLNNFYPKDINVKWKIDGSERQNGVLNSWTDQDSKDSTYSMSSTLT LTKDEYERHNSYTCEATHKTSTSPIVKSFNRNEC |
| 38 | Anti-CD30 CDR-H1 | DYYIT |
| 39 | Anti-CD30 CDR-H2 | WIYPGSGNTKYNEKFKG |
| 40 | Anti-CD30 CDR-H3 | YGNYWFAY |
| 41 | Anti-CD30 CDR-L1 | KASQSVDFDGDSYMN |
| 42 | Anti-CD30 CDR-L2 | AASNLES |
| 43 | Anti-CD30 CDR-L3 | QQSNEDPWT |
| 44 | Anti-CD30 VH | QIQLQQSGPEVVKPGASVKISCKASGYTFTDYYITWVKQKPGQGLEWIGWIYPGSGNTKY NEKFKGKATLTVDTSSSTAFMQLSSLTSEDTAVYFCANYGNYWFAYWGQGTQVTVSA |
| 45 | Anti-CD30 VL | DIVLTQSPASLAVSLGQRATISCKASQSVDFDGDSYMNWYQQKPGQPPKVLIYAASNLES GIPARFSGSGSGTDFTLNIHPVEEEDAATYYCQQSNEDPWTFGGGTKLEIK |
| 46 | Anti-CD30 HC | QIQLQQSGPEVVKPGASVKISCKASGYTFTDYYITWVKQKPGQGLEWIGWIYPGSGNTKY NEKFKGKATLTVDTSSSTAFMQLSSLTSEDTAVYFCANYGNYWFAYWGQGTQVTVSAAST KGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPELLGGPSV FLFPPKPKDTLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPPSRDELTK NQVSLTCLVKGFYPSDIAVEWESNGQPENNYKTTPPVLDSDGSFFLYSKLTVDKSRWQQG NVFSCSVMHEALHNHYTQKSLSLSPGK |
| 47 | Anti-CD30 HC v2 | QIQLQQSGPEVVKPGASVKISCKASGYTFTDYYITWVKQKPGQGLEWIGWIYPGSGNTKY NEKFKGKATLTVDTSSSTAFMQLSSLTSEDTAVYFCANYGNYWFAYWGQGTQVTVSAAST KGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPELLGGPSV FLFPPKPKDTLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPPSRDELTK NQVSLTCLVKGFYPSDIAVEWESNGQPENNYKTTPPVLDSDGSFFLYSKLTVDKSRWQQG NVFSCSVMHEALHNHYTQKSLSLSPG |
| 48 | Anti-CD30 LC | DIVLTQSPASLAVSLGQRATISCKASQSVDFDGDSYMNWYQQKPGQPPKVLIYAASNLES GIPARFSGSGSGTDFTLNIHPVEEEDAATYYCQQSNEDPWTFGGGTKLEIKR TVAAPSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC |
| 49 | Anti-CD228 CDR-H1 | SGYWN |
| 50 | Anti-CD228 CDR-H2 | YISDSGITYYNPSLKS |
| 51 | Anti-CD228 CDR-H3 | RTLATYYAMDY |
| 52 | Anti-CD228 CDR-L1 | RASQSLVHSDGNTYLH |
| 53 | Anti-CD228 CDR-L2 | RVSNRFS |
| 54 | Anti-CD228 CDR-L3 | SQSTHVPPT |
| 55 | Anti-CD228 VH | QVQLQESGPGLVKPSETLSLTCTVSGDSITSGYWNWIRQPPGKGLEYIGYISDSGITYYN PSLKSRVTISRDTSKNQYSLKLSSVTAADTAVYYCARRTLATYYAMDYWGQGTLVTVSS |
| 56 | Anti-CD228 VL | DFVMTQSPLSLPVTLGQPASISCRASQSLVHSDGNTYLHWYQQRPGQSPRLLIYRVSNRF SGVPDRFSGSGSGTDFTLKISRVEAEDVGVYYCSQSTHVPPTFGQGTKLEIK |
| 57 | Anti-CD228 HC | QVQLQESGPGLVKPSETLSLTCTVSGDSITSGYWNWIRQPPGKGLEYIGYISDSGITYYN PSLKSRVTISRDTSKNQYSLKLSSVTAADTAVYYCARRTLATYYAMDYWGQGTLVTVSSA STKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSRDEL TKNQVSLTCLVKGFYPSDIAVEWESNGQPENNYKTTPPVLDSDGSFFLYSKLTVDKSRWQ QGNVFSCSVMHEALHNHYTQKSLSLSPGK |
| 518 | Anti-CD228 HC v2 | QVQLQESGPGLVKPSETLSLTCTVSGDSITSGYWNWIRQPPGKGLEYIGYISDSGITYYN PSLKSRVTISRDTSKNQYSLKLSSVTAADTAVYYCARRTLATYYAMDYWGQGTLVTVSSA STKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSRDEL TKNQVSLTCLVKGFYPSDIAVEWESNGQPENNYKTTPPVLDSDGSFFLYSKLTVDKSRWQ QGNVFSCSVMHEALHNHYTQKSLSLSPG |
| 59 | Anti-CD228 LC | DFVMTQSPLSLPVTLGQPASISCRASQSLVHSDGNTYLHWYQQRPGQSPRLLIYRVSNRF SGVPDRFSGSGSGTDFTLKISRVEAEDVGVYYCSQSTHVPPTFGQGTKLEIKRTVAAPSV FIFPPSDEQLKSGTASVVCLLNNFYPREAKVQWKVDNALQSGNSQESVTEQDSKDSTYSL SSTLTLSKADYEKHKVYACEVTHQGLSSPVTKSFNRGEC |
| 60 | Anti-CD228 LALAKA HC | QVQLQESGPGLVKPSETLSLTCTVSGDSITSGYWNWIRQPPGKGLEYIGYISDSGITYYN PSLKSRVTISRDTSKNQYSLKLSSVTAADTAVYYCARRTLATYYAMDYWGQGTLVTVSSA STKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPEAAGGP SVFLFPPKPKDTLMISRTPEVTCVVVDVSHEDPEVKFNWYVDGVEVHNAKTKPREEQYNS TYRVVSVLTVLHQDWLNGKEYKCAVSNKALPAPIEKTISKAKGQPREPQVYTLPPSRDEL TKNQVSLTCLVKGFYPSDIAVEWESNGQPENNYKTTPPVLDSDGSFFLYSKLTVDKSRWQ QGNVFSCSVMHEALHNHYTQKSLSLSPGK |
| 61 | Anti-CD228 LALAKA HC v2 | QVQLQESGPGLVKPSETLSLTCTVSGDSITSGYWNWIRQPPGKGLEYIGYISDSGITYYN PSLKSRVTISRDTSKNQYSLKLSSVTAADTAVYYCARRTLATYYAMDYWGQGTLVTVSSA STKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPEAAGGP SVFLFPPKPKDTLMISRTPEVTCVVVDVSHEDPEVKFNWYVDGVEVHNAKTKPREEQYNS TYRVVSVLTVLHQDWLNGKEYKCAVSNKALPAPIEKTISKAKGQPREPQVYTLPPSRDEL TKNQVSLTCLVKGFYPSDIAVEWESNGQPENNYKTTPPVLDSDGSFFLYSKLTVDKSRWQ QGNVFSCSVMHEALHNHYTQKSLSLSPG |
| 62 | Anti-CD228 LALAKA LC | DFVMTQSPLSLPVTLGQPASISCRASQSLVHSDGNTYLHWYQQRPGQSPRLLIYRVSNRF SGVPDRFSGSGSGTDFTLKISRVEAEDVGVYYCSQSTHVPPTFGQGTKLEIKRTVAAPSV FIFPPSDEQLKSGTASVVCLLNNFYPREAKVQWKVDNALQSGNSQESVTEQDSKDSTYSL SSTLTLSKADYEKHKVYACEVTHQGLSSPVTKSFNRGEC |
| 63 | Anti-avB6 HC CDR-H1 | DYNVN |
| 64 | Anti-AvB6 HC CDR-H2 | VINPKYGTTRYNQKFKG |
| 65 | Anti-AvB6 HC CDR-H3 | GLNAWDY |
| 66 | Anti-AvB6 LG CDR-L1 | GASENIYGALN |
| 67 | Anti-AvB6 LG CDR-L2 | GATNLED |
| 68 | Anti-AvB6 LG CDR-L3 | QNVLTTPYT |
| 69 | Anti-AvB6 HC VH | QFQLVQSGAEVKKPGASVKVSCKASGYSFTDYNVNWVRQAPGQGLEWIGVINPKYGTTRY NQKFKGRATLTVDKSTSTAYMELSSLRSEDTAVYYCTRGLNAWDYWGQGTLVTVSS |
| 70 | Anti-AvB6 LG VL | DIQMTQSPSSLSASVGDRVTITCGASENIYGALNWYQQKPGKAPKLLIYGATNLEDGVPS RFSGSGSGRDYTFTISSLQPEDIATYYCQNVLTTPYTFGQGTKLEIK |
| 71 | Anti-AvB6 HC | QFQLVQSGAEVKKPGASVKVSCKASGYSFTDYNVNWVRQAPGQGLEWIGVINPKYGTTRY NQKFKGRATLTVDKSTSTAYMELSSLRSEDTAVYYCTRGLNAWDYWGQGTLVTVSSASTK GPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPSRDELTKN QVSLTCLVKGFYPSDIAVEWESNGQPENNYKTTPPVLDSDGSFFLYSKLTVDKSRWQQGN VFSCSVMHEALHNHYTQKSLSLSPGK |
| 72 | Anti-AvB6 HC v2 | QFQLVQSGAEVKKPGASVKVSCKASGYSFTDYNVNWVRQAPGQGLEWIGVINPKYGTTRY NQKFKGRATLTVDKSTSTAYMELSSLRSEDTAVYYCTRGLNAWDYWGQGTLVTVSSASTK GPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPSRDELTKN QVSLTCLVKGFYPSDIAVEWESNGQPENNYKTTPPVLDSDGSFFLYSKLTVDKSRWQQGN VFSCSVMHEALHNHYTQKSLSLSPG |
| 73 | Anti-AvB6 LG LC | DIQMTQSPSSLSASVGDRVTITCGASENIYGALNWYQQKPGKAPKLLIYGATNLEDGVPS RFSGSGSGRDYTFTISSLQPEDIATYYCQNVLTTPYTFGQGTKLEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 74 | Anti-AvB6 LALAKA HC | QFQLVQSGAEVKKPGASVKVSCKASGYSFTDYNVNWVRQAPGQGLEWIGVINPKYGTTRY NQKFKGRATLTVDKSTSTAYMELSSLRSEDTAVYYCTRGLNAWDYWGQGTLVTVSSASTK GPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGGPSVF LFPPKPKDTLMISRTPEVTCVVVDVSHEDPEVKFNWYVDGVEVHNAKTKPREEQYNSTYR VVSVLTVLHQDWLNGKEYKCAVSNKALPAPIEKTISKAKGQPREPQVYTLPPSRDELTKN QVSLTCLVKGFYPSDIAVEWESNGQPENNYKTTPPVLDSDGSFFLYSKLTVDKSRWQQGN VFSCSVMHEALHNHYTQKSLSLSPGK |
| 75 | Anti-AvB6 LALAKA HC v2 | QFQLVQSGAEVKKPGASVKVSCKASGYSFTDYNVNWVRQAPGQGLEWIGVINPKYGTTRY NQKFKGRATLTVDKSTSTAYMELSSLRSEDTAVYYCTRGLNAWDYWGQGTLVTVSSASTK GPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPEAAGGPSVF LFPPKPKDTLMISRTPEVTCVVVDVSHEDPEVKFNWYVDGVEVHNAKTKPREEQYNSTYR VVSVLTVLHQDWLNGKEYKCAVSNKALPAPIEKTISKAKGQPREPQVYTLPPSRDELTKN QVSLTCLVKGFYPSDIAVEWESNGQPENNYKTTPPVLDSDGSFFLYSKLTVDKSRWQQGN VFSCSVMHEALHNHYTQKSLSLSPG |
| 76 | Anti-AvB6 LG LALAKA LC | DIQMTQSPSSLSASVGDRVTITCGASENIYGALNWYQQKPGKAPKLLIYGATNLEDGVPS RFSGSGSGRDYTFTISSLQPEDIATYYCQNVLTTPYTFGQGTKLEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 77 | Anti-B7H4 CDR-H1 | SGSYYWG |
| 78 | Anti-B7H4 CDR-H2 | NIYYSGSTYYNPSLRS |
| 79 | Anti-B7H4 CDR-H3 | EGSYPNQFDP |
| 80 | Anti-B7H4 CDR-L1 | RASQSVSSNLA |
| 81 | Anti-B7H4 CDR-L2 | GASTRAT |
| 82 | Anti-B7H4 CDR-L3 | QQYHSFPFT |
| 83 | Anti-B7H4 VH | QLQLQESGPGLVKPSETLSLTCTVSGGSIKSGSYYWGWIRQPPGKGLEWIGNIYYSGSTY YNPSLRSRVTISVDTSKNQFSLKLSSVTAADTAVYYCAREGSYPNQFDPWGQGTLVTVSS |
| 84 | Anti-B7H4 VL | EIVMTQSPATLSVSPGERATLSCRASQSVSSNLAWYQQKPGQAPRLLIYGASTRATGIPA RFSGSGSGTEFTLTISSLQSEDFAVYYCQQYHSFPFTFGGGTKVEIK |
| 85 | Anti-B7H4 HC | QLQLQESGPGLVKPSETLSLTCTVSGGSIKSGSYYWGWIRQPPGKGLEWIGNIYYSGSTY YNPSLRSRVTISVDTSKNQFSLKLSSVTAADTAVYYCAREGSYPNQFDPWGQGTLVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 86 | Anti-B7H4 HC v2 | QLQLQESGPGLVKPSETLSLTCTVSGGSIKSGSYYWGWIRQPPGKGLEWIGNIYYSGSTY YNPSLRSRVTISVDTSKNQFSLKLSSVTAADTAVYYCAREGSYPNQFDPWGQGTLVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPG |
| 87 | Anti-B7H4 LC | EIVMTQSPATLSVSPGERATLSCRASQSVSSNLAWYQQKPGQAPRLLIYGASTRATGIPA RFSGSGSGTEFTLTISSLQSEDFAVYYCQQYHSFPFTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 88 | Anti-B7H4 LALAKA HC | QLQLQESGPGLVKPSETLSLTCTVSGGSIKSGSYYWGWIRQPPGKGLEWIGNIYYSGSTY YNPSLRSRVTISVDTSKNQFSLKLSSVTAADTAVYYCAREGSYPNQFDPWGQGTLVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPEAAGG PSVFLFPPKPKDTLMISRTPEVTCVVVDVSHEDPEVKFNWYVDGVEVHNAKTKPREEQYN STYRVVSVLTVLHQDWLNGKEYKCAVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 89 | Anti-B7H4 LALAKA HC v2 | QLQLQESGPGLVKPSETLSLTCTVSGGSIKSGSYYWGWIRQPPGKGLEWIGNIYYSGSTY YNPSLRSRVTISVDTSKNQFSLKLSSVTAADTAVYYCAREGSYPNQFDPWGQGTLVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPEAAGG PSVFLFPPKPKDTLMISRTPEVTCVVVDVSHEDPEVKFNWYVDGVEVHNAKTKPREEQYN STYRVVSVLTVLHQDWLNGKEYKCAVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPG |
| 90 | Anti-B7H4 LALAKA LC | EIVMTQSPATLSVSPGERATLSCRASQSVSSNLAWYQQKPGQAPRLLIYGASTRATGIPA RFSGSGSGTEFTLTISSLQSEDFAVYYCQQYHSFPFTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 91 | Anti-B7H4 CDR-H1 | GSISSSSYYWG |
| 92 | Anti-B7H4 CDR-H2 | NIYYSGSTYYNPSLKS |
| 93 | Anti-B7H4 CDR-H3 | AREGSYPNWFDP |
| 94 | Anti-B7H4 CDR-L1 | RASQSVSSNLA |
| 95 | Anti-B7H4 CDR-L2 | GASTRAT |
| 96 | Anti-B7H4 CDR-L3 | QQYHSFPFT |
| 97 | Anti-B7H4 VH | QLQLQESGPGLVKPSETLSLTCTVSGGSISSSSYYWGWIRQPPGKGLEWIGNIYYSGSTY |
| | | YNPSLKSRVTISVDTSKNQFSLKLSSVTAADTAVYYCAREGSYPNWFDPWGQGTLVTVSS |
| 98 | Anti-B7H4 VL | EIVMTQSPATLSVSPGERATLSCRASQSVSSNLAWYQQKPGQAPRLLIYGASTRATGIPA RFSGSGSGTEFTLTISSLQSEDFAVYYCQQYHSFPFTFGGGTKVEIK |
| 99 | Anti-B7H4 CDR-H1 | GSIKSGSHYWG |
| 100 | Anti-B7H4 CDR-H2 | NIYYSGSTYYNPSLRS |
| 101 | Anti-B7H4 CDR-H3 | AREGSYPNWFDP |
| 102 | Anti-B7H4 CDR-L1 | RASQSVSSNLA |
| 103 | Anti-B7H4 CDR-L2 | GASTRAT |
| 104 | Anti-B7H4 CDR-L3 | QQYHSFPF7- |
| 105 | Anti-B7H4 VH | QLQLQESGPGLVKPSETLSLTCTVSGGSIKSGSHYWGWIRQPPGKGLEWIGNIYYSGSTY YNPSLRSRVTISVDTSKNQFSLKLSSVTAADTAVYYCAREGSYPNWFDPWGQGTLVTVSS |
| 106 | Anti-B7H4 VL | EIVMTQSPATLSVSPGERATLSCRASQSVSSNLAWYQQKPGQAPRLLIYGASTRATGIPA RFSGSGSGTEFTLTISSLQSEDFAVYYCQQYHSFPFTFGGGTKVEIK |
| 107 | Anti-B7H4 CDR-H1 | GSIKSGSHYWG |
| 108 | Anti-B7H4 CDR-H2 | NIYYSGSTYYNPSLKS |
| 109 | Anti-B7H4 CDR-H3 | AREGSYPNWLDP |
| 110 | Anti-B7H4 CDR-L1 | RASQSVSSNLA |
| 111 | Anti-B7H4 CDR-L2 | GASTRAT |
| 112 | Anti-B7H4 CDR-L3 | QQYHSFPFT |
| 113 | Anti-B7H4 VH | QLQLQESGPGLVKPSETLSLTCTVSGGSIKSGSHYWGWIRQPPGKGLEWIGNIYYSGSTY YNPSLKSRVTISVDTSKNQFSLKLSSVTAADTAVYYCAREGSYPNWLDPWGQGTLVTVSS |
| 114 | Anti-B7H4 VL | EIVMTQSPATLSVSPGERATLSCRASQSVSSNLAWYQQKPGQAPRLLIYGASTRATGIPA RFSGSGSGTEFTLTISSLQSEDFAVYYCQQYHSFPFTFGGGTKVEIK |
| 115 | Anti-B7H4 CDR-H1 | GSIKSGSYYWG |
| 116 | Anti-B7H4 CDR-H2 | NIYYSGSTYYNPSLKS |
| 117 | Anti-B7H4 CDR-H3 | AREGSYPNQFDP |
| 118 | Anti-B7H4 CDR-L1 | RASQSVSSNLA |
| 119 | Anti-B7H4 CDR-L2 | GASTRAT |
| 120 | Anti-B7H4 CDR-L3 | QQYHSFPFT |
| 121 | Anti-B7H4 VH | QLQLQESGPGLVKPSETLSLTCTVSGGSIKSGSYYWGWIRQPPGKGLEWIGNIYYSGSTY YNPSLKSRVTISVDTSKNQFSLKLSSVTAADTAVYYCAREGSYPNQFDPWGQGILVTVSS |
| 122 | Anti-B7H4 VL | EIVMTQSPATLSVSPGERATLSCRASQSVSSNLAWYQQKPGQAPRLLIYGASTRATGIPA RFSGSGSGTEFTLTISSLQSEDFAVYYCQQYHSFPFTFGGGTKVEIK |
| 123 | Anti-B7H4 CDR-H1 | GSIKSGSHYWG |
| 124 | Anti-B7H4 CDR-H2 | NIYYSGSTYYNPSLKS |
| 125 | Anti-B7H4 CDR-H3 | AREGSYPNWFDP |
| 126 | Anti-B7H4 CDR-L1 | RASQSVSTNLA |
| 127 | Anti-B7H4 CDR-L2 | DASARVT |
| 128 | Anti-B7H4 CDR-L3 | QQYHSFPFT |
| 129 | Anti-B7H4 VH | QLQLQESGPGLVKPSETLSLTCTVSGGSIKSGSHYWGWIRQPPGKGLEWIGNIYYSGSTY YNPSLKSRVTMSVDTSKNQFSLKLSSVTAADTAVYYCAREGSYPNWFDPWGQGTLVTVSS |
| 130 | Anti-B7H4 VL | EIVMTQSPATLSVSPGERATLSCRASQSVSTNLAWYQQKPGQAPRLLIYDASARVTGIPA RFSGSGSGTEFTLTISSLQSEDFAVYYCQQYHSFPFTFGGGTKVEIK |
| 131 | Anti-B7H4 CDR-H1 | GSISSSSYYWG |
| 132 | Anti-B7H4 CDR-H2 | NIYYSGSTYYNPSLKS |
| 133 | Anti-B7H4 CDR-H3 | AREGSYTTVLNV |
| 134 | Anti-B7H4 CDR-L1 | RASQSVSSSYLA |
| 135 | Anti-B7H4 CDR-L2 | GASSRAT |
| 136 | Anti-B7H4 CDR-L3 | QQAASYPLT |
| 137 | Anti-B7H4 VH | QLQLQESGPGLVKPSETLSLTCTVSGGSISSSSYYWGWIRQPPGKGLEWIGNIYYSGSTY YNPSLKSRVTISVDTSKNQFSLKLSSVTAADTAVYYCAREGSYTTVLNVWGQGTMVTVSS |
| 138 | Anti-B7H4 VL | EIVLTQSPGTLSLSPGERATLSCRASQSVSSSYLAWYQQKPGQAPRLLIYGASSRATGIP DRFSGSGSGTDFTLTISRLEPEDFAVYYCQQAASYPLTFGGGTKVEIK |
| 139 | Anti-B7H4 CDR-H1 | GSIGRGSYYWG |
| 140 | Anti-B7H4 CDR-H2 | NIYYSGSTYYNPSLKS |
| 141 | Anti-B7H4 CDR-H3 | AREGSYTTVLNV |
| 142 | Anti-B7H4 CDR-L1 | RASQSVASSHLA |
| 143 | Anti-B7H4 CDR-L2 | DAVSRAT |
| 144 | Anti-B7H4 CDR-L3 | QQAASYPLT |
| 145 | Anti-B7H4 VH | QLQLQESGPGLVKPSETLSLTCTVSGGSIGRGSYYWGWIRQPPGKGLEWIGNIYYSGSTY YNPSLKSRVTISVDTSKNQFSLKLSSVTAADTAVYYCAREGSYTTVLNVWGQGTMVTVSS |
| 146 | Anti-B7H4 VL | EIVLTQSPGTLSLSPGERATLSCRASQSVASSHLAWYQQKPGQAPRLLIYDAVSRATGIP DRFSGSGSGTDFTLTISRLEPEDFAVYYCQQAASYPLTFGGGTKVEIK |
| 147 | Anti-B7H4 CDR-H1 | GSISSGGYYWS |
| 148 | Anti-B7H4 CDR-H2 | NIYYSGSTYYNPSLKS |
| 149 | Anti-B7H4 CDR-H3 | ARESSTISADFDL |
| 150 | Anti-B7H4 CDR-L1 | RASQGISRWLA |
| 151 | Anti-B7H4 CDR-L2 | AASSLQS |
| 152 | Anti-B7H4 CDR-L3 | QQAHTFPYT |
| 153 | Anti-B7H4 VH | QVQLQESGPGLVKPSQTLSLTCTVSGGSISSGGYYWSWIRQHPGKGLEWIGNIYYSGSTY YNPSLKSRVTISVDTSKNQFSLKLSSVTAADTAVYYCARESSTISADFDLWGRGTLVTVS S |
| 154 | Anti-B7H4 VL | DIQMTQSPSSVSASVGDRVTITCRASQGISRWLAWYQQKPGKAPKLLIYAASSLQSGVPS RFSGSGSGTDFTLTISSLQPEDFATYYCQQAHTFPYTFGGGTKVEIK |
| 155 | Anti-B7H4 CDR-H1 | GSISHGGYYWS |
| 156 | Anti-B7H4 CDR-H2 | NIYYSGSTYYNPSLKS |
| 157 | Anti-B7H4 CDR-H3 | ARESSTISADFDL |
| 158 | Anti-B7H4 CDR-L1 | RASQGISRWLA |
| 159 | Anti-B7H4 CDR-L2 | AASSLQS |
| 160 | Anti-B7H4 CDR-L3 | QQAHTFPYT |
| 161 | Anti-B7H4 VH | QLQLQESGPGLVKPSETLSLTCTASGGSISHGGYYWSWIRQHPGKGLEWIGNIYYSGSTY YNPSLKSRVTMSVDTSKNQFSLKLSSVTAADTAVYYCARESSTISADFDLWGRGTLVTVS S |
| 162 | Anti-B7H4 VL | DIQMTQSPSSVSASVGDRVTITCRASQGISRWLAWYQQKPGKAPKLLIYAASSLQSGVPS RFSGSGSGTDFTLTISSLQPEDFATYYCQQAHTFPYTFGGGTKVEIK |
| 163 | Anti-B7H4 CDR-H1 | GSISSGGYYWS |
| 164 | Anti-B7H4 CDR-H2 | NIYYSGSTYYNPSLKS |
| 165 | Anti-B7H4 CDR-H3 | ARGLSTIDEAFDP |
| 166 | Anti-B7H4 CDR-L1 | RASQSISSWLA |
| 167 | Anti-B7H4 CDR-L2 | KASSLES |
| 168 | Anti-B7H4 CDR-L3 | QQDNSYPYT |
| 169 | Anti-B7H4 VH | QVQLQESGPGLVKPSQTLSLTCTVSGGSISSGGYYWSWIRQHPGKGLEWIGNIYYSGSTY YNPSLKSRVTISVDTSKNQFSLKLSSVTAADTAVYYCARGLSTIDEAFDPWGQGTLVTVS S |
| 170 | Anti-B7H4 VL | DIQMTQSPSTLSASVGDRVTITCRASQSISSWLAWYQQKPGKAPKLLIYKASSLESGVPS RFSGSGSGTEFTLTISSLQPDDFATYYCQQDNSYPYTFGGGTKVEIK |
| 171 | Anti-B7H4 CDR-H1 | GSISDGSYYWS |
| 172 | Anti-B7H4 CDR-H2 | NIYYSGSTYYNPSLRS |
| 173 | Anti-B7H4 CDR-H3 | ARGLSTIDEAFDP |
| 174 | Anti-B7H4 CDR-L1 | RASQSISSWLA |
| 175 | Anti-B7H4 CDR-L2 | KASSLES |
| 176 | Anti-B7H4 CDR-L3 | QQDNSYPYT |
| 177 | Anti-B7H4 VH | QLQLQESGPGLVKPSETLSLTCTVSGGSISDGSYYWSWIRQHPGKGLEWIGNIYYSGSTY YNPSLRSRVTMSVDTSKNQFSLKLSSVTAADTAVYYCARGLSTIDEAFDPWGQGTLVTVS S |
| 178 | Anti-B7H4 VL | DIQMTQSPSTLSASVGDRVTITCRASQSISSWLAWYQQKPGKAPKLLIYKASSLESGVPS RFSGSGSGTEFTLTISSLQPDDFATYYCQQDNSYPYTFGGGTKVEIK |
| 179 | Anti-B7H4 CLR-H1 | GSISDGSYYWS |
| 180 | Anti-B7H4 CDR-H2 | NIYYSGSTYYNPSLRS |
| 181 | Anti-B7H4 CDR-H3 | ARGLSTIDEAFDP |
| 182 | Anti-B7H4 CDR-L1 | RASKSISSWLA |
| 183 | Anti-B7H4 CDR-L2 | EASSLHS |
| 184 | Anti-B7H4 CDR-L3 | QQDNSYPYT |
| 185 | Anti-B7H4 VH | QVQLQESGPGLVKPSQTLSLTCTVSGGSISDGSYYWSWIRQHPGKGLEWIGNIYYSGSTY |
| | | YNPSLRSRVTMSVDTSKNQFSLKLSSVTAADTAVYYCARGLSTIDEAFDPWGQGTLVTVS S |
| 186 | Anti-B7H4 VL | DIQMTQSPSTLSASVGDRVTITCRASKSISSWLAWYQQKPGKAPKLLIYEASSLHSGVPS RFSGSGSGTEFTLTISSLQPDDFATYYCQQDNSYPYTFGGGTKVEIK |
| 187 | Anti-B7H4 CDR-H1 | GSISSYYWS |
| 188 | Anti-B7H4 CDR-H2 | YIYSSGSTNYNPSLKS |
| 189 | Anti-B7H4 CDR-H3 | ARGSGQYAAPDYGMD |
| 190 | Anti-B7H4 CDR-L1 | RASQSISSWLA |
| 191 | Anti-B7H4 CDR-L2 | KASSLES |
| 192 | Anti-B7H4 CDR-L3 | QQDNSFPFT |
| 193 | Anti-B7H4 VH | QVQLQESGPGLVKPSETLSLTCTVSGGSISSYYWSWIRQPPGKGLEWIGYIYSSGSTNYN PSLKSRVTISVDTSKNQFSLKLSSVTAADTAVYYCARGSGQYAAPDYGMDVWGQGTTVTV SS |
| 194 | Anti-B7H4 VL | DIQMTQSPSTLSASVGDRVTITCRASQSISSWLAWYQQKPGKAPKLLIYKASSLESGVPS RFSGSGSGTEFTLTISSLQPDDFATYYCQQDNSFPFTFGGGTKVEIK |
| 195 | Anti-B7H4 CDR-H1 | GSIISYYWG |
| 196 | Anti-B7H4 CDR-H2 | YIYSSGSTSYNPSLKS |
| 197 | Anti-B7H4 CDR-H3 | ARGSGLYAAPDYGLDV |
| 198 | Anti-B7H4 CDR-L1 | RASQSISSWLA |
| 199 | Anti-B7H4 CDR-L2 | KASSLES |
| 200 | Anti-B7H4 CDR-L3 | QQDNSFPFT |
| 201 | Anti-B7H4 VH | QVQLQESGPGLVKPSETLSLTCTVSGGSIISYYWGWIRQPPGKGLEWIGYIYSSGSTSYN PSLKSRVTISVDTSKNQFSLKLSSVTAADTAVYYCARGSGLYAAPDYGLDVWGQGTTVTV SS |
| 202 | Anti-B7H4 VL | DIQMTQSPSTLSASVGDRVTITCRASQSISSWLAWYQQKPGKAPKLLIYKASSLESGVPS RFSGSGSGTEFTLTISSLQPDDFATYYCQQDNSFPFTFGGGTKVEIK |
| 203 | Anti-B7H4 CDR-H1 | FTFSSYAMS |
| 204 | Anti-B7H4 CDR-H2 | TISGSGGSTYYADSVKG |
| 205 | Anti-B7H4 CDR-H3 | ARGAGHYDLVGRY |
| 206 | Anti-B7H4 CDR-L1 | RASQSISSYLN |
| 207 | Anti-B7H4 CDR-L2 | AASSLQS |
| 208 | Anti-B7H4 CDR-L3 | QQLYSLPPT |
| 209 | Anti-B7H4 VH | EVQLLESGGGLVQPGGSLRLSCAASGFTFSSYAMSWVRQAPGKGLEWVSTISGSGGSTYY ADSVKGRFTISRDNSKNTLYLQMNSLRAEDTAVYYCARGAGHYDLVGRYWGQGTLVTVSS |
| 210 | Anti-B7H4 VL | DIQMTQSPSSLSASVGDRVTITCRASQSISSYLNWYQQKPGKAPKLLIYAASSLQSGVPS RFSGSGSGTDFTLTISSLQPEDFATYYCQQLYSLPPTFGGGTKVEIK |
| 211 | Anti-B7H4 CDR-H1 | FTFSSYAMS |
| 212 | Anti-B7H4 CDR-H2 | AISGSGGSTYYADSVKG |
| 213 | Anti-B7H4 CDR-H3 | ARVGFRALNY |
| 214 | Anti-B7H4 CDR-L1 | RASQDISSWLA |
| 215 | Anti-B7H4 CDR-L2 | AASSLQS |
| 216 | Anti-B7H4 CDR-L3 | QQATSYPPWT |
| 217 | Anti-B7H4 VH | EVQLLESGGGLVQPGGSLRLSCAASGFTFSSYAMSWVRQAPGKGLEWVSAISGSGGSTYY ADSVKGRFTISRDNSKNTLYLQMNSLRAEDTAVYYCARVGFRALNYWGQGTTVTVSS |
| 218 | Anti-B7H4 VL | DIQLTQSPSSVSASVGDRVTITCRASQDISSWLAWYQQKPGKAPKLLIYAASSLQSGVPS RFSGSGSGTDFTLTISSLQPEDFATYYCQQATSYPPWTFGGGTKVEIK |
| 219 | Anti-B7H4 CDR-H1 | GTFSSYAIS |
| 220 | Anti-B7H4 CDR-H2 | GIIPIFGTASYAQKFQG |
| 221 | Anti-B7H4 CDR-H3 | ARQQYDGRRYFGL |
| 222 | Anti-B7H4 CDR-L1 | RASQSVSSNLA |
| 223 | Anti-B7H4 CDR-L2 | SASTRAT |
| 224 | Anti-B7H4 CDR-L3 | QQVNVWPPT |
| 225 | Anti-B7H4 VH | QVQLVQSGAEVKKPGSSVKVSCKASGGTFSSYAISWVRQAPGQGLEWMGGIIPIFGTASY AQKFQGRVTITADESTSTAYMELSSLRSEDTAVYYCARQQYDGRRYFGLWGRGTLVTVSS |
| 226 | Anti-B7H4 VL | EIVMTQSPATLSVSPGERATLSCRASQSVSSNLAWYQQKPGQAPRLLIYSASTRATGIPA RFSGSGSGTEFTLTISSLQSEDFAVYYCQQVNVWPPTFGGGTKVEIK |
| 227 | Anti-B7H4 CDR-H1 | GTFSSYAIS |
| 228 | Anti-B7H4 CDR-H2 | GIIPIFGTANYAQKFQG |
| 229 | Anti-B7H4 CDR-H3 | ARGGPWFDP |
| 230 | Anti-B7H4 CDR-L1 | RASQSISSWLA |
| 231 | Anti-B7H4 CDR-L2 | KASSLES |
| 232 | Anti-B7H4 CDR-L3 | QQYNSYPPFT |
| 233 | Anti-B7H4 VH | QVQLVQSGAEVKKPGSSVKVSCKASGGTFSSYAISWVRQAPGQGLEWMGGIIPIFGTANY AQKFQGRVTITADESTSTAYMELSSLRSEDTAVYYCARGGPWFDPWGQGTLVTVSS |
| 234 | Anti-B7H4 VL | DIQMTQSPSTLSASVGDRVTITCRASQSISSWLAWYQQKPGKAPKLLIYKASSLESGVPS RFSGSGSGTEFTLTISSLQPDDFATYYCQQYNSYPPFTFGGGTKVEIK |
| 235 | Anti-B7H4 CDR-H1 | FTFSSYAMS |
| 236 | Anti-B7H4 CDR-H2 | AISGSGGSTSYADSVKG |
| 237 | Anti-B7H4 CDR-H3 | AKPSLATMLAFDI |
| 238 | Anti-B7H4 CDR-L1 | RASQSISSWLA |
| 239 | Anti-B7H4 CDR-L2 | DASSLES |
| 240 | Anti-B7H4 CDR-L3 | QQSKSYPRT |
| 241 | Anti-B7H4 VH | EVQLLESGGGLVQPGGSLRLSCAASGFTFSSYAMSWVRQAPGKGLEWVSAISGSGGSTSY ADSVKGRFTISRDNSKNTLYLQMNSLRAEDTAVYYCAKPSLATMLAFDIWGQGTMVTVSS |
| 242 | Anti-B7H4 VL | DIQMTQSPSTLSASVGDRVTITCRASQSISSWLAWYQQKPGKAPKLLIYDASSLESGVPS RFSGSGSGTEFTLTISSLQPDDFATYYCQQSKSYPRTFGGGTKVEIK |
| 243 | Anti-B7H4 CDR-H1 | GSISSSVYYWS |
| 244 | Anti-B7H4 CDR-H2 | SILVSGSTYYNPSLKS |
| 245 | Anti-B7H4 CDR-H3 | ARAVSFLDV |
| 246 | Anti-B7H4 CDR-L1 | RASQSISSYLN |
| 247 | Anti-B7H4 CDR-L2 | GASSLQS |
| 248 | Anti-B7H4 CDR-L3 | QQSYDPPWT |
| 249 | Anti-B7H4 VH | QLQLQESGPGLVKPSETLSLTCTVSGGSISSSVYYWSWIRQPPGKGLEWIGSILVSGSTY YNPSLKSRVTISVDTSKNQFSLKLSSVTAADTAVYYCARAVSFLDVWGQGTMVIVSS |
| 250 | Anti-B7H4 VL | DIQMTQSPSSLSASVGDRVTITCRASQSISSYLNWYQQKPGKAPKLLIYGASSLQSGVPS RFSGSGSGTDFTLTISSLQPEDFATYYCQQSYDPPWTFGGGTKVEIK |
| 251 | Anti-B7H4 HC | QLQLQESGPGLVKPSETLSLTCTVSGGSISSSSYYWGWIRQPPGKGLEWIGNIYYSGSTY YNPSLKSRVTISVDTSKNQFSLKLSSVTAADTAVYYCAREGSYPNWFDPWGQGTLVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 252 | Anti-B7H4 LC | EIVMTQSPATLSVSPGERATLSCRASQSVSSNLAWYQQKPGQAPRLLIYGASTRATGIPA RFSGSGSGTEFTLTISSLQSEDFAVYYCQQYHSFPFTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 253 | Anti-B7H4 HC | QLQLQESGPGLVKPSETLSLTCTVSGGSIKSGSHYWGWIRQPPGKGLEWIGNIYYSGSTY YNPSLRSRVTISVDTSKNQFSLKLSSVTAADTAVYYCAREGSYPNWFDPWGQGTLVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 254 | Anti-B7H4 LC | QLQLQESGPGLVKPSETLSLTCTVSGGSIKSGSHYWGWIRQPPGKGLEWIGNIYYSGSTY YNPSLKSRVTISVDTSKNQFSLKLSSVTAADTAVYYCAREGSYPNWLDPWGQGTLVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 255 | Anti-B7H4 HC | EIVMTQSPATLSVSPGERATLSCRASQSVSSNLAWYQQKPGQAPRLLIYGASTRATGIPA RFSGSGSGTEFTLTISSLQSEDFAVYYCQQYHSFPFTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 256 | Anti-B7H4 LC | EIVMTQSPATLSVSPGERATLSCRASQSVSSNLAWYQQKPGQAPRLLIYGASTRATGIPA RFSGSGSGTEFTLTISSLQSEDFAVYYCQQYHSFPFTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 257 | Anti-B7H4 HC | QLQLQESGPGLVKPSETLSLTCTVSGGSIKSGSYYWGWIRQPPGKGLEWIGNIYYSGSTY YNPSLKSRVTISVDTSKNQFSLKLSSVTAADTAVYYCAREGSYPNQFDPWGQGILVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 258 | Anti-B7H4 LC | EIVMTQSPATLSVSPGERATLSCRASQSVSSNLAWYQQKPGQAPRLLIYGASTRATGIPA RFSGSGSGTEFTLTISSLQSEDFAVYYCQQYHSFPFTFGGGTKVEIKRTVAAPSVFIFPP |
| | | SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 259 | Anti-B7H4 HC | QLQLQESGPGLVKPSETLSLTCTVSGGSIKSGSHYWGWIRQPPGKGLEWIGNIYYSGSTY YNPSLKSRVTMSVDTSKNQFSLKLSSVTAADTAVYYCAREGSYPNWFDPWGQGTLVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 260 | Anti-B7H4 LC | EIVMTQSPATLSVSPGERATLSCRASQSVSTNLAWYQQKPGQAPRLLIYDASARVTGIPA RFSGSGSGTEFTLTISSLQSEDFAVYYCQQYHSFPFTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 261 | Anti-B7H4 H C | QLQLQESGPGLVKPSETLSLTCTVSGGSISSSSYYWGWIRQPPGKGLEWIGNIYYSGSTY YNPSLKSRVTISVDTSKNQFSLKLSSVTAADTAVYYCAREGSYTTVLNVWGQGTMVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 262 | Anti-B7H4 LC | EIVLTQSPGTLSLSPGERATLSCRASQSVSSSYLAWYQQKPGQAPRLLIYGASSRATGIP DRFSGSGSGTDFTLTISRLEPEDFAVYYCQQAASYPLTFGGGTKVEIKRTVAAPSVFIFP PSDEQLKSGTASVVCLLNNFYPREAKVQWKVDNALQSGNSQESVTEQDSKDSTYSLSSTL TLSKADYEKHKVYACEVTHQGLSSPVTKSFNRGEC |
| 263 | Anti-B7H4 HC | QLQLQESGPGLVKPSETLSLTCTVSGGSIGRGSYYWGWIRQPPGKGLEWIGNIYYSGSTY YNPSLKSRVTISVDTSKNQFSLKLSSVTAADTAVYYCAREGSYTTVLNVWGQGTMVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 264 | Anti-B7H4 LC | EIVLTQSPGTLSLSPGERATLSCRASQSVASSHLAWYQQKPGQAPRLLIYDAVSRATGIP DRFSGSGSGTDFTLTISRLEPEDFAVYYCQQAASYPLTFGGGTKVEIKRTVAAPSVFIFP PSDEQLKSGTASVVCLLNNFYPREAKVQWKVDNALQSGNSQESVTEQDSKDSTYSLSSTL TLSKADYEKHKVYACEVTHQGLSSPVTKSFNRGEC |
| 265 | Anti-B7H4 HC | QVQLQESGPGLVKPSQTLSLTCTVSGGSISSGGYYWSWIRQHPGKGLEWIGNIYYSGSTY YNPSLKSRVTISVDTSKNQFSLKLSSVTAADTAVYYCARESSTISADFDLWGRGTLVTVS SASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSRD ELTKNQVSLTCLVKGFYPSDIAVEWESNGQPENNYKTTPPVLDSDGSFFLYSKLTVDKSR WQQGNVFSCSVMHEALHNHYTQKSLSLSPGK |
| 266 | Anti-B7H4 LC | DIQMTQSPSSVSASVGDRVTITCRASQGISRWLAWYQQKPGKAPKLLIYAASSLQSGVPS RFSGSGSGTDFTLTISSLQPEDFATYYCQQAHTFPYTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 267 | Anti-B7H4 HC | QLQLQESGPGLVKPSETLSLTCTASGGSISHGGYYWSWIRQHPGKGLEWIGNIYYSGSTY YNPSLKSRVTMSVDTSKNQFSLKLSSVTAADTAVYYCARESSTISADFDLWGRGTLVTVS SASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSRD ELTKNQVSLTCLVKGFYPSDIAVEWESNGQPENNYKTTPPVLDSDGSFFLYSKLTVDKSR WQQGNVFSCSVMHEALHNHYTQKSLSLSPGK |
| 268 | Anti-B7H4 LC | DIQMTQSPSSVSASVGDRVTITCRASQGISRWLAWYQQKPGKAPKLLIYAASSLQSGVPS RFSGSGSGTDFTLTISSLQPEDFATYYCQQAHTFPYTFGGGTKVEIKRTVAAPSVFIFPP SDEQLKSGTASVVCLLNNFYPREAKVQWKVDNALQSGNSQESVTEQDSKDSTYSLSSTLT |
| | | LSKADYEKHKVYACEVTHQGLSSPVTKSFNRGEC |
| 269 | Anti-B7H4 H C | QVQLQESGPGLVKPSQTLSLTCTVSGGSISSGGYYWSWIRQHPGKGLEWIGNIYYSGSTY YNPSLKSRVTISVDTSKNQFSLKLSSVTAADTAVYYCARGLSTIDEAFDPWGQGTLVTVS SASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSRD ELTKNQVSLTCLVKGFYPSDIAVEWESNGQPENNYKTTPPVLDSDGSFFLYSKLTVDKSR WQQGNVFSCSVMHEALHNHYTQKSLSLSPGK |
| 270 | Anti-B7H4 LC | DIQMTQSPSTLSASVGDRVTITCRASQSISSWLAWYQQKPGKAPKLLIYKASSLESGVPS RFSGSGSGTEFTLTISSLQPDDFATYYCQQDNSYPYTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 271 | Anti-B7H4 HC | QLQLQESGPGLVKPSETLSLTCTVSGGSISDGSYYWSWIRQHPGKGLEWIGNIYYSGSTY YNPSLRSRVTMSVDTSKNQFSLKLSSVTAADTAVYYCARGLSTIDEAFDPWGQGTLVTVS SASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSRD ELTKNQVSLTCLVKGFYPSDIAVEWESNGQPENNYKTTPPVLDSDGSFFLYSKLTVDKSR WQQGNVFSCSVMHEALHNHYTQKSLSLSPGK |
| 272 | Anti-B7H4 LC | DIQMTQSPSTLSASVGDRVTITCRASQSISSWLAWYQQKPGKAPKLLIYKASSLESGVPS RFSGSGSGTEFTLTISSLQPDDFATYYCQQDNSYPYTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 273 | Anti-B7H4 H C | QVQLQESGPGLVKPSQTLSLTCTVSGGSISDGSYYWSWIRQHPGKGLEWIGNIYYSGSTY YNPSLRSRVTMSVDTSKNQFSLKLSSVTAADTAVYYCARGLSTIDEAFDPWGQGTLVTVS SASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSRD ELTKNQVSLTCLVKGFYPSDIAVEWESNGQPENNYKTTPPVLDSDGSFFLYSKLTVDKSR WQQGNVFSCSVMHEALHNHYTQKSLSLSPGK |
| 274 | Anti-B7H4 LC | DIQMTQSPSTLSASVGDRVTITCRASKSISSWLAWYQQKPGKAPKLLIYEASSLHSGVPS RFSGSGSGTEFTLTISSLQPDDFATYYCQQDNSYPYTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 275 | Anti-B7H4 HC | QVQLQESGPGLVKPSETLSLTCTVSGGSISSYYWSWIRQPPGKGLEWIGYIYSSGSTNYN PSLKSRVTISVDTSKNQFSLKLSSVTAADTAVYYCARGSGQYAAPDYGMDVWGQGTTVTV SSASTKGPSVFPLAPSSKSTSGGTAALGCLVKDYFPEPVTVSWNSGALTSGVHTFPAVLQ SSGLYSLSSVVTVPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREPQVYTLPPSR DELTKNQVSLTCLVKGFYPSDIAVEWESNGQPENNYKTTPPVLDSDGSFFLYSKLTVDKS RWQQGNVFSCSVMHEALHNHYTQKSLSLSPGK |
| 276 | Anti-B7H4 LC | DIQMTQSPSTLSASVGDRVTITCRASQSISSWLAWYQQKPGKAPKLLIYKASSLESGVPS RFSGSGSGTEFTLTISSLQPDDFATYYCQQDNSFPFTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 277 | Anti-B7H4 HC | QVQLQESGPGLVKPSETLSLTCTVSGGSIISYYWGWIRQPPGKGLEWIGYIYSSGSTSYN PSLKSRVTISVDTSKNQFSLKLSSVTAADTAVYYCARGSGLYAAPDYGLDVWGQGTTVTV SSASTKGPSVFPLAPSSKSTSGGTAALGCLVKDYFPEPVTVSWNSGALTSGVHTFPAVLQ SSGLYSLSSVVTVPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREPQVYTLPPSR DELTKNQVSLTCLVKGFYPSDIAVEWESNGQPENNYKTTPPVLDSDGSFFLYSKLTVDKS RWQQGNVFSCSVMHEALHNHYTQKSLSLSPGK |
| 278 | Anti-B7H4 LC | DIQMTQSPSTLSASVGDRVTITCRASQSISSWLAWYQQKPGKAPKLLIYKASSLESGVPS RFSGSGSGTEFTLTISSLQPDDFATYYCQQDNSFPFTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 279 | Anti-B7H4 HC | EVQLLESGGGLVQPGGSLRLSCAASGFTFSSYAMSWVRQAPGKGLEWVSTISGSGGSTYY ADSVKGRFTISRDNSKNTLYLQMNSLRAEDTAVYYCARGAGHYDLVGRYWGQGTLVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 280 | Anti-B7H4 LC | DIQMTQSPSSLSASVGDRVTITCRASQSISSYLNWYQQKPGKAPKLLIYAASSLQSGVPS RFSGSGSGTDFTLTISSLQPEDFATYYCQQLYSLPPTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 281 | Anti-B7H4 HC | EVQLLESGGGLVQPGGSLRLSCAASGFTFSSYAMSWVRQAPGKGLEWVSAISGSGGSTYY ADSVKGRFTISRDNSKNTLYLQMNSLRAEDTAVYYCARVGFRALNYWGQGTTVTVSSAST KGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPELLGGPSV FLFPPKPKDTLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPPSRDELTK NQVSLTCLVKGFYPSDIAVEWESNGQPENNYKTTPPVLDSDGSFFLYSKLTVDKSRWQQG NVFSCSVMHEALHNHYTQKSLSLSPGK |
| 282 | Anti-B7H4 LC | DIQLTQSPSSVSASVGDRVTITCRASQDISSWLAWYQQKPGKAPKLLIYAASSLQSGVPS RFSGSGSGTDFTLTISSLQPEDFATYYCQQATSYPPWTFGGGTKVEIKRTVAAPSVFIFP PSDEQLKSGTASVVCLLNNFYPREAKVQWKVDNALQSGNSQESVTEQDSKDSTYSLSSTL TLSKADYEKHKVYACEVTHQGLSSPVTKSFNRGEC |
| 283 | Anti-B7H4 HC | QVQLVQSGAEVKKPGSSVKVSCKASGGTFSSYAISWVRQAPGQGLEWMGGIIPIFGTASY AQKFQGRVTITADESTSTAYMELSSLRSEDTAVYYCARQQYDGRRYFGLWGRGTLVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 284 | Anti-B7H4 LC | EIVMTQSPATLSVSPGERATLSCRASQSVSSNLAWYQQKPGQAPRLLIYSASTRATGIPA RFSGSGSGTEFTLTISSLQSEDFAVYYCQQVNVWPPTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 285 | Anti-B7H4 HC | QVQLVQSGAEVKKPGSSVKVSCKASGGTFSSYAISWVRQAPGQGLEWMGGIIPIFGTANY AQKFQGRVTITADESTSTAYMELSSLRSEDTAVYYCARGGPWFDPWGQGTLVTVSSASTK GPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPSRDELTKN QVSLTCLVKGFYPSDIAVEWESNGQPENNYKTTPPVLDSDGSFFLYSKLTVDKSRWQQGN VFSCSVMHEALHNHYTQKSLSLSPGK |
| 286 | Anti-B7H4 LC | DIQMTQSPSTLSASVGDRVTITCRASQSISSWLAWYQQKPGKAPKLLIYKASSLESGVPS RFSGSGSGTEFTLTISSLQPDDFATYYCQQYNSYPPFTFGGGTKVEIKRTVAAPSVFIFP PSDEQLKSGTASVVCLLNNFYPREAKVQWKVDNALQSGNSQESVTEQDSKDSTYSLSSTL TLSKADYEKHKVYACEVTHQGLSSPVTKSFNRGEC |
| 287 | Anti-B7H4 HC | EVQLLESGGGLVQPGGSLRLSCAASGFTFSSYAMSWVRQAPGKGLEWVSAISGSGGSTSY ADSVKGRFTISRDNSKNTLYLQMNSLRAEDTAVYYCAKPSLATMLAFDIWGQGTMVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 288 | Anti-B7H4 LC | DIQMTQSPSTLSASVGDRVTITCRASQSISSWLAWYQQKPGKAPKLLIYDASSLESGVPS RFSGSGSGTEFTLTISSLQPDDFATYYCQQSKSYPRTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 289 | Anti-B7H4 HC | QLQLQESGPGLVKPSETLSLTCTVSGGSISSSVYYWSWIRQPPGKGLEWIGSILVSGSTY |
| | | YNPSLKSRVTISVDTSKNQFSLKLSSVTAADTAVYYCARAVSFLDVWGQGTMVIVSSAST KGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPELLGGPSV FLFPPKPKDTLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPPSRDELTK NQVSLTCLVKGFYPSDIAVEWESNGQPENNYKTTPPVLDSDGSFFLYSKLTVDKSRWQQG NVFSCSVMHEALHNHYTQKSLSLSPGK |
| 290 | Anti-B7H4 LC | DIQMTQSPSSLSASVGDRVTITCRASQSISSYLNWYQQKPGKAPKLLIYGASSLQSGVPS RFSGSGSGTDFTLTISSLQPEDFATYYCQQSYDPPWTFGGGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 291 | Anti-CD70 CDR-H1 | NYGMN |
| 292 | Anti-CD70CDR-H2 | WINTYTGEPTYADAFKG |
| 293 | Anti-CD70 CDR-H3 | DYGDYGMDY |
| 294 | Anti-CD70 CDR-L1 | RASKSVSTSGYSFMH |
| 295 | Anti-CD70 CDR-L2 | LASNLES |
| 296 | Anti-CD70 CDR-L3 | QHSREVPWT |
| 297 | Anti-CD70 VH | QVQLVQSGAEVKKPGASVKVSCKASGYTFTNYGMNWVRQAPGQGLKWMGWINTYTGEPTY ADAFKGRVTMTRDTSISTAYMELSRLRSDDTAVYYCARDYGDYGMDYWGQGTTVTVSS |
| 298 | Anti-CD70 VL | DIVMTQSPDSLAVSLGERATINCRASKSVSTSGYSFMHWYQQKPGQPPKLLIYLASNLES GVPDRFSGSGSGTDFTLTISSLQAEDVAVYYCQHSREVPWTFGQGTKVEIK |
| 299 | Anti-CD70 HC | QVQLVQSGAEVKKPGASVKVSCKASGYTFTNYGMNWVRQAPGQGLKWMGWINTYTGEPTY ADAFKGRVTMTRDTSISTAYMELSRLRSDDTAVYYCARDYGDYGMDYWGQGTTVTVSSAS TKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPELLGGPS VFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPSRDELT KNQVSLTCLVKGFYPSDIAVEWESNGQPENNYKTTPPVLDSDGSFFLYSKLTVDKSRWQQ GNVFSCSVMHEALHNHYTQKSLSLSPGK |
| 300 | Anti-CD70 LC | DIVMTQSPDSLAVSLGERATINCRASKSVSTSGYSFMHWYQQKPGQPPKLLIYLASNLES GVPDRFSGSGSGTDFTLTISSLQAEDVAVYYCQHSREVPWTFGQGTKVEIKRTVAAPSVF IFPPSDEQLKSGTASVVCLLNNFYPREAKVQWKVDNALQSGNSQESVTEQDSKDSTYSLS STLTLSKADYEKHKVYACEVTHQGLSSPVTKSFNRGEC |
| 301 | Anti-TROP2 CDR-H1 | NYGMN |
| 302 | Anti-TROP2 CDR-H2 | WINTYTGEPTYTDDFKG |
| 303 | Anti-TROP2 CDR-H3 | GGFGSSYWYFDV |
| 304 | Anti-TROP2 CDR-L1 | KASQDVSIAVA |
| 305 | Anti-TROP2 CDR-L2 | SASYRYT |
| 306 | Anti-TROP2 CDR-L3 | QQHYITPLT |
| 307 | Anti-TROP2 VH | QVQLQQSGSELKKPGASVKVSCKASGYTFTNYGMNWVKQAPGQGLKWMGWINTYTGEPTY TDDFKGRFAFSLDTSVSTAYLQISSLKADDTAVYFCARGGFGSSYWYFDVWGQGSLVTVS S |
| 308 | Anti-TROP2 VL | DIQLTQSPSSLSASVGDRVSITCKASQDVSIAVAWYQQKPGKAPKLLIYSASYRYTGVPD RFSGSGSGTDFTLTISSLQPEDFAVYYCQQHYITPLTFGAGTKVEIK |
| 309 | Anti-TROP2 CDR-H1 | TAGMQ |
| 310 | Anti-TROP2 CDR-H2 | WINTHSGVPKYAEDFKG |
| 311 | Anti-TROP2 CDR-H3 | SGFGSSYWYFDV |
| 312 | Anti-TROP2 CDR-L1 | KASQDVSTAVA |
| 313 | Anti-TROP2 CDR-L2 | SASYRYT |
| 314 | Anti-TROP2 CDR-L3 | QQHYITPLT |
| 315 | Anti-TROP2 VH | QVQLVQSGAEVKKPGASVKVSCKASGYTFTTAGMQWVRQAPGQGLEWMGWINTHSGVPKY AEDFKGRVTISADTSTSTAYLQLSSLKSEDTAVYYCARSGFGSSYWYFDVWGQGTLVTVS S |
| 316 | Anti-TROP2 VL | DIQMTQSPSSLSASVGDRVTITCKASQDVSTAVAWYQQKPGKAPKLLIYSASYRYTGVPS RFSGSGSGTDFTLTISSLQPEDFAVYYCQQHYITPLTFGQGTKLEIK |
| 317 | MICA CDR-H1 | SQNIY |
| 318 | MICA CDR-H2 | YIEPYNVVPMYNPKFKG |
| 319 | MICA CDR-H3 | SGSSNFDY |
| 320 | MICA CDR-L1 | SASSSISSHYLH |
| 321 | MICA CDR-L2 | RTSNLAS |
| 322 | MICA CDR-L3 | QQGSSLPLT |
| 323 | Anti-MICA VH | EIQLVQSGAEVKKPGASVKVSCKASGYAFTSQNIYWVRQAPGQGLEWIGYIEPYNVVPMY NPKFKGRATLTVDKSTSTAYLELSSLRSEDTAVYYCARSGSSNFDYWGQGTLVTVSS |
| 324 | Anti-MICA VL | DIQLTQSPSSLSASVGDRVTITCSASSSISSHYLHWYQQKPGKSPKLLIYRTSNLASGVP SRFSGSGSGTDYTLTISSLQPEDFATYYCQQGSSLPLTFGQGTKVEIK |
| 325 | Anti-MICA CDR-H1 | NYAMH |
| 326 | Anti-MICA CDR-H2 | LIWYDGSNKFYGDSVKG |
| 327 | Anti-MICA CDR-H3 | EGSGHY |
| 328 | Anti-MICA CDR-L1 | RASQGISSALA |
| 329 | Anti-MICA CDR-L2 | DASSLES |
| 330 | Anti-MICA CDR-L3 | QQFNSYPIT |
| 331 | Anti-MICA VH | QVQLVESGGGVVQPGRSLRLSCAASGFTFSNYAMHWVRQAPGEGLEWVALIWYDGSNKFY GDSVKGRFTISRDNSKNTLYLQMNSLSAEDTAVYYCAREGSGHYWGQGTLVTVSS |
| 332 | Anti-MICA VL | AIQLTQSPSSLSASVGDRVTITCRASQGISSALAWYQQKPGKVPKSLIYDASSLESGVPS RFSGSGSGTDFTLTISSLQPEDFATYYCQQFNSYPITFGQGTRLEIK |
| 333 | Anti-MICA CDR-H1 | NYAMS |
| 334 | Anti-MICA CDR-H2 | YISPGGDYIYYADSVKG |
| 335 | Anti-MICA CDR-H3 | DRRHYGSYAMDY |
| 336 | Anti-MICA CDR-L1 | RSSKSLLHSNLNTYLY |
| 337 | Anti-MICA CDR-L2 | RMSNLAS |
| 338 | Anti-MICA CDR-L3 | MQHLEYPFT |
| 339 | Anti-MICA VH | QVQLVESGGGLVKPGGSLRLSCAASGFTFSNYAMSWIRQAPGKGLEWVSYISPGGDYIYY ADSVKGRFTISRDNAKNSLYLQMNSLRAEDTAVYYCTTDRRHYGSYAMDYWGQGTLVTVS S |
| 340 | Anti-MICA VL | DIVMTQSPLSLPVTPGEPASISCRSSKSLLHSNLNTYLYWFLQKPGQSPQILIYRMSNLA SGVPDRFSGSGSGTAFTLKISRVEAEDVGVYYCMQHLEYPFTFGPGTKLEIK |
| 341 | Anti-MICA CDR-H1 | TYAFH |
| 342 | Anti-MICA CDR-H2 | GIVPIFGTLKYAQKFQD |
| 343 | Anti-MICA CDR-H3 | AIQLEGRPFDH |
| 344 | Anti-MICA CDR-L1 | RASQGITSYLA |
| 345 | Anti-MICA CDR-L2 | AASALQS |
| 346 | Anti-MICA CDR-L3 | QQVNRGAAIT |
| 347 | Anti-MICA VH | QVQLVQSGAEVKKPGSSVRVSCRASGGSSTTYAFHWVRQAPGQGLEWMGGIVPIFGTLKY AQKFQDRVTLTADKSTGTAYMELNSLRLDDTAVYYCARAIQLEGRPFDHWGQGTQVTVSA |
| 348 | Anti-MICA VL | DIQLTQSPSFLSASVGDRVTITCRASQGITSYLAWYQQKPGKAPKLLIYAASALQSGVPS RFSGRGSGTEFTLTISSLQPEDFATYYCQQVNRGAAITFGHGTRLDIK |
| 349 | Anti-ITGav/CD51 CDR-H1 | RYTMH |
| 350 | Anti-ITGav/CD51 CDR-H2 | VISFDGSNKYYVDSVKG |
| 351 | Anti-ITGav/CD51 CDR-H3 | EARGSYAFDI |
| 352 | Anti-ITGav/CD51 CDR-L1 | RASQSVSSYLA |
| 353 | Anti-ITGav/CD51 CDR-L2 | DASNRAT |
| 354 | Anti-ITGav/CD51 CDR-L3 | QQRSNWPPFT |
| 355 | Anti-ITGav/CD51 VH | QVQLVESGGGVVQPGRSRRLSCAASGFTFSRYTMHWVRQAPGKGLEWVAVISFDGSNKYY VDSVKGRFTISRDNSENTLYLQVNILRAEDTAVYYCAREARGSYAFDIWGQGTMVTVSS EIVLTQSPATLSLSPGERATLSCRASQSVSSYLAWYQQKPGQAPRLLIYDASNRATGIPA RFSGSGSGTDFTLTISSLEPEDFAVYYCQQRSNWPPFTFGPGTKVDIK |
| 356 | Anti-ITGav/CD51 VL | EIVLTQSPATLSLSPGERATLSCRASQSVSSYLAWYQQKPGQAPRLLIYDASNRATGIPA RFSGSGSGTDFTLTISSLEPEDFAVYYCQQRSNWPPFTFGPGTKVDIK |
| 357 | Anti-ITGav CDR-H1 | SFWMH |
| 358 | Anti-ITGav CDR-H2 | YINPRSGYTEYNEIFRD |
| 359 | Anti-ITGav CDR-H3 | FLGRGAMDY |
| 360 | Anti-ITGav CDR-L1 | RASQDISNYLA |
| 361 | Anti-ITGav CDR-L2 | YTSKIHS |
| 362 | Anti-ITGav CDR-L3 | QQGNTFPYT |
| 363 | Anti-ITGav VH | |
| 364 | Anti-ITGav VL | |
| 365 | Anti-gpA33 CDR-H1 | TSSYYWG |
| 366 | Anti-gpA33 CDR-H2 | TIYYNGSTYYSPSLKS |
| 367 | Anti-gpA33 CDR-H3 | QGYDIKINIDV |
| 368 | Anti-gpA33 CDR-L1 | RASQSVSSYLA |
| 369 | Anti-gpA33 CDR-L2 | VASNRAT |
| 370 | Anti-gpA33 CDR-L3 | QQRSNWPLT |
| 371 | Anti-gpA33 VH | QLQLQESGPGLVKPSETLSLTCTVSGGSISTSSYYWGWIRQPPGKGLEWIGTIYYNGSTY YSPSLKSRVSISVDTSKNQFSLKLSSVTAADTSVYYCARQGYDIKINIDVWGQGTTVTVS S |
| 372 | Anti-gpA33 VL | EIVLTQSPATLSLSPGERATLSCRASQSVSSYLAWYQQKPGQAPRLLIYVASNRATGIPA RFSGSGSGTDFTLTISSLEPEDFAVYYCQQRSNWPLTFGGGTKVEIK |
| 373 | Anti-IL1Rap CDR-H1 | SSWMN |
| 374 | Anti-IL1Rap CDR-H2 | RIYPGDGNTHYAQKFQG |
| 375 | Anti-IL1Rap CDR-H3 | GYLDPMDY |
| 376 | Anti-IL1Rap CDR-L1 | QASQGINNYLN |
| 377 | Anti-IL1Rap CDR-L2 | YTSGLHA |
| 378 | Anti-IL1Rap CDR-L3 | QQYSILPWT |
| 379 | Anti-IL1Rap VH | QVQLVQSGAEVKKPGSSVKVSCKASGYAFTSSWMNWVRQAPGQGLEWMGRIYPGDGNTHY AQKFQGRVTLTADKSTSTAYMELSSLRSEDTAVYYCGEGYLDPMDYWGQGTLVTVSS |
| 380 | Anti-IL1Rap VL | DIQMTQSPSSLSASVGDRVTITCQASQGINNYLNWYQQKPGKAPKLLIHYTSGLHAGVPS RFSGSGSGTDYTLTISSLEPEDVATYYCQQYSILPWTFGGGTKVEIK |
| 381 | Anti-EpCAM CDR-H1 | SYGMH |
| 382 | Anti-EpCAM CDR-H2 | VISYDGSNKYYADSVKG |
| 383 | Anti-EpCAM CDR-H3 | DMGWGSGWRPYYYYGMDV |
| 384 | Anti-EpCAM CDR-L1 | RTSQSISSYLN |
| 385 | Anti-EpCAM CDR-L2 | WASTRES |
| 386 | Anti-EpCAM CDR-L3 | QQSYDIPYT |
| 387 | Anti-EpCAM VH | EVQLLESGGGVVQPGRSLRLSCAASGFTFSSYGMHWVRQAPGKGLEWVAVISYDGSNKYY ADSVKGRFTISRDNSKNTLYLQMNSLRAEDTAVYYCAKDMGWGSGWRPYYYYGMDVWGQG TTVTVSS |
| 388 | Anti-EpCAM VL | ELQMTQSPSSLSASVGDRVTITCRTSQSISSYLNWYQQKPGQPPKLLIYWASTRESGVPD RFSGSGSGTDFTLTISSLQPEDSATYYCQQSYDIPYTFGQGTKLEIK |
| 389 | Anti-EpCAM CDR-H1 | NYWMS |
| 390 | Anti-EpCAM CDR-H2 | NIKQDGSEKFYADSVKG |
| 391 | Anti-EpCAM CDR-H3 | VGPSWEQDY |
| 392 | Anti-EpCAM CDR-L1 | TGSSSNIGSYYGVH |
| 393 | Anti-EpCAM CDR-L2 | SDTNRPS |
| 394 | Anti-EpCAM CDR-L3 | QSYDKGFGHRV |
| 395 | Anti-EpCAM VH | EVQLVESGGGLVQPGGSLRLSCAASGFTFSNYWMSWVRQAPGKGLEWVANIKQDGSEKFY ADSVKGRFTISRDNAKNSLYLQMNSLRAEDTAVYYCARVGPSWEQDYWGQGTLVTVSA |
| 396 | Anti-EpCAM VL | QSVLTQPPSVSGAPGQRVTISCTGSSSNIGSYYGVHWYQQLPGTAPKLLIYSDTNRPSGV PDRFSGSKSGTSASLAITGLQAEDEADYYCQSYDKGFGHRVFGGGTKLTVL |
| 397 | Anti-EpCAM CDR-H1 | SYAIS |
| 398 | Anti-EpCAM CDR-H2 | GIIPIFGTANYAQKFQG |
| 399 | Anti-EpCAM CDR-H3 | GLLWNY |
| 400 | Anti-EpCAM CDR-L1 | RASQSVSSNLA |
| 401 | Anti-EpCAM CDR-L2 | GASTTAS |
| 402 | Anti-EpCAM CDR-L3 | QQYNNWPPAYT |
| 403 | Anti-EpCAM VH | QVQLVQSGAEVKKPGSSVKVSCKASGGTFSSYAISWVRQAPGQGLEWMGGIIPIFGTANY AQKFQGRVTITADESTSTAYMELSSLRSEDTAVYYCARGLLWNYWGQGTLVTVSS |
| 404 | Anti-EpCAM VL | EIVMTQSPATLSVSPGERATLSCRASQSVSSNLAWYQQKPGQAPRLIIYGASTTASGIPA RFSASGSGTDFTLTISSLQSEDFAVYYCQQYNNWPPAYTFGQGTKLEIK |
| 405 | Anti-EpCAM CDR-H1 | NYGMN |
| 406 | Anti-EpCAM CDR-H2 | WINTYTGEPTYGEDFKG |
| 407 | Anti-EpCAM CDR-H3 | FGNYVDY |
| 408 | Anti-EpCAM CDR-L1 | RSSKNLLHSNGITYLY |
| 409 | Anti-EpCAM CDR-L2 | QMSNLAS |
| 410 | Anti-EpCAM CDR-L3 | AQNLEIPRT |
| 411 | Anti-EpCAM VH | QVQLVQSGPEVKKPGASVKVSCKASGYTFTNYGMNWVRQAPGQGLEWMGWINTYTGEPTY GEDFKGRFAFSLDTSASTAYMELSSLRSEDTAVYFCARFGNYVDYWGQGSLVTVSS |
| 412 | Anti-EpCAM VL | DIVMTQSPLSLPVTPGEPASISCRSSKNLLHSNGITYLYWYLQKPGQSPQLLIYQMSNLA SGVPDRFSSSGSGTDFTLKISRVEAEDVGVYYCAQNLEIPRTFGQGTKVEIK |
| 413 | Anti-EpCAM CDR-H1 | KYGMN |
| 414 | Anti-EpCAM CDR-H2 | WINTYTEEPTYGDDFKG |
| 415 | Anti-EpCAM CDR-H3 | FGSAVDY |
| 416 | Anti-EpCAM CDR-L1 | RSSKSLLHSNGITYLY |
| 417 | Anti-EpCAM CDR-L2 | QMSNRAS |
| 418 | Anti-EpCAM CDR-L3 | AQNLELPRT |
| 419 | Anti-EpCAM VH | QIQLVQSGPEVKKPGESVKISCKASGYTFTKYGMNWVKQAPGQGLKWMGWINTYTEEPTY GDDFKGRFTFTLDTSTSTAYLEISSLRSEDTATYFCARFGSAVDYWGQGTLVTVSS |
| 420 | Anti-EpCAM VL | DIVMTQSALSNPVTLGESGSISCRSSKSLLHSNGITYLYWYLQKPGQSPQLLIYQMSNRA SGVPDRFSSSGSGTDFTLKISRVEAEDVGVYYCAQNLELPRTFGQGTKLEMKR |
| 421 | Anti-EpCAM CDR-H1 | DYSMH |
| 422 | Anti-EpCAM CDR-H2 | WINTETGEPTYADDFKG |
| 423 | Anti-EpCAM CDR-H3 | TAVY |
| 424 | Anti-EpCAM CDR-L1 | RASQEISVSLS |
| 425 | Anti-EpCAM CDR-L2 | ATSTLDS |
| 426 | Anti-EpCAM CDR-L3 | LQYASYPWT |
| 427 | Anti-EpCAM VH | QVKLQESGPELKKPGETVKISCKASGYTFTDYSMHWVKQAPGKGLKWMGWINTETGEPTY ADDFKGRFAFSLETSASTAYLQINNLKNEDTATYFCARTAVYWGQGTTVTVSS |
| 428 | Anti-EpCAM VL | DIQMTQSPSSLSASLGERVSLTCRASQEISVSLSWLQQEPDGTIKRLIYATSTLDSGVPK RFSGSRSGSDYSLTISSLESEDFVDYYCLQYASYPWTFGGGTKLEIKR |
| 429 | Anti-CD352 CDR-H1 | NYGMN |
| 430 | Anti-CD352 CDR-H2 | WINTYSGEPRYADDFKG |
| 431 | Anti-CD352 CDR-H3 | DYGRWYFDV |
| 432 | Anti-CD352 CDR-L1 | RASSSVSHMH |
| 433 | Anti-CD352 CDR-L2 | ATSNLAS |
| 434 | Anti-CD352 CDR-L3 | QQWSSTPRT |
| 435 | Anti-CD352 VH | QIQLVQSGSELKKPGASVKVSCKASGYTFTNYGMNWVRQAPGQDLKWMGWINTYSGEPRY ADDFKGRFVFSLDKSVNTAYLQISSLKAEDTAVYYCARDYGRWYFDVWGQGTTVTVSS |
| 436 | Anti-CD352 VL | QIVLSQSPATLSLSPGERATMSCRASSSVSHMHWYQQKPGQAPRPWIYATSNLASGVPAR FSGSGSGTDYTLTISSLEPEDFAVYYCQQWSSTPRTFGGGTKVEIKR |
| 437 | Anti-CS1 CDR-H1 | RYWMS |
| 438 | Anti-CS1 CDR-H2 | EINPDSSTINYAPSLKD |
| 439 | Anti-CS1 CDR-H3 | PDGNYWYFDV |
| 440 | Anti-CSl CDR-L1 | KASQDVGIAVA |
| 441 | Anti-CSl CDR-L2 | WASTRHT |
| 442 | Anti-CS1 CDR-L3 | QQYSSYPYT |
| 443 | Anti-CSl VH | EVQLVESGGGLVQPGGSLRLSCAASGFDFSRYWMSWVRQAPGKGLEWIGEINPDSSTINY APSLKDKFIISRDNAKNSLYLQMNSLRAEDTAVYYCARPDGNYWYFDVWGQGTLVTVSS |
| 444 | Anti-CSl VL | DIQMTQSPSSLSASVGDRVTITCKASQDVGIAVAWYQQKPGKVPKLLIYWASTRHTGVPD RFSGSGSGTDFTLTISSLQPEDVATYYCQQYSSYPYTFGQGTKVEIKR |
| 445 | Anti-CD38 CDR-H1 | SFAMS |
| 446 | Anti-CD38 CDR-H2 | AISGSGGGTYYADSVKG |
| 447 | Anti-CD38 CDR-H3 | DKILWFGEPVFDY |
| 448 | Anti-CD38 CDR-L1 | RASQSVSSYLA |
| 449 | Anti-CD38 CDR-L2 | DASNRAT |
| 450 | Anti-CD38 CDR-L3 | QQRSNWPPT |
| 451 | Anti-CD38 VH | |
| 452 | Anti-CD38 VL | EIVLTQSPATLSLSPGERATLSCRASQSVSSYLAWYQQKPGQAPRLLIYDASNRATGIPA RFSGSGSGTDFTLTISSLEPEDFAVYYCQQRSNWPPTFGQGTKVEIKR |
| 453 | Anti-CD25 CDR-H1 | SY RMH |
| 454 | Anti-CD25 CDR-H2 | YINPSTGYTEYNQKFKD |
| 455 | Anti-CD25 CDR-H3 | GGGVFDY |
| 456 | Anti-CD25 CDR-L1 | SASSSISYMH |
| 457 | Anti-CD25 CDR-L2 | TTSNLAS |
| 458 | Anti-CD25 CDR-L3 | HQRSTYPLT |
| 459 | Anti-CD25 VH | QVQLVQSGAEVKKPGSSVKVSCKASGYTFTSYRMHWVRQAPGQGLEWIGYINPSTGYTEY NQKFKDKATITADESTNTAYMELSSLRSEDTAVYYCARGGGVFDYWGQGTLVTVSS |
| 460 | Anti-CD25 VL | DIQMTQSPSTLSASVGDRVTITCSASSSISYMHWYQQKPGKAPKLLIYTTSNLASGVPAR FSGSGSGTEFTLTISSLQPDDFATYYCHQRSTYPLTFGQGTKVEVK |
| 461 | Anti-ADAM9 CDR-H1 | SYWMH |
| 462 | Anti-ADAM9 CDR-H2 | EIIPINGHTNYNEKFKS |
| 463 | Anti-ADAM9 CDR-H3 | GGYYYYGSRDYFDY |
| 464 | Anti-ADAM9 CDR-L1 | KASQSVDYDGDSYMN |
| 465 | Anti-ADAM9 CDR-L2 | AASDLES |
| 466 | Anti-ADAM9 CDR-L3 | QQSHEDPFT |
| 467 | Anti-ADAM9 VH | QVQLQQPGAELVKPGASVKLSCKASGYTFTSYWMHWVKQRPGQGLEWIGEIIPINGHTNY NEKFKSKATLTLDKSSSTAYMQLSSLASEDSAVYYCARGGYYYYGSRDYFDYWGQGTTLT VSS DIVLTQSPASLAVSLGQRATISCKASQSVDYDGDSYMNWYQQIPGQPPKLLIYAASDLES GIPARFSGSGSGTDFTLNIHPVEEEDAATYYCQQSHEDPFTFGGGTKLEIK |
| 468 | Anti-ADAM9 VL | DIVLTQSPASLAVSLGQRATISCKASQSVDYDGDSYMNWYQQIPGQPPKLLIYAASDLES GIPARFSGSGSGTDFTLNIHPVEEEDAATYYCQQSHEDPFTFGGGTKLEIK |
| 469 | Anti-ADAM9 CDR-H1 | SYWMH |
| 470 | Anti-ADAM9 CDR-H2 | EIIPIFGHTNYNEKFKS |
| 471 | Anti-ADAM9 CDR-H3 | GGYYYYPRQGFLDY |
| 472 | Anti-ADAM9 CDR-L1 | KASQSVDYSGDSYMN |
| 473 | Anti-ADAM9 CDR-L2 | AASDLES |
| 474 | Anti-ADAM9 CDR-L3 | QQSHEDPFT |
| 475 | Anti-ADAM9 VH | EVQLVESGGGLVKPGGSLRLSCAASGFTFSSYWMHWVRQAPGKGLEWVGEIIPIFGHTNY NEKFKSRFTISLDNSKNTLYLQMGSLRAEDTAVYYCARGGYYYYPRQGFLDYWGQGTTVT VSS DIVMTQSPDSLAVSLGERATISCKASQSVDYSGDSYMNWYQQKPGQPPKLLIYAASDLES GIPARFSGSGSGTDFTLTISSLEPEDFATYYCQQSHEDPFTFGQGTKLEIK |
| 476 | Anti-ADAM9 VL | DIVMTQSPDSLAVSLGERATISCKASQSVDYSGDSYMNWYQQKPGQPPKLLIYAASDLES GIPARFSGSGSGTDFTLTISSLEPEDFATYYCQQSHEDPFTFGQGTKLEIK |
| 477 | Anti-CD59 CDR-H1 | SYGMN |
| 478 | Anti-CD59 CDR-H2 | YISSSSSTIYYADSVKG |
| 479 | Anti-CD59 CDR-H3 | GPGMDV |
| 480 | Anti-CD59 CDR-L1 | KSSQSVLYSSNNKNYLA |
| 481 | Anti-CD59 CDR-L2 | WASTRES |
| 482 | Anti-CD59 CDR-L3 | QQYYSTPQLT |
| 483 | Anti-CD59 VH | QVQLQQSGGGVVQPGRSLGLSCAASGFTFSSYGMNWVRQAPGKGLEWVSYISSSSSTIYY ADSVKGRFTISRDNSKNTLYLQMNSLRAEDTAVYYCARGPGMDVWGQGTTVTVS |
| 484 | Anti-CD59 VL | DIVLTQSPDSLAVSLGERATINCKSSQSVLYSSNNKNYLAWYQQKPGQPPKLLIYWASTR ESGVPDRFSGSGSGTDFTPAISSLQAEDVAVYYCQQYYSTPQLTFGGGTKVDIK |
| 485 | Anti-CD19 (hBU12) CDR-H1 | TSGMGVG |
| 486 | Anti-CD19 (hBU12) CDR-H2 | HIWWDDDKRYNPALKS |
| 487 | Anti-CD19 (hBU12) CDR-H3 | MELWSYYFDY |
| 488 | Anti-CD19 (hBU12) CDR-L1 | SASSSVSYMH |
| 489 | Anti-CD19 (hBU12) CDR-L2 | DTSKLAS |
| 490 | Anti-CD19 (hBU12) CDR-L3 | FQGSVYPFT |
| 491 | Anti-CD19 (hBU12) VH | QVQLQESGPGLVKPSQTLSLTCTVSGGSISTSGMGVGWIRQHPGKGLEWIGHIWWDDDKR YNPALKSRVTISVDTSKNQFSLKLSSVTAADTAVYYCARMELWSYYFDYWGQGTLVTVSS |
| 492 | Anti-CD19 (hBU12) VL | EIVLTQSPATLSLSPGERATLSCSASSSVSYMHWYQQKPGQAPRLLIYDTSKLASGIPAR FSGSGSGTDFTLTISSLEPEDVAVYYCFQGSVYPFTFGQGTKLEIKR |
| 493 | Anti-CD19 (hBU12) HC | QVQLQESGPGLVKPSQTLSLTCTVSGGSISTSGMGVGWIRQHPGKGLEWIGHIWWDDDKR YNPALKSRVTISVDTSKNQFSLKLSSVTAADTAVYYCARMELWSYYFDYWGQGTLVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPGK |
| 494 | Anti-CD19 (hBU12) LC | EIVLTQSPATLSLSPGERATLSCSASSSVSYMHWYQQKPGQAPRLLIYDTSKLASGIPAR FSGSGSGTDFTLTISSLEPEDVAVYYCFQGSVYPFTFGQGTKLEIKRTVAAPSVFIFPPS DEQLKSGTASVVCLLNNFYPREAKVQWKVDNALQSGNSQESVTEQDSKDSTYSLSSTLTL SKADYEKHKVYACEVTHQGLSSPVTKSFNRGEC |
| 495 | Anti-CD138 CDR-H1 | NYWIE |
| 496 | Anti-CD138 CDR-H2 | EILPGTGRTIYNEKFKG |
| 497 | Anti-CD138 CDR-H3 | RDYYGNFYYAMDY |
| 498 | Anti-CD138 CDR-L1 | SASQGINNYLN |
| 499 | Anti-CD138 CDR-L2 | YTSTLQS |
| 500 | Anti-CD138 CDR-L3 | QQYSKLPRT |
| 501 | Anti-CD138 VH | QVQLQQSGSELMMPGASVKISCKATGYTFSNYWIEWVKQRPGHGLEWIGEILPGTGRTIY NEKFKGKATFTADISSNTVQMQLSSLTSEDSAVYYCARRDYYGNFYYAMDYWGQGTSVTV SS |
| 502 | Anti-CD138 VL | DIQMTQSTSSLSASLGDRVTISCSASQGINNYLNWYQQKPDGTVELLIYYTSTLQSGVPS RFSGSGSGTDYSLTISNLEPEDIGTYYCQQYSKLPRTFGGGTKLEIK |
| 503 | Anti-CD166 CDR-H1 | TYGMGVG |
| 504 | Anti-CD166 CDR-H2 | NIWWSEDKHYSPSLKS |
| 505 | Anti-CD166 CDR-H3 | IDYGNDYAFTY |
| 506 | Anti-CD166 CDR-L1 | RSSKSLLHSNGITYLY |
| 507 | Anti-CD166 CDR-L2 | QMSNLAS |
| 508 | Anti-CD166 CDR-L3 | AQNLELPYT |
| 509 | Anti-CD166 VH | QITLKESGPTLVKPTQTLTLTCTFSGFSLSTYGMGVGWIRQPPGKALEWLANIWWSEDKH YSPSLKSRLTITKDTSKNQVVLTITNVDPVDTATYYCVQIDYGNDYAFTYWGQGTLVTVS S |
| 510 | Anti-CD166 VL | DIVMTQSPLSLPVTPGEPASISCRSSKSLLHSNGITYLYWYLQKPGQSPQLLIYQMSNLA SGVPDRFSGSGSGTDFTLKISRVEAEDVGVYYCAQNLELPYTFGQGTKLEIK |
| 511 | Anti-CD56 CDR-H1 | SFGMH |
| 512 | Anti-CD56 CDR-H2 | YISSGSFTIYYADSVKG |
| 513 | Anti-CD56 CDR-H3 | MRKGYAMDY |
| 514 | Anti-CD56 CDR-L1 | RSSQIIIHSDGNTYLE |
| 515 | Anti-CD56 CDR-L2 | KVSNRFS |
| 516 | Anti-CD56 CDR-L3 | FQGSHVPHT |
| 517 | Anti-CD56 VH | QVQLVESGGGVVQPGRSLRLSCAASGFTFSSFGMHWVRQAPGKGLEWVAYISSGSFTIYY ADSVKGRFTISRDNSKNTLYLQMNSLRAEDTAVYYCARMRKGYAMDYWGQGTLVTVSS |
| 518 | Anti-CD56 VL | DVVMTQSPLSLPVTLGQPASISCRSSQIIIHSDGNTYLEWFQQRPGQSPRRLIYKVSNRF SGVPDRFSGSGSGTDFTLKISRVEAEDVGVYYCFQGSHVPHTFGQGTKVEIK |
| 519 | Anti-CD74 CDR-H1 | NYGVN |
| 520 | Anti-CD74 CDR-H2 | WINPNTGEPTFDDDFKG |
| 521 | Anti-CD74 CDR-H3 | SRGKNEAWFAY |
| 522 | Anti-CD74 CDR-L1 | RSSQSLVHRNGNTYLH |
| 523 | Anti-CD74 CDR-L2 | TVSNRFS |
| 524 | Anti-CD74 CDR-L3 | SQSSHVPPT |
| 525 | Anti-CD74 VH | QVQLQQSGSELKKPGASVKVSCKASGYTFTNYGVNWIKQAPGQGLQWMGWINPNTGEPTF DDDFKGRFAFSLDTSVSTAYLQISSLKADDTAVYFCSRSRGKNEAWFAYWGQGTLVTVSS |
| 526 | Anti-CD74 VL | DIQLTQSPLSLPVTLGQPASISCRSSQSLVHRNGNTYLHWFQQRPGQSPRLLIYTVSNRF SGVPDRFSGSGSGTDFTLKISRVEAEDVGVYFCSQSSHVPPTFGAGTRLEIK |
| 527 | Anti-CEACAM5 CDR-H1 | TYWMS |
| 528 | Anti-CEACAM5 CDR-H2 | EIHPDSSTINYAPSLKD |
| 529 | Anti-CEACAM5 CDR-H3 | LYFGFPWFAY |
| 530 | Anti-CEACAM5 CDR-L1 | KASQDVGTSVA |
| 531 | Anti-CEACAM5 CDR-L2 | WTSTRHT |
| 532 | Anti-CEACAM5 CDR-L3 | QQYSLYRS |
| 533 | Anti-CEACAM5 VH | EVQLVESGGGVVQPGRSLRLSCSASGFDFTTYWMSWVRQAPGKGLEWIGEIHPDSSTINY APSLKDRFTISRDNAKNTLFLQMDSLRPEDTGVYFCASLYFGFPWFAYWGQGTPVTVSS |
| 534 | Anti-CEACAM5 VL | DIQLTQSPSSLSASVGDRVTITCKASQDVGTSVAWYQQKPGKAPKLLIYWTSTRHTGVPS RFSGSGSGTDFTFTISSLQPEDIATYYCQQYSLYRSFGQGTKVEIK |
| 535 | Anti-CanAg CDR-H1 | YYGMN |
| 536 | Anti-CanAg CDR-H2 | WIDTTTGEPTYAQKFQG |
| 537 | Anti-CanAg CDR-H3 | RGPYNWYFDV |
| 538 | Anti-CanAg CDR-L1 | RSSKSLLHSNGNTYLY |
| 539 | Anti-CanAg CDR-L2 | RMSNLVS |
| 540 | Anti-CanAg CDR-L3 | LQHLEYPFT |
| 541 | Anti-CanAg VH | QVQLVQSGAEVKKPGETVKISCKASDYTFTYYGMNWVKQAPGQGLKWMGWIDTTTGEPTY AQKFQGRIAFSLETSASTAYLQIKSLKSEDTATYFCARRGPYNWYFDVWGQGTTVTVSS |
| 542 | Anti-CanAg VL | DIVMTQSPLSVPVTPGEPVSISCRSSKSLLHSNGNTYLYWFLQRPGQSPQLLIYRMSNLV SGVPDRFSGSGSGTAFTLRISRVEAEDVGVYYCLQHLEYPFTFGPGTKLELK |
| 543 | Anti-DLL-3 CDR-H1 | NYGMN |
| 544 | Anti-DLL-3 CDR-H2 | WINTYTGEPTYADDFKG |
| 545 | Anti-DLL-3 CDR-H3 | IGDSSPSDY |
| 546 | Anti-DLL-3 CDR-L1 | KASQSVSNDVV |
| 547 | Anti-DLL-3 CDR-L2 | YASNRYT |
| 548 | Anti-DLL-3 CDR-L3 | QQDYTSPWT |
| 549 | Anti-DLL-3 VH | QVQLVQSGAEVKKPGASVKVSCKASGYTFTNYGMNWVRQAPGQGLEWMGWINTYTGEPTY ADDFKGRVTMTTDTSTSTAYMELRSLRSDDTAVYYCARIGDSSPSDYWGQGTLVTVSS |
| 550 | Anti-DLL-3 VL | EIVMTQSPATLSVSPGERATLSCKASQSVSNDVVWYQQKPGQAPRLLIYYASNRYTGIPA RFSGSGSGTEFTLTISSLQSEDFAVYYCQQDYTSPWTFGQGTKLEIK |
| 551 | Anti-DPEP-3 CDR-H1 | SYWIE |
| 552 | Anti-DPEP-3 CDR-H2 | EILPGSGNTYYNERFKD |
| 553 | Anti-DPEP-3 CDR-H3 | RAAAYYSNPEWFAY |
| 554 | Anti-DPEP-3 CDR-L1 | TASSSVNSFYLH |
| 555 | Anti-DPEP-3 CDR-L2 | STSNLAS |
| 556 | Anti-DPEP-3 CDR-L3 | HQYHRSPYT |
| 557 | Anti-DPEP-3 VH | QVQLVQSGAEVKKPGSSVKVSCKASGGTFSSYWIEWVRQAPGQGLEWMGEILPGSGNTYY NERFKDRVTITADESTSTAYMELSSLRSEDTAVYYCARRAAAYYSNPEWFAYWGQGTLVT VSS |
| 558 | Anti-DPEP-3 VL | EIVLTQSPATLSLSPGERATLSCTASSSVNSFYLHWYQQKPGLAPRLLIYSTSNLASGIP DRFSGSGSGTDFTLTISRLEPEDFAVYYCHQYHRSPYTFGQGTKLEIK |
| 559 | Anti-EGFR CDR-H1 | SYWMQ |
| 560 | Anti-EGFR CDR-H2 | TIYPGDGDTTYTQKFQG |
| 561 | Anti-EGFR CDR-H3 | YDAPGYAMDY |
| 562 | Anti-EGFR CDR-L1 | RASQDINNYLA |
| 563 | Anti-EGFR CDR-L2 | YTSTLHP |
| 564 | Anti-EGFR CDR-L3 | LQYDNLLYT |
| 565 | Anti-EGFR VH | QVQLVQSGAEVAKPGASVKLSCKASGYTFTSYWMQWVKQRPGQGLECIGTIYPGDGDTTY TQKFQGKATLTADKSSSTAYMQLSSLRSEDSAVYYCARYDAPGYAMDYWGQGTLVTVSS DIQMTQSPSSLSASVGDRVTITCRASQDINNYLAWYQHKPGKGPKLLIHYTSTLHPGIPS RFSGSGSGRDYSFSISSLEPEDIATYYCLQYDNLLYTFGQGTKLEIK |
| 566 | Anti-EGFR VL | DIQMTQSPSSLSASVGDRVTITCRASQDINNYLAWYQHKPGKGPKLLIHYTSTLHPGIPS RFSGSGSGRDYSFSISSLEPEDIATYYCLQYDNLLYTFGQGTKLEIK |
| 567 | Anti-EGFR CDR-H1 | RDFAWN |
| 568 | Anti-EGFR CDR-H2 | YISYNGNTRYQPSLKS |
| 569 | Anti-EGFR CDR-H3 | ASRGFPY |
| 570 | Anti-EGFR CDR-L1 | HSSQDINSNIG |
| 571 | Anti-EGFR CDR-L2 | HGTNLDD |
| 572 | Anti-EGFR CDR-L3 | VQYAQFPWT |
| 573 | Anti-EGFR VH | EVQLQESGPGLVKPSQTLSLTCTVSGYSISRDFAWNWIRQPPGKGLEWMGYISYNGNTRY QPSLKSRITISRDTSKNQFFLKLNSVTAADTATYYCVTASRGFPYWGQGTLVTVSS |
| 574 | Anti-EGFR VL | DIQMTQSPSSMSVSVGDRVTITCHSSQDINSNIGWLQQKPGKSFKGLIYHGTNLDDGVPS RFSGSGSGTDYTLTISSLQPEDFATYYCVQYAQFPWTFGGGTKLEIK |
| 575 | Anti-EGFR CDR-H1 | NYGVH |
| 576 | Anti-EGFR CDR-H2 | VIWSGGNTDYNTPFTS |
| 577 | Anti-EGFR CDR-H3 | ALTYYDYEFAY |
| 578 | Anti-EGFR CDR-L1 | RASQSIGTNIH |
| 579 | Anti-EGFR CDR-L2 | YASESIS |
| 580 | Anti-EGFR CDR-L3 | QQNNNWPTT |
| 581 | Anti-EGFR VH | QVQLKQSGPGLVQPSQSLSITCTVSGFSLTNYGVHWVRQSPGKGLEWLGVIWSGGNTDYN TPFTSRLSINKDNSKSQVFFKMNSLQSNDTAIYYCARALTYYDYEFAYWGQGTLVTVSA |
| 582 | Anti-EGFR VL | DILLTQSPVILSVSPGERVSFSCRASQSIGTNIHWYQQRTNGSPRLLIKYASESISGIPS RFSGSGSGTDFTLSINSVESEDIADYYCQQNNNWPTTFGAGTKLELK |
| 583 | Anti-FRa CDR-H1 | GYFMN |
| 584 | Anti-FRa CDR-H2 | RIHPYDGDTFYNQKFQG |
| 585 | Anti-FRa CDR-H3 | YDGSRAMDY |
| 586 | Anti-FRa CDR-L1 | KASQSVSFAGTSLMH |
| 587 | Anti-FRa CDR-L2 | RASNLEA |
| 588 | Anti-FRa CDR-L3 | QQSREYPYT |
| 589 | Anti-FRa VH | QVQLVQSGAEVVKPGASVKISCKASGYTFTGYFMNWVKQSPGQSLEWIGRIHPYDGDTFY NQKFQGKATLTVDKSSNTAHMELLSLTSEDFAVYYCTRYDGSRAMDYWGQGTTVTVSS |
| 590 | Anti-FRa VL | DIVLTQSPLSLAVSLGQPAIISCKASQSVSFAGTSLMHWYHQKPGQQPRLLIYRASNLEA GVPDRFSGSGSKTDFTLTISPVEAEDAATYYCQQSREYPYTFGGGTKLEIK |
| 591 | Anti-FRa CDR-H1 | GYGLS |
| 592 | Anti-FRa CDR-H2 | MISSGGSYTYYADSVKG |
| 593 | Anti-FRa CDR-H3 | HGDDPAWFAY |
| 594 | Anti-FRa CDR-L1 | SVSSSISSNNLH |
| 595 | Anti-FRa CDR-L2 | GTSNLAS |
| 596 | Anti-FRa CDR-L3 | QQWSSYPYMYT |
| 597 | Anti-FRa VH | EVQLVESGGGVVQPGRSLRLSCSASGFTFSGYGLSWVRQAPGKGLEWVAMISSGGSYTYY ADSVKGRFAISRDNAKNTLFLQMDSLRPEDTGVYFCARHGDDPAWFAYWGQGTPVTVSS |
| 598 | Anti-FRa VL | DIQLTQSPSSLSASVGDRVTITCSVSSSISSNNLHWYQQKPGKAPKPWIYGTSNLASGVP SRFSGSGSGTDYTFTISSLQPEDIATYYCQQWSSYPYMYTFGQGTKVEIK |
| 599 | Anti-MUC-1 CDR-H1 | NYWMN |
| 600 | Anti-MUC-1 CDR-H2 | EIRLKSNNYTTHYAESVKG |
| 601 | Anti-MUC-1 CDR-H3 | HYYFDY |
| 602 | Anti-MUC-1 CDR-L1 | RSSKSLLHSNGITYFF |
| 603 | Anti-MUC-1 CDR-L2 | QMSNLAS |
| 604 | Anti-MUC-1 CDR-L3 | AQNLELPPT |
| 605 | Anti-MUC-1 VH | EVQLVESGGGLVQPGGSMRLSCVASGFPFSNYWMNWVRQAPGKGLEWVGEIRLKSNNYTT HYAESVKGRFTISRDDSKNSLYLQMNSLKTEDTAVYYCTRHYYFDYWGQGTLVTVSS |
| 606 | Anti-MUC-1 VL | DIVMTQSPLSNPVTPGEPASISCRSSKSLLHSNGITYFFWYLQKPGQSPQLLIYQMSNLA SGVPDRFSGSGSGTDFTLRISRVEAEDVGVYYCAQNLELPPTFGQGTKVEIK |
| 607 | Anti-Mesothelin CDR-H1 | SYWIG |
| 608 | Anti-Mesothelin CDR-H2 | IIDPGDSRTRYSPSFQG |
| 609 | Anti-Mesothelin CDR-H3 | GQLYGGTYMDG |
| 610 | Anti-Mesothelin CDR-L1 | TGTSSDIGGYNSVS |
| 611 | Anti-Mesothelin CDR-L2 | GVNNRPS |
| 612 | Anti - Mesothelin CDR-L3 | SSYDIESATPV |
| 613 | Anti-Mesothelin VH | QVELVQSGAEVKKPGESLKISCKGSGYSFTSYWIGWVRQAPGKGLEWMGIIDPGDSRTRY SPSFQGQVTISADKSISTAYLQWSSLKASDTAMYYCARGQLYGGTYMDGWGQGTLVTVSS |
| 614 | Anti-Mesothelin VL | DIALTQPASVSGSPGQSITISCTGTSSDIGGYNSVSWYQQHPGKAPKLMIYGVNNRPSGV SNRFSGSKSGNTASLTISGLQAEDEADYYCSSYDIESATPVFGGGTKLTVL |
| 615 | Anti-ROR-1 CDR-H1 | AYNIH |
| 616 | Anti-ROR-1 CDR-H2 | SFDPYDGGSSYNQKFKD |
| 617 | Anti-ROR-1 CDR-H3 | GWYYFDY |
| 618 | Anti-ROR-1 CDR-L1 | RASKSISKYLA |
| 619 | Anti-ROR-1 CDR-L2 | SGSTLQS |
| 620 | Anti-ROR-1 CDR-L3 | QQHDESPYT |
| 621 | Anti-ROR-1 VH | QVQLQESGPGLVKPSQTLSLTCTVSGYAFTAYNIHWVRQAPGQGLEWMGSFDPYDGGSSY NQKFKDRLTISKDTSKNQVVLTMTNMDPVDTATYYCARGWYYFDYWGHGTLVTVSS |
| 622 | Anti-ROR-1 VL | DIVMTQTPLSLPVTPGEPASISCRASKSISKYLAWYQQKPGQAPRLLIYSGSTLQSGIPP RFSGSGYGTDFTLTINNIESEDAAYYFCQQHDESPYTFGEGTKVEIK |
| 623 | Anti-B7-H3 CDR-H1 | SFGMH |
| 624 | Anti-B7-H3 CDR-H2 | YISSDSSAIYYADTVKG |
| 625 | Anti-B7-H3 CDR-H3 | GRENIYYGSRLDY |
| 626 | Anti-B7-H3 CDR-L1 | KASQNVDTNVA |
| 627 | Anti-B7-H3 CDR-L2 | SASYRYS |
| 628 | Anti-B7-H3 CDR-L3 | QQYNNYPFT |
| 629 | Anti-B7-H3 VH | DVQLVESGGGLVQPGGSRKLSCAASGFTFSSFGMHWVRQAPEKGLEWVAYISSDSSAIYY ADTVKGRFTISRDNPKNTLFLQMTSLRSEDTAMYYCGRGRENIYYGSRLDYWGQGTTLTV SS |
| 630 | Anti-B7-H3 VL | DIAMTQSQKFMSTSVGDRVSVTCKASQNVDTNVAWYQQKPGQSPKALIYSASYRYSGVPD RFTGSGSGTDFTLTINNVQSEDLAEYFCQQYNNYPFTFGSGTKLEIK |
| 631 | Anti-B7-H3 CDR-H1 | SYGMS |
| 632 | Anti-B7-H3 CDR-H2 | TINSGGSNTYYPDSLKG |
| 633 | Anti-B7-H3 CDR-H3 | HDGGAMDY |
| 634 | Anti-B7-H3 CDR-L1 | RASESIYSYLA |
| 635 | Anti-B7-H3 CDR-L2 | NTKTLPE |
| 636 | Anti-B7-H3 CDR-L3 | QHHYGTPPWT |
| 637 | Anti-B7-H3 VH | EVQLVESGGGLVKPGGSLRLSCAASGFTFSSYGMSWVRQAPGKGLEWVATINSGGSNTYY PDSLKGRFTISRDNAKNSLYLQMNSLRAEDTAVYYCARHDGGAMDYWGQGTTVTVSS |
| 638 | Anti-B7-H3 VL | DIQMTQSPSSLSASVGDRVTITCRASESIYSYLAWYQQKPGKAPKLLVYNTKTLPEGVPS RFSGSGSGTDFTLTISSLQPEDFATYYCQHHYGTPPWTFGQGTRLEIK |
| 639 | Anti-B7-H3 CDR-H1 | SFGMH |
| 640 | Anti-B7-H3 CDR-H2 | YISSGSGTIYYADTVKG |
| 641 | Anti-B7-H3 CDR-H3 | HGYRYEGFDY |
| 642 | Anti-B7-H3 CDR-L1 | KASQNVDTNVA |
| 643 | Anti-B7-H3 CDR-L2 | SASYRYS |
| 644 | Anti-B7-H3 CDR-L3 | QQYNNYPFT |
| 645 | Anti-B7-H3 VH | EVQLVESGGGLVQPGGSLRLSCAASGFTFSSFGMHWVRQAPGKGLEWVAYISSGSGTIYY ADTVKGRFTISRDNAKNSLYLQMNSLRAEDTAVYYCARHGYRYEGFDYWGQGTTVTVSS |
| 646 | Anti-B7-H3 VL | DIQMTQSPSFLSASVGDRVTITCKASQNVDTNVAWYQQKPGKAPKALIYSASYRYSGVPS RFSGSGSGTDFTLTISSLQPEDFAEYFCQQYNNYPFTFGQGTKLEIK |
| 647 | Anti-B7-H3 CDR-H1 | NYVMH |
| 648 | Anti-B7-H3 CDR-H2 | YINPYNDDVKYNEKFKG |
| 649 | Anti-B7-H3 CDR-H3 | WGYYGSPLYYFDY |
| 650 | Anti-B7-H3 CDR-L1 | RASSRLIYMH |
| 651 | Anti-B7-H3 CDR-L2 | ATSNLAS |
| 652 | Anti-B7-H3 CDR-L3 | QQWNSNPPT |
| 653 | Anti-B7-H3 VH | EVQLQQSGPELVKPGASVKMSCKASGYTFTNYVMHWVKQKPGQGLEWIGYINPYNDDVKY NEKFKGKATQTSDKSSSTAYMELSSLTSEDSAVYYCARWGYYGSPLYYFDYWGQGTTLTV SS |
| 654 | Anti-B7-H3 VL | QIVLSQSPTILSASPGEKVTMTCRASSRLIYMHWYQQKPGSSPKPWIYATSNLASGVPAR FSGSGSGTSYSLTISRVEAEDAATYYCQQWNSNPPTFGTGTKLELK |
| 655 | Anti-B7-H3 CDR-H1 | NYVMH |
| 656 | Anti-B7-H3 CDR-H2 | YINPYNDDVKYNEKFKG |
| 657 | Anti-B7-H3 CDR-H3 | WGYYGSPLYYFDY |
| 658 | Anti-B7-H3 CDR-L1 | RASSRLIYMH |
| 659 | Anti-B7-H3 CDR-L2 | ATSNLAS |
| 660 | Anti-B7-H3 CDR-L3 | QQWNSNPPT |
| 661 | Anti-B7-H3 VH | QVQLVQSGAEVKKPGSSVKVSCKASGYTFTNYVMHWVRQAPGQGLEWMGYINPYNDDVKY NEKFKGRVTITADESTSTAYMELSSLRSEDTAVYYCARWGYYGSPLYYFDYWGQGTLVTV SS |
| 662 | Anti-B7-H3 VL | EIVLTQSPATLSLSPGERATLSCRASSRLIYMHWYQQKPGQAPRPLIYATSNLASGIPAR FSGSGSGTDFTLTISSLEPEDFAVYYCQQWNSNPPTFGQGTKVEIK |
| 663 | Anti-B7-H3 CDR-H1 | SYTIH |
| 664 | Anti-B7-H3 CDR-H2 | YINPNSRNTDYAQKFQG |
| 665 | Anti-B7-H3 CDR-H3 | YSGSTPYWYFDV |
| 666 | Anti-B7-H3 CDR-L1 | RASSSVSYMN |
| 667 | Anti-B7-H3 CDR-L2 | ATSNLAS |
| 668 | Anti-B7-H3 CDR-L3 | QQWSSNPLT |
| 669 | Anti-B7-H3 VH | EVQLVQSGAEVKKPGSSVKVSCKASGYSFTSYTIHWVRQAPGQGLEWMGYINPNSRNTDY AQKFQGRVTLTADKSTSTAYMELSSLRSEDTAVYYCARYSGSTPYWYFDVWGQGTTVTVS S |
| 670 | Anti-B7-H3 VL | DIQLTQSPSFLSASVGDRVTITCRASSSVSYMNWYQQKPGKSPKPWIYATSNLASGVPSR FSVSVSGTEHTLTISSLQPEDFATYYCQQWSSNPLTFGQGTKLEIK |
| 671 | Anti-B7-H3 CDR-H1 | SYWMH |
| 672 | Anti-B7-H3 CDR-H2 | LIHPDSGSTNYNEMFKN |
| 673 | Anti-B7-H3 CDR-H3 | GGRLYFDY |
| 674 | Anti-B7-H3 CDR-L1 | RSSQSLVHSNGDTYLR |
| 675 | Anti-B7-H3 CDR-L2 | KVSNRFS |
| 676 | Anti-B7-H3 CDR-L3 | SQSTHVPYT |
| 677 | Anti-B7-H3 VH | EVQLVQSGAEVKKPGSSVKVSCKASGYTFSSYWMHWVRQAPGQGLEWIGLIHPDSGSTNY NEMFKNRATLTVDRSTSTAYVELSSLRSEDTAVYFCAGGGRLYFDYWGQGTTVTVSS |
| 678 | Anti-B7-H3 VL | DVVMTQSPLSLPVTPGEPASISCRSSQSLVHSNGDTYLRWYLQKPGQSPQLLIYKVSNRF SGVPDRFSGSGSGTDFTLKISRVEAEDVGVYYCSQSTHVPYTFGGGTKVEIK |
| 679 | Anti-B7-H3 CDR-H1 | SYWMH |
| 680 | Anti-B7-H3 CDR-H2 | LIHPESGSTNYNEMFKN |
| 681 | Anti-B7-H3 CDR-H3 | GGRLYFDY |
| 682 | Anti-B7-H3 CDR-L1 | RSSQSLVHSNQDTYLR |
| 683 | Anti-B7-H3 CDR-L2 | KVSNRFS |
| 684 | Anti-B7-H3 CDR-L3 | SQSTHVPYT |
| 685 | Anti-B7-H3 VH | EVQLVQSGAEVKKPGSSVKVSCKASGYTFSSYWMHWVRQAPGQGLEWIGLIHPESGSTNY NEMFKNRATLTVDRSTSTAYMELSSLRSEDTAVYYCAGGGRLYFDYWGQGTTVTVSS |
| 686 | Anti-B7-H3 VL | DIVMTQSPLSLPVTPGEPASISCRSSQSLVHSNQDTYLRWYLQKPGQSPQLLIYKVSNRF SGVPDRFSGSGSGTDFTLKISRVEAEDVGVYYCSQSTHVPYTFGGGTKVEIK |
| 687 | Anti-B7-H3 CDR-H1 | SGYSWH |
| 688 | Anti-B7-H3 CDR-H2 | YIHSSGSTNYNPSLKS |
| 689 | Anti-B7-H3 CDR-H3 | YDDYFEY |
| 690 | Anti-B7-H3 CDR-L1 | KASQNVGFNVAW |
| 691 | Anti-B7-H3 CDR-L2 | SASYRYS |
| 692 | Anti-B7-H3 CDR-L3 | QQYNWYPFT |
| 693 | Anti-B7-H3 VH | EVQLQESGPGLVKPSETLSLTCAVTGYSITSGYSWHWIRQFPGNGLEWMGYIHSSGSTNY NPSLKSRISISRDTSKNQFFLKLSSVTAADTAVYYCAGYDDYFEYWGQGTTVTVSS |
| 694 | Anti-B7-H3 VL | DIQMTQSPSSLSASVGDRVTITCKASQNVGFNVAWYQQKPGKSPKALIYSASYRYSGVPS RFSGSGSGTDFTLTISSLQPEDFAEYFCQQYNWYPFTFGQGTKLEIK |
| 695 | Anti-B7-H3 CDR-H1 | NYDIN |
| 696 | Anti-B7-H3 CDR-H2 | WIFPGDDSTQYNEKFKG |
| 697 | Anti-B7-H3 CDR-H3 | QTTGTWFAY |
| 698 | Anti-B7-H3 CDR-L1 | RASQSISDYLY |
| 699 | Anti-B7-H3 CDR-L2 | YASQSIS |
| 700 | Anti-B7-H3 CDR-L3 | QNGHSFPLT |
| 701 | Anti-B7-H3 VH | QVQLVQSGAEVVKPGASVKLSCKTSGYTFTNYDINWVRQRPGQGLEWIGWIFPGDDSTQY NEKFKGKATLTTDTSTSTAYMELSSLRSEDTAVYFCARQTTGTWFAYWGQGTLVTVSS |
| 702 | Anti-B7-H3 VL | EIVMTQSPATLSVSPGERVTLSCRASQSISDYLYWYQQKSHESPRLLIKYASQSISGIPA RFSGSGSGSEFTLTINSVEPEDVGVYYCQNGHSFPLTFGQGTKLELK |
| 703 | Anti-B7-H3 VH | QVQLQQSGAEVKKPGSSVKVSCKASGGTFSSYAISWVRQAPGQGLEWMGGIIPILGIANY AQKFQGRVTITADESTSTAYMELSSLRSEDTAVYYCARGGSGSYHMDVWGKGTTVTVSS |
| 704 | Anti-B7-H3 VL | EIVLTQSPATLSLSPGERATLSCRASQSVSSYLAWYQQKPGQAPRLLIYDASNRATGIPA RFSGSGSGTDFTLTISSLEPEDFAVYYCQQRSNWPPRITFGQGTRLEIK |
| 705 | Anti-B7-H3 CDR-H1 | IYNVH |
| 706 | Anti-B7-H3 CDR-H2 | TIFPGNGDTSYNQKFKD |
| 707 | Anti-B7-H3 CDR-H3 | WDDGNVGFAH |
| 708 | Anti-B7-H3 CDR-L1 | RASENINNYLT |
| 709 | Anti-B7-H3 CDR-L2 | HAKTLAE |
| 710 | Anti-B7-H3 CDR-L3 | QHHYGTPPT |
| 711 | Anti-B7-H3 VH | QVQLQQPGAELVKPGASVKMSCKASGYTFTIYNVHWIKQTPGQGLEWMGTIFPGNGDTSY NQKFKDKATLTTDKSSKTAYMQLNSLTSEDSAVYYCARWDDGNVGFAHWGQGTLVTVSA |
| 712 | Anti-B7-H3 VL | DIQMTQSPASLSASVGETVTITCRASENINNYLTWFQQKQGKSPQLLVYHAKTLAEGVPS RFSGSGSGTQFSLKINSLQPEDFGSYYCQHHYGTPPTFGGGTKLEIK |
| 713 | Anti-B7-H3 VH | EVQLVQSGAEVKKPGASVKVSCKASGYTFTIYNVHWVRQAPGQGLEWMGTIFPGNGDTSY NQKFKDKVTMTTDTSTSTAYMELSSLRSEDTAVYYCARWDDGNVGFAHWGQGTLVTVSS |
| 714 | Anti-B7-H3 VL | DIQMTQSPSSLSASVGDRVTITCRASENINNYLTWFQQKQGKSPQLLIYHAKTLAEGVPS RFSGSGSGTDFTLTISSLQPEDFATYYCQHHYGTPPTFGGGTKVEIK |
| 715 | Anti-B7-H3 VH | EVQLVQSGAEVKKPGASVKVSCKASGYTFTIYNVHWIRQAPGQGLEWMGTIFPGNGDTSY NQKFKDRATLTTDKSTKTAYMELRSLRSDDTAVYYCARWDDGNVGFAHWGQGTLVTVSS |
| 716 | Anti-B7-H3 VL | DIQMTQSPSSLSASVGDRVTITCRASENINNYLTWFQQKPGKAPKLLVYHAKTLAEGVPS RFSGSGSGTQFTLTISSLQPEDFATYYCQHHYGTPPTFGQGTKLEIK |
| 717 | Anti-HER3 H | QVQLQQWGAGLLKPSETLSLTCAVYGGSFSGYYWSWIRQPPGKGLEWIGEINHSGSTNYN PSLKSRVTISVETSKNQFSLKLSSVTAADTAVYYCARDKWTWYFDLWGRGTLVTVSSAST KGPSVFPLAPSSKSTSGGTAALGCLVKDYFPEPVTVSWNSGALTSGVHTFPAVLQSSGLY SLSSVVTVPSSSLGTQTYICNVNHKPSNTKVDKRVEPKSCDKTHTCPPCPAPELLGGPSV FLFPPKPKDTLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPPSREEMTK NQVSLTCLVKGFYPSDIAVEWESNGQPENNYKTTPPVLDSDGSFFLYSKLTVDKSRWQQG NVFSCSVMHEALHNHYTQKSLSLSPGK |
| 718 | Anti-HER3 L | DIEMTQSPDSLAVSLGERATINCRSSQSVLYSSSNRNYLAWYQQNPGQPPKLLIYWASTR ESGVPDRFSGSGSGTDFTLTISSLQAEDVAVYYCQQYYSTPRTFGQGTKVEIKRTVAAPS VFIFPPSDEQLKSGTASVVCLLNNFYPREAKVQWKVDNALQSGNSQESVTEQDSKDSTYS LSSTLTLSKADYEKHKVYACEVTHQGLSSPVTKSFNRGEC |
| 719 | Anti-HER3 H | EVQLLESGGGLVQPGGSLRLSCAASGFTFSHYVMAWVRQAPGKGLEWVSSISSSGGWTLY ADSVKGRFTISRDNSKNTLYLQMNSLRAEDTAVYYCTRGLKMATIFDYWGQGTLVTVSSA STKGPSVFPLAPCSRSTSESTAALGCLVKDYFPEPVTVSWNSGALTSGVHTFPAVLQSSG LYSLSSVVTVPSSNFGTQTYTCNVDHKPSNTKVDKTVERKCCVECPPCPAPPVAGPSVFL FPPKPKDTLMISRTPEVTCVVVDVSHEDPEVQFNWYVDGVEVHNAKTKPREEQFNSTFRV VSVLTVVHQDWLNGKEYKCKVSNKGLPAPIEKTISKTKGQPREPQVYTLPPSREEMTKNQ VSLTCLVKGFYPSDIAVEWESNGQPENNYKTTPPMLDSDGSFFLYSKLTVDKSRWQQGNV FSCSVMHEALHNHYTQKSLSLSPGK |
| 720 | Anti-HER3 L | QSALTQPASVSGSPGQSITISCTGTSSDVGSYNVVSWYQQHPGKAPKLIIYEVSQRPSGV SNRFSGSKSGNTASLTISGLQTEDEADYYCCSYAGSSIFVIFGGGTKVTVLGQPKAAPSV TLFPPSSEELQANKATLVCLVSDFYPGAVTVAWKADGSPVKVGVETTKPSKQSNNKYAAS SYLSLTPEQWKSHRSYSCRVTHEGSTVEKTVAPAECS |
| 721 | Anti-HER3 H | EVQLLESGGGLVQPGGSLRLSCAASGFTFSSYAMSWVRQAPGKGLEWVSAINSQGKSTYY ADSVKGRFTISRDNSKNTLYLQMNSLRAEDTAVYYCARWGDEGFDIWGQGTLVTVSSAST KGPSVFPLAPSSKSTSGGTAALGCLVKDYFPEPVTVSWNSGALTSGVHTFPAVLQSSGLY SLSSVVTVPSSSLGTQTYICNVNHKPSNTKVDKRVEPKSCDKTHTCPPCPAPELLGGPSV FLFPPKPKDTLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPPSREEMTK NQVSLTCLVKGFYPSDIAVEWESNGQPENNYKTTPPVLDSDGSFFLYSKLTVDKSRWQQG NVFSCSVMHEALHNHYTQKSLSLSPGK |
| 722 | Anti-HER3 L | DIQMTQSPSSLSASVGDRVTITCRASQGISNWLAWYQQKPGKAPKLLIYGASSLQSGVPS RFSGSGSGTDFTLTISSLQPEDFATYYCQQYSSFPTTFGQGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC |
| 723 | Anti-HER3 H | QVQLVQSGAEVKKPGASVKVSCKASGYTFRSSYISWVRQAPGQGLEWMGWIYAGTGSPSY NQKLQGRVTMTTDTSTSTAYMELRSLRSDDTAVYYCARHRDYYSNSLTYWGQGTLVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPG |
| 724 | Anti-HER3 L | DIVMTQSPDSLAVSLGERATINCKSSQSVLNSGNQKNYLTWYQQKPGQPPKLLIYWASTR ESGVPDRFSGSGSGTDFTLTISSLQAEDVAVYYCQSDYSYPYTFGQGTKLEIKRTVAAPS VFIFPPSDEQLKSGTASVVCLLNNFYPREAKVQWKVDNALQSGNSQESVTEQDSKDSTYS LSSTLTLSKADYEKHKVYACEVTHQGLSSPVTKSFNRGEC |
| 725 | Anti-PTK7 CDR-H1 | TSNMGVG |
| 726 | Anti-PTK7 CDR-H2 | HIWWDDDKYYSPSLKS |
| 727 | Anti-PTK7 CDR-H3 | SNYGYAWFAY |
| 728 | Anti-PTK7 CDR-L1 | KASQDIYPYLN |
| 729 | Anti-PTK7 CDR-L2 | RTNRLLD |
| 730 | Anti-PTK7 CDR-L3 | LQYDEFPLT |
| 731 | Anti-PTK7 VH | QITLKESGPTLVKPTQTLTLTCTFSGFSLSTSNMGVGWIRQPPGKALEWLAHIWWDDDKY YSPSLKSRLTITKDTSKNQVVLTMTNMDPVDTATYYCVRSNYGYAWFAYWGQGTLVTVSS DIQMTQSPSSLSASVGDRVTITCKASQDIYPYLNWFQQKPGKAPKTLIYRTNRLLDGVPS RFSGSGSGTDFTFTISSLQPEDIATYYCLQYDEFPLTFGAGTKLEIK |
| 732 | Anti-PTK7 VL | DIQMTQSPSSLSASVGDRVTITCKASQDIYPYLNWFQQKPGKAPKTLIYRTNRLLDGVPS RFSGSGSGTDFTFTISSLQPEDIATYYCLQYDEFPLTFGAGTKLEIK |
| 733 | Anti-PTK7 CDR-H1 | DYAVH |
| 734 | Anti-PTK7 CDR-H2 | VISTYNDYTYNNQDFKG |
| 735 | Anti-PTK7 CDR-H3 | GNSYFYALDY |
| 736 | Anti-PTK7 CDR-L1 | RASESVDSYGKSFMH |
| 737 | Anti-PTK7 CDR-L2 | RASNLES |
| 738 | Anti-PTK7 CDR-L3 | QQSNEDPWT |
| 739 | Anti-PTK7 VH | QVQLVQSGPEVKKPGASVKVSCKASGYTFTDYAVHWVRQAPGKRLEWIGVISTYNDYTYN NQDFKGRVTMTRDTSASTAYMELSRLRSEDTAVYYCARGNSYFYALDYWGQGTSVTVSS |
| 740 | Anti-PTK7 VL | EIVLTQSPATLSLSPGERATLSCRASESVDSYGKSFMHWYQQKPGQAPRLLIYRASNLES GIPARFSGSGSGTDFTLTISSLEPEDFAVYYCQQSNEDPWTFGGGTKLEIK |
| 741 | Anti-PTK7 CDR-H1 | RYWMS |
| 742 | Anti-PTK7 CDR-H2 | DLNPDSSAINYVDSVKG |
| 743 | Anti-PTK7 CDR-H3 | ITTLVPYTMDF |
| 744 | Anti-PTK7 CDR-L1 | ITNTDIDDDMN |
| 745 | Anti-PTK7 CDR-L2 | EGNGLRP |
| 746 | Anti-PTK7 CDR-L3 | LQSDNLPLT |
| 747 | Anti-PTK7 VH | EVQLVESGGGLVQPGGSLRLSCAASGFDFSRYWMSWVRQAPGKGLEWIGDLNPDSSAINY VDSVKGRFTISRDNAKNSLYLQMNSLRAEDTAVYYCTLITTLVPYTMDFWGQGTSVTVSS |
| 748 | Anti-PTK7 VL | ETTLTQSPAFMSATPGDKVNISCITNTDIDDDMNWYQQKPGEAAILLISEGNGLRPGIPP RFSGSGYGTDFTLTINNIESEDAAYYFCLQSDNLPLTFGSGTKLEIK |
| 749 | Anti-ZIP6 CDR-H1 | DYYMH |
| 750 | Anti-ZIP6 CDR-H2 | WIDPENGDTEYGPKFQG |
| 751 | Anti-ZIP6 CDR-H3 | HNAHYGTWFAY |
| 752 | Anti-ZIP6 CDR-L1 | RSSQSLLHSSGNTYLE |
| 753 | Anti-ZIP6 CDR-L2 | KISTRFS |
| 754 | Anti-ZIP6 CDR-L3 | FQGSHVPYT |
| 755 | Anti-ZIP6 VH | QVQLVQSGAEVKKPGASVKVSCKASGLTIEDYYMHWVRQAPGQGLEWMGWIDPENGDTEY GPKFQGRVTMTRDTSINTAYMELSRLRSDDTAVYYCAVHNAHYGTWFAYWGQGTLVTVSS |
| 756 | Anti-ZIP6 VL | DVVMTQSPLSLPVTLGQPASISCRSSQSLLHSSGNTYLEWYQQRPGQSPRPLIYKISTRF SGVPDRFSGSGSGTDFTLKISRVEAEDVGVYYCFQGSHVPYTFGGGTKVEIK |
| 757 | Anti-ZIP6 HC | QVQLVQSGAEVKKPGASVKVSCKASGLTIEDYYMHWVRQAPGQGLEWMGWIDPENGDTEY GPKFQGRVTMTRDTSINTAYMELSRLRSDDTAVYYCAVHNAHYGTWFAYWGQGTLVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRW QQGNVFSCSVMHEALHNHYTQKSLSLSPG |
| 758 | Anti-ZIP6 LC | DVVMTQSPLSLPVTLGQPASISCRSSQSLLHSSGNTYLEWYQQRPGQSPRPLIYKISTRF SGVPDRFSGSGSGTDFTLKISRVEAEDVGVYYCFQGSHVPYTFGGGTKVEIKRTVAAPSV FIFPPSDEQLKSGTASVVCLLNNFYPREAKVQWKVDNALQSGNSQESVTEQDSKDSTYSL SSTLTLSKADYEKHKVYACEVTHQGLSSPVTKSFNRGEC |
| 759 | Anti-Integrin αvβ6 CDR-H1 | GYFMN |
| 760 | Anti-Integrin αvβ6 CDR-H2 | LINPYNGDSFYNQKFKG |
| 761 | Anti-Integrin αvβ6 CDR-H3 | GLRRDFDY |
| 762 | Anti-Integrin αvβ6 CDR-L1 | KSSQSLLDSDGKTYLN |
| 763 | Anti-Integrin αvβ6 CDR-L2 | LVSELDS |
| 764 | Anti-Integrin αvβ6 CDR-L3 | WQGTHFPRT |
| 765 | Anti-Integrin αvβ6 V H | QVQLVQSGAEVKKPGASVKVSCKASGYSFSGYFMNWVRQAPGQGLEWMGLINPYNGDSFY NQKFKGRVTMTRQTSTSTVYMELSSLRSEDTAVYYCVRGLRRDFDYWGQGTLVTVSS |
| 766 | Anti-Integrin αvβ6 VL | DVVMTQSPLSLPVTLGQPASISCKSSQSLLDSDGKTYLNWLFQRPGQSPRRLIYLVSELD SGVPDRFSGSGSGTDFTLKISRVEAEDVGVYYCWQGTHFPRTFGGGTKLEIK |
| 767 | Anti-CD48 CDR-H1 | DFGMN |
| 768 | Anti-CD48 CDR-H2 | WINTFTGEPSYGNVFKG |
| 769 | Anti-CD48 CDR-H3 | RHGNGNVFDS |
| 770 | Anti-CD48 CDR-L1 | RASQSIGSNIH |
| 771 | Anti-CD48 CDR-L2 | YTSESIS |
| 772 | Anti-CD48 CDR-L3 | QQSNSWPLT |
| 773 | Anti-CD48 VH | QVQLVQSGSELKKPGASVKVSCKASGYTFTDFGMNWVRQAPGQGLEWMGWINTFTGEPSY GNVFKGRFVFSLDTSVSTAYLQISSLKAEDTAVYYCARRHGNGNVFDSWGQGTLVTVSS |
| 774 | Anti-CD48 VL | EIVLTQSPDFQSVTPKEKVTITCRASQSIGSNIHWYQQKPDQSPKLLIKYTSESISGVPS RFSGSGSGTDFTLTINSLEAEDAATYYCQQSNSWPLTFGGGTKVEIKR |
| 775 | Anti-IGF-1R CDR-H1 | SYAIS |
| 776 | Anti-IGF-1R CDR-H2 | GIIPIFGTANYAQKFQG |
| 777 | Anti-IGF-1R CDR-H3 | APLRFLEWSTQDHYYYYYMDV |
| 778 | Anti-IGF-1R CDR-L1 | QGDSLRSYYAT |
| 779 | Anti-IGF-1R CDR-L2 | GENKRPS |
| 780 | Anti-IGF-1R CDR-L3 | KSRDGSGQHLV |
| 781 | Anti-IGF-1R VH | EVQLVQSGAEVKKPGSSVKVSCKASGGTFSSYAISWVRQAPGQGLEWMGGIIPIFGTANY AQKFQGRVTITADKSTSTAYMELSSLRSEDTAVYYCARAPLRFLEWSTQDHYYYYYMDVW GKGTTVTVSS |
| 782 | Anti-IGF-1R VL | SSELTQDPAVSVALGQTVRITCQGDSLRSYYATWYQQKPGQAPILVIYGENKRPSGIPDR FSGSSSGNTASLTITGAQAEDEADYYCKSRDGSGQHLVFGGGTKLTVL |
| 783 | Anti-Claudin-18.2 CDR-H1 | SYWIN |
| 784 | Anti-Claudin-18.2 CDR-H2 | NIYPSDSYTNYNQKFKD |
| 785 | Anti-Claudin-18.2 CDR-H3 | SWRGNSFDY |
| 786 | Anti-Claudin-18.2 CDR-L1 | KSSQSLLNSGNQKNYLT |
| 787 | Anti-Claudin-18.2 CDR-L2 | WASTRES |
| 788 | Anti-Claudin-18.2 CDR-L3 | QNDYSYPFT |
| 789 | Anti-Claudin-18.2 VH | QVQLQQPGAELVRPGASVKLSCKASGYTFTSYWINWVKQRPGQGLEWIGNIYPSDSYTNY NQKFKDKATLTVDKSSSTAYMQLSSPTSEDSAVYYCTRSWRGNSFDYWGQGTTLTVSS |
| 790 | Anti-Claudin-18.2 VL | DIVMTQSPSSLTVTAGEKVTMSCKSSQSLLNSGNQKNYLTWYQQKPGQPPKLLIYWASTR ESGVPDRFTGSGSGTDFTLTISSVQAEDLAVYYCQNDYSYPFTFGSGTKLEIK |
| 791 | Anti-Claudin-18.2 CDR-H1 | NYGMN |
| 792 | Anti-Claudin-18.2 CDR-H2 | WINTNTGEPTYAEEFKG |
| 793 | Anti-Claudin-18.2 CDR-H3 | LGFGNAMDY |
| 794 | Anti-Claudin-18.2 CDR-L1 | KSSQSLLNSGNQKNYLT |
| 795 | Anti-Claudin-18.2 CDR-L2 | WASTRES |
| 796 | Anti-Claudin-18.2 CDR-L3 | QNDYSYPLT |
| 797 | Anti- | QIQLVQSGPELKKPGETVKISCKASGYTFTNYGMNWVKQAPGKGLKWMGWINTNTGEPTY |
| | Claudin-18.2 VH | AEEFKGRFAFSLETSASTAYLQINNLKNEDTATYFCARLGFGNAMDYWGQGTSVTVSS |
| 798 | Anti-Claudin-18.2 VL | DIVMTQSPSSLTVTAGEKVTMSCKSSQSLLNSGNQKNYLTWYQQKPGQPPKLLIYWASTR ESGVPDRFTGSGSGTDFTLTISSVQAEDLAVYYCQNDYSYPLTFGAGTKLELK |
| 799 | Anti-Nectin-4 CDR-H1 | SYNMN |
| 800 | Anti-Nectin-4 CDR-H2 | YISSSSSTIYYADSVKG |
| 801 | Anti-Nectin-4 CDR-H3 | AYYYGMDV |
| 802 | Anti-Nectin-4 CDR-L1 | RASQGISGWLA |
| 803 | Anti-Nectin-4 CDR-L2 | AASTLQS |
| 804 | Anti-Nectin-4 CDR-L3 | QQANSFPPT |
| 805 | Anti-Nectin-4 VH | EVQLVESGGGLVQPGGSLRLSCAASGFTFSSYNMNWVRQAPGKGLEWVSYISSSSSTIYY ADSVKGRFTISRDNAKNSLSLQMNSLRDEDTAVYYCARAYYYGMDVWGQGTTVTVSS |
| 806 | Anti-Nectin-4 VL | DIQMTQSPSSVSASVGDRVTITCRASQGISGWLAWYQQKPGKAPKFLIYAASTLQSGVPS RFSGSGSGTDFTLTISSLQPEDFATYYCQQANSFPPTFGGGTKVEIK |
| 807 | Anti-SLTRK6 CDR-H1 | SYGMH |
| 808 | Anti-SLTRK6 CDR-H2 | VIWYDGSNQYYADSVKG |
| 809 | Anti-SLTRK6 CDR-H3 | GLTSGRYGMDV |
| 810 | Anti-SLTRK6 CDR-L1 | RSSQSLLLSHGFNYLD |
| 811 | Anti-SLTRK6 CDR-L2 | LGSSRAS |
| 812 | Anti-SLTRK6 CDR-L3 | MQPLQIPWT |
| 813 | Anti-SLTRK6 VH | QVQLVESGGGVVQPGRSLRLSCAASGFTFSSYGMHWVRQAPGKGLEWVAVIWYDGSNQYY ADSVKGRFTISRDNSKNTLFLQMHSLRAEDTAVYYCARGLTSGRYGMDVWGQGTTVTVSS |
| 814 | Anti-SLTRK6 VL | DIVMTQSPLSLPVTPGEPASISCRSSQSLLLSHGFNYLDWYLQKPGQSPQLLIYLGSSRA SGVPDRFSGSGSGTDFTLKISRVEAEDVGLYYCMQPLQIPWTFGQGTKVEIK |
| 815 | Anti-CD142 (TF) CDR-H1 | NYAMS |
| 816 | Anti-CD142 (TF) CDR-H2 | SISGSGDYTYYTDSVKG |
| 817 | Anti-CD142 (TF) CDR-H3 | SPWGYYLDS |
| 818 | Anti-CD142 (TF) CDR-L1 | RASQGISSRLA |
| 819 | Anti-CD142 (TF) CDR-L2 | AASSLQS |
| 820 | Anti-CD142 (TF) CDR-L3 | QQYNSYPYT |
| 821 | Anti-CD142 (TF) VH | EVQLLESGGGLVQPGGSLRLSCAASGFTFSNYAMSWVRQAPGKGLEWVSSISGSGDYTYY TDSVKGRFTISRDNSKNTLYLQMNSLRAEDTAVYYCARSPWGYYLDSWGQGTLVTVSS |
| 822 | Anti-CD142 (TF) VL | DIQMTQSPPSLSASAGDRVTITCRASQGISSRLAWYQQKPEKAPKSLIYAASSLQSGVPS RFSGSGSGTDFTLTISSLQPEDFATYYCQQYNSYPYTFGQGTKLEIK |
| 823 | Anti-h2G12/STn CDR-H1 | DHAIH |
| 824 | Anti-STn CDR-H2 | YFSPGNDDIKYNEKFRG |
| 825 | Anti-STn CDR-H3 | SLSTPY |
| 826 | Anti-STn CDR-L1 | KSSQSLLNRGNHKNYLT |
| 827 | Anti-STn CDR-L2 | WASTRES |
| 828 | Anti-STn CDR-L3 | QNDYTYPYT |
| 829 | Anti-STn VH | EVQLVQSGAEVKKPGASVKVSCKASGYTFTDHAIHWVRQAPGQGLEWMGYFSPGNDDIKY NEKFRGRVTMTADKSSSTAYMELRSLRSDDTAVYFCKRSLSTPYWGQGTLVTVSS |
| 830 | Anti-STn VL | DIVMTQSPDSLAVSLGERATINCKSSQSLLNRGNHKNYLTWYQQKPGQPPKLLIYWASTR ESGVPDRFSGSGSGTDFTLTISSLQAEDVAVYYCQNDYTYPYTFGQGTKVEIK |
| 831 | Anti-CD20 CDR-H1 | SYNMH |
| 832 | Anti-CD20 CDR-H2 | AIYPGNGDTSYNQKFKG |
| 833 | Anti-CD20 CDR-H3 | STYYGGDWYFNV |
| 834 | Anti-CD20 CDR-L1 | RASSSVSYIH |
| 835 | Anti-CD20 CDR-L2 | ATSNLAS |
| 836 | Anti-CD20 CDR-L3 | QQWTSNPPT |
| 837 | Anti-CD20 VH | |
| 838 | Anti-CD20 VL | |
| 839 | Anti-HER2 CDR-H1 | DTYIH |
| 840 | Anti-HER2 CDR-H2 | RIYPTNGYTRYADSVKG |
| 841 | Anti-HER2 CDR-H3 | WGGDGFYAMDY |
| 842 | Anti-HER2 CDR-L1 | RASQDVNTAVA |
| 843 | Anti-HER2 CDR-L2 | SASFLYS |
| 844 | Anti-HER2 CDR-L3 | QQHYTTPPT |
| 845 | Anti-HER2 VH | |
| 846 | Anti-HER2 VL | |
| 847 | Anti-CD79b CDR-H1 | SYWIE |
| 848 | Anti-CD79b CDR-H2 | EILPGGGDTNYNEIFKG |
| 849 | Anti-CD79b CDR-H3 | RVPIRLDY |
| 850 | Anti-CD79b CDR-L1 | KASQSVDYEGDSFLN |
| 851 | Anti-CD79b CDR-L2 | AASNLES |
| 852 | Anti-CD79b CDR-L3 | QQSNEDPLT |
| 853 | Anti-CD79b VH | |
| 854 | Anti-CD79b VL | |
| 855 | Anti-NaPi2B CDR-H1 | DFAMS |
| 856 | Anti-NaPi2B CDR-H2 | TIGRVAFHTYYPDSMKG |
| 857 | Anti-NaPi2B CDR-H3 | HRGFDVGHFDF |
| 858 | Anti-NaPi2B CDR-L1 | RSSETLVHSSGNTYLE |
| 859 | Anti-NaPi2B CDR-L2 | RVSNRFS |
| 860 | Anti-NaPi2B CDR-L3 | FQGSFNPLT |
| 861 | Anti-NaPi2B VH | EVQLVESGGGLVQPGGSLRLSCAASGFSFSDFAMSWVRQAPGKGLEWVATIGRVAFHTYY PDSMKGRFTISRDNSKNTLYLQMNSLRAEDTAVYYCARHRGFDVGHFDFWGQGTLVTVSS |
| 862 | Anti-NaPi2B VL | DIQMTQSPSSLSASVGDRVTITCRSSETLVHSSGNTYLEWYQQKPGKAPKLLIYRVSNRF SGVPSRFSGSGSGTDFTLTISSLQPEDFATYYCFQGSFNPLTFGQGTKVEIK |
| 863 | Anti-Muc16 CDR-H1 | NDYAWN |
| 864 | Anti-Muc16 CDR-H2 | YISYSGYTTYNPSLKS |
| 865 | Anti-Muc16 CDR-H3 | WTSGLDY |
| 866 | Anti-Muc16 CDR-L1 | KASDLIHNWLA |
| 867 | Anti-Muc16 CDR-L2 | GATSLET |
| 868 | Anti-Muc16 CDR-L3 | QQYWTTPFT |
| 869 | Anti-Muc16 VH | EVQLVESGGGLVQPGGSLRLSCAASGYSITNDYAWNWVRQAPGKGLEWVGYISYSGYTTY NPSLKSRFTISRDTSKNTLYLQMNSLRAEDTAVYYCARWTSGLDYWGQGTLVTVSS |
| 870 | Anti-Muc16 VL | DIQMTQSPSSLSASVGDRVTITCKASDLIHNWLAWYQQKPGKAPKLLIYGATSLETGVPS RFSGSGSGTDFTLTISSLQPEDFATYYCQQYWTTPFTFGQGTKVEIK |
| 871 | Anti-STEAP1 CDR-H1 | SDYAWN |
| 872 | Anti-STEAP1 CDR-H2 | YISNSGSTSYNPSLKS |
| 873 | Anti-STEAP1 CDR-H3 | ERNYDYDDYYYAMDY |
| 874 | Anti-STEAP1 CDR-L1 | KSSQSLLYRSNQKNYLA |
| 875 | Anti-STEAP1 CDR-L2 | WASTRES |
| 876 | Anti-STEAP1 CDR-L3 | QQYYNYPRT |
| 877 | Anti-STEAP1 VH | EVQLVESGGGLVQPGGSLRLSCAVSGYSITSDYAWNWVRQAPGKGLEWVGYISNSGSTSY NPSLKSRFTISRDTSKNTLYLQMNSLRAEDTAVYYCARERNYDYDDYYYAMDYWGQGTLV TVSS |
| 878 | Anti-STEAP1 VL | DIQMTQSPSSLSASVGDRVTITCKSSQSLLYRSNQKNYLAWYQQKPGKAPKLLIYWASTR ESGVPSRFSGSGSGTDFTLTISSLQPEDFATYYCQQYYNYPRTFGQGTKVEIK |
| 879 | Anti-BCMA CDR-H1 | NYWMH |
| 880 | Anti-BCMA CDR-H2 | ATYRGHSDTYYNQKFKG |
| 881 | Anti-BCMA CDR-H3 | GAIYDGYDVLDN |
| 882 | Anti-BCMA CDR-L1 | SASQDISNYLN |
| 883 | Anti-BCMA CDR-L2 | YTSNLHS |
| 884 | Anti-BCMA CDR-L3 | QQYRKLPWT |
| 885 | Anti-BCMA VH | QVQLVQSGAEVKKPGSSVKVSCKASGGTFSNYWMHWVRQAPGQGLEWMGATYRGHSDTYY NQKFKGRVTITADKSTSTAYMELSSLRSEDTAVYYCARGAIYDGYDVLDNWGQGTLVTVS S |
| 886 | Anti-BCMA VL | DIQMTQSPSSLSASVGDRVTITCSASQDISNYLNWYQQKPGKAPKLLIYYTSNLHSGVPS RFSGSGSGTDFTLTISSLQPEDFATYYCQQYRKLPWTFGQGTKLEIK |
| 887 | Anti-c-Met CDR-H1 | AYTMH |
| 888 | Anti-c-Met CDR-H2 | WIKPNNGLANYAQKFQG |
| 889 | Anti-c-Met CDR-H3 | SEITTEFDY |
| 890 | Anti-c-Met CDR-L1 | KSSESVDSYANSFLH |
| 891 | Anti-c-Met CDR-L2 | RASTRES |
| 892 | Anti-c-Met CDR-L3 | QQSKEDPLT |
| 893 | Anti-c-Met VH | QVQLVQSGAEVKKPGASVKVSCKASGYIFTAYTMHWVRQAPGQGLEWMGWIKPNNGLANY AQKFQGRVTMTRDTSISTAYMELSRLRSDDTAVYYCARSEITTEFDYWGQGTLVTVSS |
| 894 | Anti-c-Met VL | DIVMTQSPDSLAVSLGERATINCKSSESVDSYANSFLHWYQQKPGQPPKLLIYRASTRES GVPDRFSGSGSGTDFTLTISSLQAEDVAVYYCQQSKEDPLTFGGGTKVEIK |
| 895 | Anti-EGFR CDR-H1 | SDFAWN |
| 896 | Anti-EGFR CDR-H2 | YISYSGNTRYQPSLKS |
| 897 | Anti-EGFR CDR-H3 | AGRGFPY |
| 898 | Anti-EGFR CDR-L1 | HSSQDINSNIG |
| 899 | Anti-EGFR CDR-L2 | HGTNLDD |
| 900 | Anti-EGFR CDR-L3 | VQYAQFPWT |
| 901 | Anti-EGFR VH | QVQLQESGPGLVKPSQTLSLTCTVSGYSISSDFAWNWIRQPPGKGLEWMGYISYSGNTRY QPSLKSRITISRDTSKNQFFLKLNSVTAADTATYYCVTAGRGFPYWGQGTLVTVSS |
| 902 | Anti-EGFR VL | DIQMTQSPSSMSVSVGDRVTITCHSSQDINSNIGWLQQKPGKSFKGLIYHGTNLDDGVPS RFSGSGSGTDYTLTISSLQPEDFATYYCVQYAQFPWTFGGGTKLEIK |
| 903 | Anti-SLAMF7 CDR-H1 | DYYMA |
| 904 | Anti-SLAMF7 CDR-H2 | SINYDGSSTYYVDSVKG |
| 905 | Anti-SLAMF7 CDR-H3 | DRGYYFDY |
| 906 | Anti-SLAMF7 CDR-L1 | RSSQSLVHSNGNTYLH |
| 907 | Anti-SLAMF7 CDR-L2 | KVSNRFS |
| 908 | Anti-SLAMF7 CDR-L3 | SQSTHVPPFT |
| 909 | Anti-SLAMF7 VH | EVQLVESGGGLVQPGGSLRLSCAASGFTFSDYYMAWVRQAPGKGLEWVASINYDGSSTYY VDSVKGRFTISRDNAKNSLYLQMNSLRAEDTAVYYCARDRGYYFDYWGQGTTVTVSS |
| 910 | Anti-SLAMF7 VL | DVVMTQTPLSLSVTPGQPASISCRSSQSLVHSNGNTYLHWYLQKPGQSPQLLIYKVSNRF SGVPDRFSGSGSGTDFTLKISRVEAEDVGVYFCSQSTHVPPFTFGGGTKVEIK |
| 911 | Anti-C4.4a CDR-H1 | NAWMS |
| 912 | Anti-C4.4a CDR-H2 | YISSSGSTIYYADSVKG |
| 913 | Anti-C4.4a CDR-H3 | EGLWAFDY |
| 914 | Anti-C4.4a CDR-L1 | TGSSSNIGAGYVVH |
| 915 | Anti-C4.4a CDR-L2 | DNNKRPS |
| 916 | Anti-C4.4a CDR-L3 | AAWDDRLNGPV |
| 917 | Anti-C4.4a VH | EVQLLESGGGLVQPGGSLRLSCAASGFTFSNAWMSWVRQAPGKGLEWVSYISSSGSTIYY ADSVKGRFTISRDNSKNTLYLQMNSLRAEDTAVYYCAREGLWAFDYWGQGTLVTVSS |
| 918 | Anti-C4.4a VL | ESVLTQPPSVSGAPGQRVTISCTGSSSNIGAGYVVHWYQQLPGTAPKLLIYDNNKRPSGV PDRFSGSKSGTSASLAISGLRSEDEADYYCAAWDDRLNGPVFGGGTKLTVL |
| 919 | Anti-GCC CDR-H1 | GYYWS |
| 920 | Anti-GCC CDR-H2 | EINHRGNTNDNPSLKS |
| 921 | Anti-GCC CDR-H3 | ERGYTYGNFDH |
| 922 | Anti-GCC CDR-L1 | RASQSVSRNLA |
| 923 | Anti-GCC CDR-L2 | GASTRAT |
| 924 | Anti-GCC CDR-L3 | QQYKTWPRT |
| 925 | Anti-GCC VH | QVQLQQWGAGLLKPSETLSLTCAVFGGSFSGYYWSWIRQPPGKGLEWIGEINHRGNTNDN PSLKSRVTISVDTSKNQFALKLSSVTAADTAVYYCARERGYTYGNFDHWGQGTLVTVSS |
| 926 | Anti-GCC VL | EIVMTQSPATLSVSPGERATLSCRASQSVSRNLAWYQQKPGQAPRLLIYGASTRATGIPA RFSGSGSGTEFTLTIGSLQSEDFAVYYCQQYKTWPRTFGQGTNVEIK |
| 927 | Anti-Axl CDR-H1 | SYAMN |
| 928 | Anti-Axl CDR-H2 | TTSGSGASTYYADSVKG |
| 929 | Anti-Axl CDR-H3 | IWIAFDI |
| 930 | Anti-Axl CDR-L1 | RASQSVSSSYLA |
| 931 | Anti-Axl CDR-L2 | GASSRAT |
| 932 | Anti-Axl CDR-L3 | QQYGSSPYT |
| 933 | Anti-Axl VH | EVQLLESGGGLVQPGGSLRLSCAASGFTFSSYAMNWVRQAPGKGLEWVSTTSGSGASTYY ADSVKGRFTISRDNSKNTLYLQMNSLRAEDTAVYYCAKIWIAFDIWGQGTMVTVSS |
| 934 | Anti-Axl VL | EIVLTQSPGTLSLSPGERATLSCRASQSVSSSYLAWYQQKPGQAPRLLIYGASSRATGIP DRFSGSGSGTDFTLTISRLEPEDFAVYYCQQYGSSPYTFGQGTKLEIK |
| 935 | Anti-gpNMB CDR-H1 | SFNYYWS |
| 936 | Anti-gpNMB CDR-H2 | YIYYSGSTYSNPSLKS |
| 937 | Anti-gpNMB CDR-H3 | GYNWNYFDY |
| 938 | Anti-gpNMB CDR-L1 | RASQSVDNNLV |
| 939 | Anti-gpNMB CDR-L2 | GASTRAT |
| 940 | Anti-gpNMB CDR-L3 | QQYNNWPPWT |
| 941 | Anti-gpNMB VH | |
| | | |
| 942 | Anti-gpNMB VL | |
| 943 | Anti-gpNMB HC | QVQLQESGPGLVKPSQTLSLTCTVSGGSISSFNYYWSWIRHHPGKGLEWIGYIYYSGSTY SNPSLKSRVTISVDTSKNQFSLTLSSVTAADTAVYYCARGYNWNYFDYWGQGTLVTVSSA STKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVYTLPPSRDEL TKNQVSLTCLVKGFYPSDIAVEWESNGQPENNYKTTPPVLDSDGSFFLYSKLTVDKSRWQ QGNVFSCSVMHEALHNHYTQKSLSLSPGK |
| 944 | Anti-gpNMB LC | EIVMTQSPATLSVSPGERATLSCRASQSVDNNLVWYQQKPGQAPRLLIYGASTRATGIPA RFSGSGSGTEFTLTISSLQSEDFAVYYCQQYNNWPPWTFGQGTKVEIKRTVAAPSVFIFP PSDEQLKSGTASVVCLLNNFYPREAKVQWKVDNALQSGNSQESVTEQDSKDSTYSLSSTL TLSKADYEKHKVYACEVTHQGLSSPVTKSFNRGEC |
| 945 | Anti-Prolactin receptor CDR-H1 | TYWMH |
| 946 | Anti-Prolactin receptor CDR-H2 | EIDPSDSYSNYNQKFKD |
| 947 | Anti-Prolactin receptor CDR-H3 | NGGLGPAWFSY |
| 948 | Anti-Prolactin receptor CDR-L1 | KASQYVGTAVA |
| 949 | Anti-Prolactin receptor CDR-L2 | SASNRYT |
| 950 | Anti-Prolactin receptor CDR-L3 | QQYSSYPWT |
| 951 | Anti-Prolactin receptor VH | EVQLVQSGAEVKKPGSSVKVSCKASGYTFTTYWMHWVRQAPGQGLEWIGEIDPSDSYSNY NQKFKDRATLTVDKSTSTAYMELSSLRSEDTAVYYCARNGGLGPAWFSYWGQGTLVTVSS |
| 952 | Anti-Prolactin receptor VL | DIQMTQSPSSVSASVGDRVTITCKASQYVGTAVAWYQQKPGKSPKLLIYSASNRYTGVPS RFSDSGSGTDFTLTISSLQPEDFATYFCQQYSSYPWTFGGGTKVEIK |
| 953 | Anti-FGFR2 CDR-H1 | SYAMS |
| 954 | Anti-FGFR2 CDR-H2 | AISGSGTSTYYADSVKG |
| 955 | Anti-FGFR2 CDR-H3 | VRYNWNHGDWFDP |
| 956 | Anti-FGFR2 CDR-L1 | SGSSSNIGNNYVS |
| 957 | Anti-FGFR2 CDR-L2 | ENYNRPA |
| 958 | Anti-FGFR2 CDR-L3 | SSWDDSLNYWV |
| 959 | Anti-FGFR2 VH | EVQLLESGGGLVQPGGSLRLSCAASGFTFSSYAMSWVRQAPGKGLEWVSAISGSGTSTYY ADSVKGRFTISRDNSKNTLYLQMNSLRAEDTAVYYCARVRYNWNHGDWFDPWGQGTLVTV SS |
| 960 | Anti-FGFR2 VL | QSVLTQPPSASGTPGQRVTISCSGSSSNIGNNYVSWYQQLPGTAPKLLIYENYNRPAGVP DRFSGSKSGTSASLAISGLRSEDEADYYCSSWDDSLNYWVFGGGTKLTVL |
| 961 | Anti-CDCP1 CDR-H1 | SYGMS |
| 962 | Anti-CDCP1 CDR-H2 | TISSGGSYKYYVDSVKG |
| 963 | Anti-CDCP1 CDR-H3 | HPDYDGVWFAY |
| 964 | Anti-CDCP1 CDR-L1 | SVSSSVFYVH |
| 965 | Anti-CDCP1 CDR-L2 | DTSKLAS |
| 966 | Anti-CDCP1 CDR-L3 | QQWNSNPPT |
| 967 | Anti-CDCP1 VH | EVQLVESGGGLVQPGGSLRLSCAASGFTFNSYGMSWVRQAPGKGLEWVATISSGGSYKYY VDSVKGRFTISRDNAKNSLYLQMNSLRAEDTAVYYCARHPDYDGVWFAYWGQGTLVTVSS |
| 968 | Anti-CDCP1 VL | DIQMTQSPSSLSASVGDRVTITCSVSSSVFYVHWYQQKPGKAPKLLIYDTSKLASGVPSR FSGSGSGTDFTFTISSLQPEDIATYYCQQWNSNPPTFGGGTKVEIK |
| 969 | Anti-CDCP1 CDR-H1 | SYGMS |
| 970 | Anti-CDCP1 CDR-H2 | TISSGGSYTYYPDSVKG |
| 971 | Anti-CDCP1 CDR-H3 | HPDYDGVWFAY |
| 972 | Anti-CDCP1 CDR-L1 | SVSSSVFYVH |
| 973 | Anti-CDCP1 CDR-L2 | DTSKLAS |
| 974 | Anti-CDCP1 CDR-L3 | QQWNSNPPT |
| 975 | Anti-CDCP1 VH | EVQLVESGGDLVKPGGSLKLSCAASGFTFNSYGMSWVRQTPDKRLEWVATISSGGSYTYY PDSVKGRFTISRDNAKNTLYLQMSSLKSEDTAMYYCARHPDYDGVWFAYWGQGTLVTVSA |
| 976 | Anti-CDCP1 VL | QIVLTQSPAIMSASPGEKVTMTCSVSSSVFYVHWYQQKSGTSPKRWIYDTSKLASGVPAR FSGSGSGTSYSLTISSMEAEDAATYYCQQWNSNPPTFGGGTKLEIK |
| 977 | Anti-CDCP1 CDR-H1 | SYYMH |
| 978 | Anti-CDCP1 CDR-H2 | IINPSGGSTSYAQKFQG |
| 979 | Anti-CDCP1 CDR-H3 | DGVLRYFDWLLDYYYYMDV |
| 980 | Anti-CDCP1 CDR-L1 | RASQSVGSYLA |
| 981 | Anti-CDCP1 CDR-L2 | DASNRAT |
| 982 | Anti-CDCP1 CDR-L3 | QQRANVFT |
| 983 | Anti-CDCP1 VH | EVQLVQSGAEVKKPGASVKVSCKASGYTFTSYYMHWVRQAPGQGLEWMGIINPSGGSTSY AQKFQGRVTMTRDTSTSTVYMELSSLRSEDTAVYYCARDGVLRYFDWLLDYYYYMDVWGK GTTVTVSS |
| 984 | Anti-CDCP1 VL | EIVLTQSPATLSLSPGERATLSCRASQSVGSYLAWYQQRPGQAPRLLIYDASNRATGIPA RFSGSGSGTDFTLTISSLEPEDFAVYYCQQRANVFTFGQGTKVEIK |
| 985 | Anti-CDCP1 CDR-H1 | SYYMH |
| 986 | Anti-CDCP1 CDR-H2 | IINPSGGSTSYAQKFQG |
| 987 | Anti-CDCP1 CDR-H3 | DAELRHFDHLLDYHYYMDV |
| 988 | Anti-CDCP1 CDR-L1 | RASQSVGSYLA |
| 989 | Anti-CDCP1 CDR-L2 | DASNRAT |
| 990 | Anti-CDCP1 CDR-L3 | QQRAQEFT |
| 991 | Anti-CDCP1 VH | EVQLVQSGAEVKKPGASVKVSCKASGYTFTSYYMHWVRQAPGQGLEWMGIINPSGGSTSY AQKFQGRVTMTRDTSTSTVYMELSSLRSEDTAVYYCARDAELRHFDHLLDYHYYMDVWGQ GTTVTVSS |
| 992 | Anti-CDCP1 VL | EIVMTQSPATLSLSPGERATLSCRASQSVGSYLAWYQQKPGQAPRLLIYDASNRATGIPA RFSGSGSGTDFTLTISSLQPEDFAVYYCQQRAQEFTFGQGTKVEIK |
| 993 | Anti-ASCT2 VH | QVQLVQSGSELKKPGAPVKVSCKASGYTFSTFGMSWVRQAPGQGLKWMGWIHTYAGVPIY GDDFKGRFVFSLDTSVSTAYLQISSLKAEDTAVYFCARRSDNYRYFFDYWGQGTTVTVSS |
| 994 | Anti-ASCT2 VL | DIQMTQSPSSLSASLGDRVTITCRASQDIRNYLNWYQQKPGKAPKLLIYYTSRLHSGVPS RFSGSGSGTDYTLTISSLQPEDFATYFCQQGHTLPPTFGQGTKLEIK |
| 995 | Anti-ASCT2 VH | QIQLVQSGPELKKPGAPVKISCKASGYTFTTFGMSWVKQAPGQGLKWMGWIHTYAGVPIY GDDFKGRFVFSLDTSVSTAYLQISSVKAEDTATYFCARRSDNYRYFFDYWGQGTTLTVSS |
| 996 | Anti-ASCT2 VL | DIQMTQSPSSLSASLGDRVTITCRASQDIRNYLNWYQQKPGKAPKLLIYYTSRLHSGVPS RFSGSGSGTDYTLTISSLQPEDFATYFCQQGHTLPPTFGQGTKLEIK |
| 997 | Anti-ASCT2 CDR-H1 | NYYMA |
| 998 | Anti-ASCT2 CDR-H2 | SITKGGGNTYYRDSVKG |
| 999 | Anti-ASCT2 CDR-H3 | QVTIAAVSTSYFDS |
| 1000 | Anti-ASCT2 CDR-L1 | KTNQKVDYYGNSYVY |
| 1001 | Anti-ASCT2 CDR-L2 | LASNLAS |
| 1002 | Anti-ASCT2 CDR-L3 | QQSRNLPYT |
| 1003 | Anti-ASCT2 VH | EVQLVESGGGLVQSGRSIRLSCAASGFSFSNYYMAWVRQAPSKGLEWVASITKGGGNTYY RDSVKGRFTFSRDNAKSTLYLQMDSLRSEDTATYYCARQVTIAAVSTSYFDSWGQGVMVT VSS |
| 1004 | Anti-ASCT2 VL | DIVLTQSPALAVSLGQRATISCKTNQKVDYYGNSYVYWYQQKPGQQPKLLIYLASNLASG IPARFSGRGSGTDFTLTIDPVEADDTATYYCQQSRNLPYTFGAGTKLELK |
| 1005 | Anti-CD123 CDR-H1 | DYYMK |
| 1006 | Anti-CD123 CDR-H2 | DIIPSNGATFYNQKFKG |
| 1007 | Anti-CD123 CDR-H3 | SHLLRASWFAY |
| 1008 | Anti-CD123 CDR-L1 | KSSQSLLNSGNQKNYLT |
| 1009 | Anti-CD123 CDR-L2 | WASTRES |
| 1010 | Anti-CD123 CDR-L3 | QNDYSYPYT |
| 1011 | Anti-CD123 VH | QVQLVQSGAEVKKPGASVKMSCKASGYTFTDYYMKWVKQAPGQGLEWIGDIIPSNGATFY NQKFKGKATLTVDRSISTAYMHLNRLRSDDTAVYYCTRSHLLRASWFAYWGQGTLVTVSS |
| 1012 | Anti-CD123 VL | DFVMTQSPDSLAVSLGERATINCKSSQSLLNSGNQKNYLTWYLQKPGQPPKLLIYWASTR ESGVPDRFSGSGSGTDFTLTISSLQAEDVAVYYCQNDYSYPYTFGQGTKLEIK |
| 1013 | Anti-GPC3 CDR-H1 | DYEMH |
| 1014 | Anti-GPC3 CDR-H2 | GIDPETGGTAYNQKFKG |
| 1015 | Anti-GPC3 CDR-H3 | YYSFAY |
| 1016 | Anti-GPC3 CDR-L1 | RSSQSIVHSNANTYLQ |
| 1017 | Anti-GPC3 CDR-L2 | KVSNRFS |
| 1018 | Anti-GPC3 CDR-L3 | FQVSHVPYT |
| 1019 | Anti-GPC3 VH | EVQLVQSGAEVKKPGATVKISCKVSGYTFTDYEMHWVQQAPGKGLEWMGGIDPETGGTAY NQKFKGRVTLTADKSTDTAYMELSSLRSEDTAVYYCGRYYSFAYWGQGTLVTVSS |
| 1020 | Anti-GPC3 VL | DVVMTQSPLSLPVTLGQPASISCRSSQSIVHSNANTYLQWFQQRPGQSPRLLIYKVSNRF SGVPDRFSGSGSGTDFTLKISRVEAEDVGVYYCFQVSHVPYTFGQGTKLEIK |
| 1021 | Anti-TIGIT CDR-H1 | SYAIS |
| 1022 | Anti-TIGIT CDR-H2 | SIIPIFGTANYAQKFQG |
| 1023 | Anti-TIGIT CDR-H3 | GPSEVGAILGYVWFDP |
| 1024 | Anti-TIGIT CDR-L1 | RSSQSLLHSNGYNYLD |
| 1025 | Anti-TIGIT CDR-L2 | LGSNRAS |
| 1026 | Anti-TIGIT CDR-L3 | MQARRIPIT |
| 1027 | Anti-TIGIT VH | QVQLVQSGAEVKKPGSSVKVSCKASGGTFSSYAISWVRQAPGQGLEWMGSIIPIFGTANY AQKFQGRVTITADESTSTAYMELSSLRSEDTAVYYCARGPSEVGAILGYVWFDPWGQGTL VTVSS |
| 1028 | Anti-TIGIT VL | DIVMTQSPLSLPVTPGEPASISCRSSQSLLHSNGYNYLDWYLQKPGQSPQLLIYLGSNRA SGVPDRFSGSGSGTDFTLKISRVEAEDVGVYYCMQARRIPITFGGGTKVEIK |
| 1029 | Anti-CD33 CDR-H1 | NYDIN |
| 1030 | Anti-CD33 CDR-H2 | WIYPGDGSTKYNEKFKA |
| 1031 | Anti-CD33 CDR-H3 | GYEDAMDY |
| 1032 | Anti-CD33 CDR-L1 | KASQDINSYLS |
| 1033 | Anti-CD33 CDR-L2 | RANRLVD |
| 1034 | Anti-CD33 CDR-L3 | LQYDEFPLT |
| 1035 | Anti-CD33 VH | QVQLVQSGAE VKKPGASVKV SCKASGYTFT NYDINWVRQA PGQGLEWIGW IYPGDGSTKY NEKFKAKATL TADTSTSTAY MELRSLRSDD TAVYYCASGY EDAMDYWGQG TTVTVSS |
| 1036 | Anti-CD33 VL | DIQMTQSPSSLSASVGDRVTINCKASQDINSYLSWFQQKPGKAPKTLIYRANRVDGVPSR FSGSGSGQDYTLT ISSLQPEDFATYYCLQYDEFPLTFGGGTKVEIK |
| 1037 | Anti-BCMA CDR-H1 | DYYIH |
| 1038 | Anti-BCMA CDR-H2 | YINPNSGYTNYAQKFQG |
| 1039 | Anti-BCMA CDR-H3 | YMWERVTGFFDF |
| 1040 | Anti-BCMA CDR-L1 | LASEDISDDLA |
| 1041 | Anti-BCMA CDR-L2 | TTSSLQS |
| 1042 | Anti-BCMA CDR-L3 | QQTYKFPPT |
| 1043 | Anti-BCMA VH | QVQLVQSGAEVKKPGASVKLSCKASGYTFTDYYIHWVRQAPGQGLEWIGYINPNSGYTNY AQKFQGRATMTADKSINTAYVELSRLRSDDTAVYFCTRYMWERVTGFFDFWGQGTMVTVS S |
| 1044 | Anti-BCMA VL | DIQMTQSPSSVSASVGDRVTITCLASEDISDDLAWYQQKPGKAPKVLVYTTSSLQSGVPS RFSGSGSGTDFTLTISSLQPEDFATYFCQQTYKFPPTFGGGTKVEIK |

## Claims

1. A compound of Formula (I): 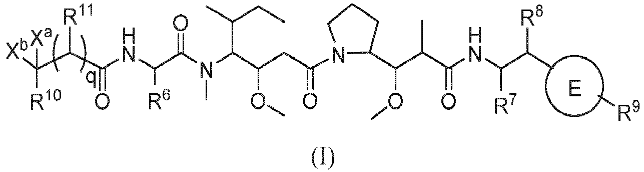 or a salt thereof, wherein
X^{b} is -NR¹R²; and X^{a} is 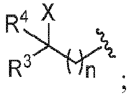 or
X^{a} and X^{b} are taken together with the carbon atom to which they are attached to form 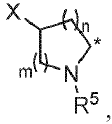 wherein the asterisk denotes the carbon atom of Formula (I) that bears the X^{a} and X^{b} groups;
R¹, R², R³, R⁴, R^{a}, R^{b}, R⁵, and R¹⁰ are each independently H or C₁-C₄ alkyl;
X is H, OH, -C(O)NR^{a}R^{b}, -S(O)₂R^{a}, -S(O)-R^{a} -S(O)₂NR^{a}R^{b}, -NHS(O)₂R^{a}, or -NHC(O)R^{a};
R⁶ is C₁-C₄ alkyl optionally substituted with OH;
R⁷ is H, C₁-C₄ alkyl optionally substituted with one or two OH moieties, or 5-6 membered heteroaryl;
E is phenyl or 5-6 membered heteroaryl;
R⁸, R⁹, and R¹¹ are each independently H or OH;
n is 0, 1, 2, or 3;
m is 1, 2, 3, or 4; and
q is 0 or 1,
wherein when X is H, at least two of R⁷, R⁸, R⁹, and R¹¹ comprise an OH moiety.

2. A compound of Formula (II): 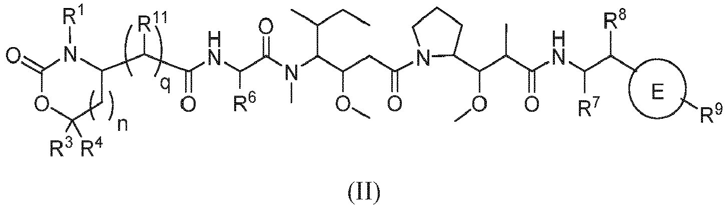 or a salt thereof, wherein
R¹, R³, and R⁴ are independently H or C₁-C₄ alkyl;
R⁶ is C₁-C₄ alkyl optionally substituted with OH;
R⁷ is H, C₁-C₄ alkyl optionally substituted with one or two OH moieties, or 5-6 membered heteroaryl;
E is phenyl or 5-6 membered heteroaryl;
R⁸, R⁹, and R¹¹ are each independently H or OH;
n is 0, 1, or 2; and
q is 0 or 1.

3. The compound of claim 1, or a salt thereof, wherein
X^{a} is 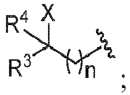
X^{b} is -NR¹R²;
R¹ is H or methyl;
R² is methyl;
X is OH;
R³ and R⁴ are H;
R⁶ is isopropyl;
R⁷ is -CH₂OH;
R⁸ is H;
E is phenyl; and
R⁹ is H.

4. The compound of claim 1, wherein the compound is 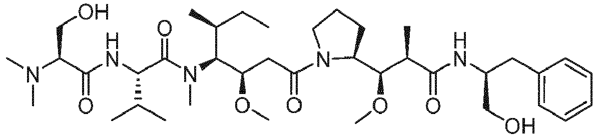 or a salt thereof.

5. A Drug-Linker compound of the following formula:
Q-D,
or a salt thereof, wherein
Q is a Linker Unit selected from the group consisting of:
(i) Z'-A-RL-,
(ii) Z'-A-RL-Y-,
(iii) Z'-A-S^{∗}-RL-,
(iv) Z'-A-S^{∗}-RL-Y-,
(v) Z'-A-B(S^{∗})-RL-,
(vi) Z'-A-B(S^{∗})-RL-Y-,
(vii) Z'-A-,
(viii) Z'-A-S^{∗}-W-,
(ix) Z'-A-B(S^{∗})-W-,
(x) Z'-A-S^{∗}-W-RL-, and
(xi) Z'-A-B(S^{∗})-W-RL-;
Z' is a Stretcher Unit precursor;
A is a bond or a Connector Unit;
B is a Parallel Connector Unit;
S^{∗} is a Partitioning Agent;
RL is a Releasable Linker;
W is an Amino Acid Unit;
Y is a Spacer Unit; and
D is a Drug Unit of Formula (I'): 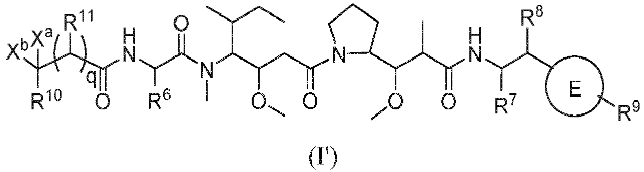 wherein
X^{b} is -NR²-^{#} or -N⁺R¹R⁵-^{#}, wherein the # denotes the point of attachment to Q, and X^{a} 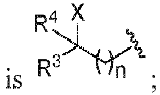 or
X^{a} and X^{b} are taken together with the carbon atom to which they are attached to form 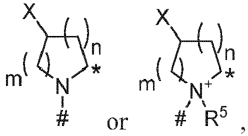 wherein the asterisk denotes the carbon atom of Formula (I) that bears the X^{a} and X^{b} groups, and the # denotes the point of attachment to Q;
R¹ and R⁵ are independently C₁-C₄ alkyl;
X is H, OH, -C(O)NR^{a}R^{b}, -S(O)₂R^{a}, -S(O)-R^{a} -S(O)₂NR^{a}R^{b}, -NHS(O)₂R^{a}, or -NHC(O)R^{a};
R², R³, R⁴, R¹⁰, R^{a}, and R^{b} are each independently H or C₁-C₄ alkyl;
R⁶ is C₁-C₄ alkyl optionally substituted with OH;
R⁷ is H, C₁-C₄ alkyl optionally substituted with one or two OH moieties, or 5-6 membered heteroaryl;
E is phenyl or 5-6 membered heteroaryl;
R⁸, R⁹, and R¹¹ are each independently H or OH;
n is 0, 1, 2, or 3;
m is 1, 2, 3, or 4;
q is 0 or 1; and
wherein when X is H, at least two of R⁷, R⁸, R⁹, and R¹¹ comprise an OH moiety.

6. The Drug-Linker compound of claim 5, wherein the compound is 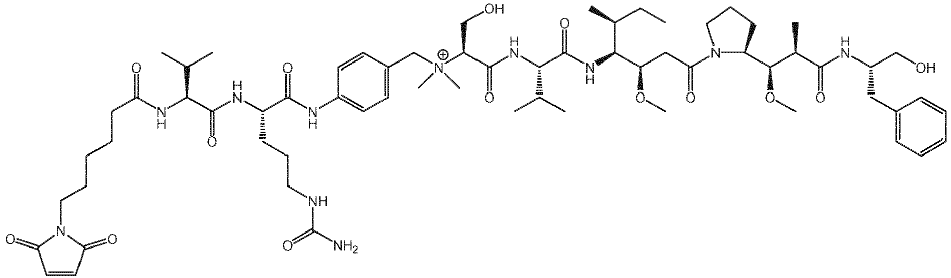 or a salt thereof.

7. The Drug-Linker compound of claim 5, wherein the compound is 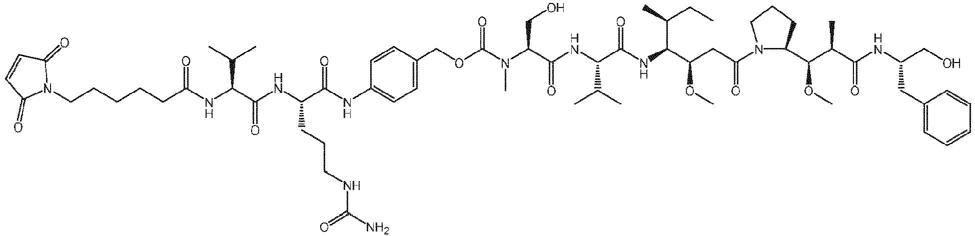 or 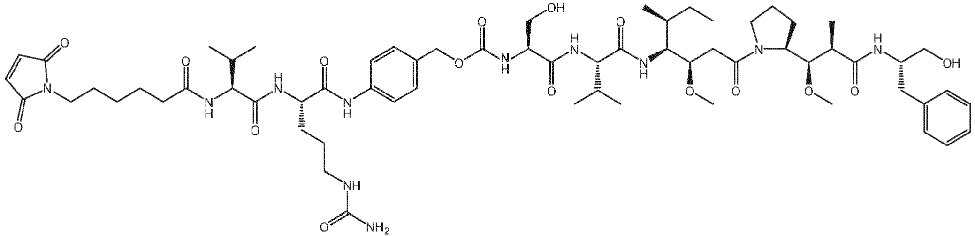 or a salt thereof.

8. A Ligand-Drug Conjugate compound of the formula:
L-(Q-D)ₚ
or a pharmaceutically acceptable salt thereof, wherein
L is a Ligand Unit;
Q is a Linker Unit selected from the group consisting of:
(i) Z'-A-RL-,
(ii) Z'-A-RL-Y-,
(iii) Z'-A-S^{∗}-RL-,
(iv) Z'-A-S^{∗}-RL-Y-,
(v) Z'-A-B(S^{∗})-RL-,
(vi) Z'-A-B(S^{∗})-RL-Y-,
(vii) Z'-A-,
(viii) Z'-A-S^{∗}-W-,
(ix) Z'-A-B(S^{∗})-W-,
(x) Z'-A-S^{∗}-W-RL-, and
(xi) Z'-A-B(S^{∗})-W-RL-;
Z' is a Stretcher Unit precursor;
A is a bond or a Connector Unit;
B is a Parallel Connector Unit;
S^{∗} is a Partitioning Agent;
RL is a Releasable Linker;
W is a Amino Acid Unit;
Y is a Spacer Unit; and
D is a Drug Unit of Formula (I'): 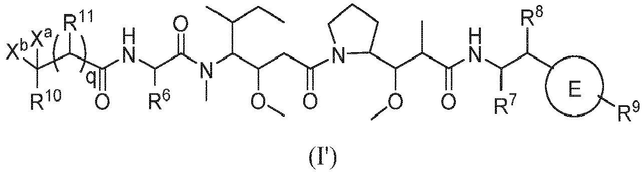 wherein
X^{b} is -NR²-^{#} or -N⁺R¹R⁵-^{#}, wherein the # denotes the point of attachment to Q, and X^{a} is 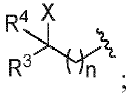 or
X^{a} and X^{b} are taken together with the carbon atom to which they are attached to form 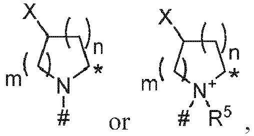 wherein the asterisk denotes the carbon atom of Formula (I) that bears the X^{a} and X^{b} groups, and the # denotes the point of attachment to Q;
R¹ and R⁵ are independently C₁-C₄ alkyl;
X is H, OH, -C(O)NR^{a}R^{b}, -S(O)₂R^{a}, -S(O)-R^{a} -S(O)₂NR^{a}R^{b}, -NHS(O)₂R^{a}, or -NHC(O)R^{a};
R², R³, R⁴, R¹⁰, R^{a}, and R^{b} are each independently H or C₁-C₄ alkyl;
R⁶ is C₁-C₄ alkyl optionally substituted with OH;
R⁷ is H, C₁-C₄ alkyl optionally substituted with one or two OH moieties, or 5-6 membered heteroaryl;
E is phenyl or 5-6 membered heteroaryl;
R⁸, R⁹, and R¹¹ are each independently H or OH;
n is 0, 1, 2, or 3;
m is 1, 2, 3, or 4;
q is 0 or 1;
p is an integer ranging from 1 to 12; and
wherein when X is H, at least two of R⁷, R⁸, R⁹, and R¹¹ comprise an OH moiety.

9. The Ligand-Drug Conjugate compound of claim 8, wherein the compound is 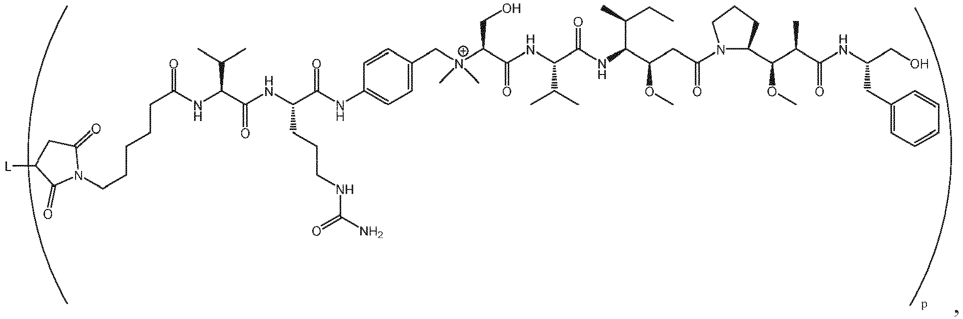 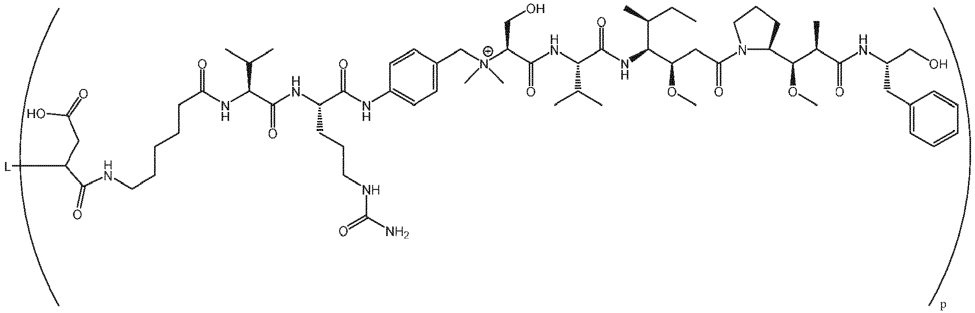 , or 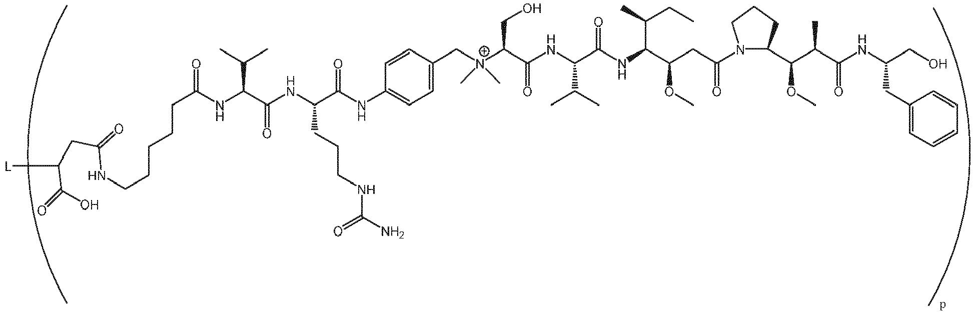 or a pharmaceutically acceptable salt thereof.

10. The Ligand-Drug Conjugate compound of claim 8, wherein the compound is 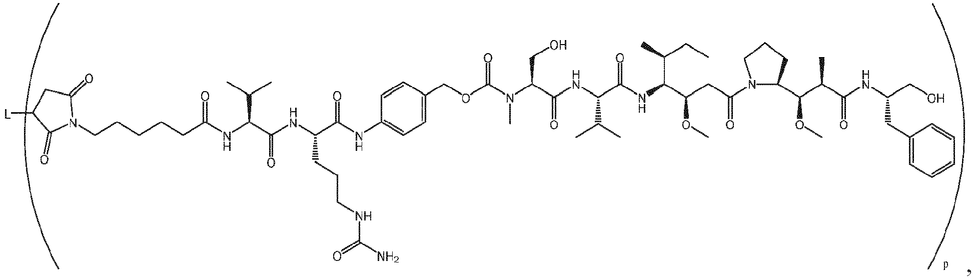 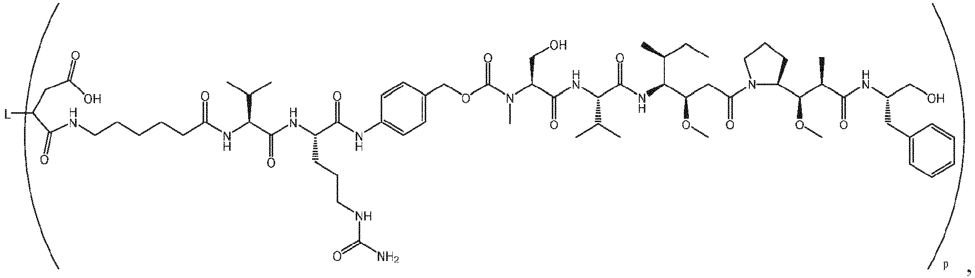 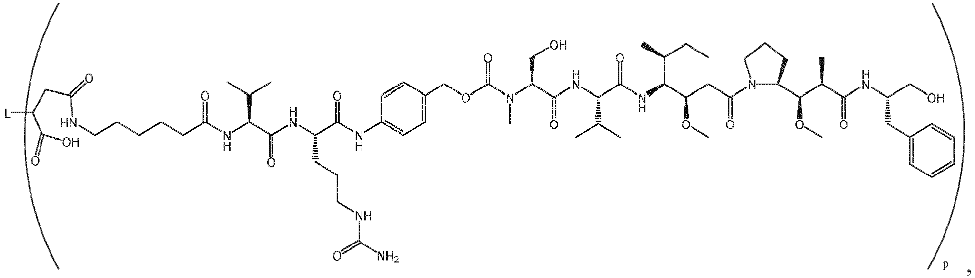 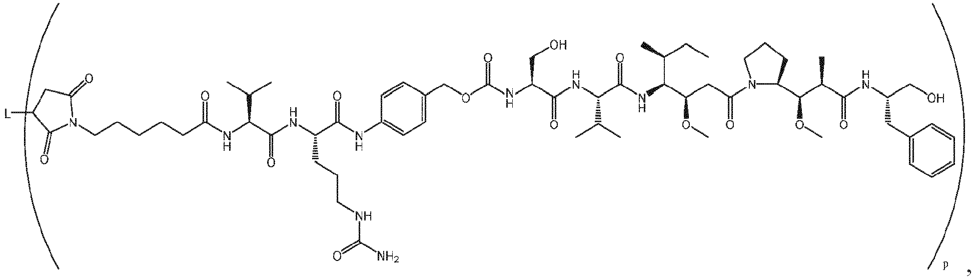 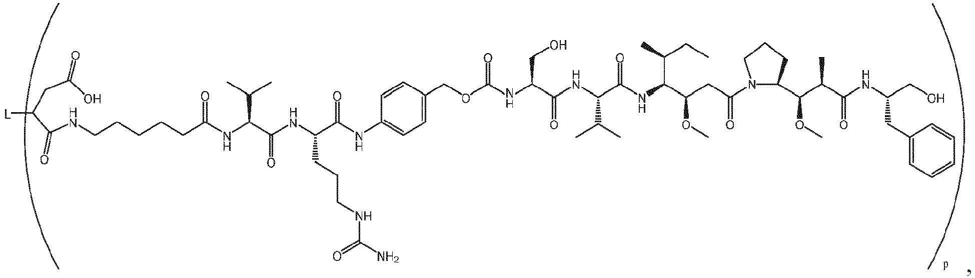 or 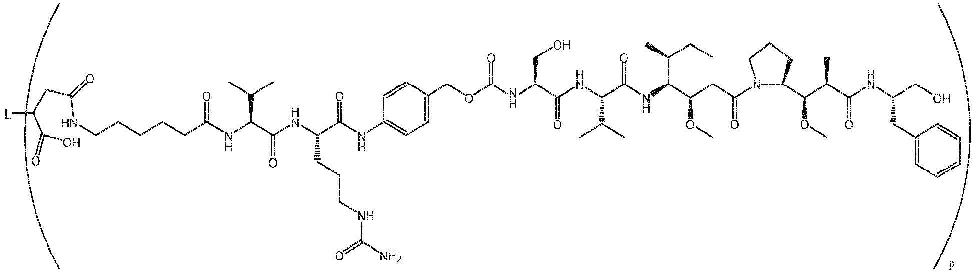 or a pharmaceutically acceptable salt thereof.

11. A pharmaceutical composition comprising the Ligand-Drug Conjugate compound of any one of claims 8-10 and a pharmaceutically acceptable excipient.

12. A method of treating cancer comprising administering a therapeutically effective amount of the Ligand-Drug Conjugate compound of any one of claims 8-10, or a pharmaceutically acceptable salt thereof, to a subject in need thereof.

13. A compound, wherein the compound is selected from the group consisting of the compounds listed in Table 1 or a salt thereof.

14. A Drug-Linker compound, wherein the Drug-Linker compound is selected from the group consisting of the compounds listed in Table 2 or a salt thereof.

15. A Ligand-Drug Conjugate compound, wherein the Ligand-Drug Conjugate compound is selected from the group consisting of the compounds listed in Table 3, or a salt thereof, and p is an integer ranging from 1 to 12.
